# EUROPEAN PATENT APPLICATION

(11) **EP 4 609 857 A1**
(43) Date of publication of application: **03.09.2025**
(21) Application number: 23886235.3
(22) Date of filing: 31.10.2023
(51) Int. Cl.: A61K 9/107, A61K 47/34, A61K 47/32, A61K 31/7088, C12N 15/88

(54) **POLYMER-BASED NUCLEIC ACID MOLECULE DELIVERY VEHICLE HAVING IONIZATION MOIETY**

(30) Priority: 01.11.2022 KR 20220143417
(71) Applicant: POSTECH Research and Business Development Foundation, Pohang-si, Gyeongsangbuk-do 37673 (KR); Omniamed Co., Ltd, Pohang-si Gyeongsangbuk-do 37673 (KR)
(72) Inventor: KIM, Won Jong, Pohang-si Gyeongsangbuk-do 37673 (KR); KIM, Hong Lyun, Pohang-si Gyeongsangbuk-do 37673 (KR)
(74) Representative: Prüfer & Partner mbB Patentanwälte · Rechtsanwälte
(86) International application number: PCT/KR2023/017176
(87) International publication number: WO 2024/096539

(57) **Abstract**

The present invention relates to a polymer-based nucleic acid molecule (e.g., mRNA) delivery vehicle having an ionizable moiety. Specifically, the present invention provides a novel polymer capable of delivering all negatively charged genetic materials to a desired site according to the monomers and the length of the polymer. The polymer according to the present invention can be effectively used in gene therapeutic agents or vaccines comprising genetic material, such as mRNA, for therapeutic or preventive purposes.

## Description

### Technical Field

The present invention relates to a polymer-based nucleic acid molecule delivery vehicle having an ionizable moiety, and more specifically, to a polymer based on poly beta-aminoester or poly beta-aminoacrylate. Furthermore, the polymer according to the present invention can provide high mRNA delivery efficiency through pH sensitivity by attaching PEG to both ends to synthesize a triple copolymer.

### Background Art

mRNA is a substance that carries genetic information for producing proteins in the human body. Through mRNA delivery, it is possible to induce the production of proteins that are originally present in the human body, as well as the production of proteins that do not exist in the body. Since the outbreak of COVID-19, the development of mRNA delivery vehicles has been actively progressing as mRNA vaccines that activate immune responses through mRNA delivery have been utilized. Several mRNA delivery vehicles based on lipid nanoparticles have been developed, but they have problems due to rapid diffusion to organs other than the target site (off-target effect) and high immunogenicity, which can cause various side effects such as myocarditis and pericarditis. In addition, since vaccines are inoculated up to 2 to 4 times, the possibility of exposure to these side effects increases. Therefore, it is necessary to develop an mRNA delivery vehicle with high mRNA expression efficiency, low off-target effect, relatively low immunogenicity, and low side effects.

In order to improve the disadvantages of the above mRNA delivery vehicle, the present researchers studied mRNA delivery using polymers. As a result, a novel polymer that has pH sensitivity and has high mRNA delivery efficiency in vivo was newly discovered. Based on the above, the present researchers completed the present invention.

### Detailed Description of Invention

### Technical Problem

An object of the present invention is to provide a nucleic acid molecule delivery vehicle based on a polymer having pH sensitivity for high nucleic acid molecule delivery efficiency and low side effects.

One aspect of the object of the present invention is to provide a mRNA delivery vehicle based on a polymer having a relatively long duration of expression in order to overcome the short-term expression of mRNA in general.

Another object of the present invention is to provide a novel polymer for providing a nucleic acid molecule delivery vehicle based on a polymer having pH sensitivity.

### Solution to Problem

In order to achieve the above object, the present invention provides a polymer suitable for intracellular nucleic acid delivery, comprising a repeating unit of Formula 1 below: 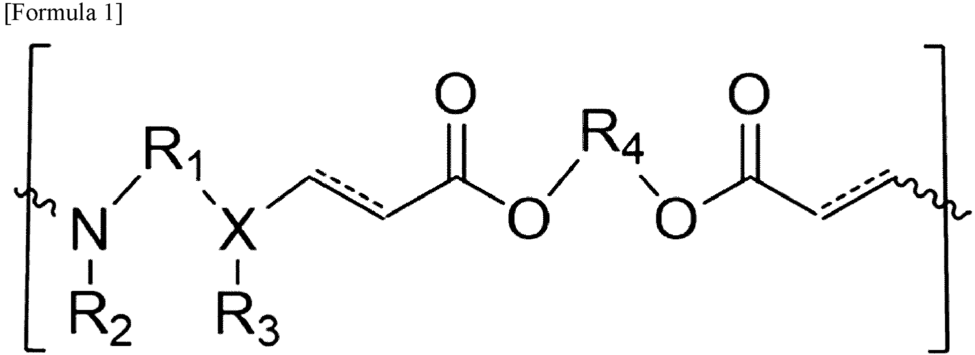 in the formula,
R1 is a bond or a C1-6 alkylene,
R2 and R3 are each independently (i) hydrogen, (ii) C1-6 alkyl which may be substituted with alkylamine or hydroxy, (iii) a 5- or 6-membered carbocycle or heterocycle, or (iv) C1-6 alkyl substituted with a 5- or 6-membered carbocycle or heterocycle,
said R2 and R3 may be linked together to form a C1-6 alkylene,
said 5- or 6-membered carbocycle or heterocycle may be substituted with C1-3 alkyl or C1-3 alkoxyacyl,
X is a nitrogen atom or a bond, and when X is a bond, said R3 is absent,
R4 is a linear or branched C1-15 alkylene, 1 to 5 carbon atoms of said alkylene may be replaced by oxygen or sulfur, and
- - - - - may represent a single bond or a double bond.

The above repeating unit may be n (an integer between 1 and 500), and in this case, Formula 1 may be represented as follows. 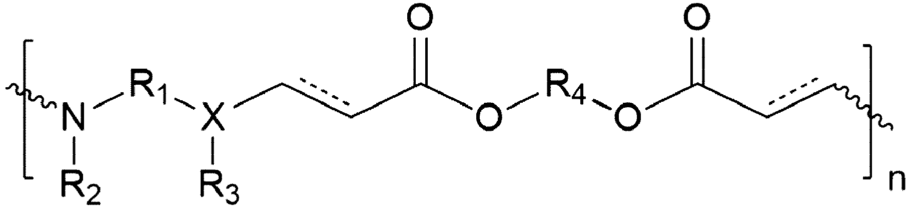 (the definitions of the groups are as defined in relation to Formula 1 above)

The present invention provides a polymer having a repeating unit of Formula 2 below: 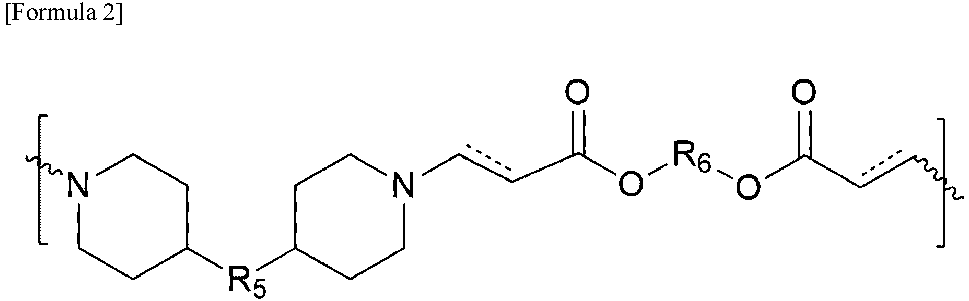 in the formula,
R5 is a C1-6 alkylene,
R6 is a linear or branched C1-15 alkylene, 1 to 5 carbon atoms of said alkylene may be replaced by oxygen or sulfur, and
- - - - - may represent a single bond or a double bond.

The above repeating unit may be n (an integer between 1 and 500), and in this case, Formula 2 may be represented as follows. 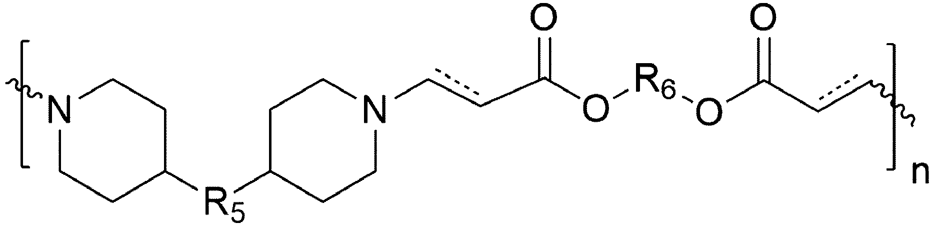 (the definitions of the groups are as defined in relation to Formula 2 above)

In one embodiment, the polymer may be a polymer in which the repeating unit of Formula 1 is formed by a reaction between any one of the monomers of Formulas 3 to 13 below and any one of the monomers of Formulas 14 to 23 below.

| Formula | Structure |
|---|---|
| 3 | 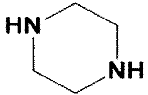 |
| 4 | 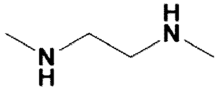 |
| 5 | 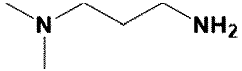 |
| 6 | 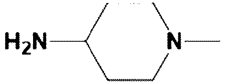 |
| 7 | 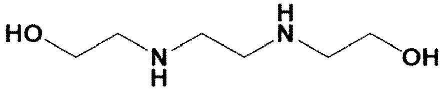 |
| 8 | 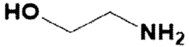 |
| 9 | 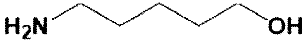 |
| 10 | 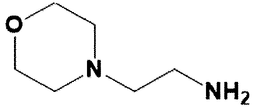 |
| 11 | 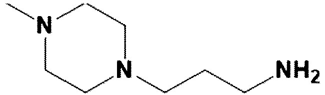 |
| 12 | 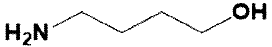 |
| 13 | 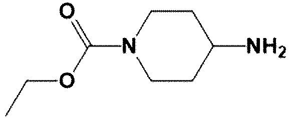 |
| 14 | 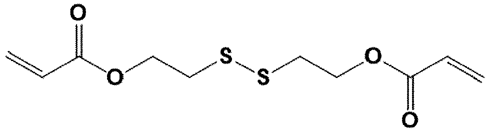 |
| 15 | 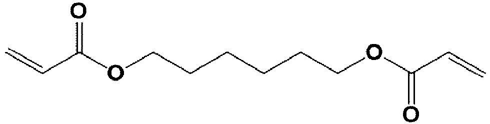 |
| 16 | 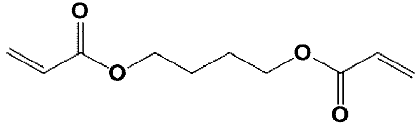 |
| 17 | 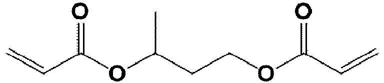 |
| 18 | 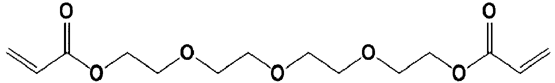 |
| 19 | 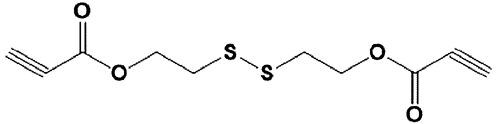 |
| 20 | 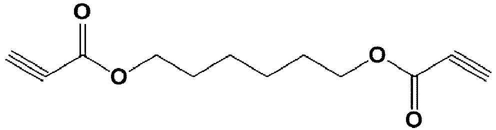 |
| 21 | 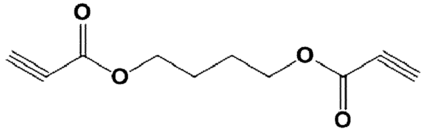 |
| 22 | 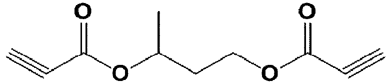 |
| 23 | 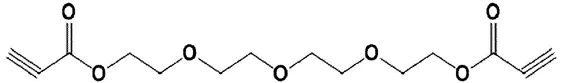 |

In one embodiment, the polymer may be a polymer in which the repeating unit of Formula 2 is formed by a reaction between a monomer of Formula 24 below and any one of the monomers of Formulas 14 to 23 below.

| Formula | Structure |
|---|---|
| 14 | 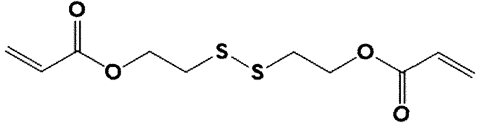 |
| 15 | 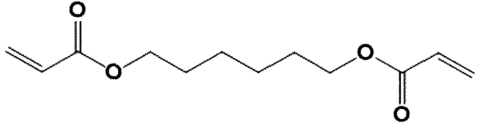 |
| 16 | 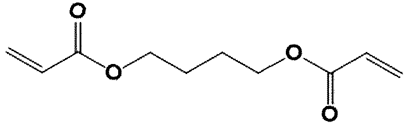 |
| 17 | 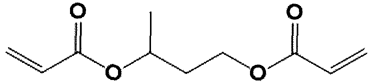 |
| 18 | 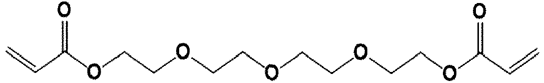 |
| 19 | 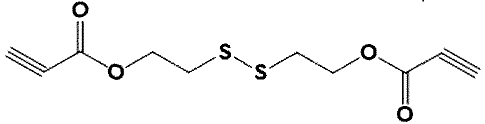 |
| 20 | 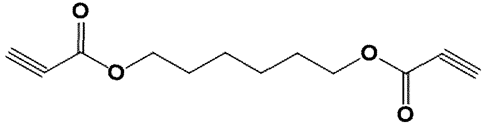 |
| 21 | 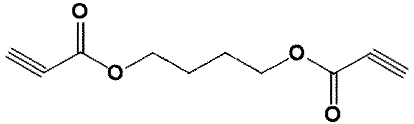 |
| 22 | 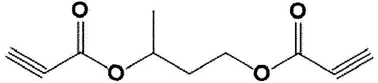 |
| 23 | 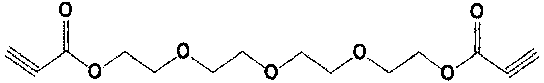 |
| 24 | 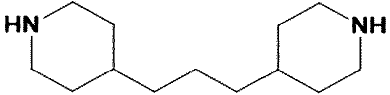 |

The present invention provides a polymer comprising a repeating unit selected from the group consisting of repeating units set forth in the table below.

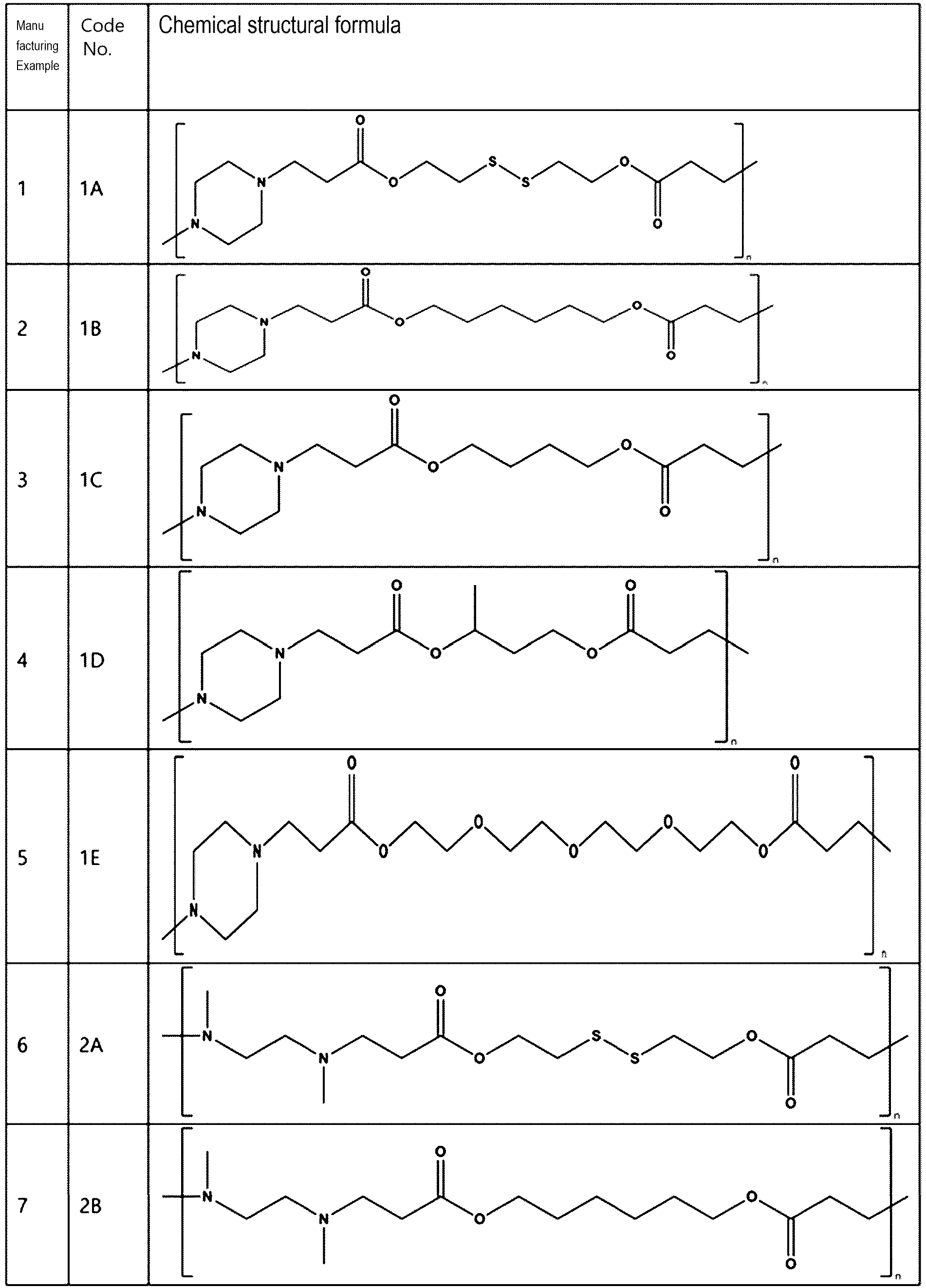

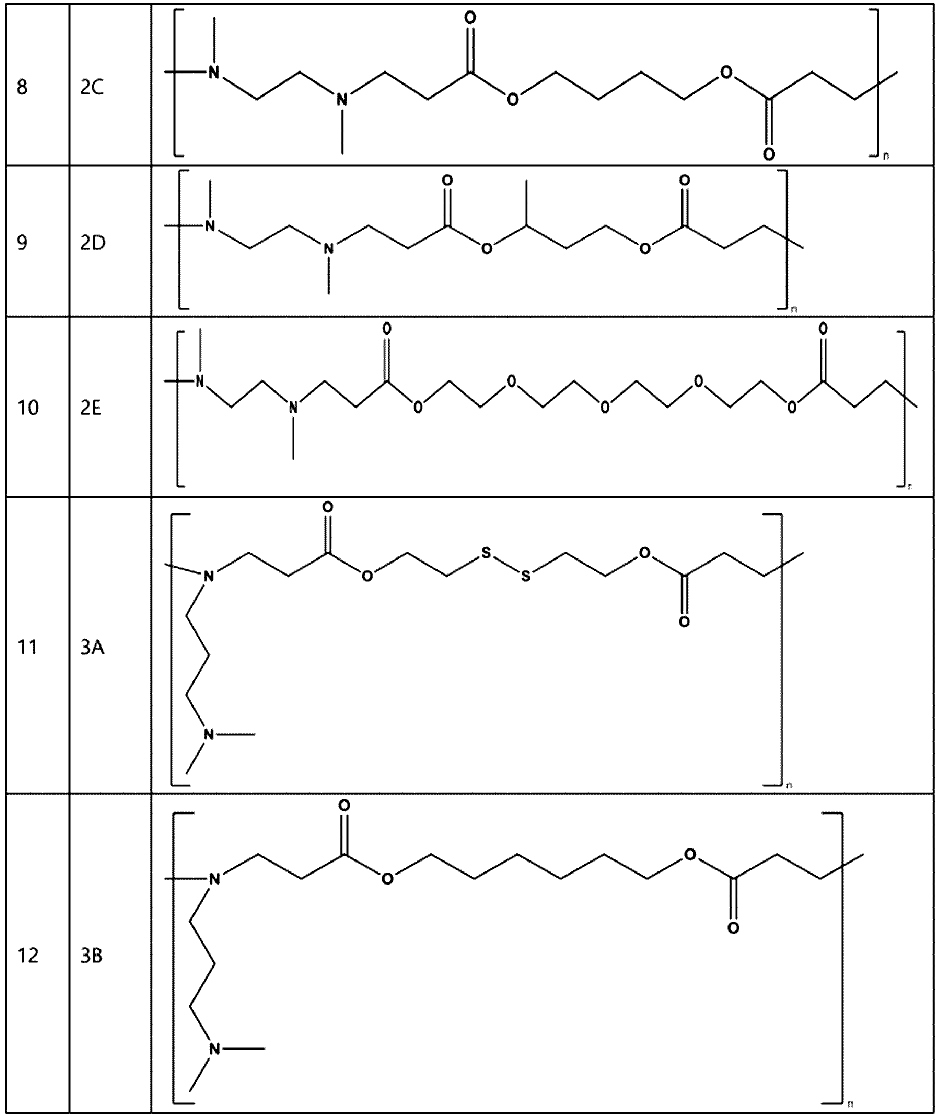

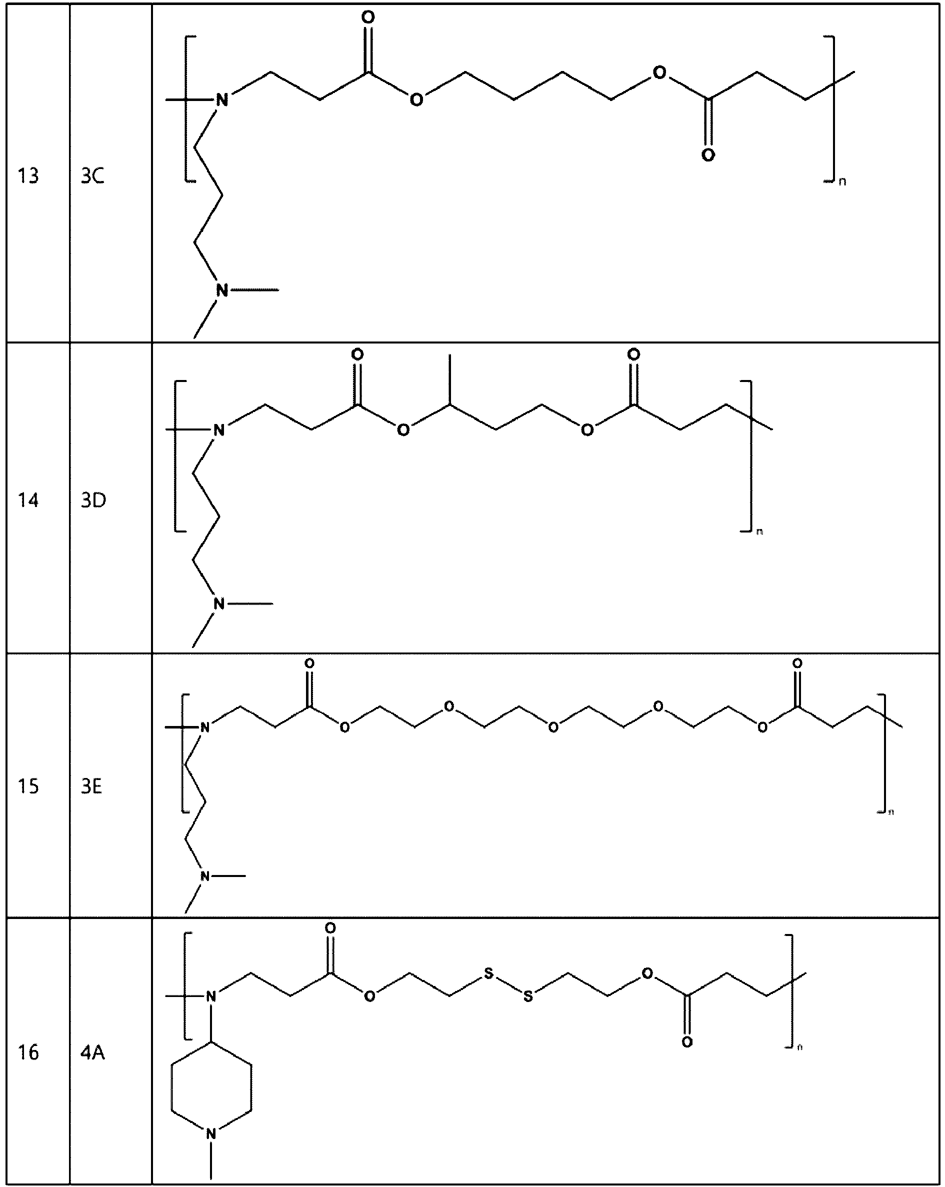

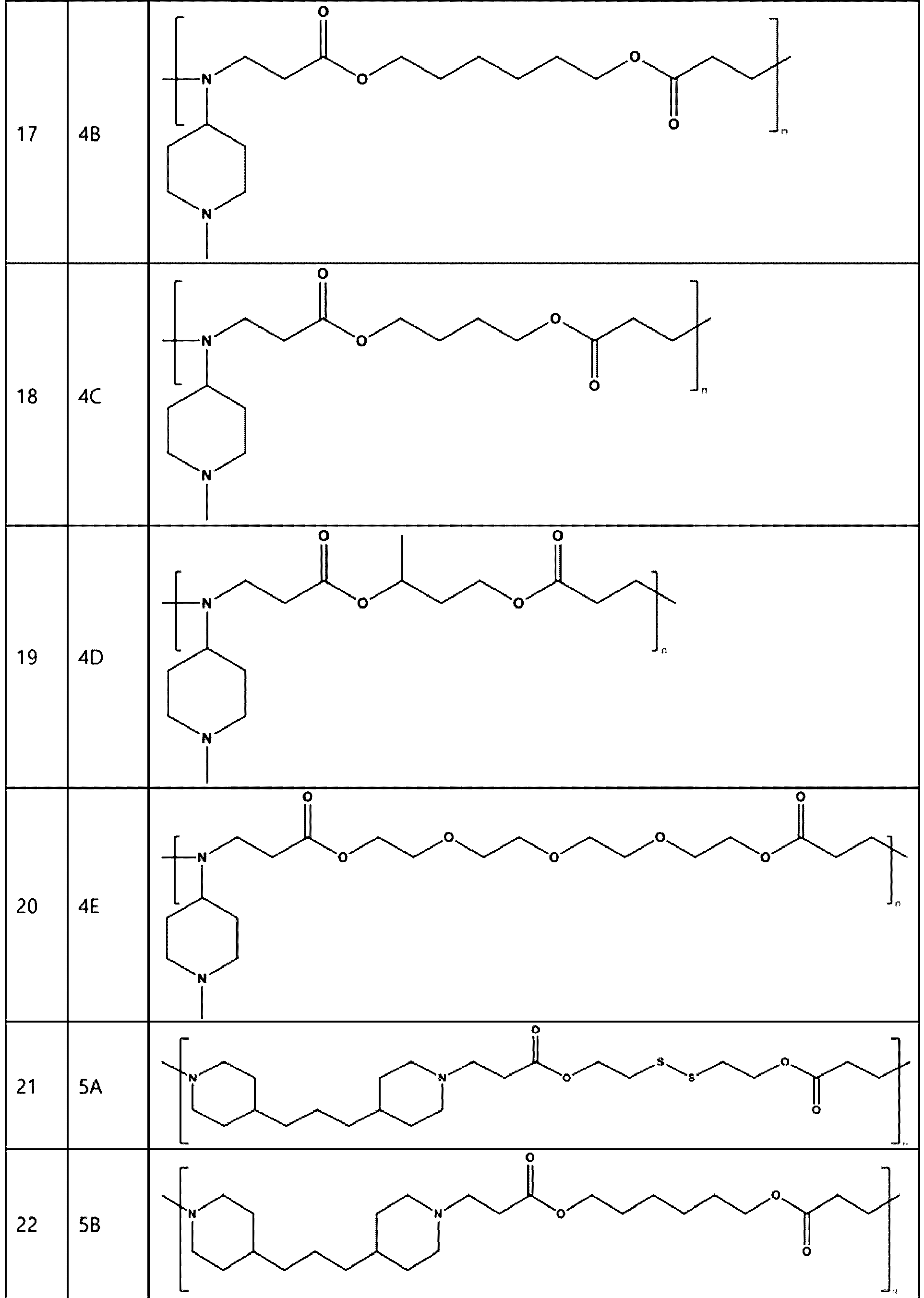

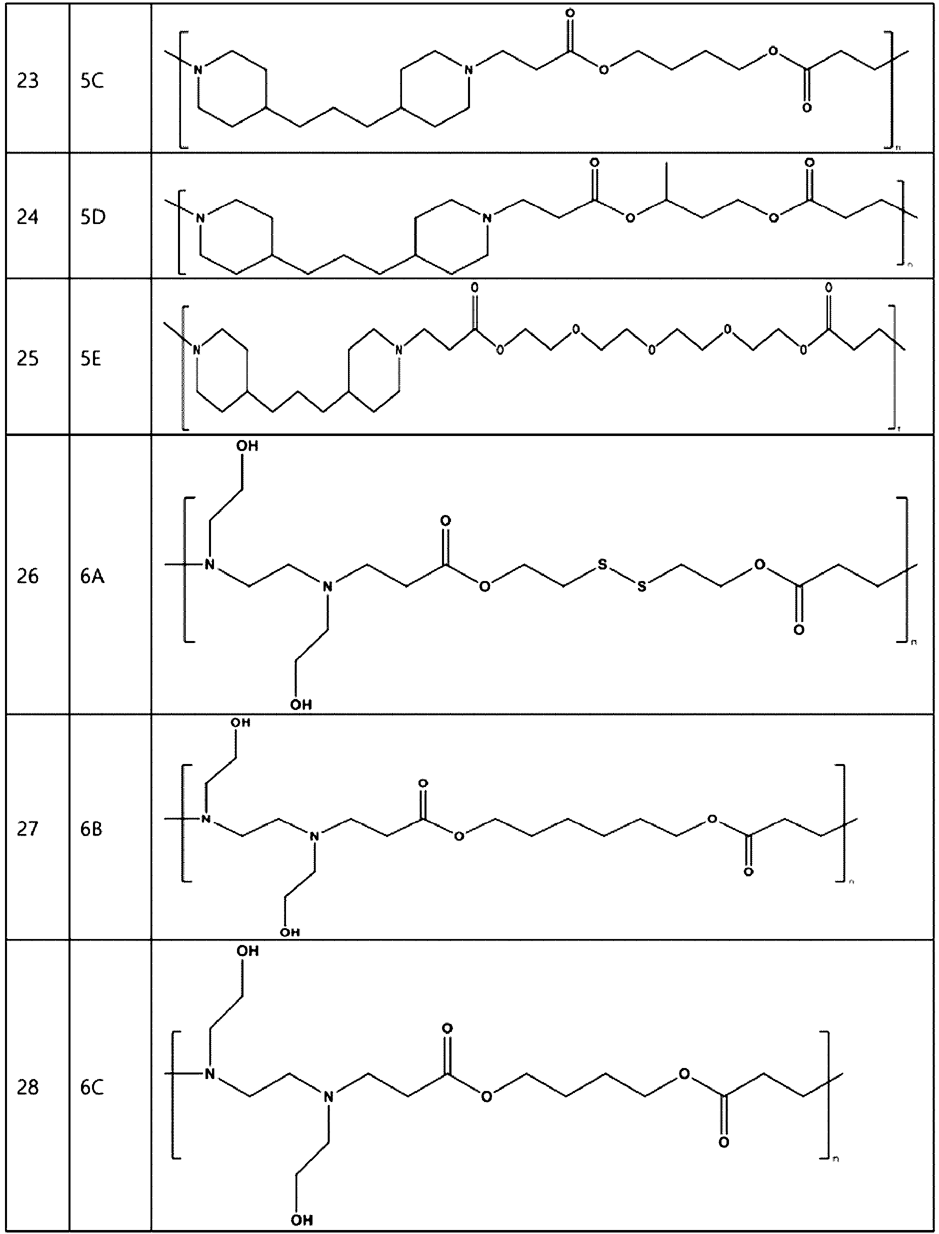

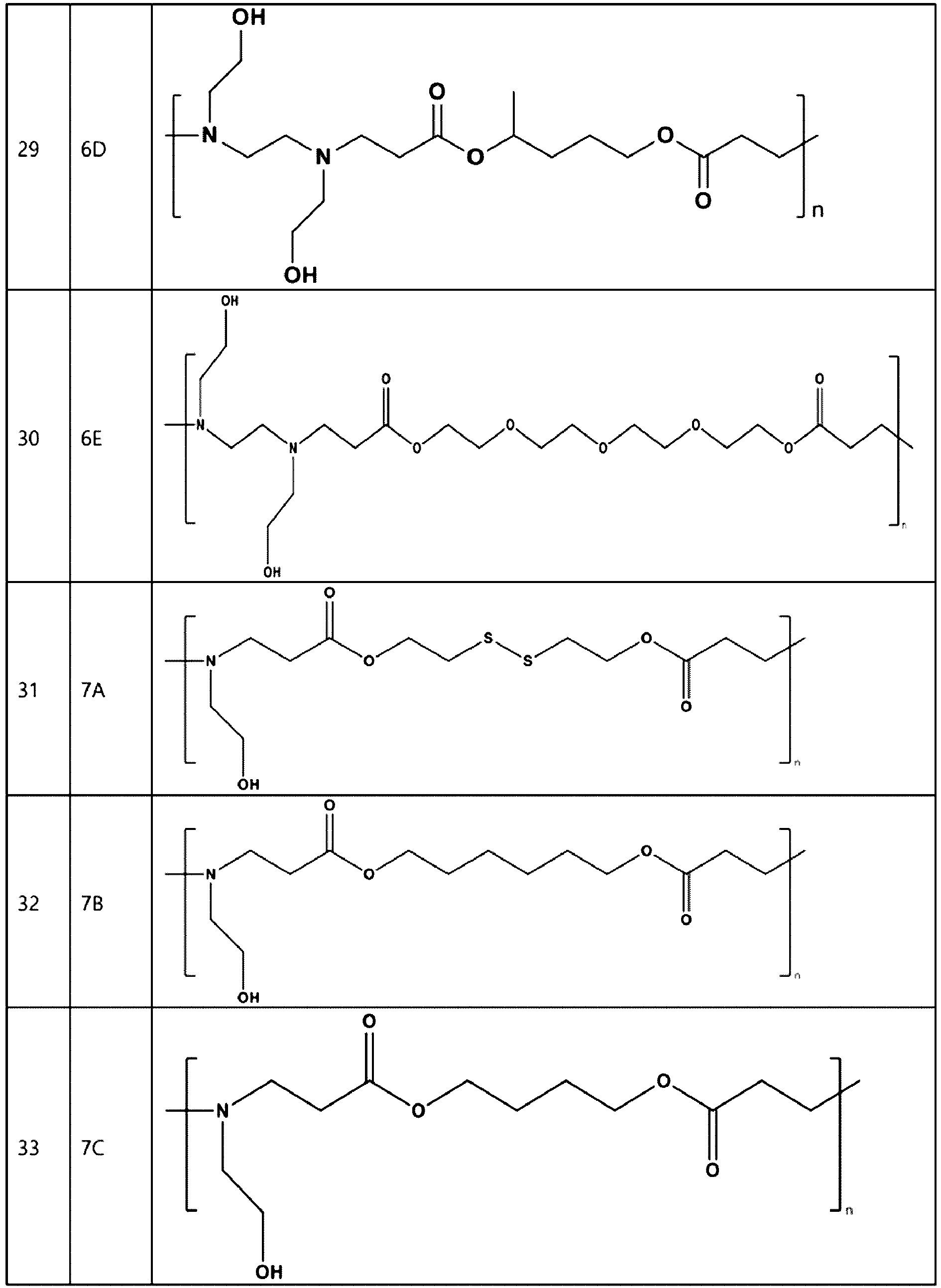

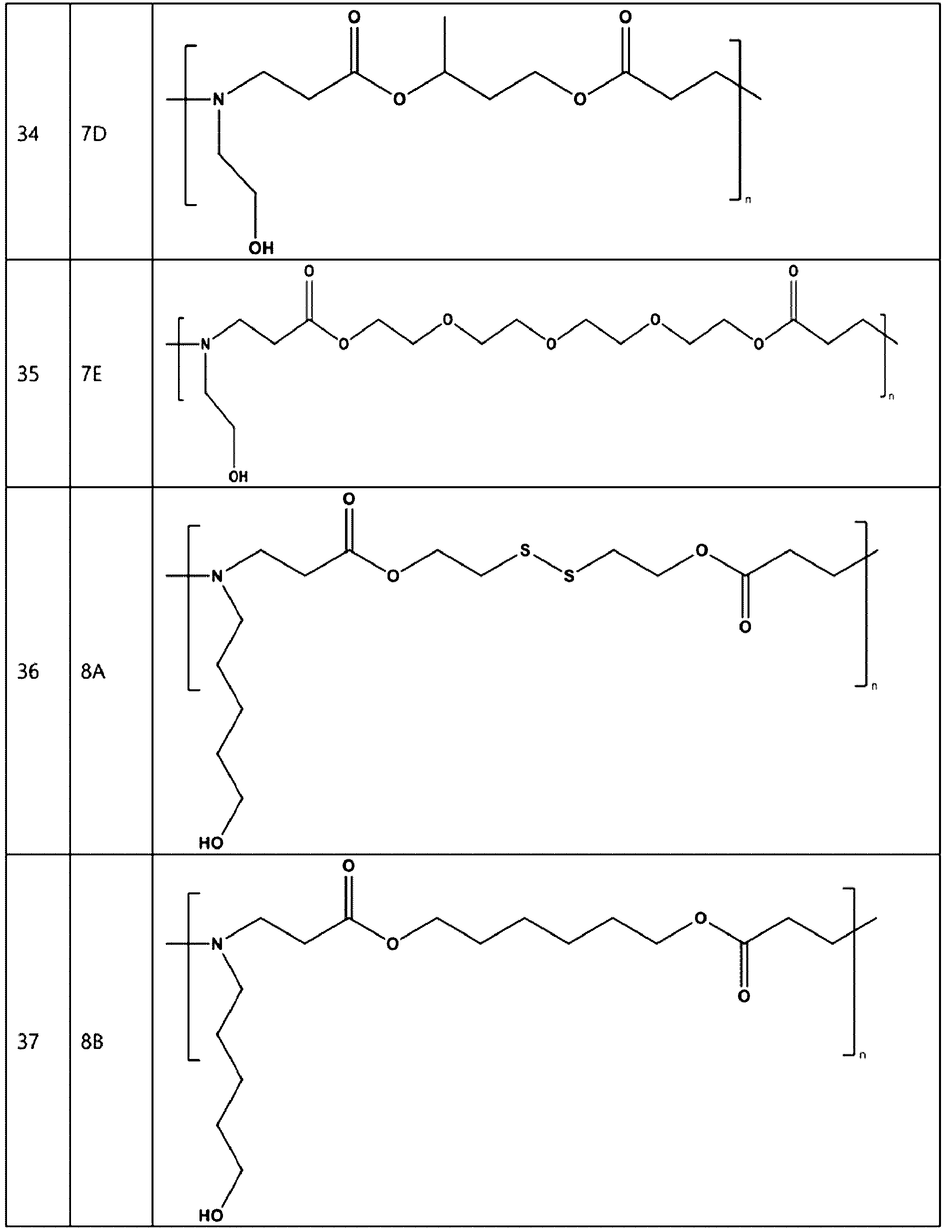

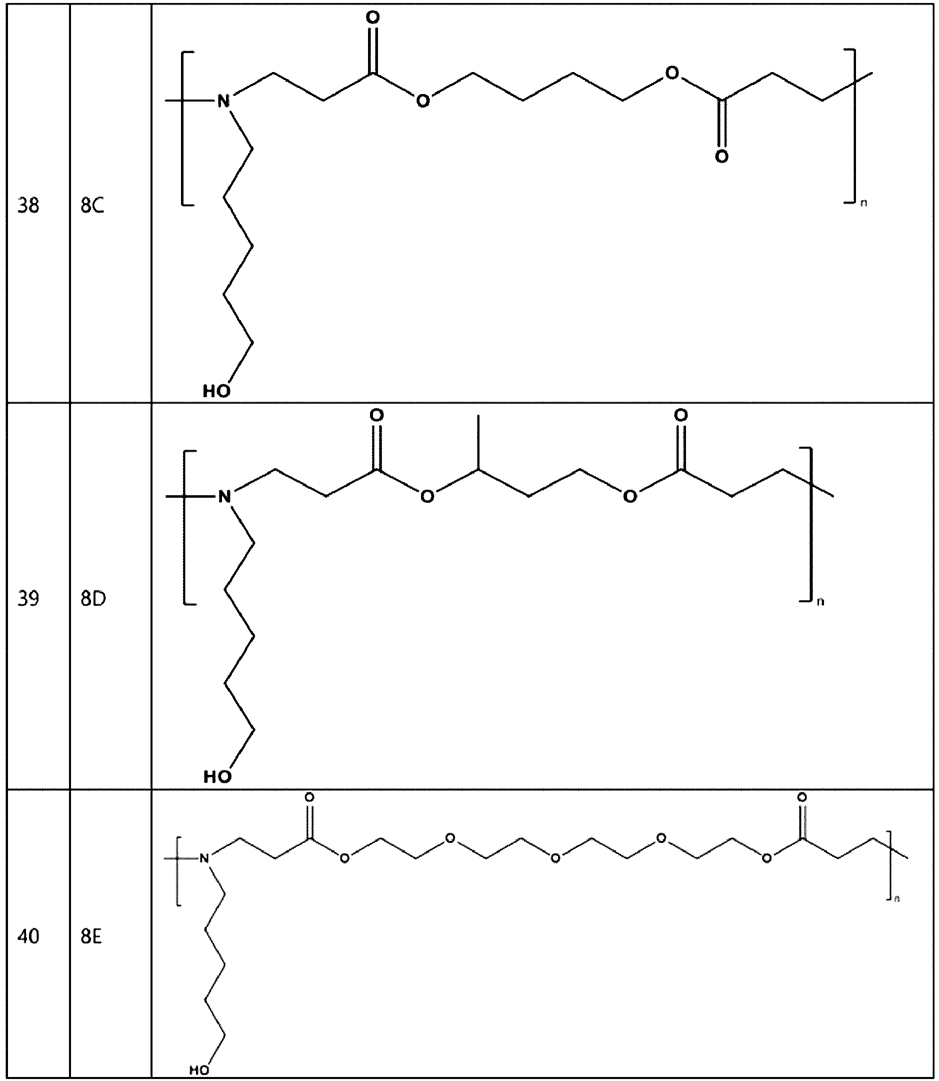

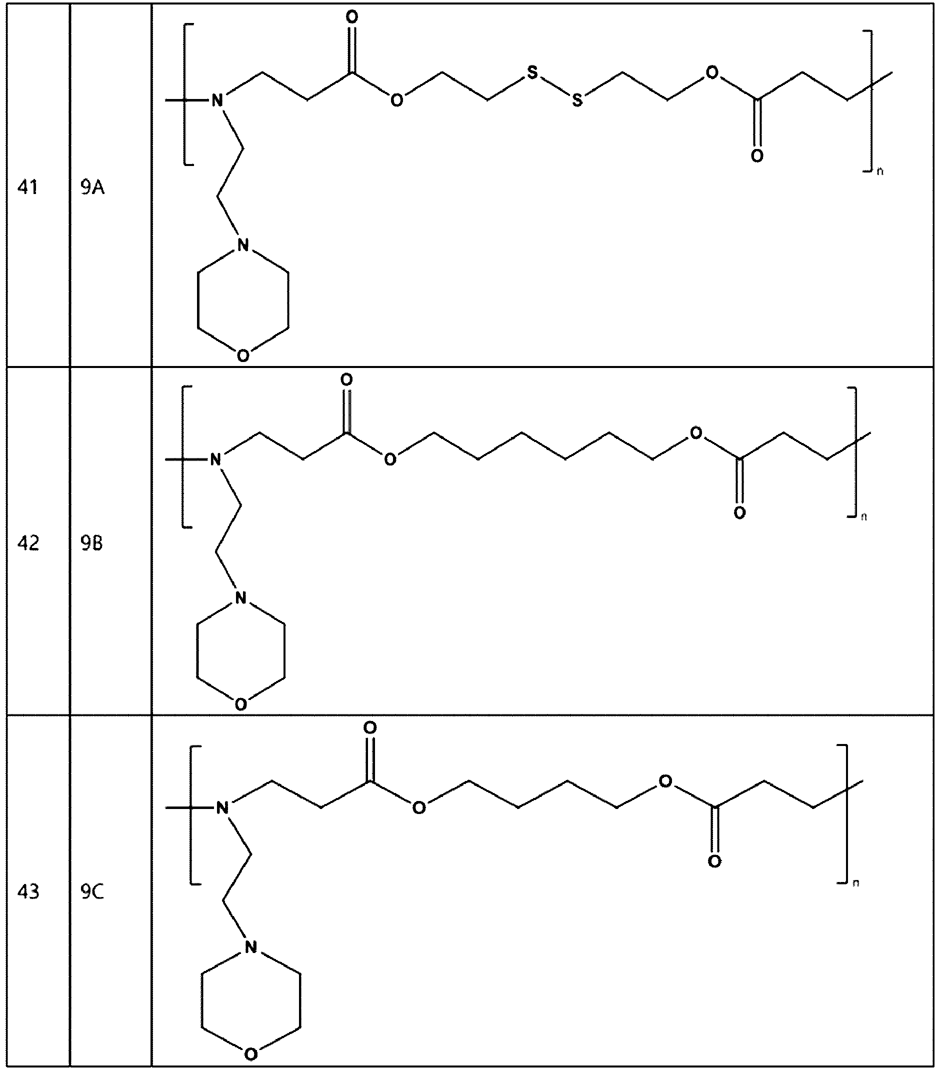

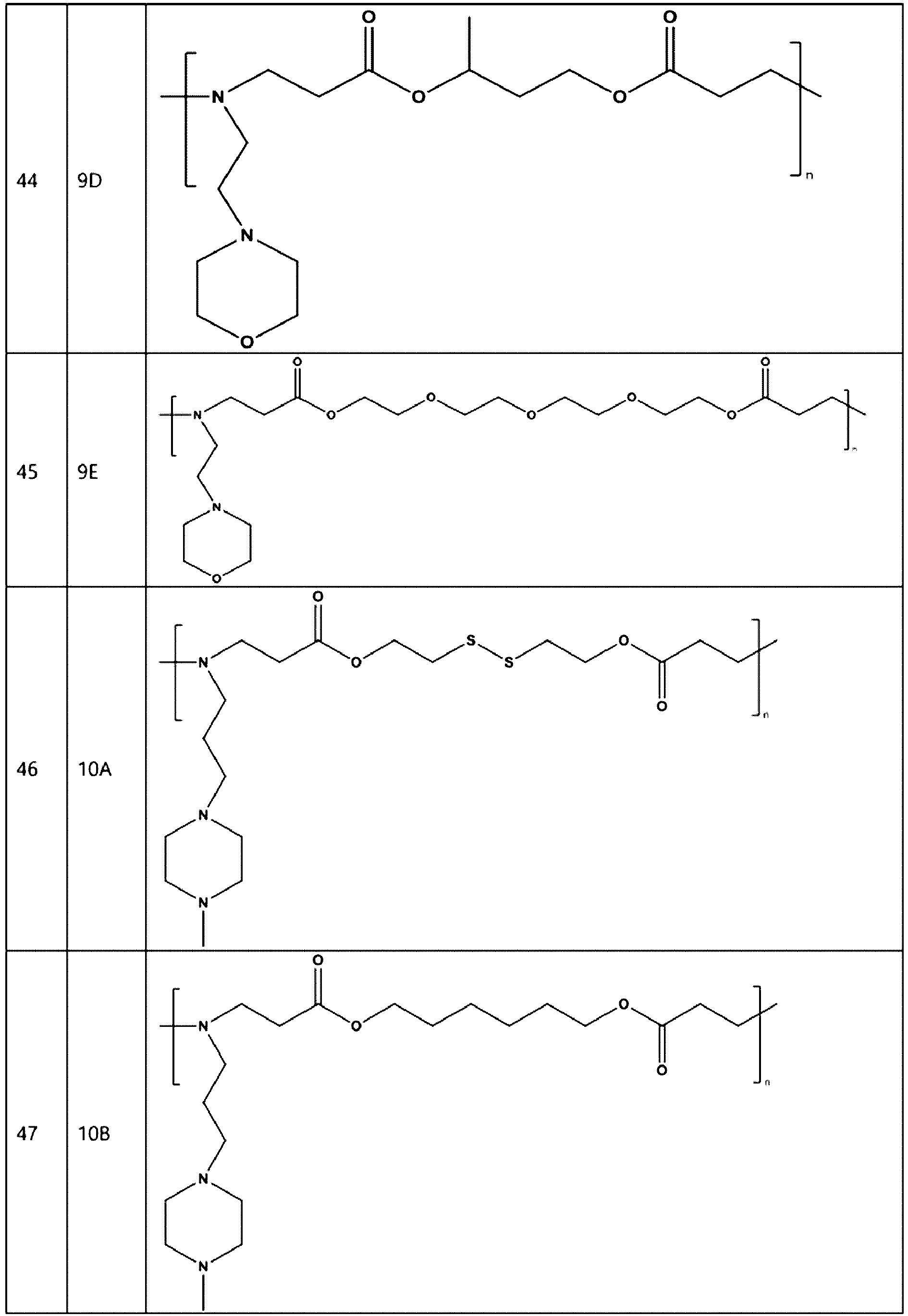

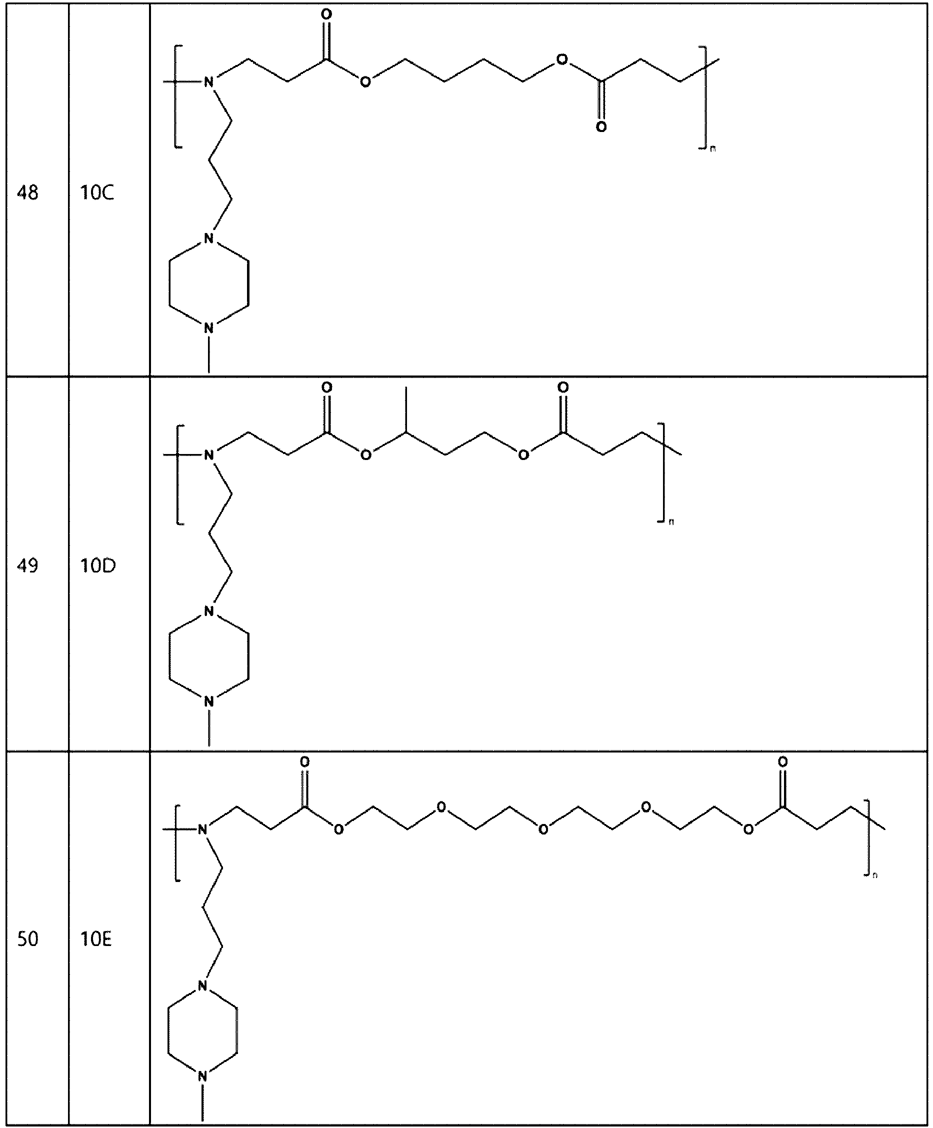

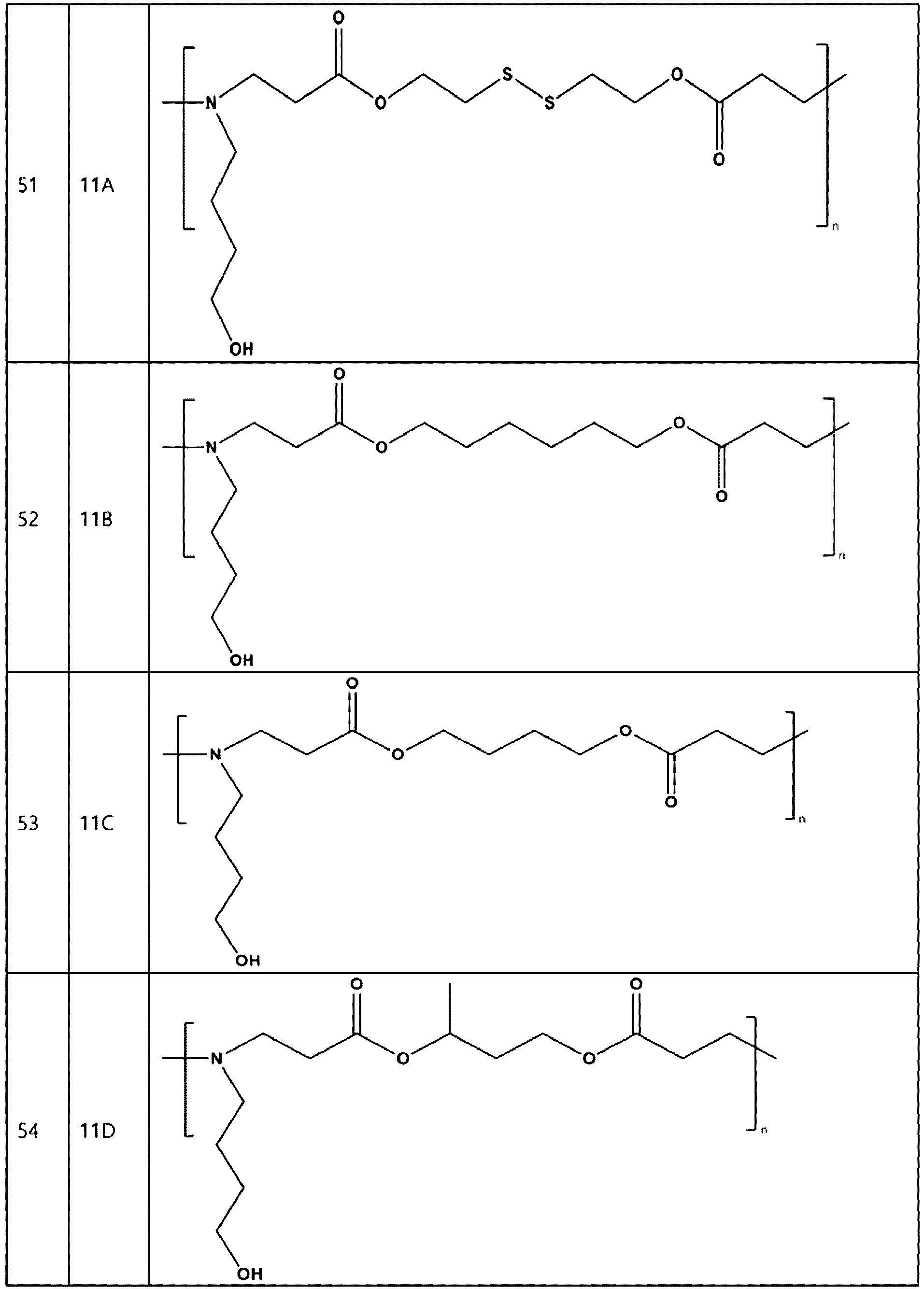

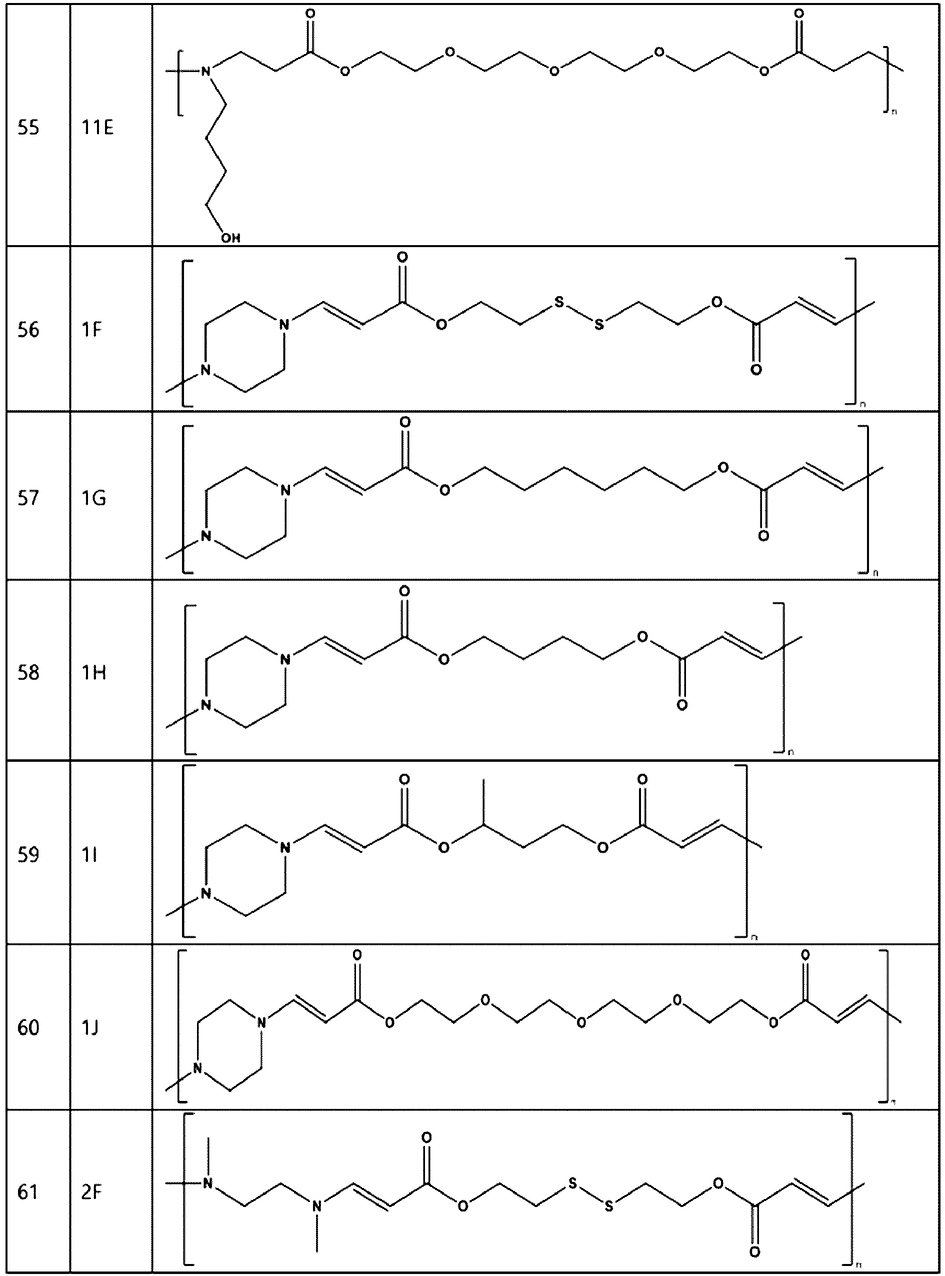

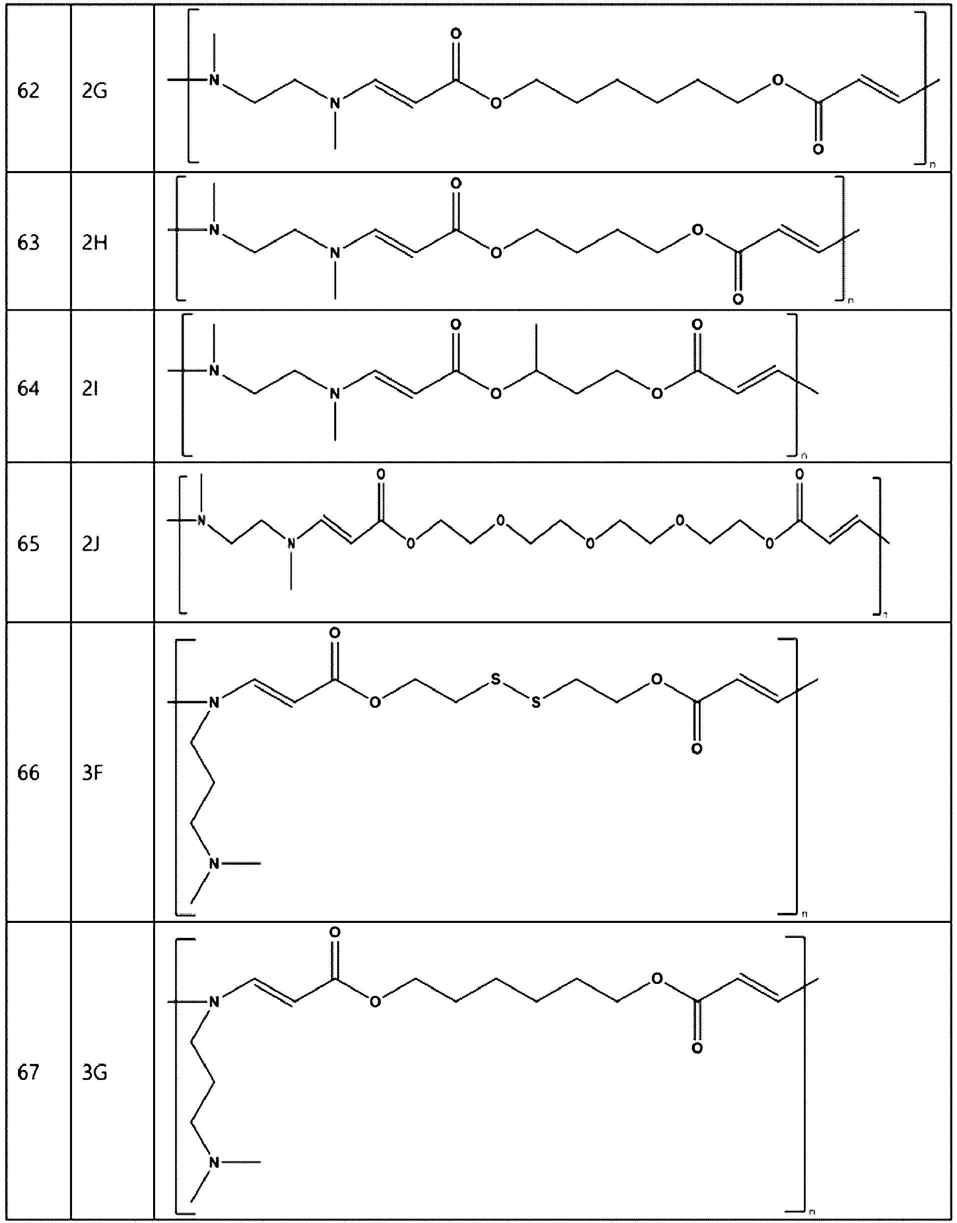

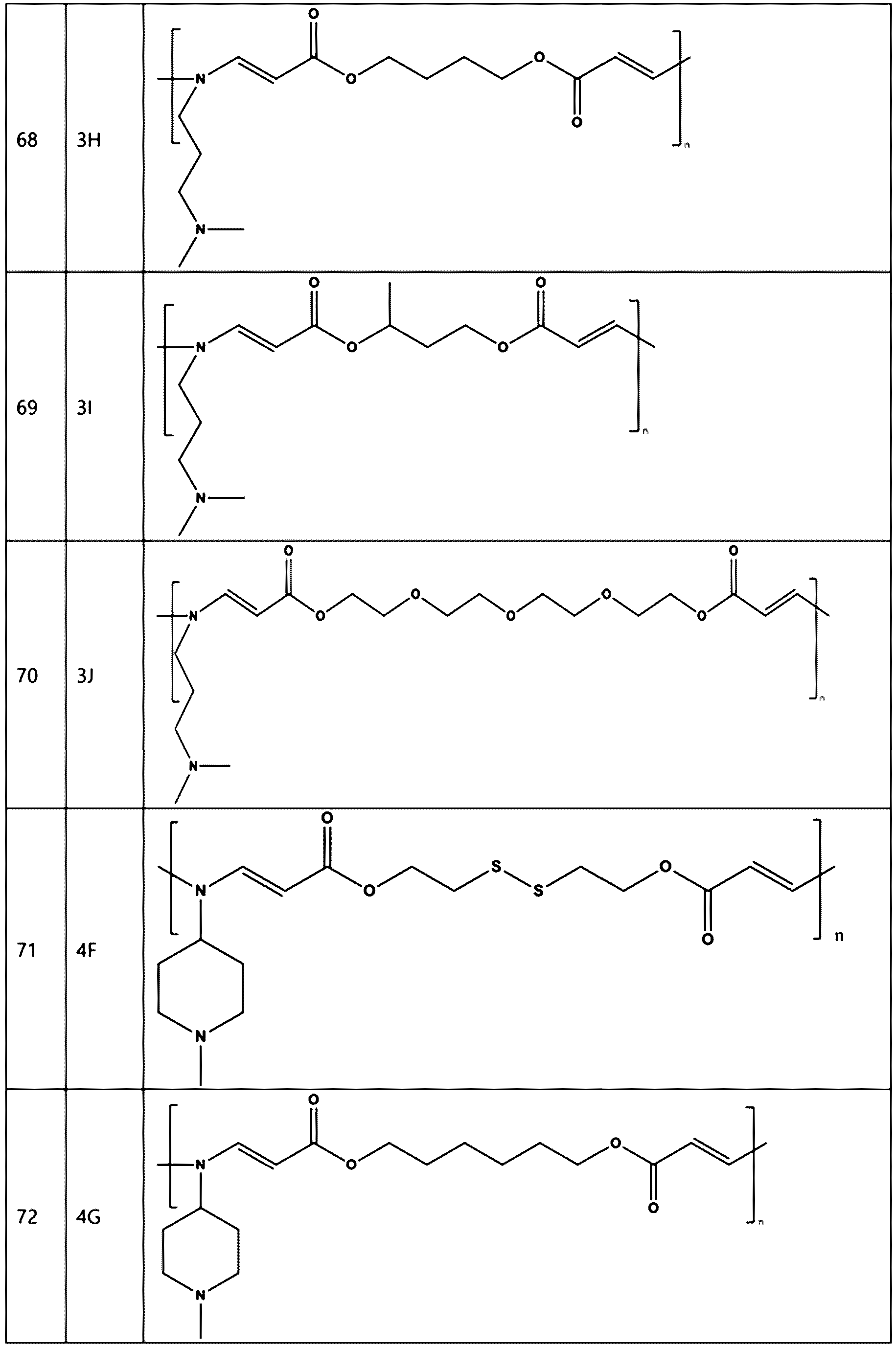

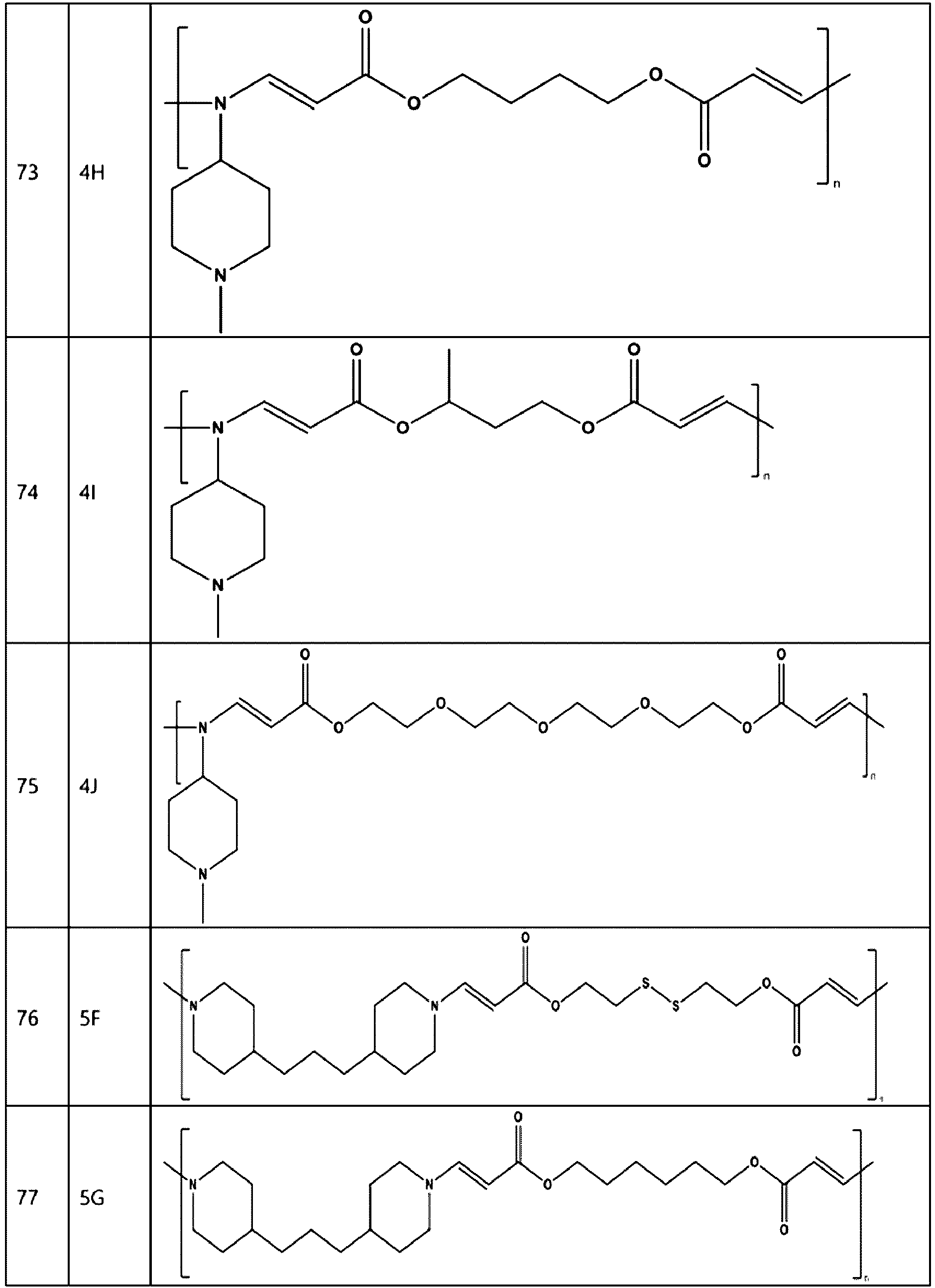

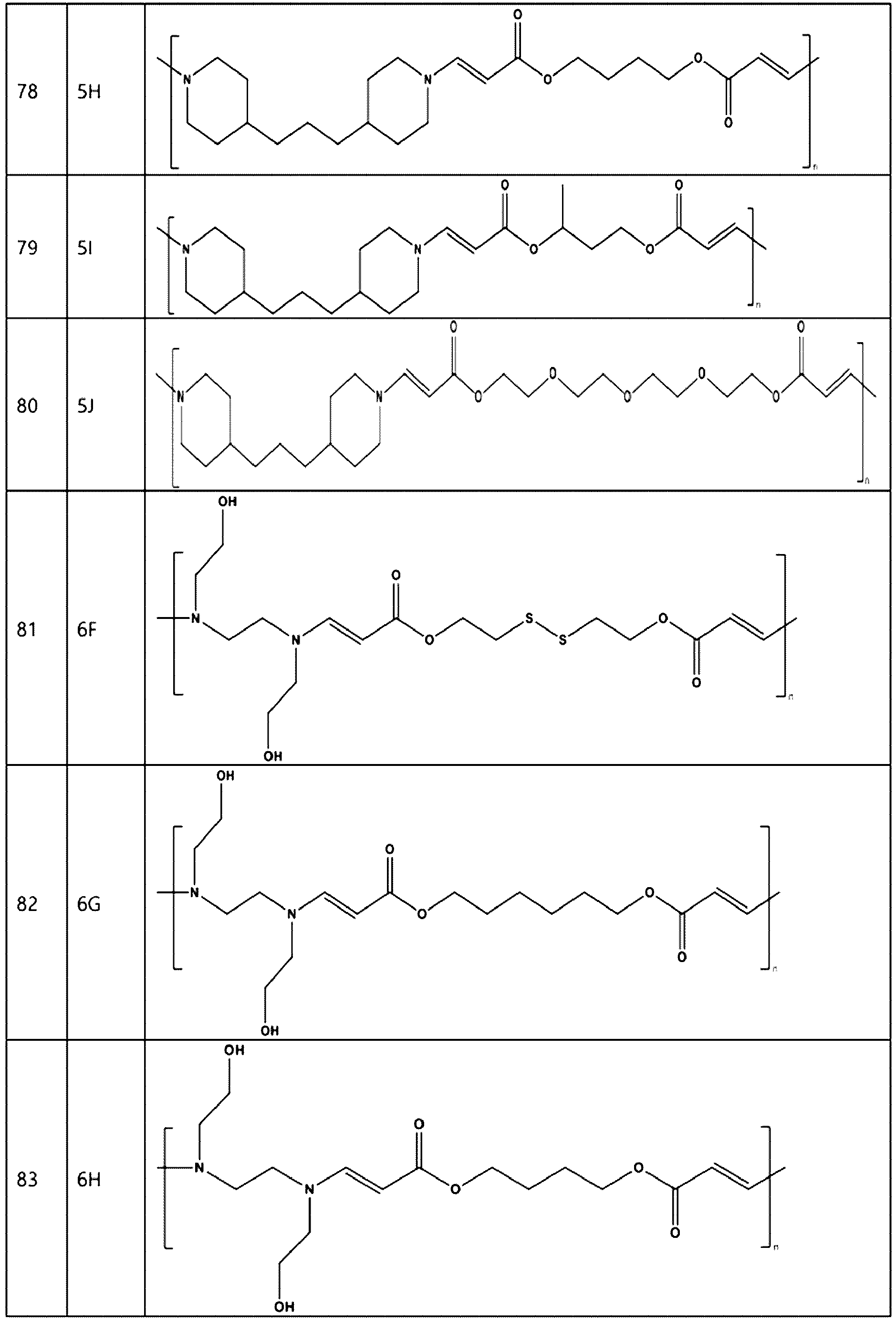

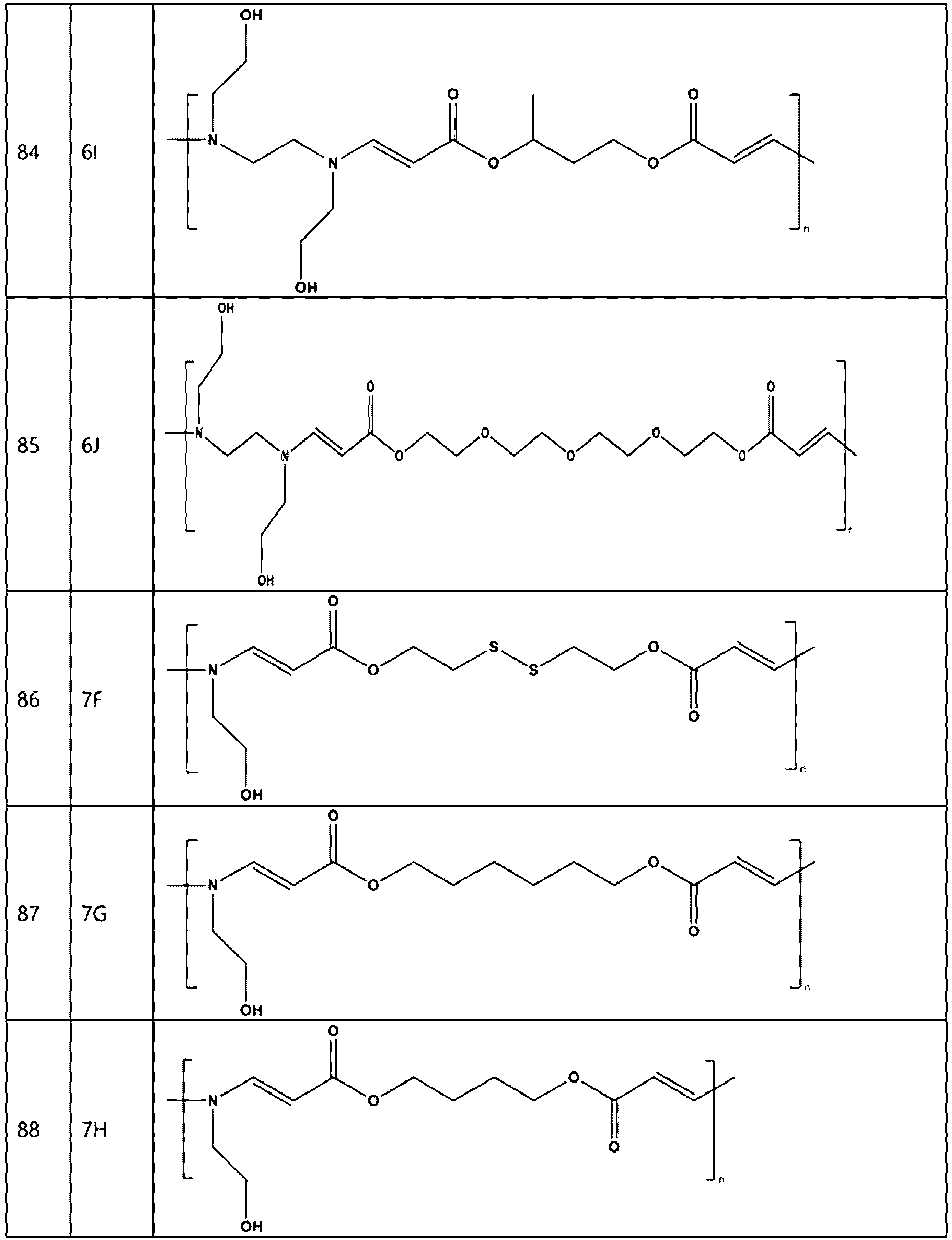

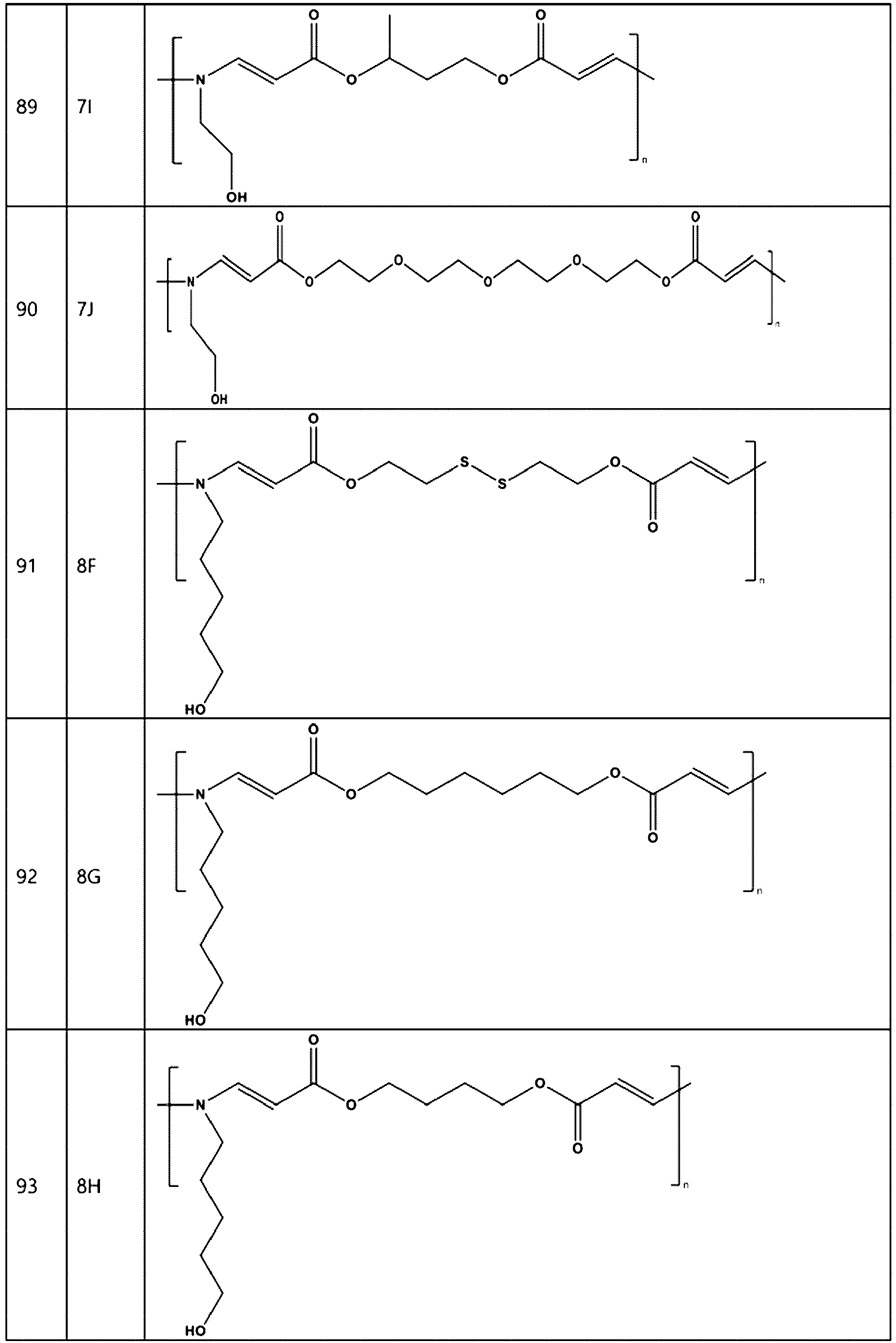

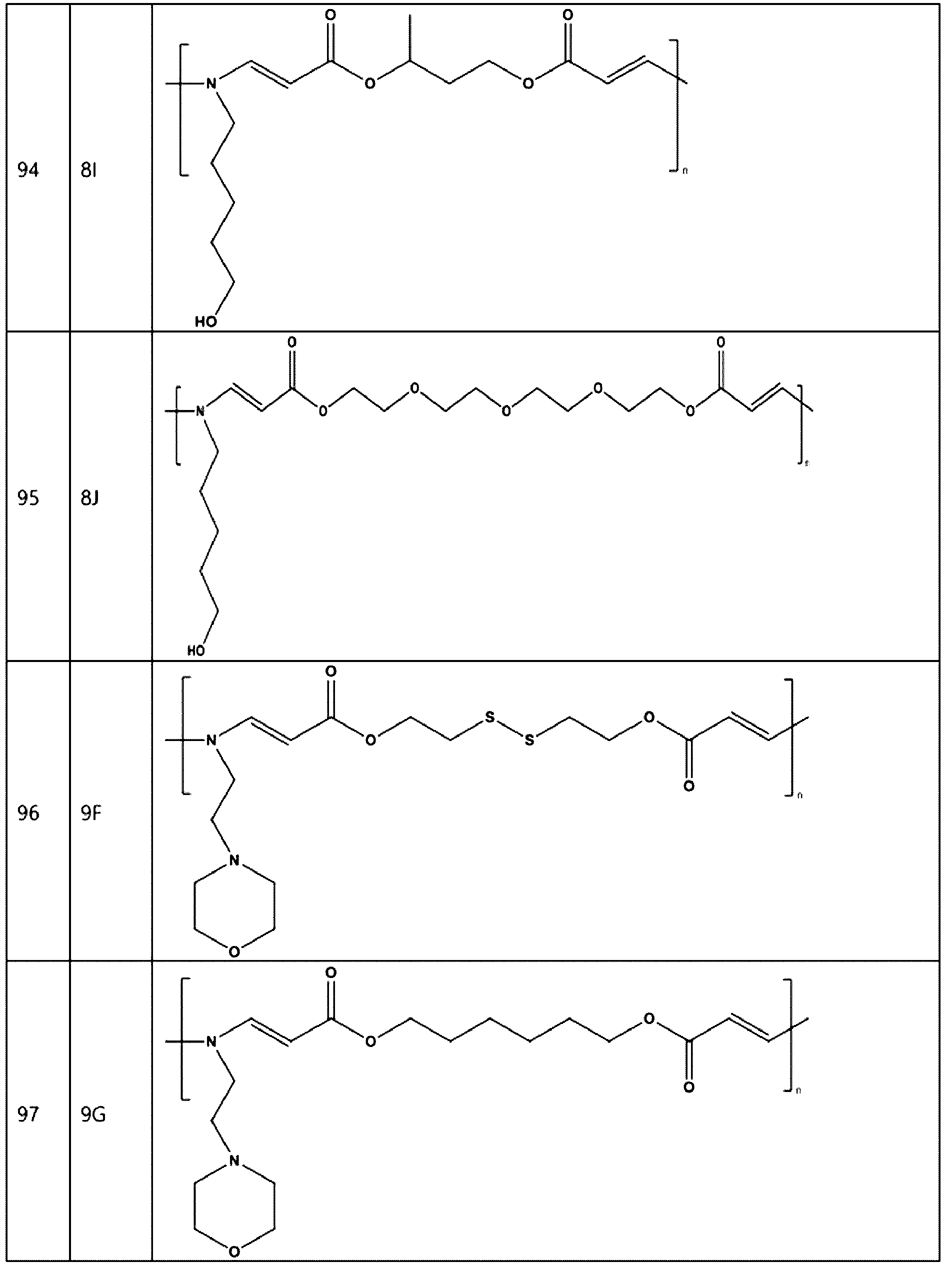

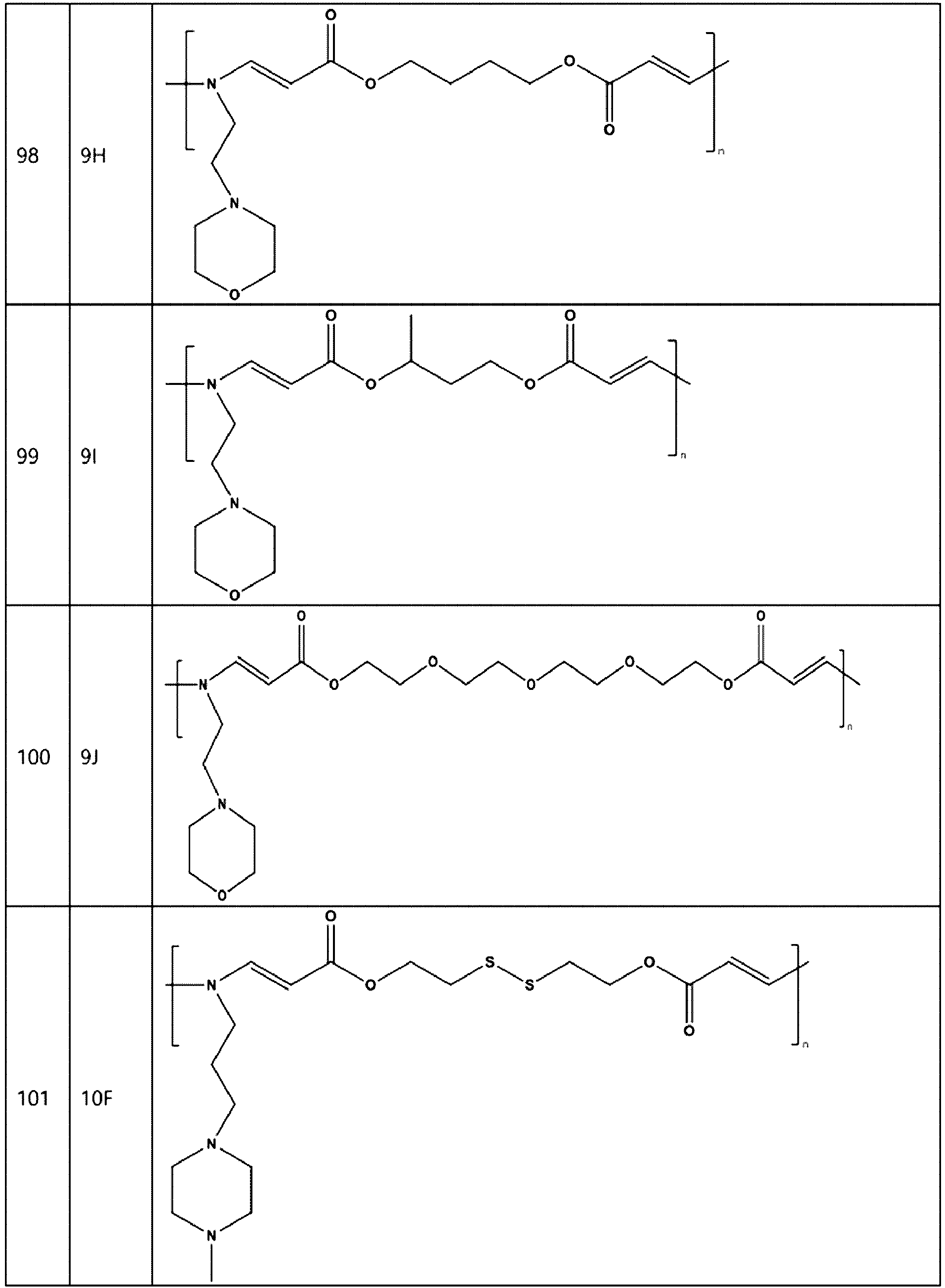

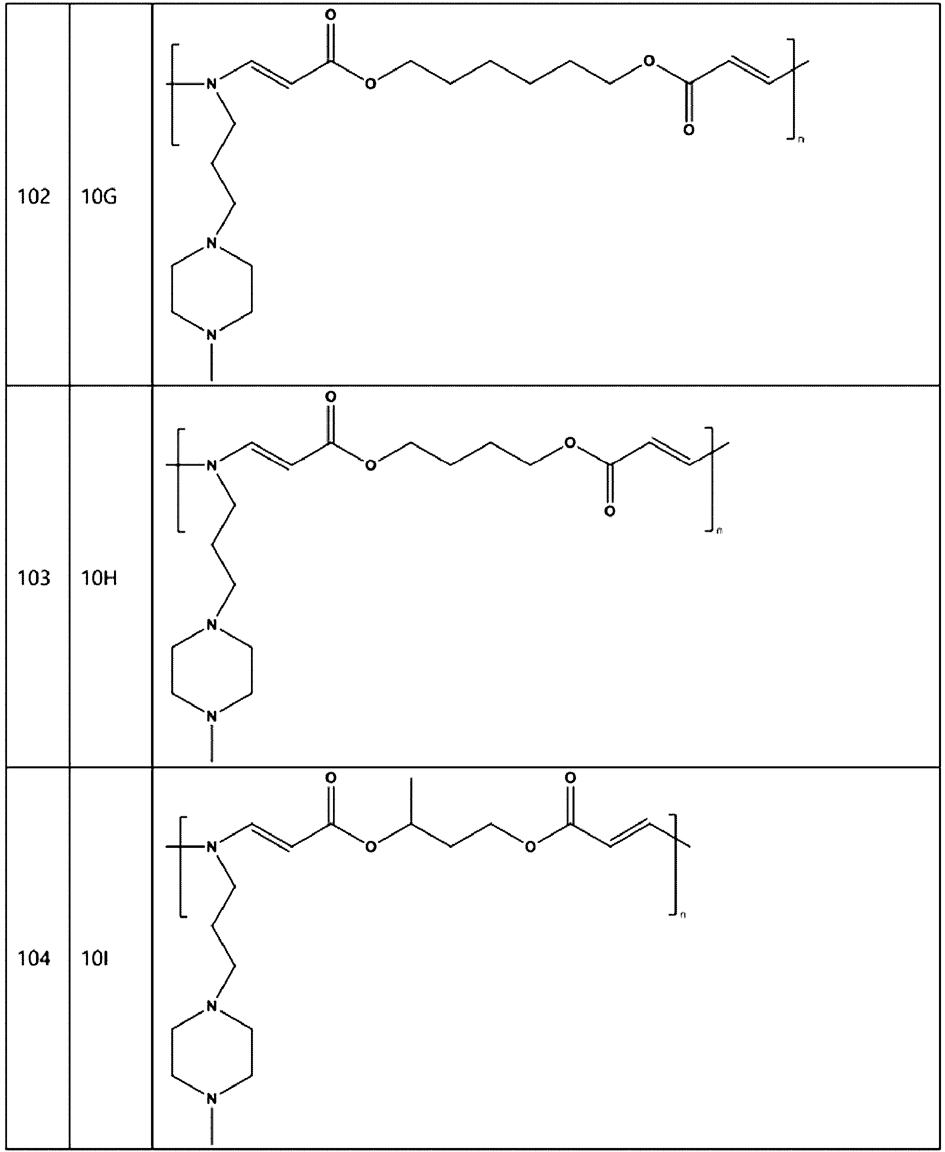

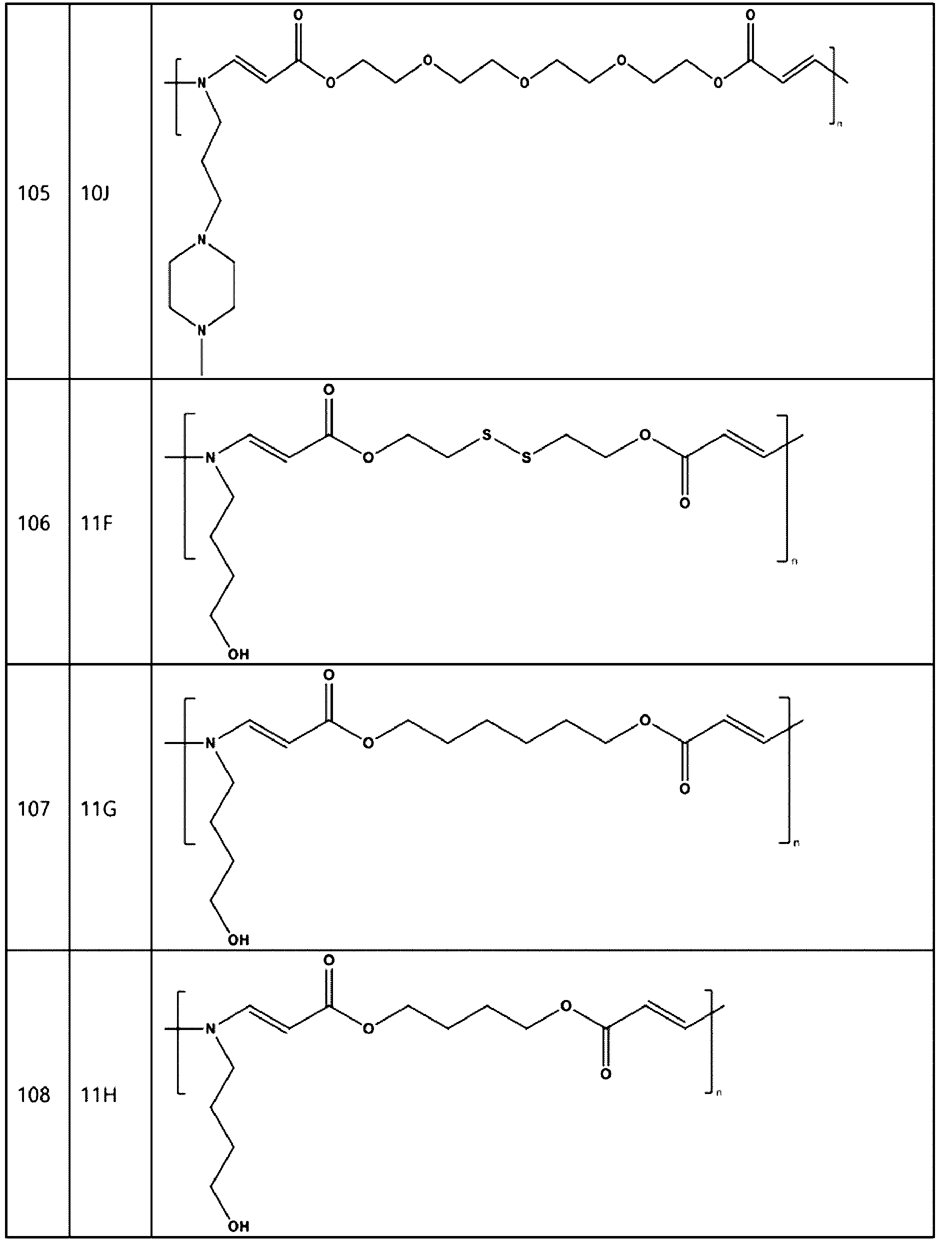

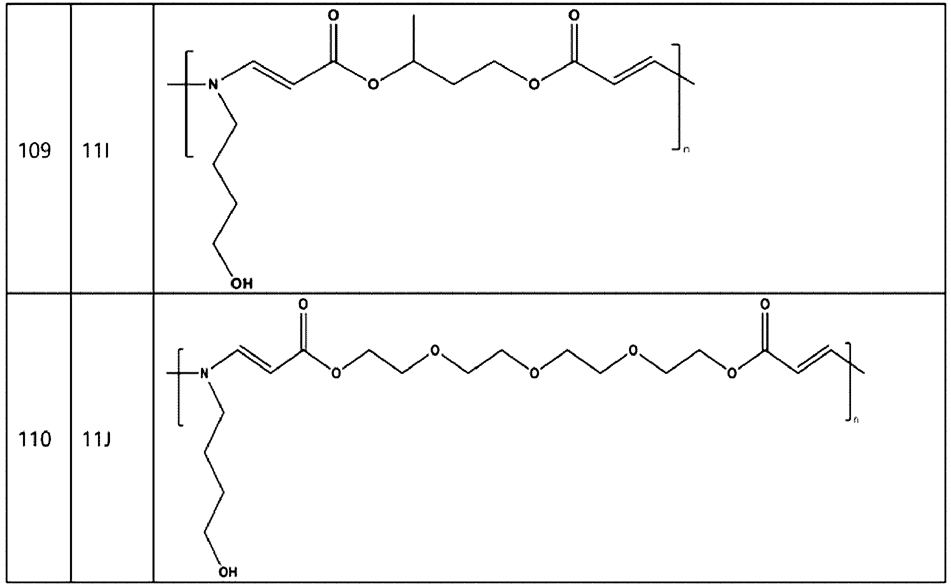

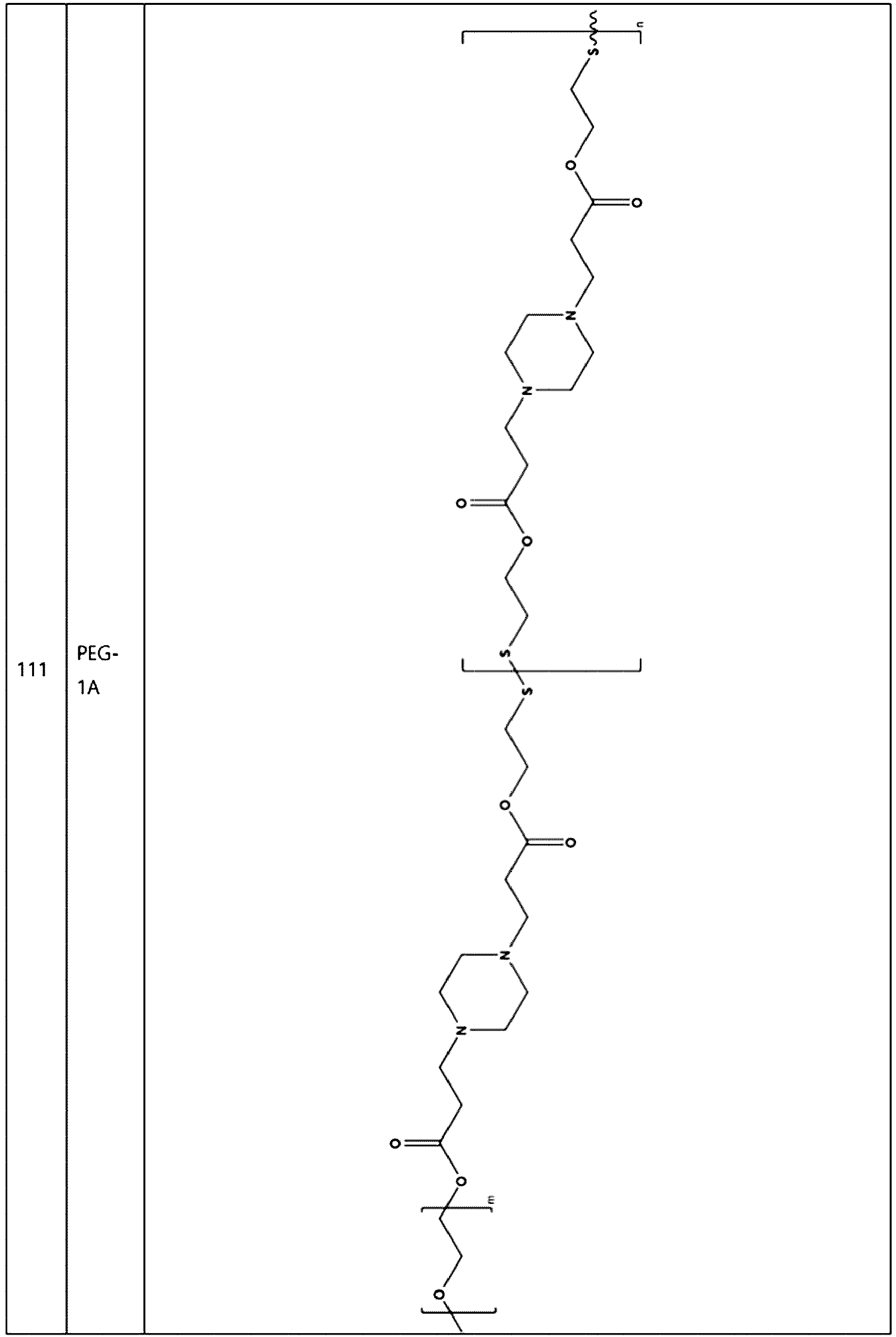

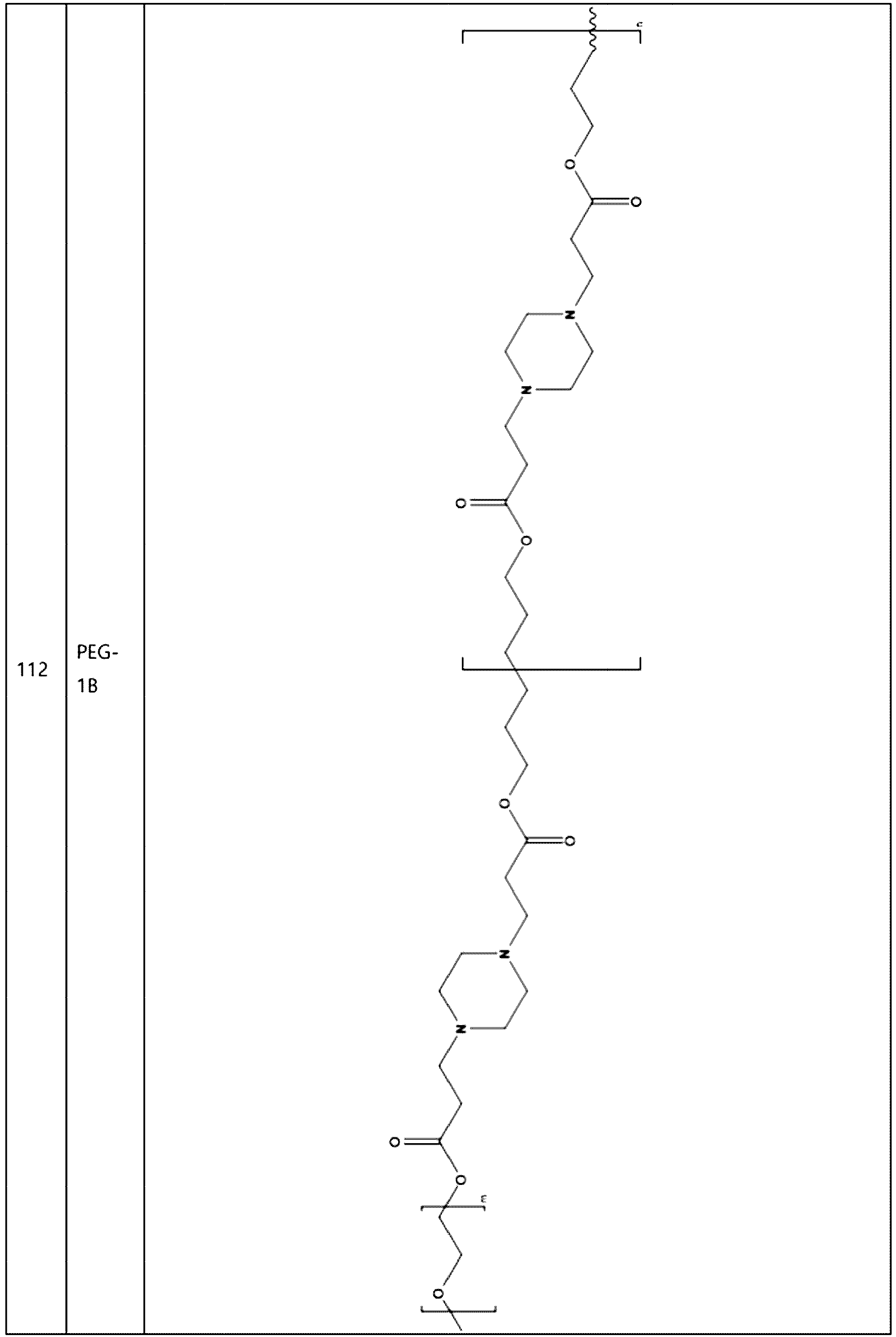

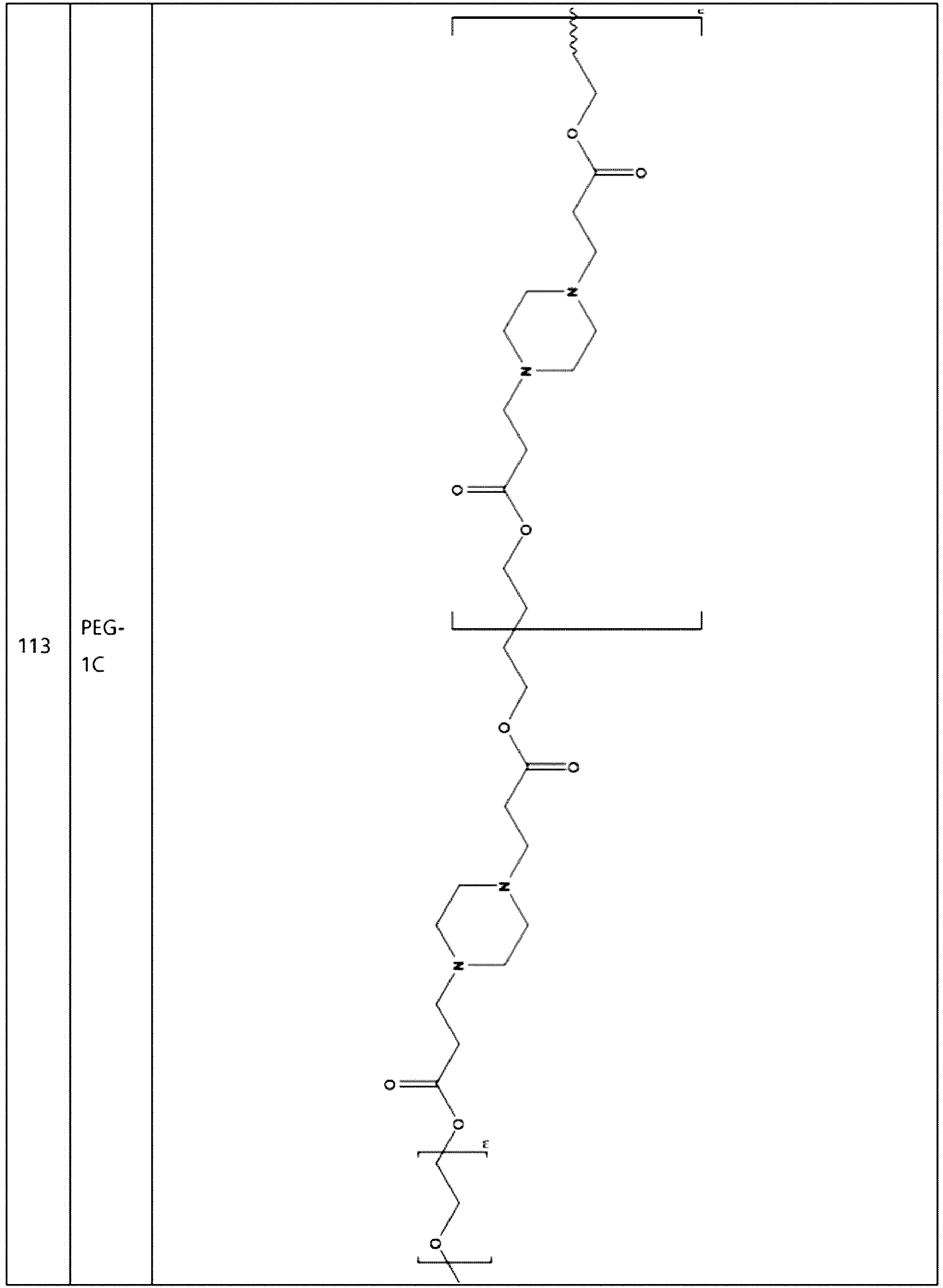

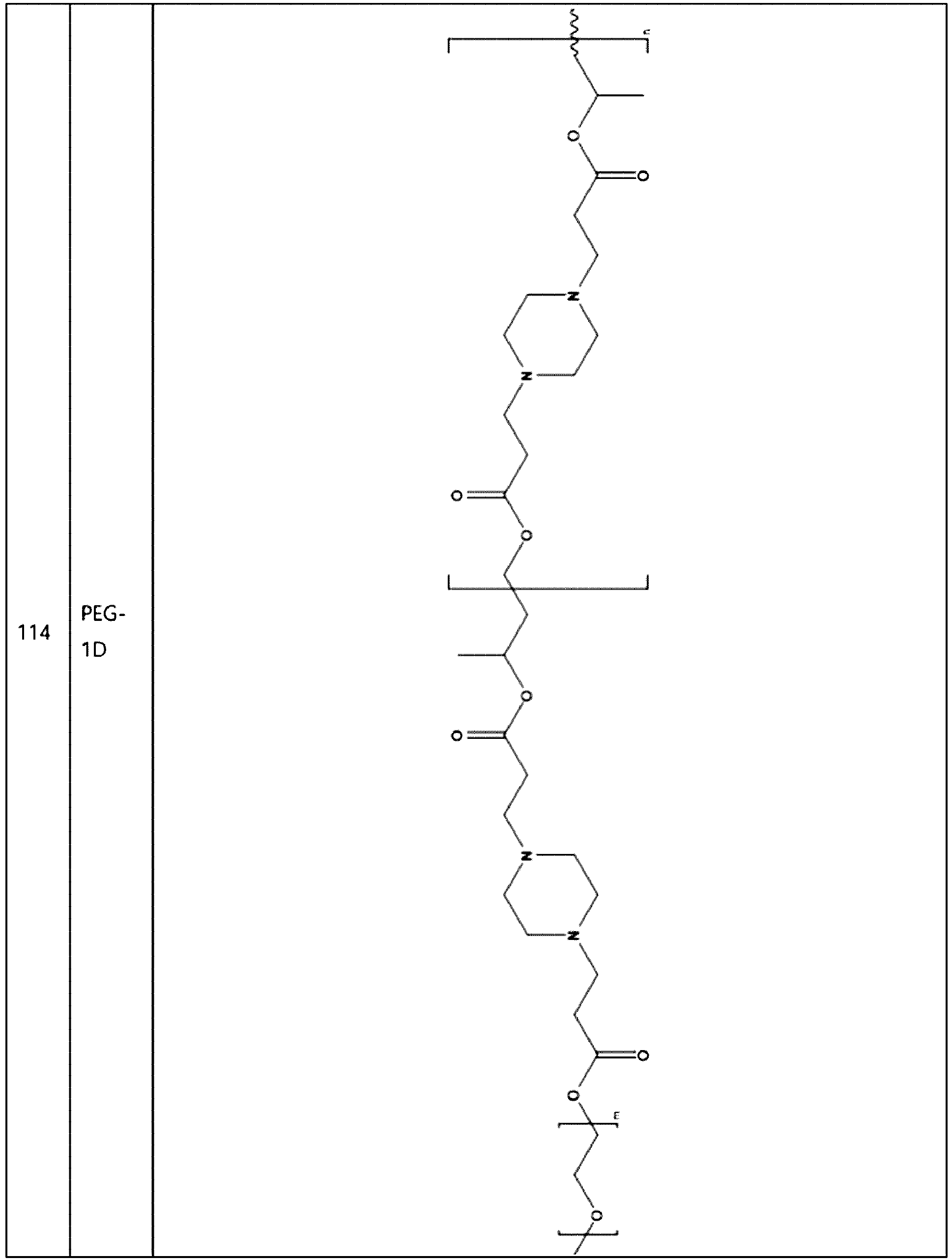

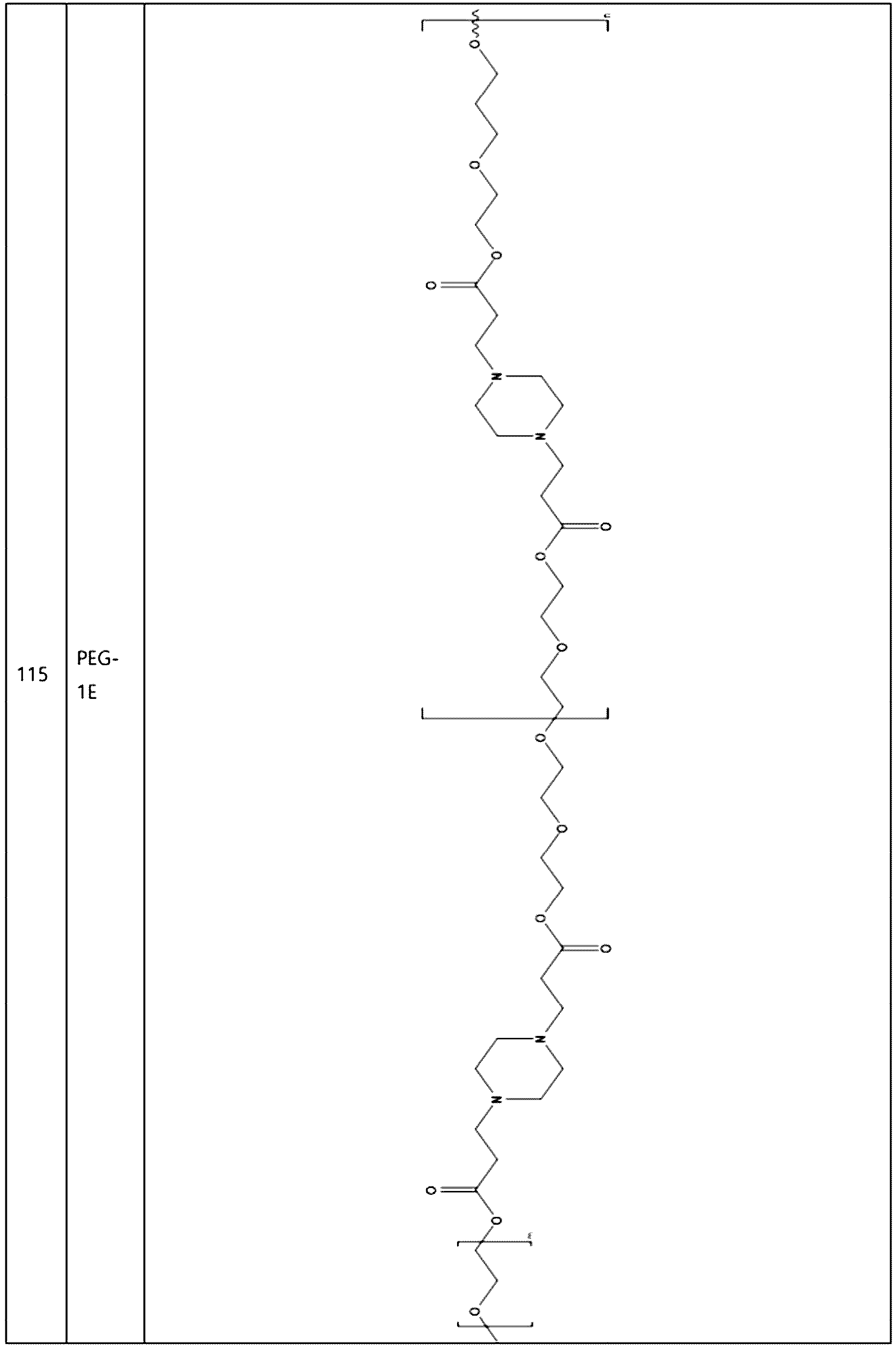

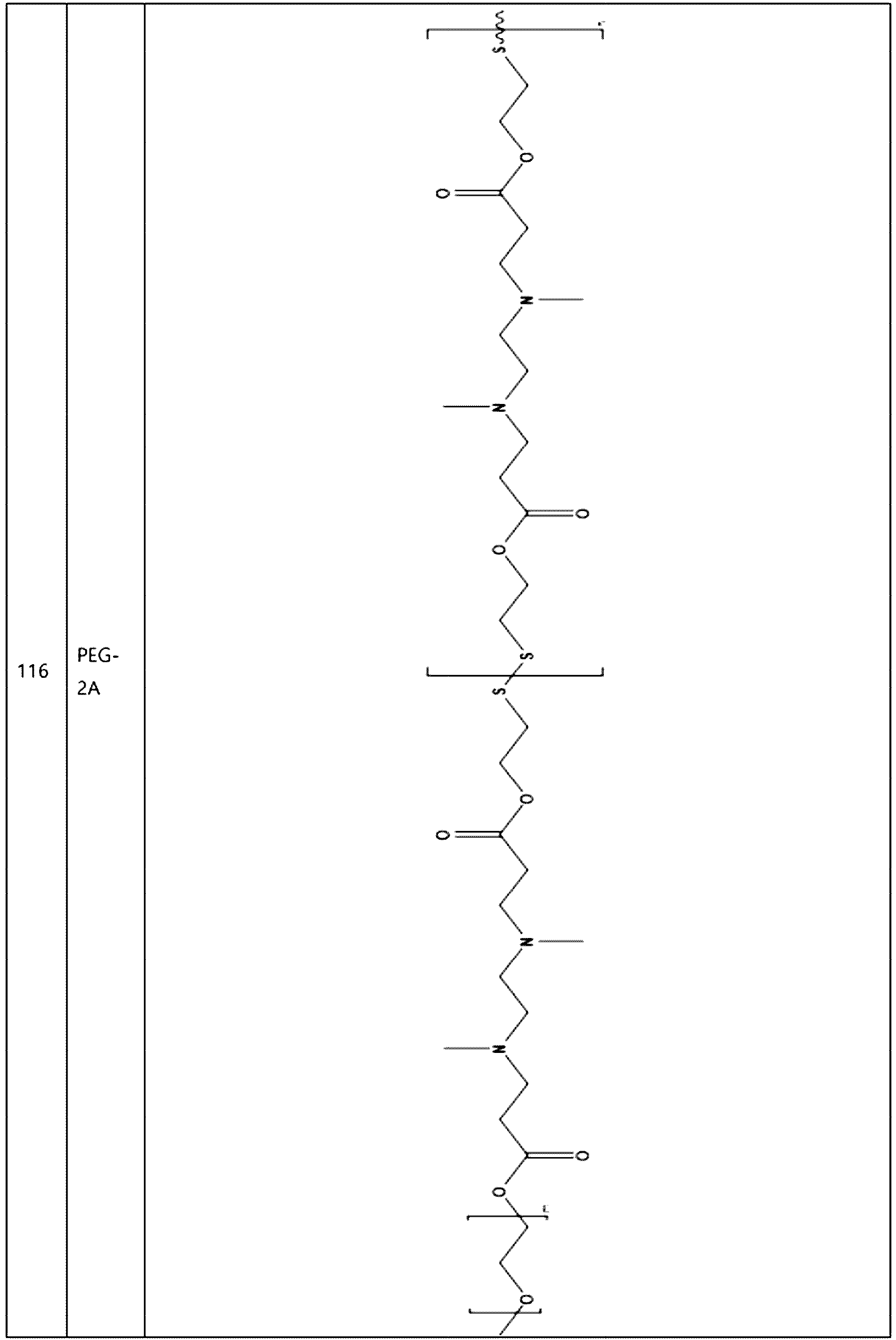

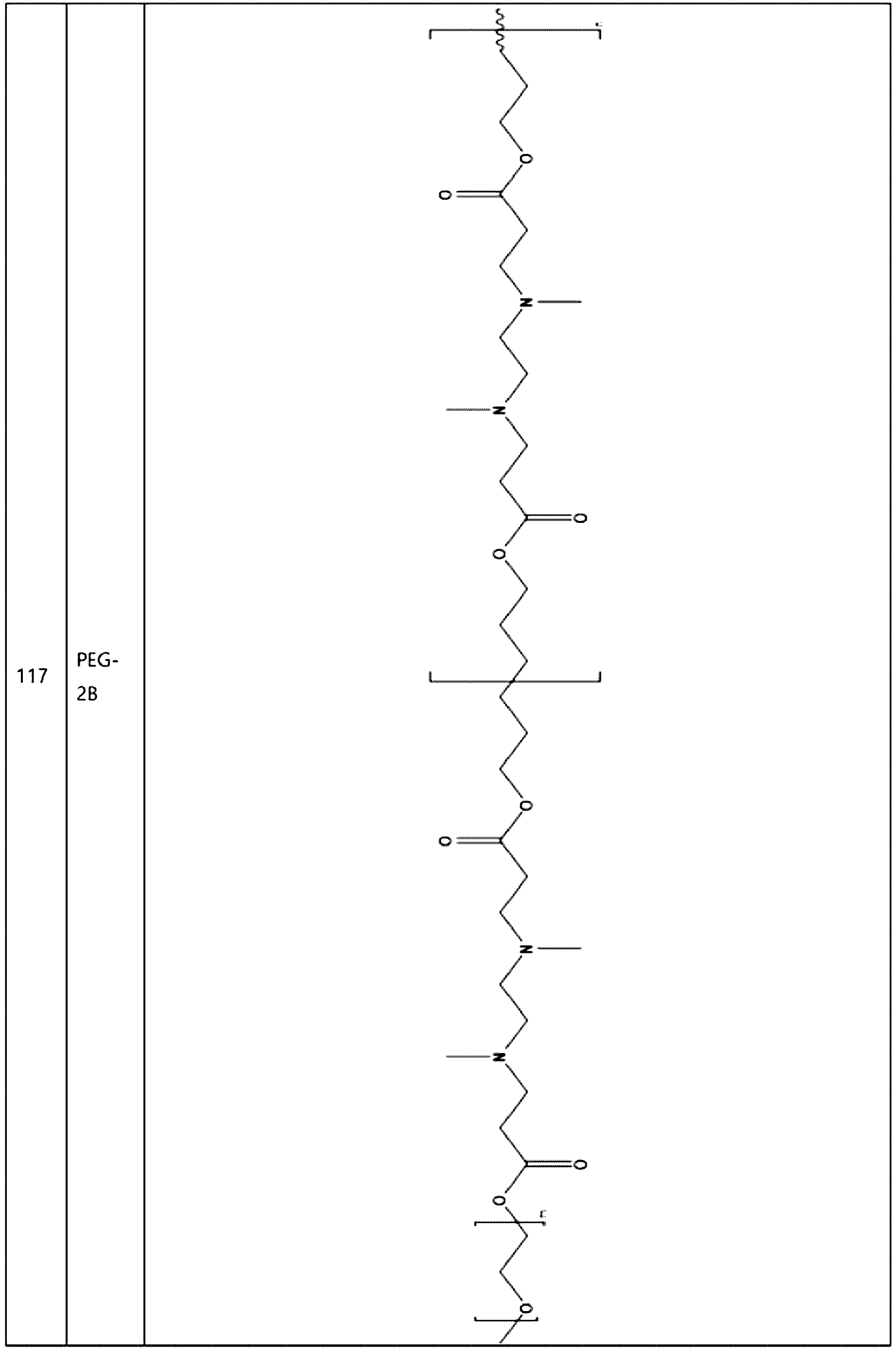

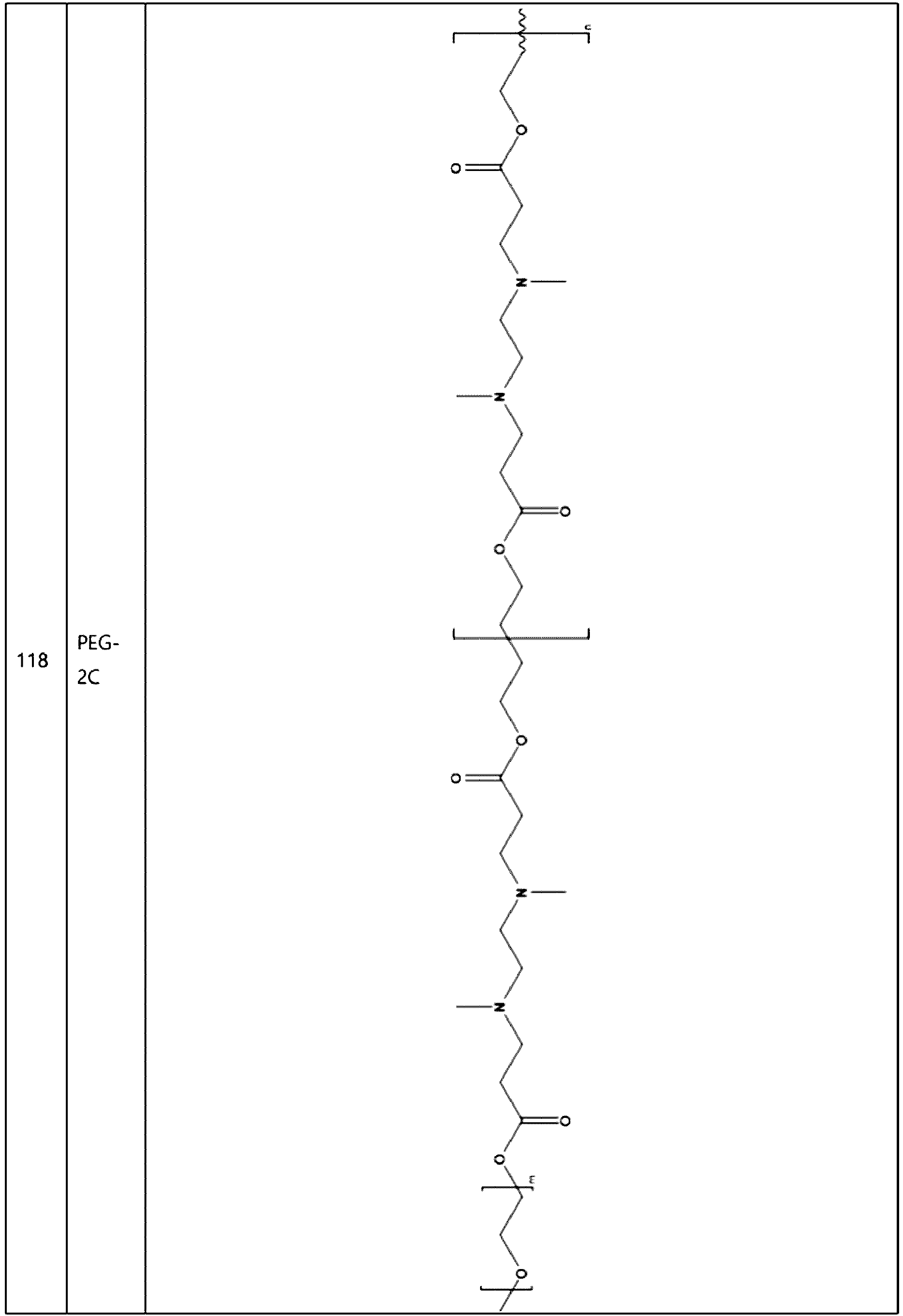

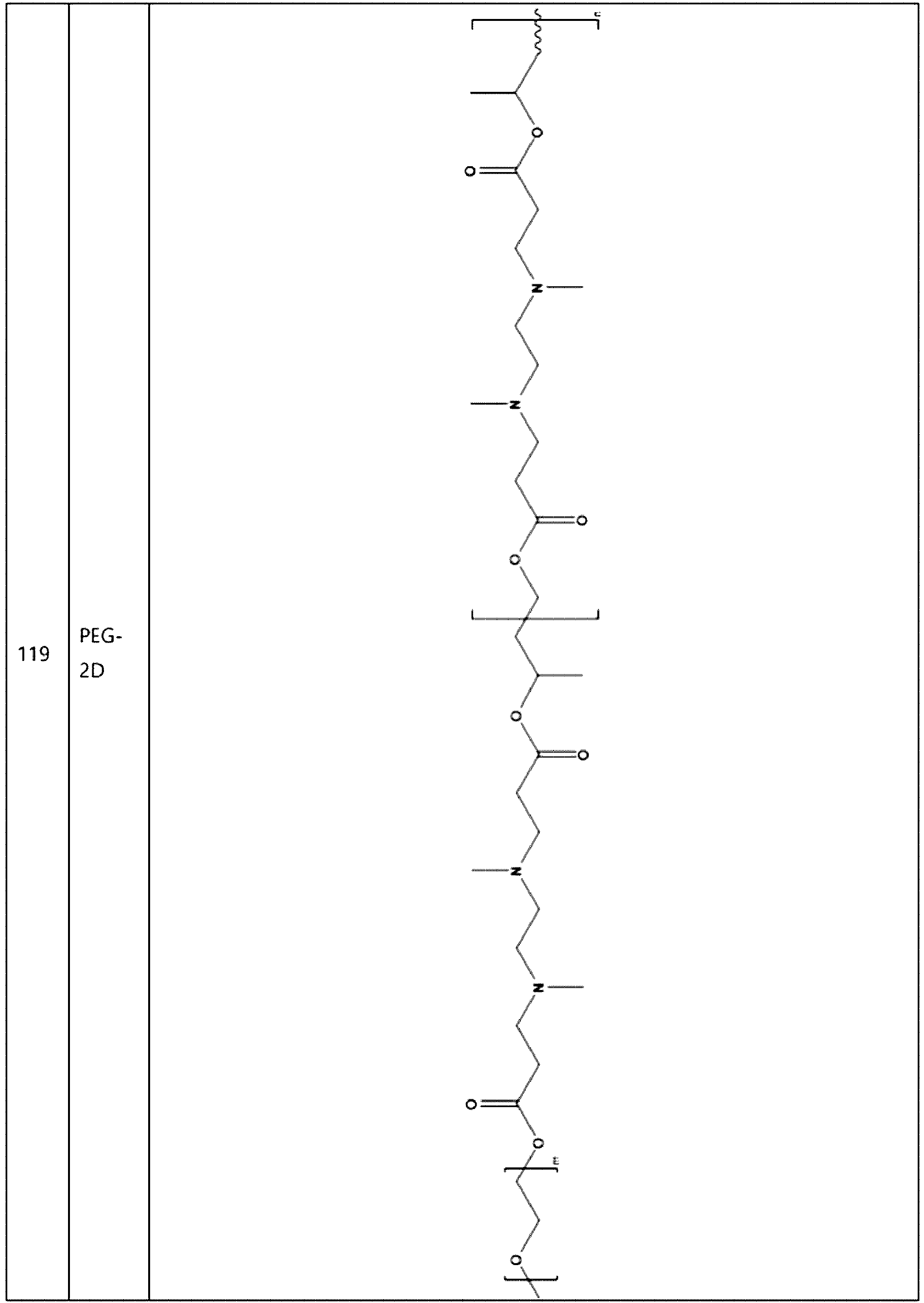

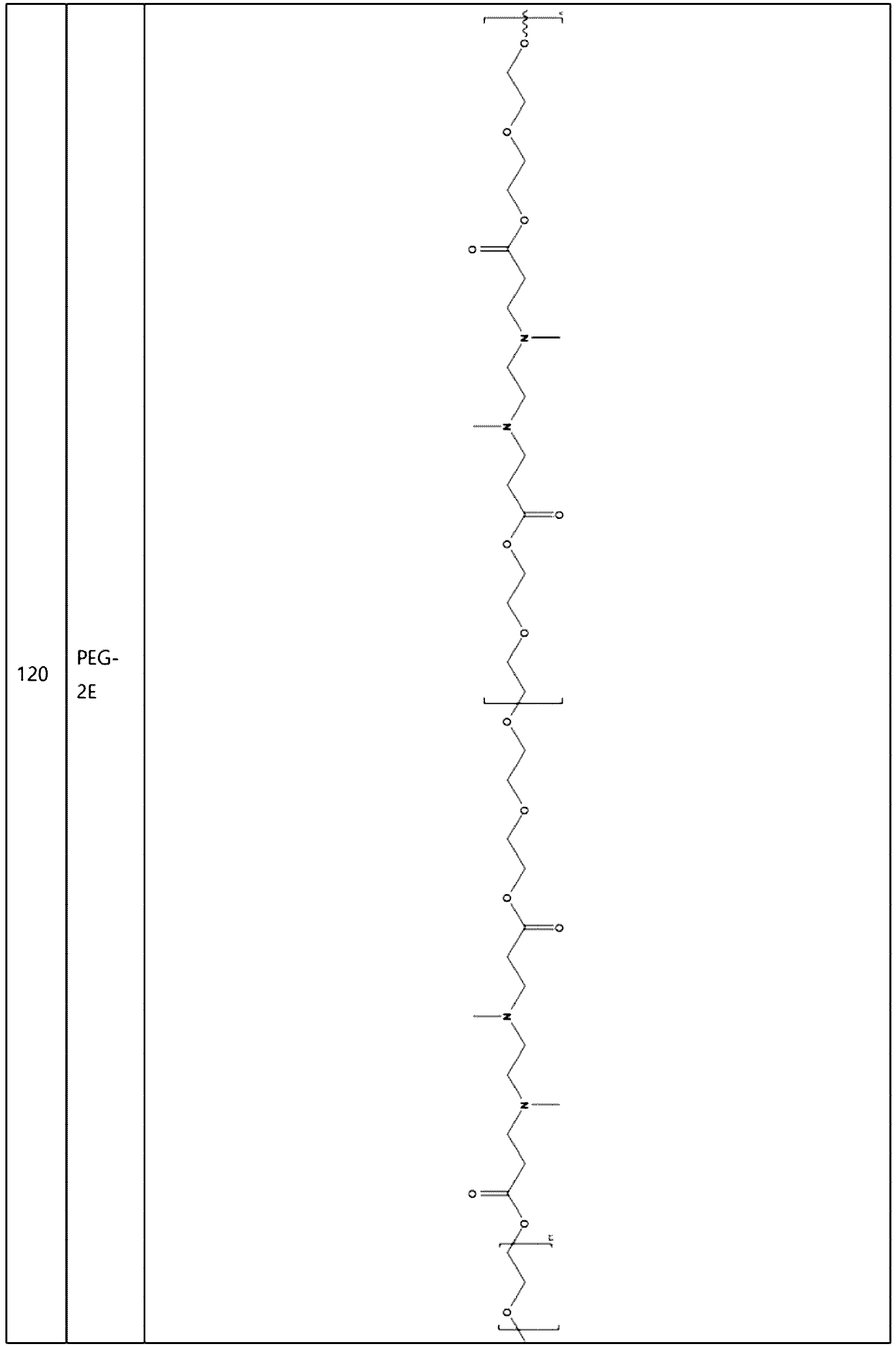

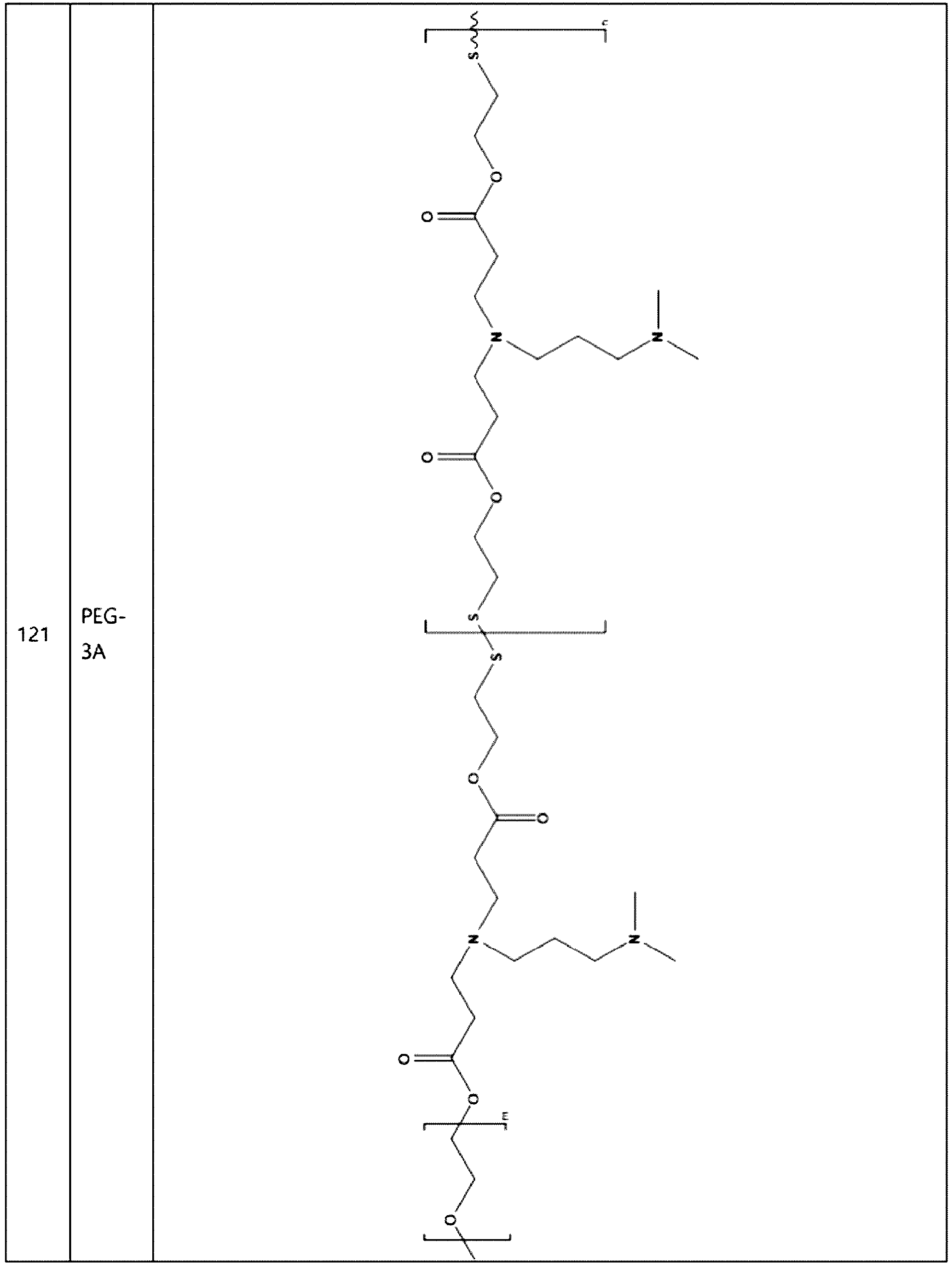

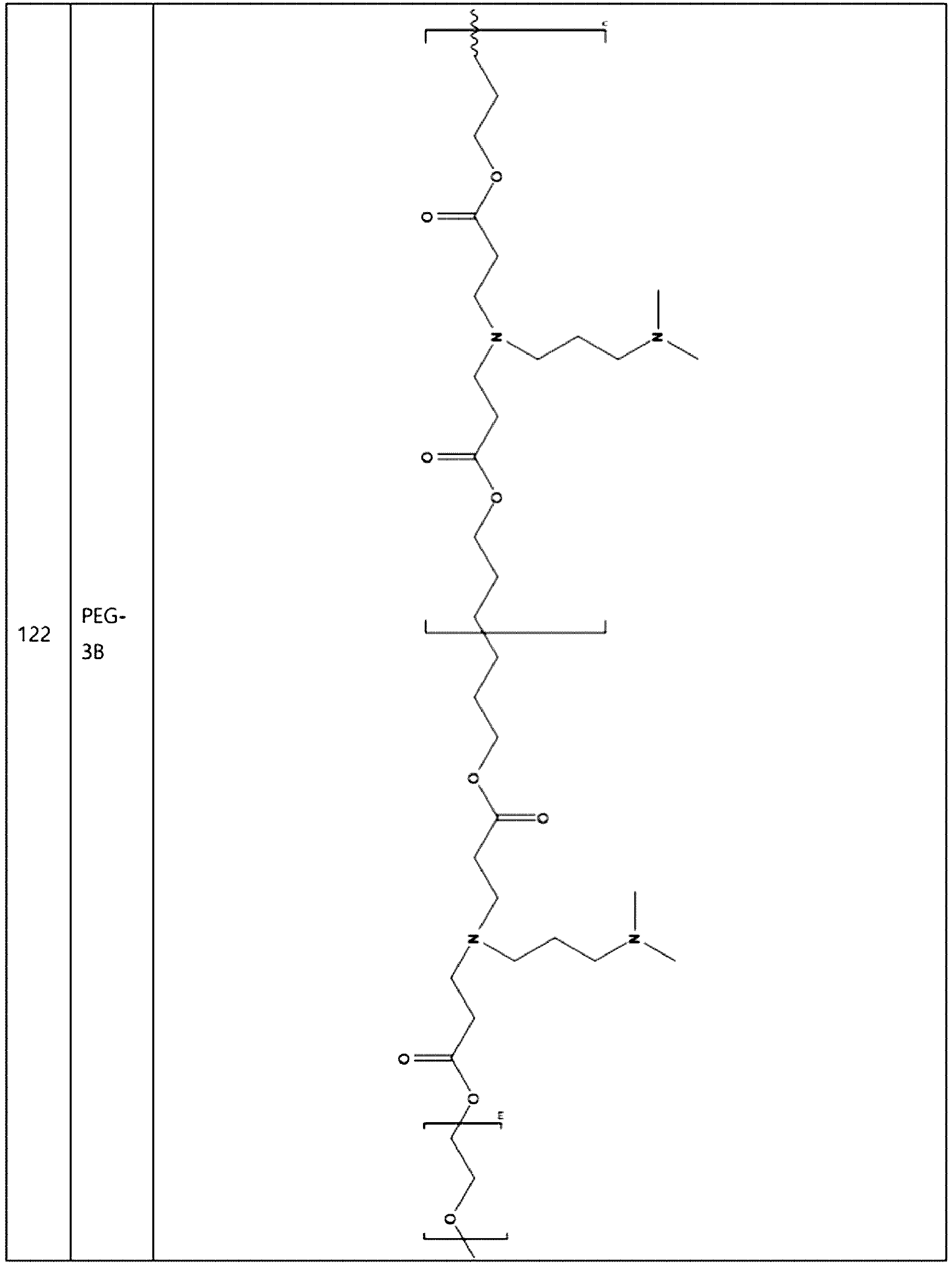

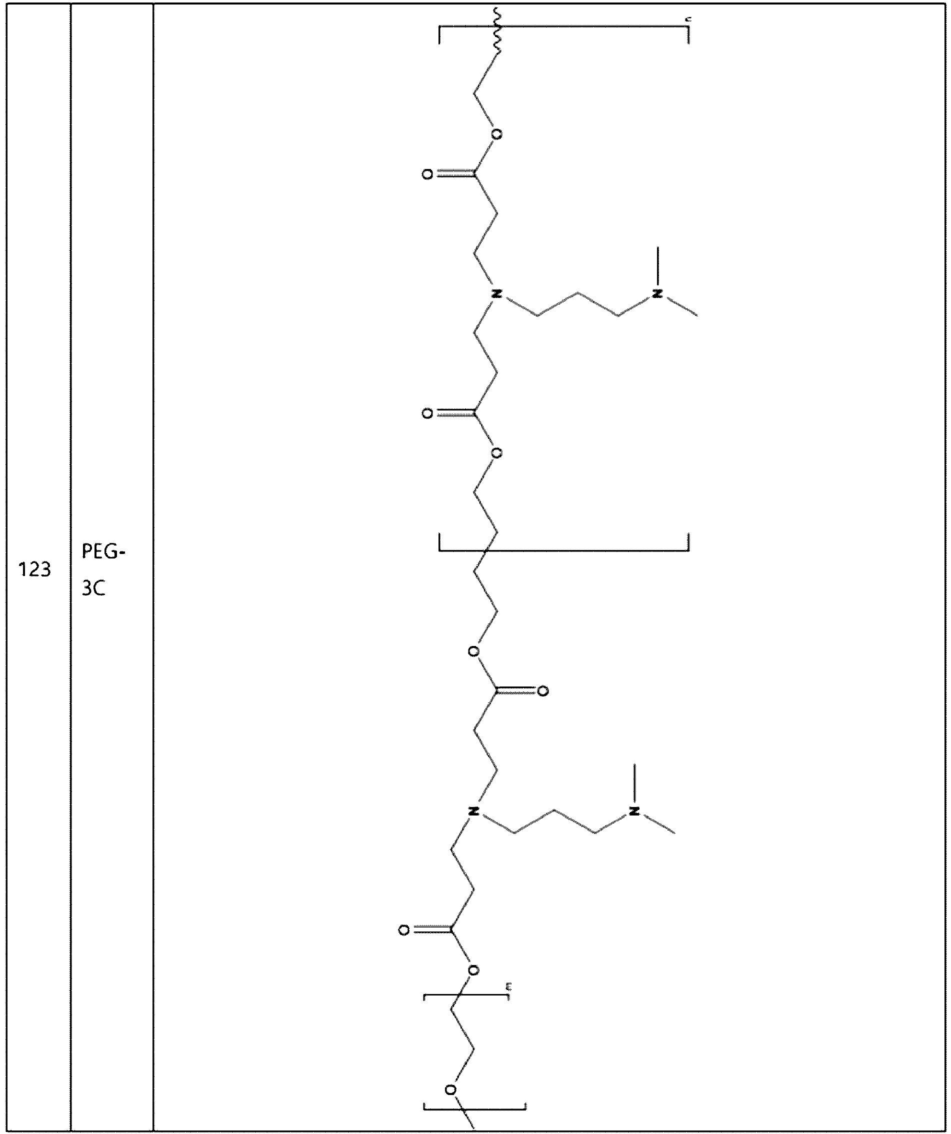

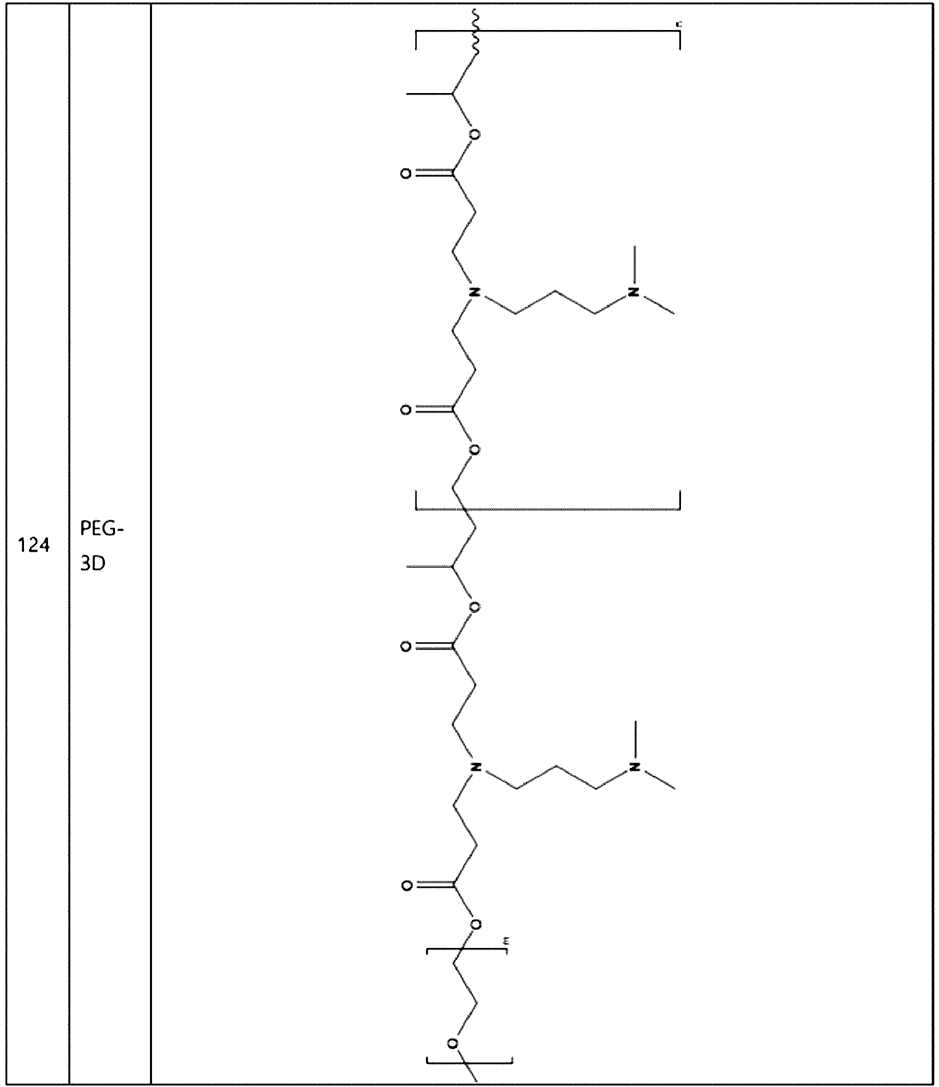

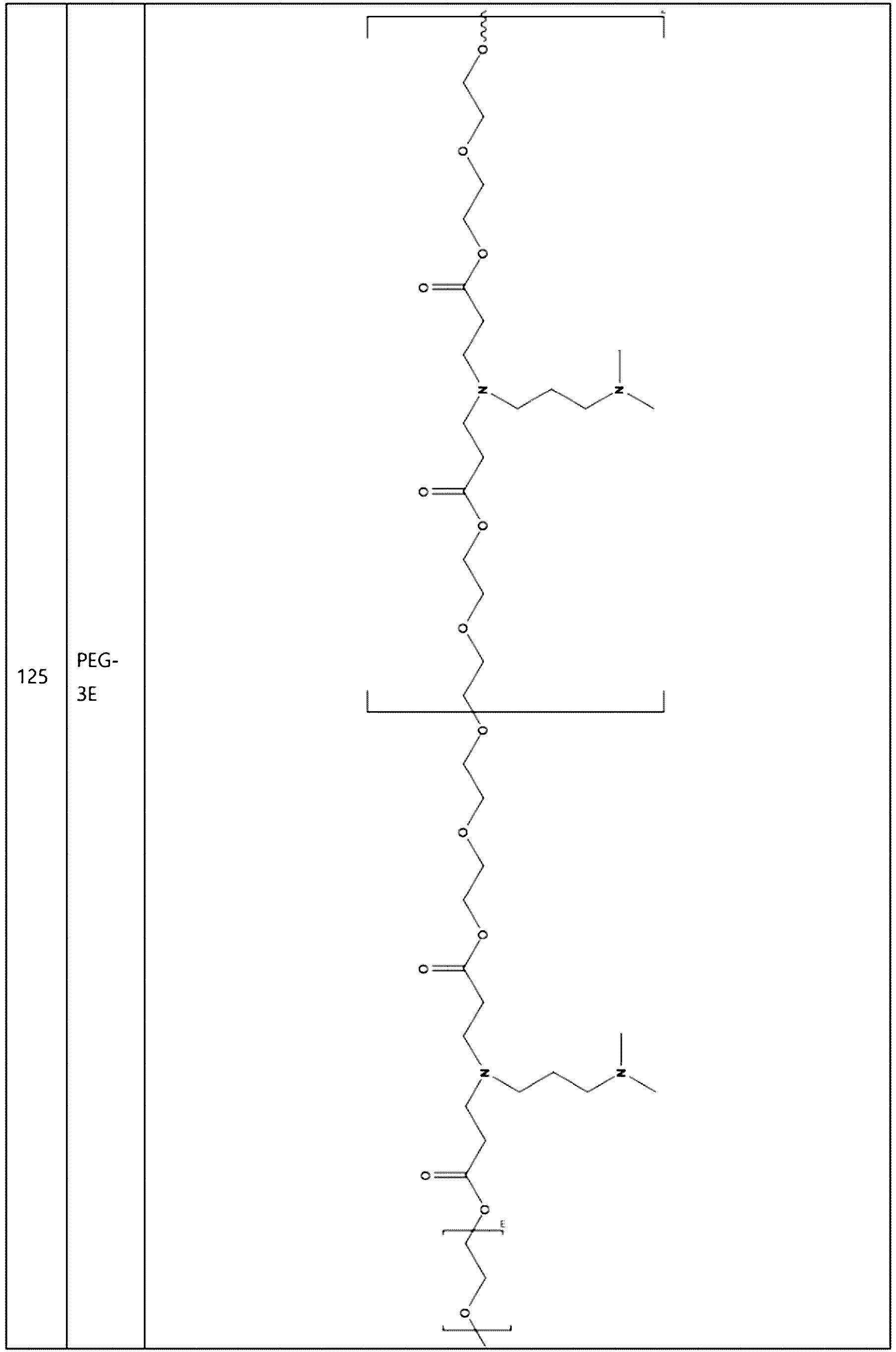

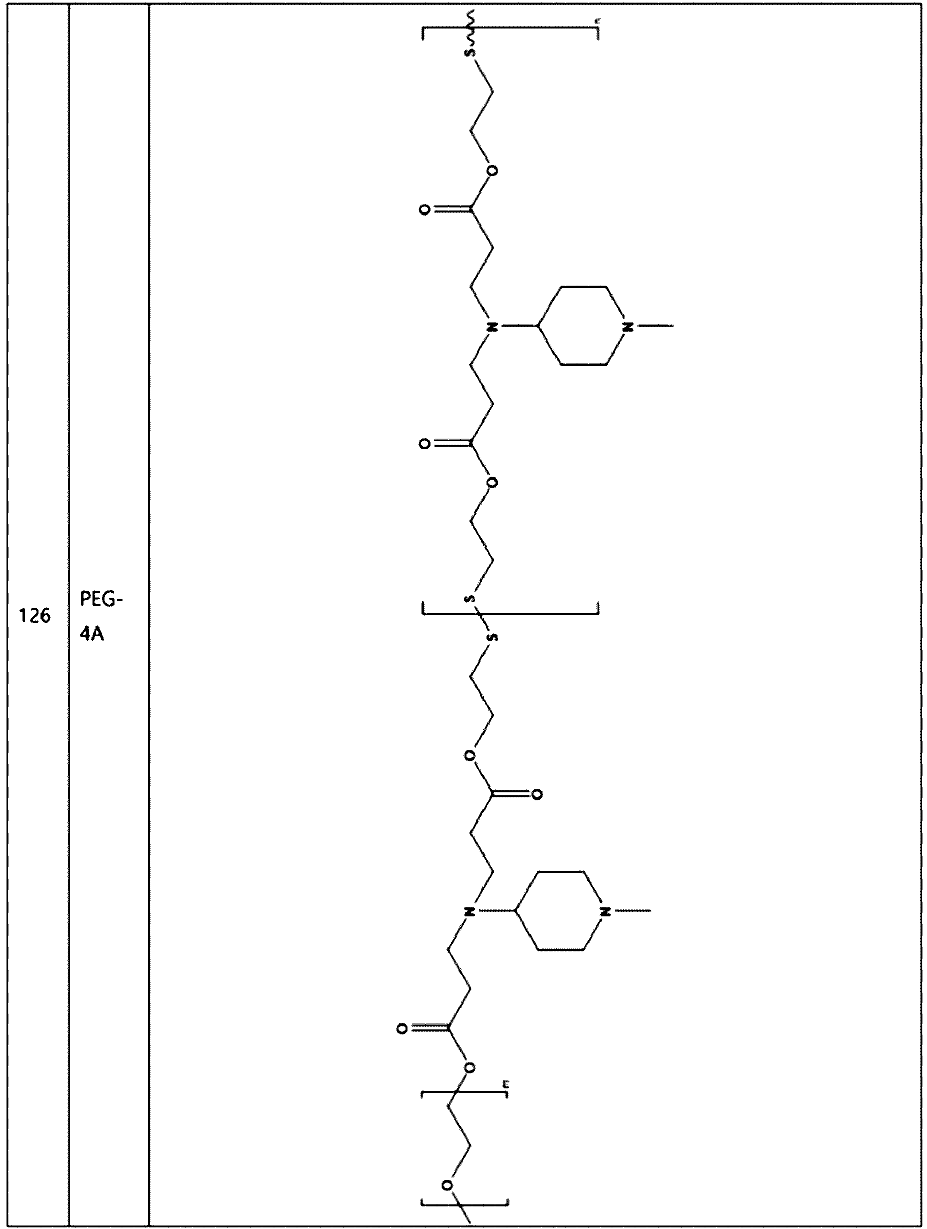

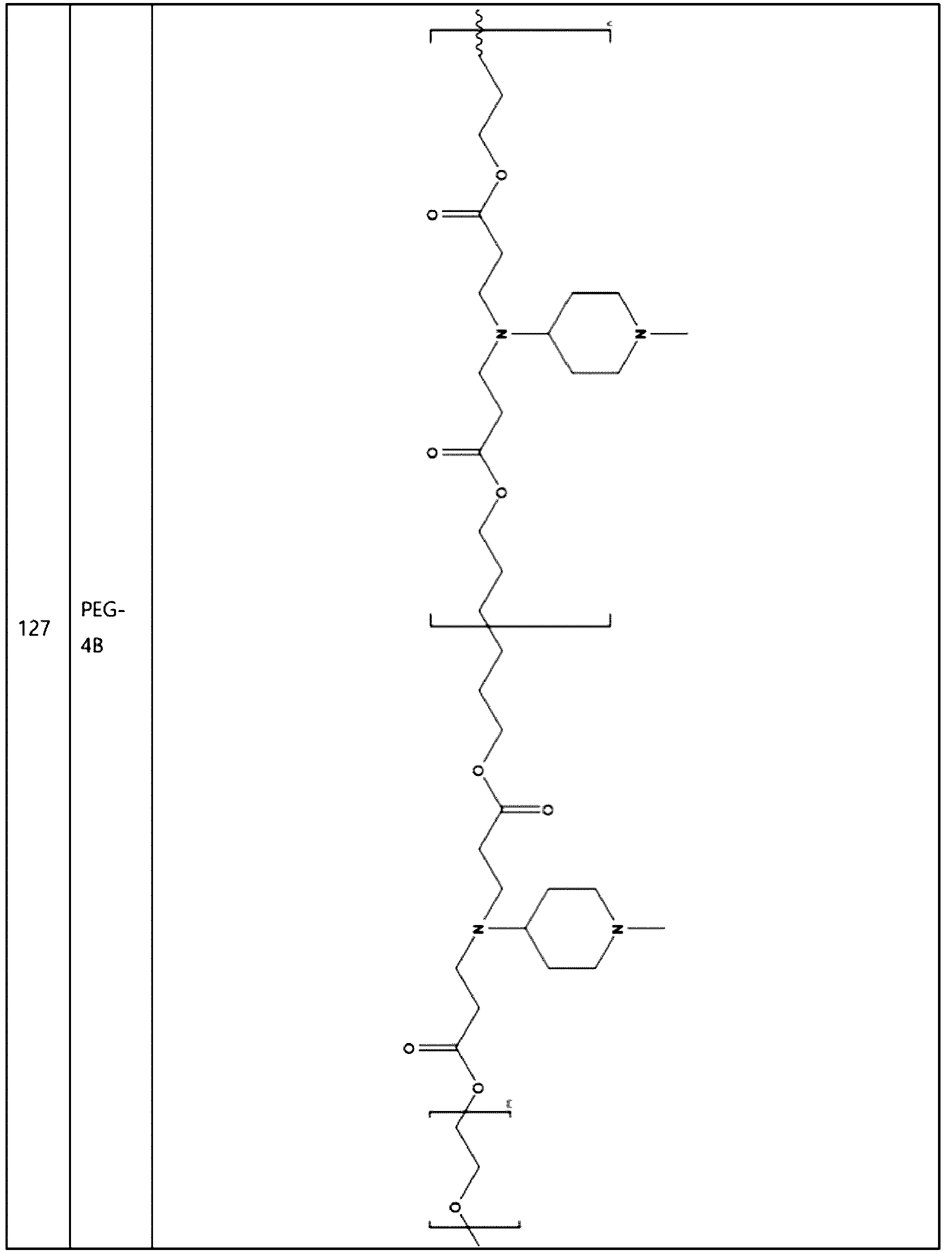

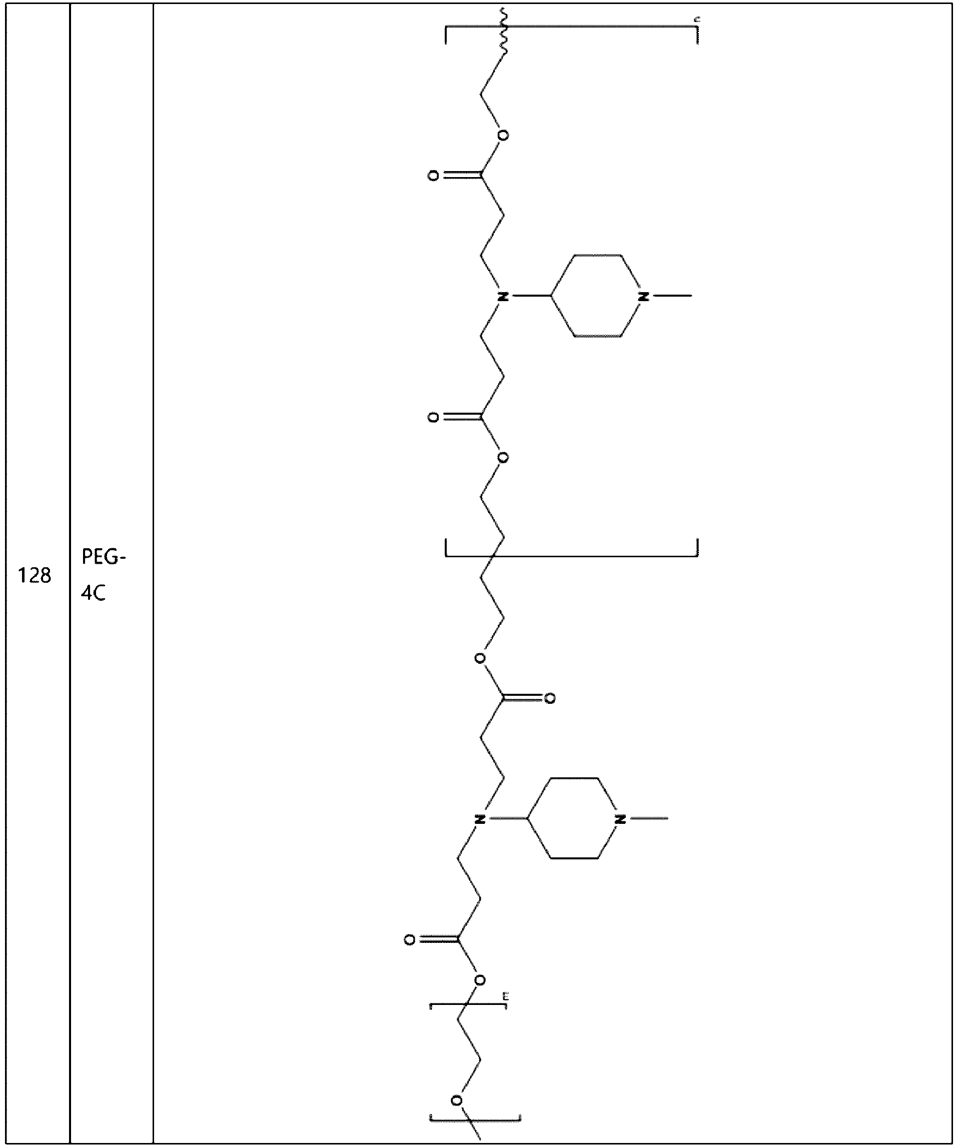

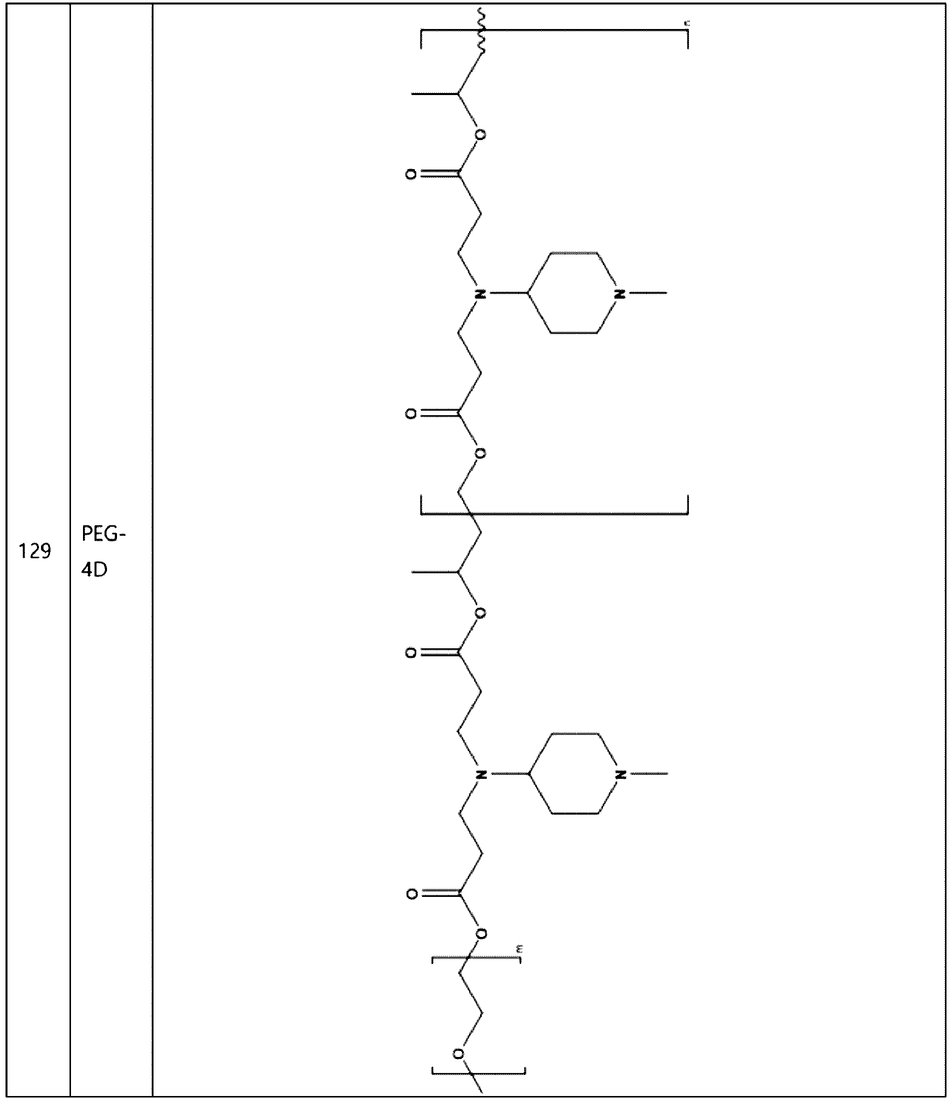

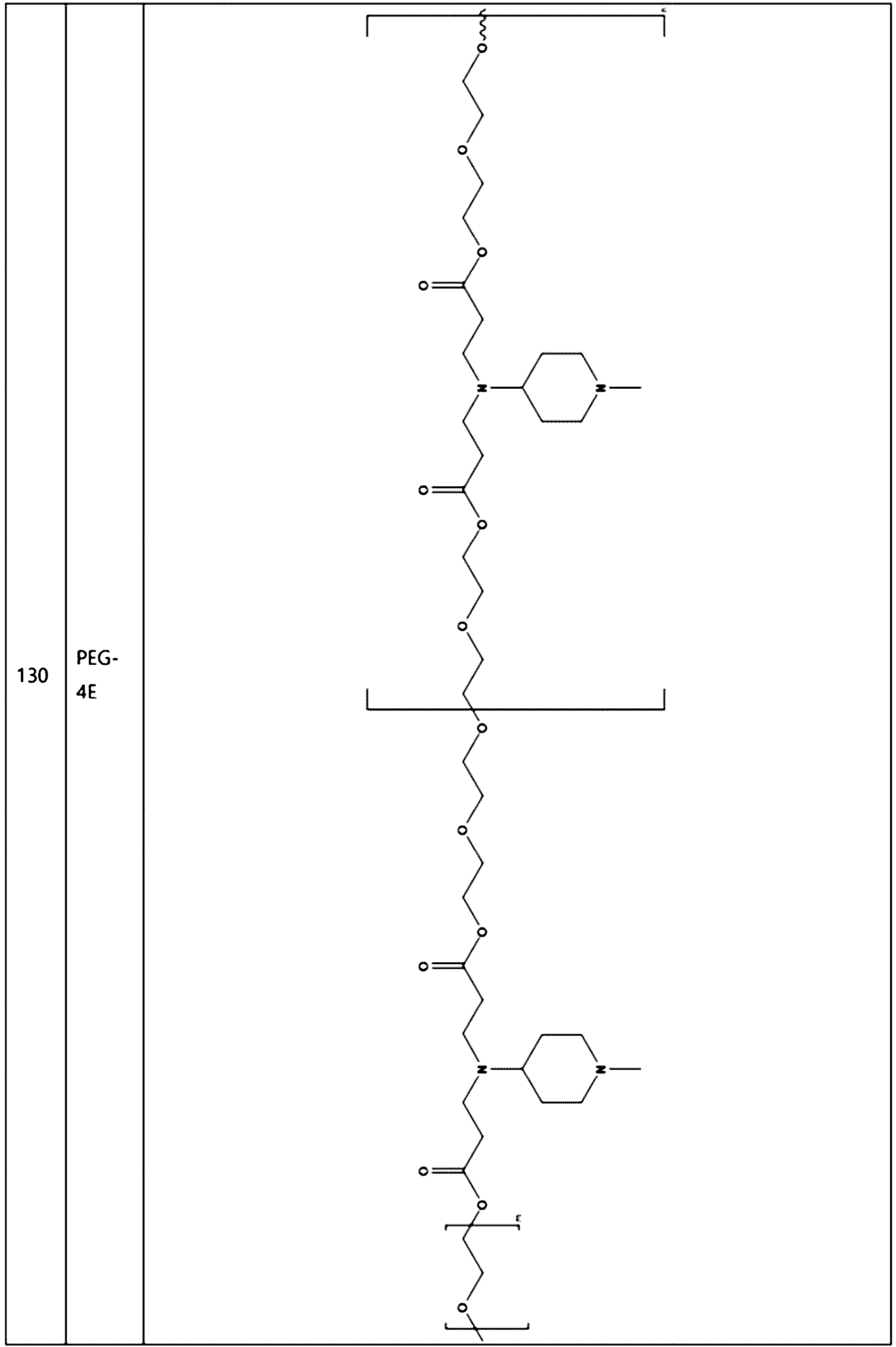

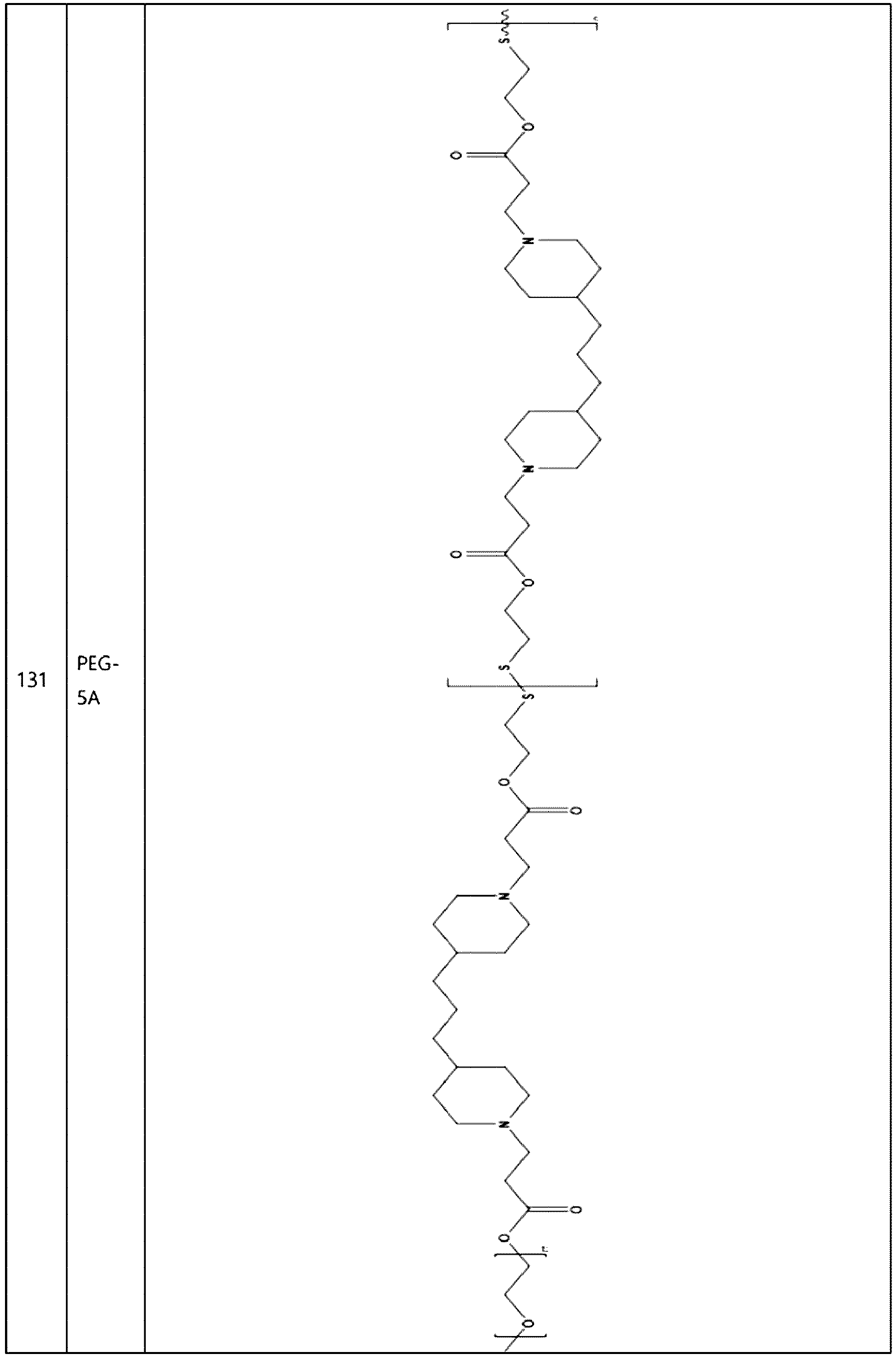

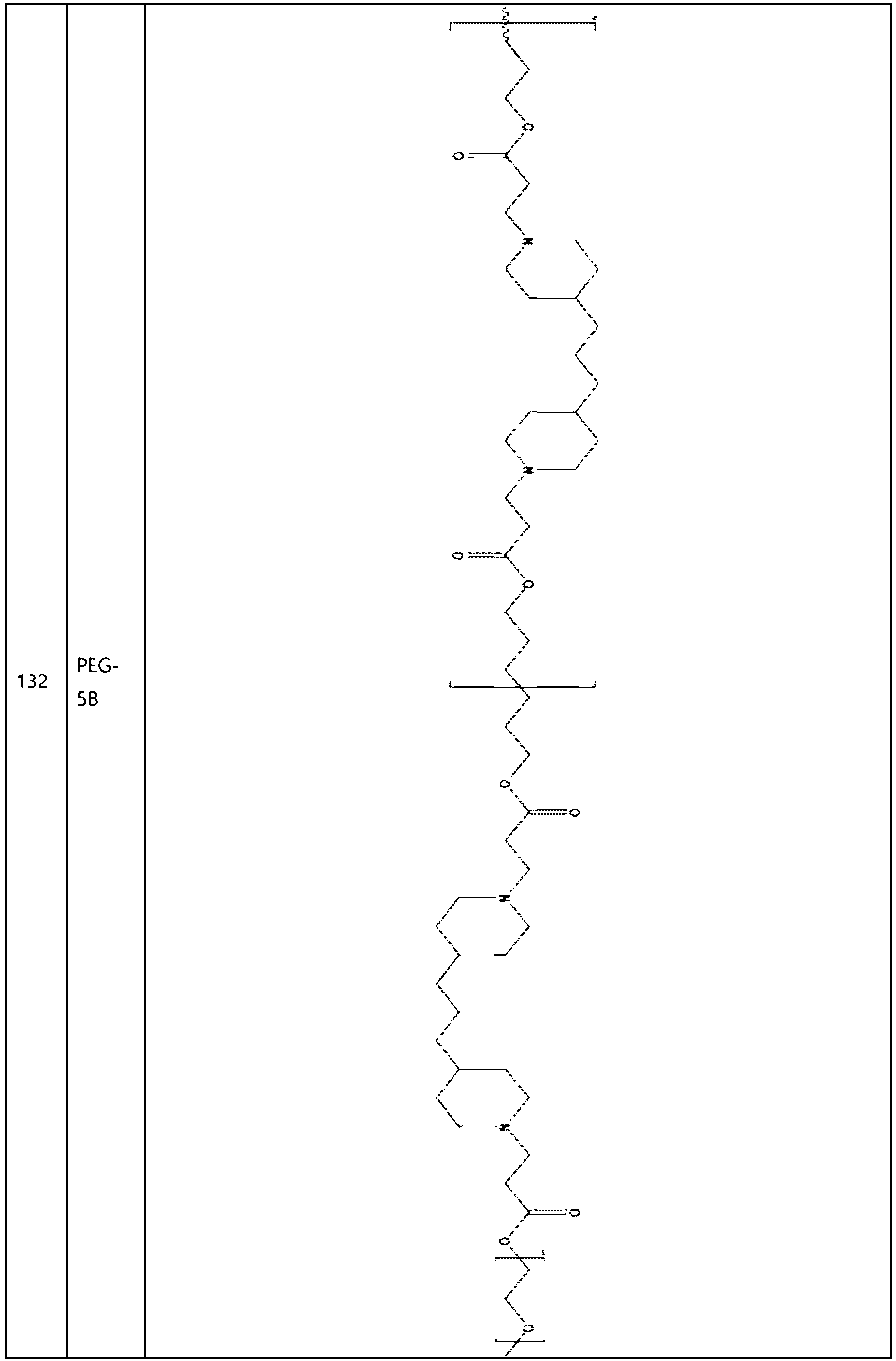

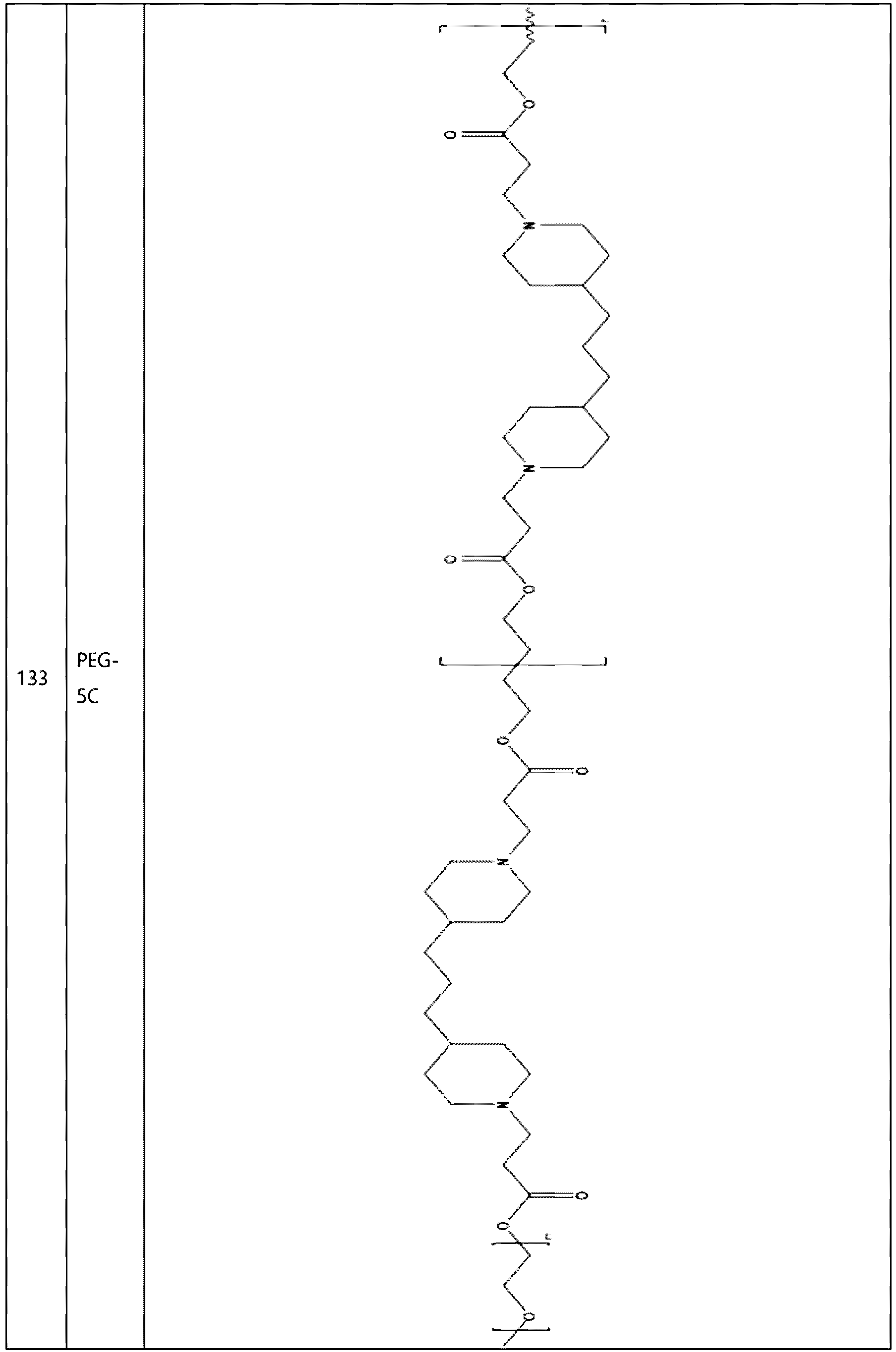

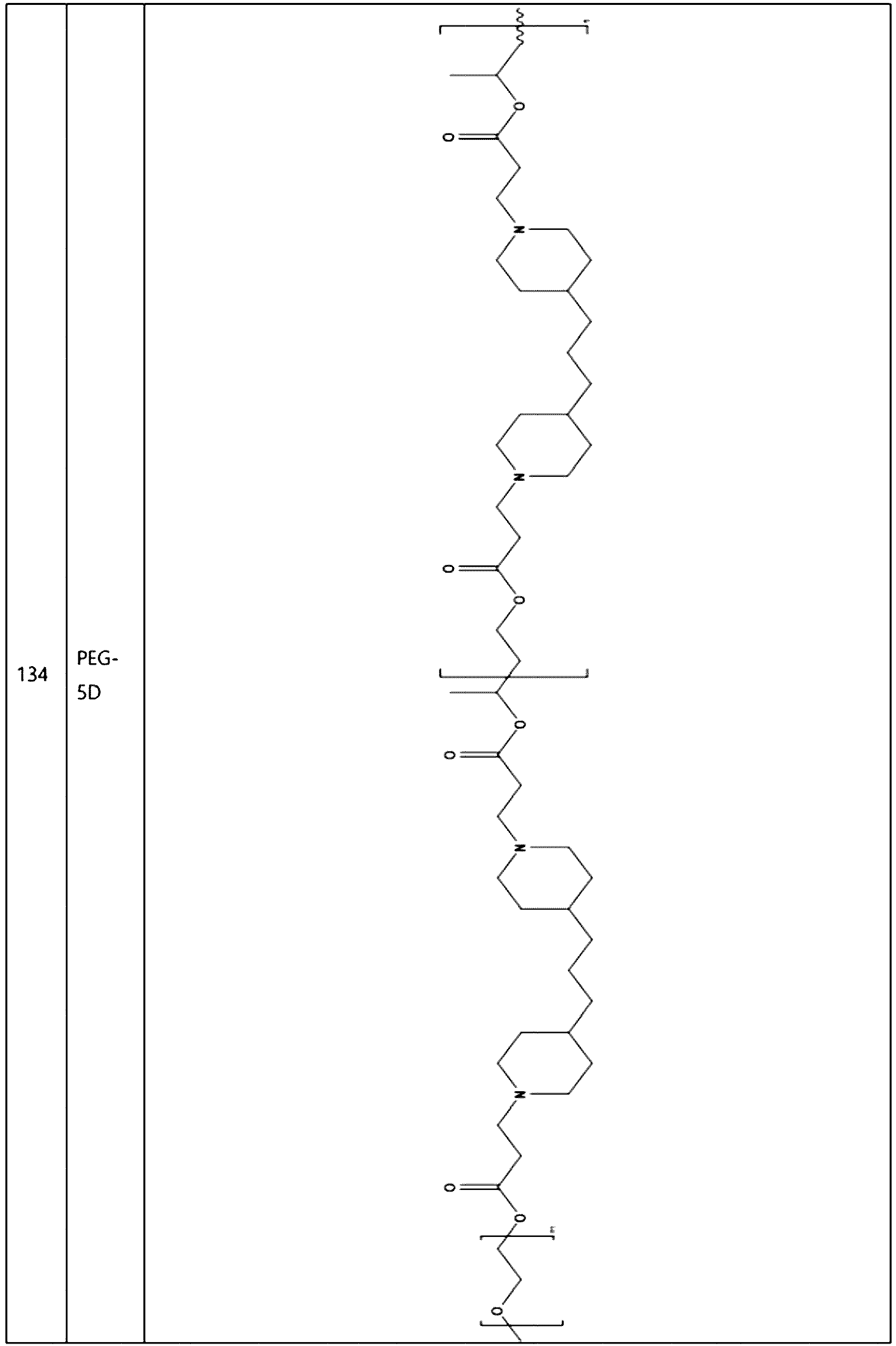

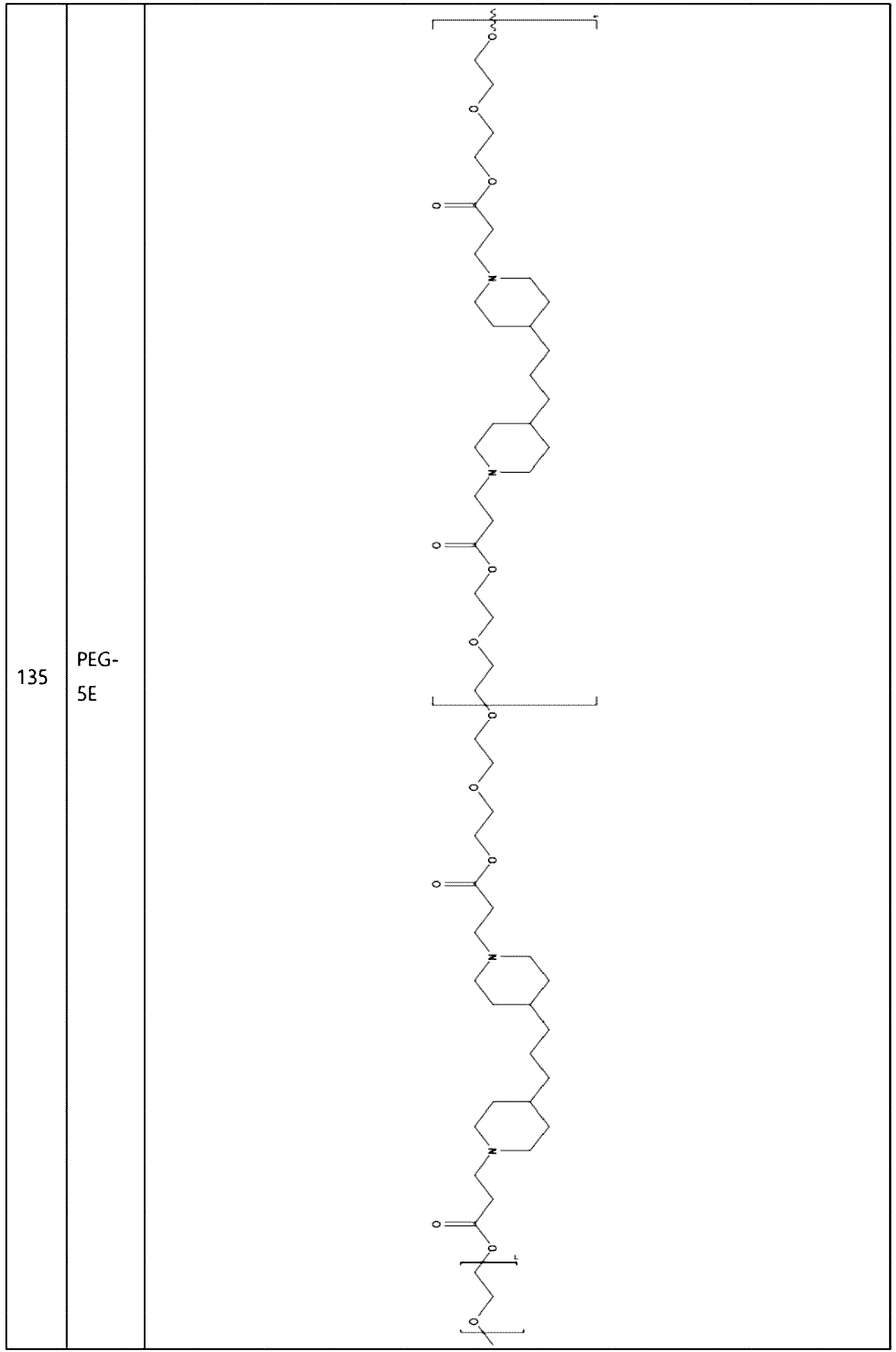

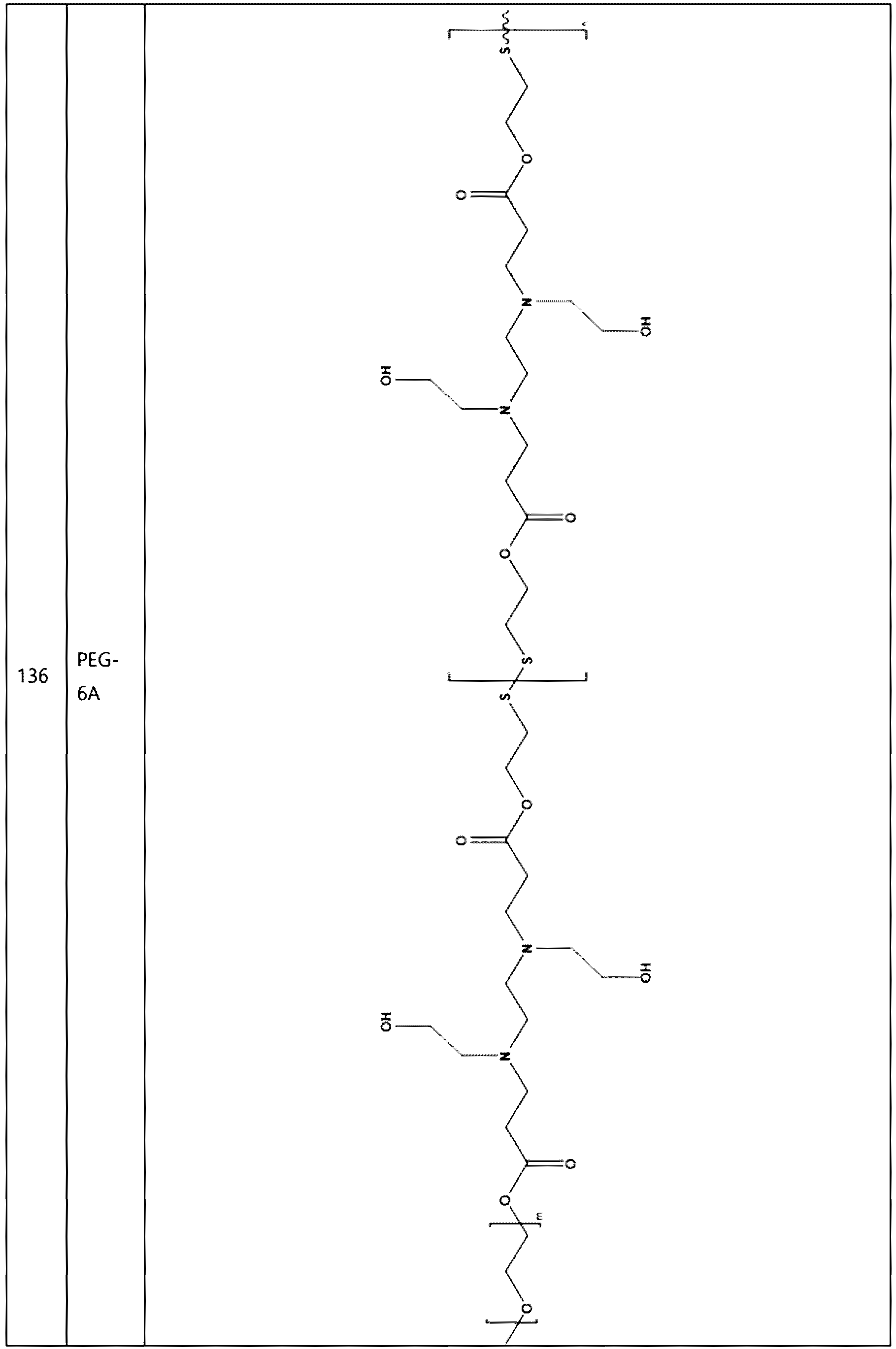

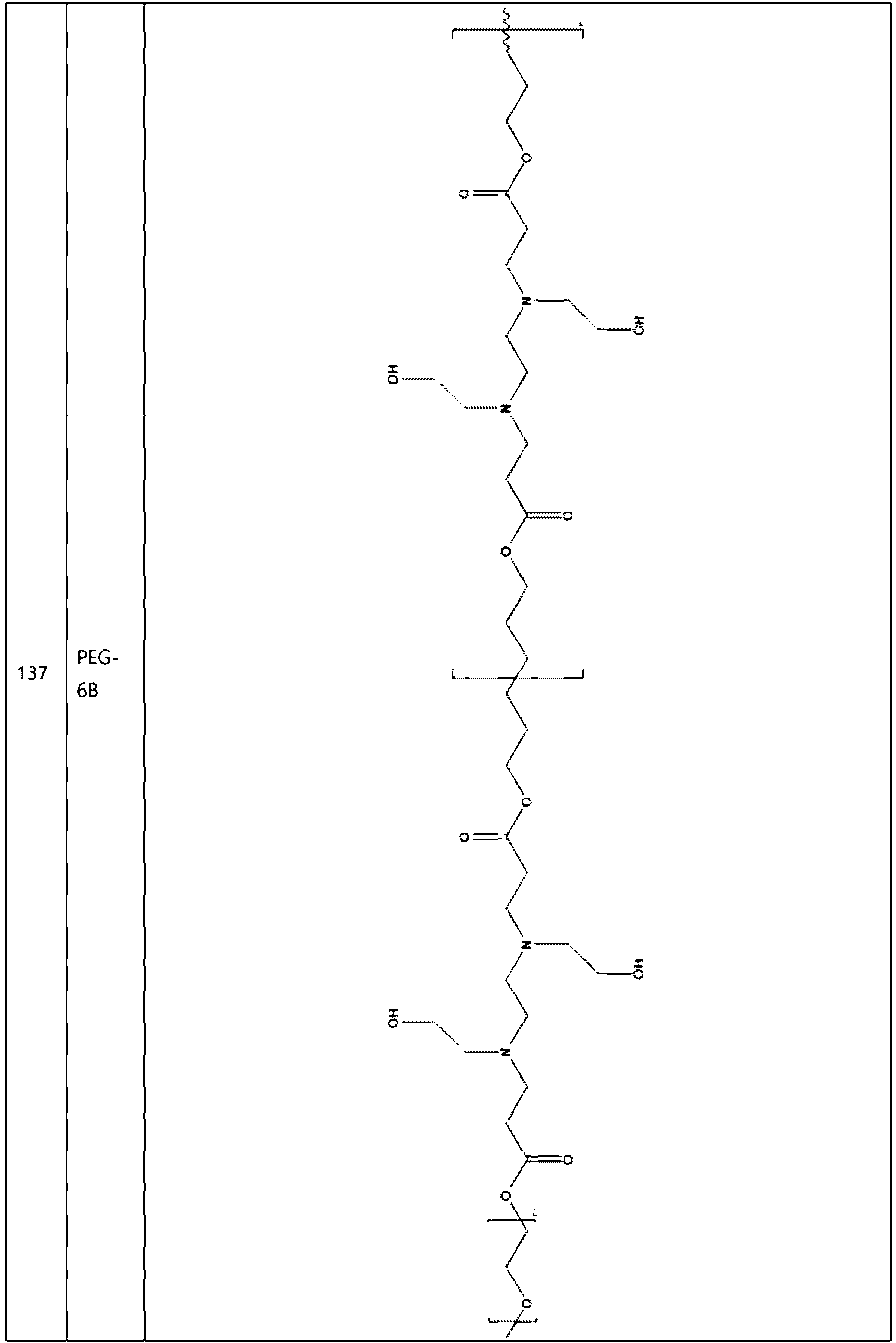

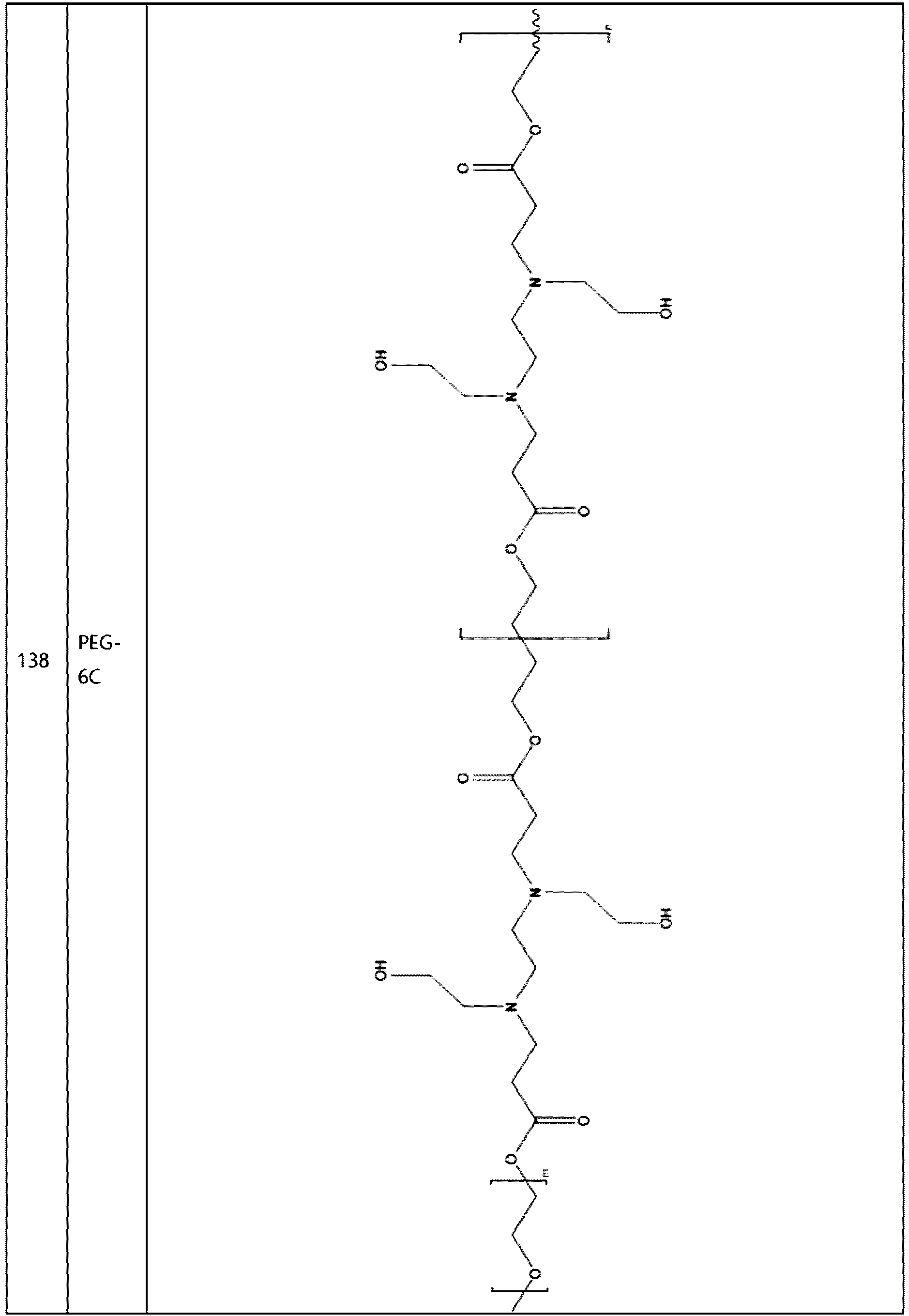

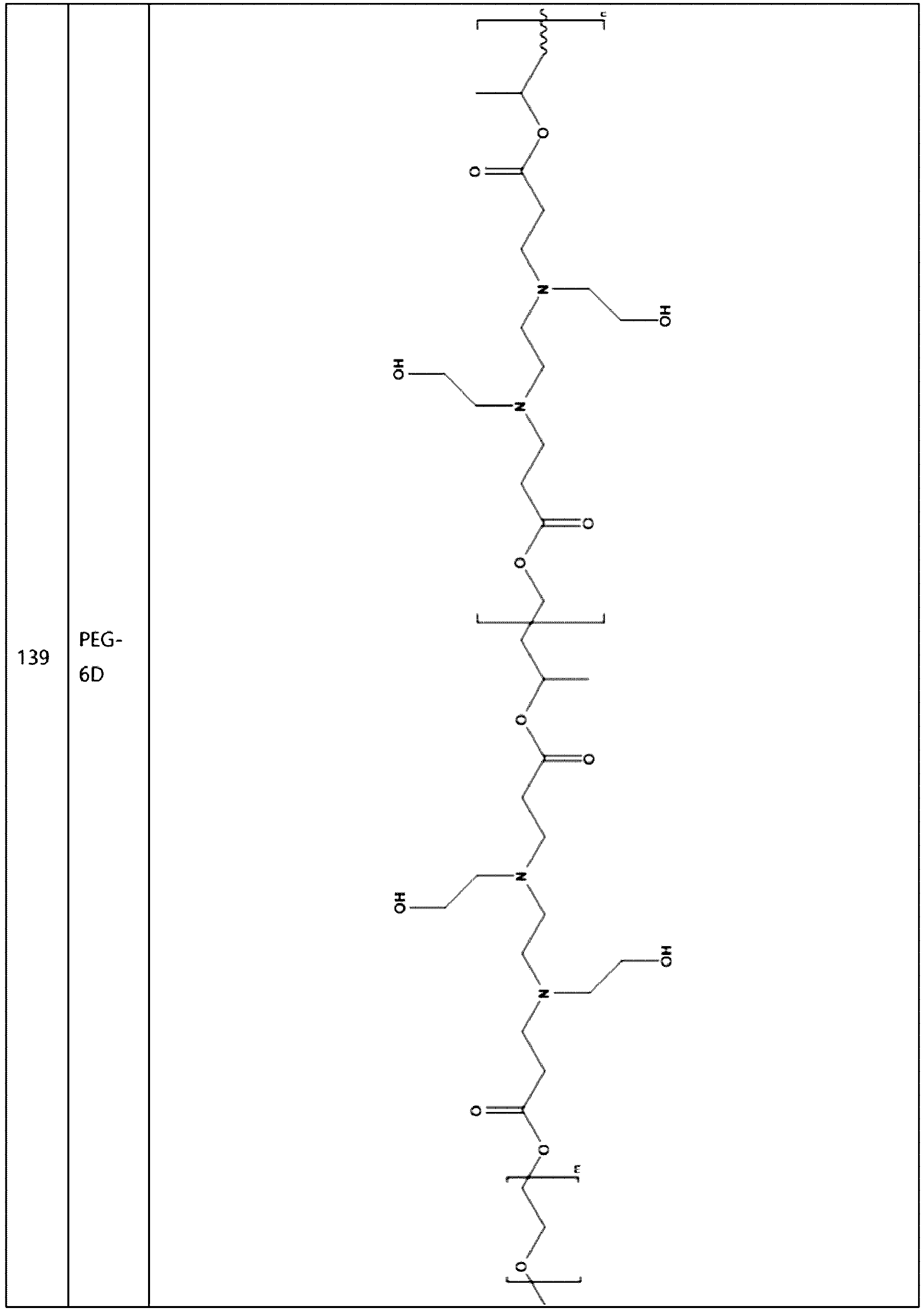

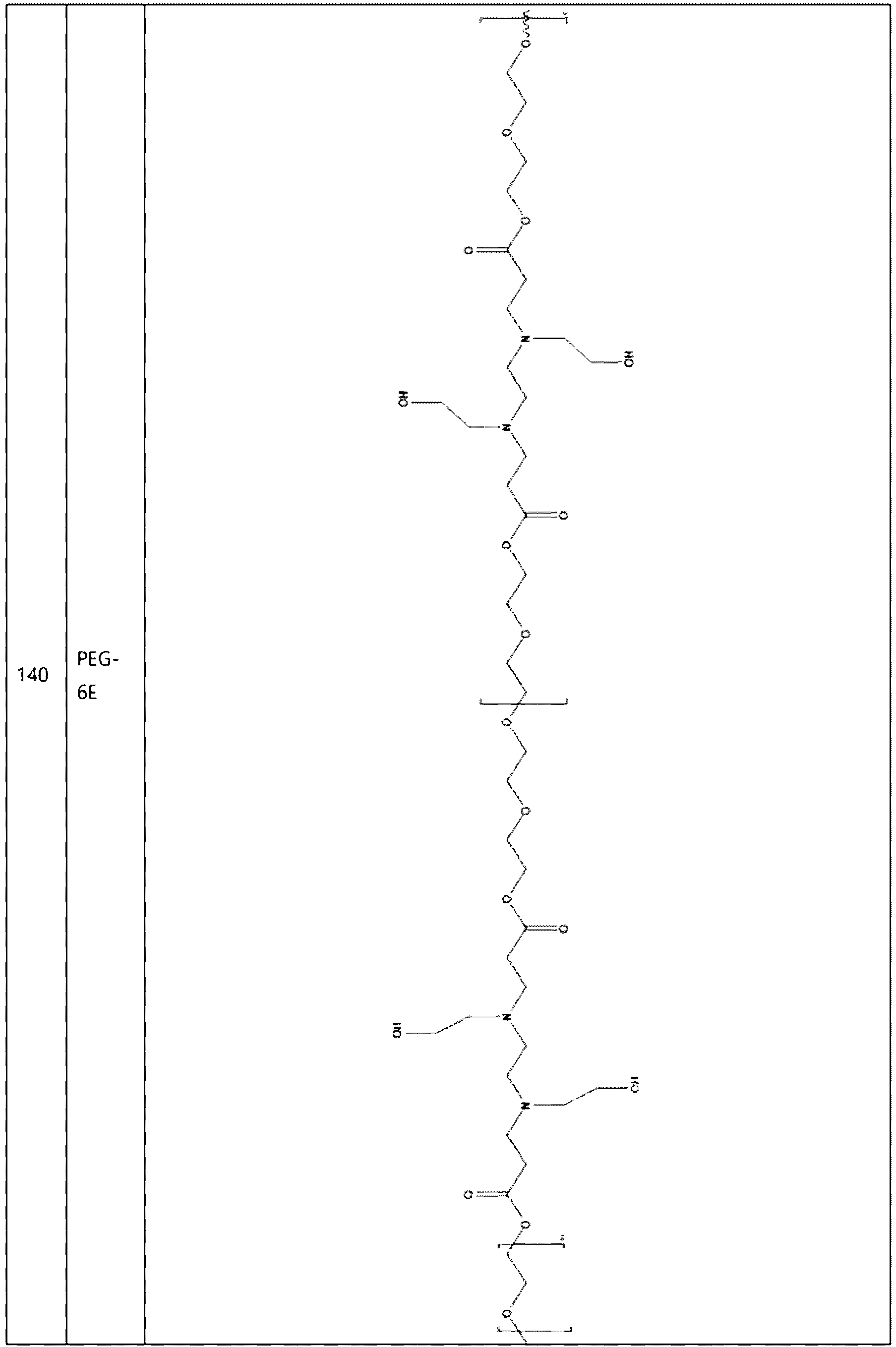

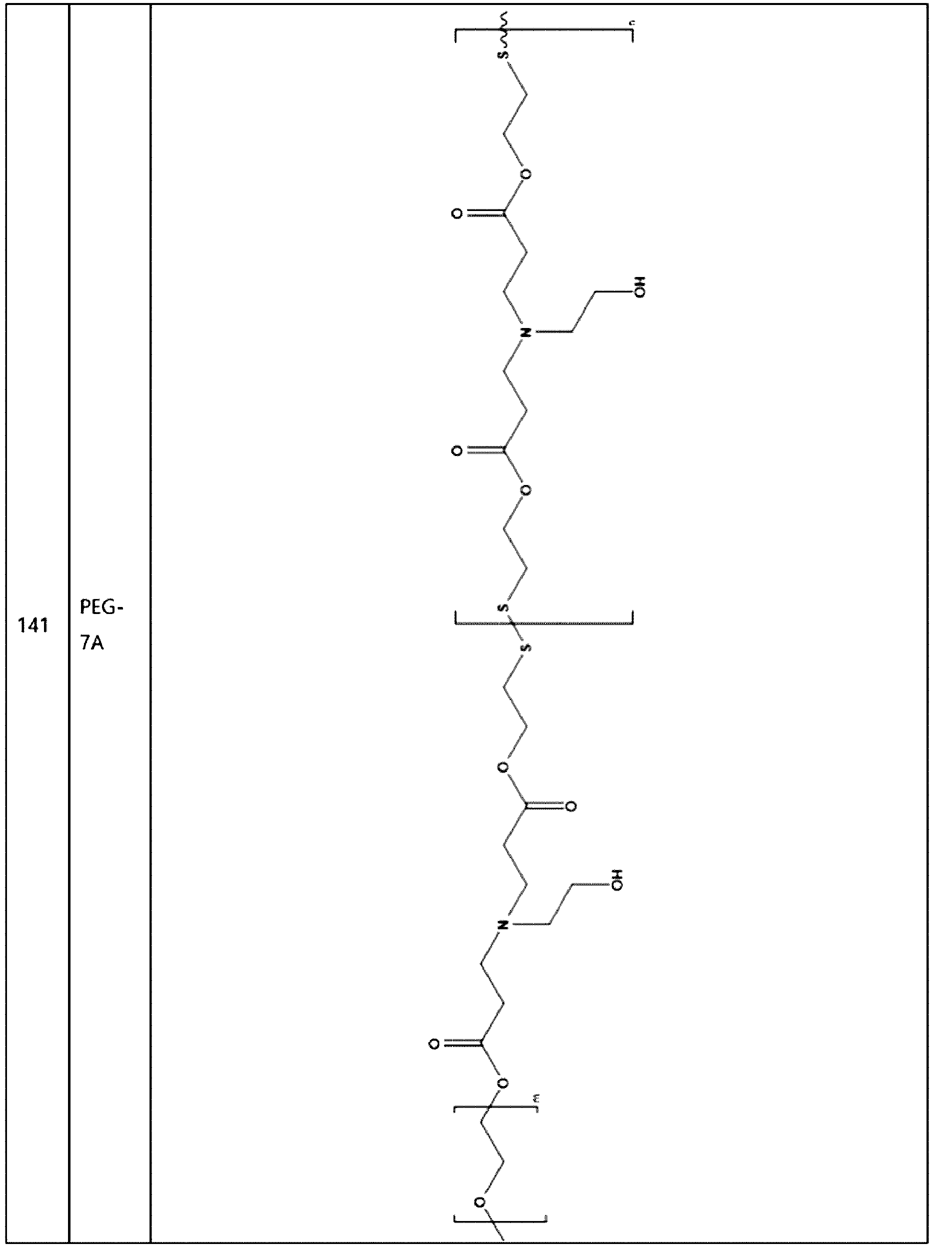

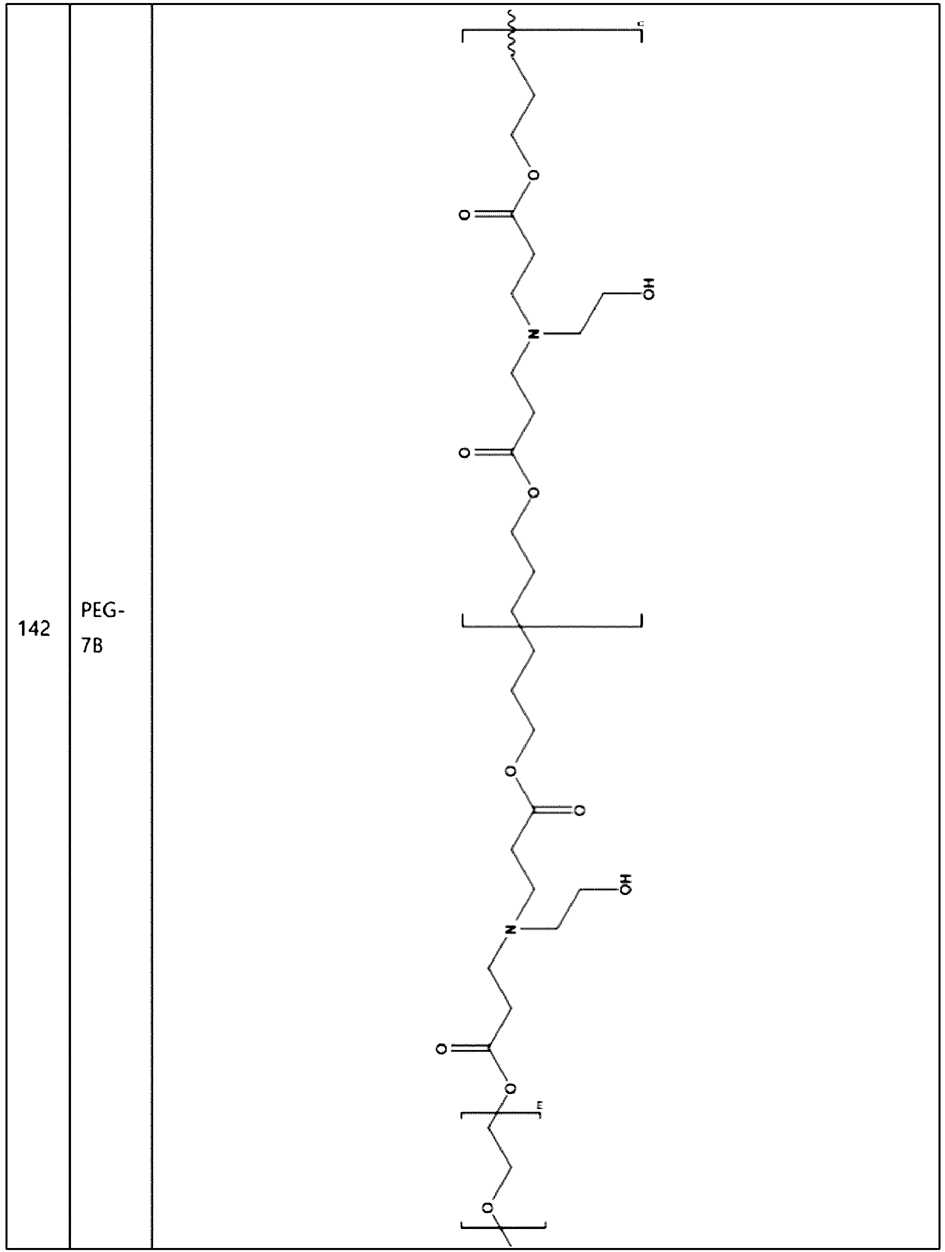

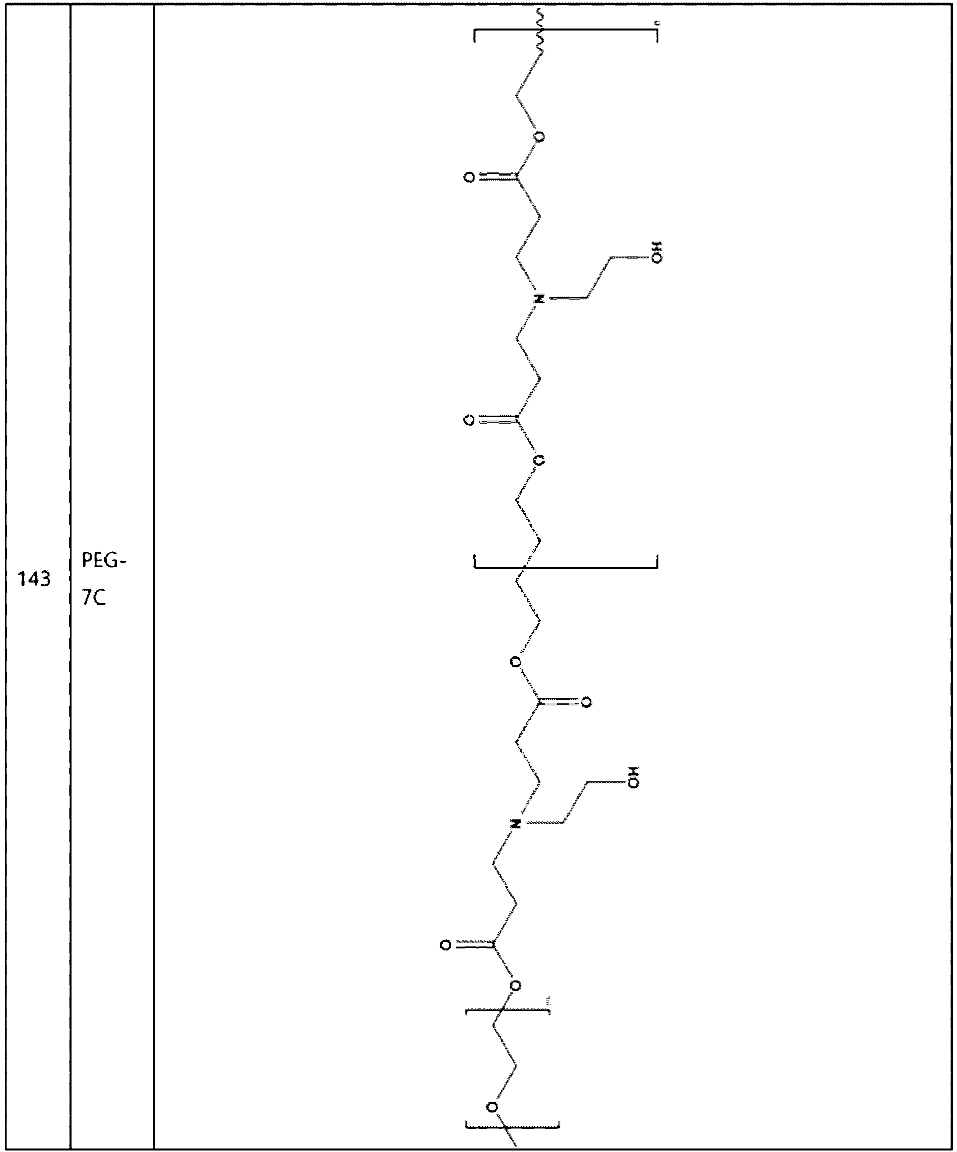

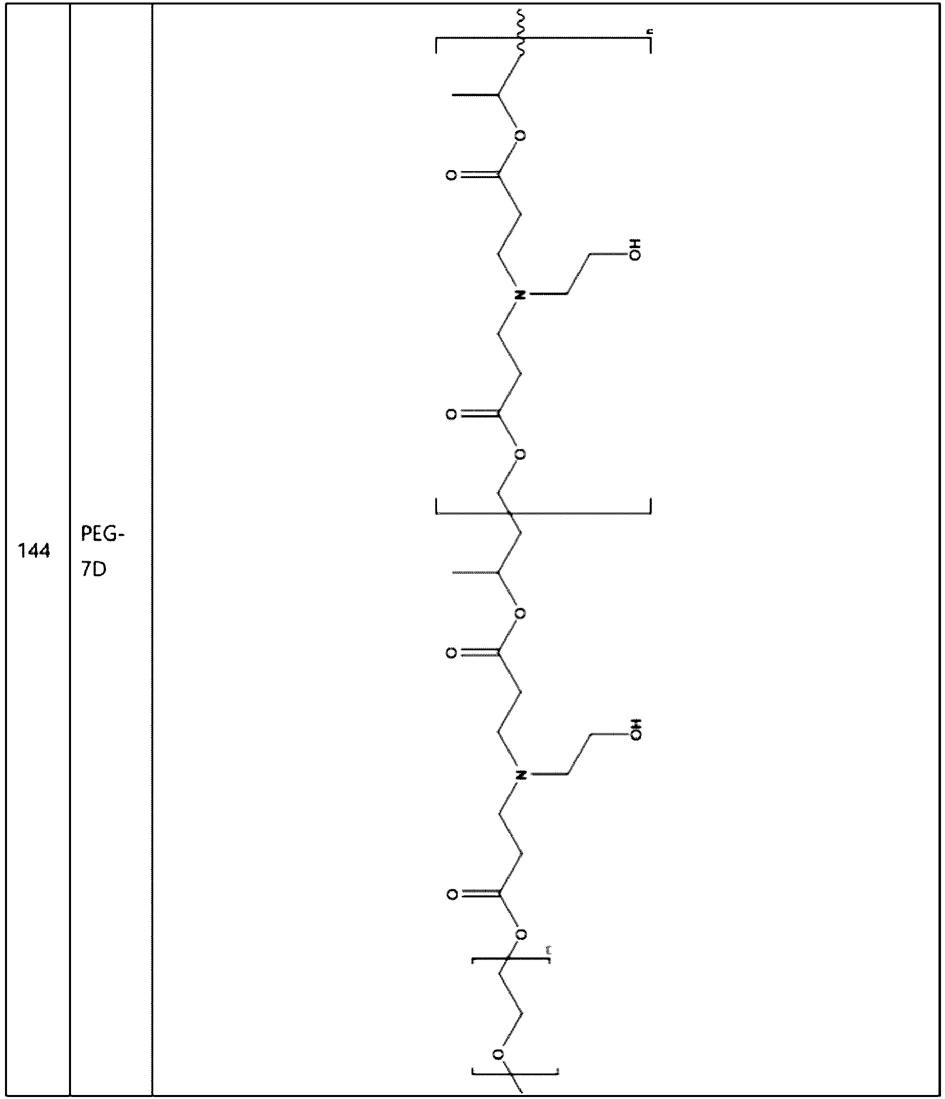

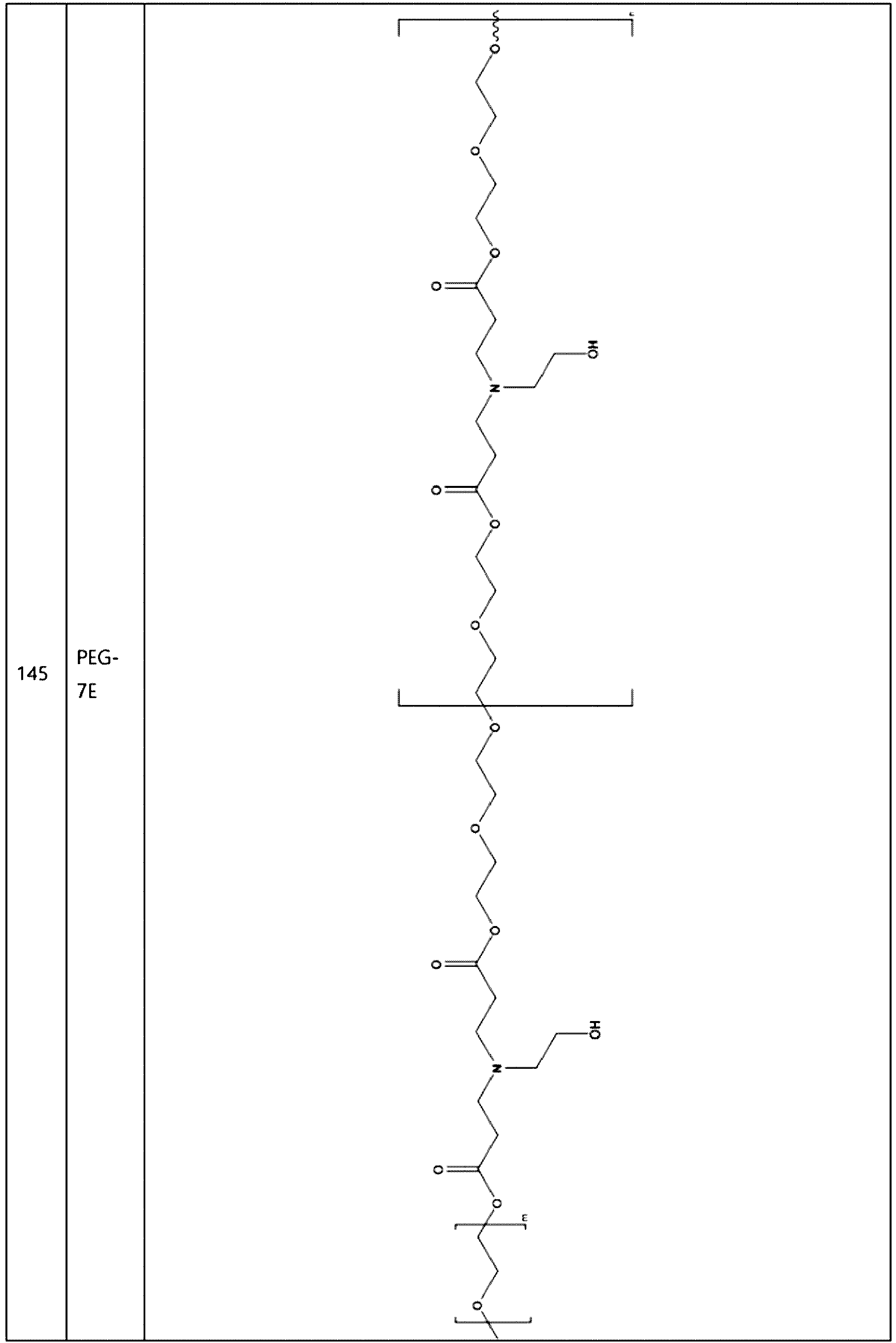

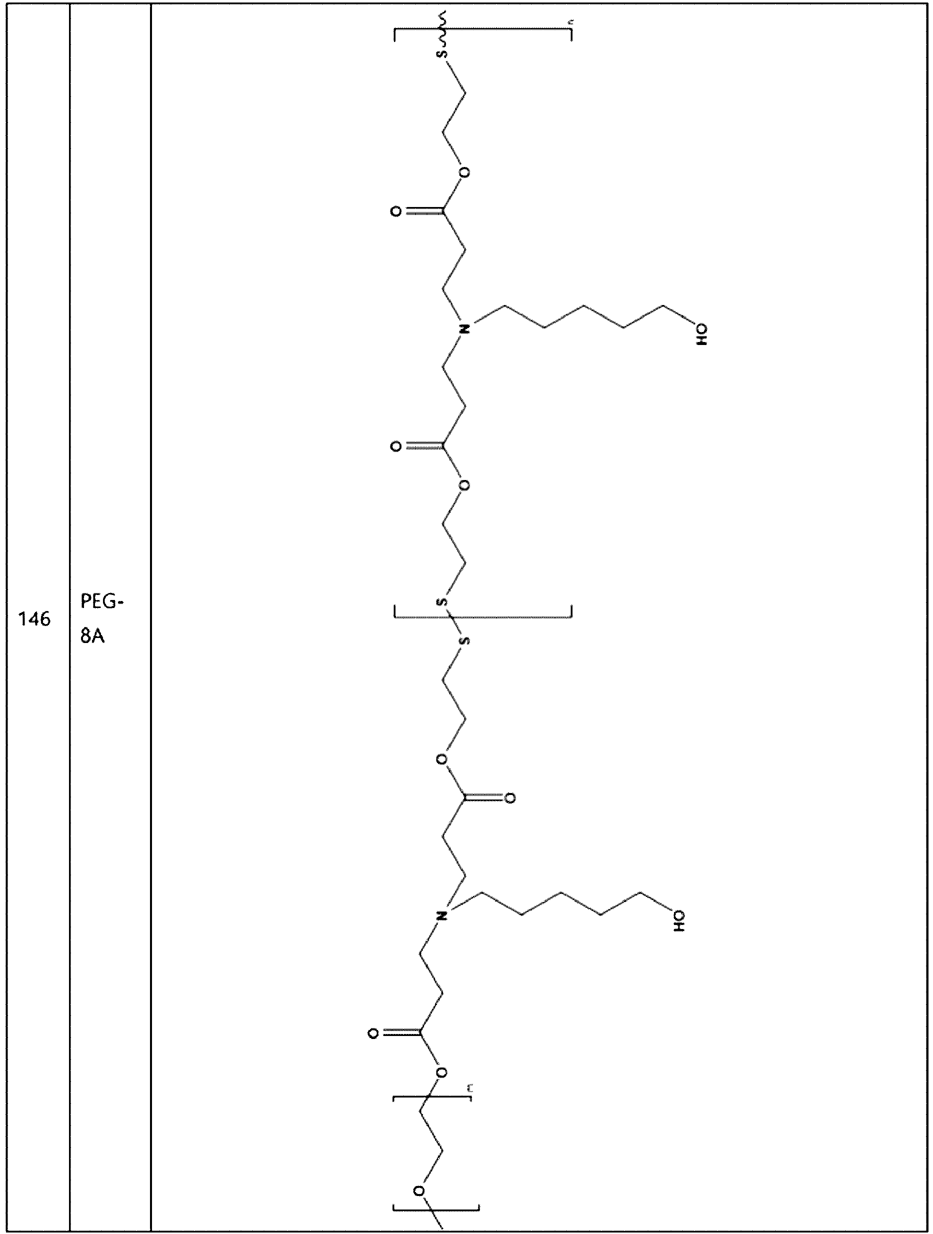

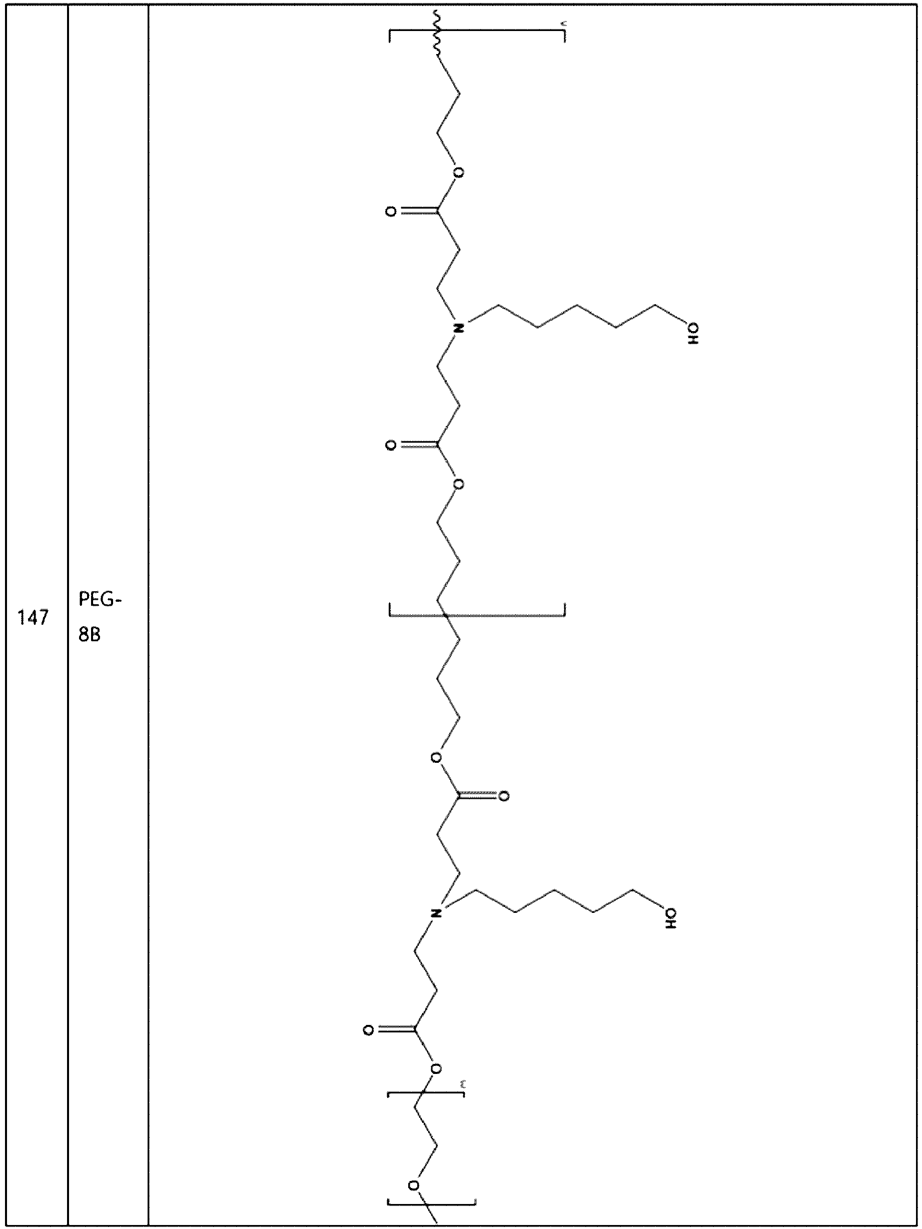

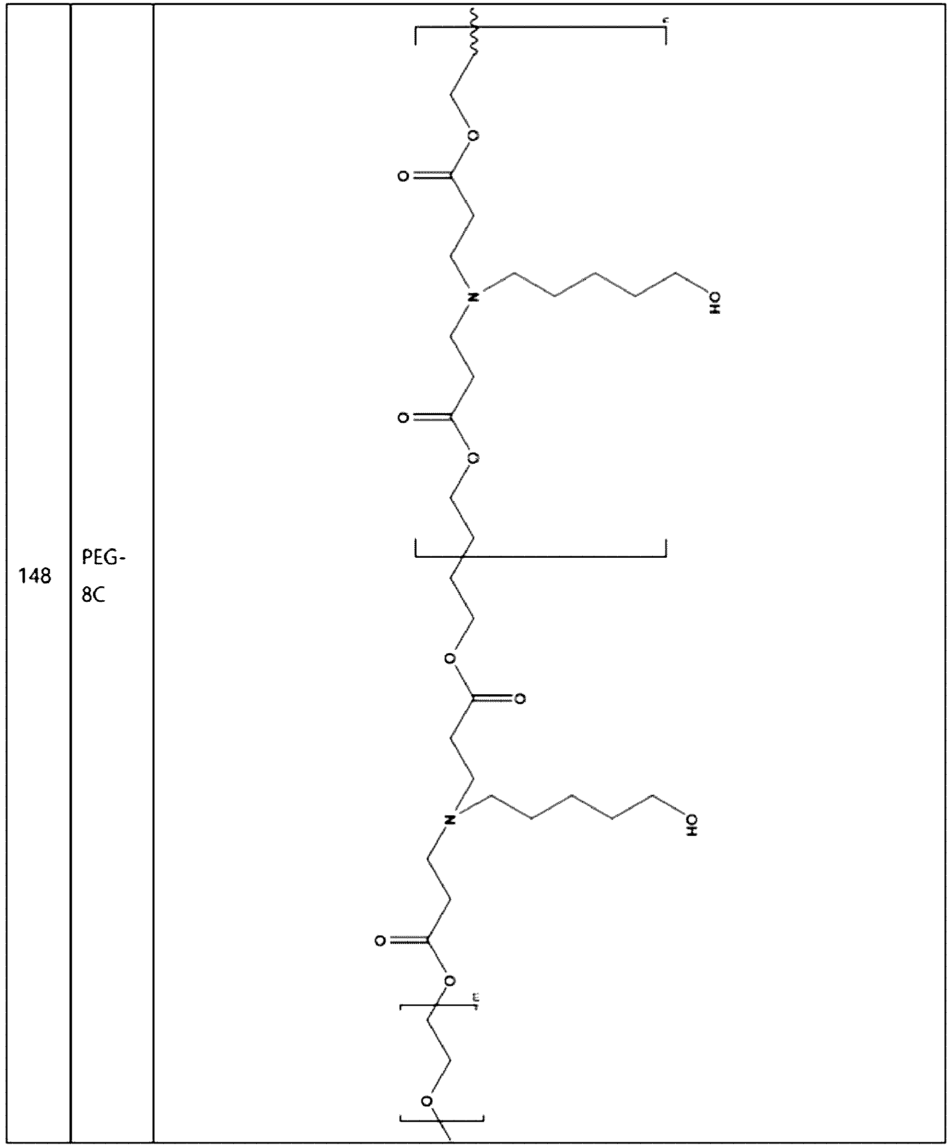

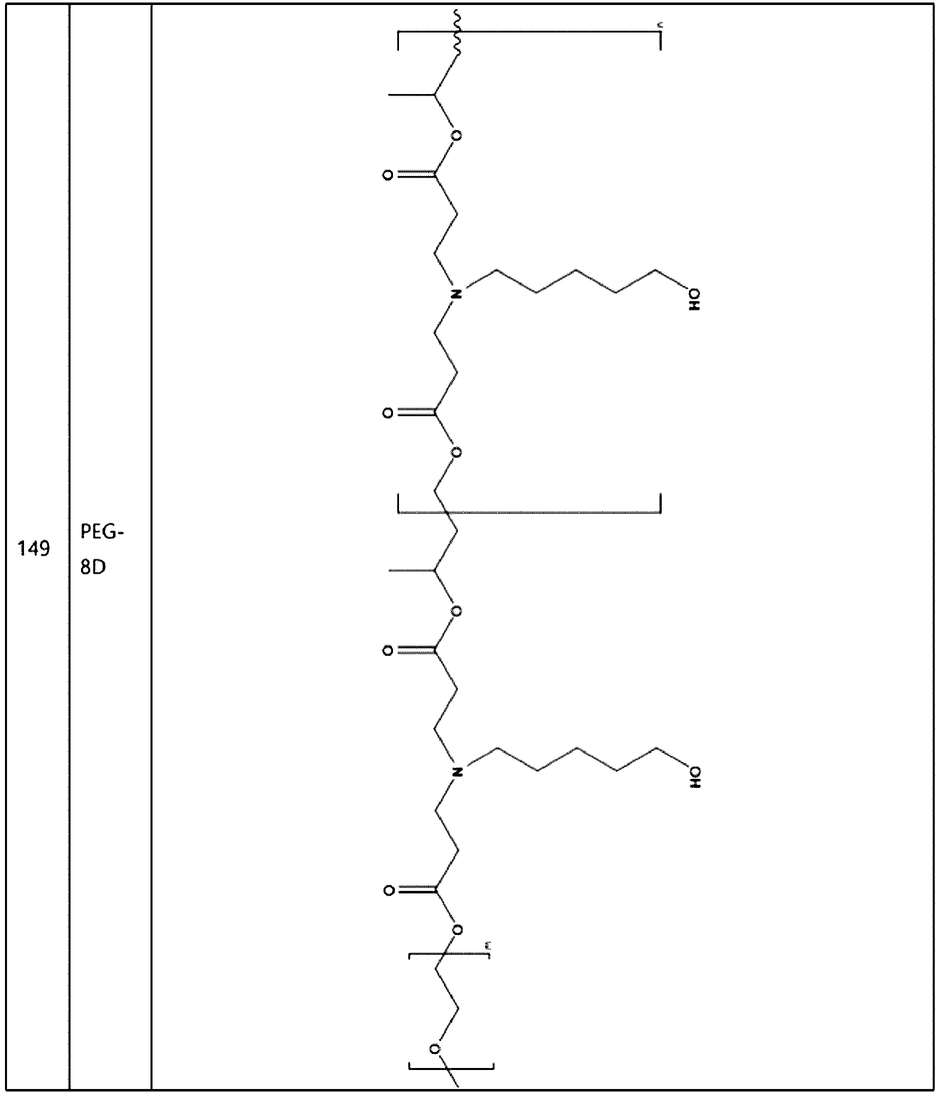

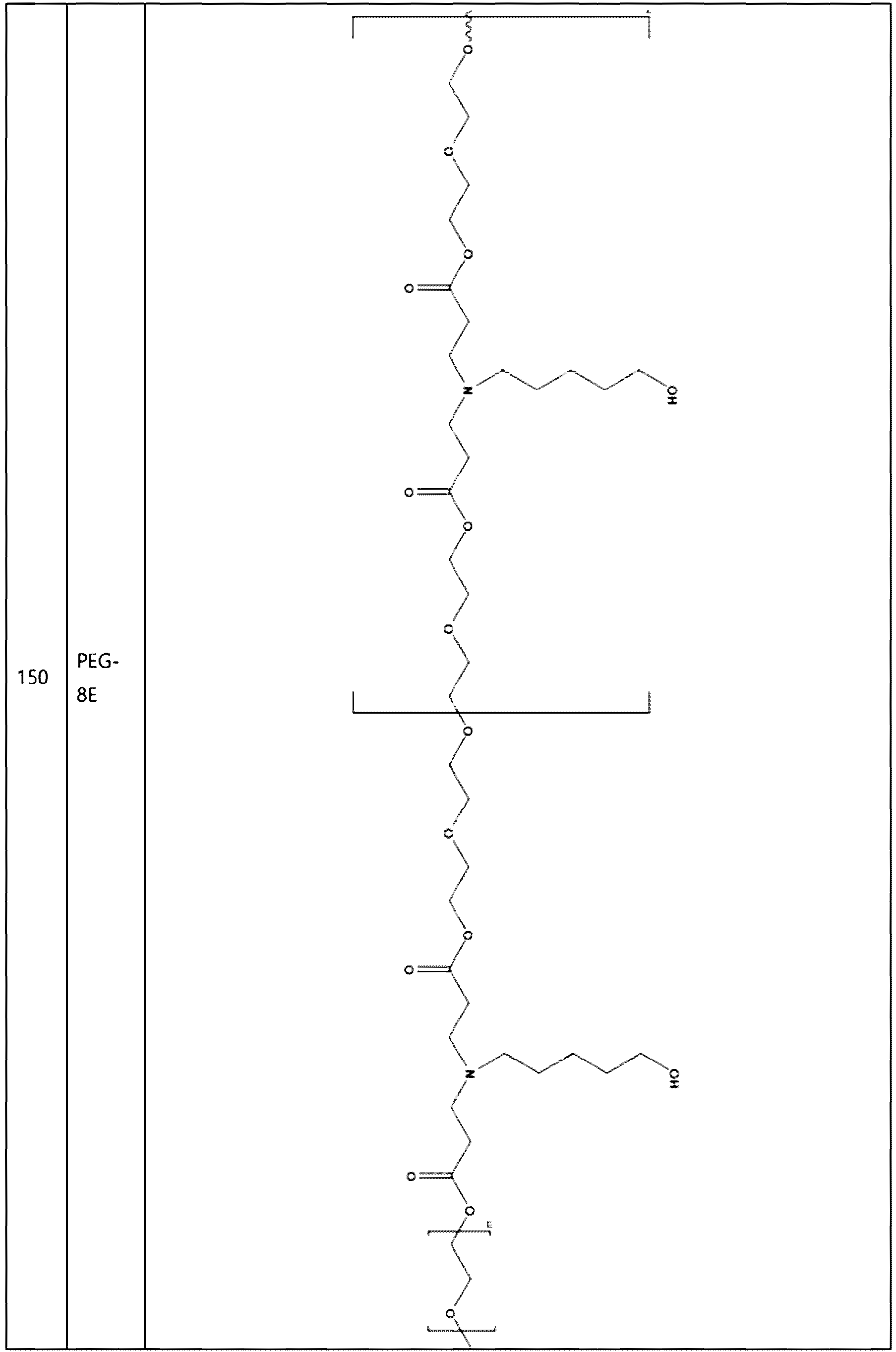

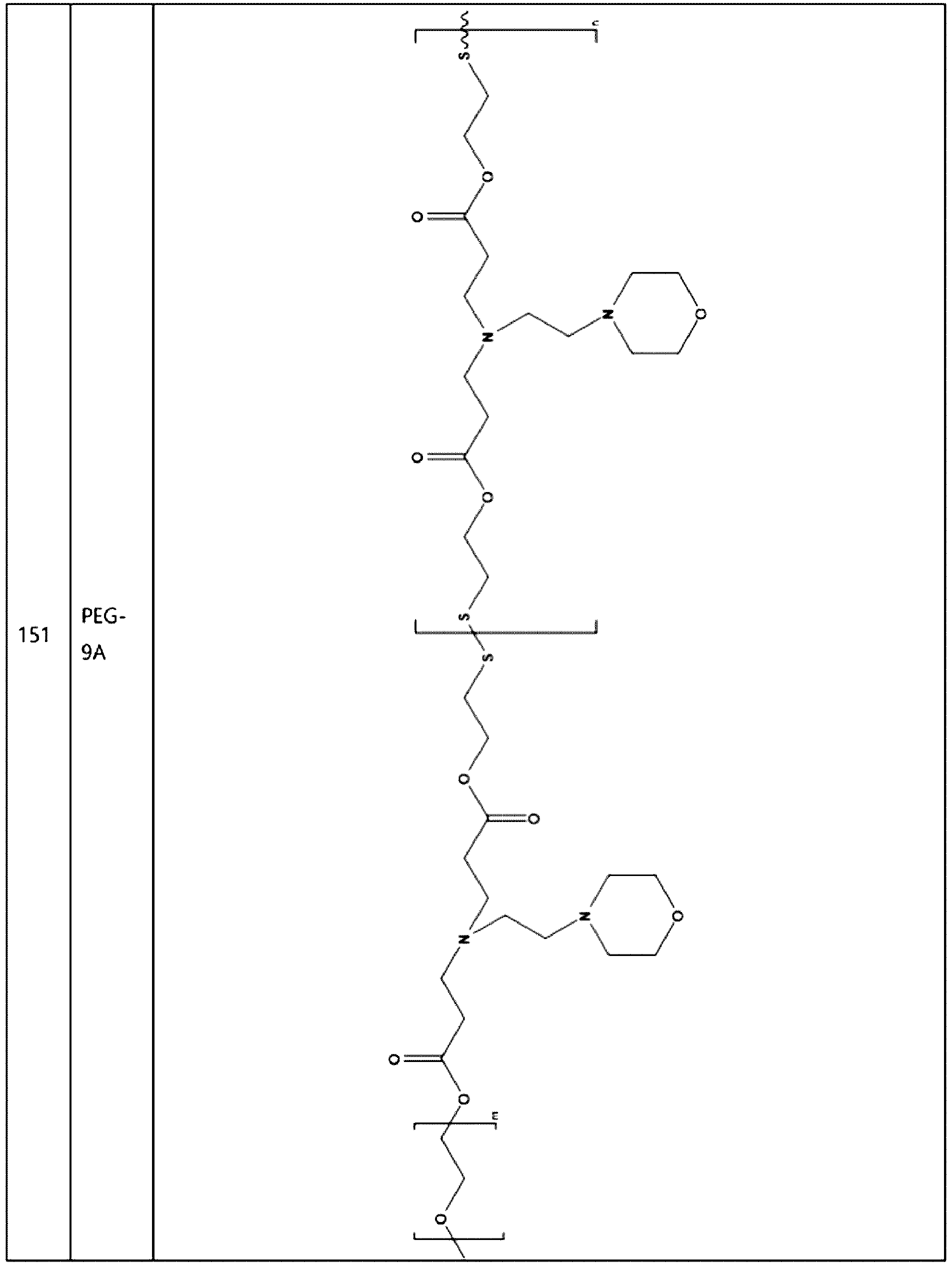

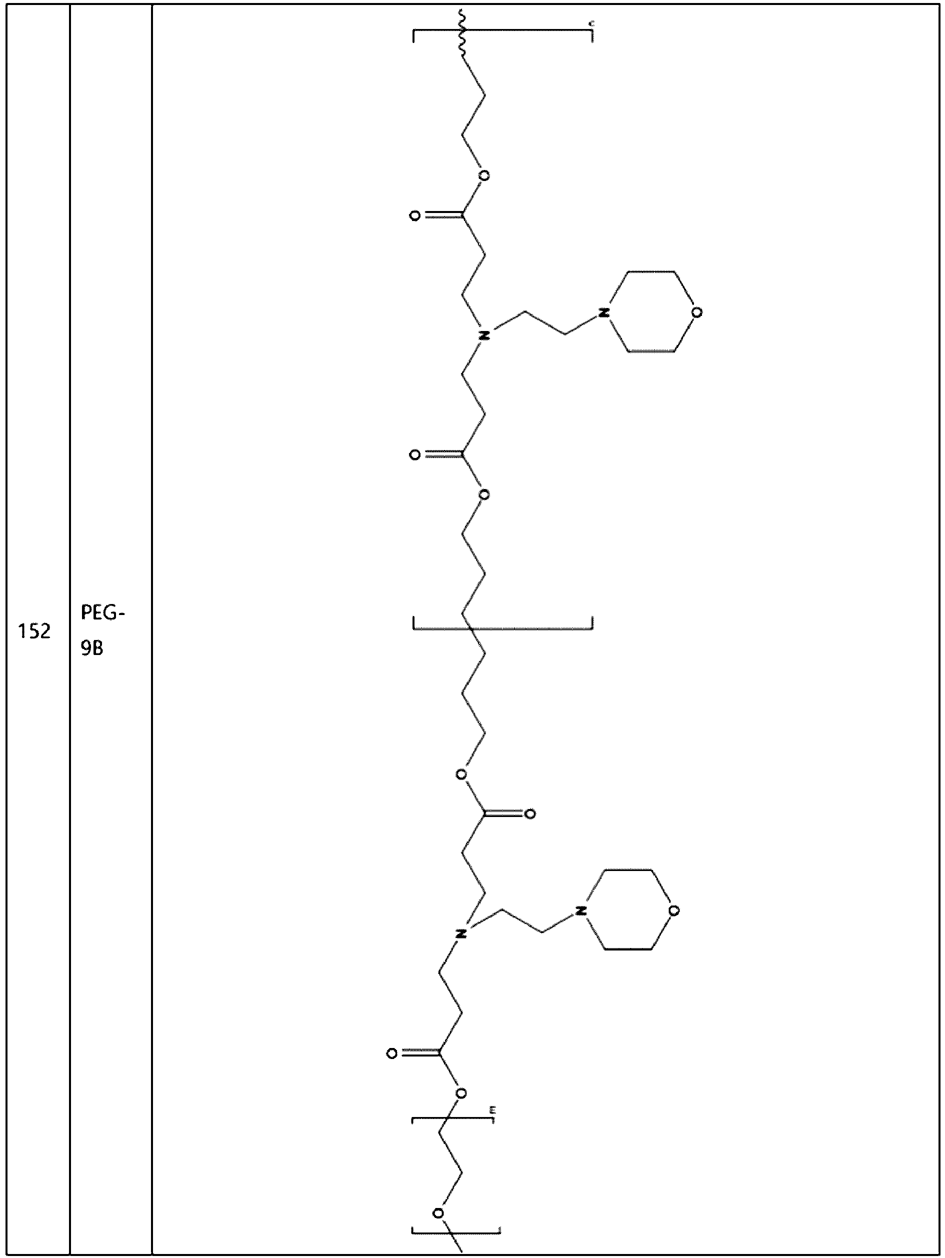

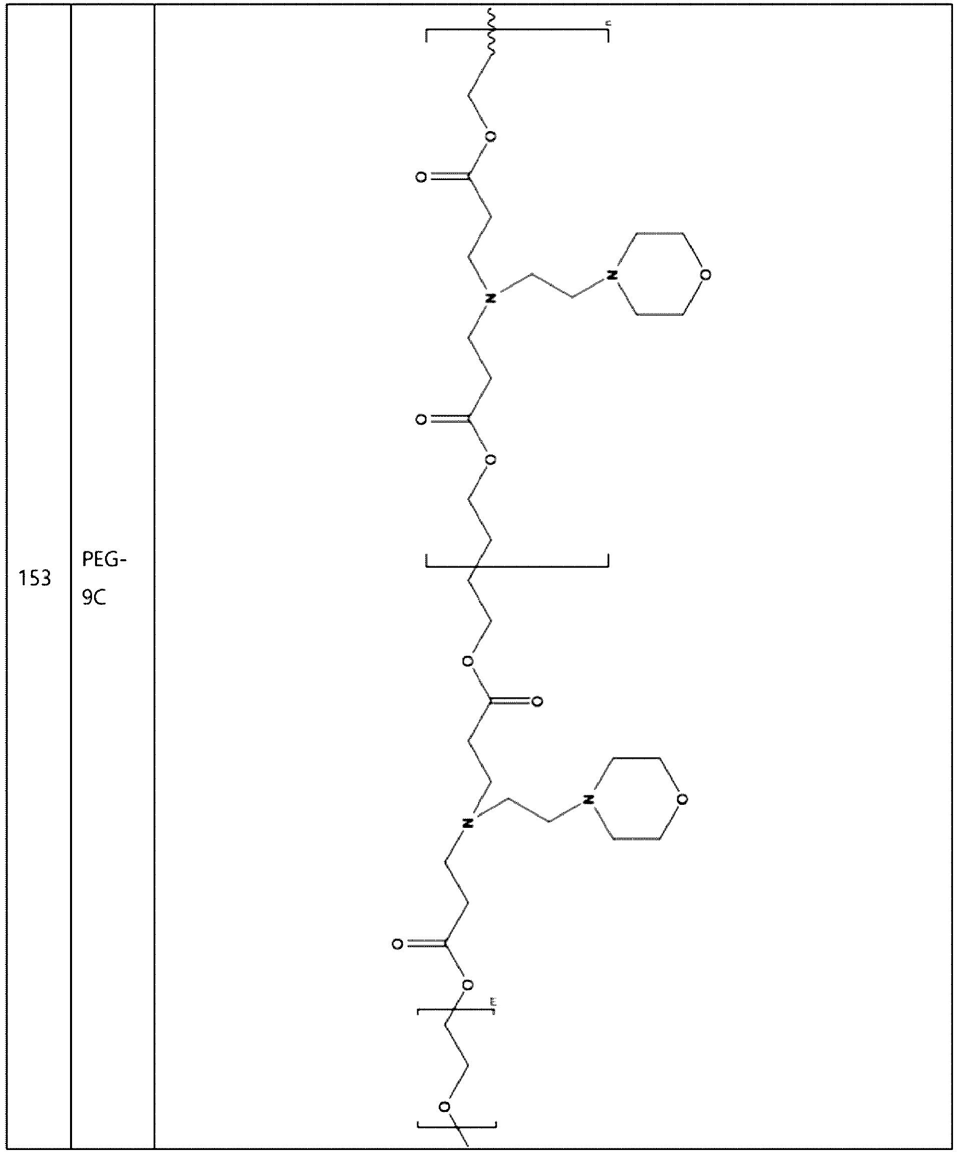

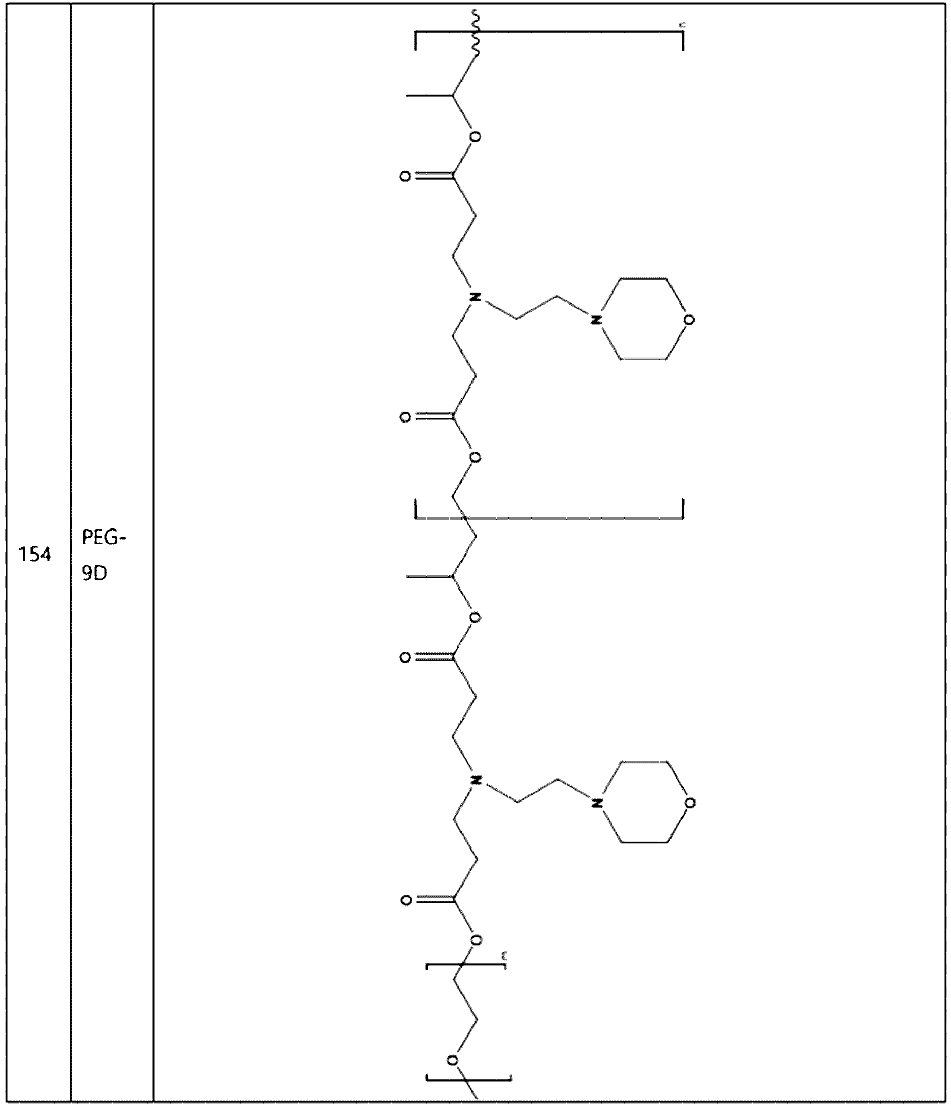

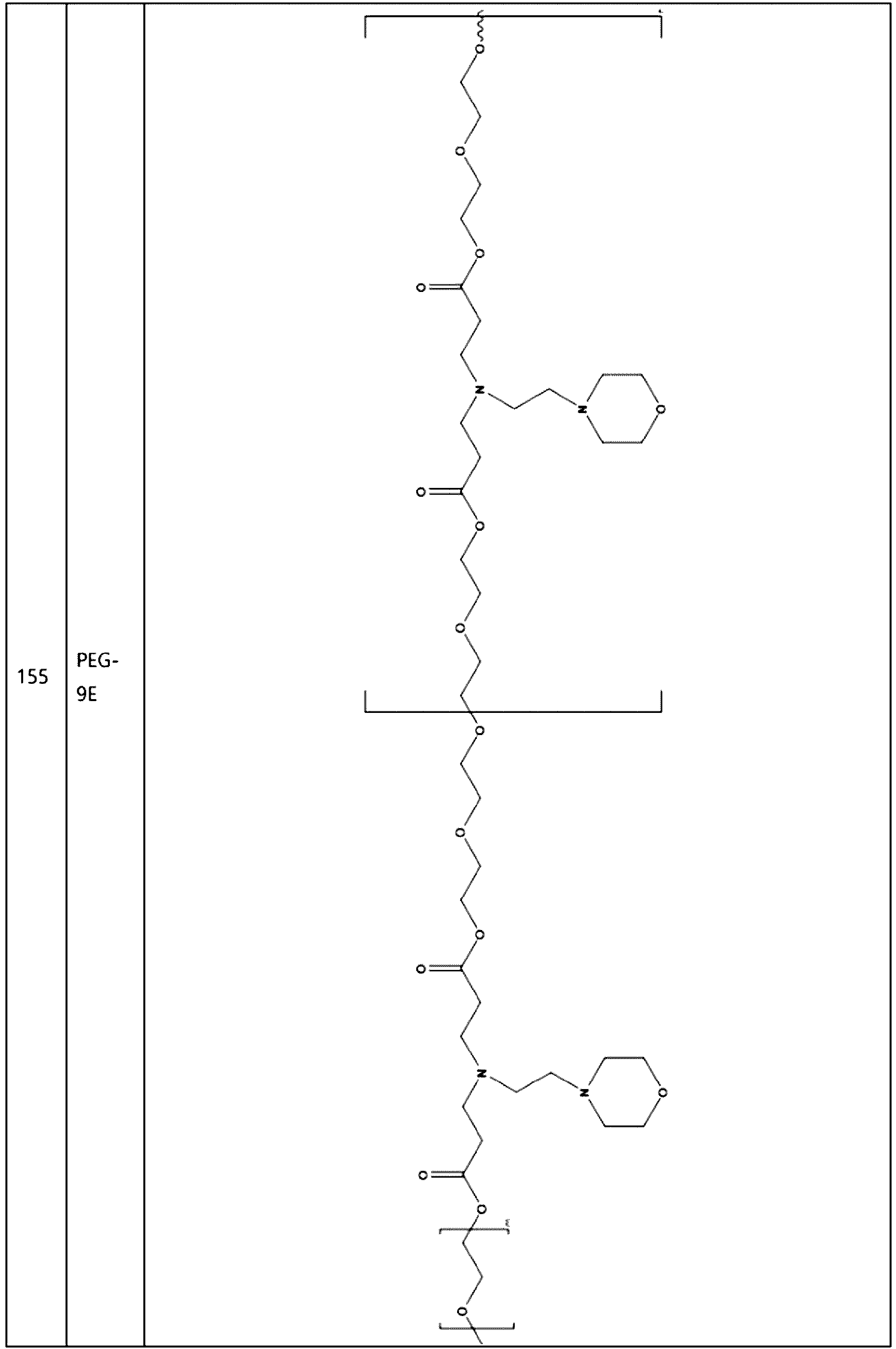

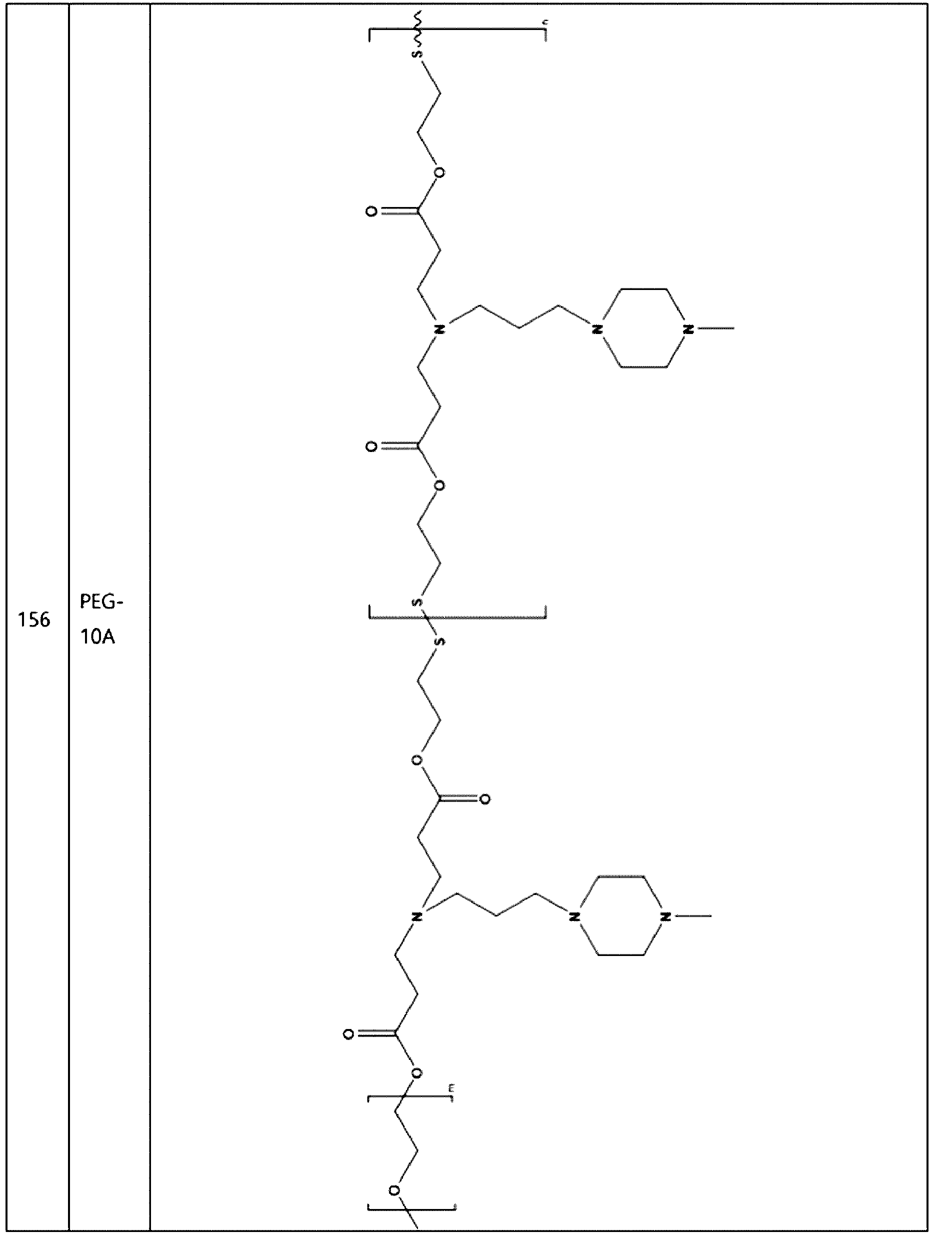

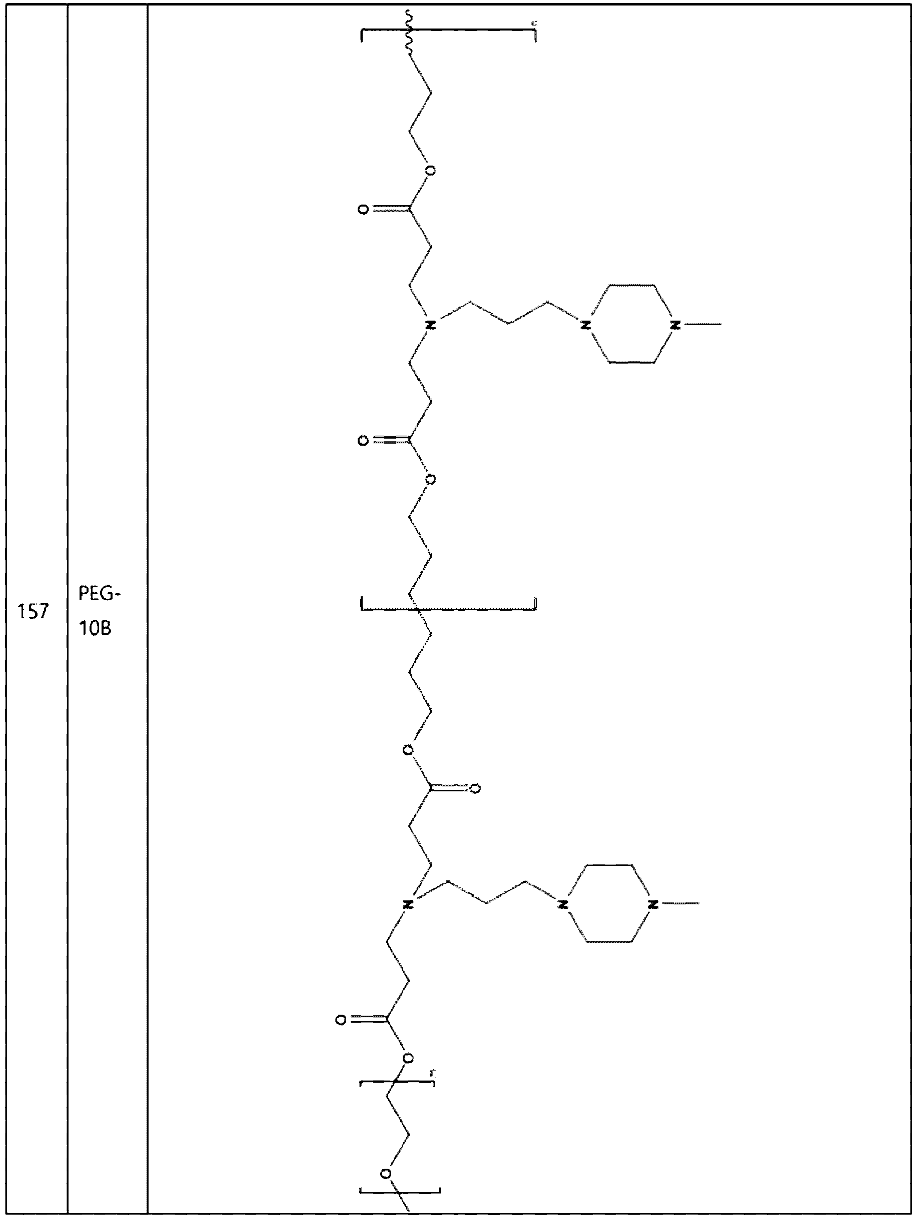

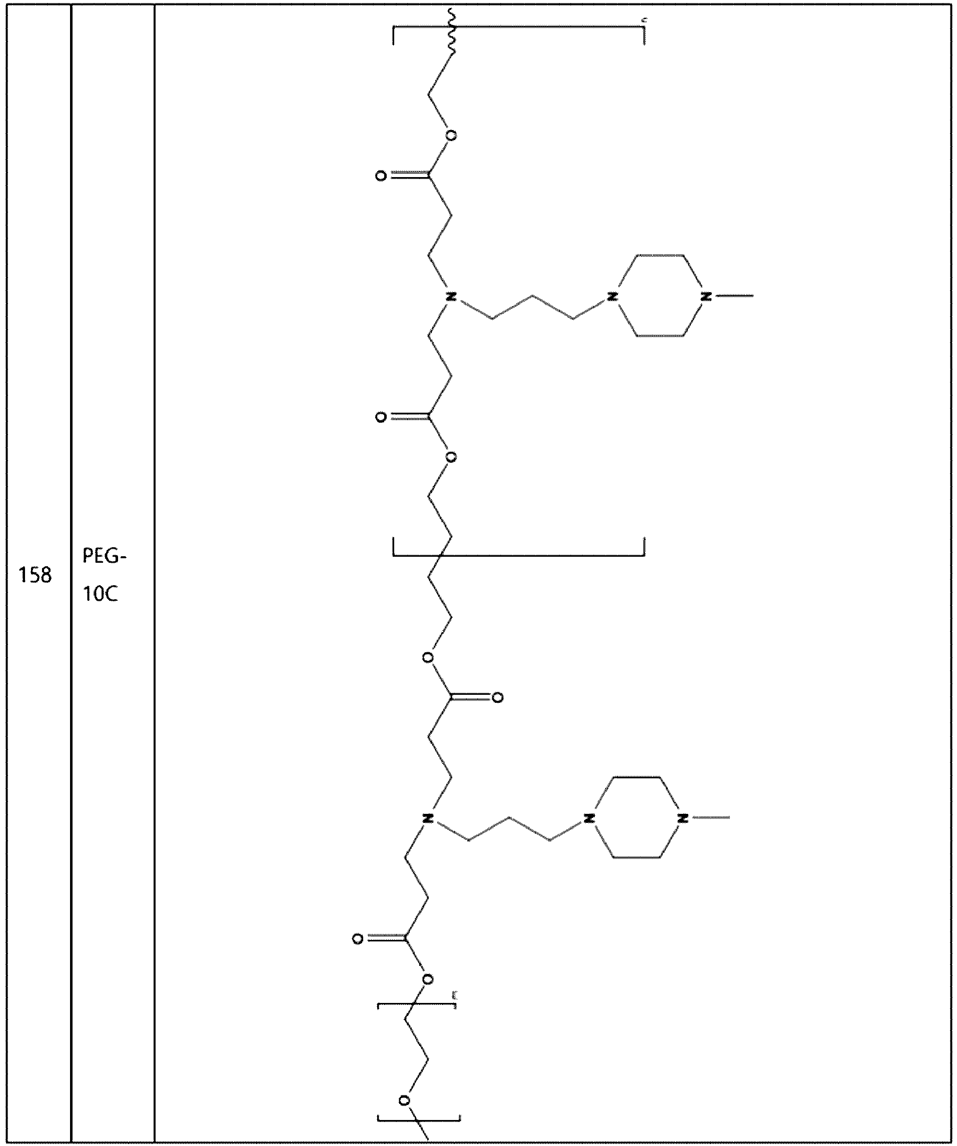

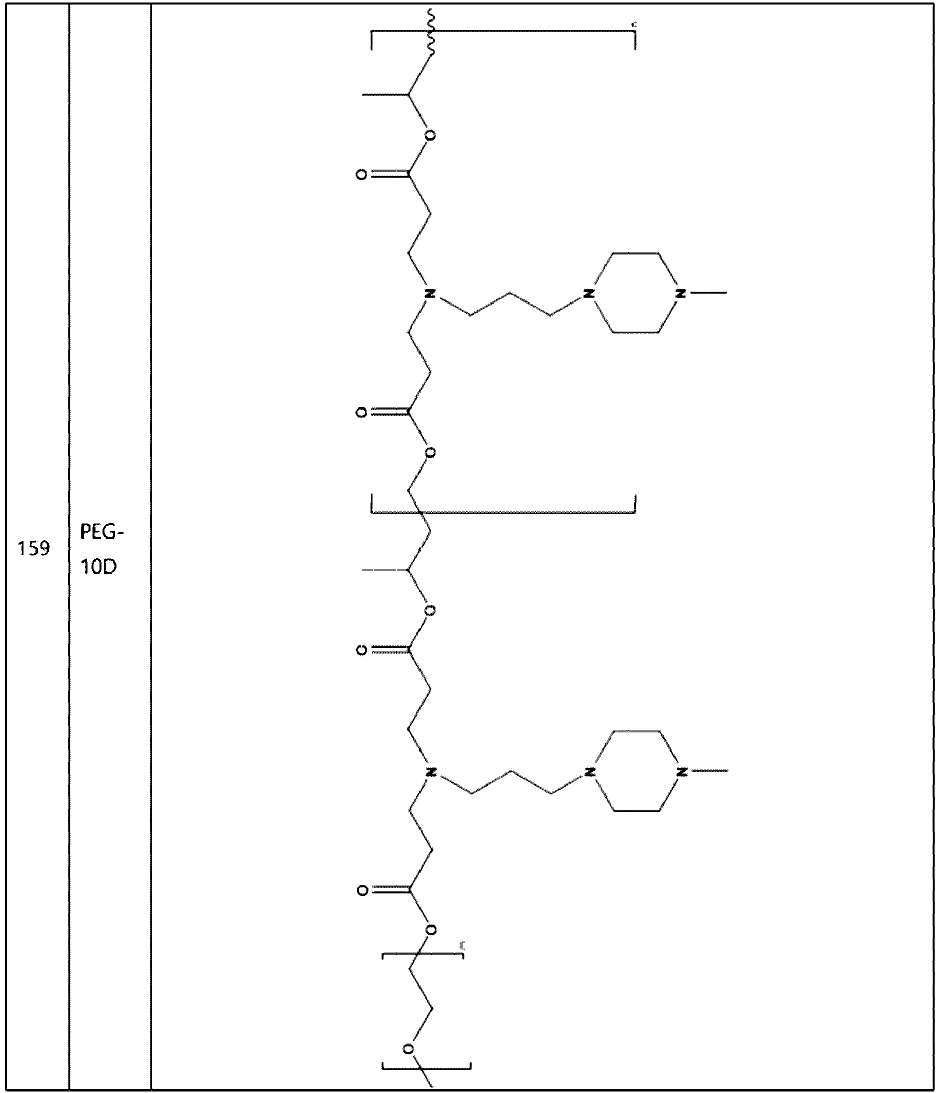

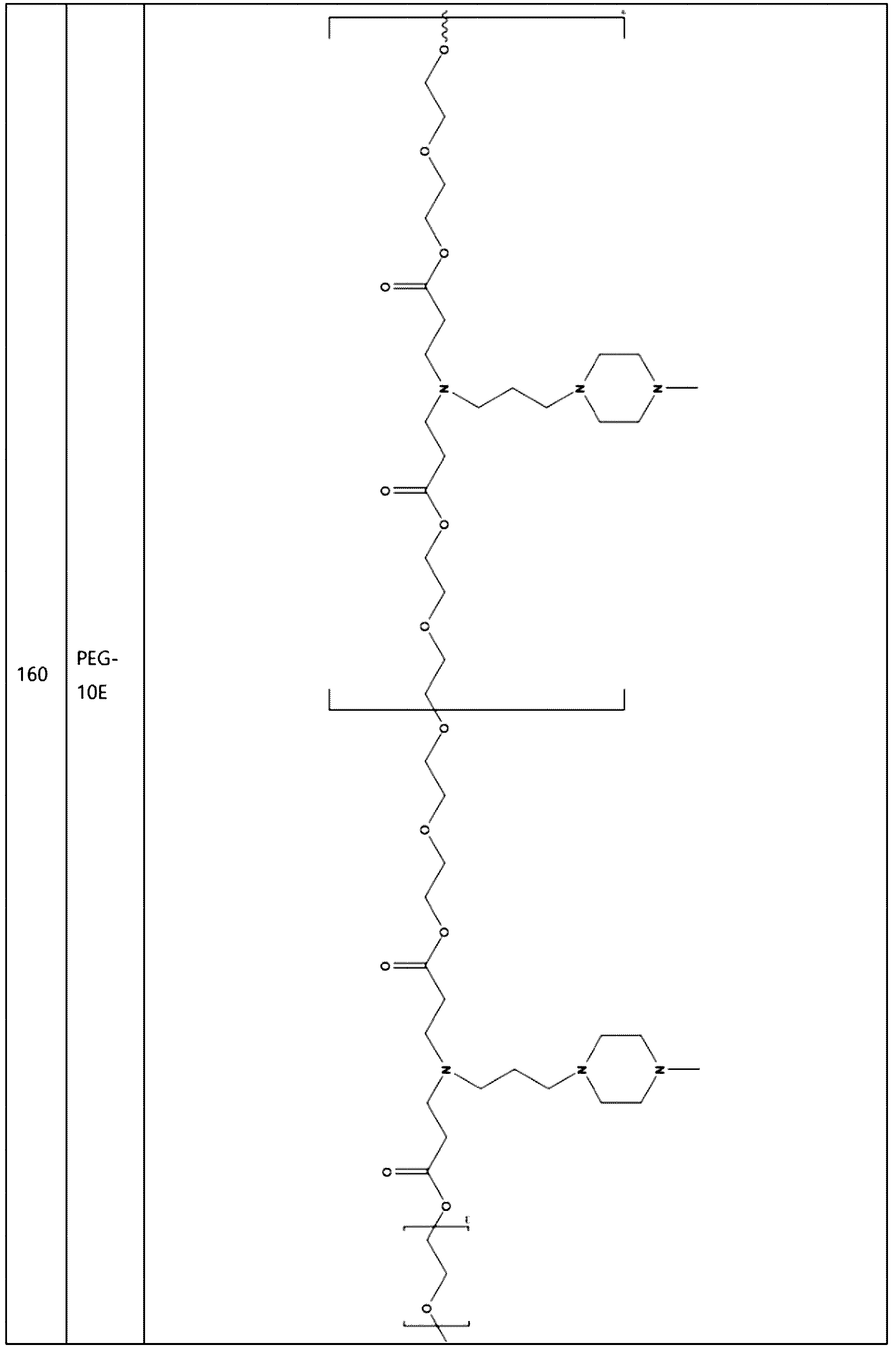

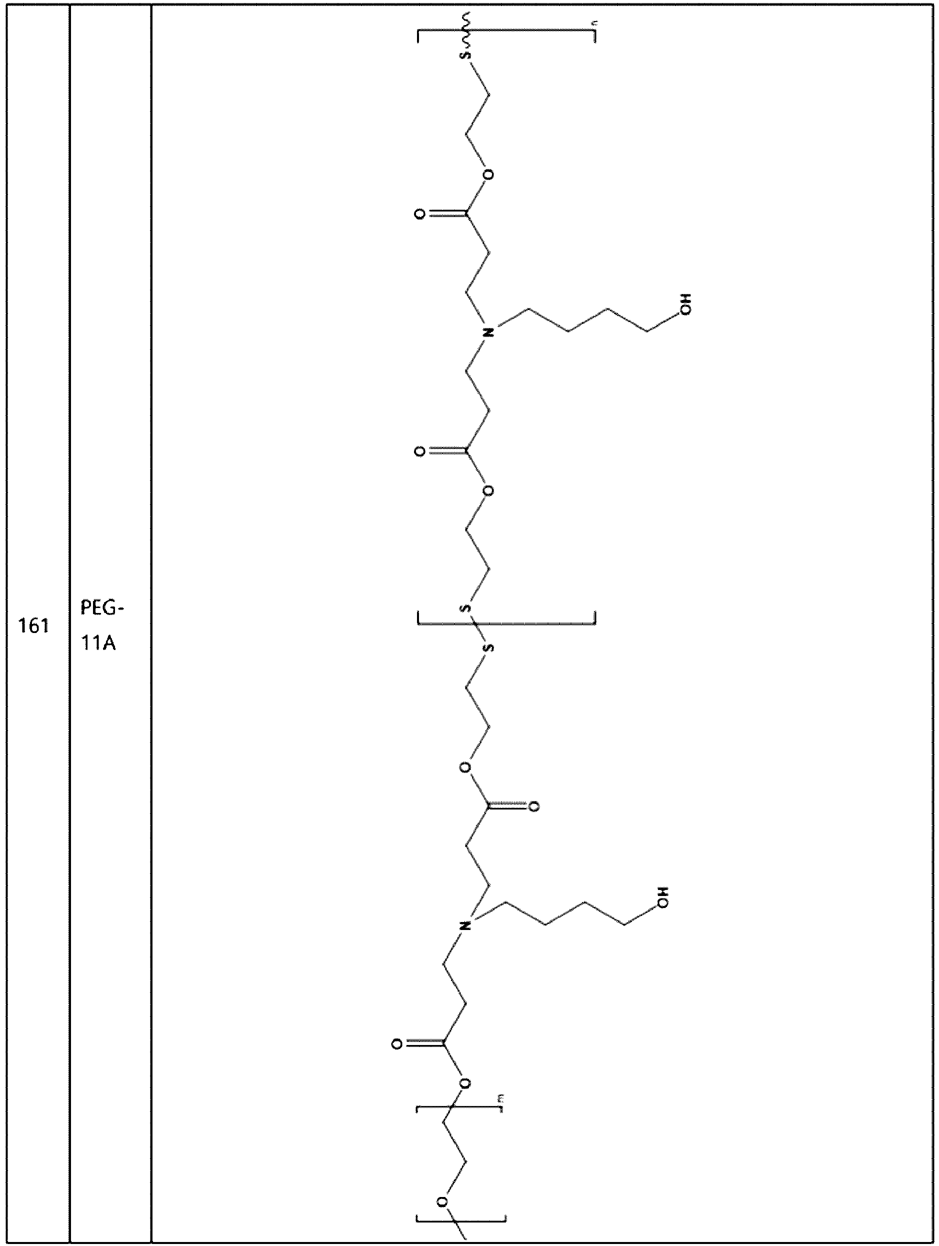

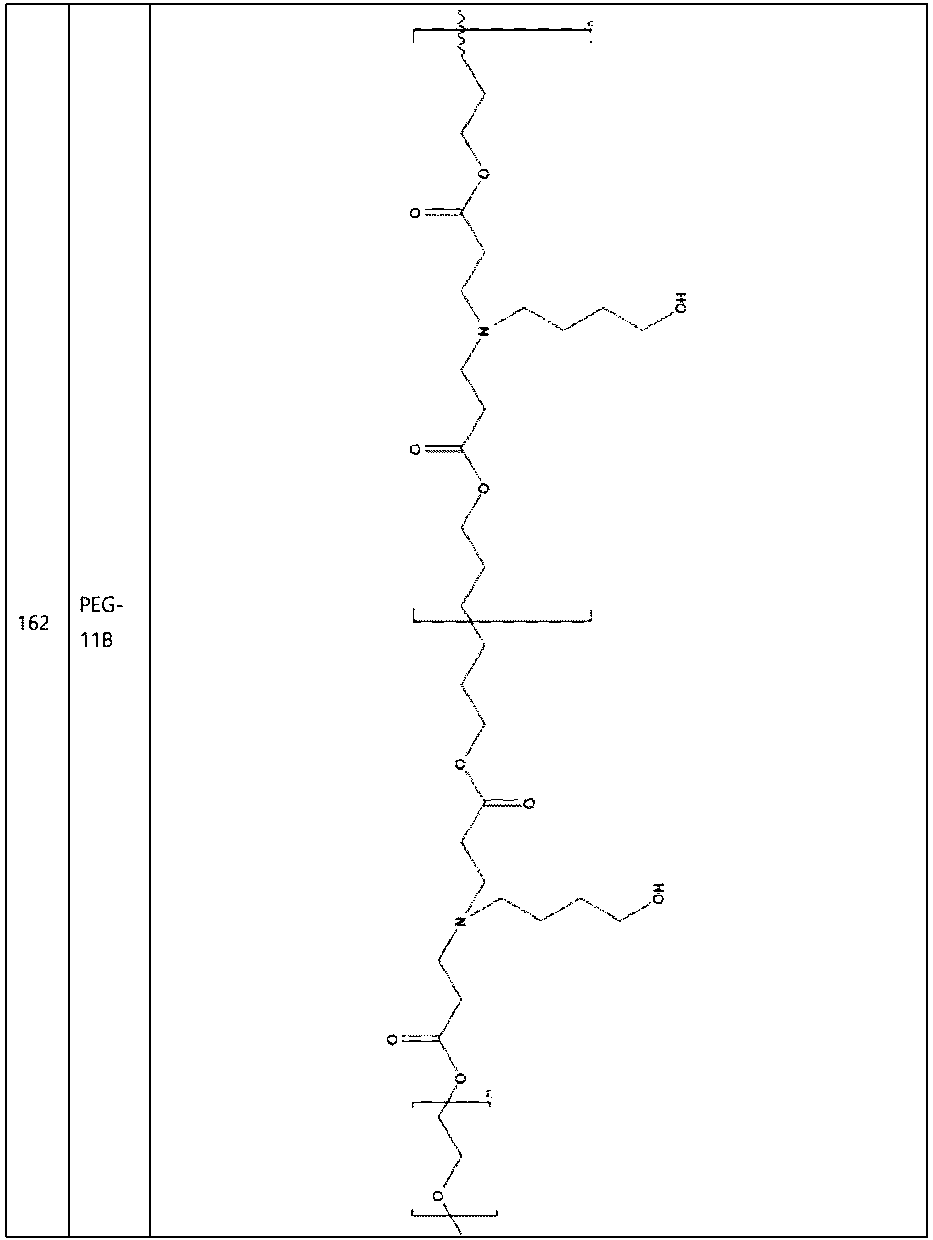

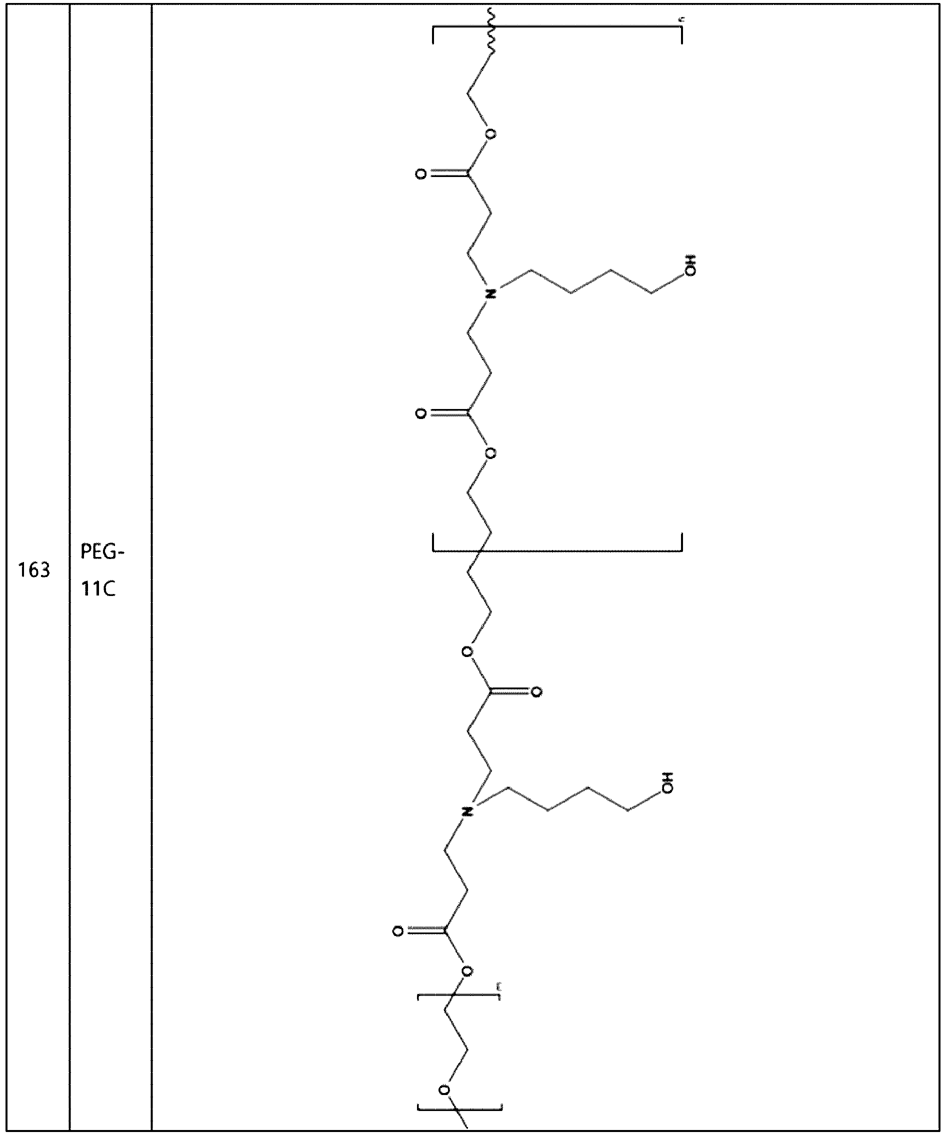

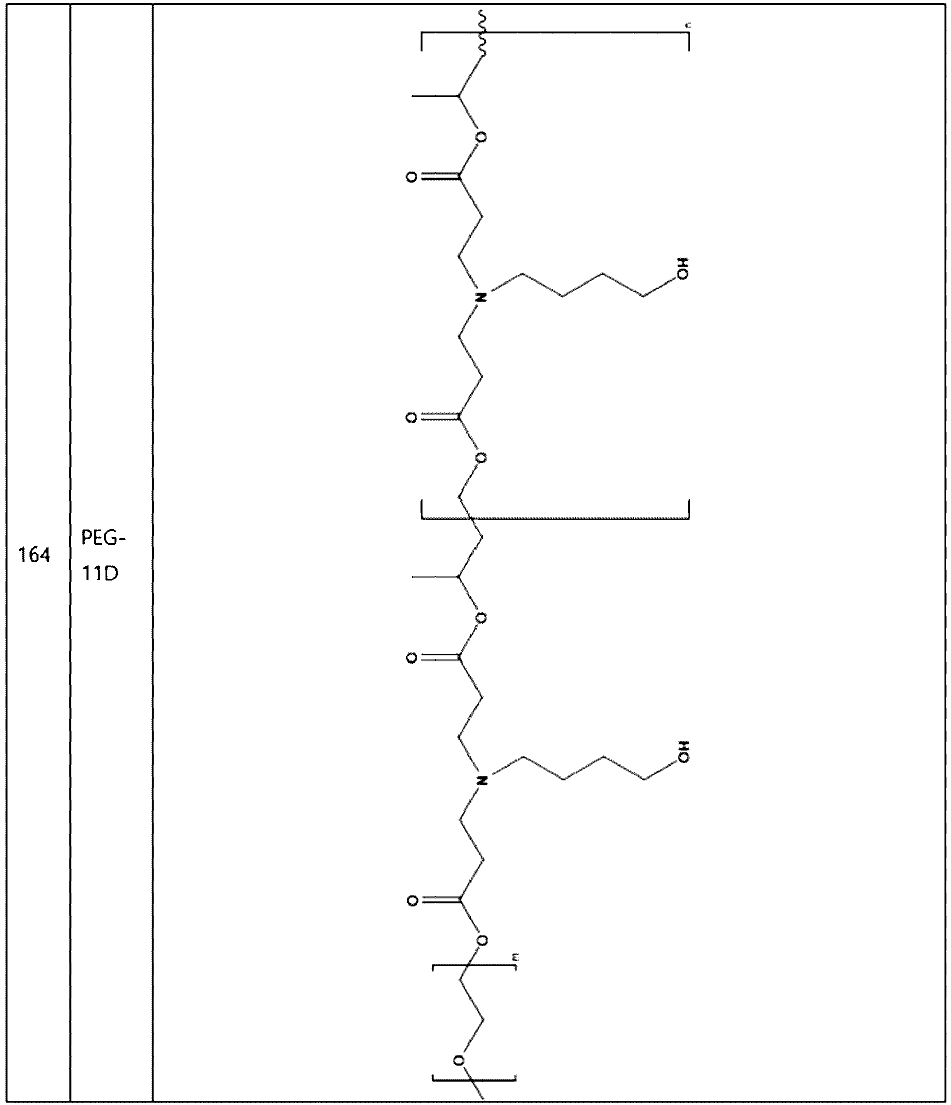

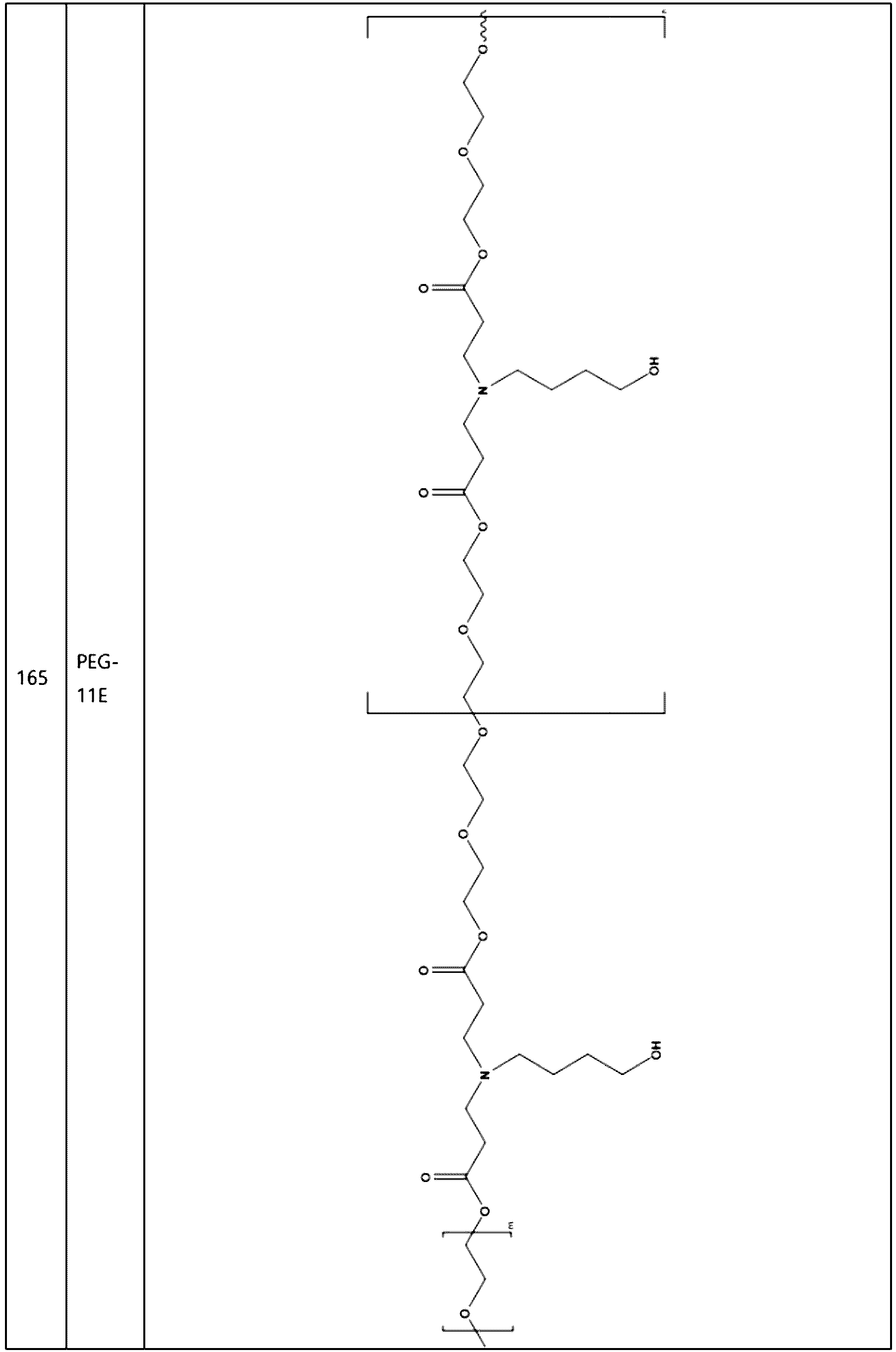

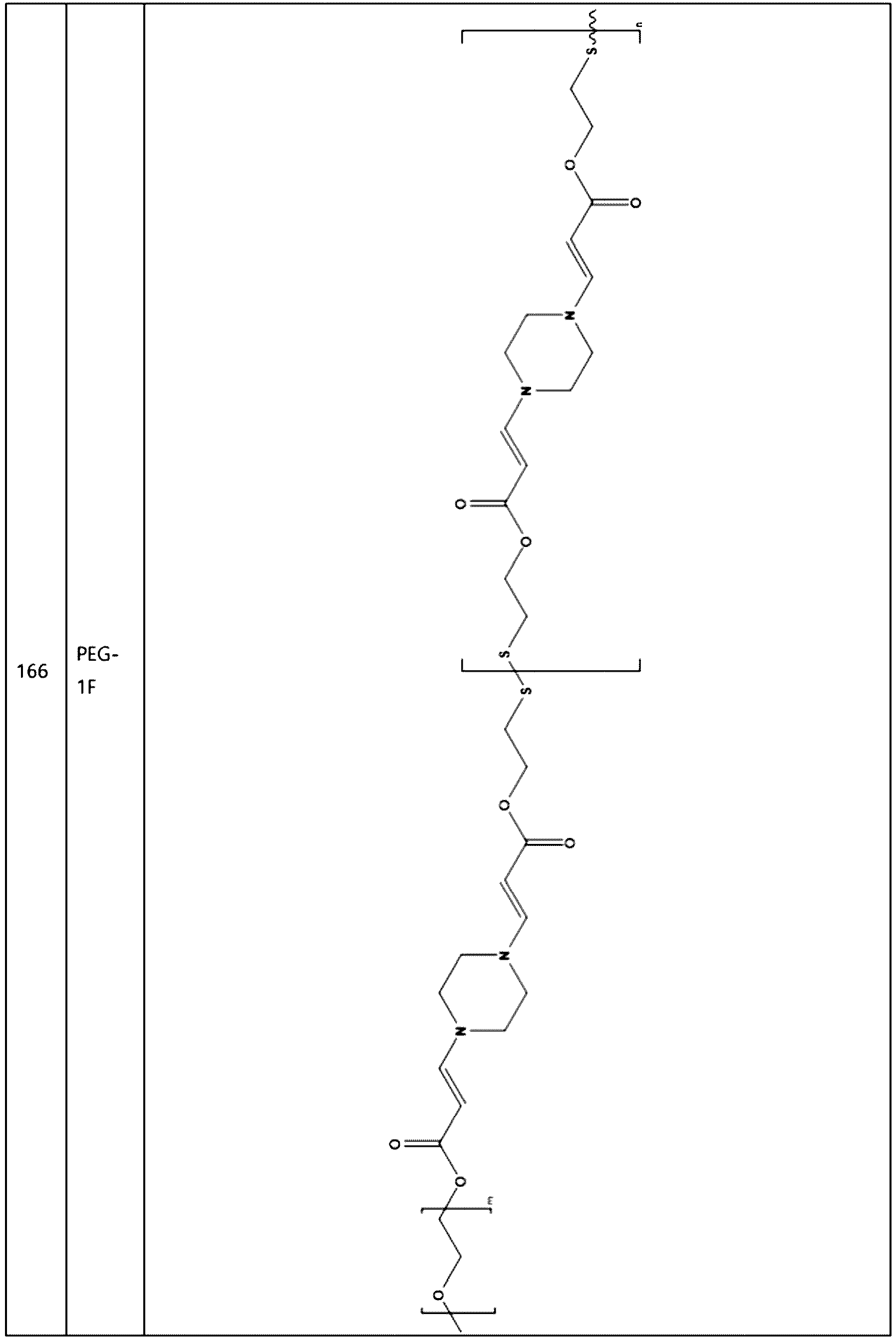

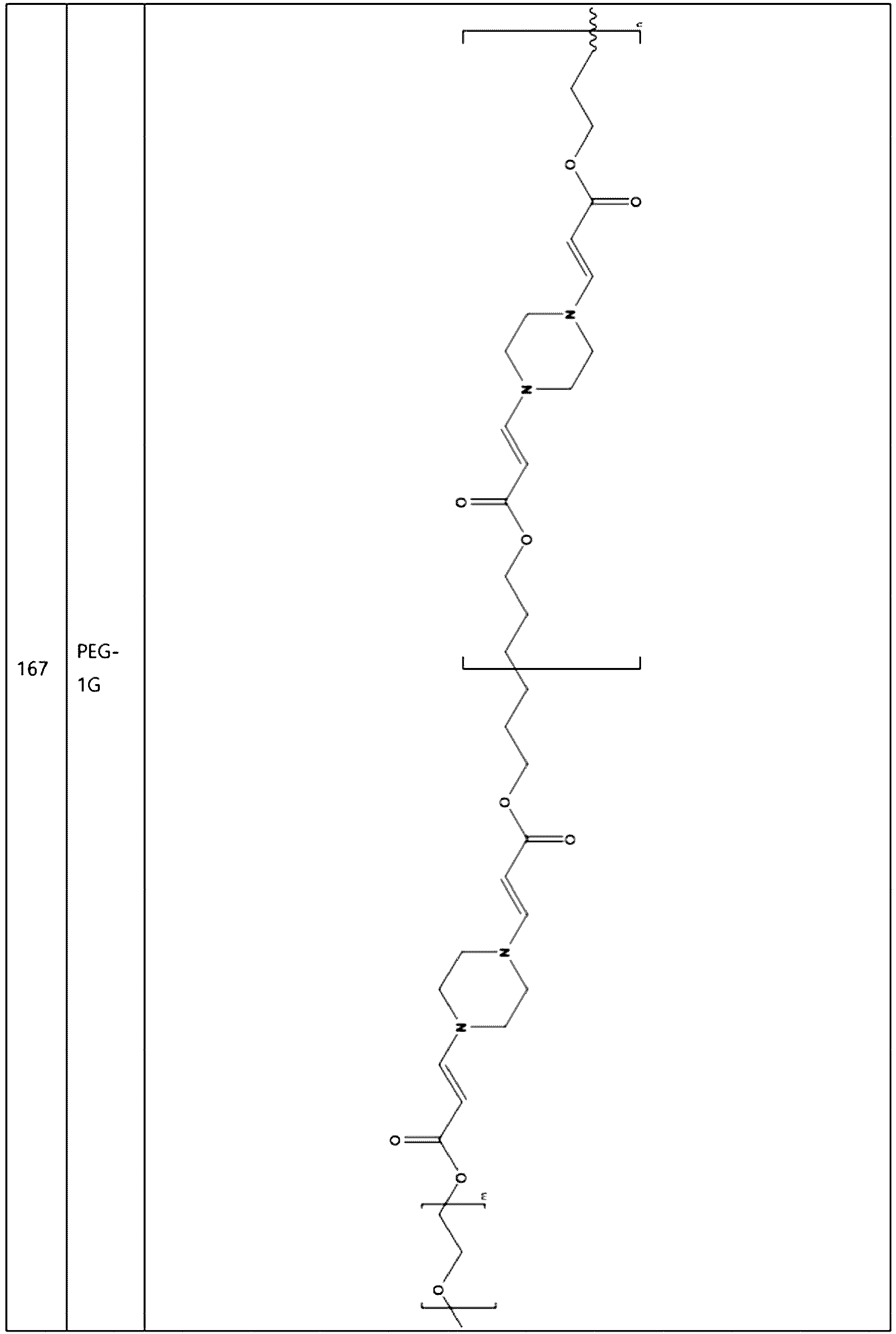

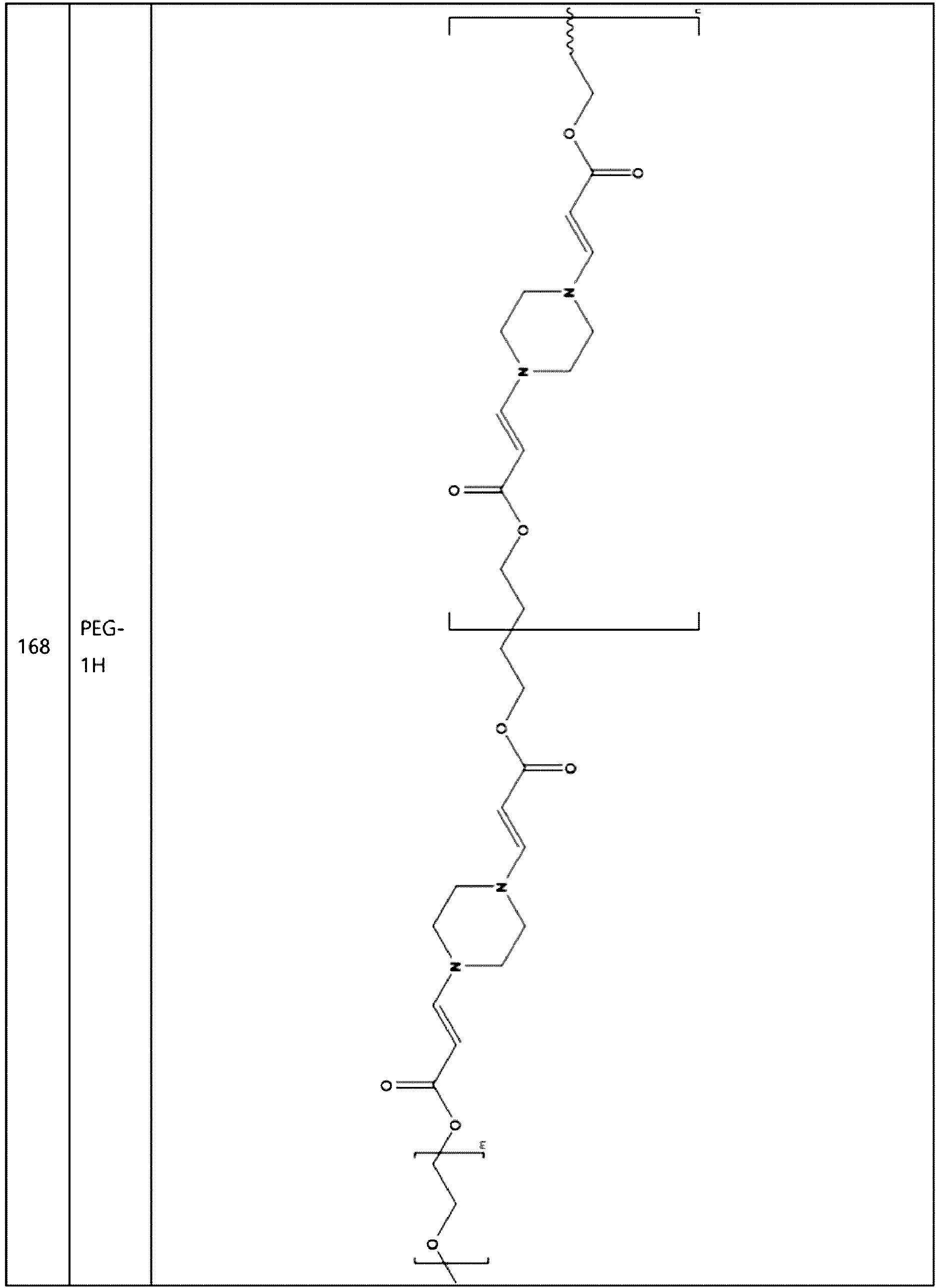

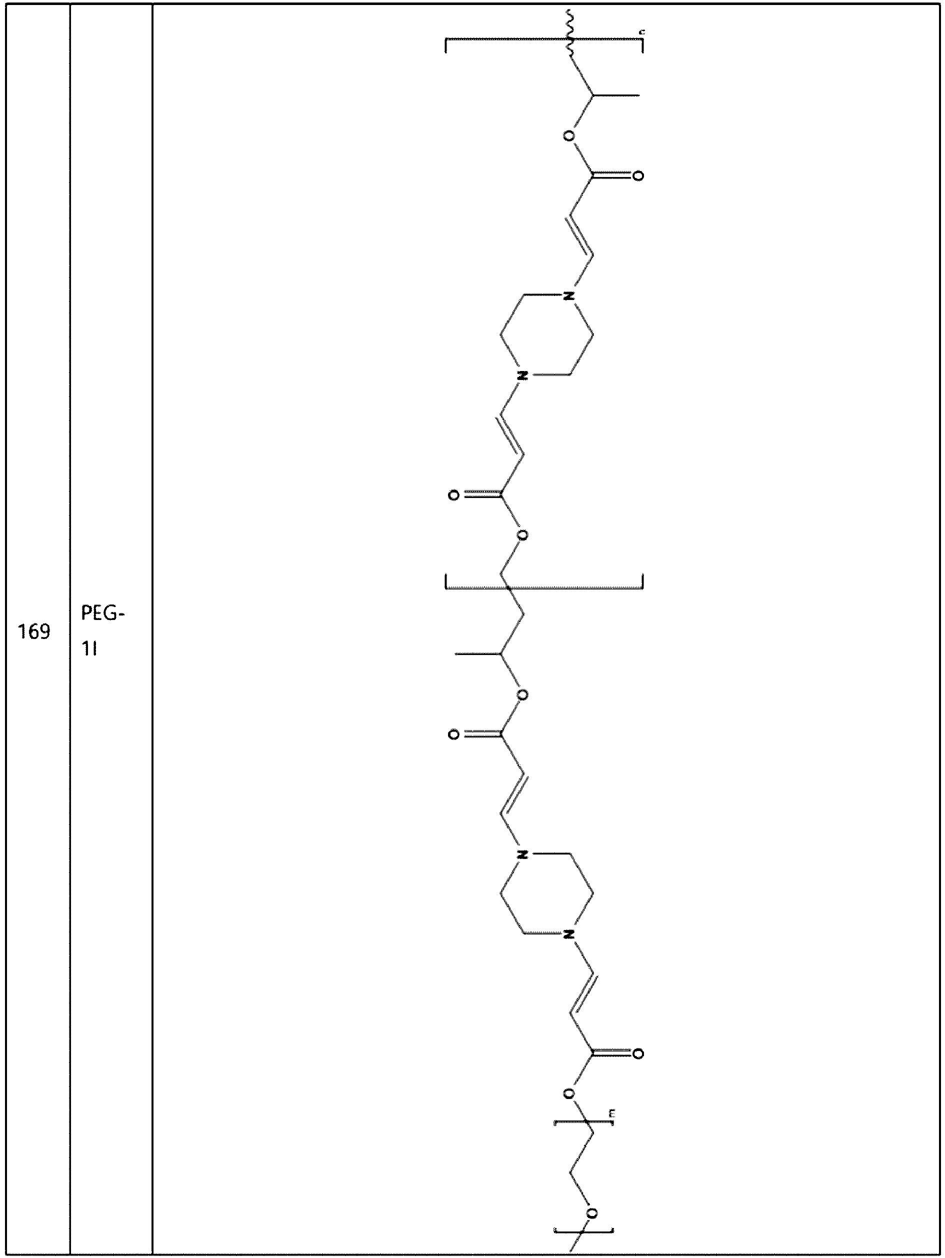

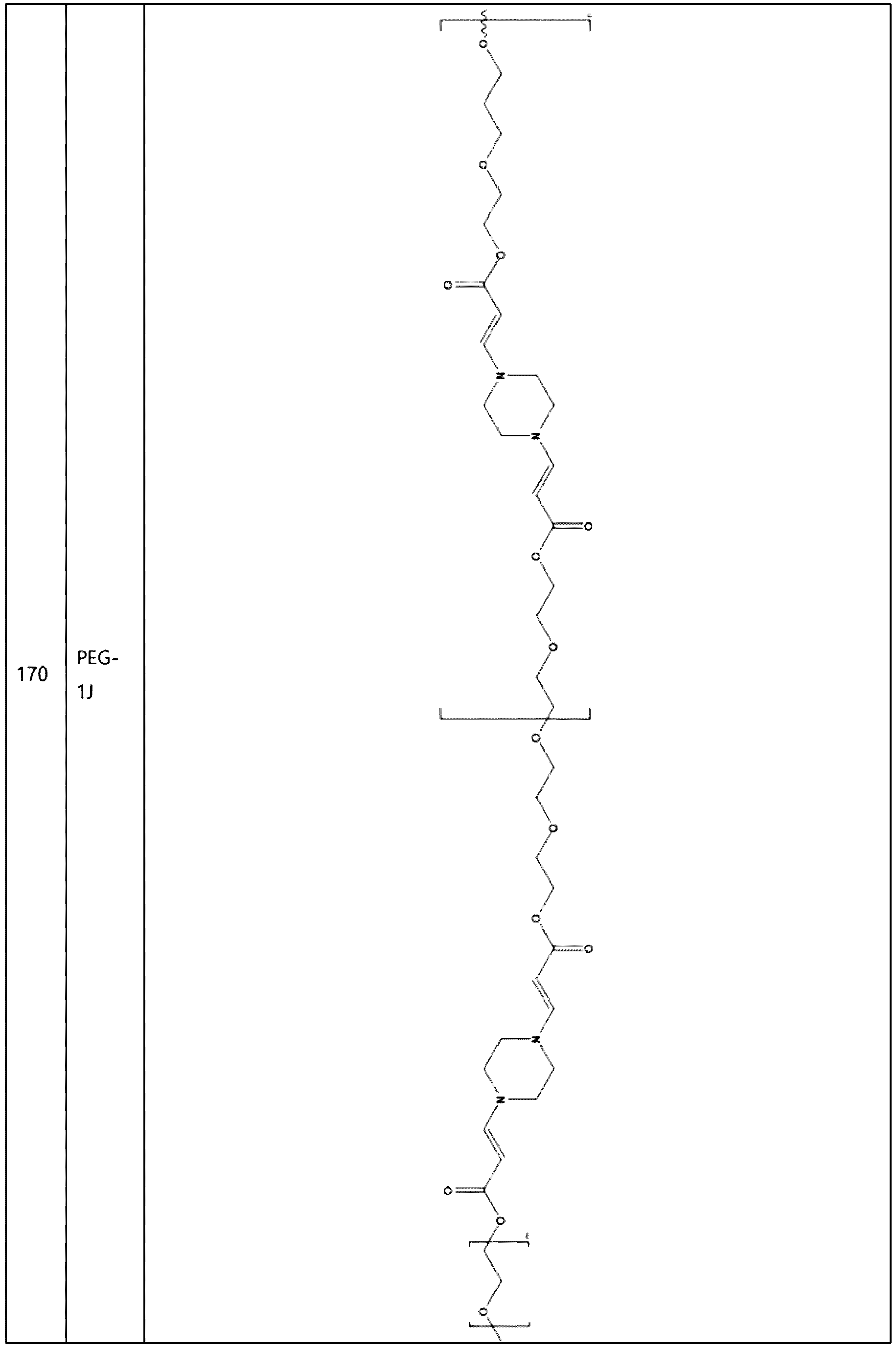

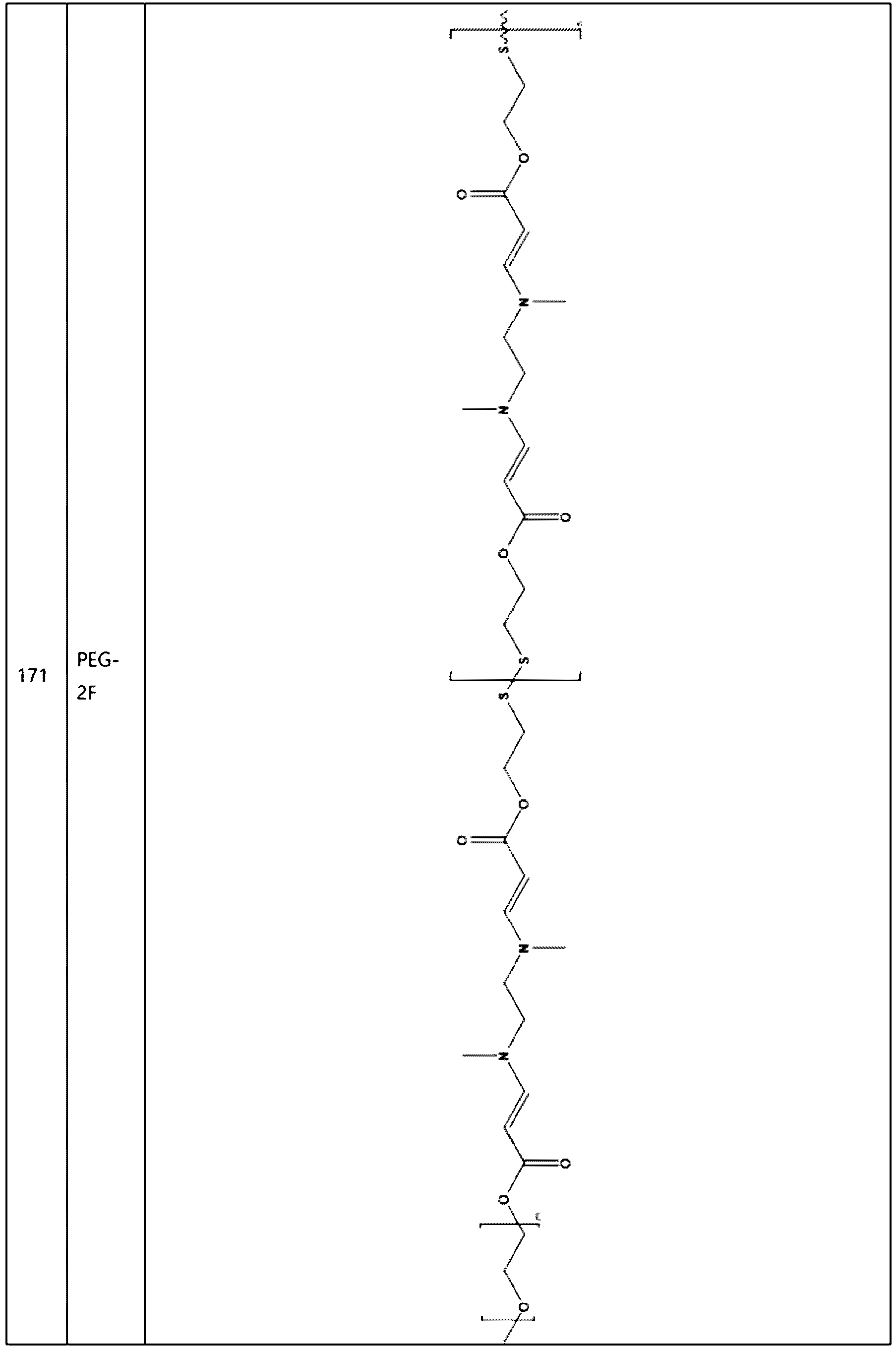

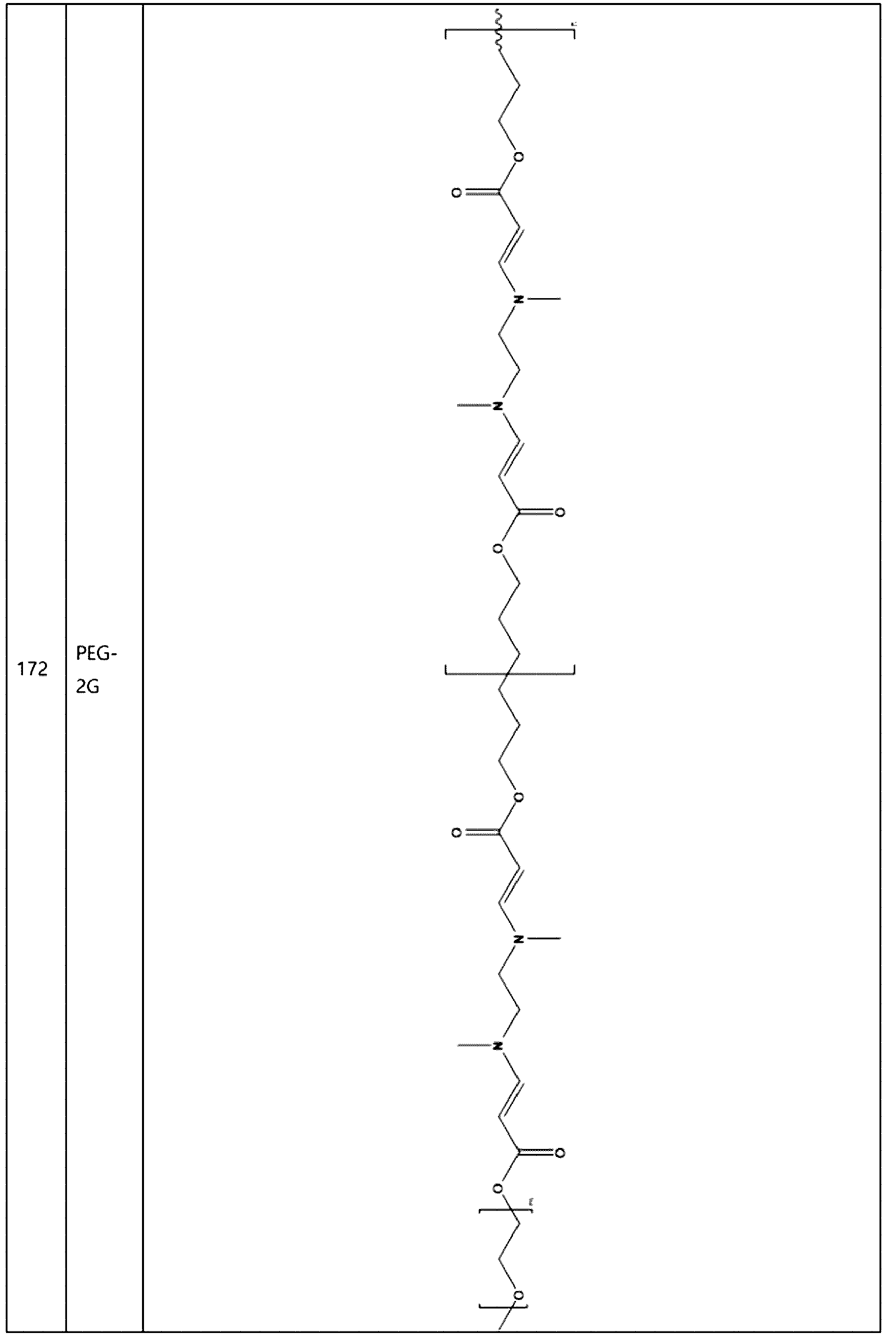

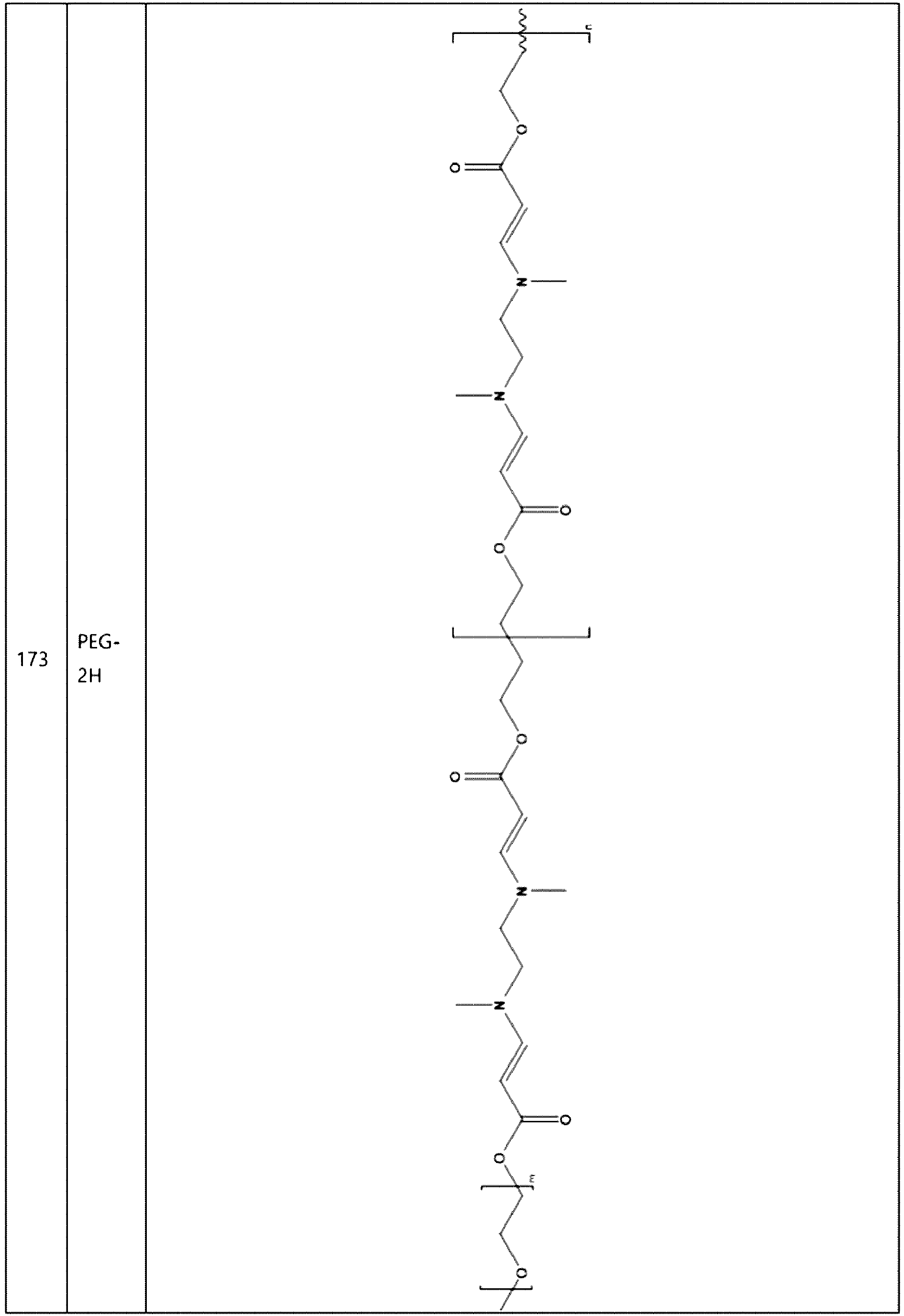

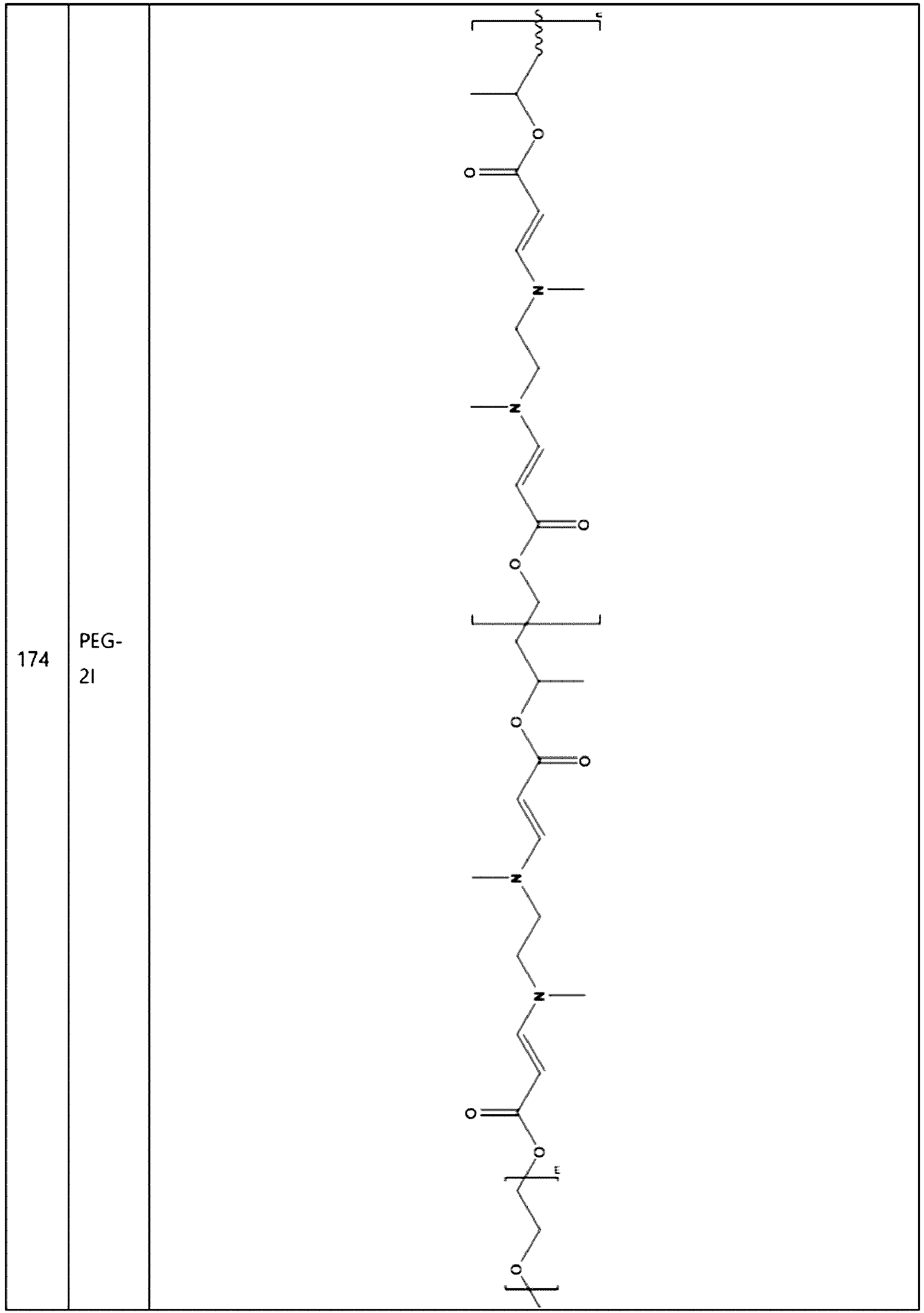

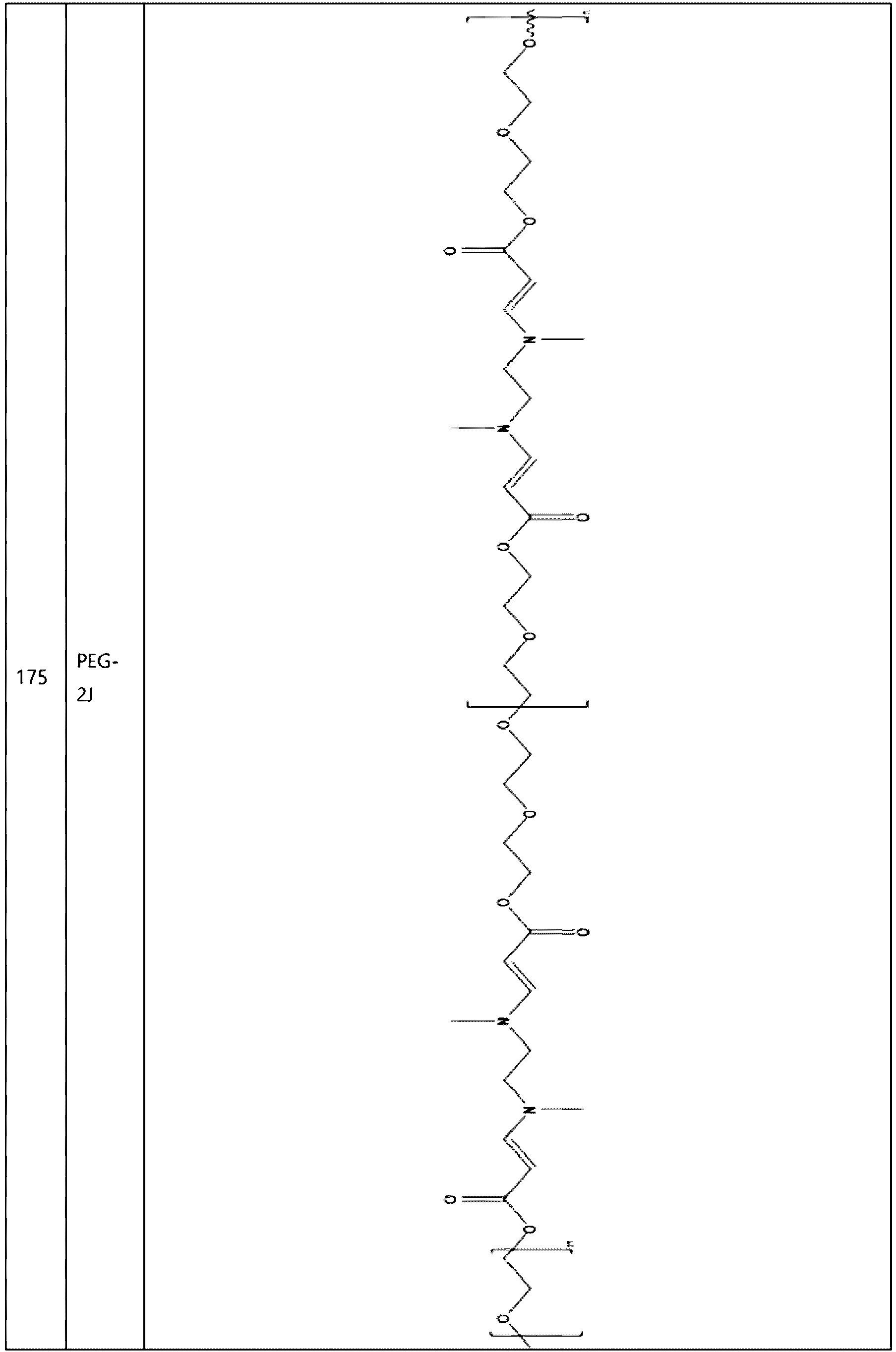

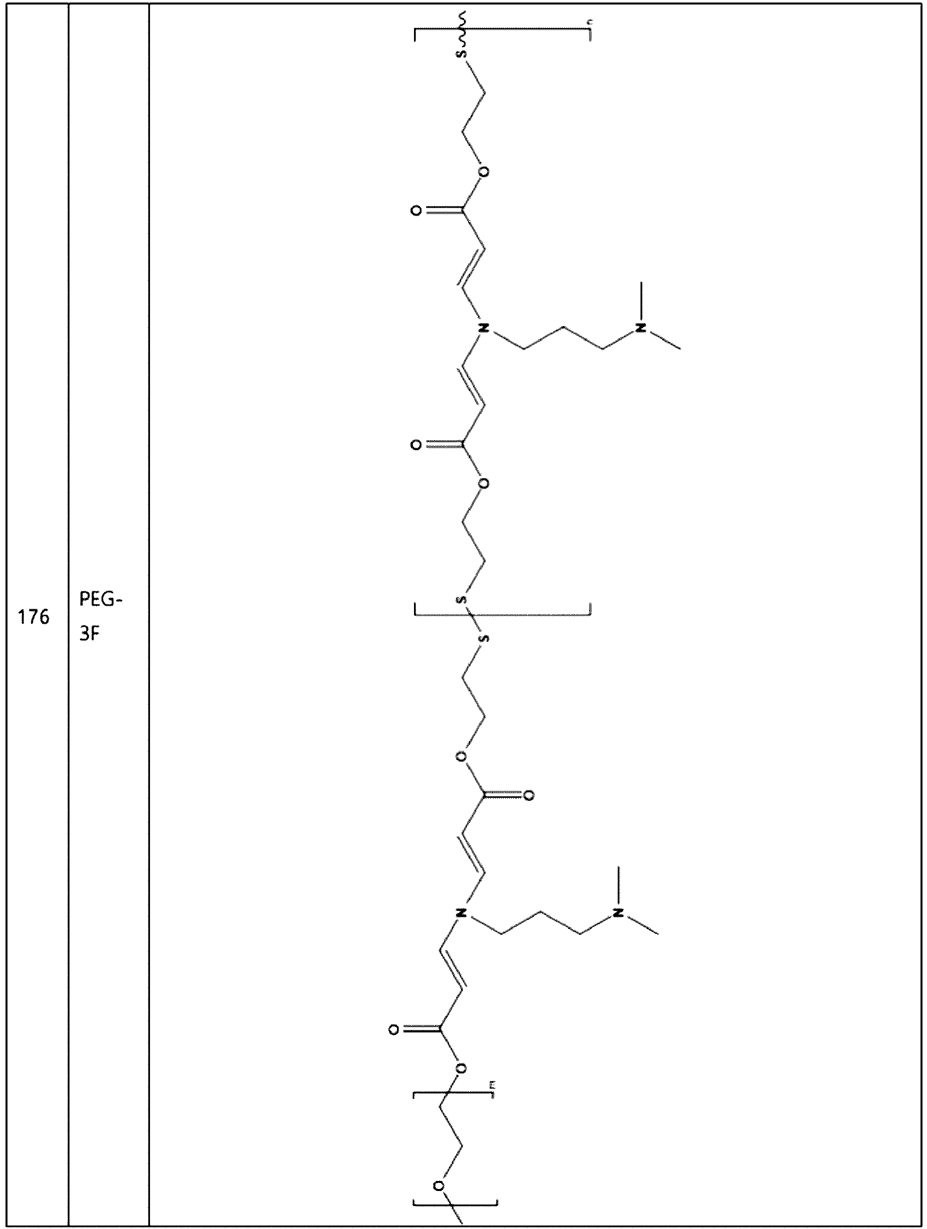

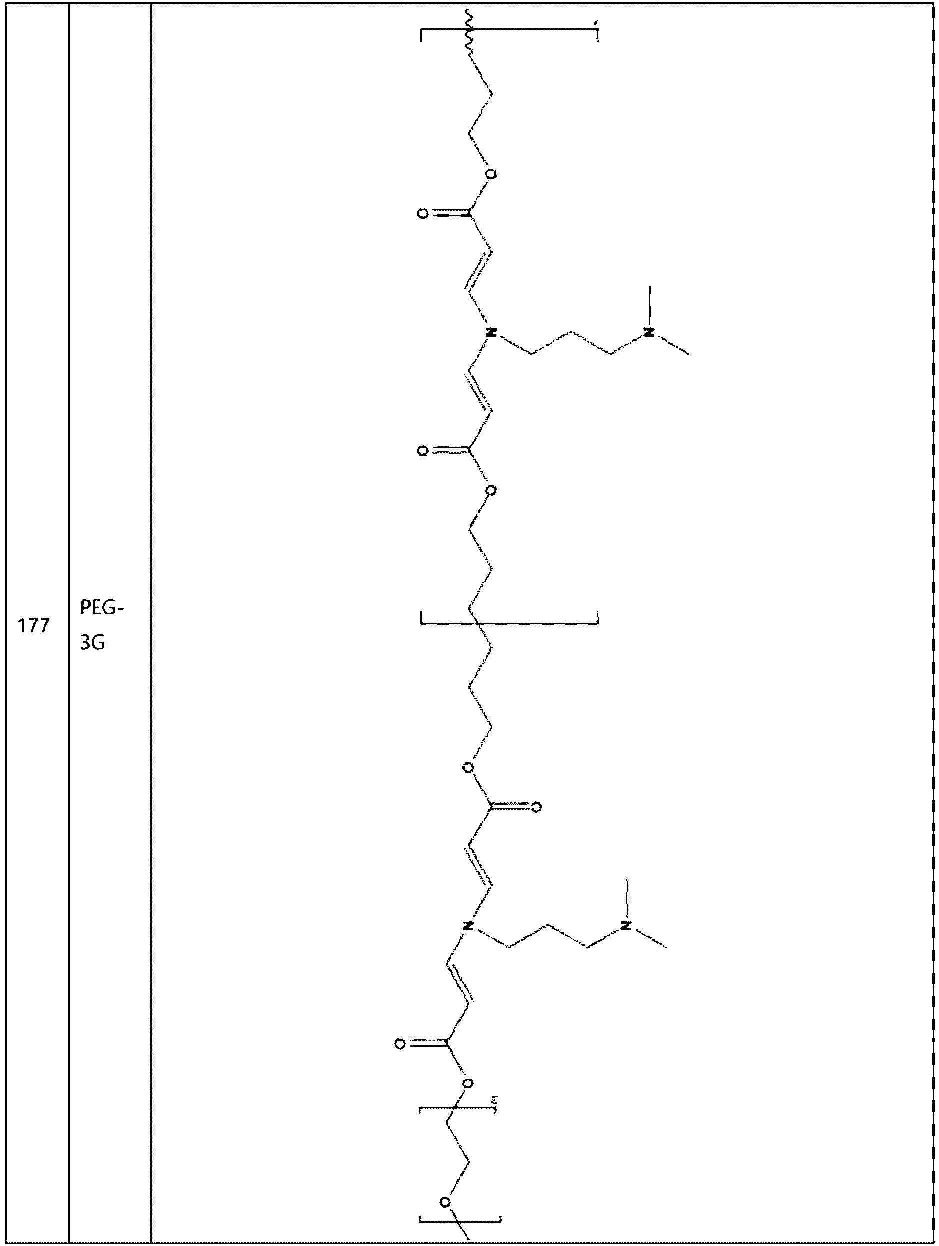

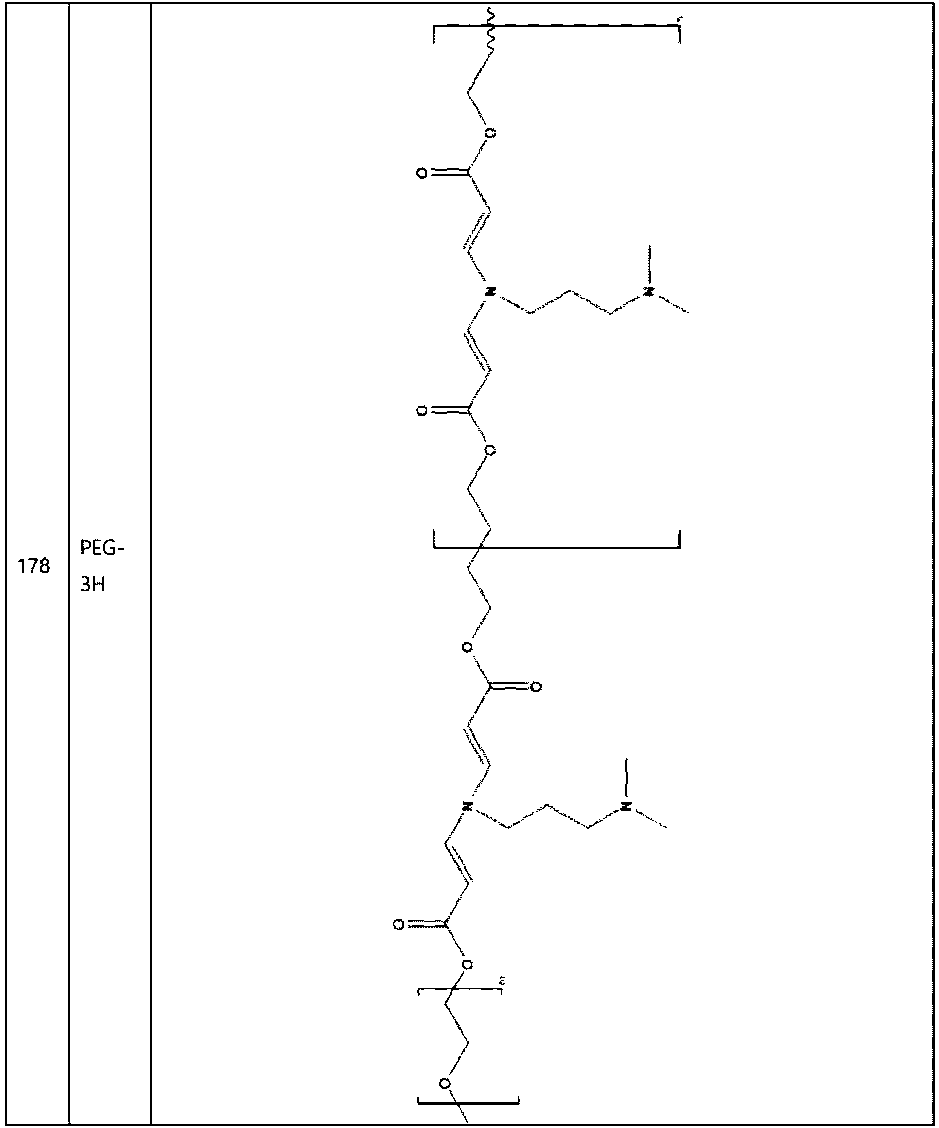

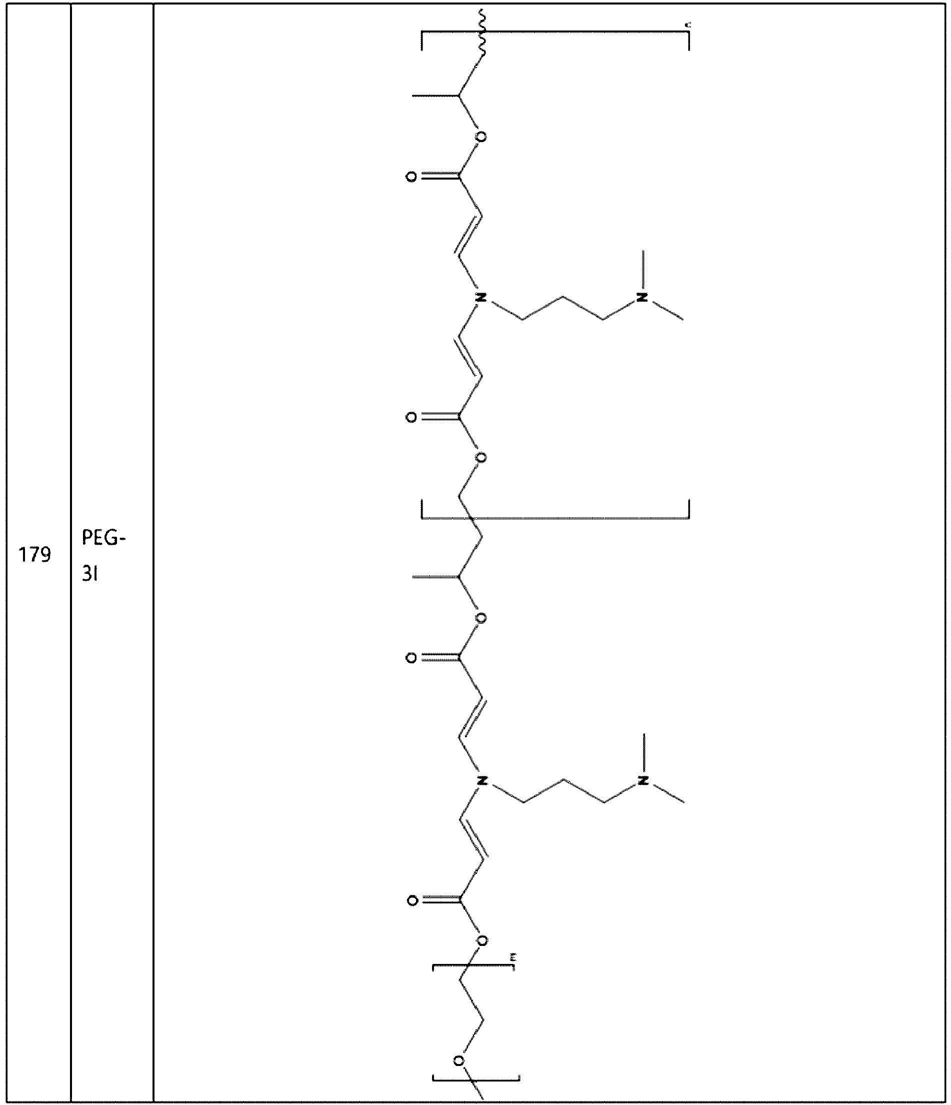

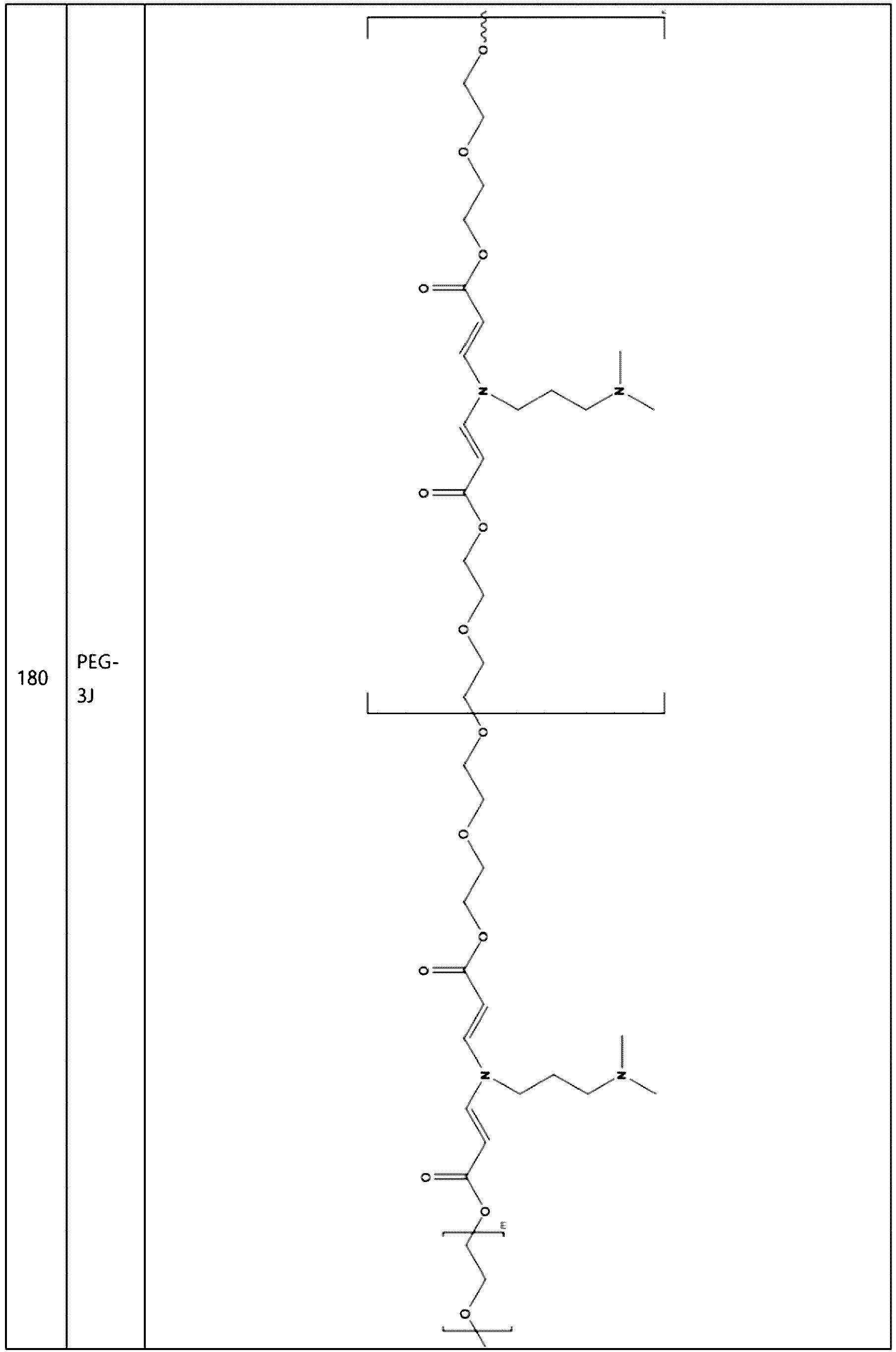

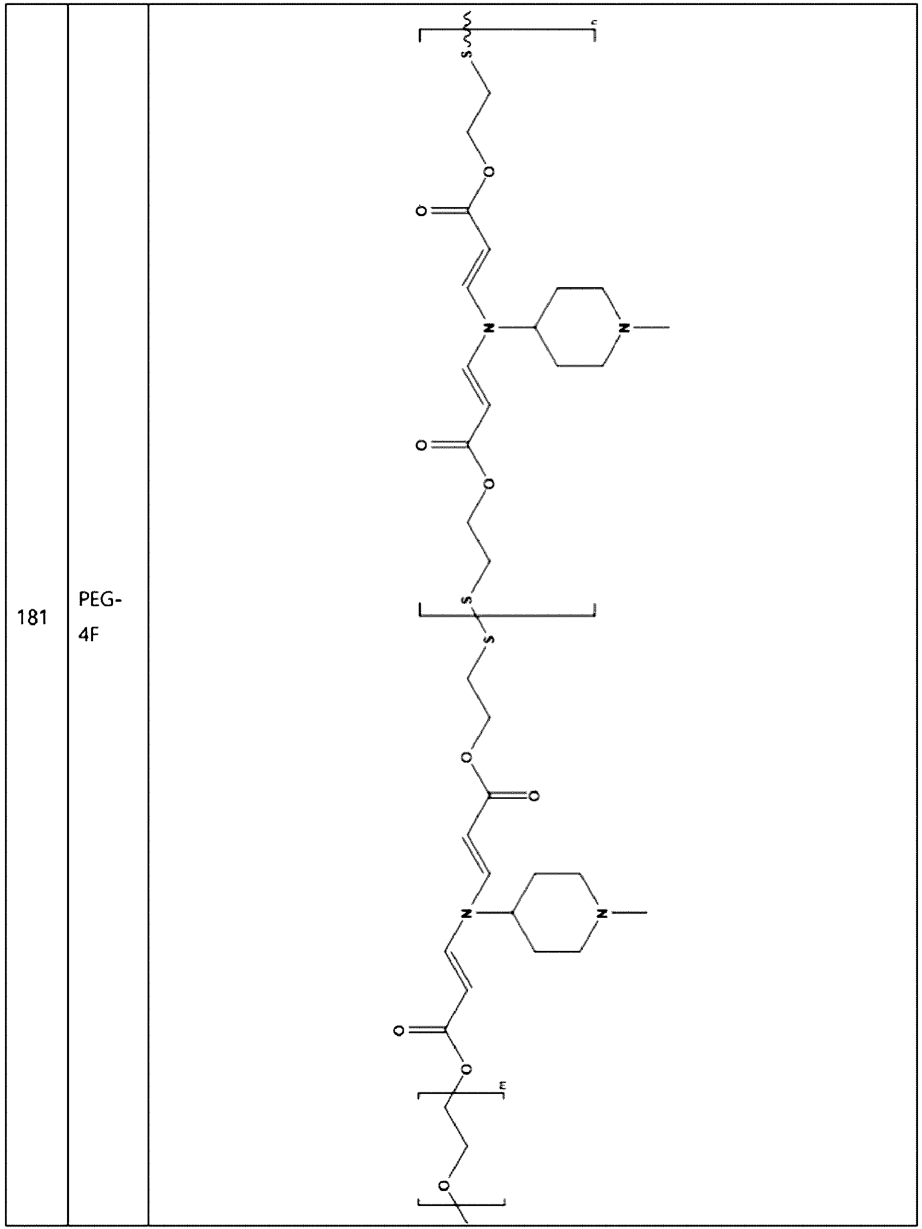

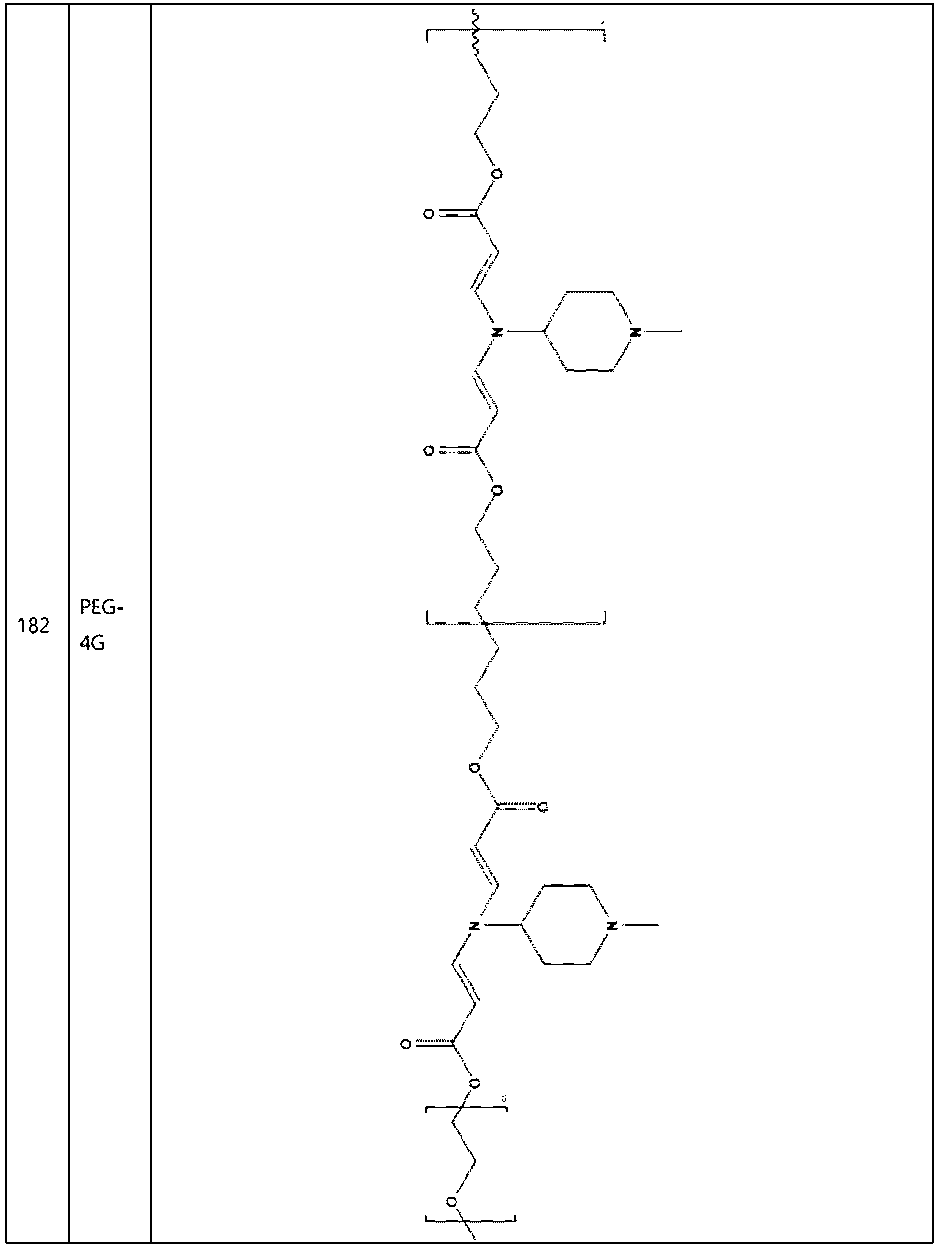

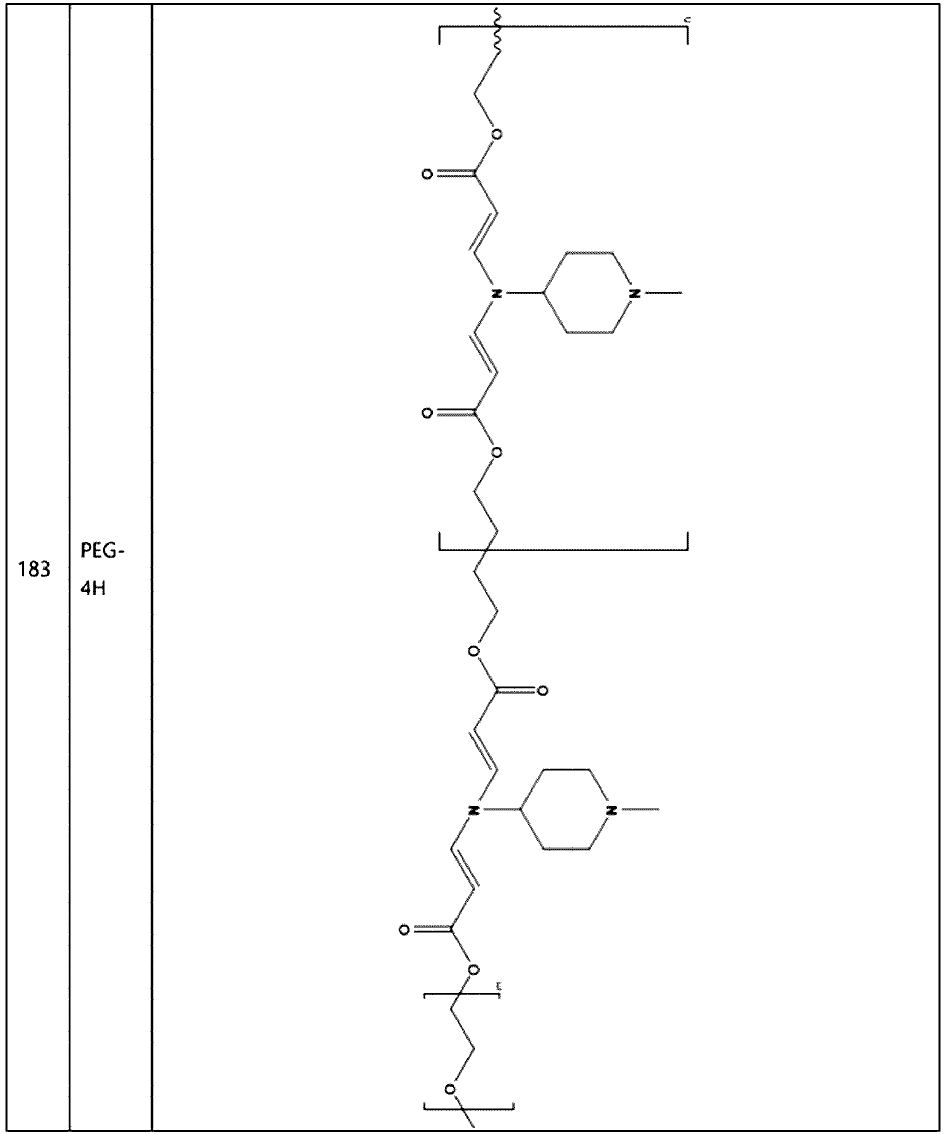

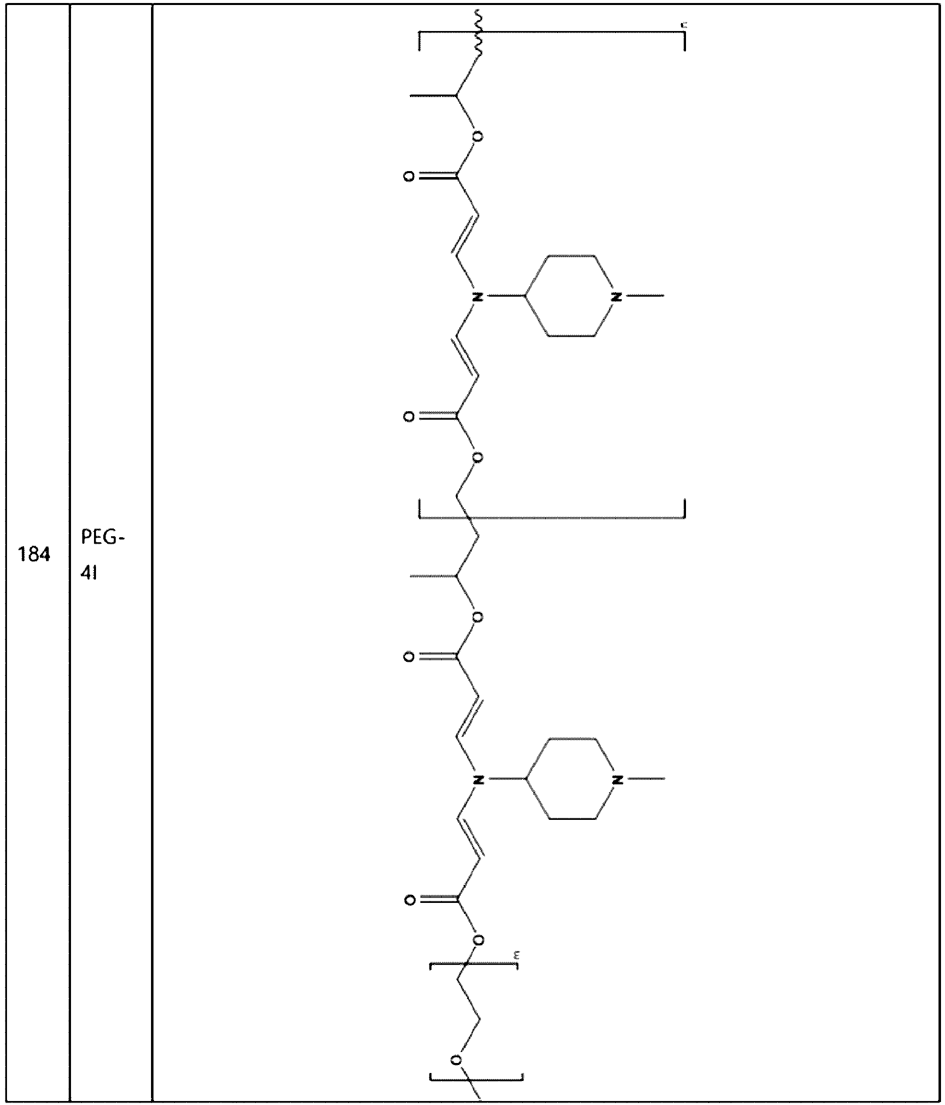

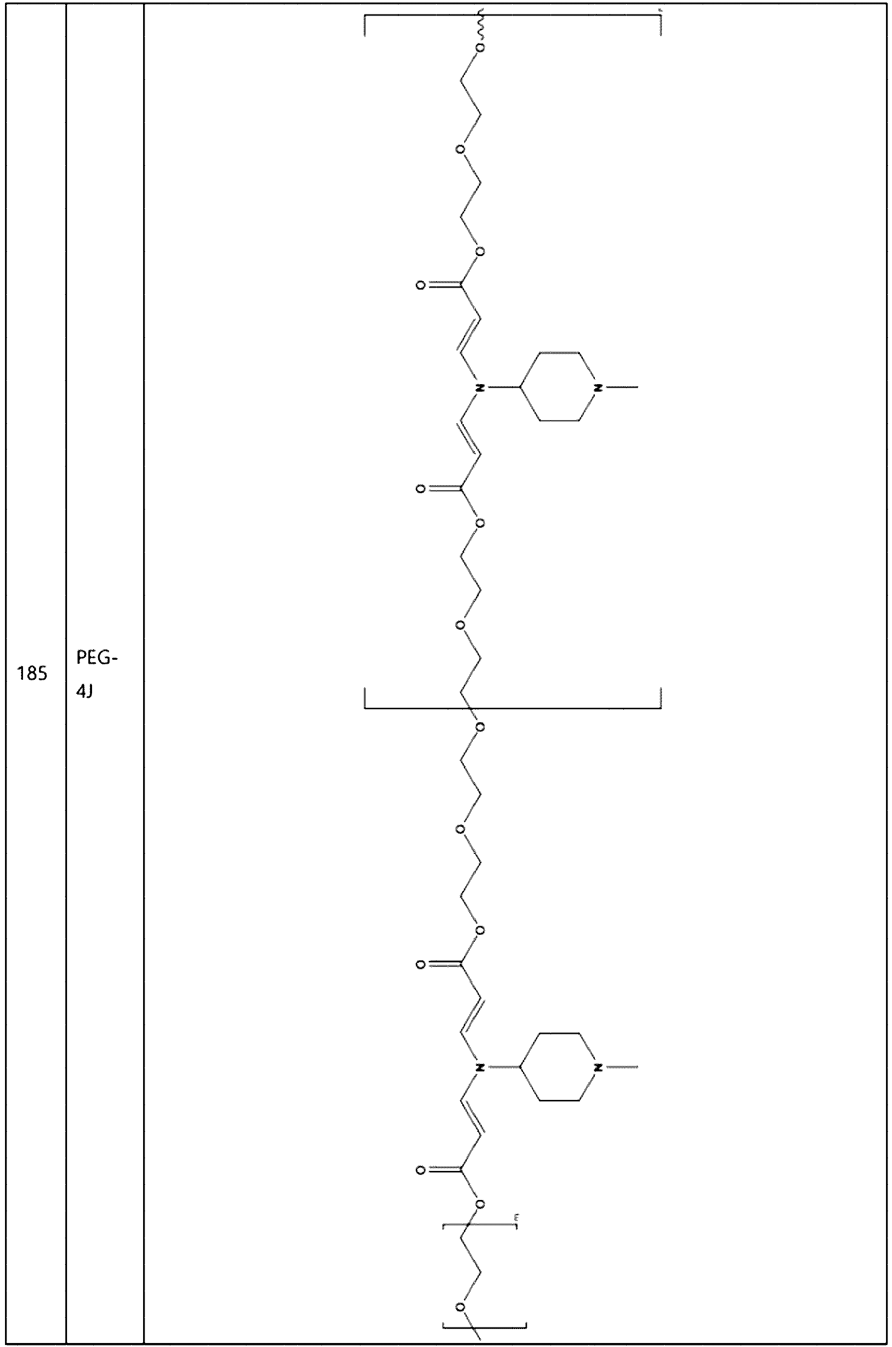

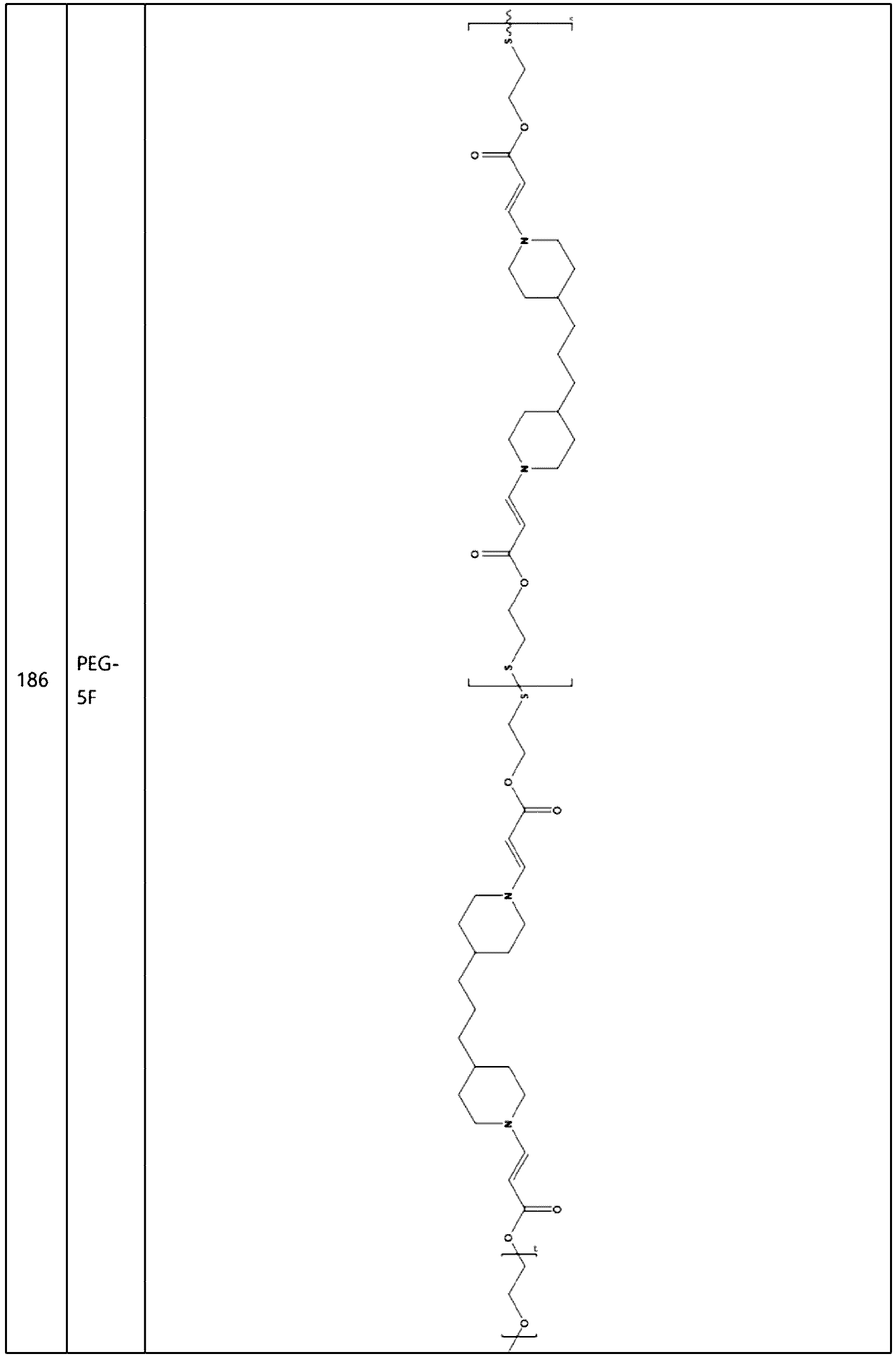

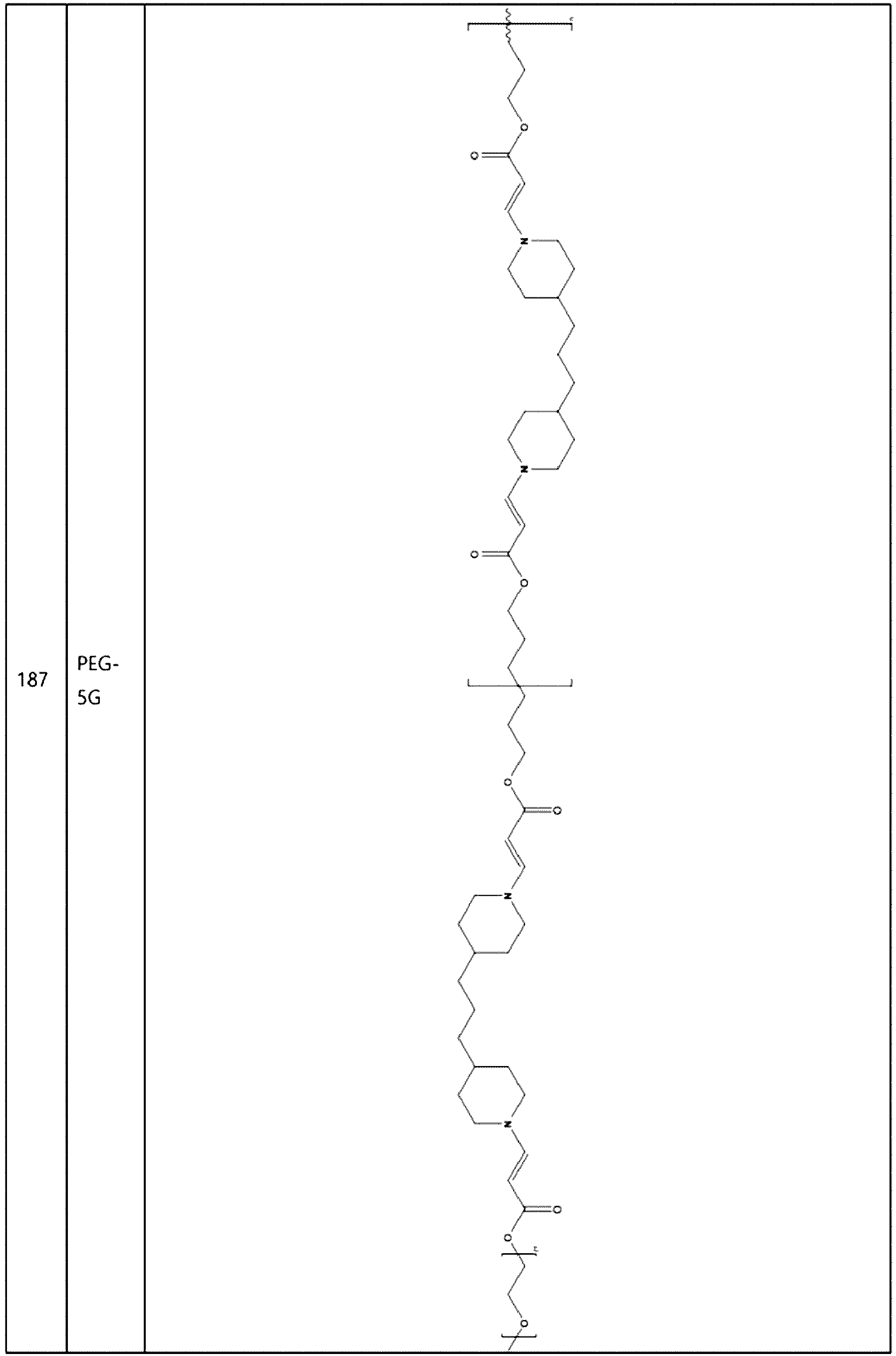

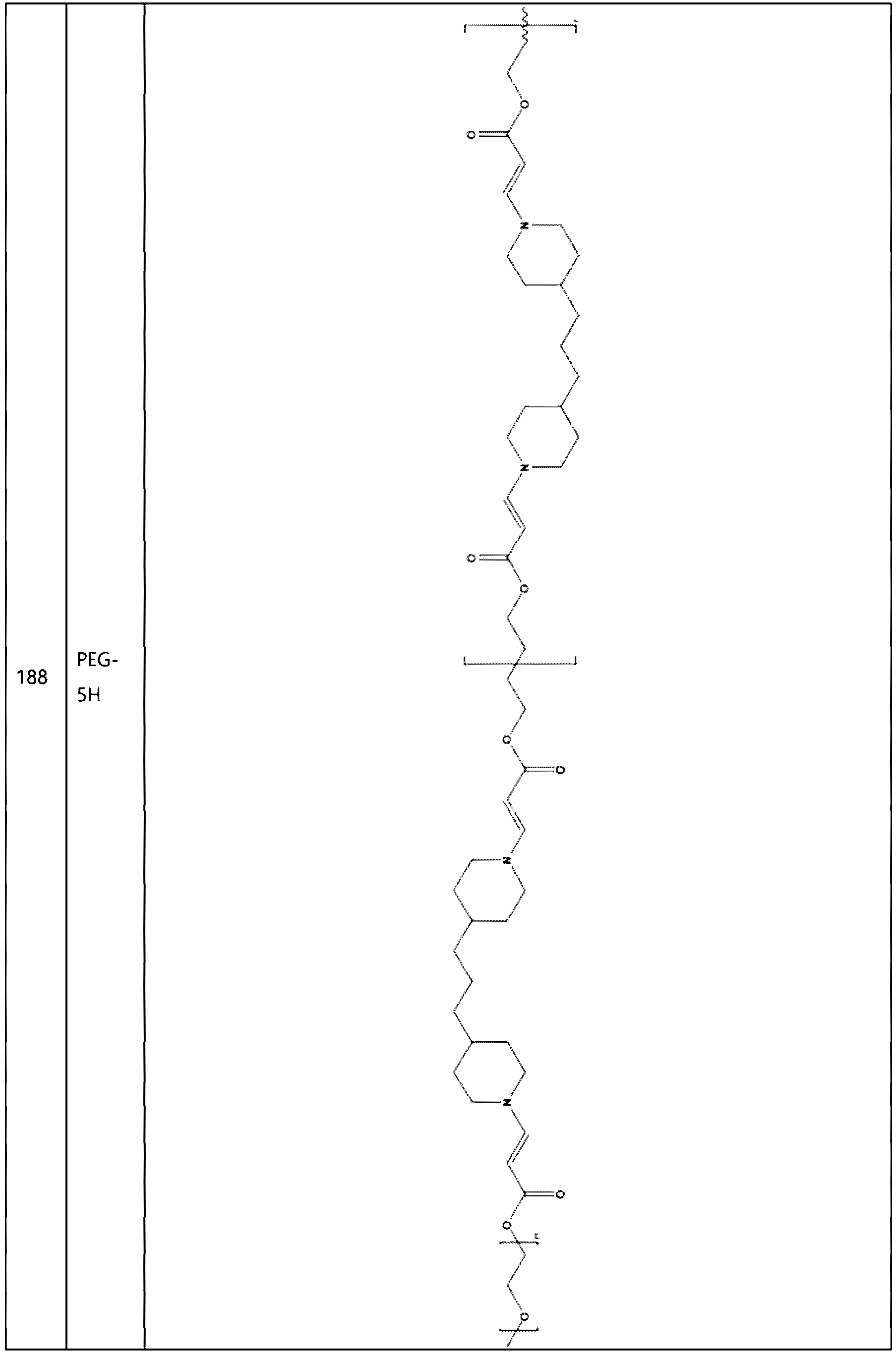

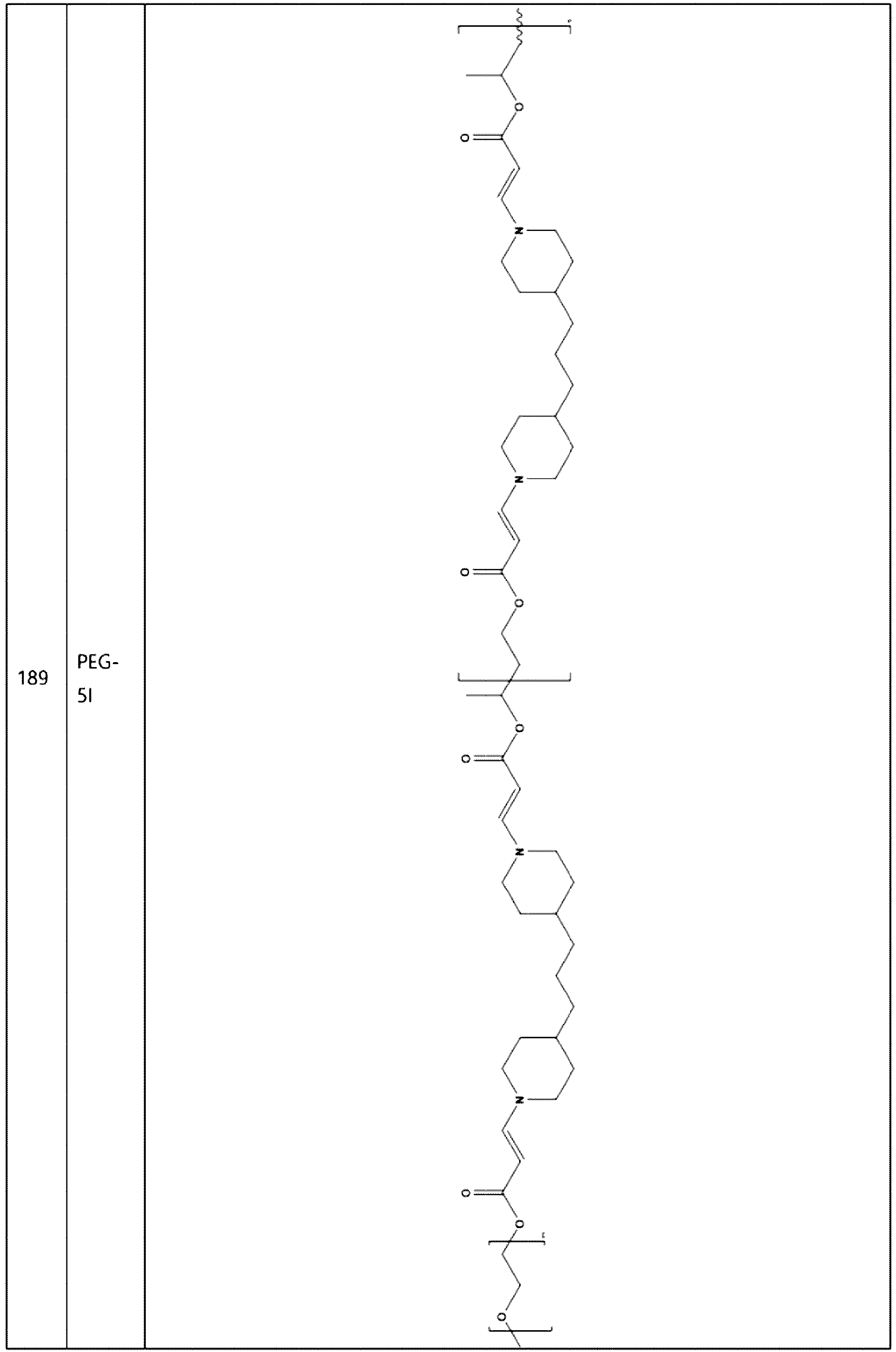

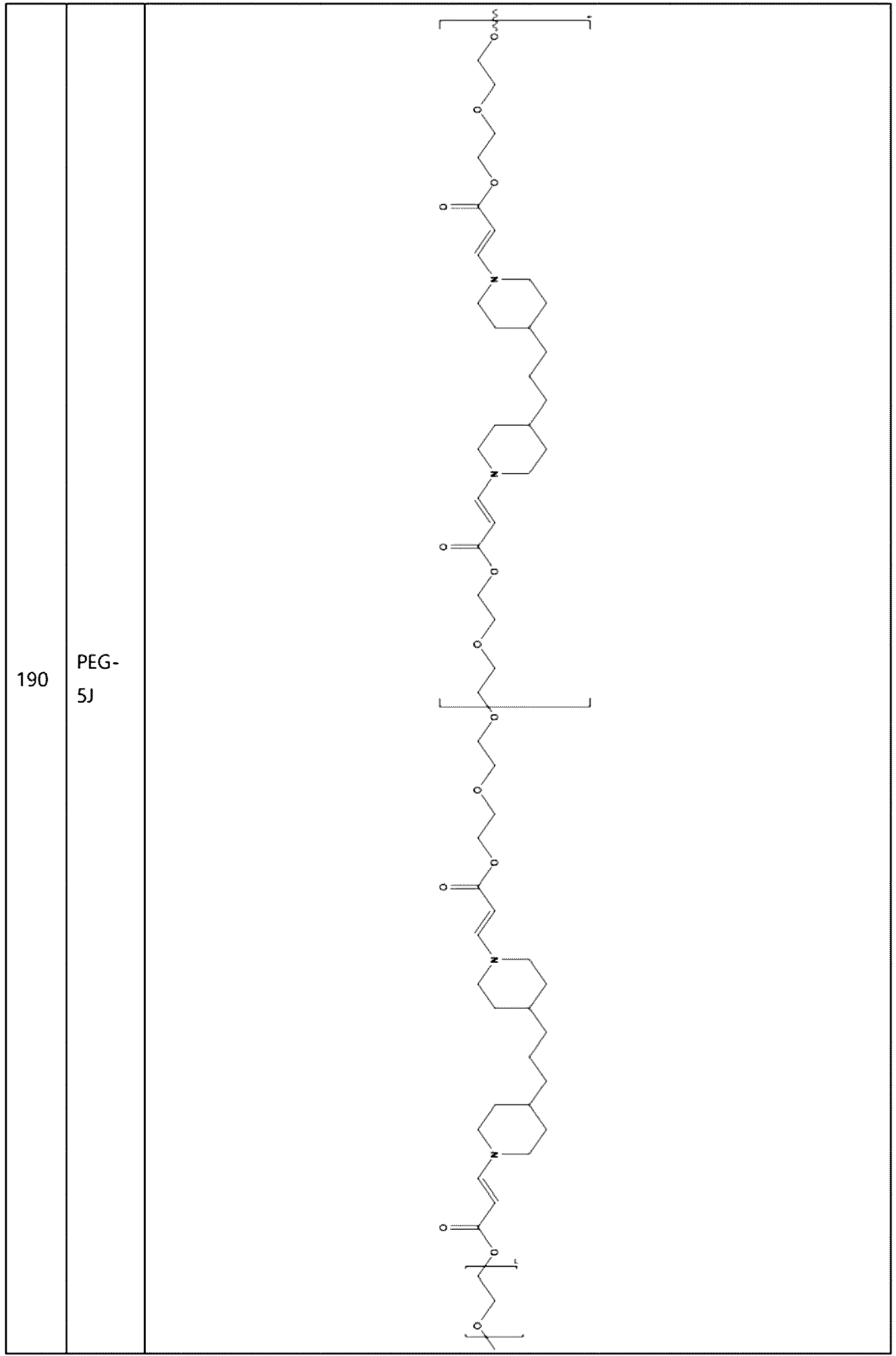

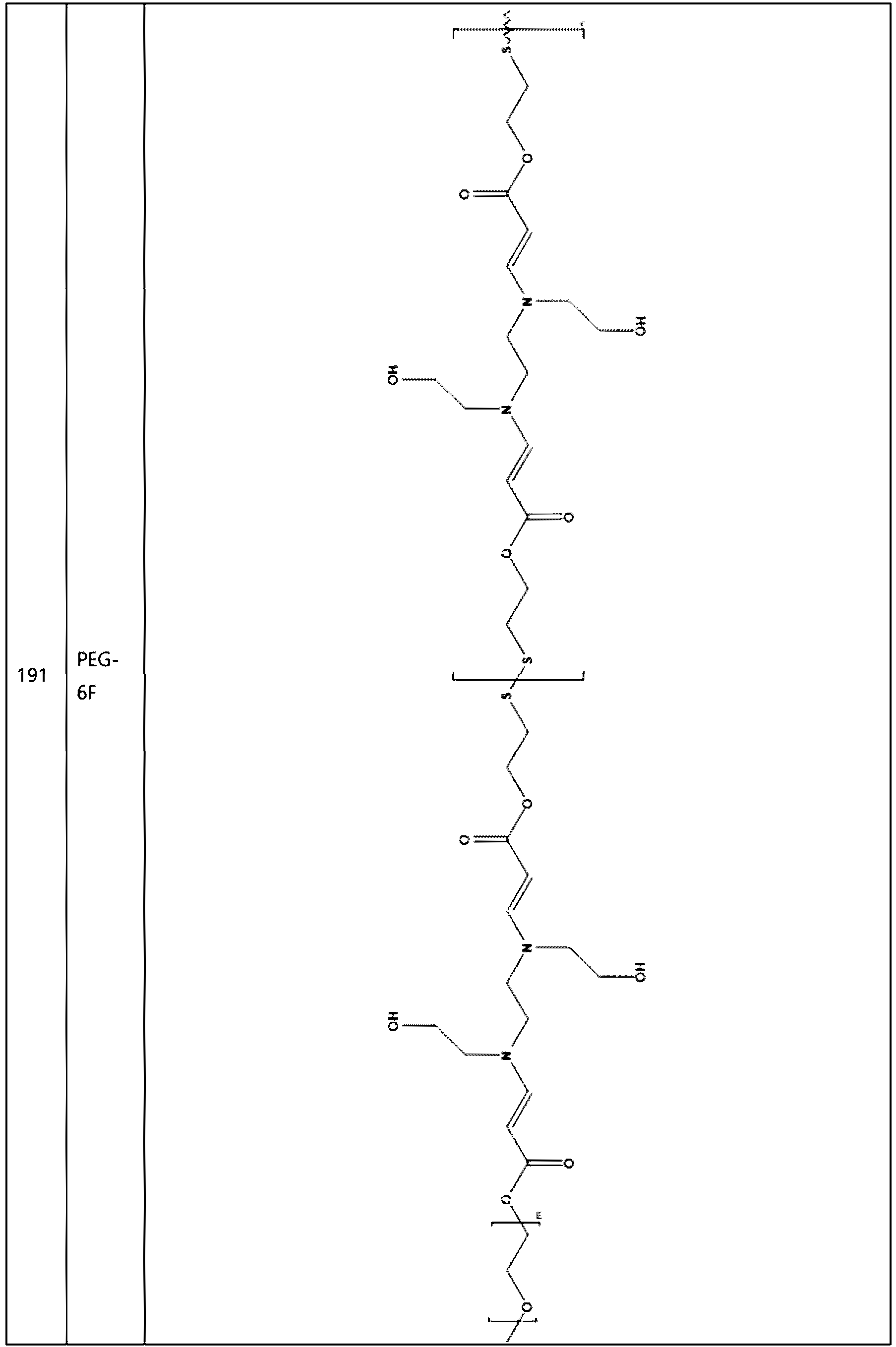

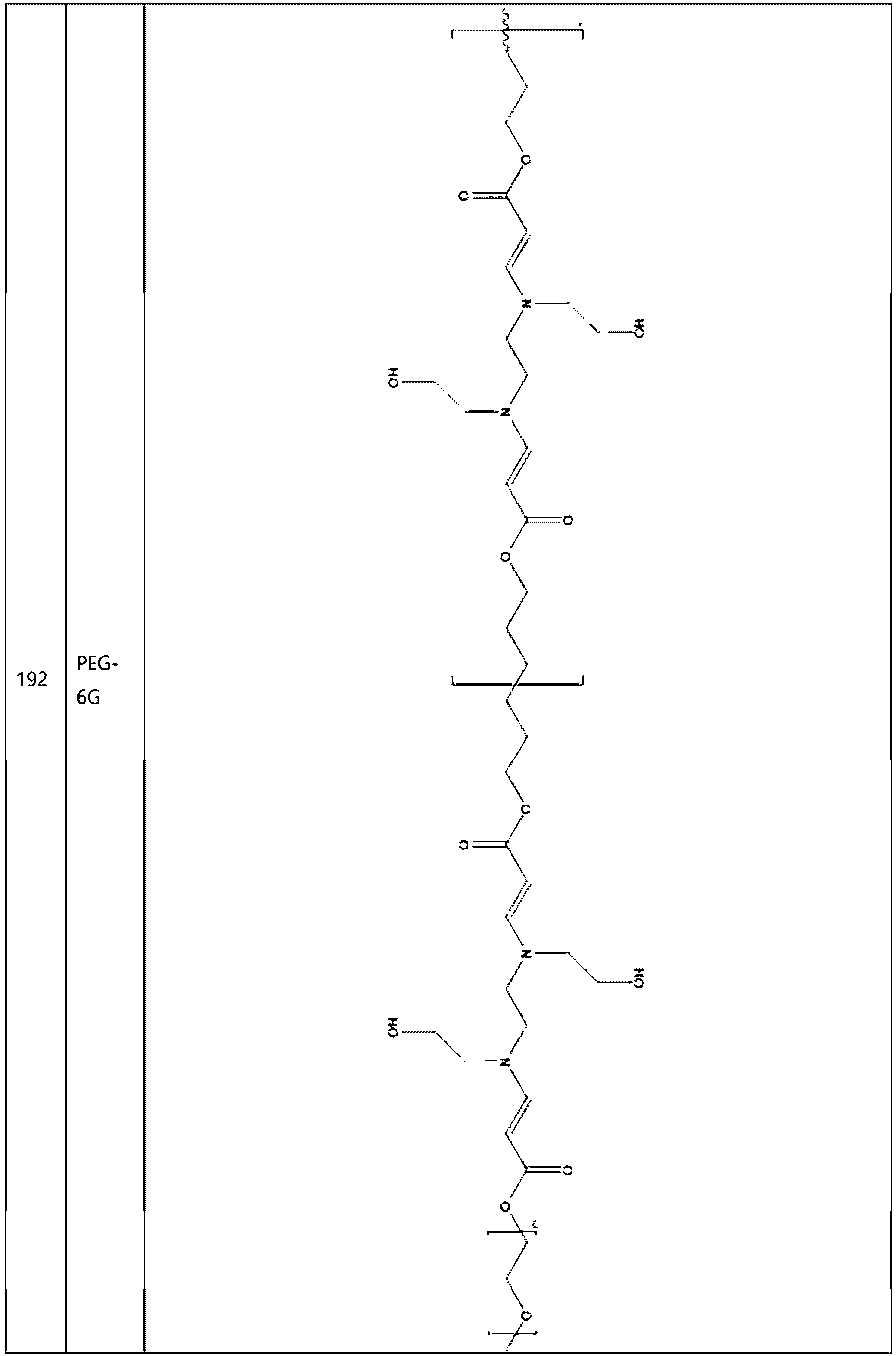

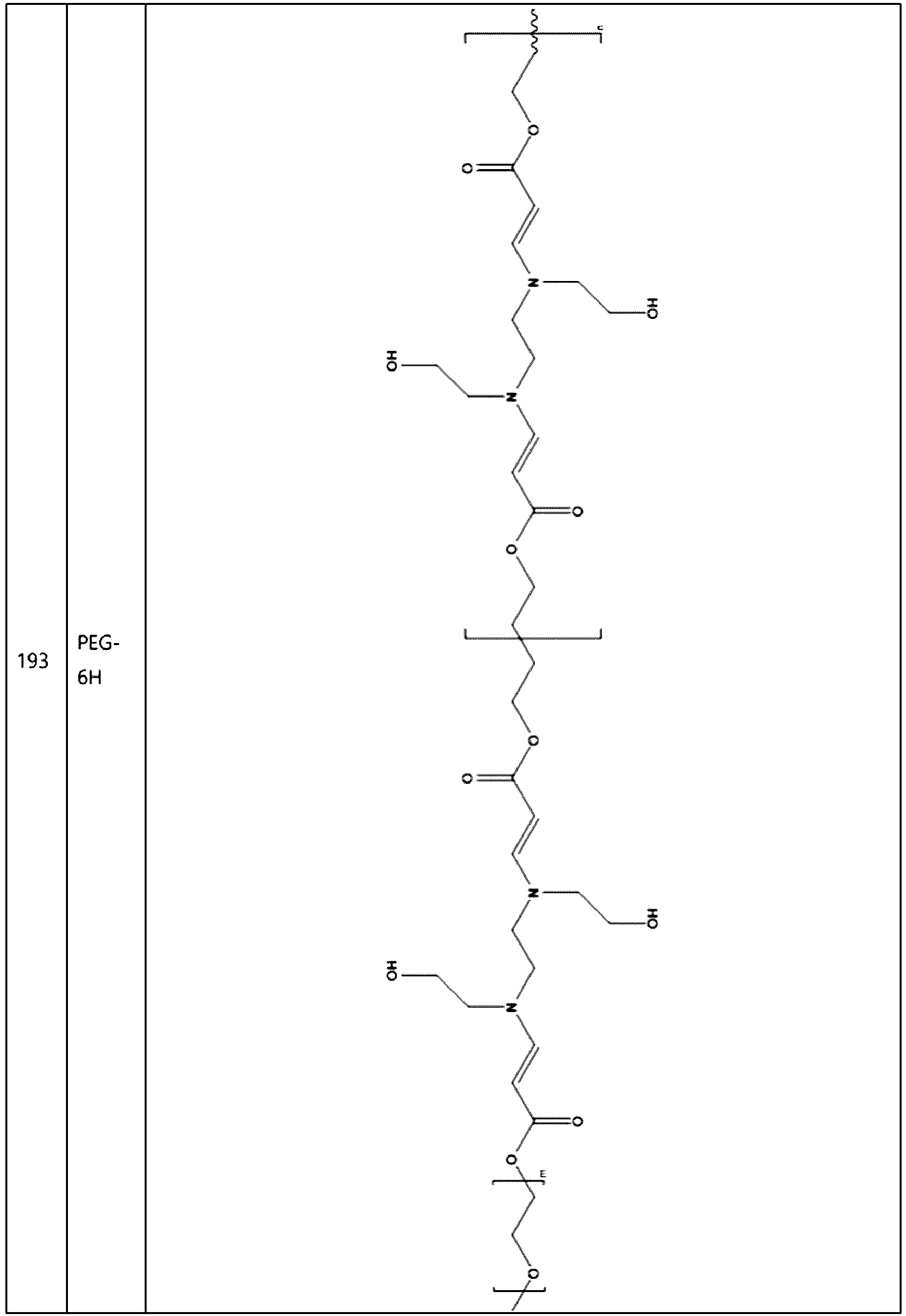

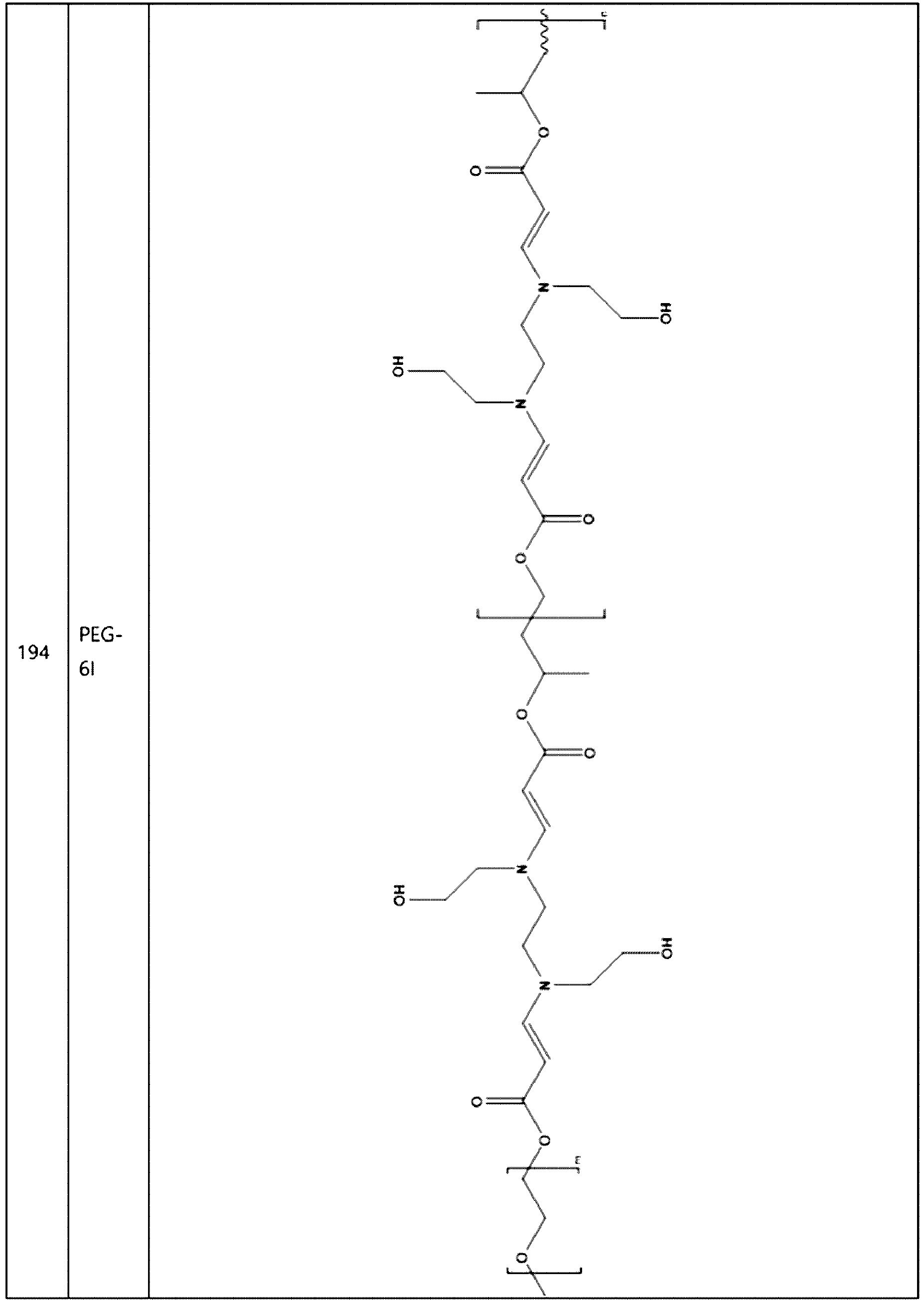

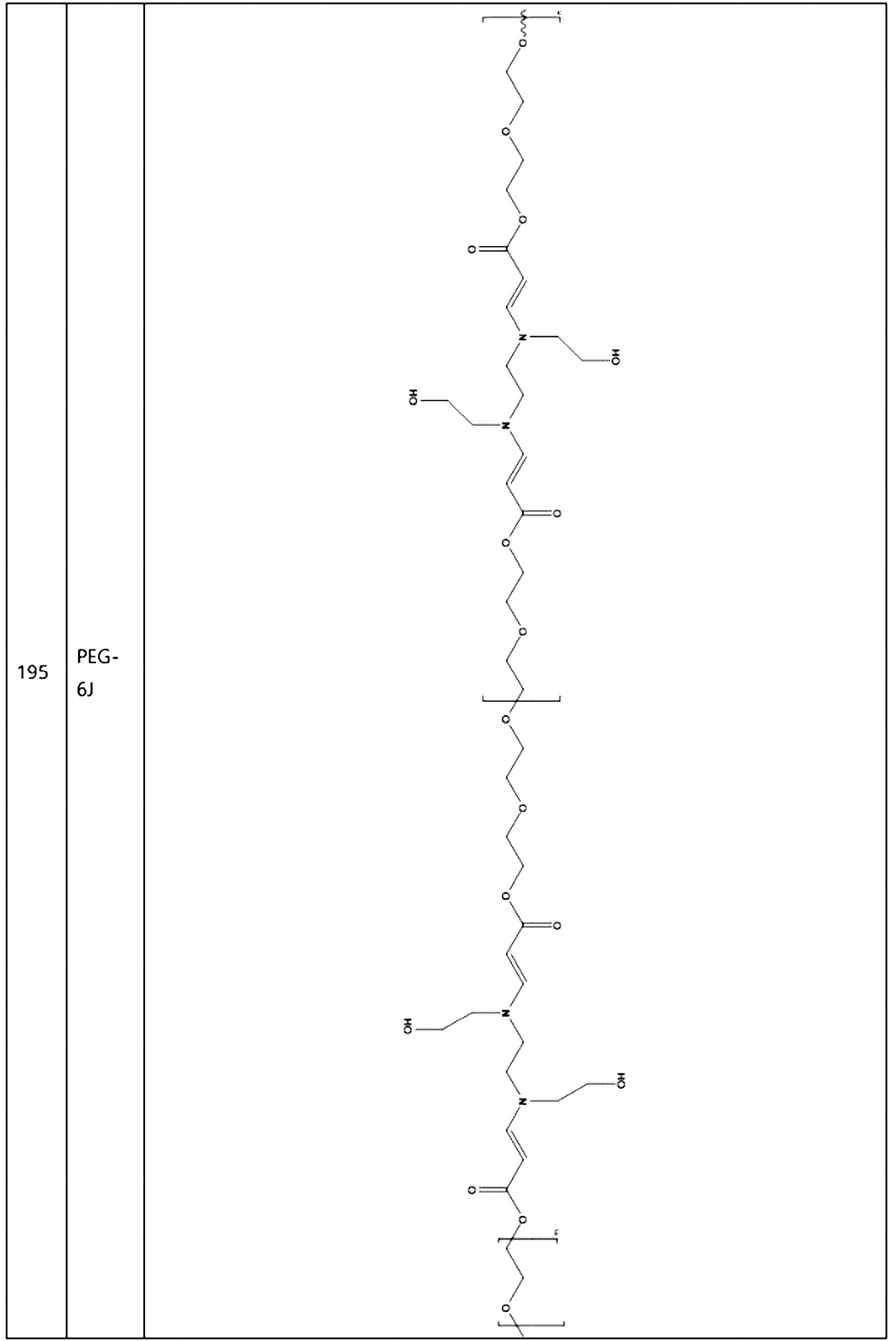

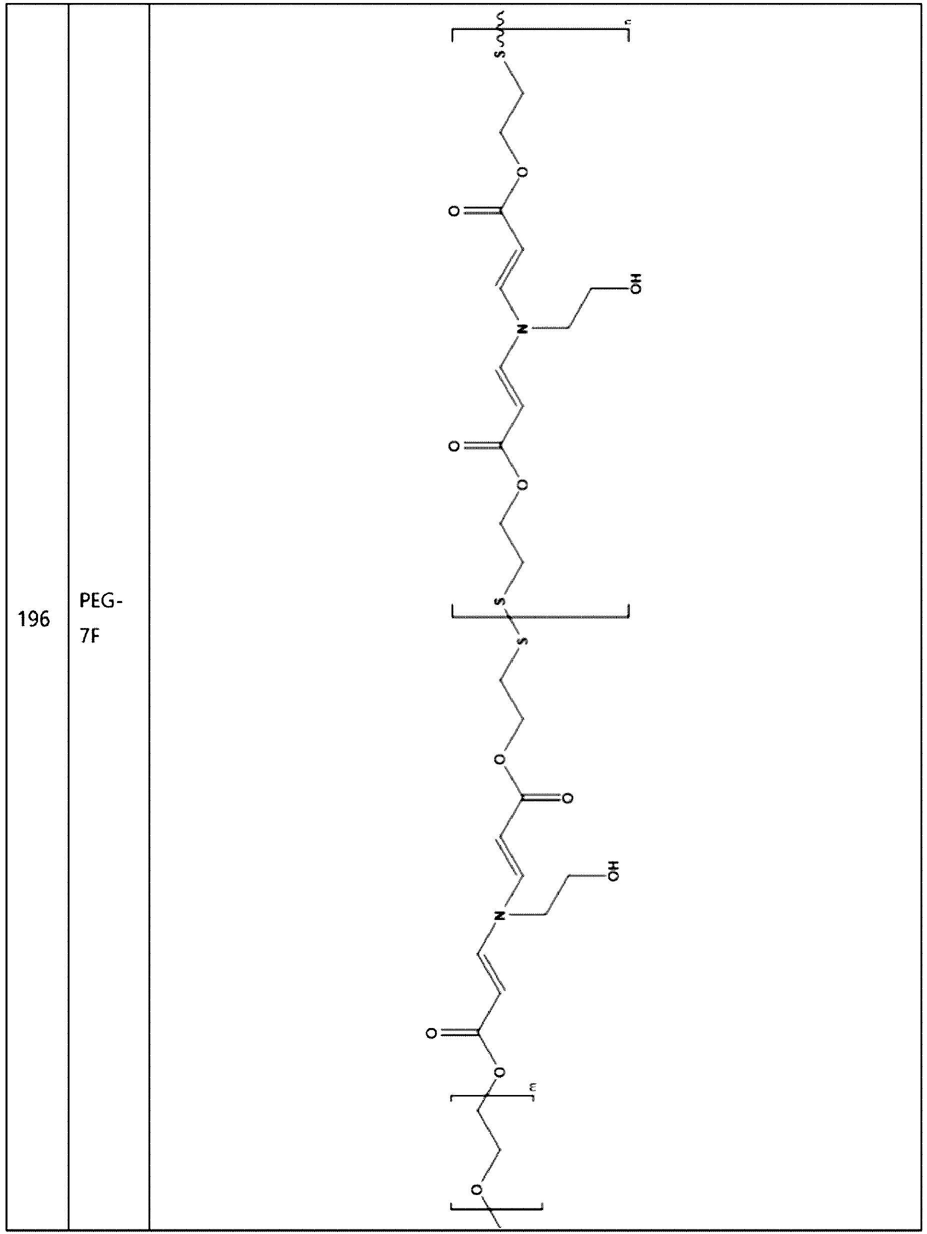

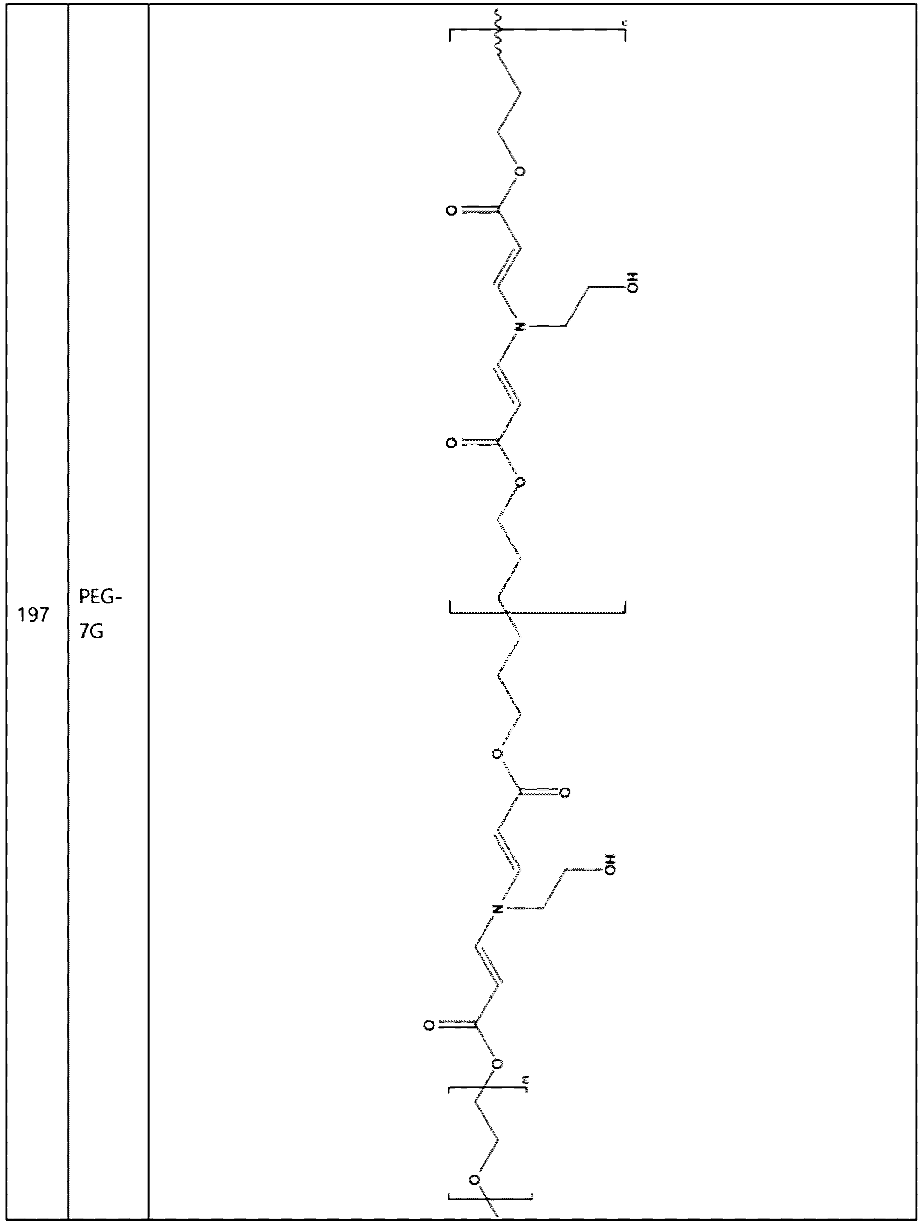

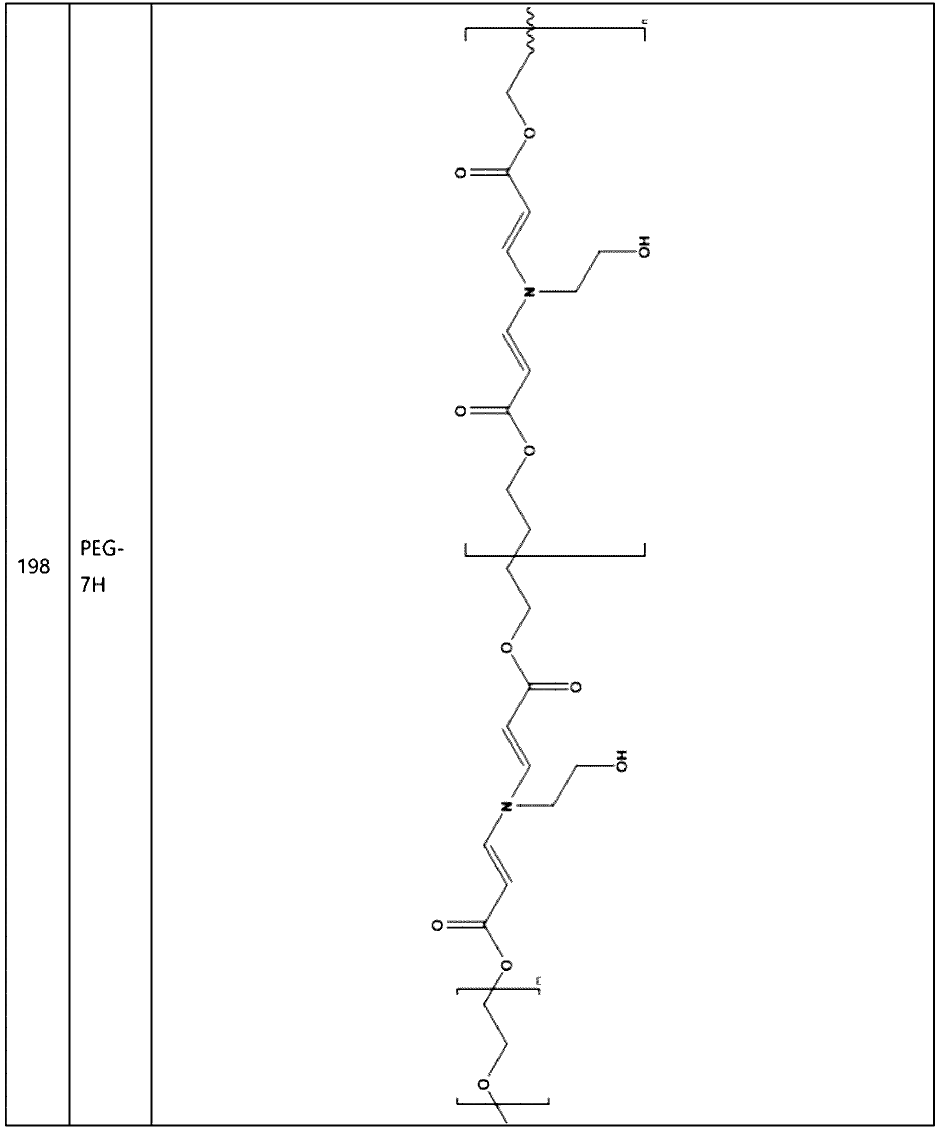

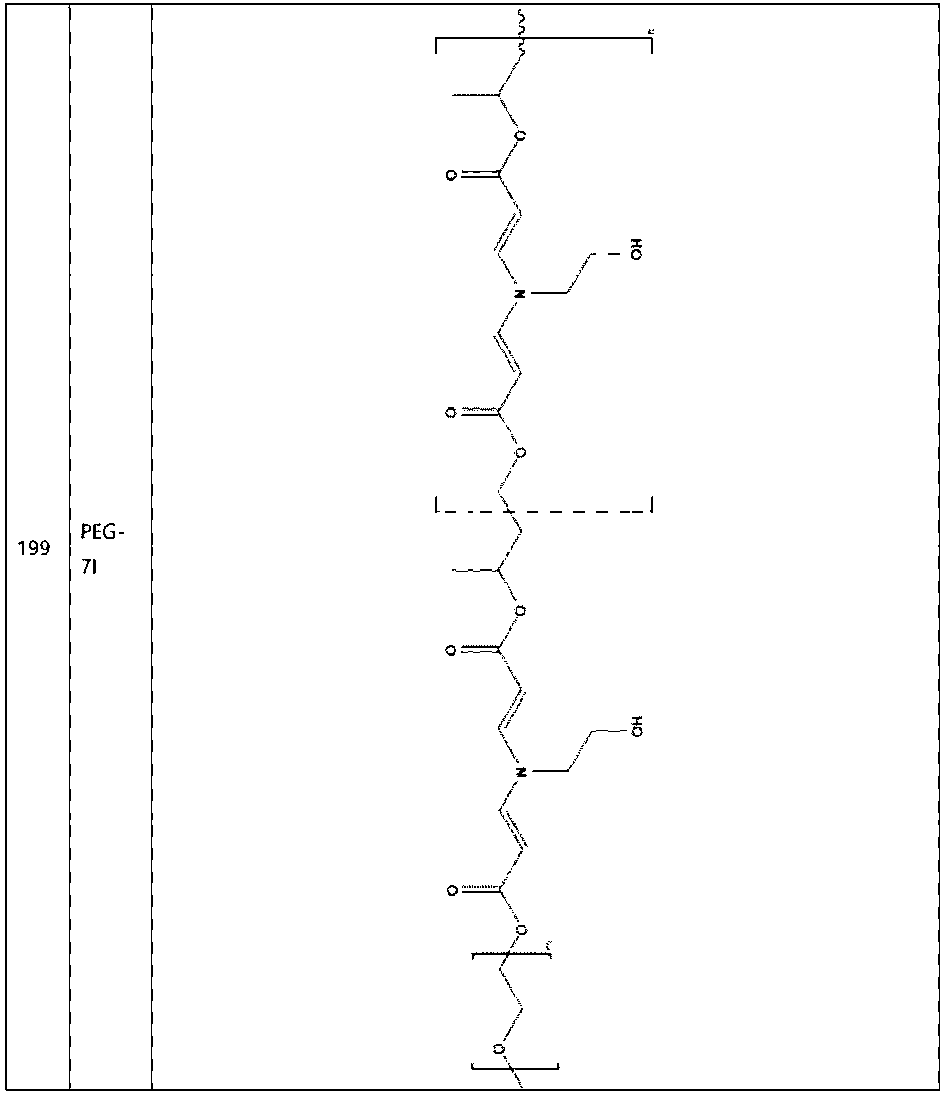

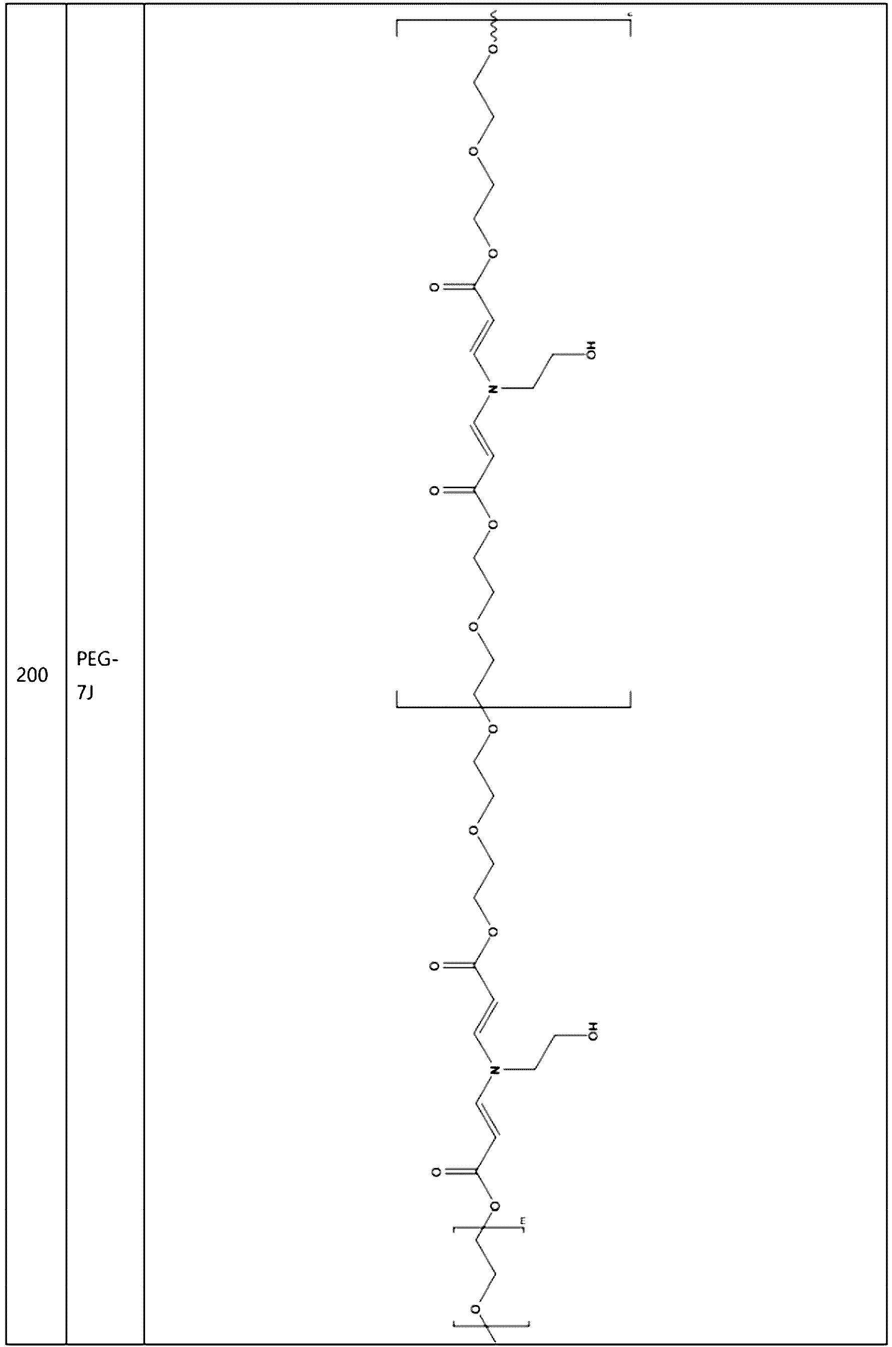

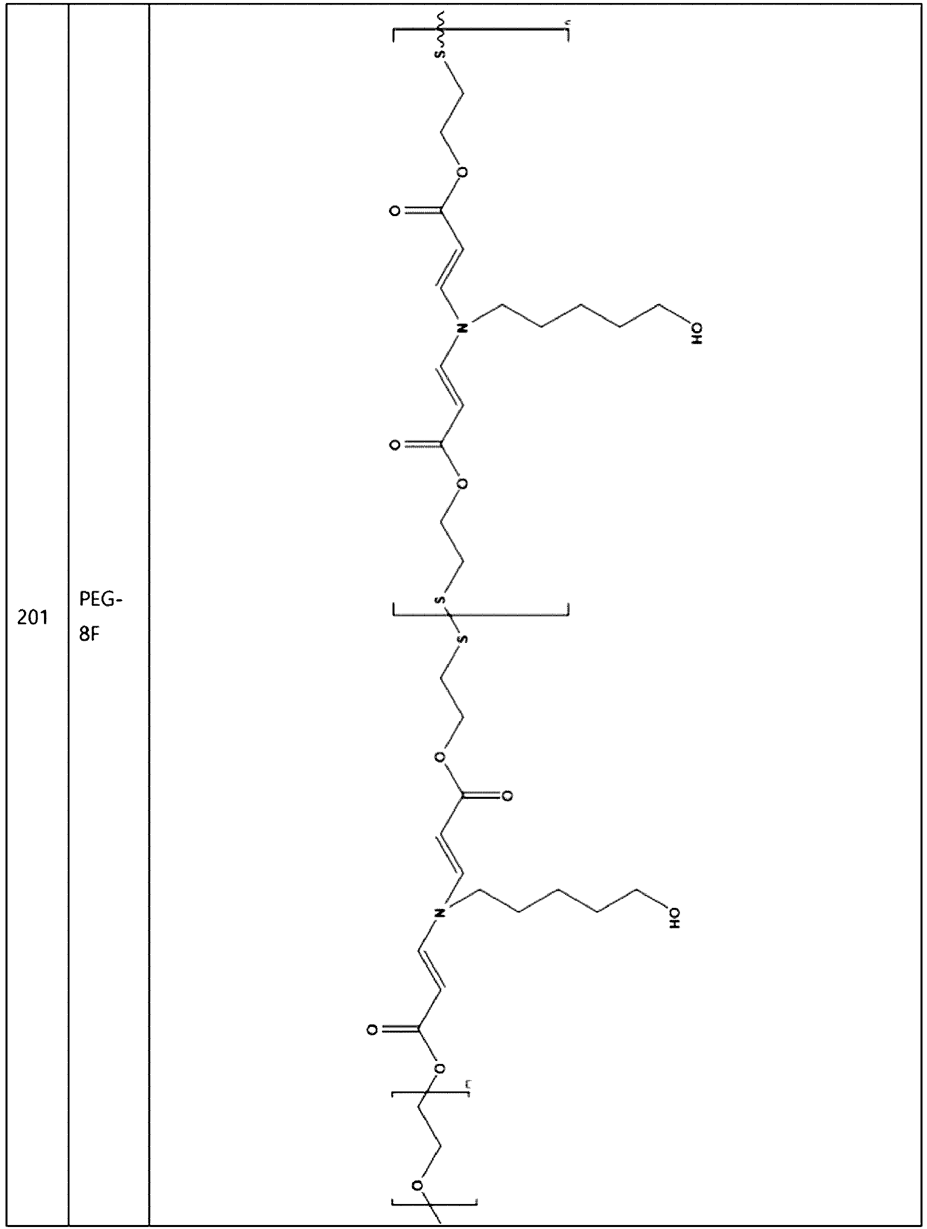

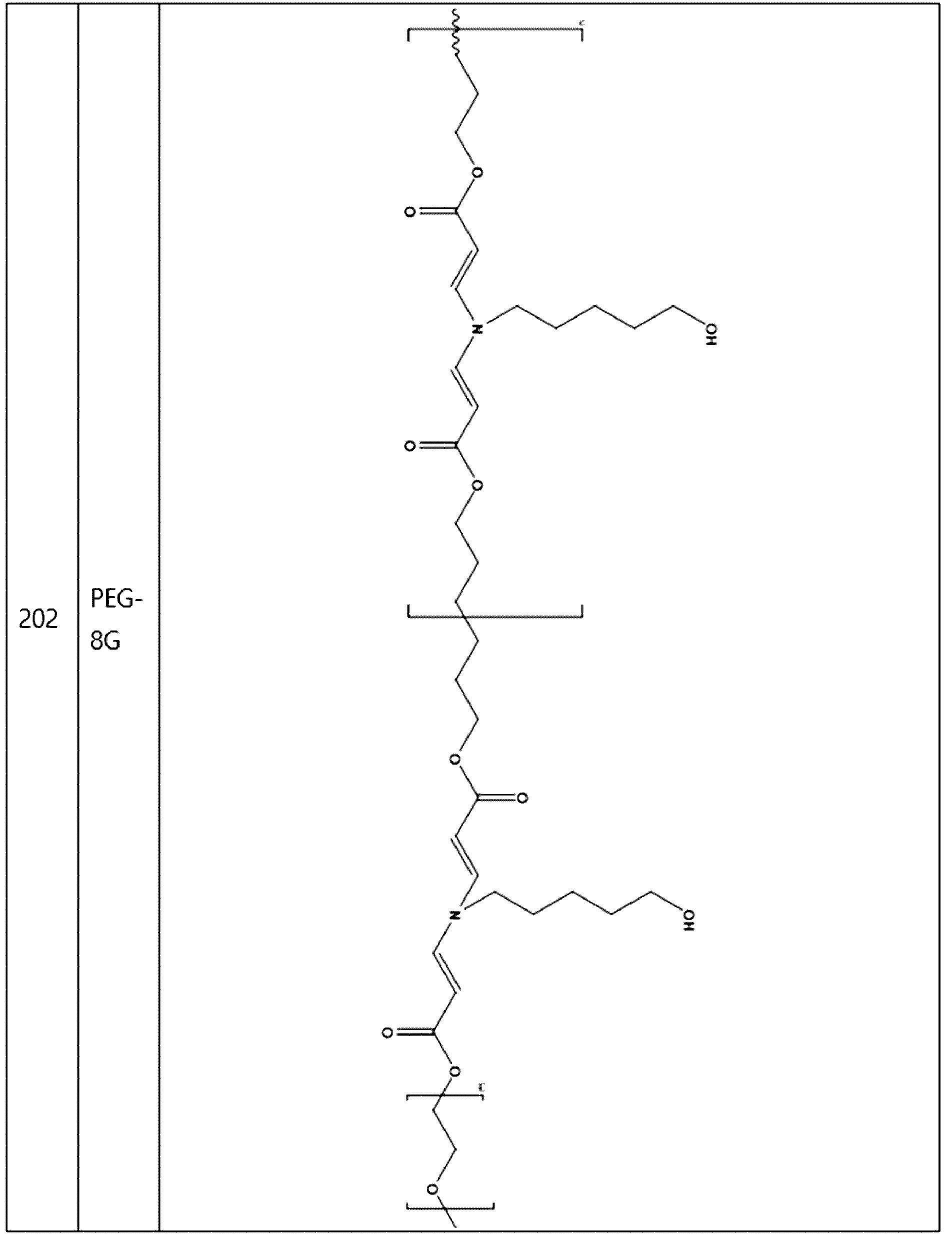

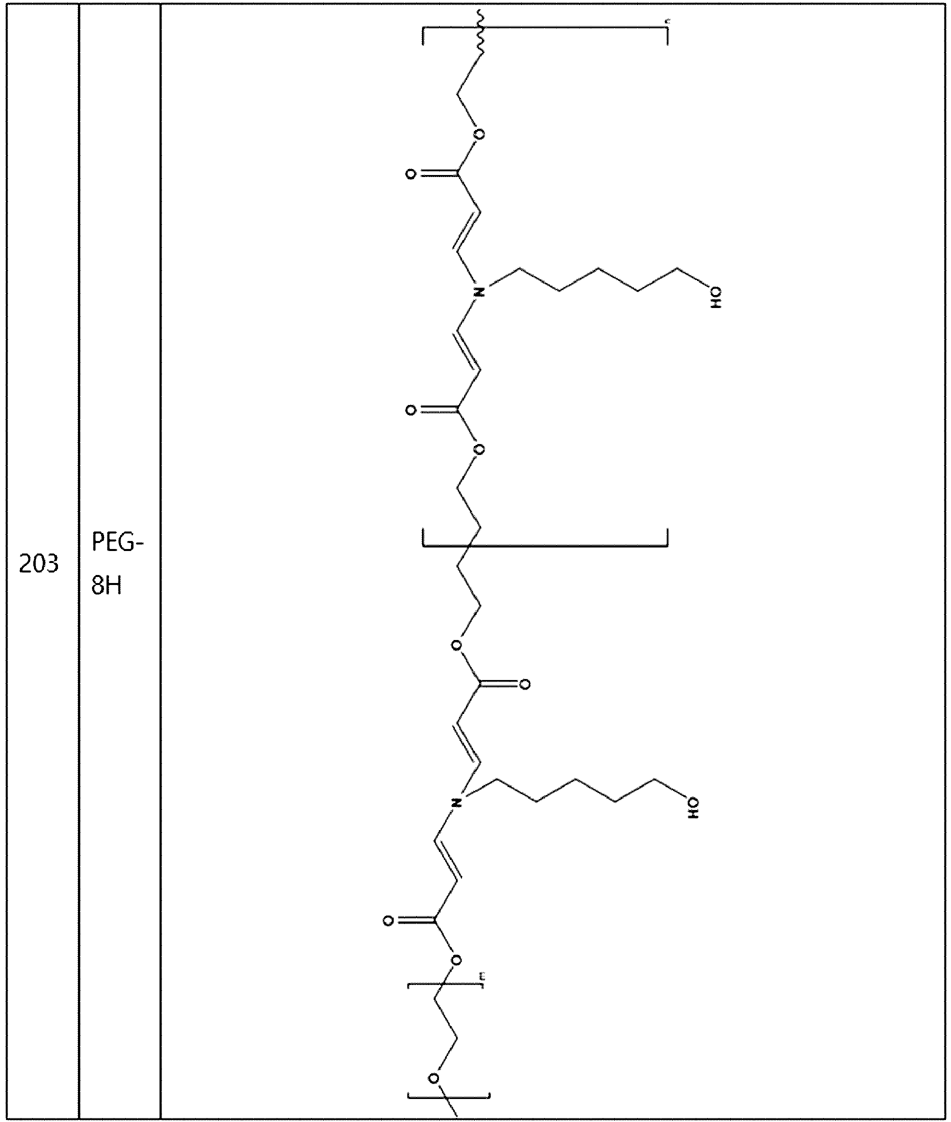

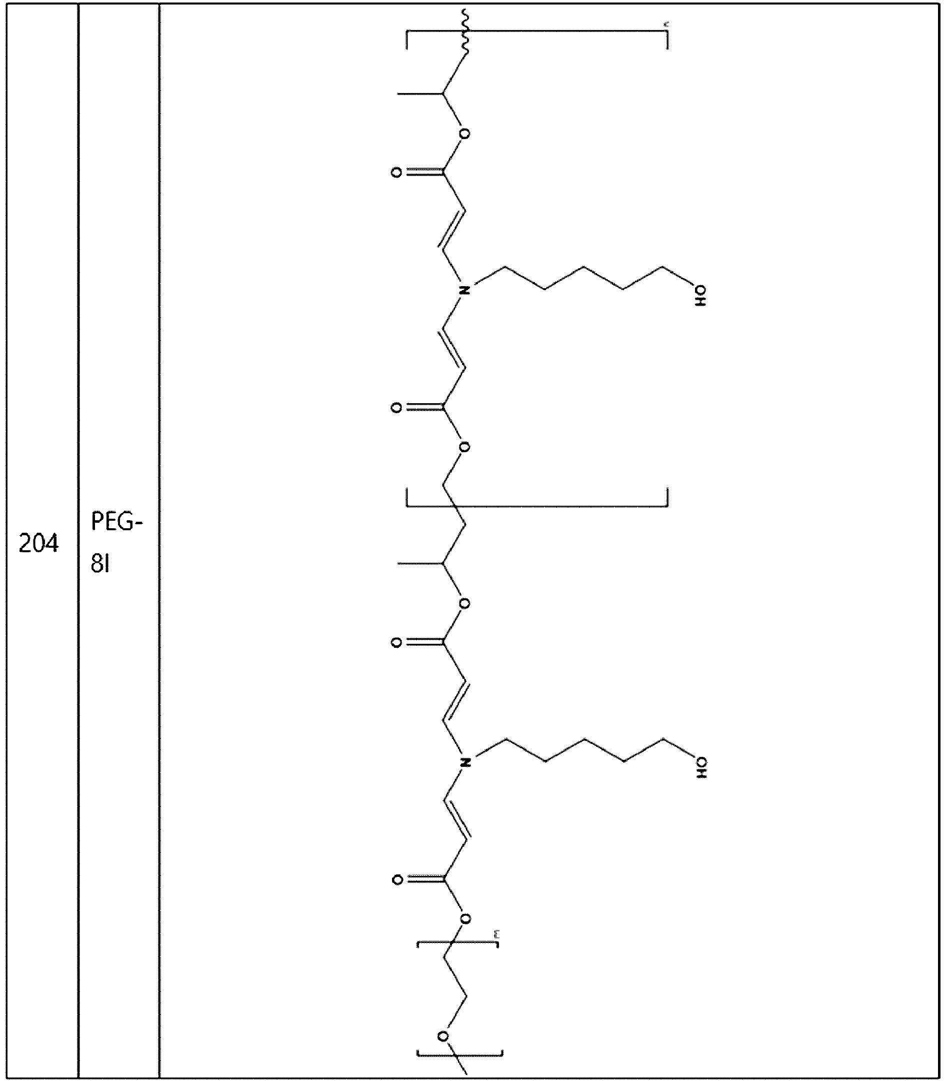

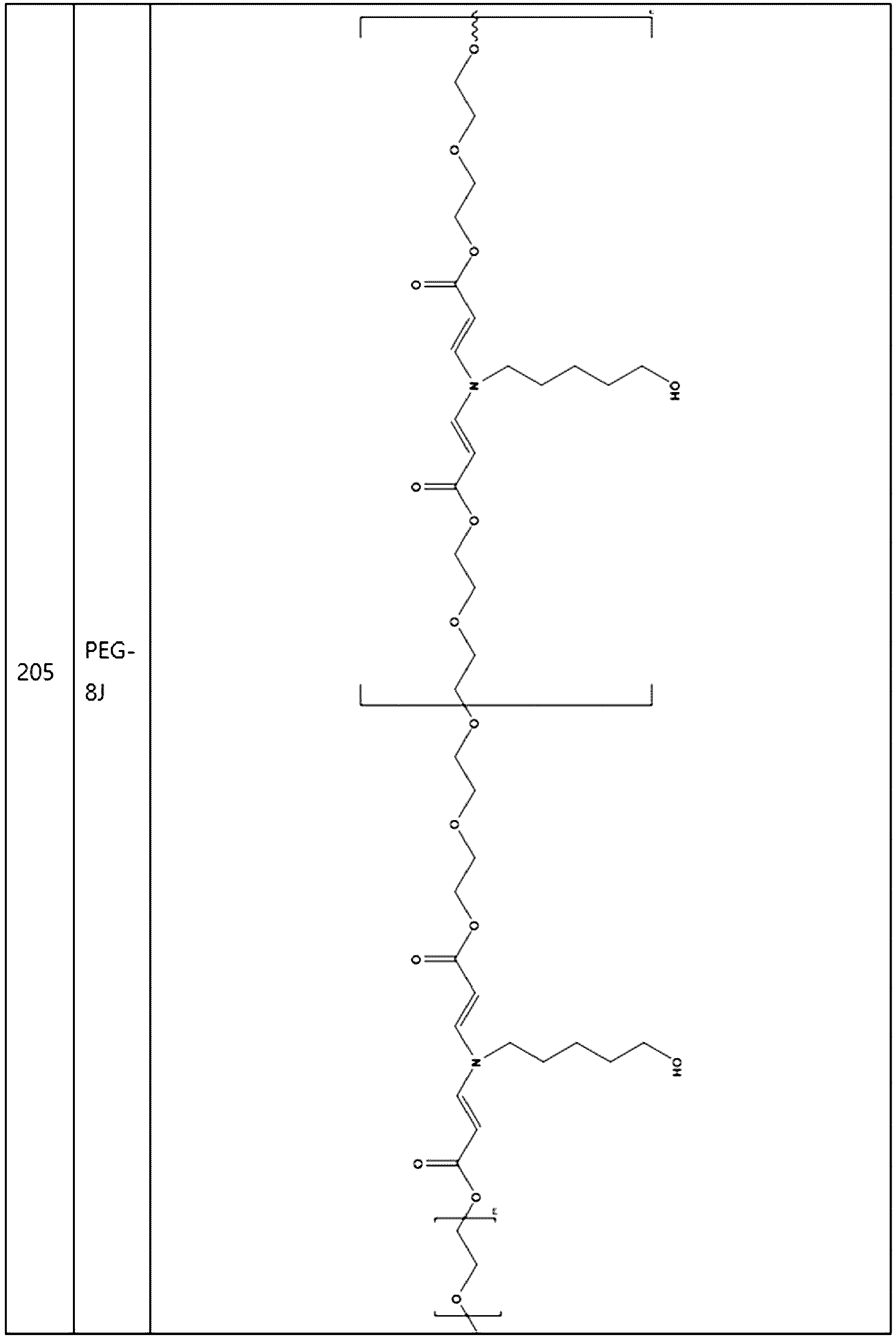

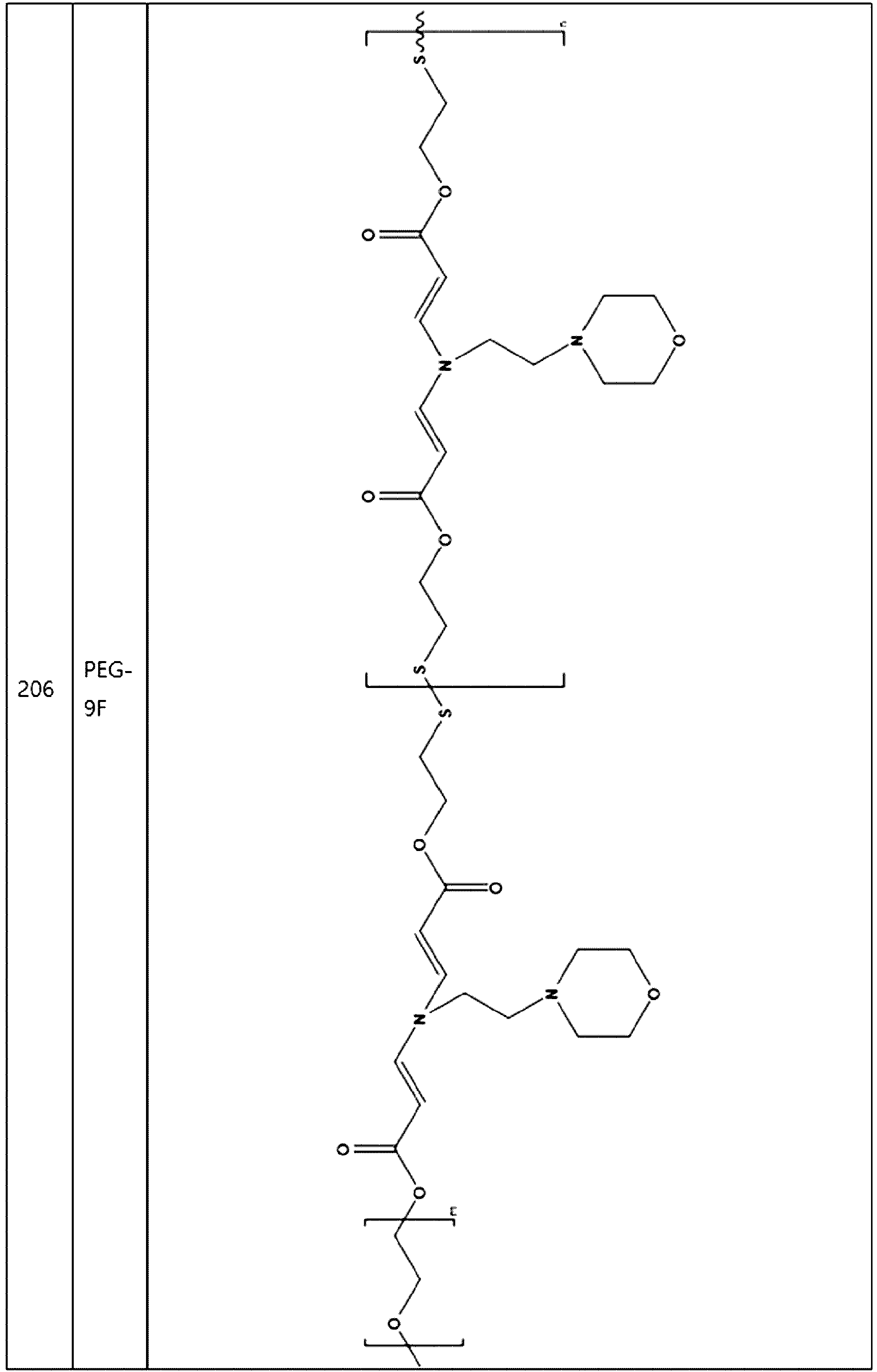

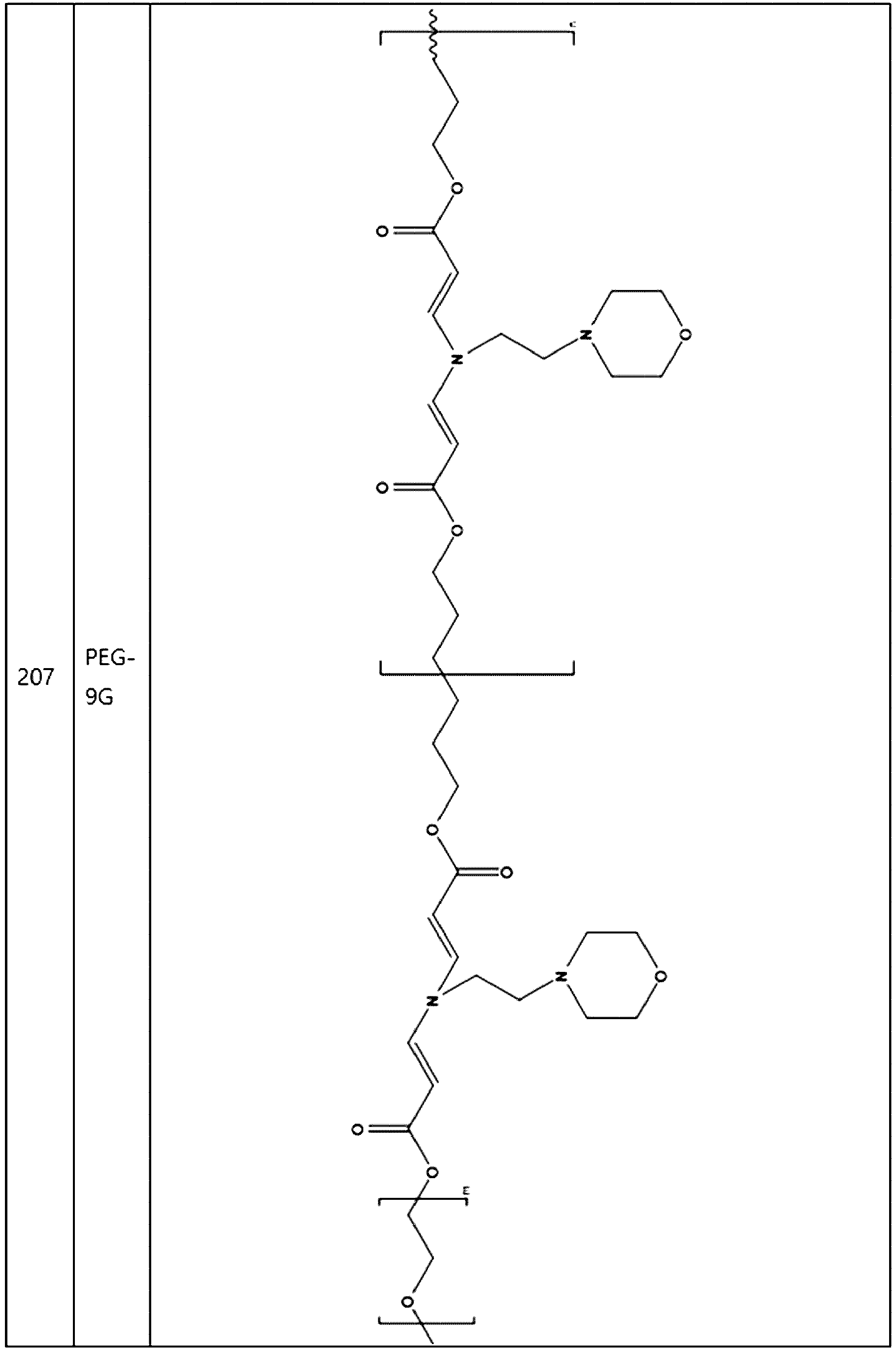

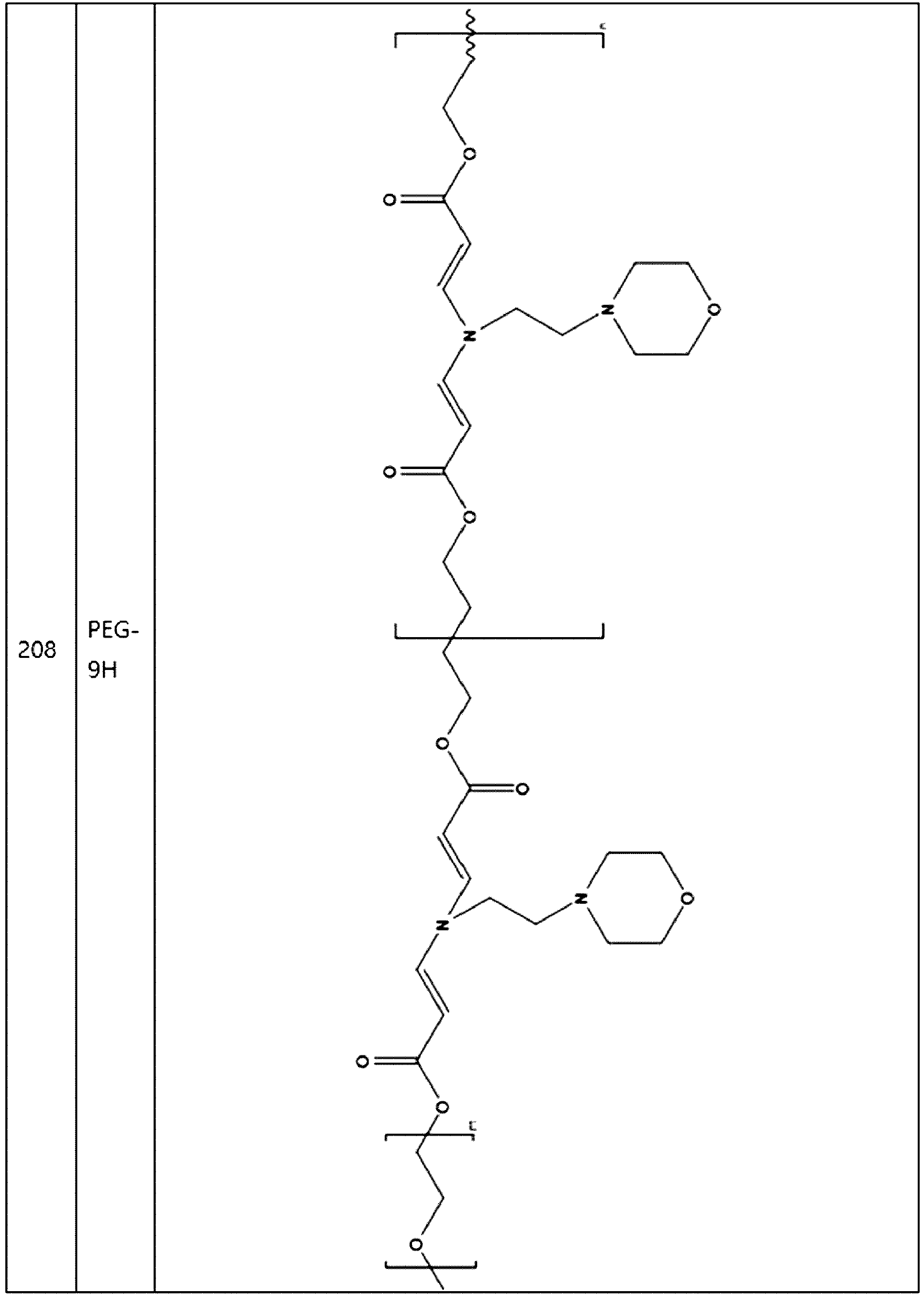

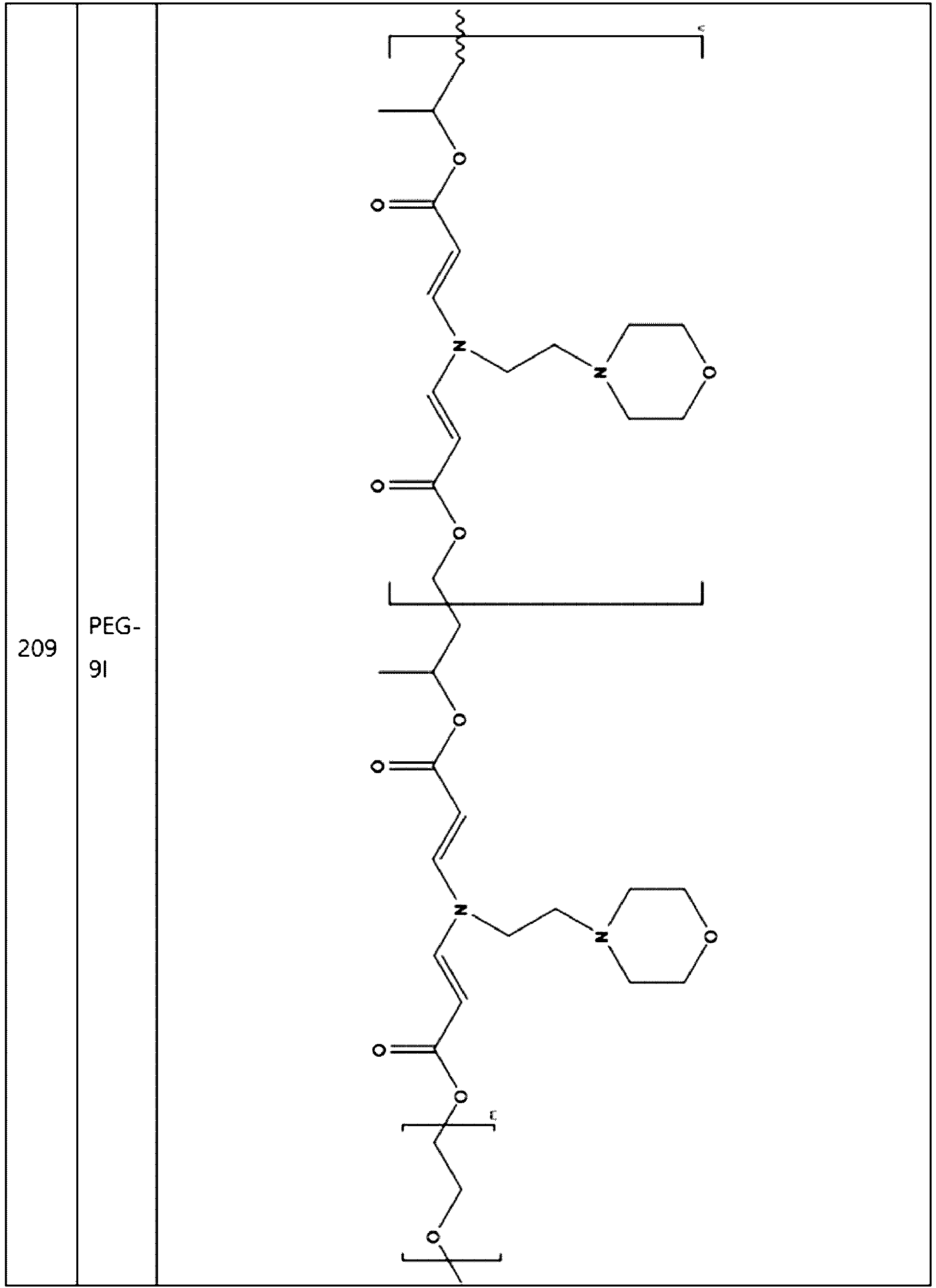

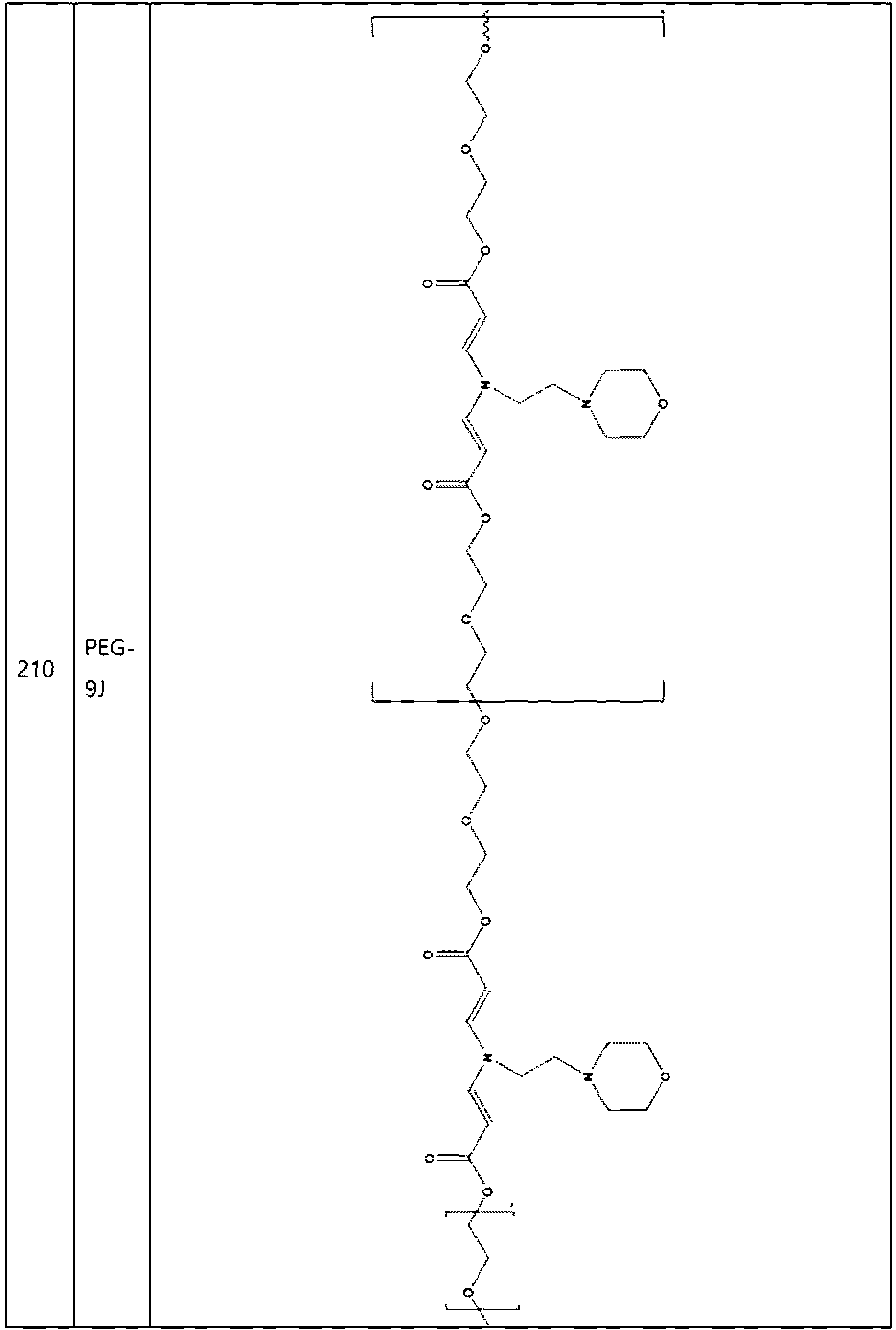

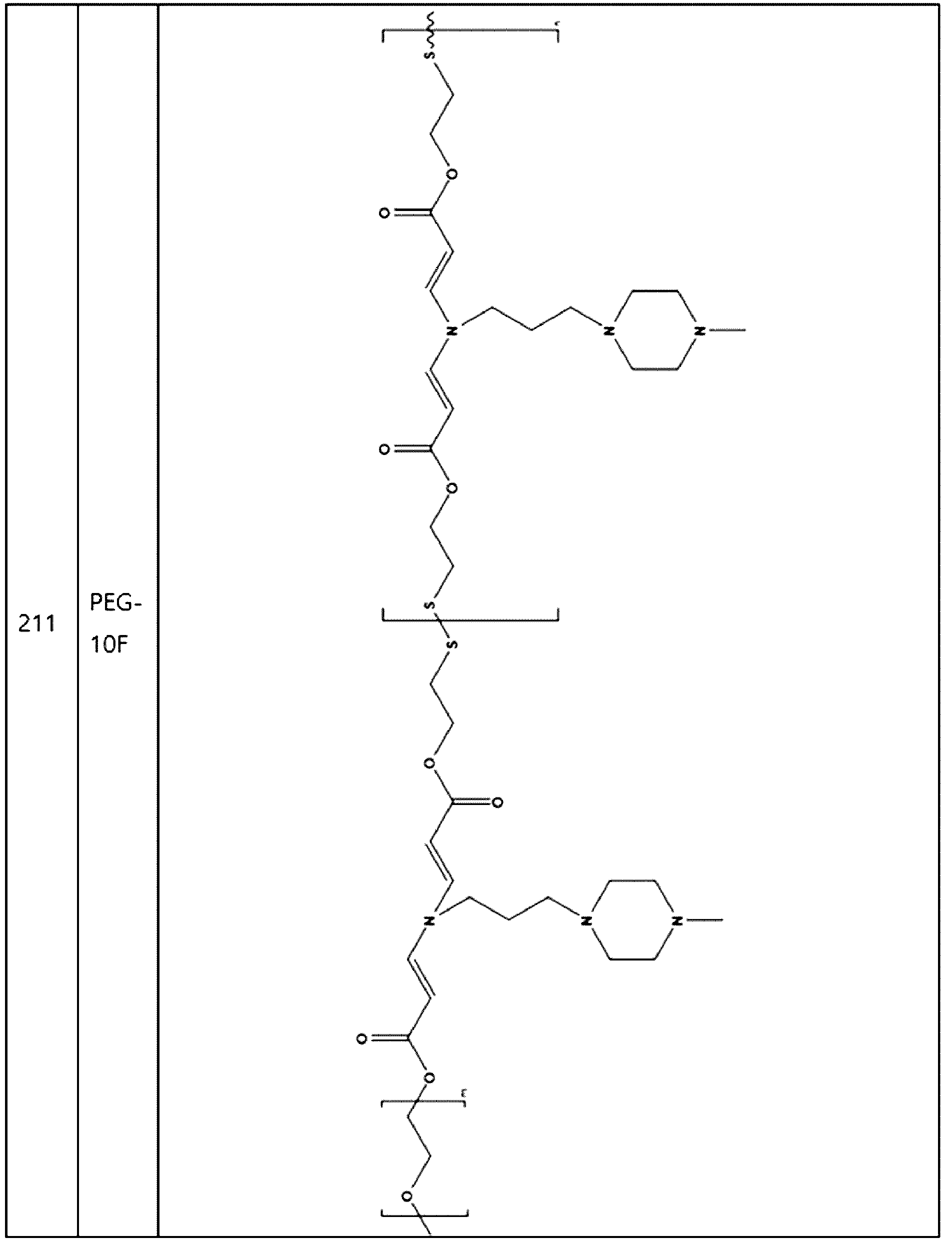

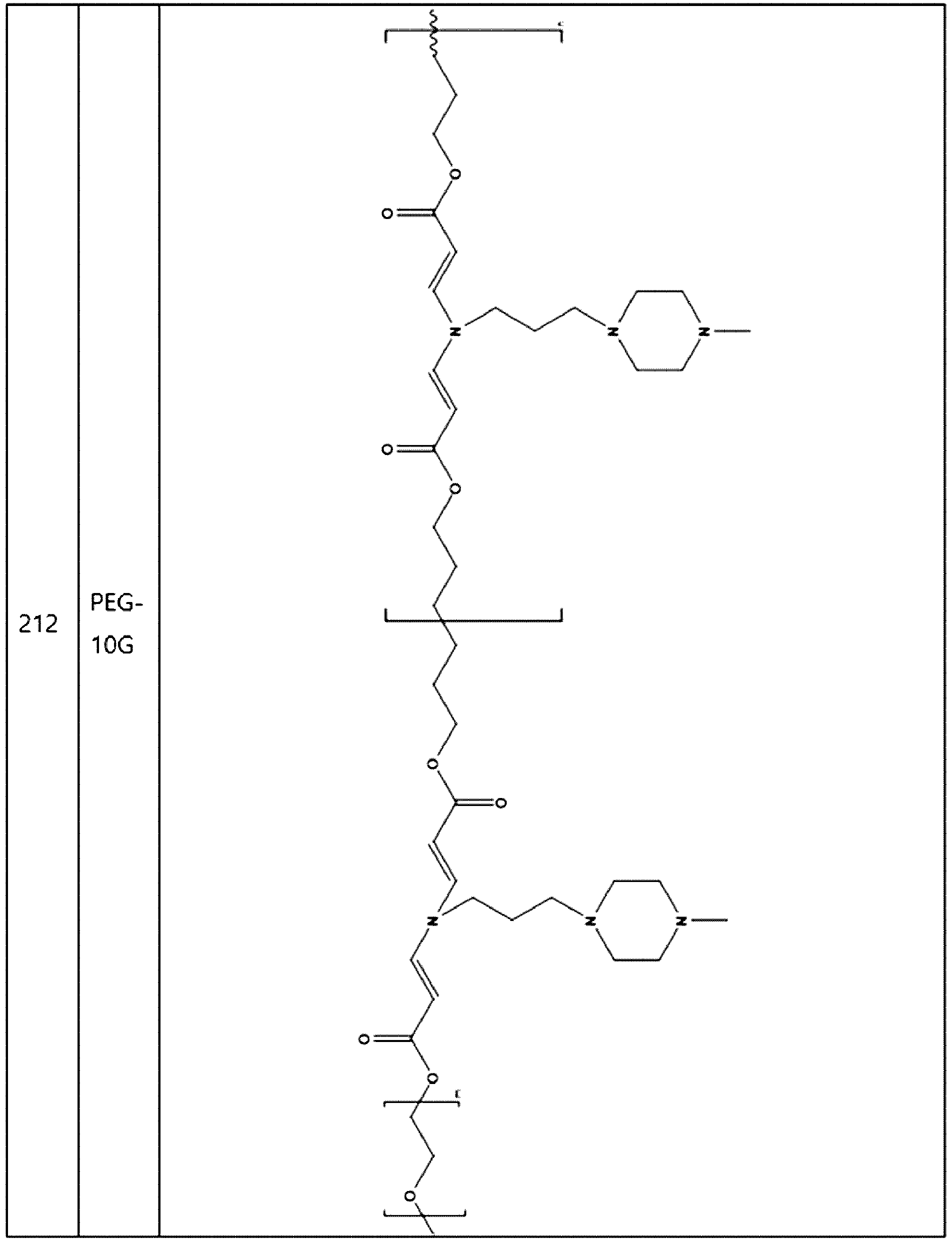

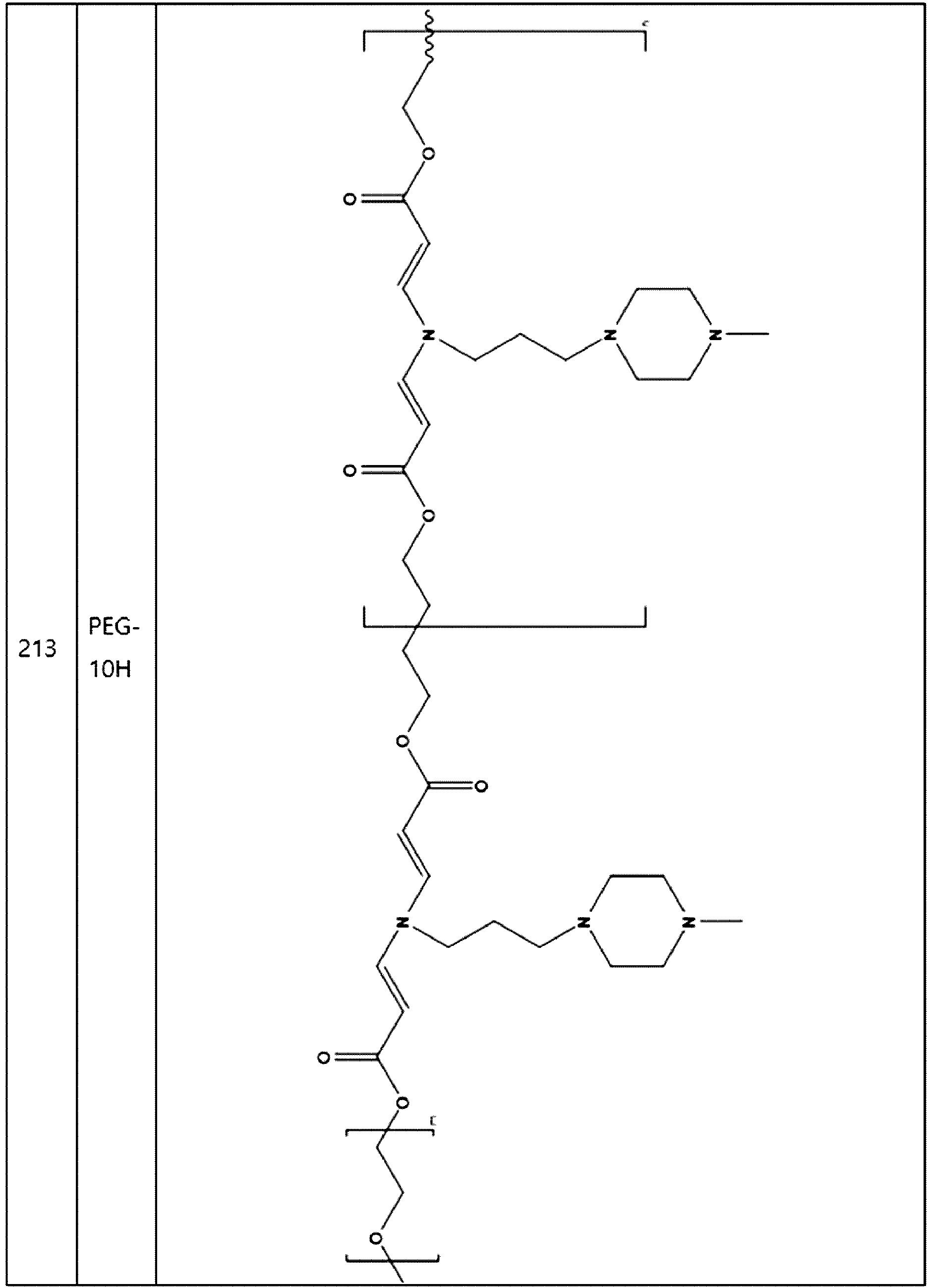

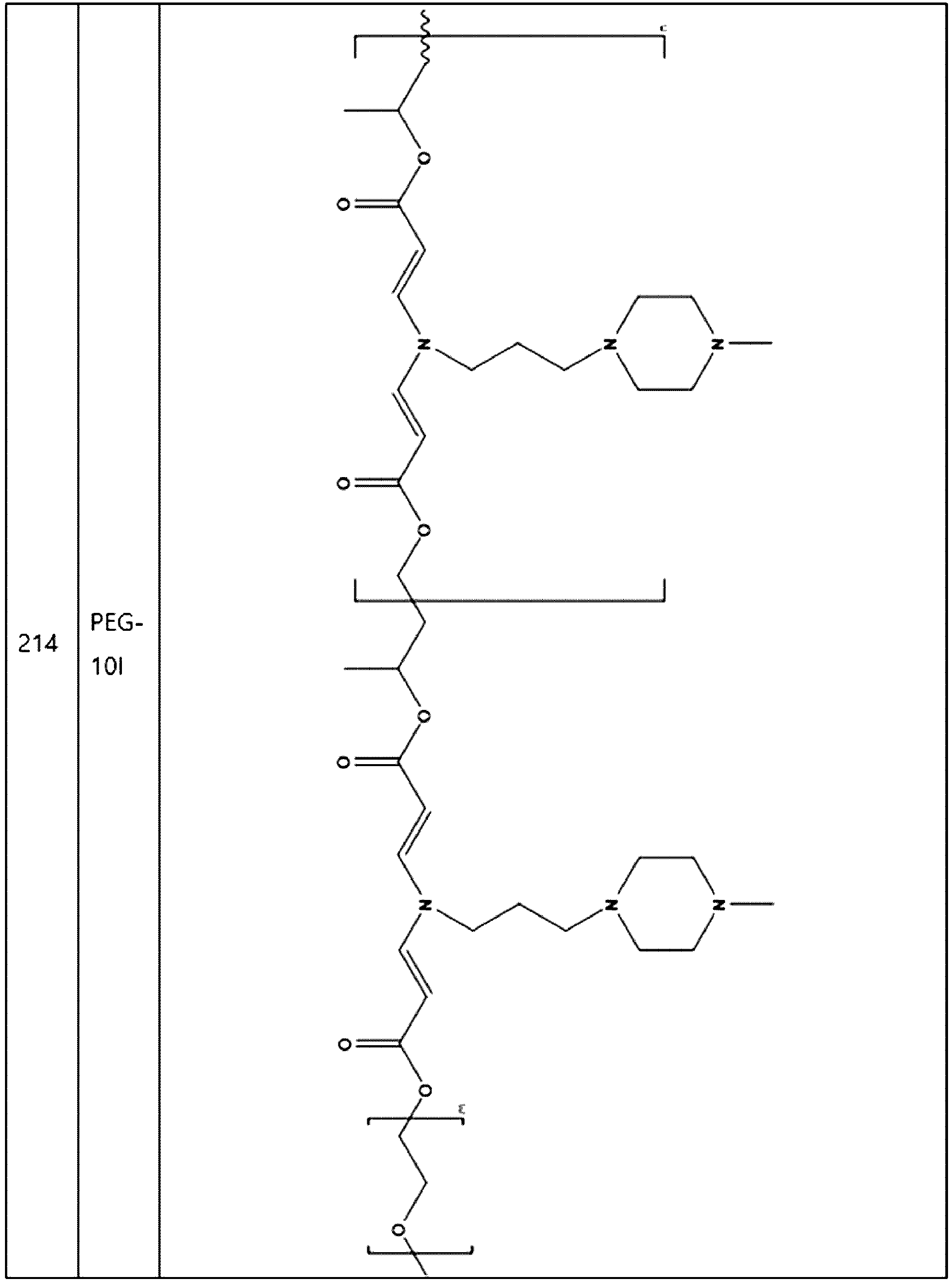

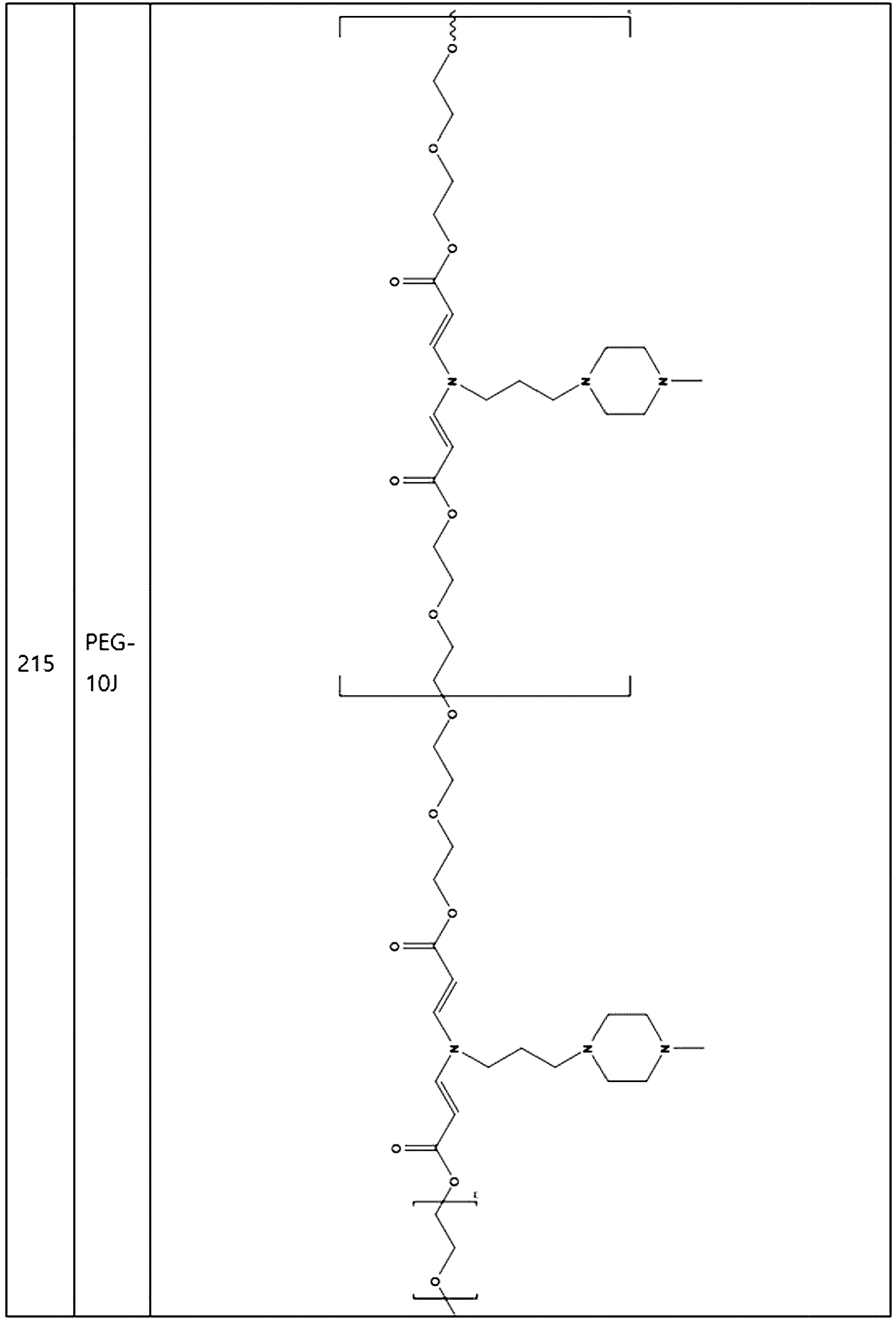

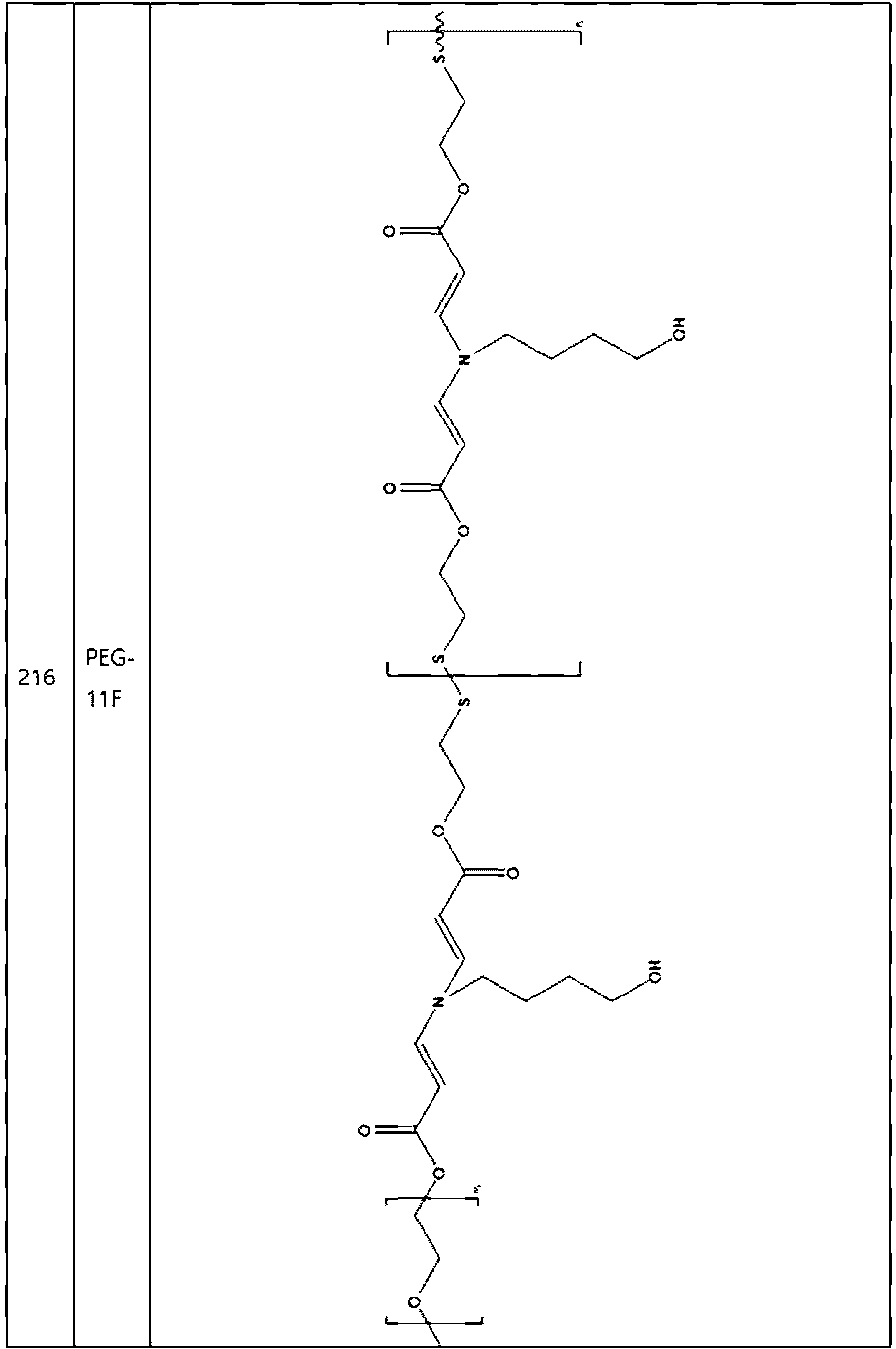

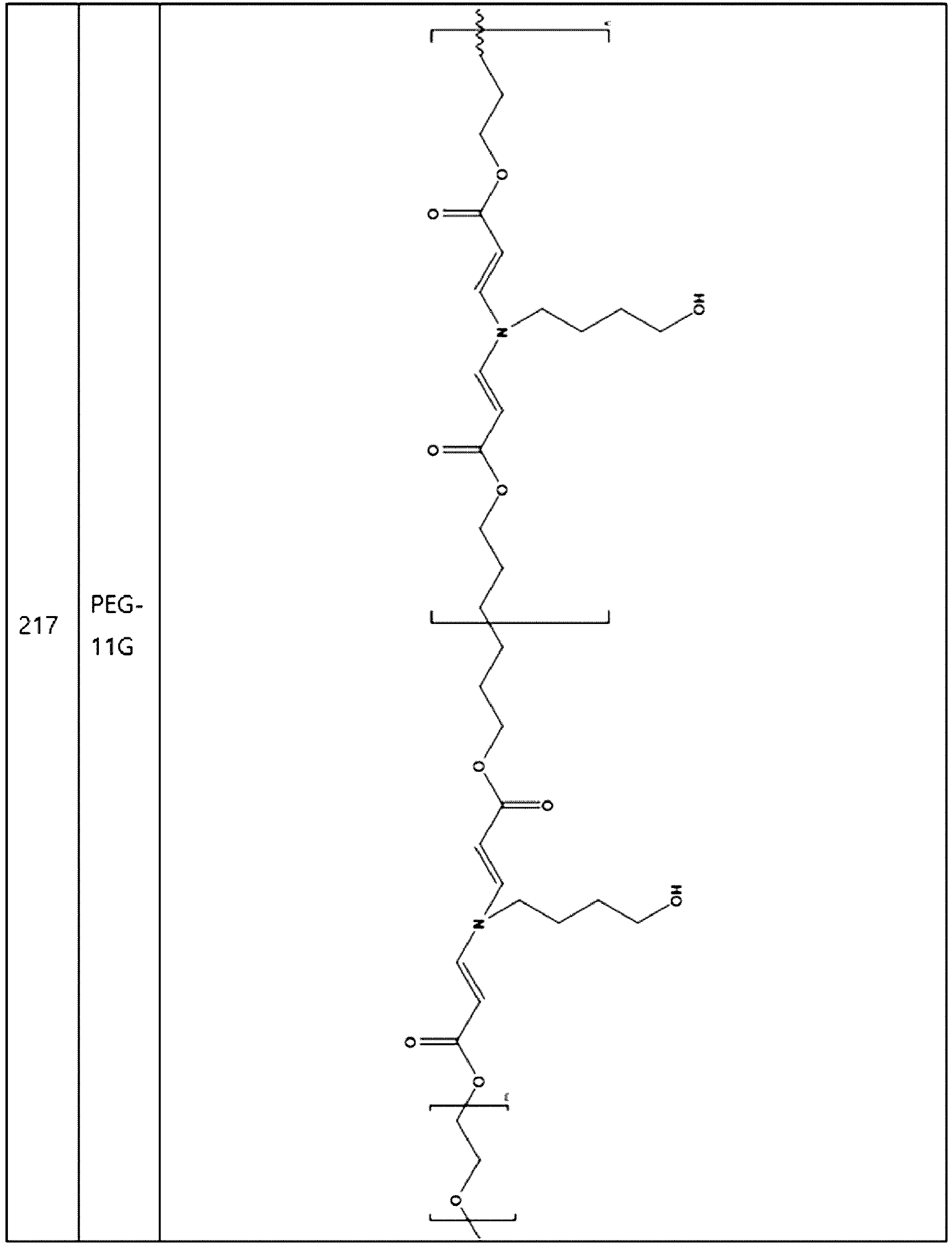

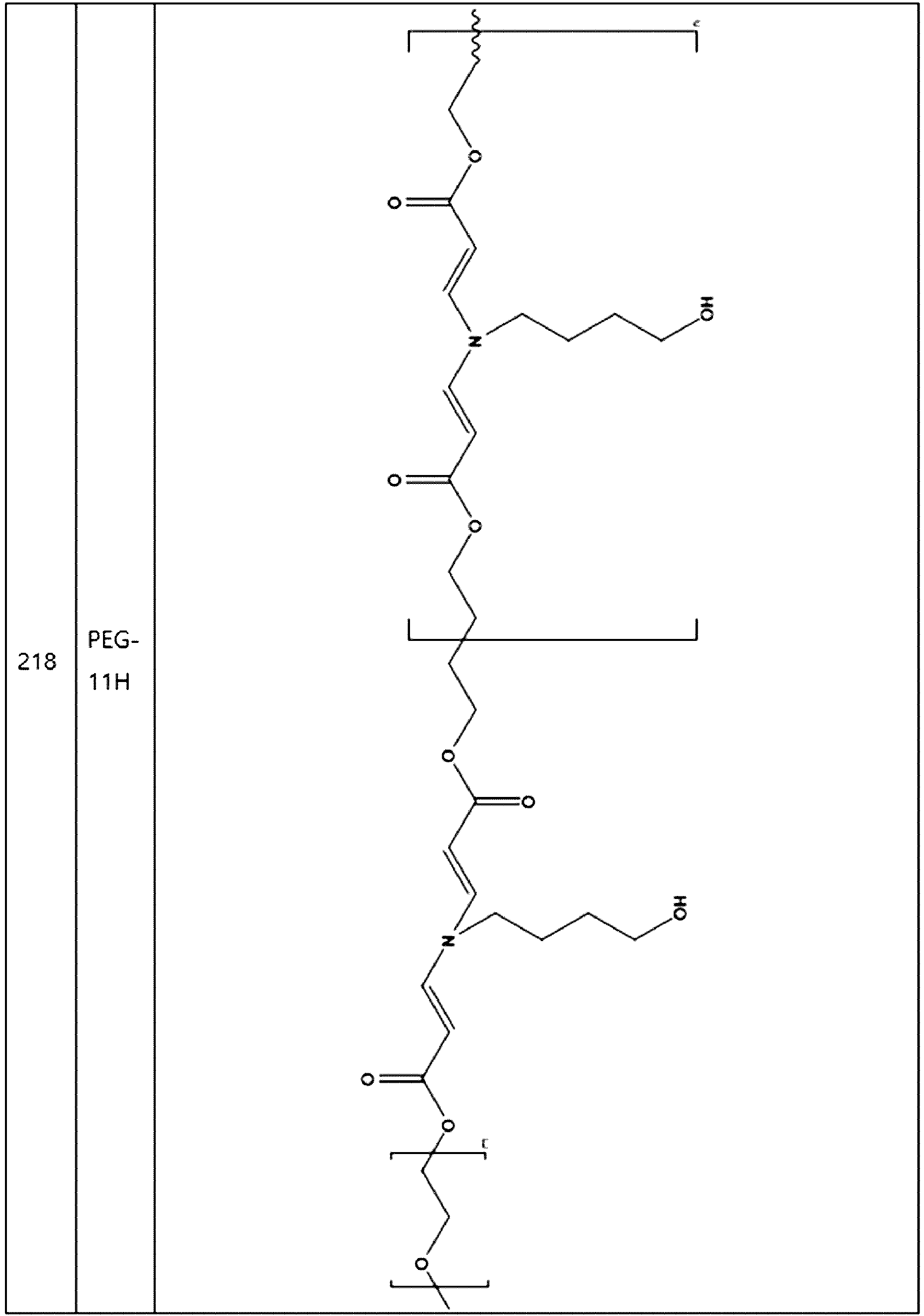

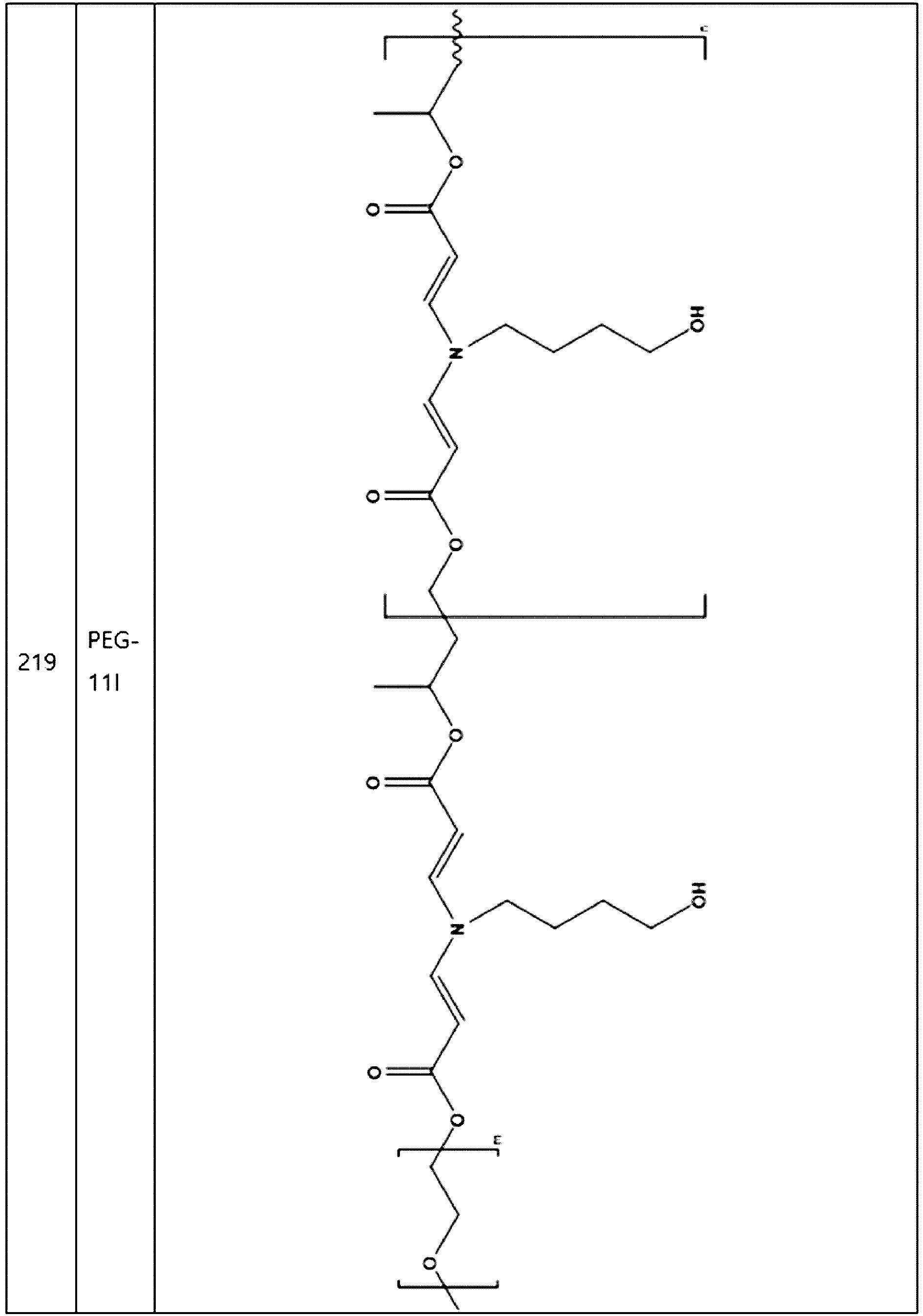

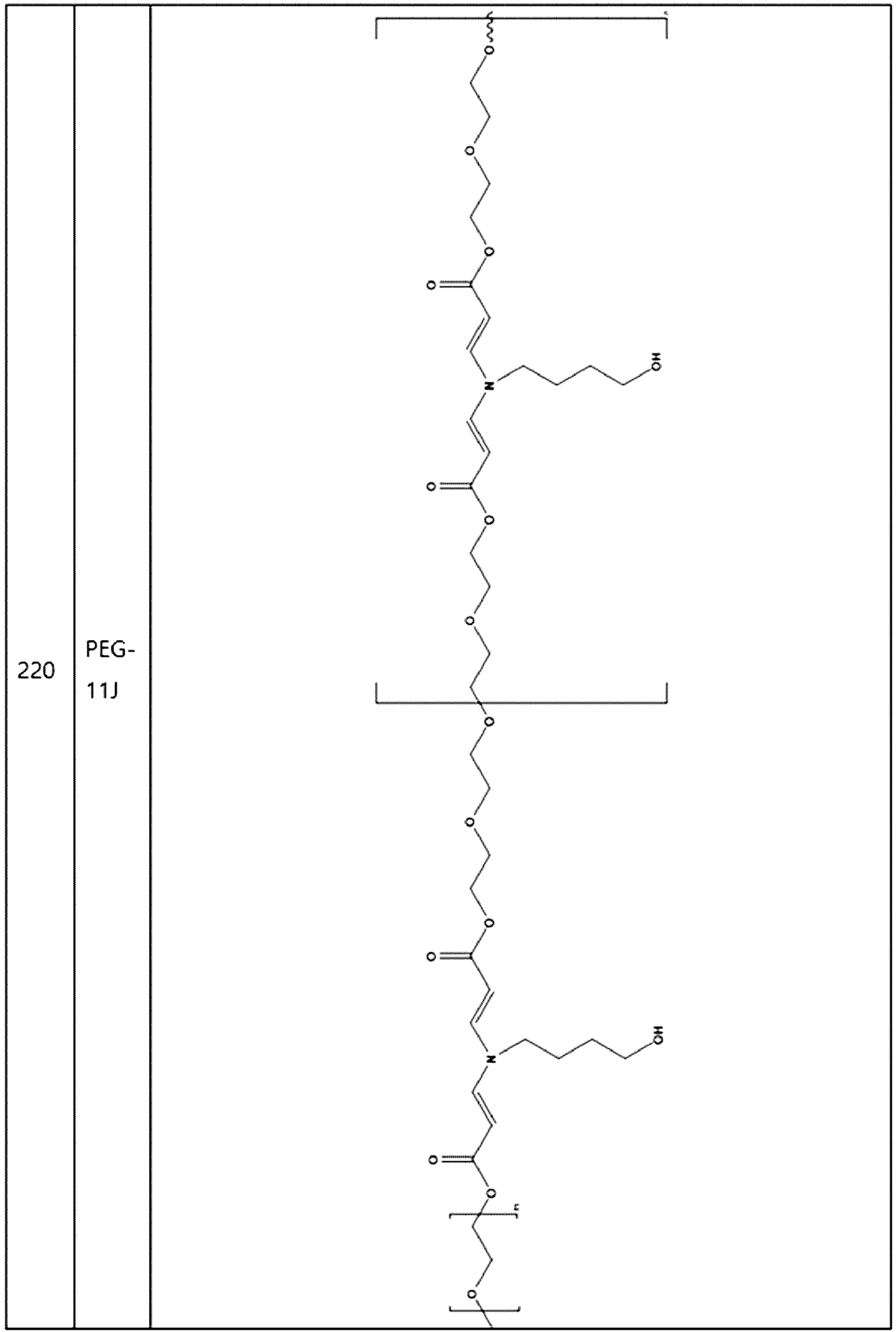

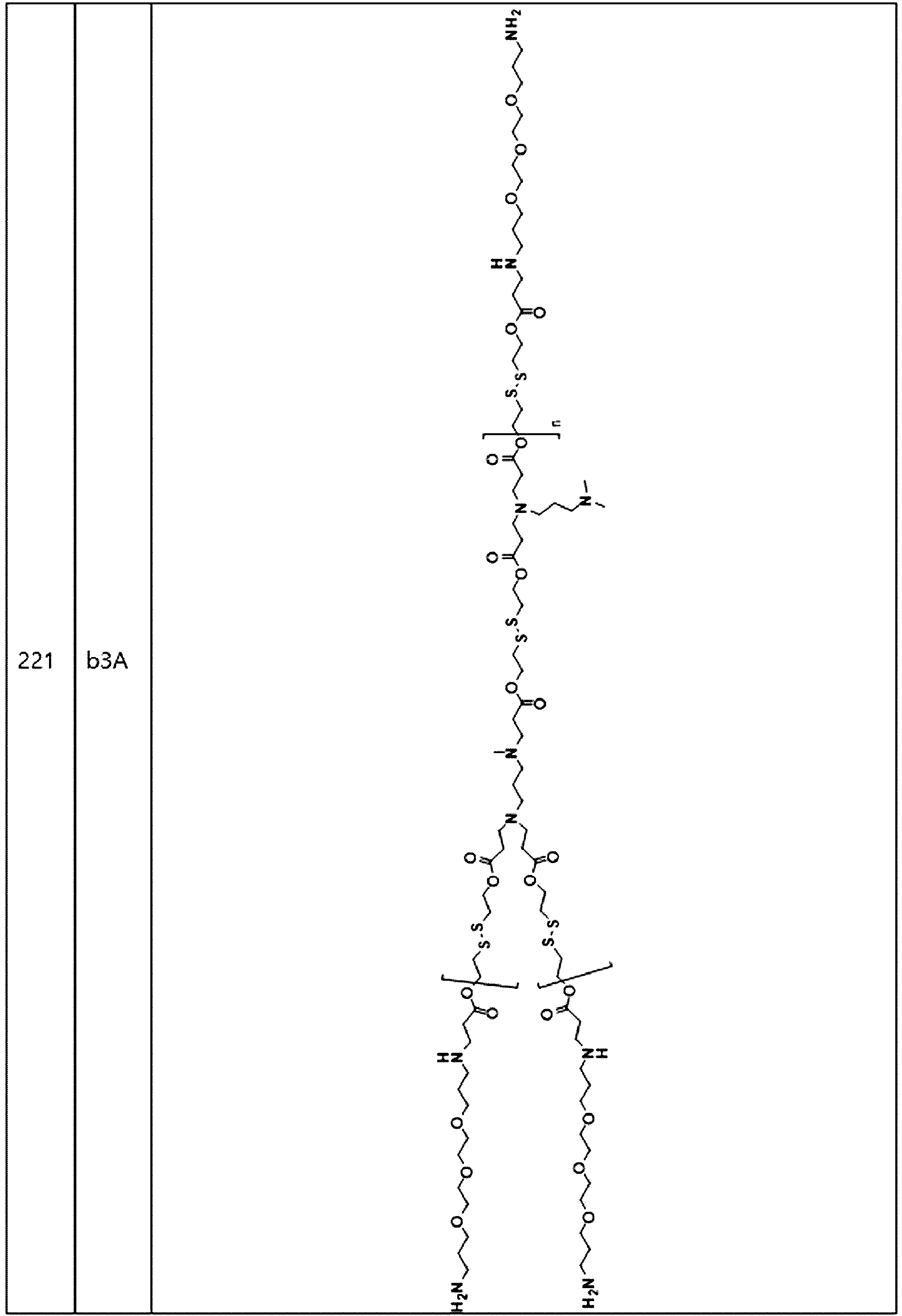

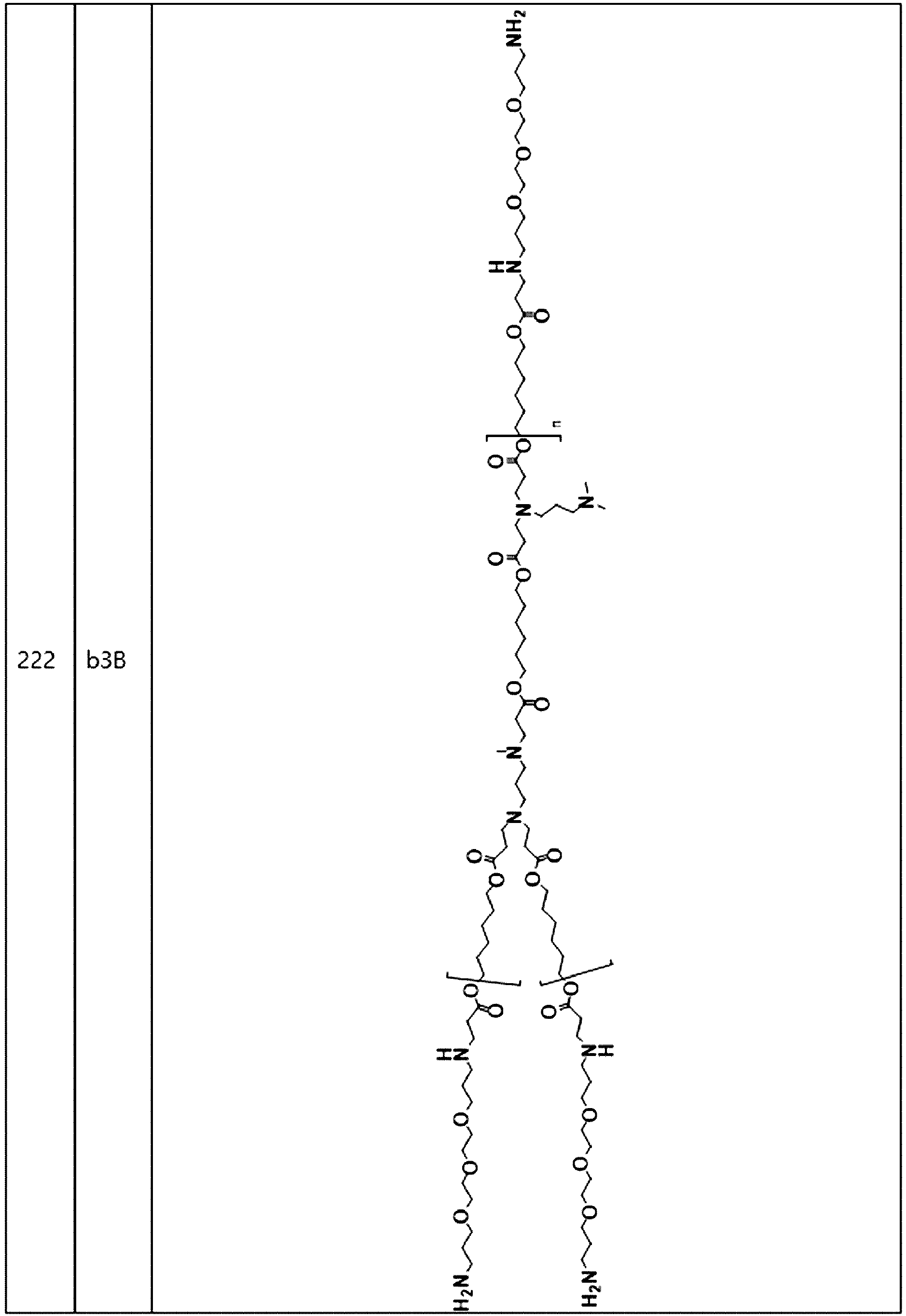

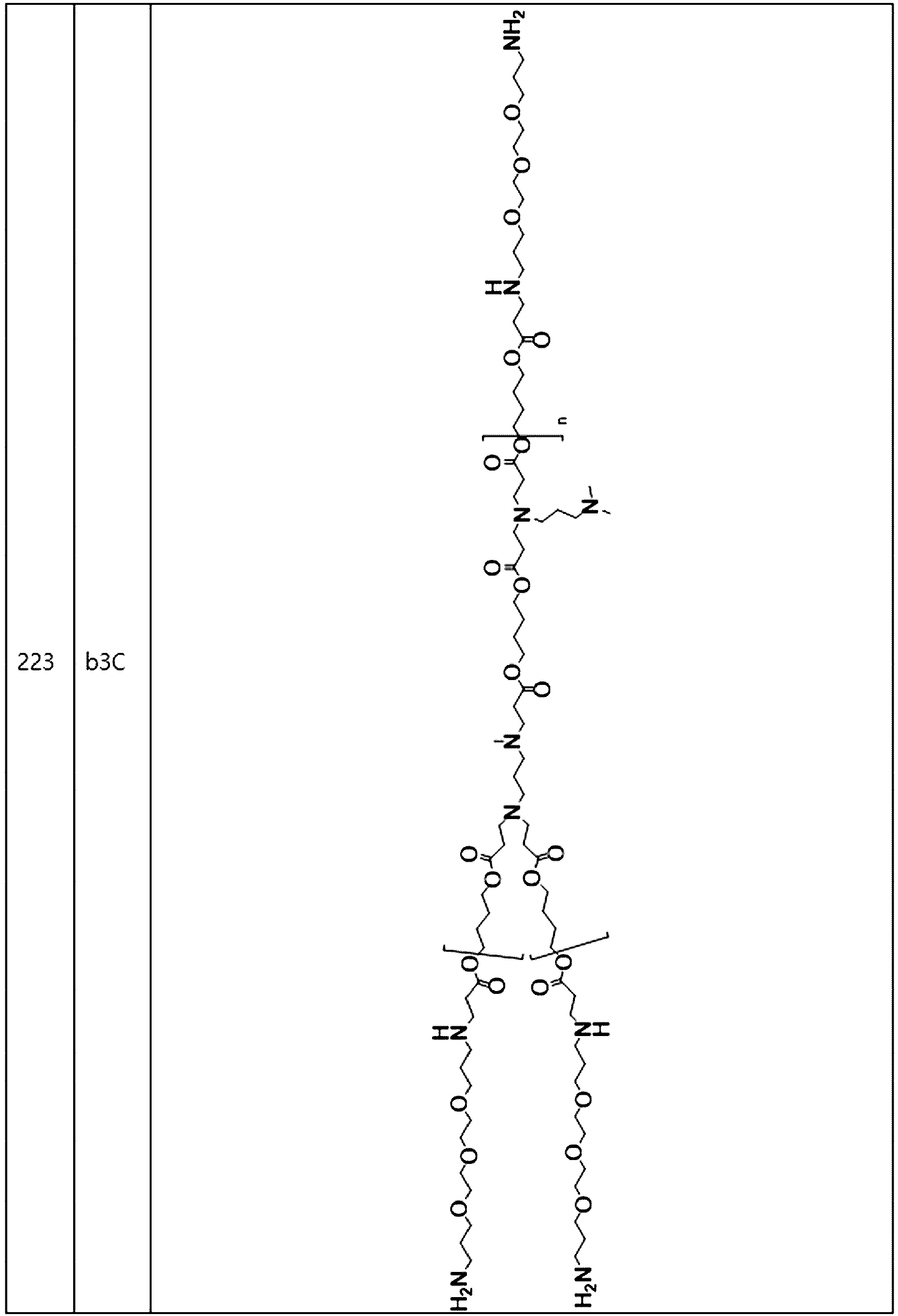

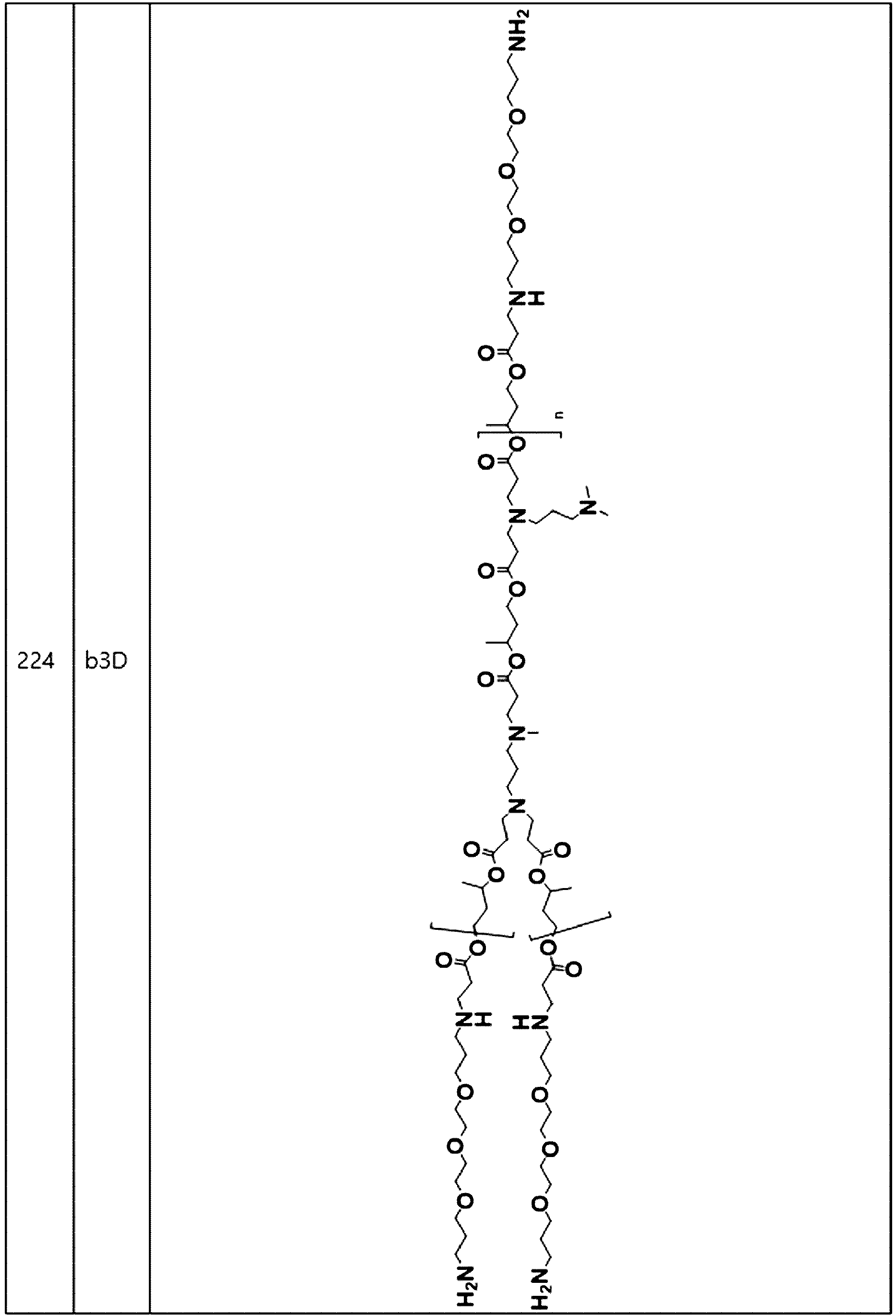

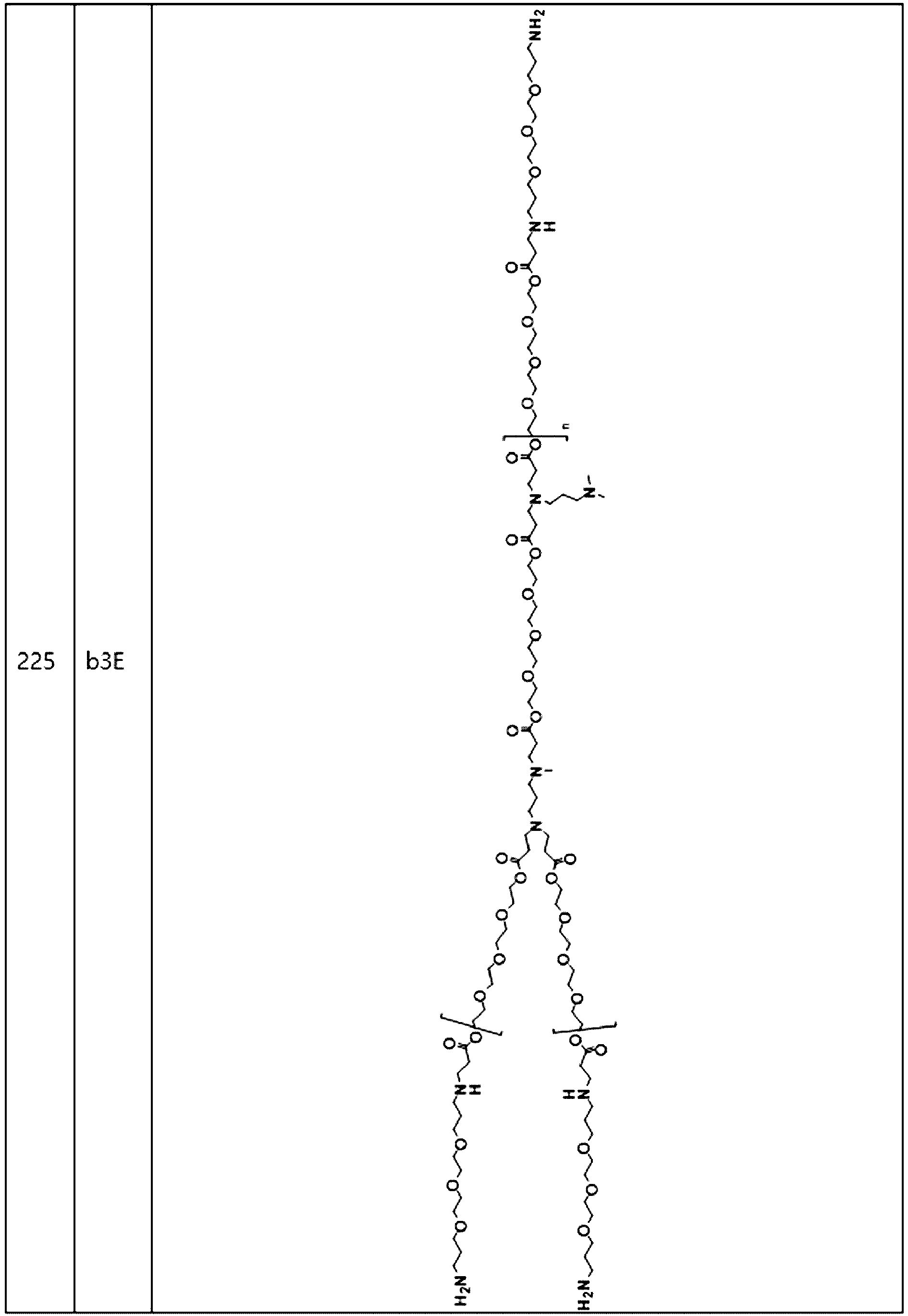

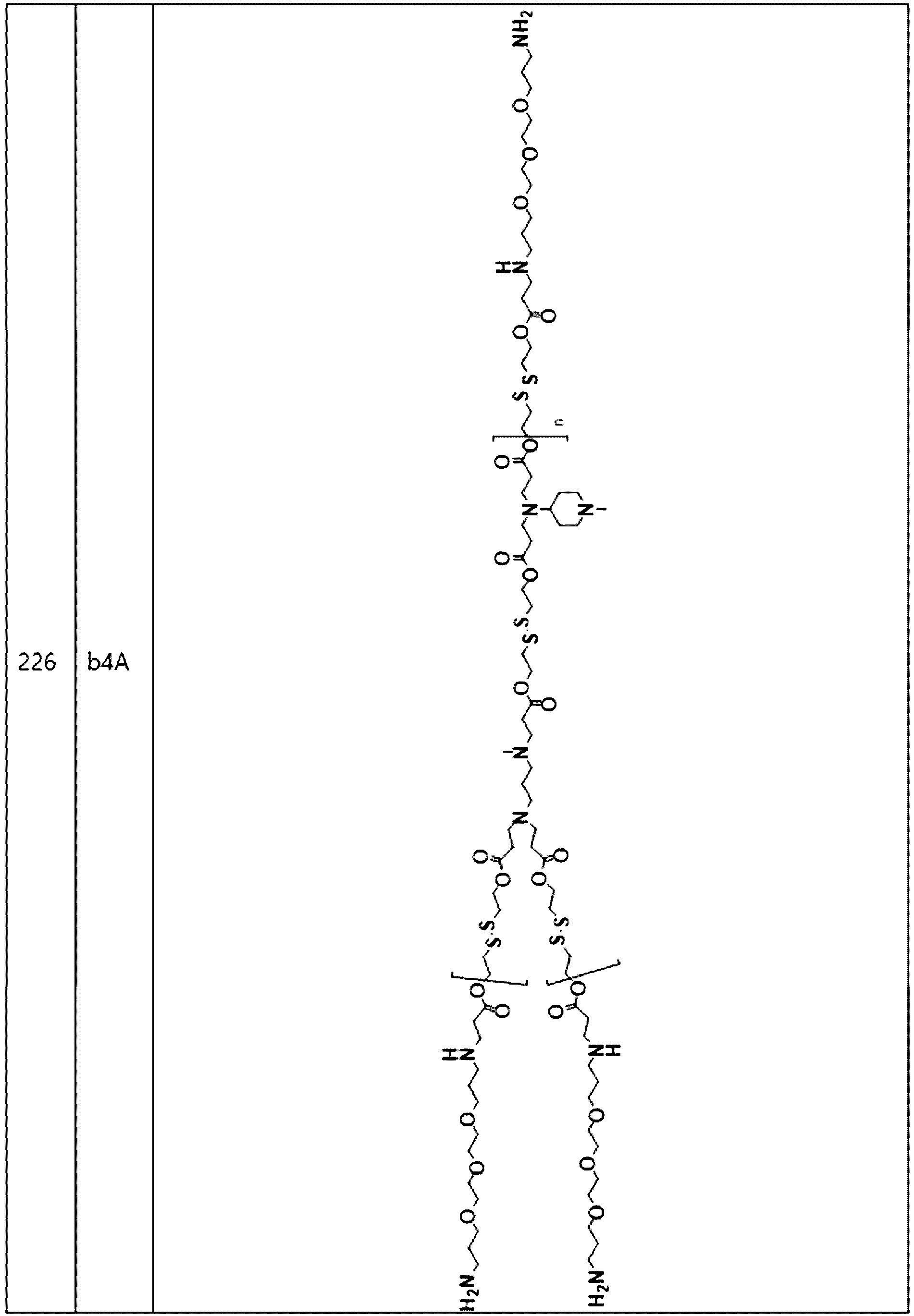

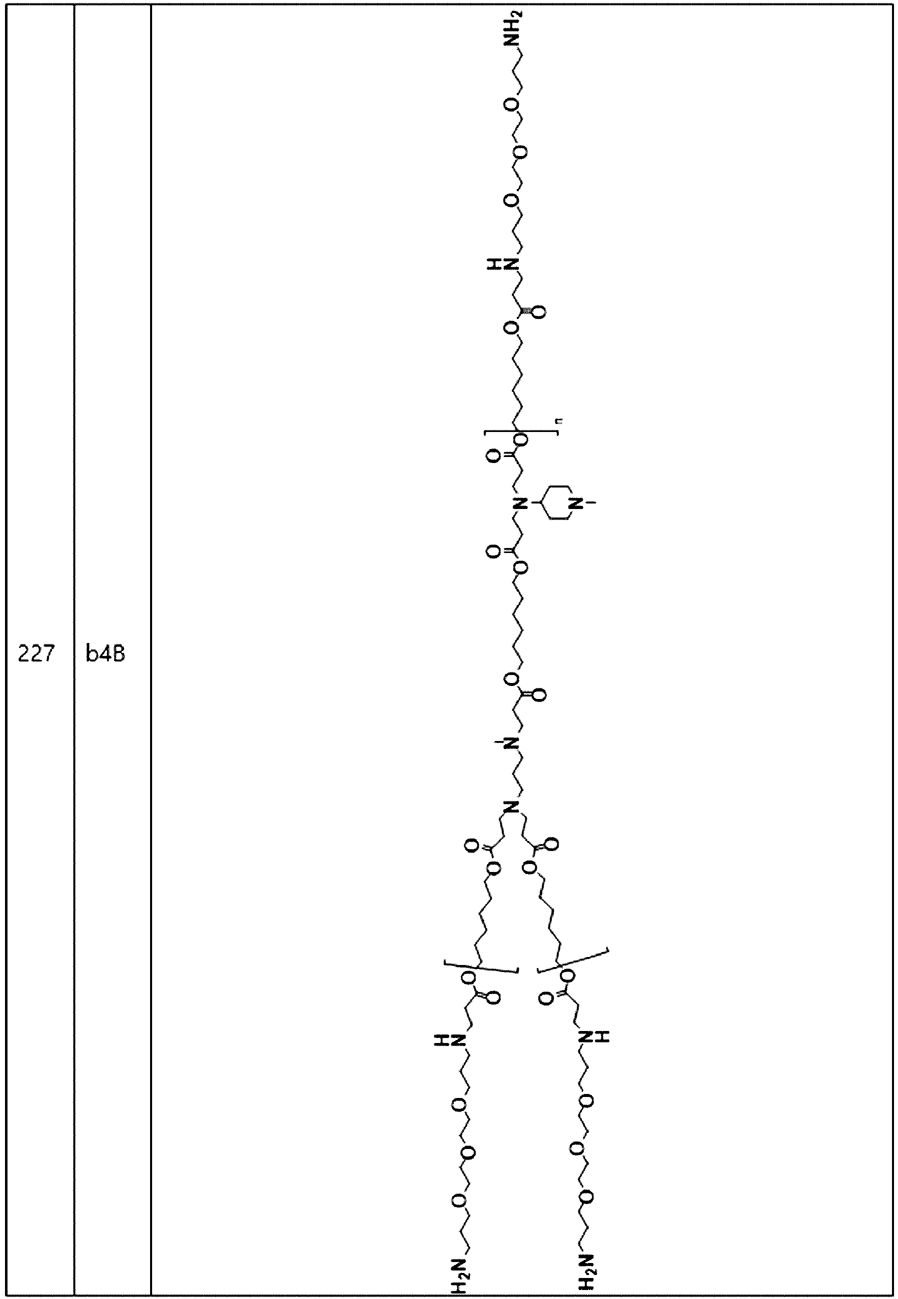

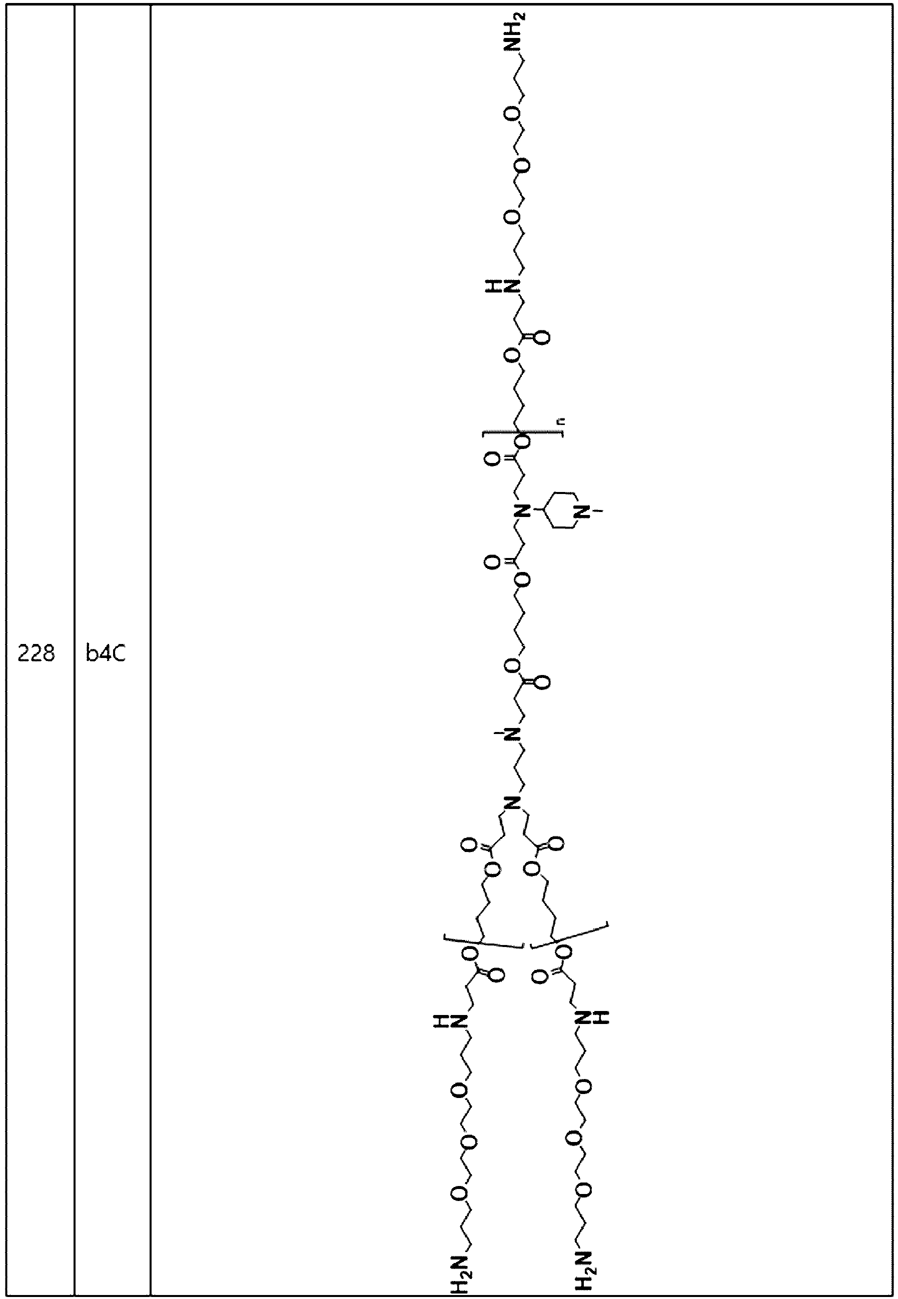

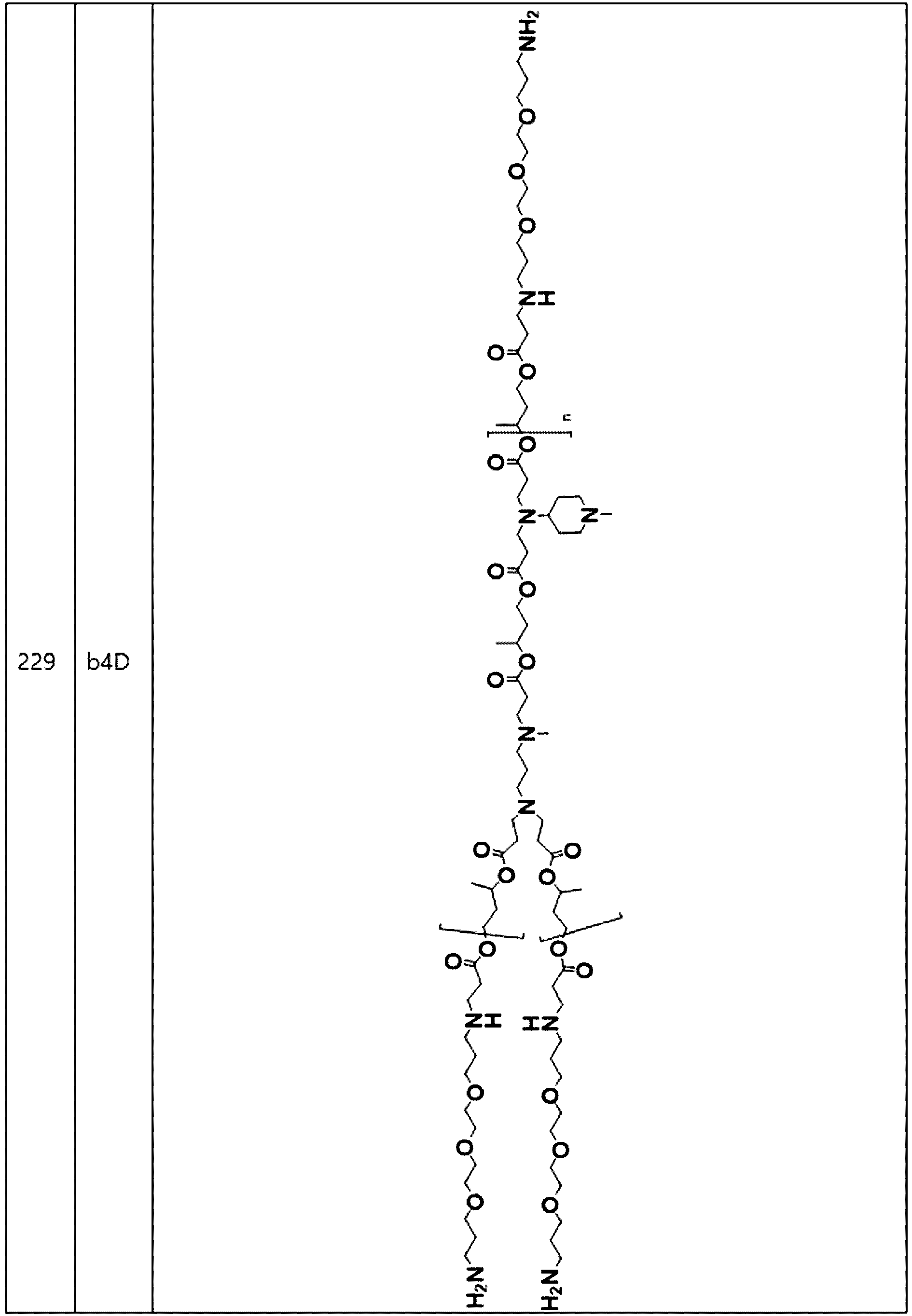

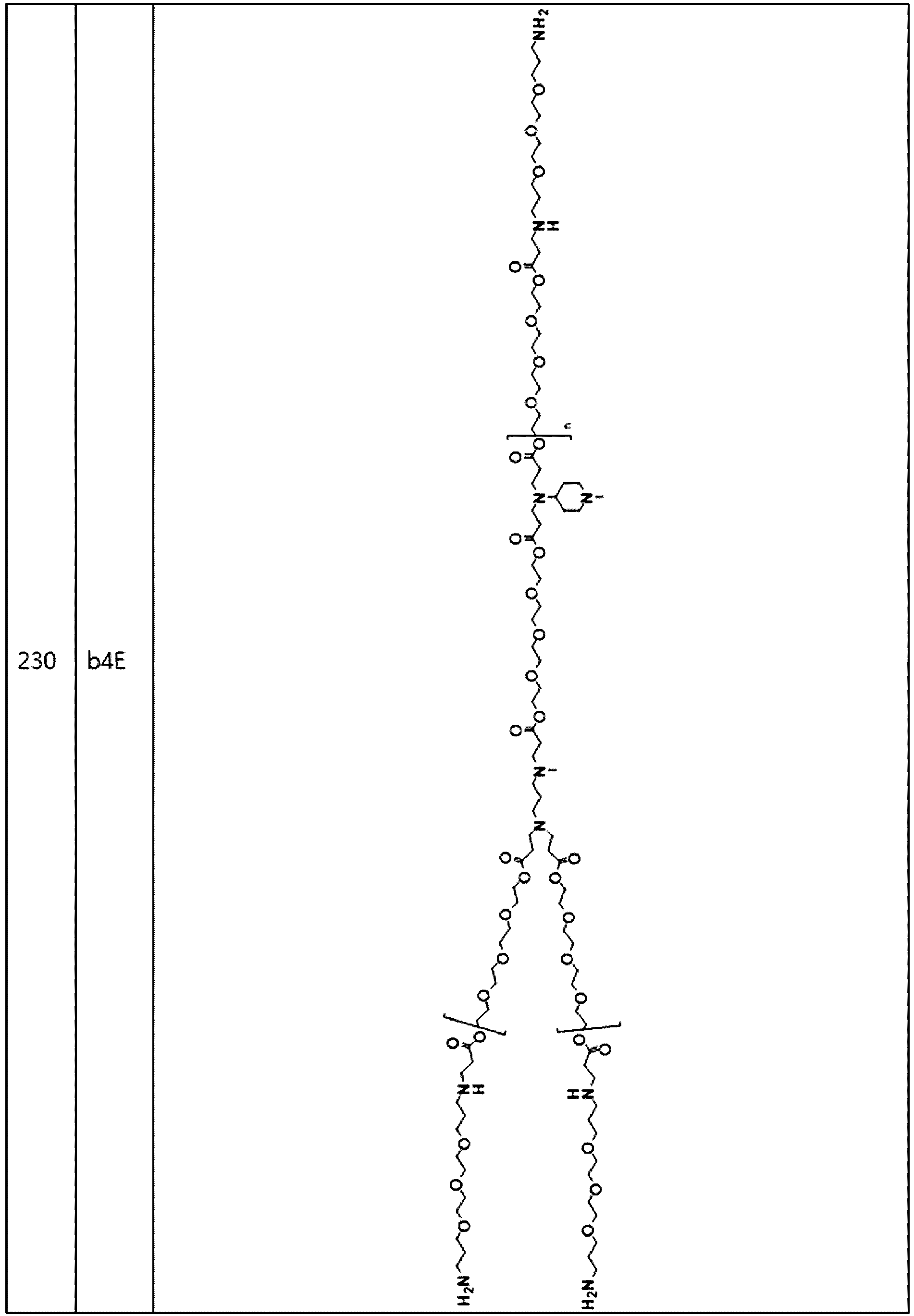

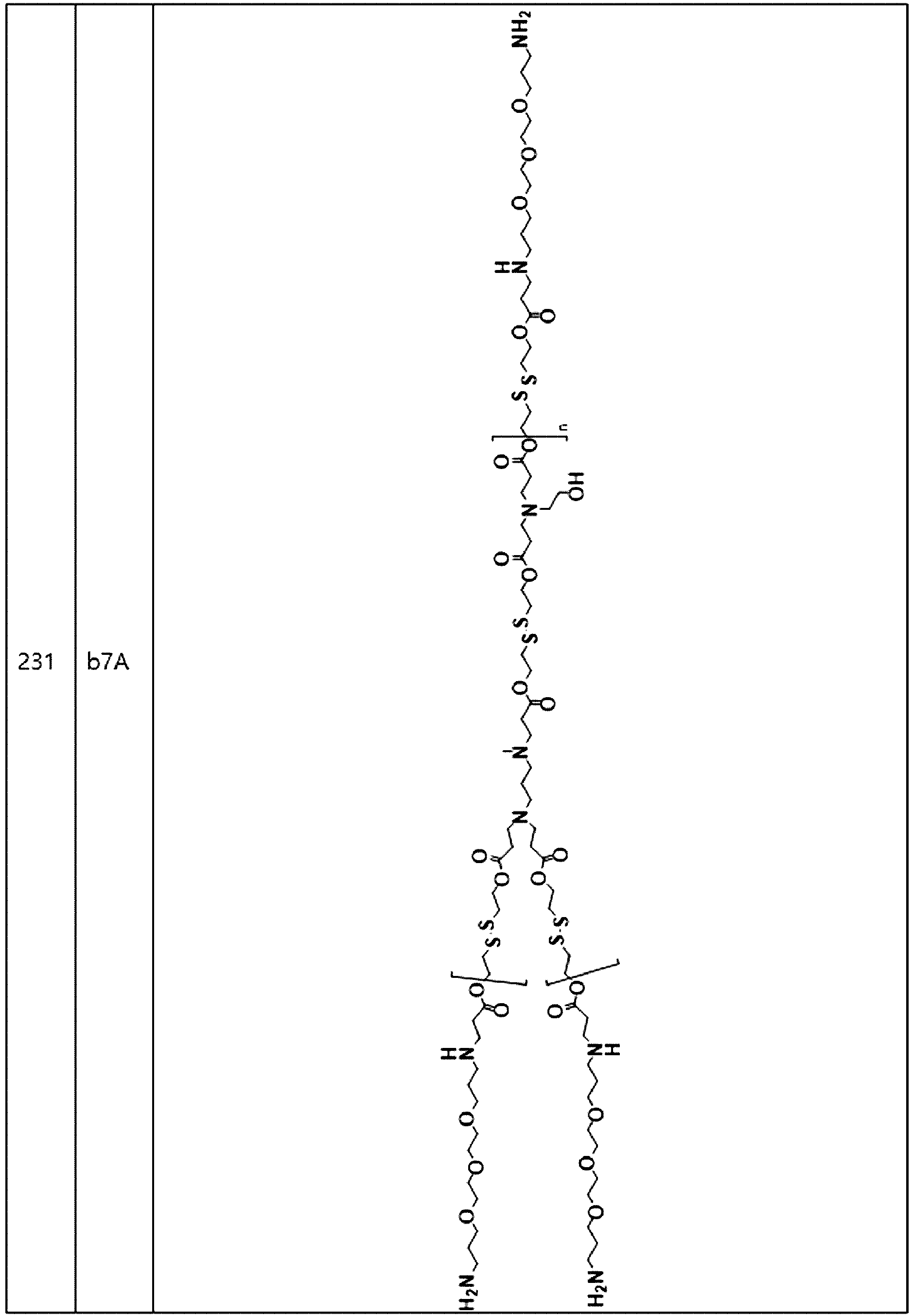

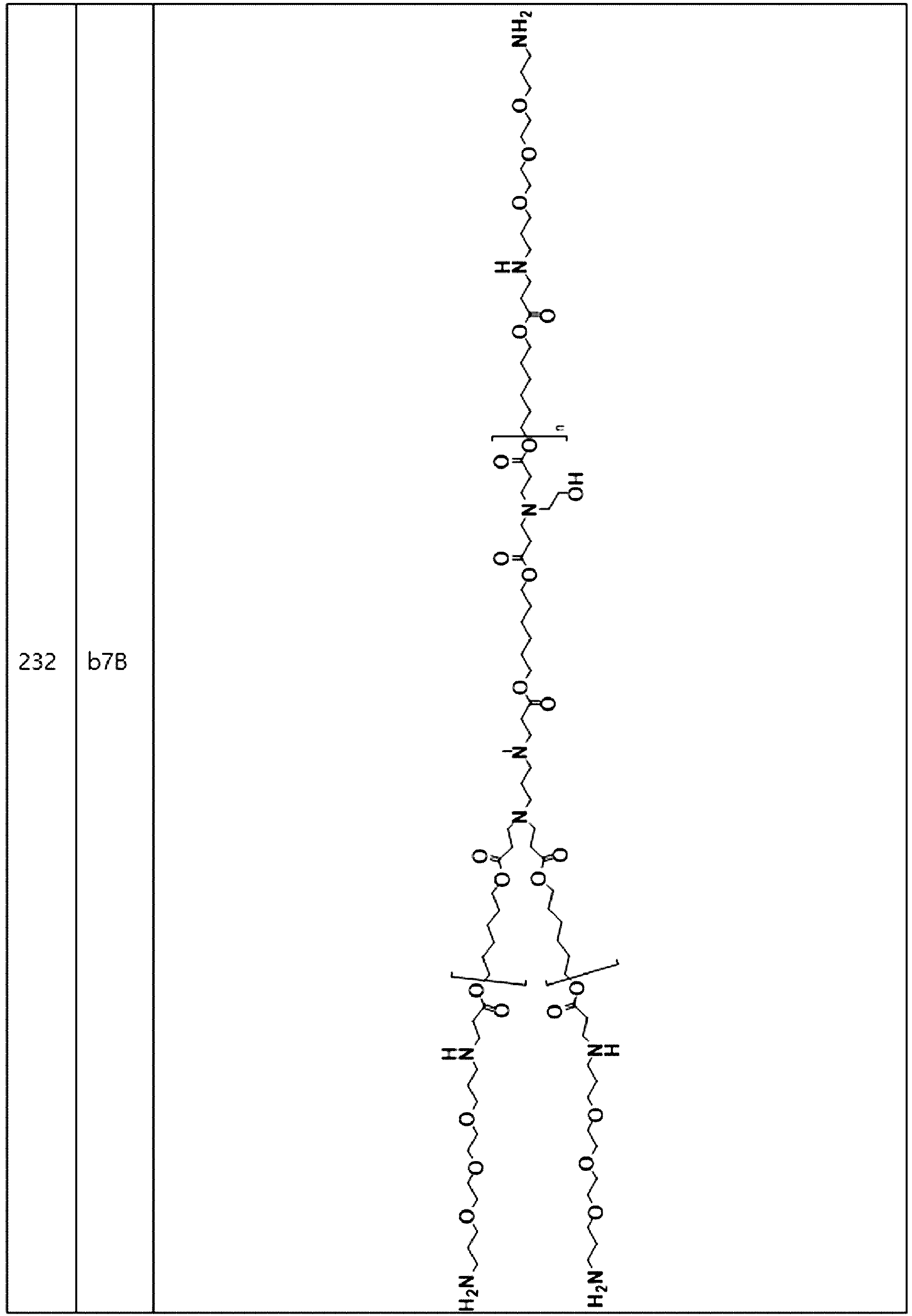

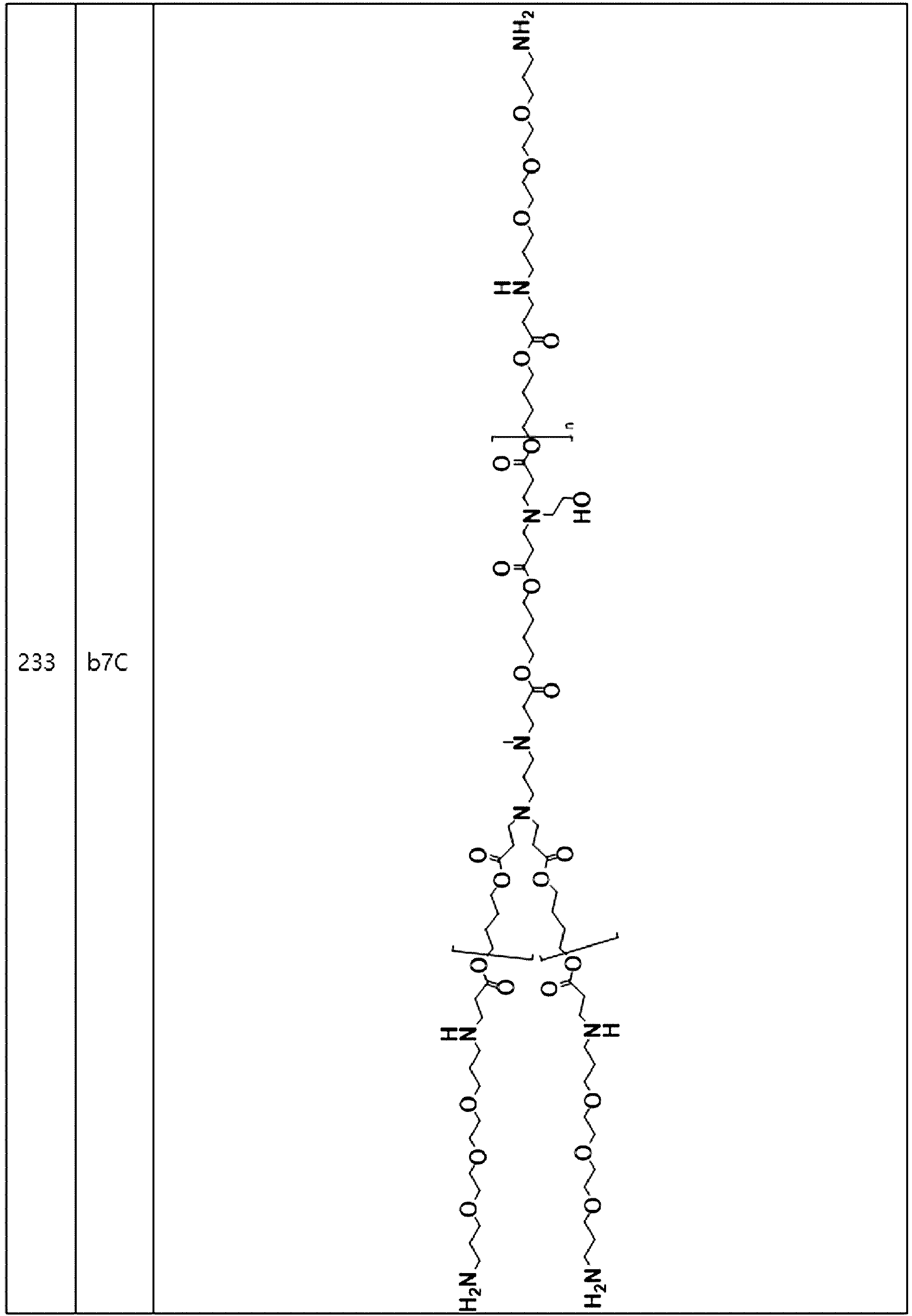

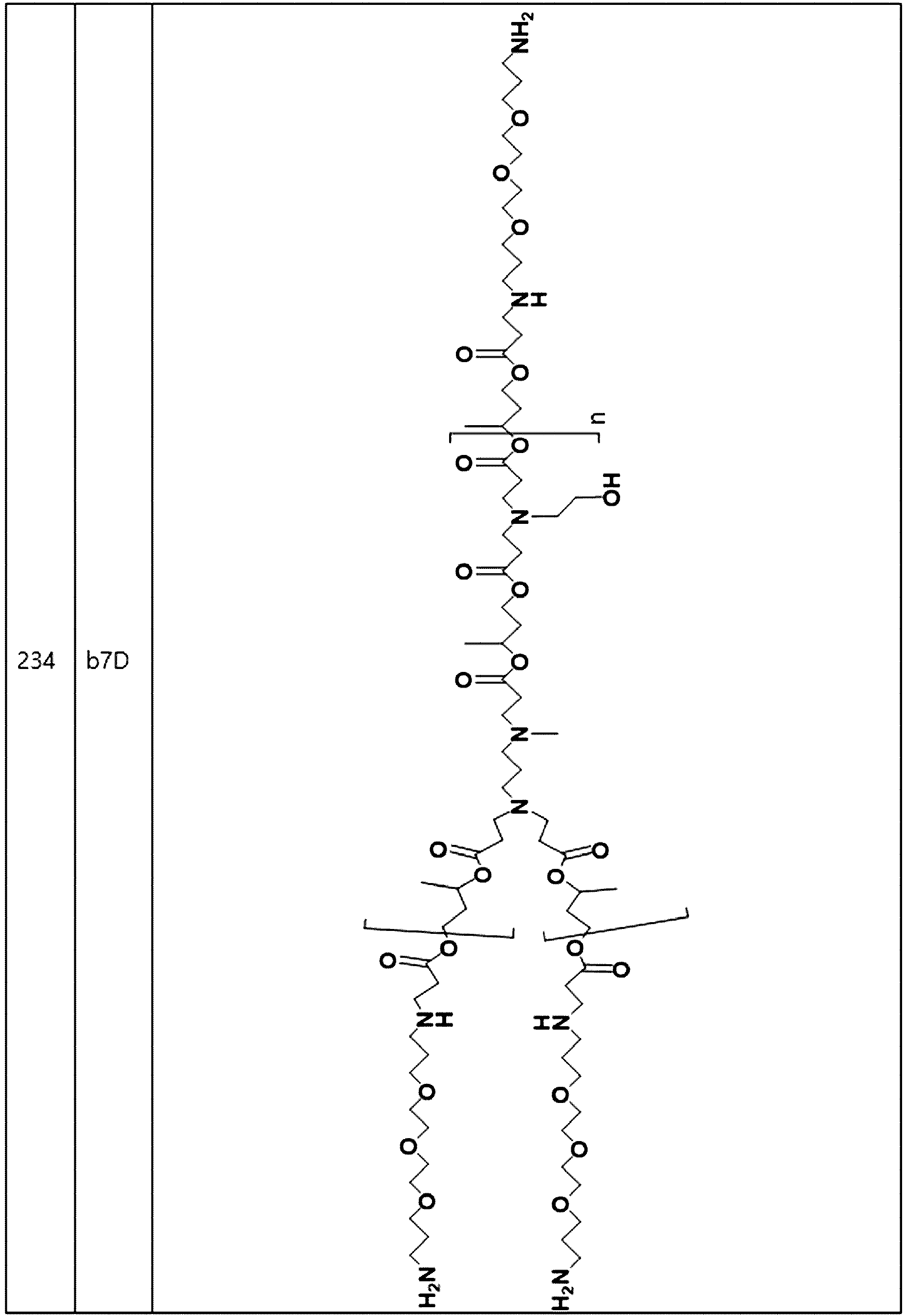

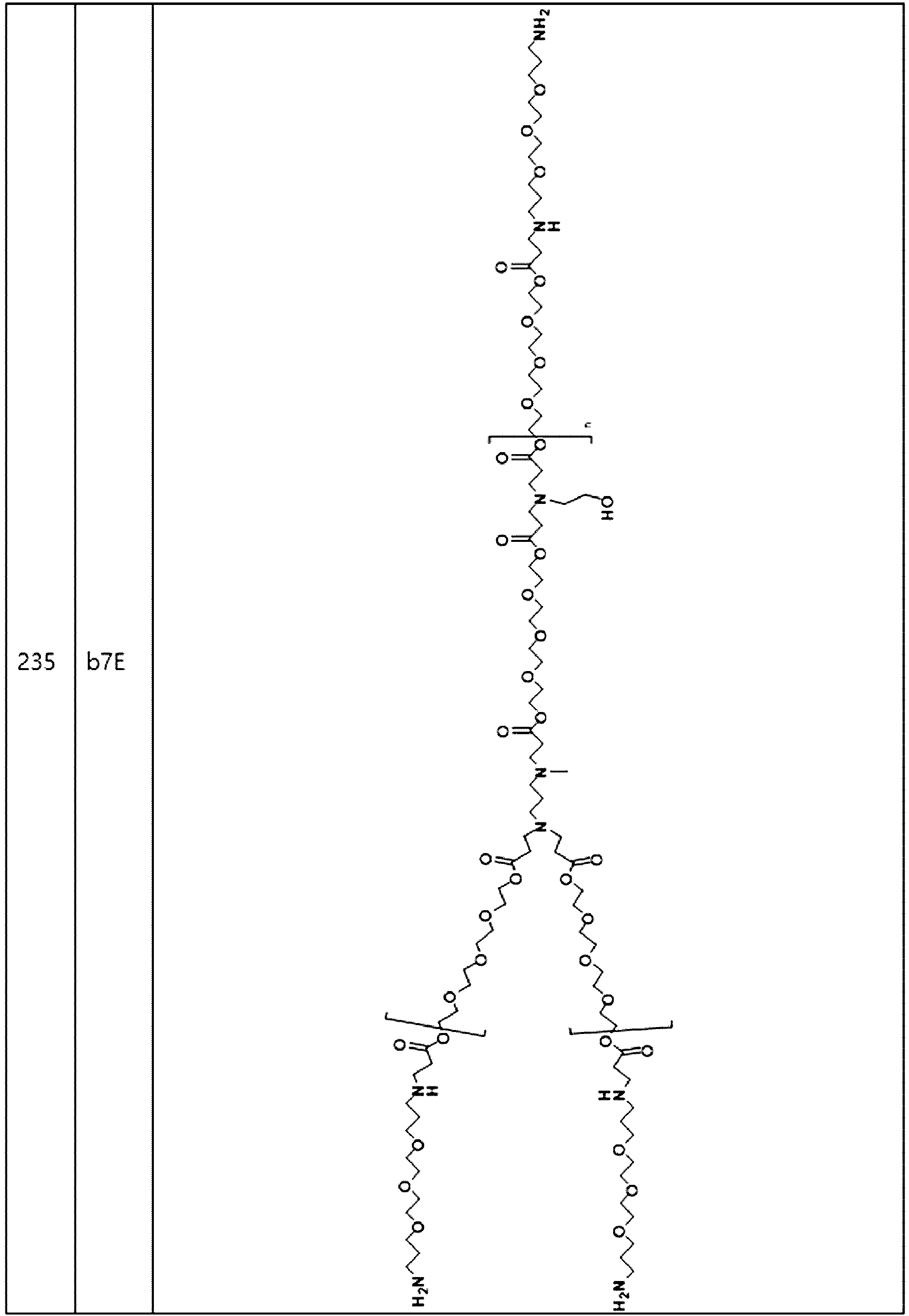

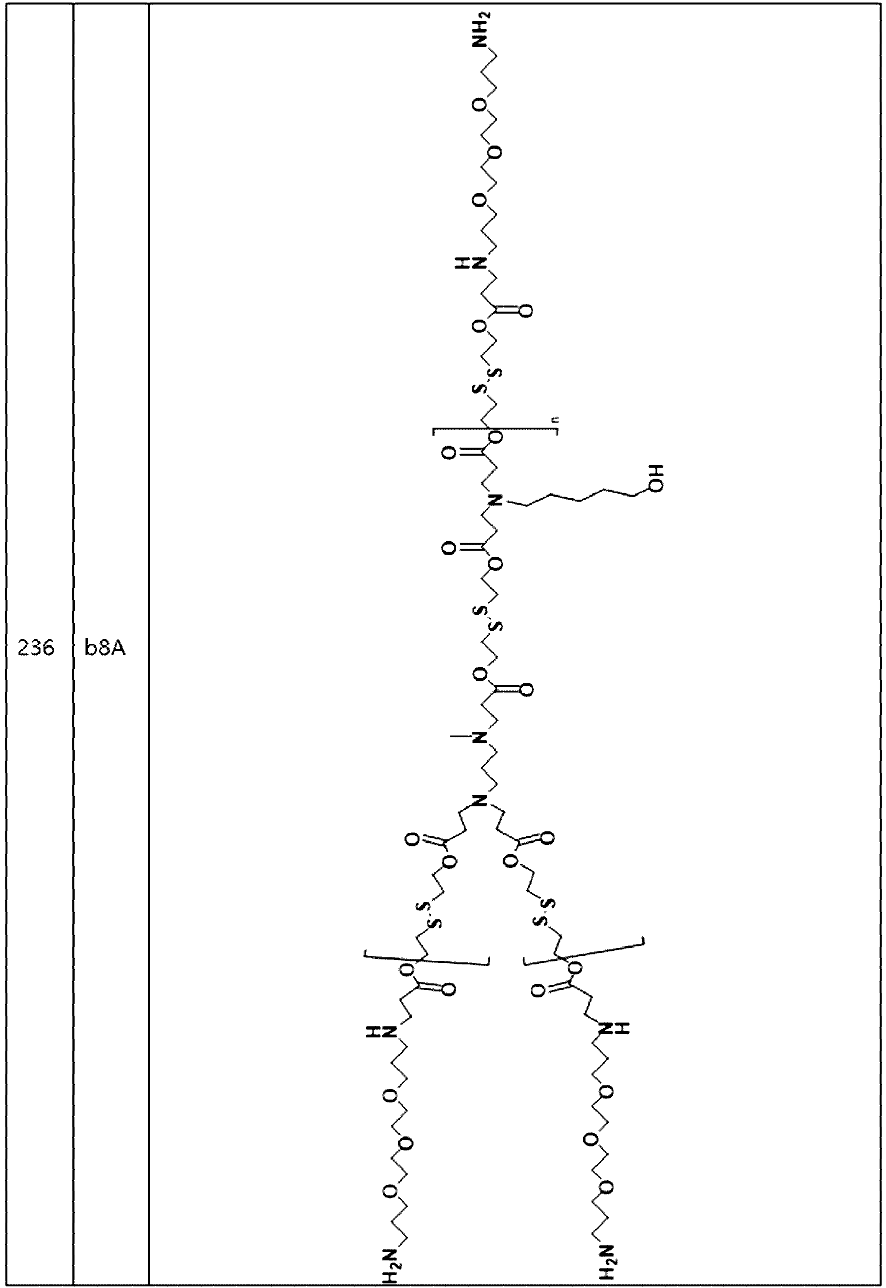

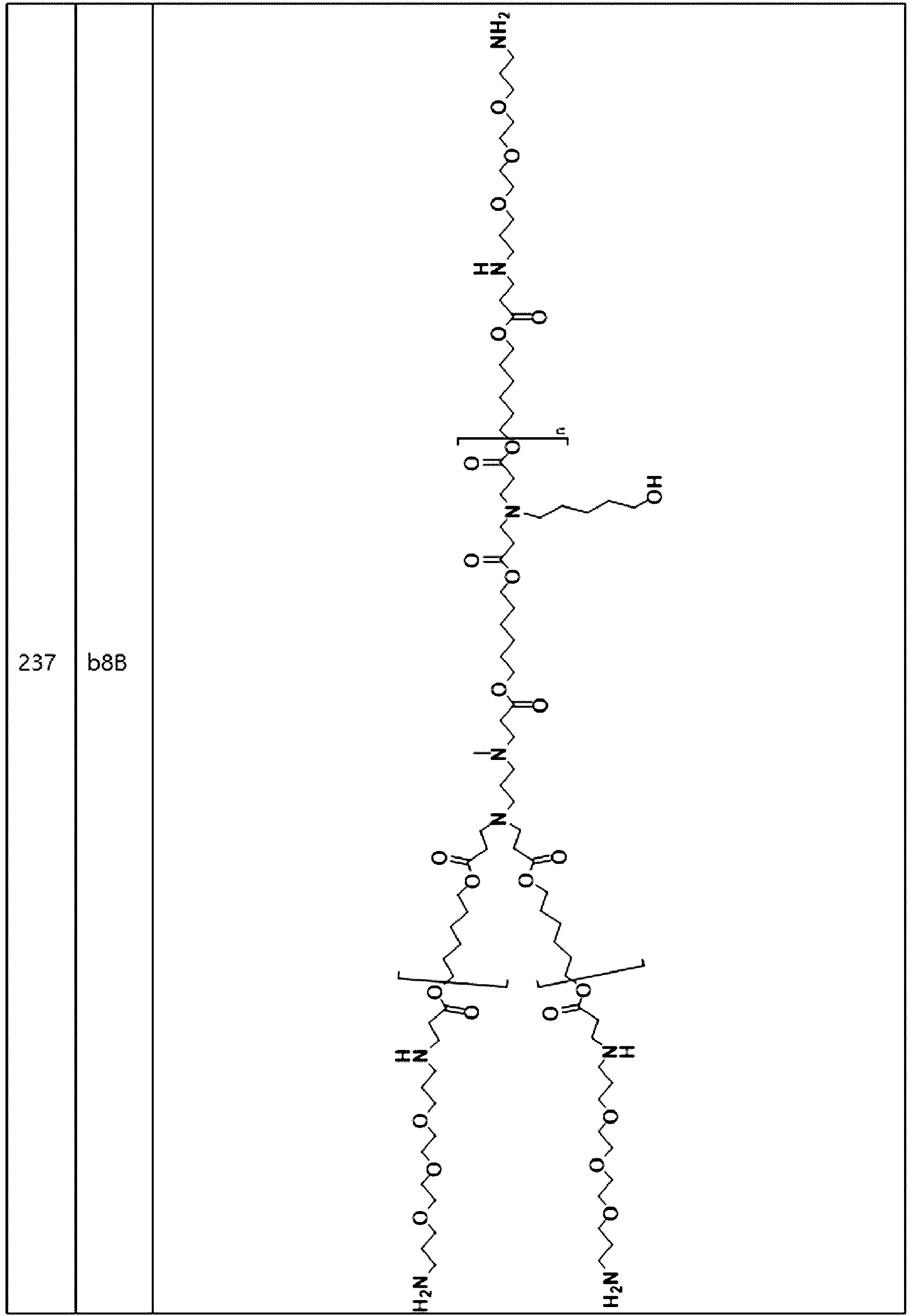

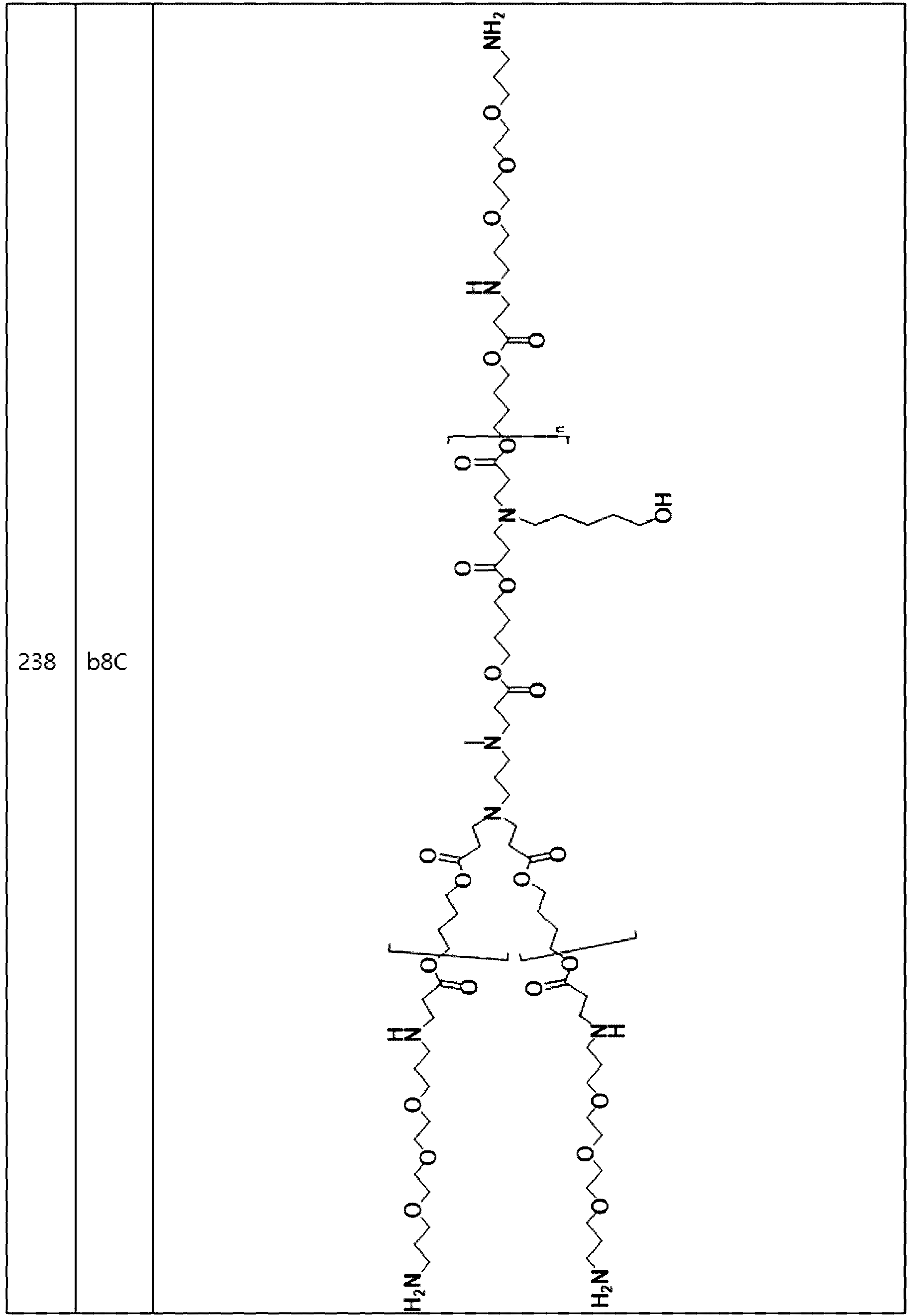

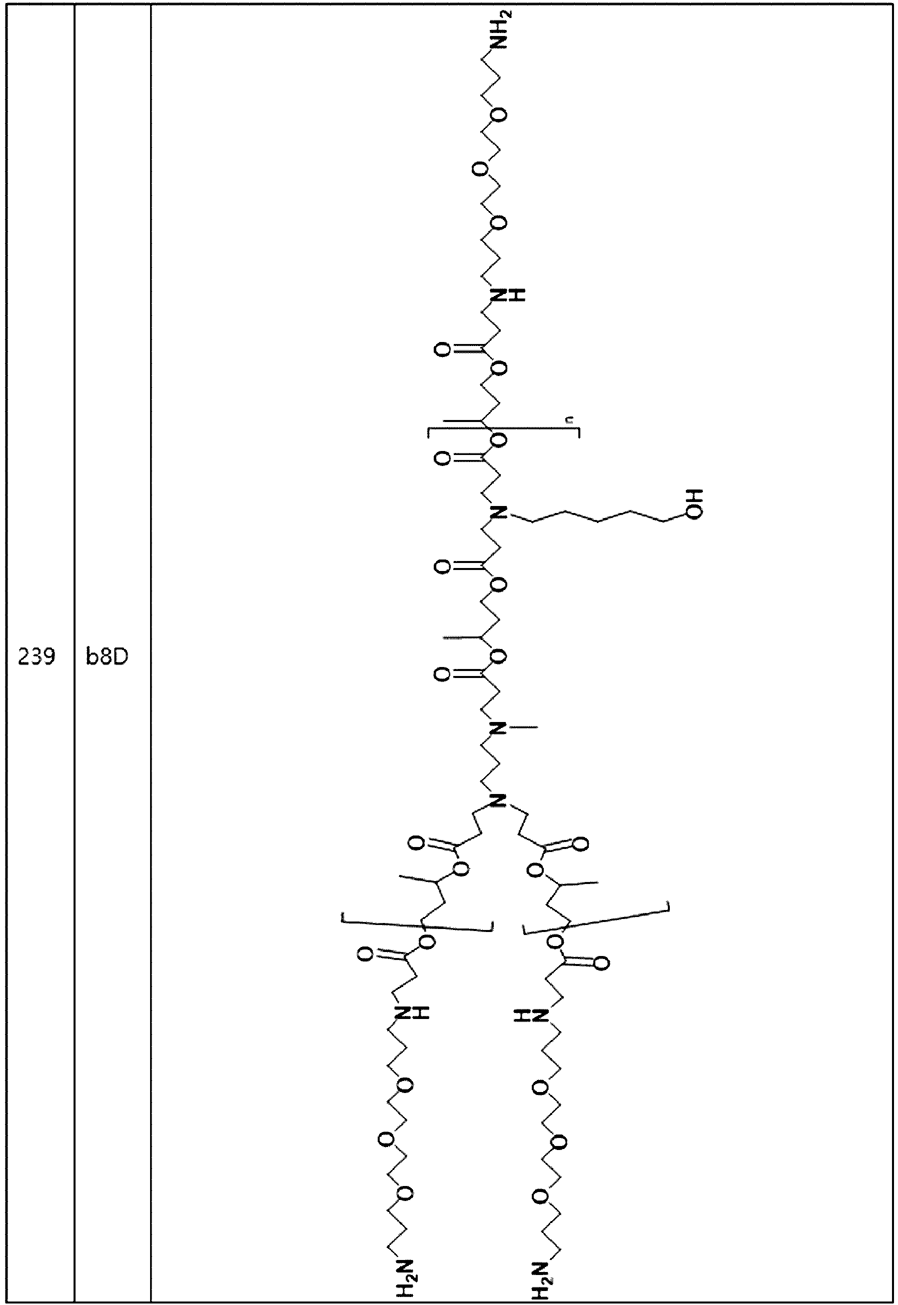

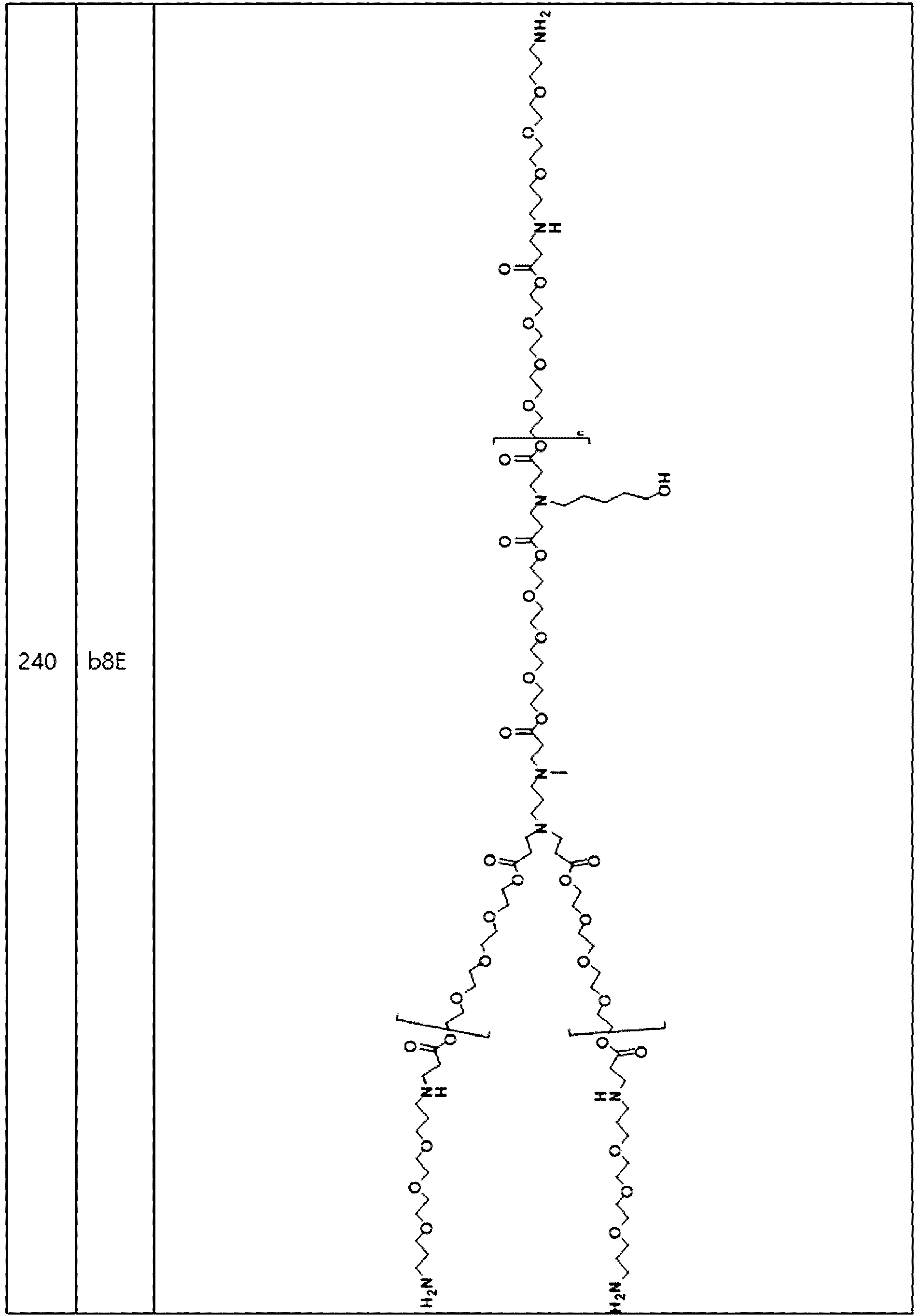

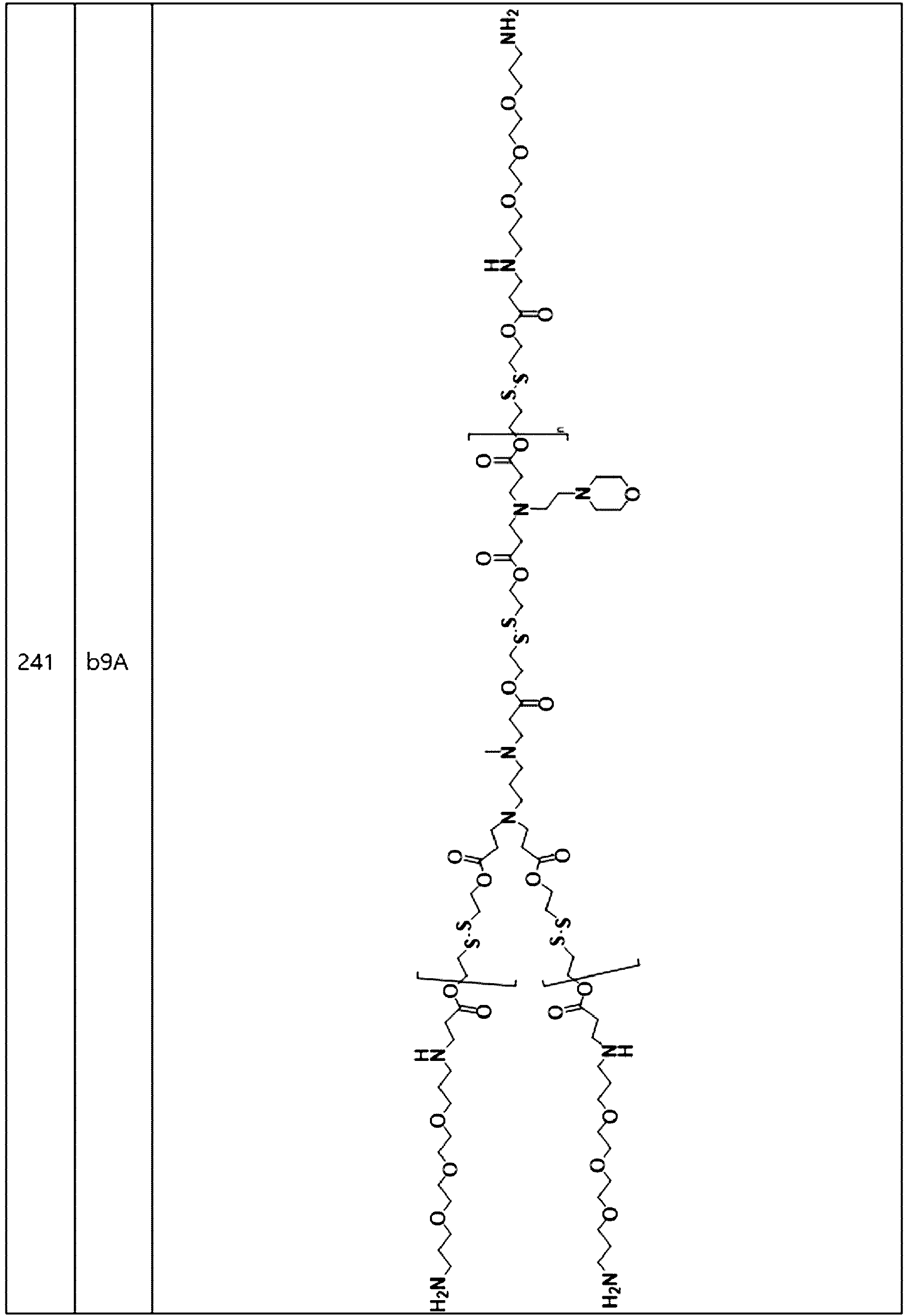

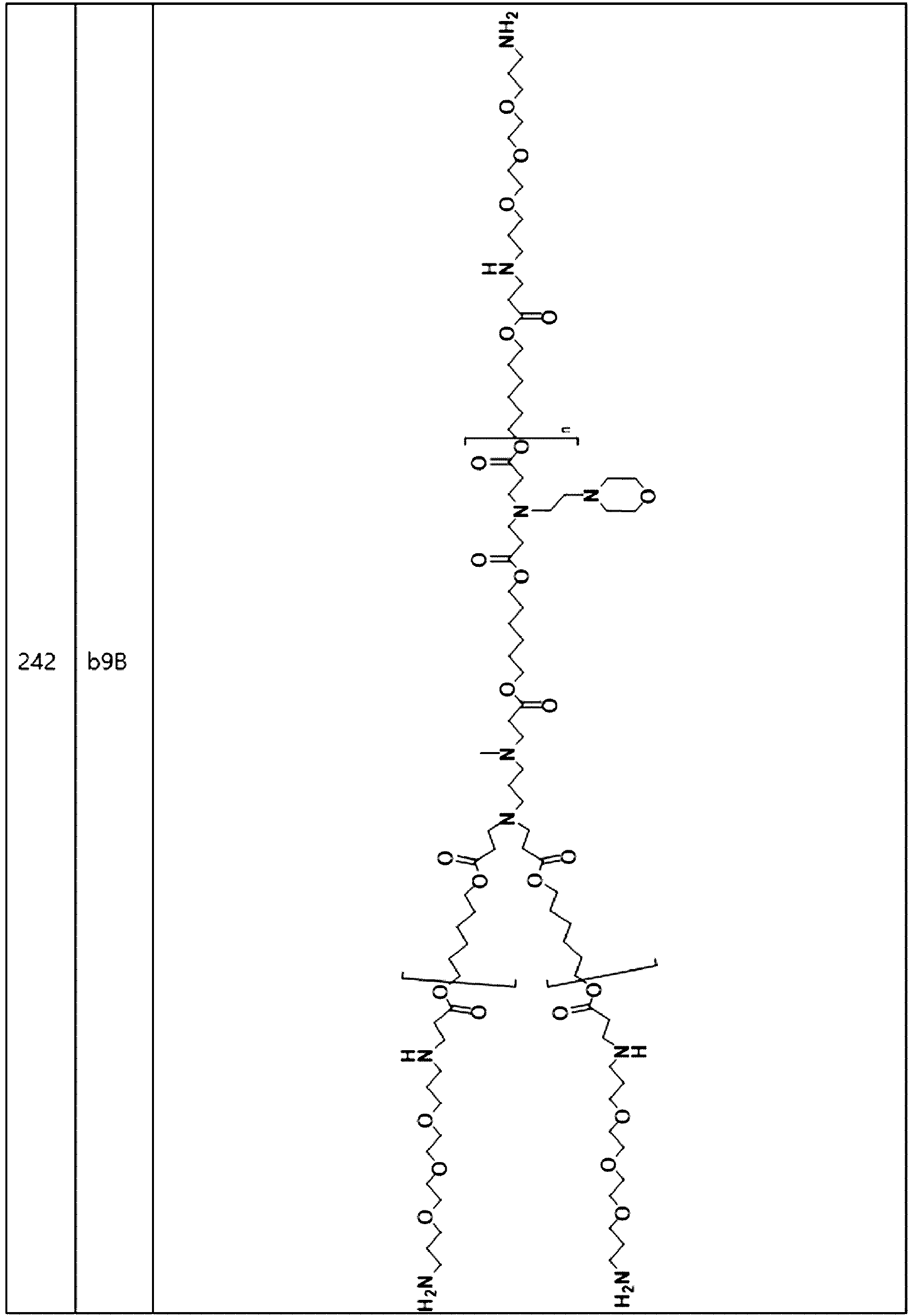

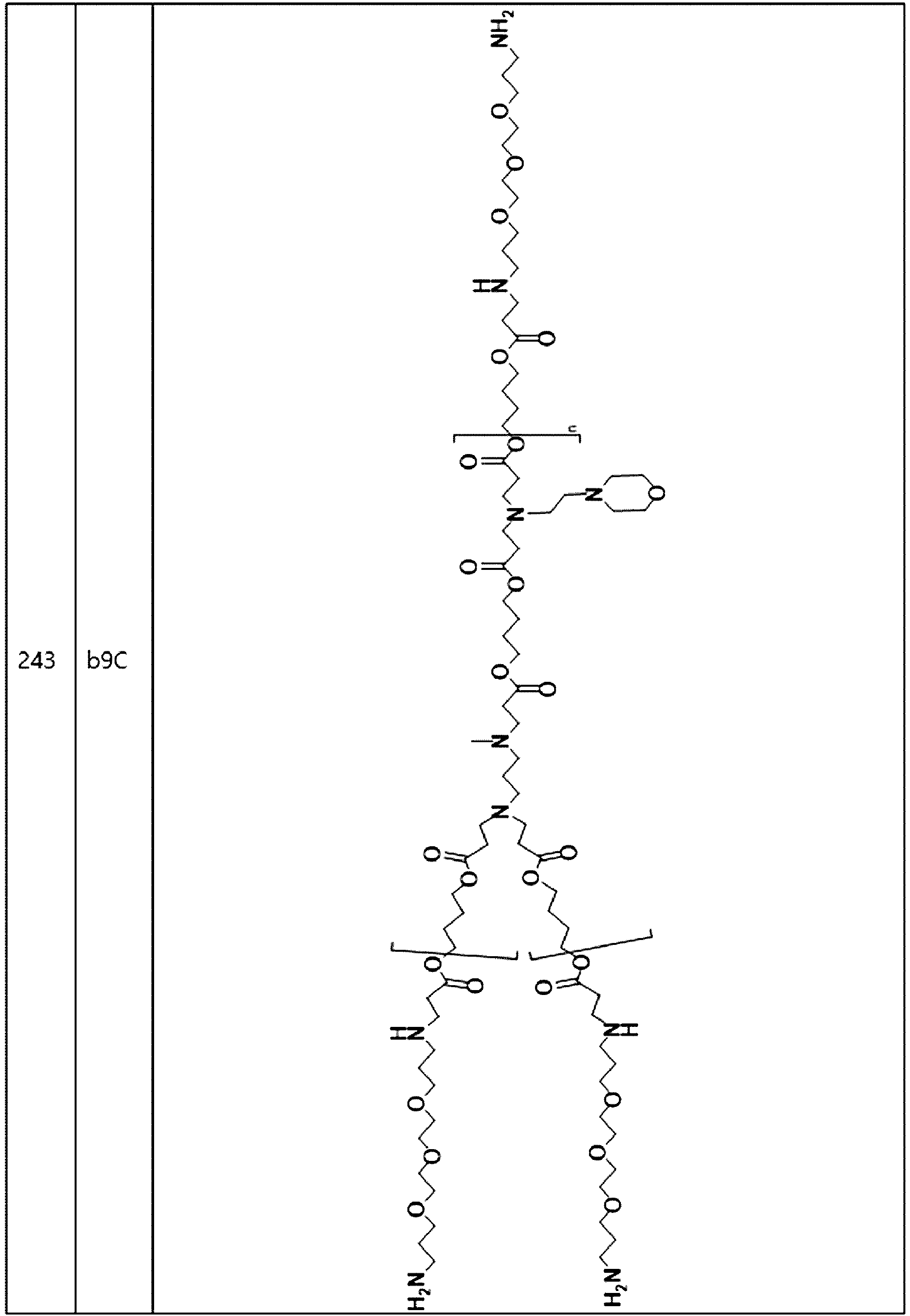

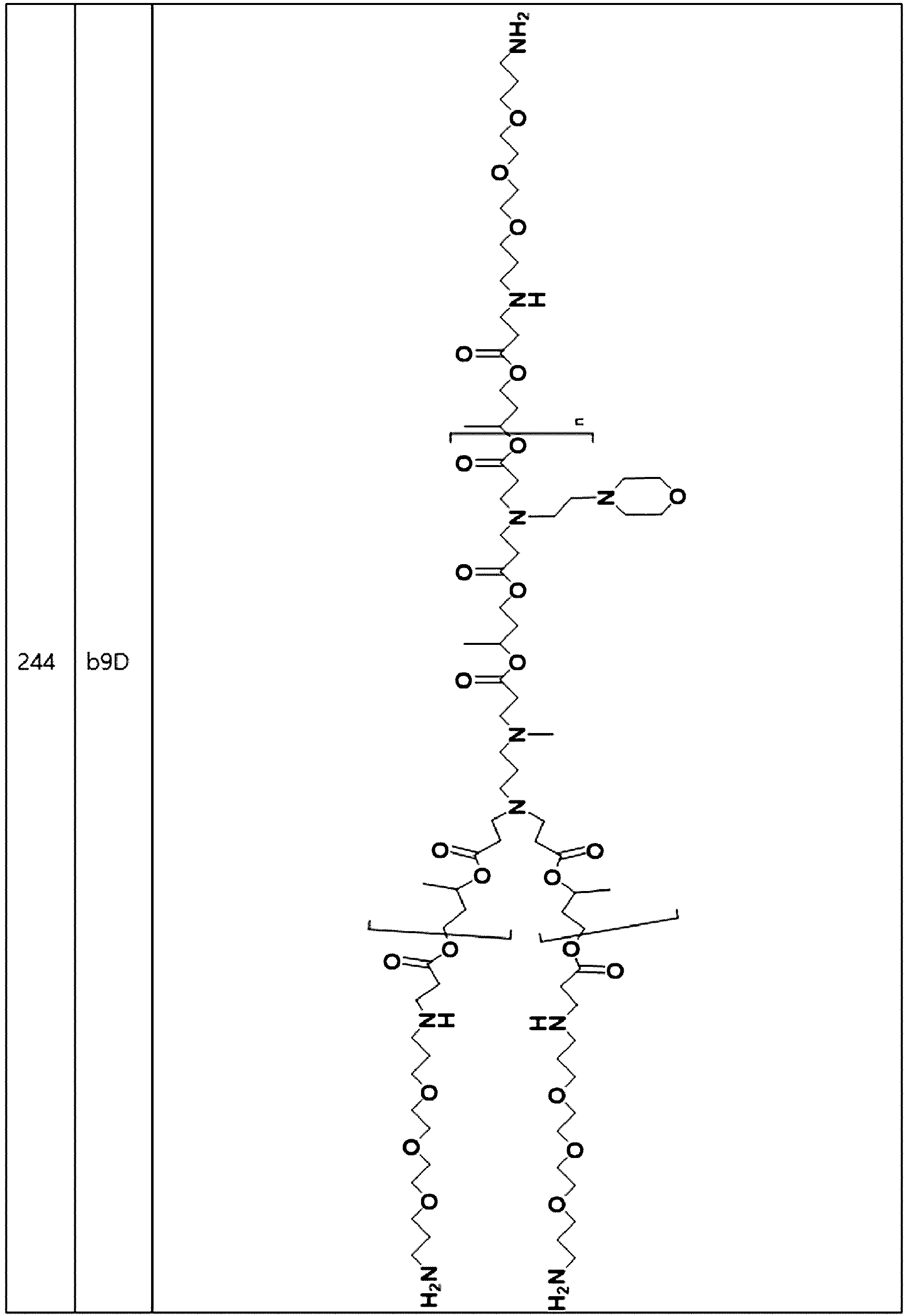

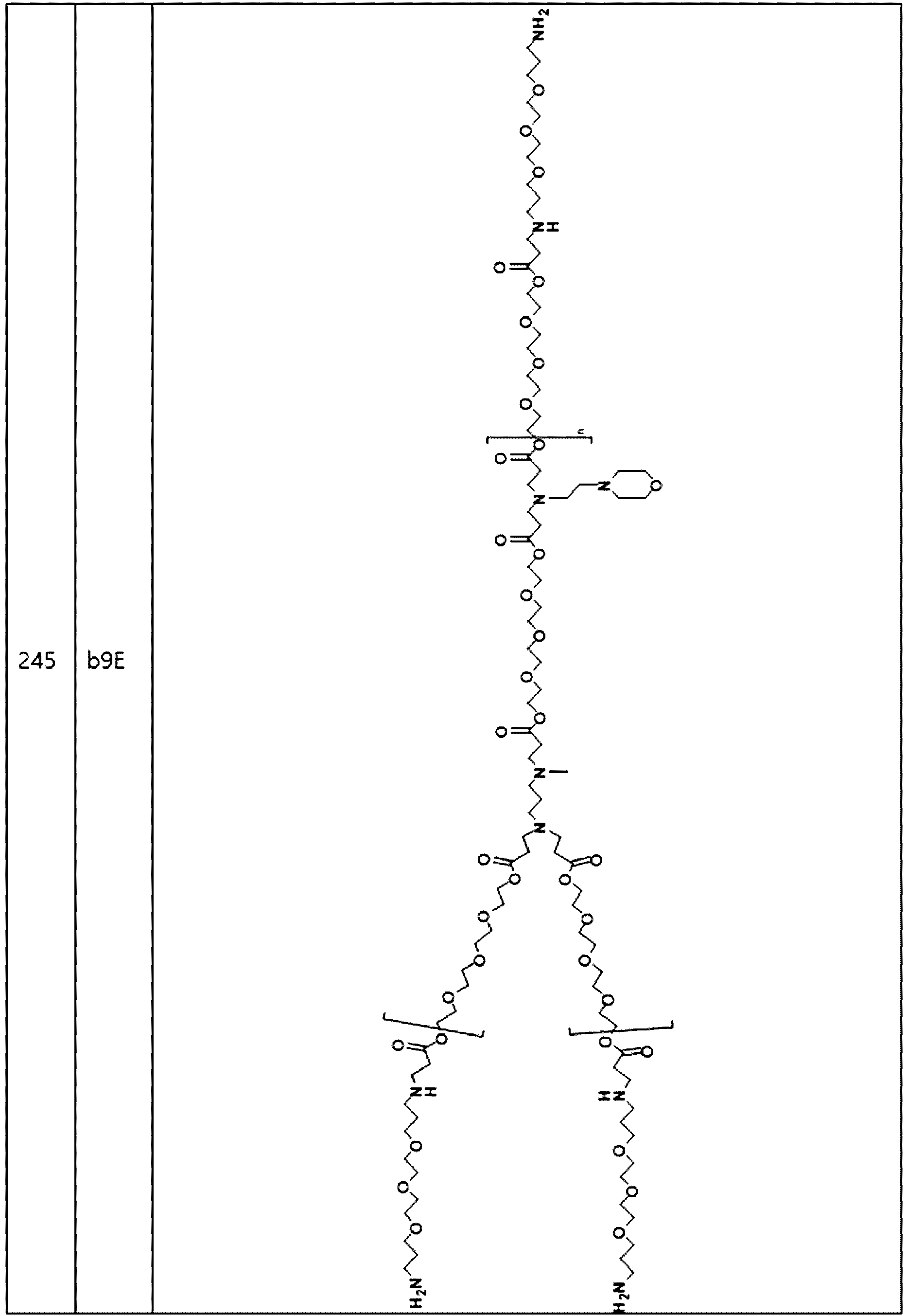

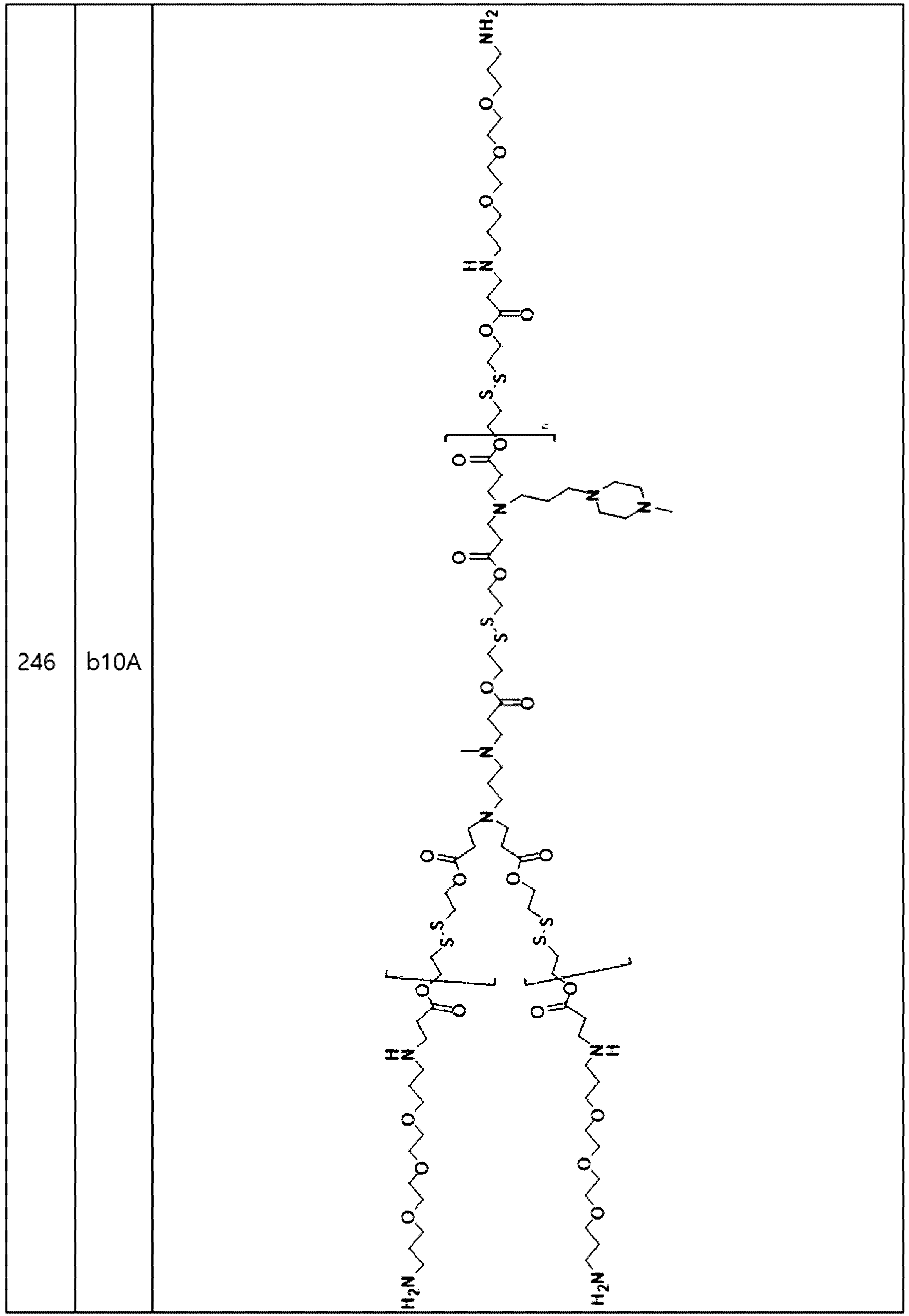

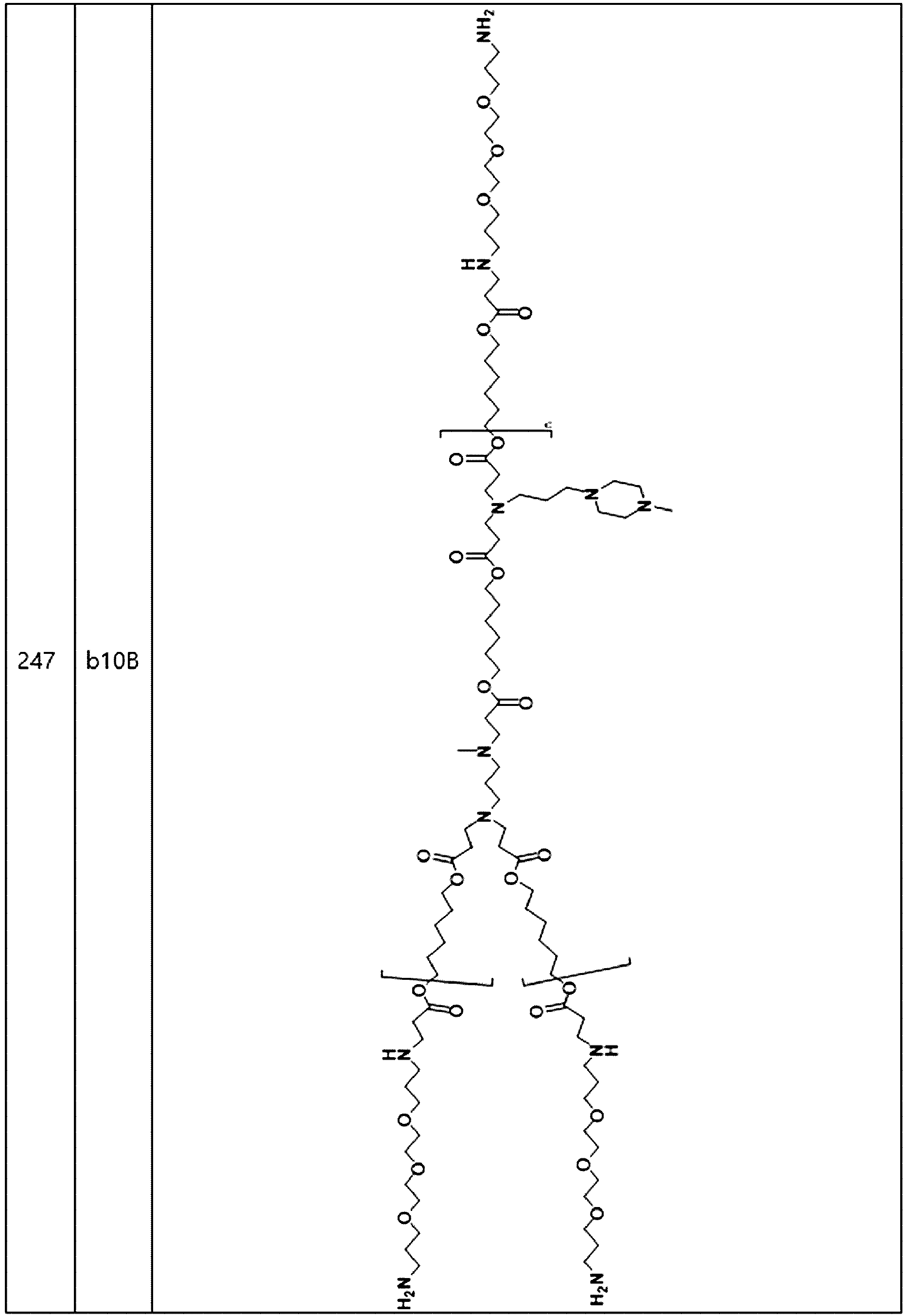

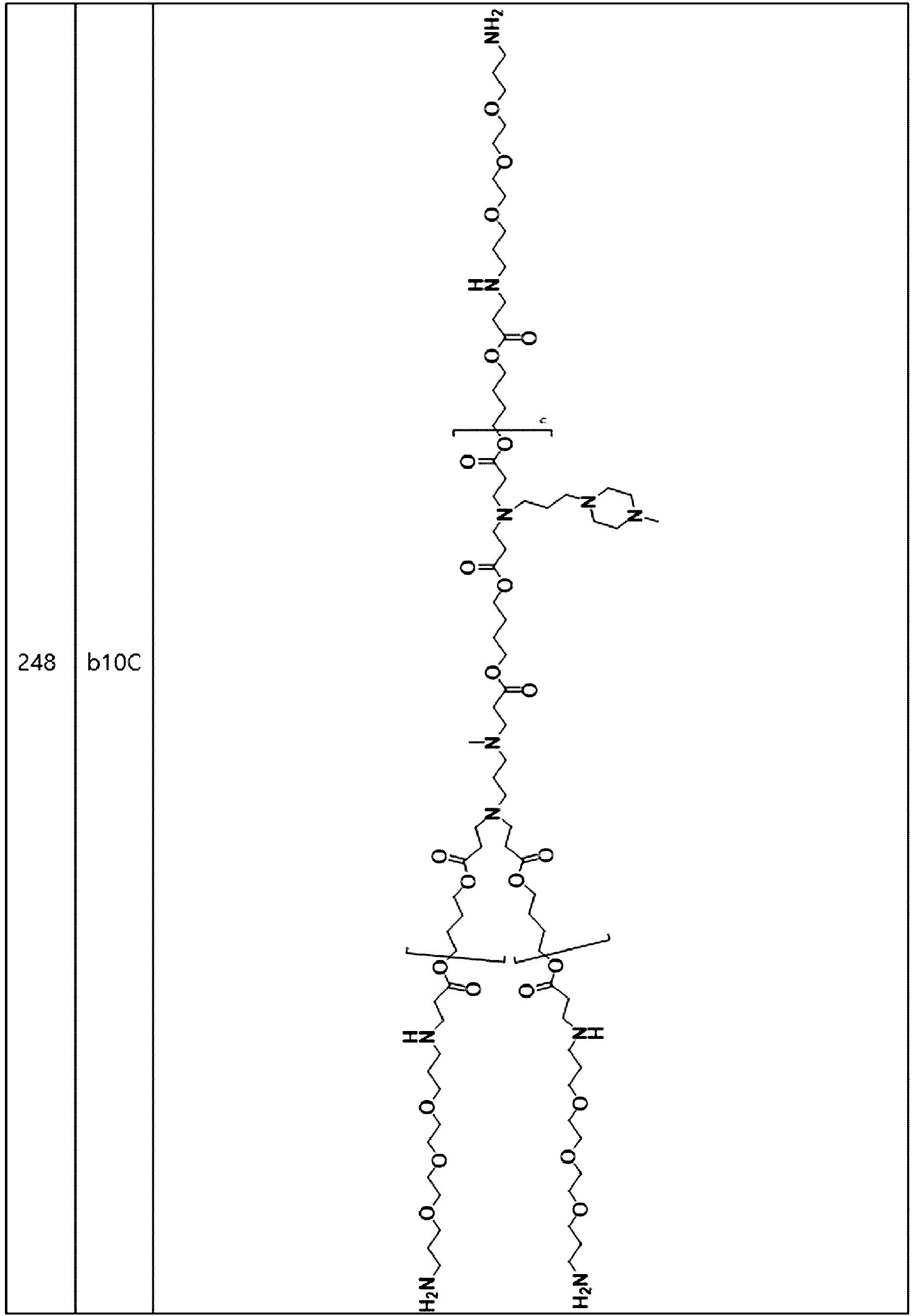

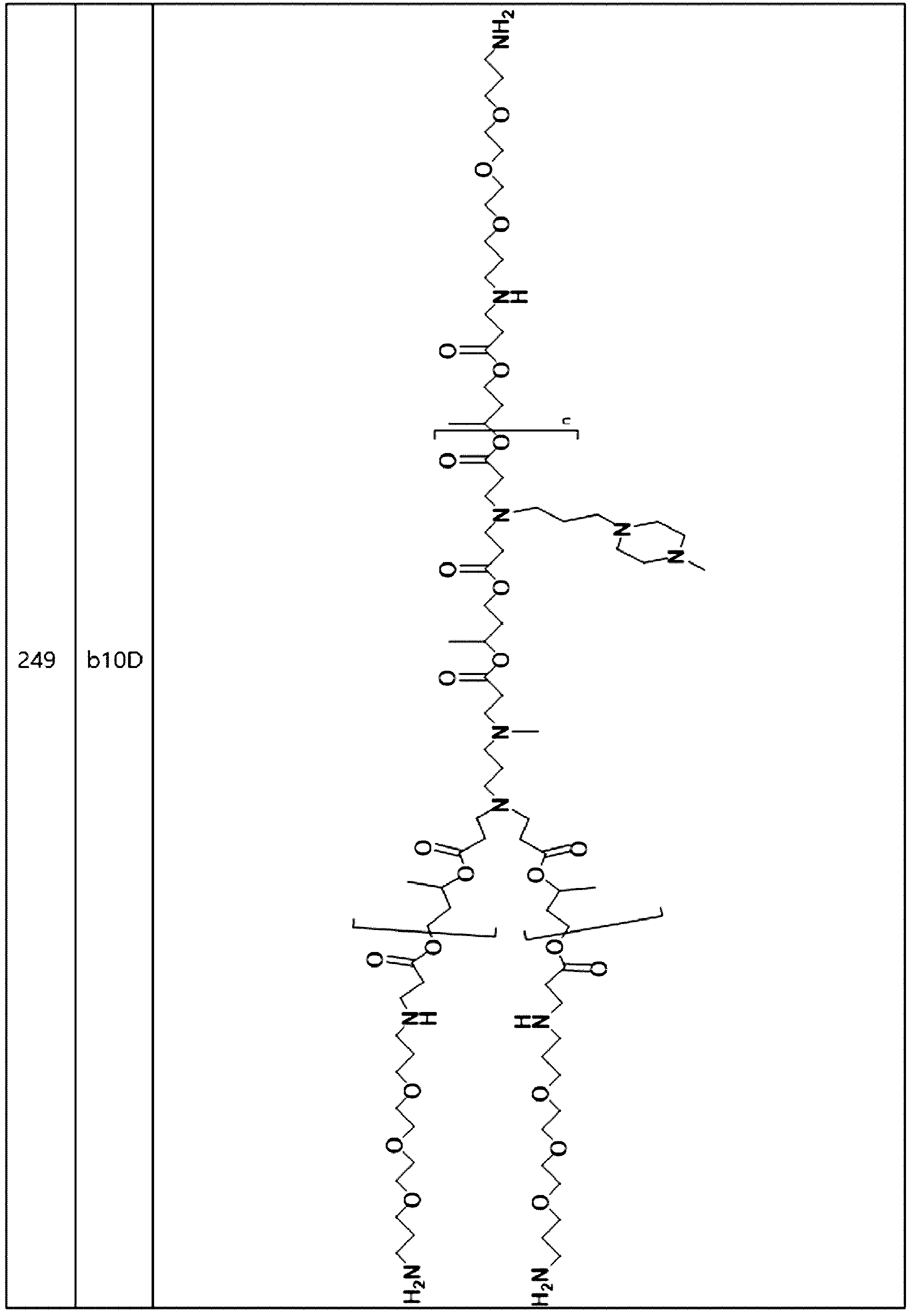

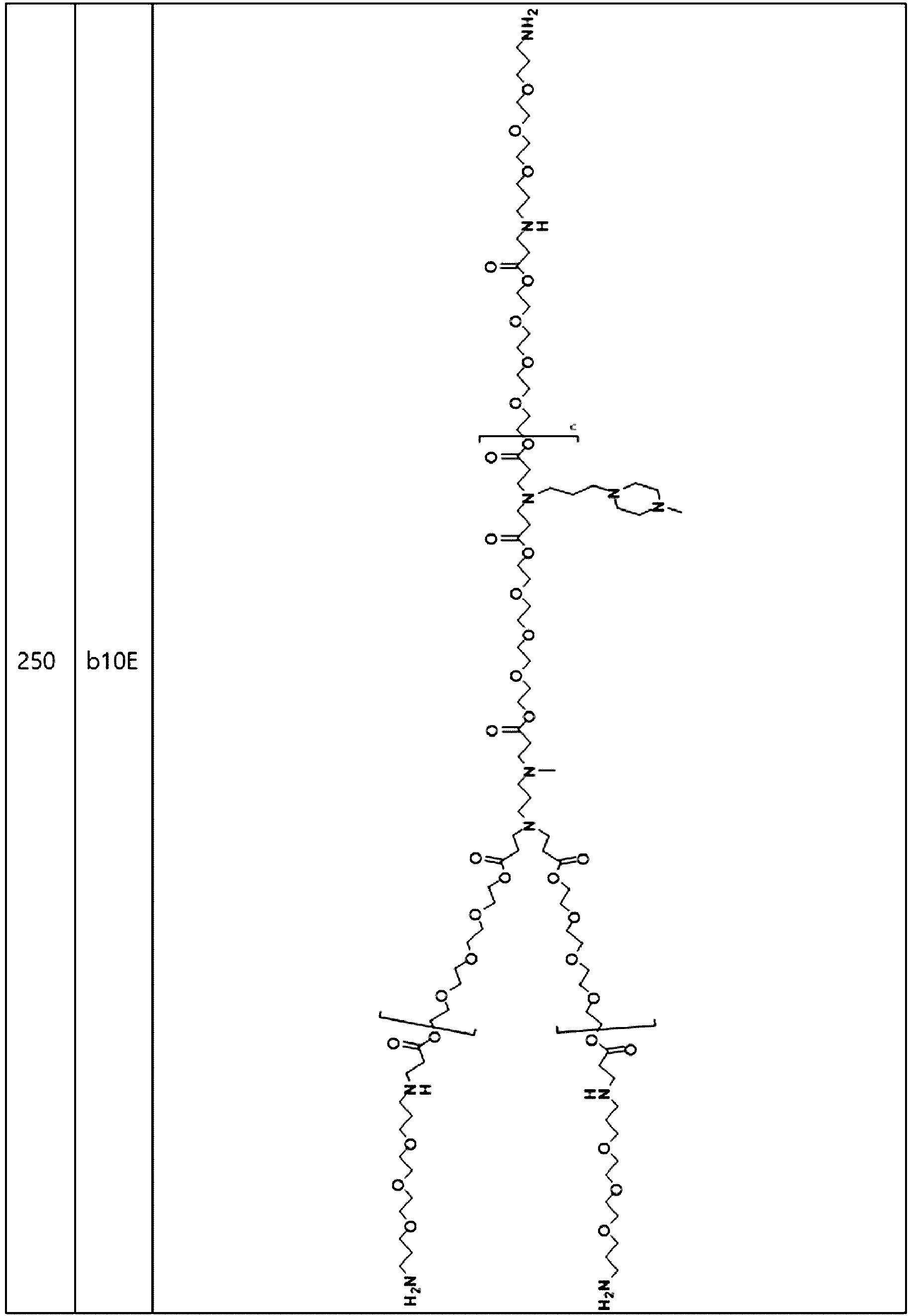

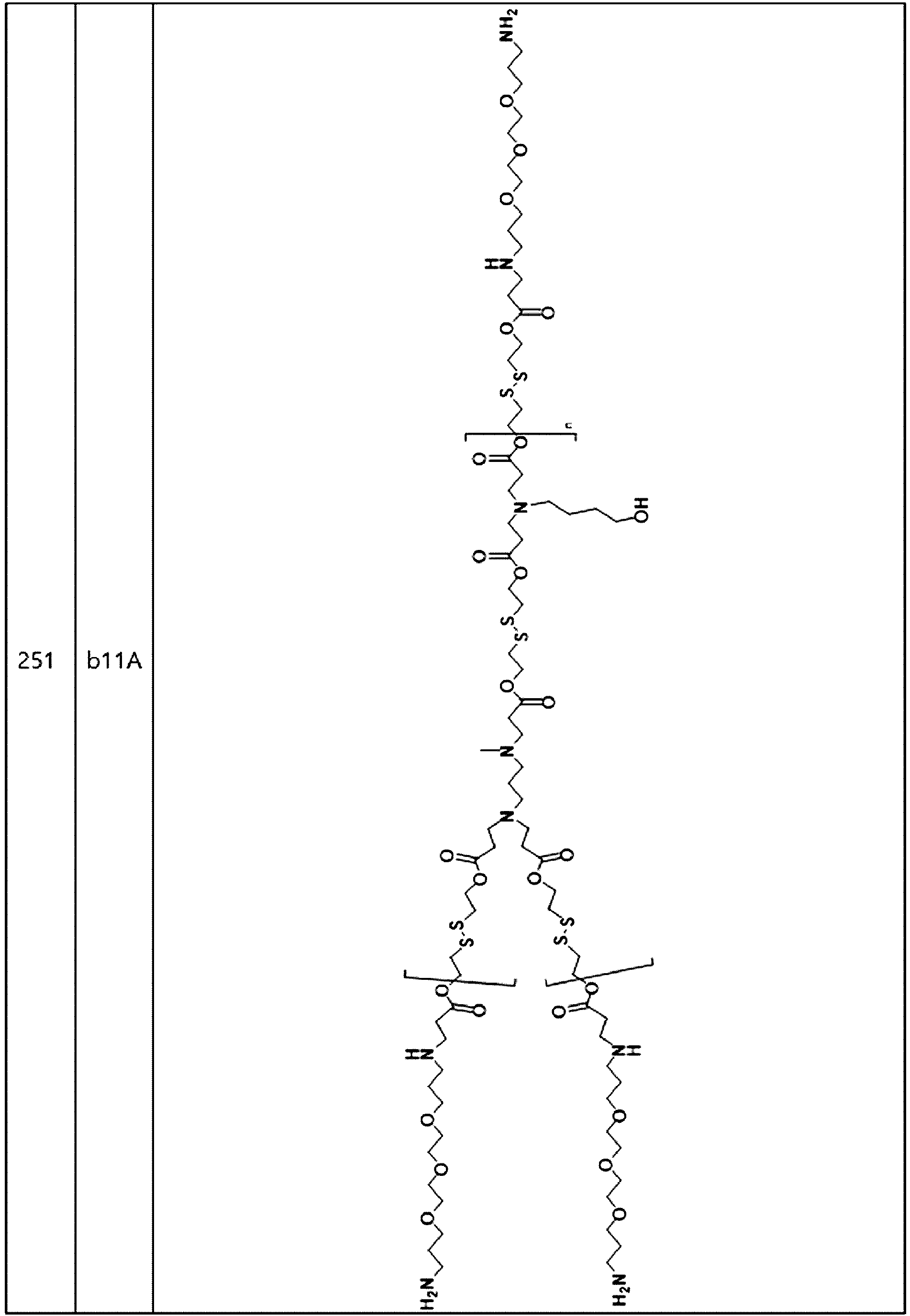

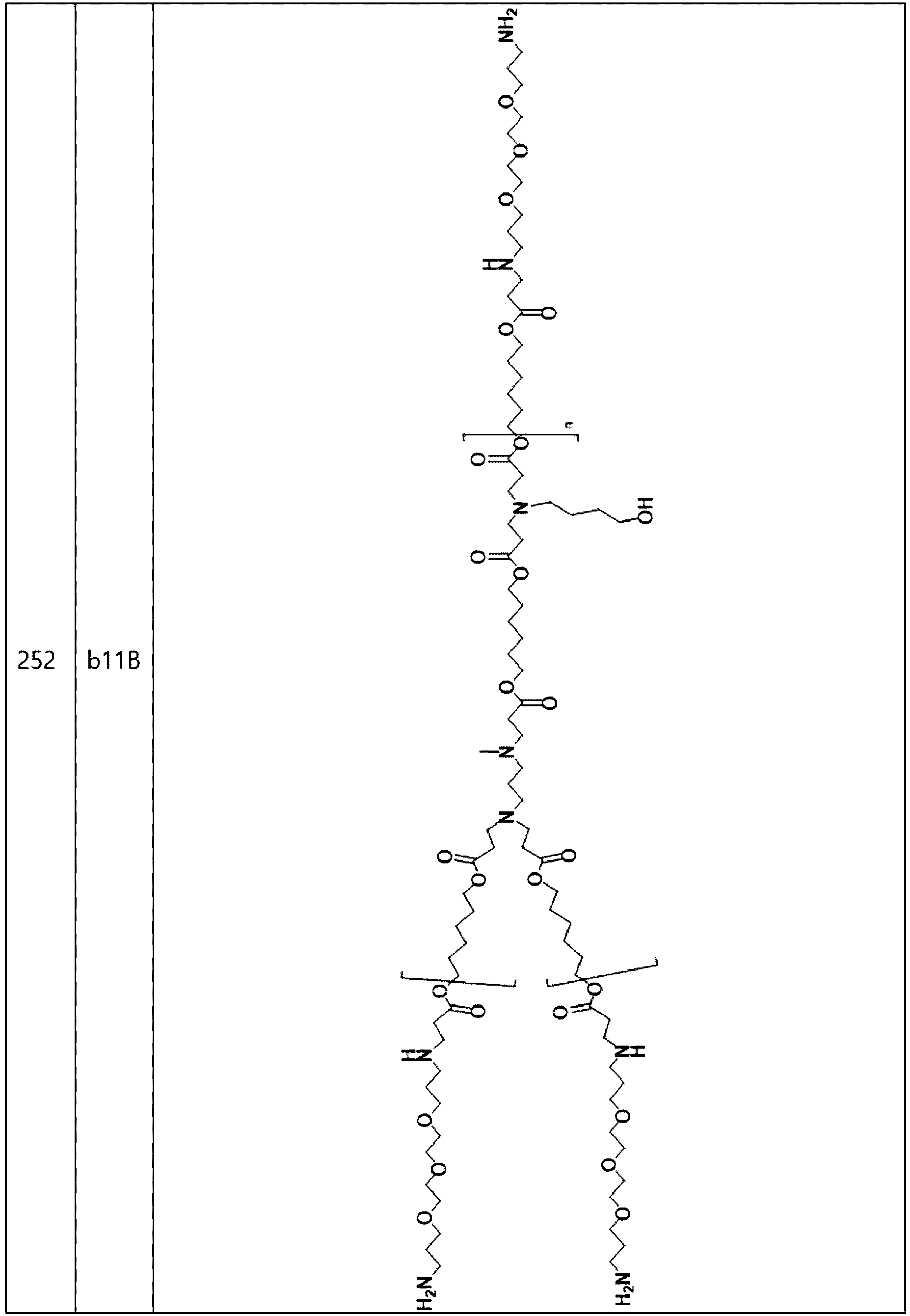

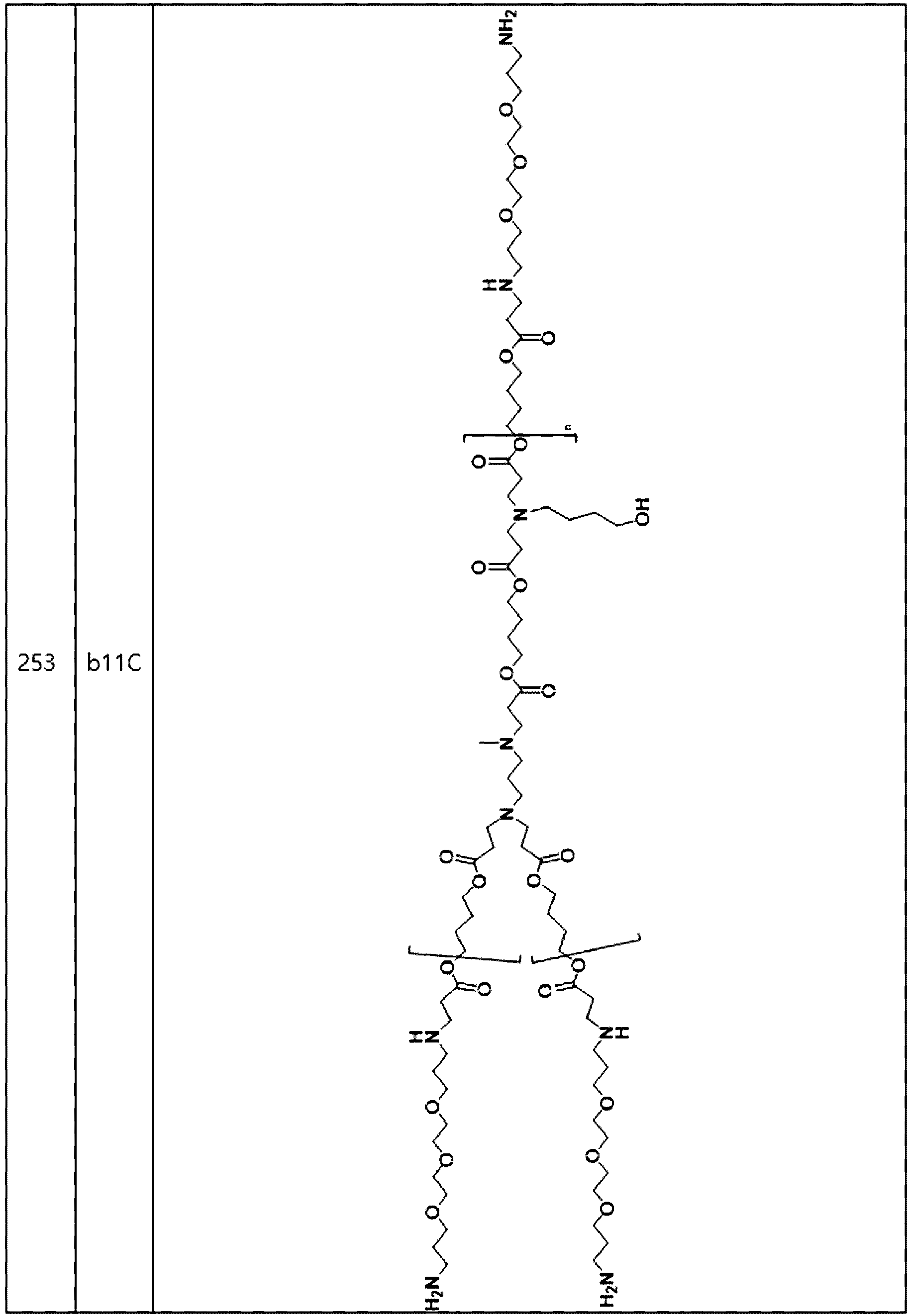

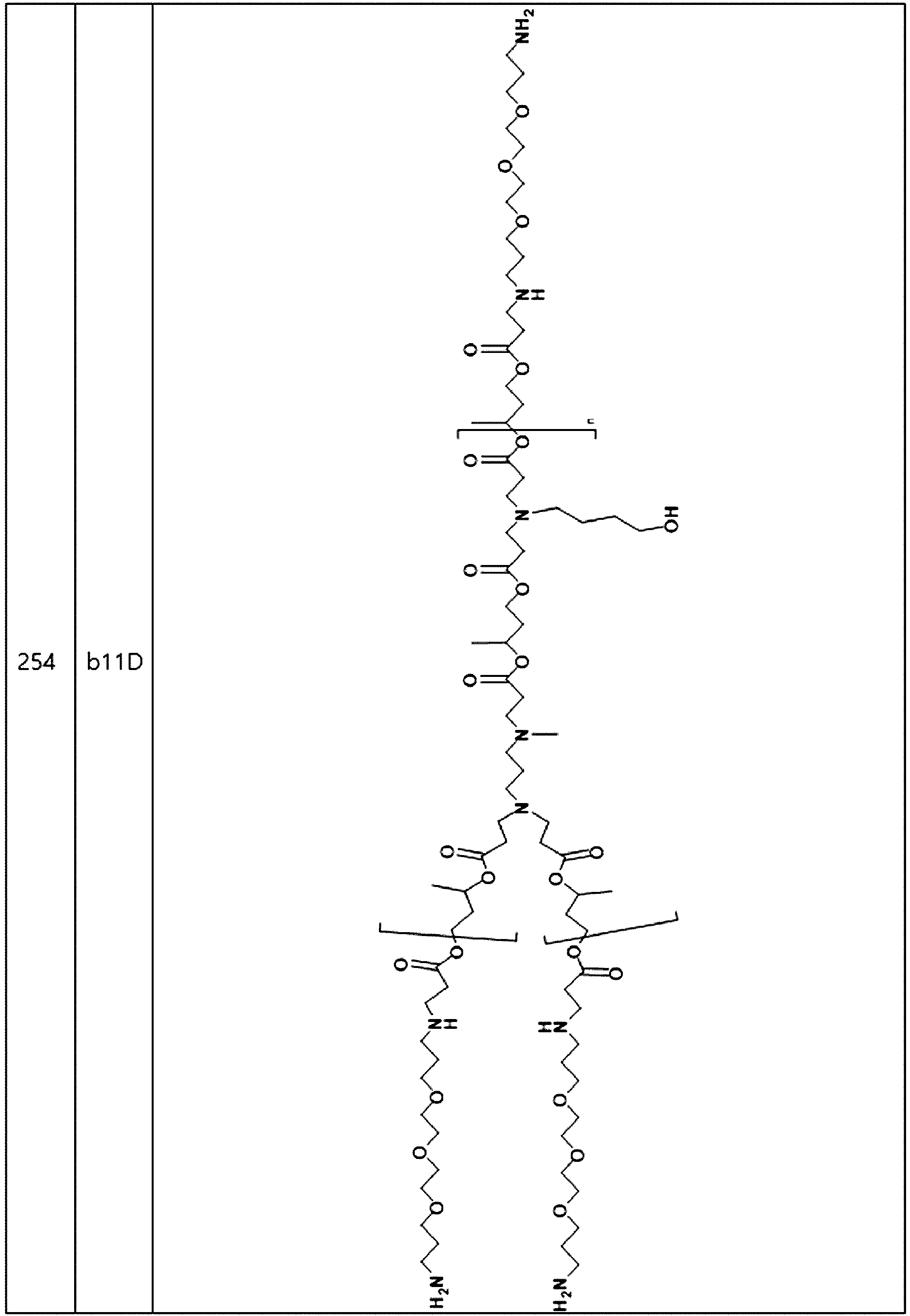

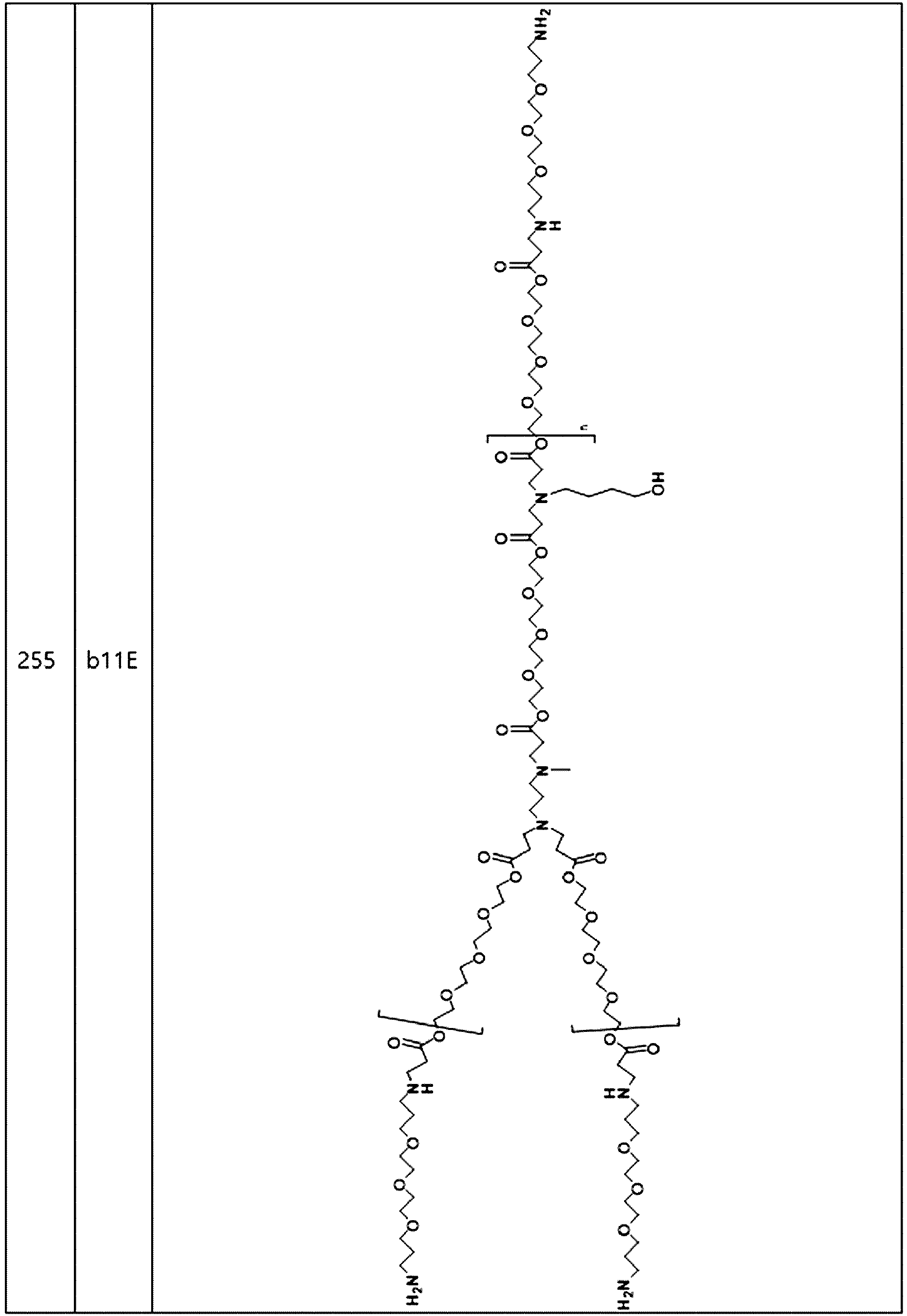

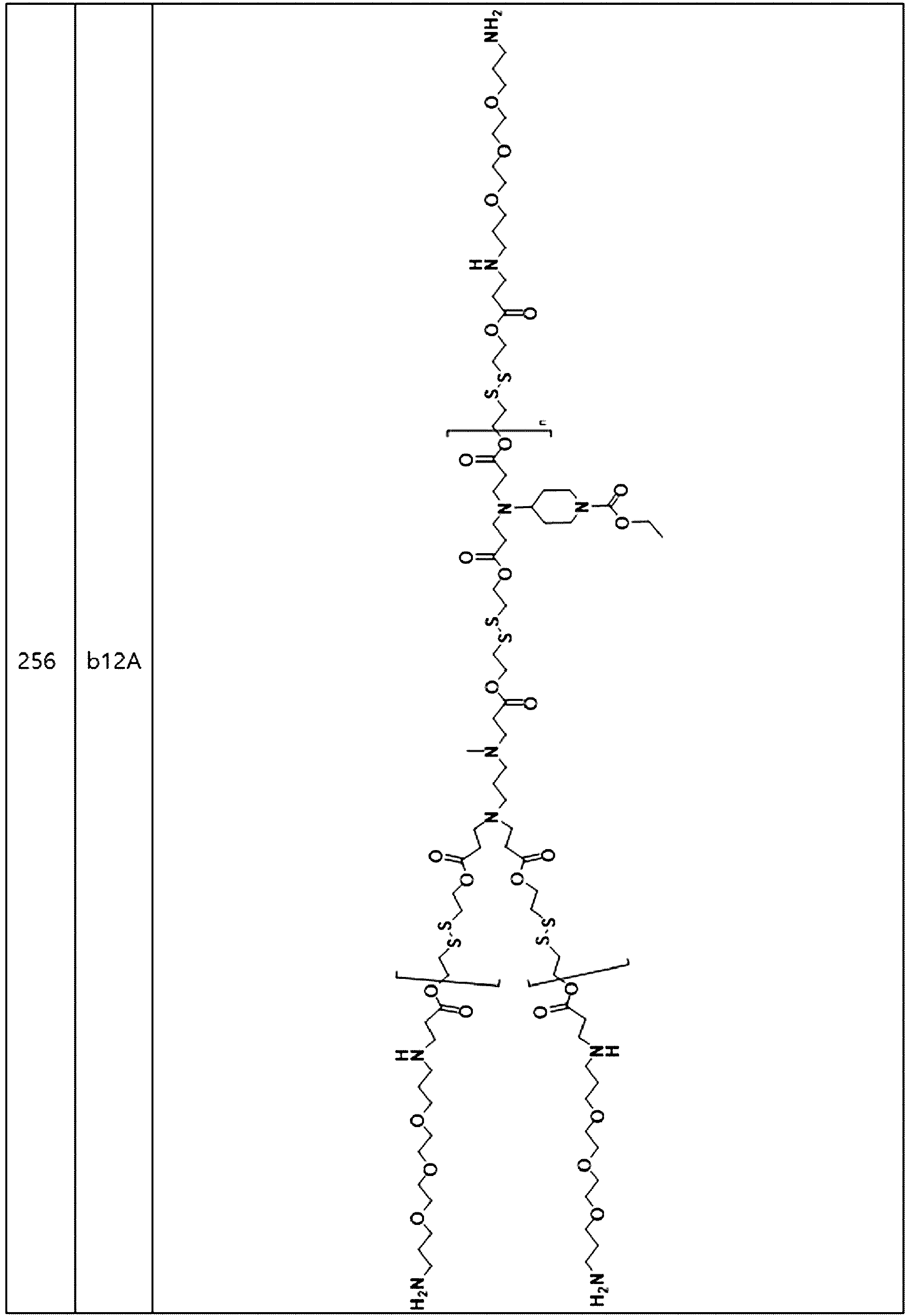

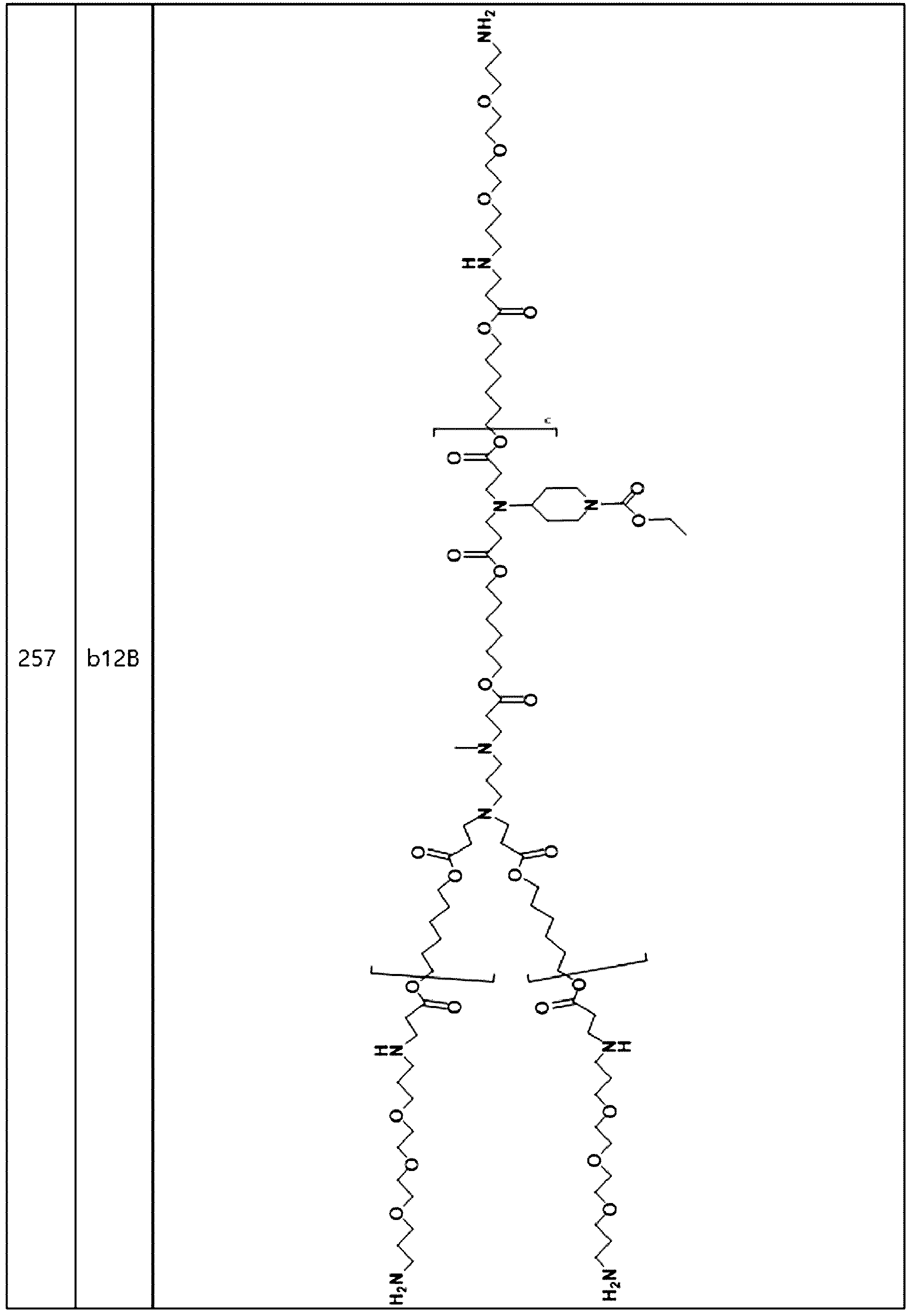

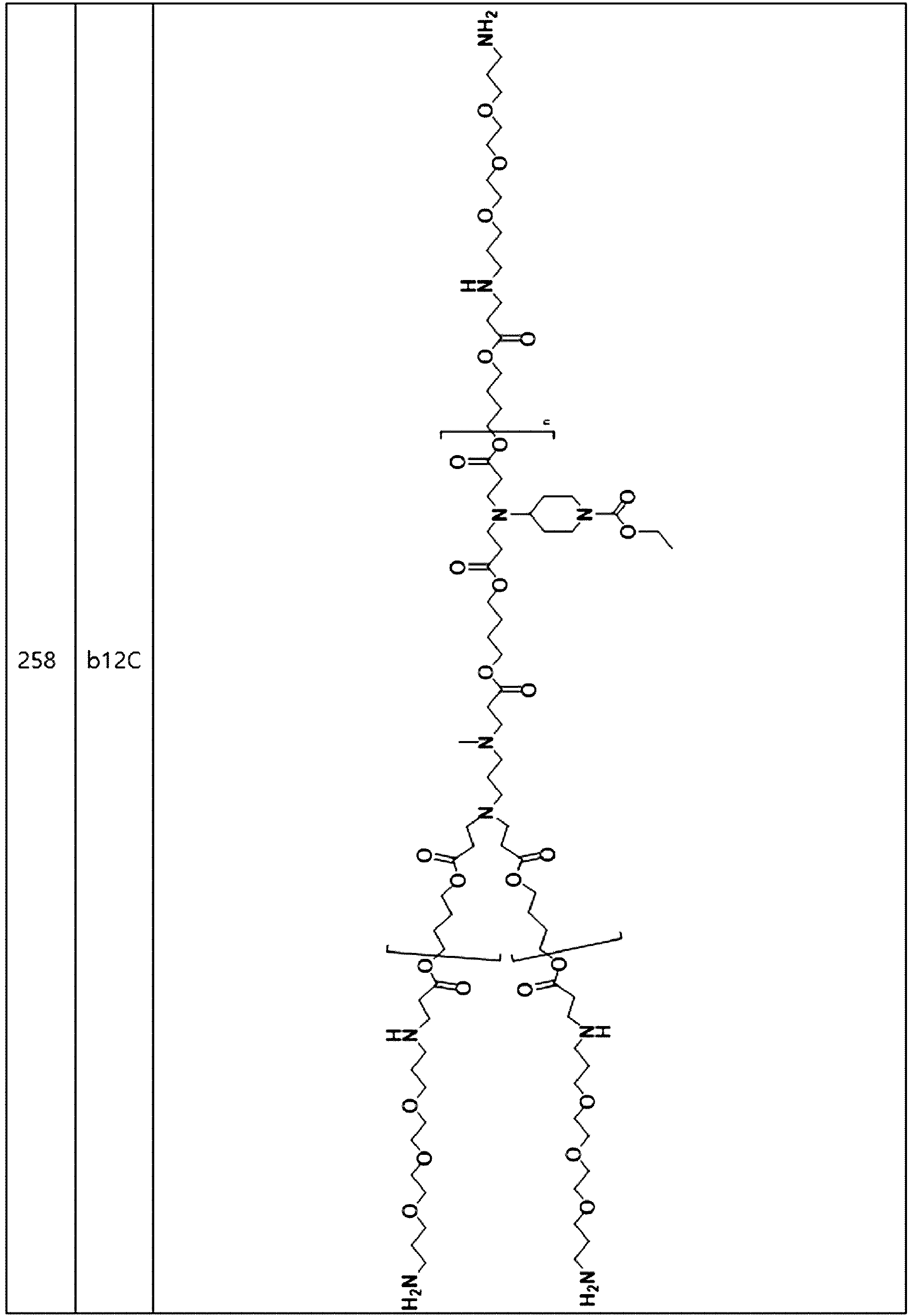

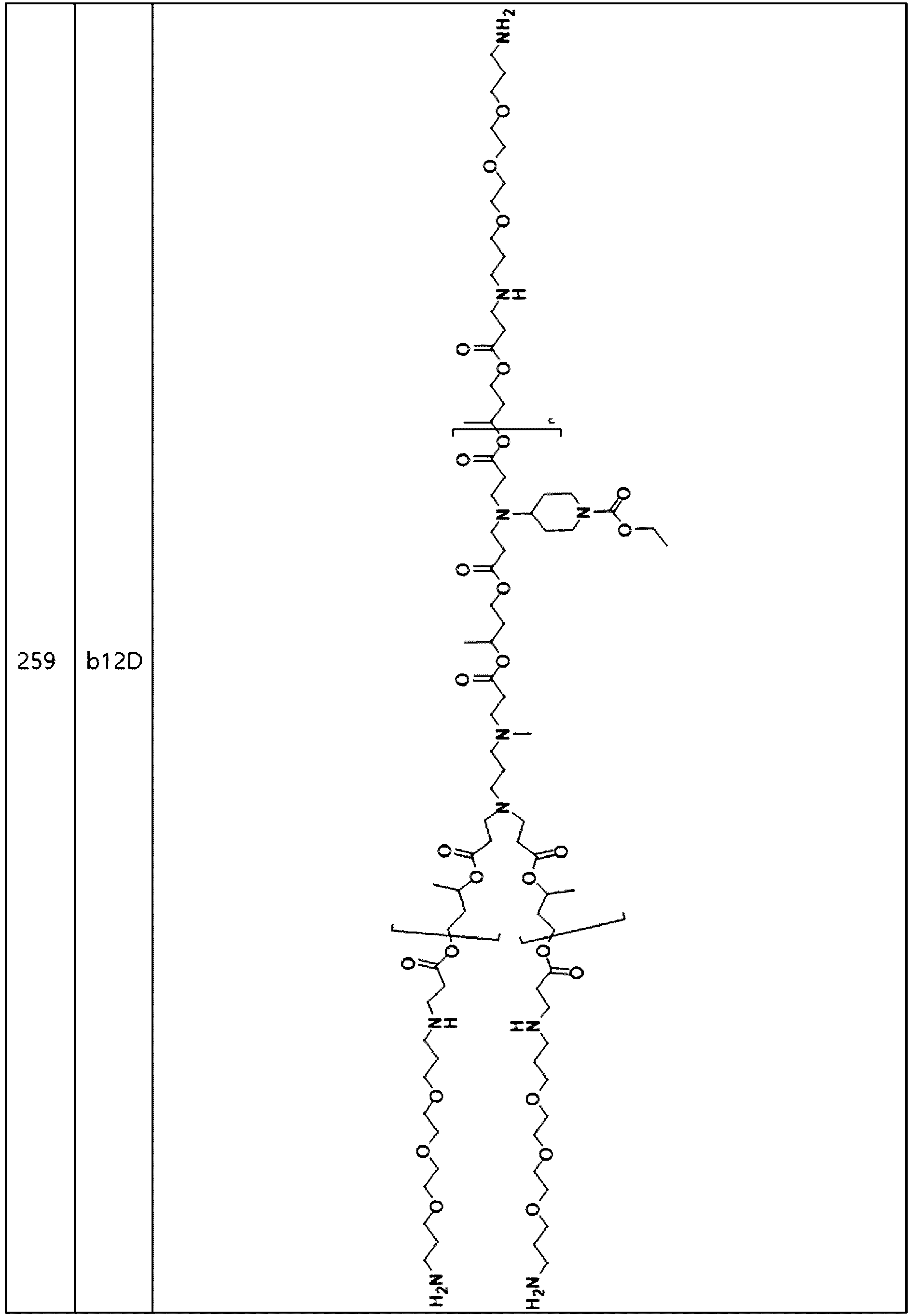

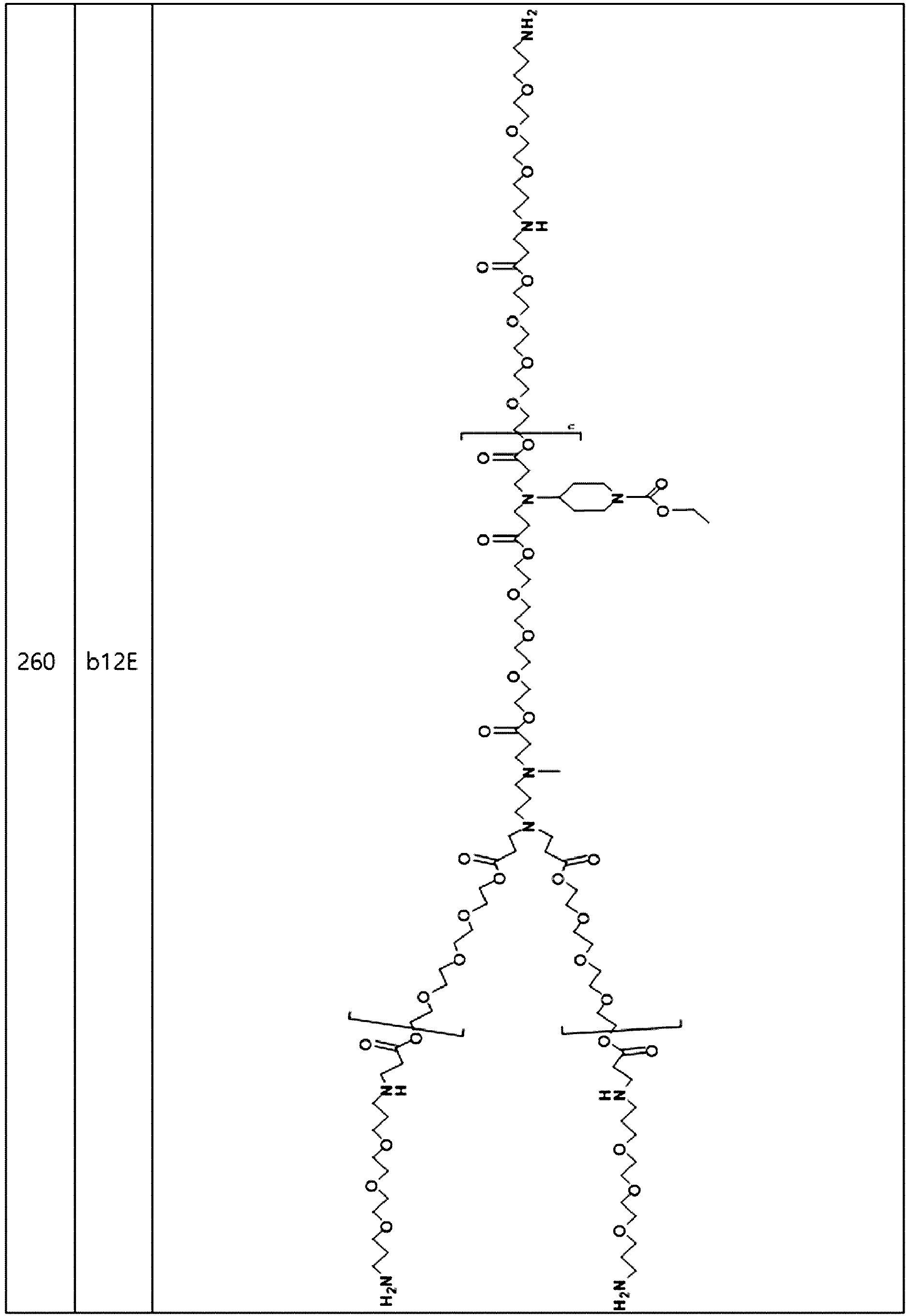

(in the above formulas, n is an integer between 1 and 500)

In one embodiment, the polymer may further comprise a branched monomer of Formula 25 below.

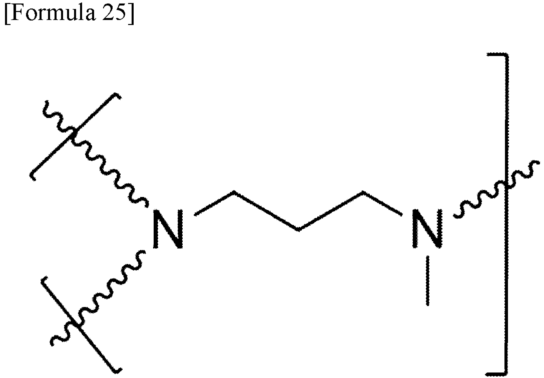

When the polymer according to the present invention comprises a structure of Formula 25 above, the polymer may comprise a structure of Formula 26 below. 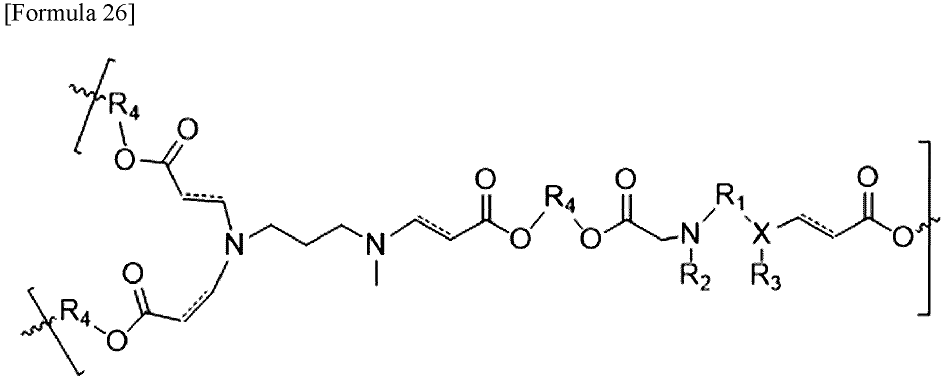 in the formula,
R1 is a bond or a C1-6 alkylene,
R2 and R3 are each independently (i) hydrogen, (ii) C1-6 alkyl which may be substituted with alkylamine or hydroxy, (iii) a 5- or 6-membered carbocycle or heterocycle, or (iv) C1-6 alkyl substituted with a 5- or 6-membered carbocycle or heterocycle,
said R2 and R3 may be linked together to form a C1-6 alkylene,
said 5- or 6-membered carbocycle or heterocycle may be substituted with C1-3 alkyl or C1-3 alkoxyacyl,
X is a nitrogen atom or a bond, and when X is a bond, said R3 is absent,
R4 is a linear or branched C1-15 alkylene, 1 to 5 carbon atoms of said alkylene may be replaced by oxygen or sulfur, and
- - - - - may represent a single bond or a double bond.

The above repeating unit may be n (an integer between 1 and 500), and in this case, Formula 26 may be represented as follows. 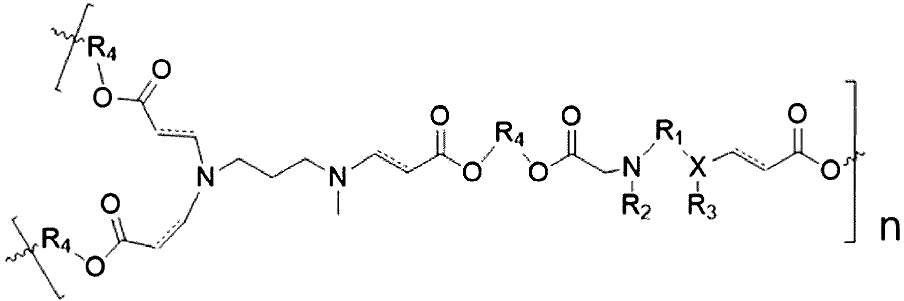 (the definitions of the groups are as defined in relation to Formula 26 above)

When the polymer according to the present invention comprises a structure of Formula 25 above, the polymer may comprise a structure of Formula 27 below. 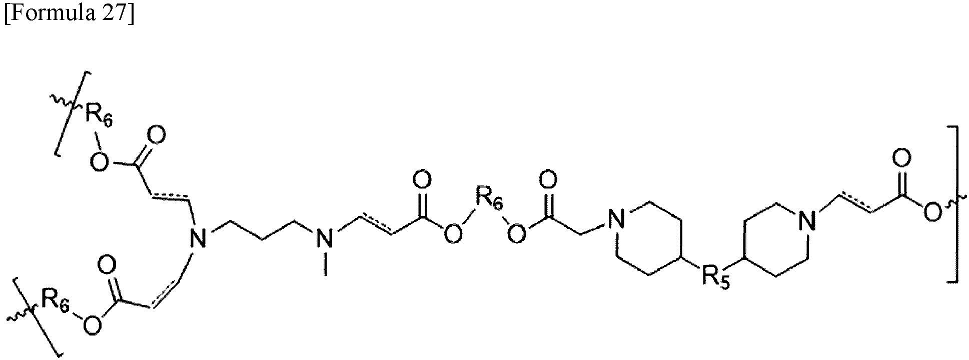 in the formula,
R5 is a C1-6 alkylene,
R6 is a linear or branched C1-15 alkylene, 1 to 5 carbon atoms of said alkylene may be replaced by oxygen or sulfur, and
- - - - - may represent a single bond or a double bond.

The above repeating unit may be n (an integer between 1 and 500), and in this case, Formula 27 may be represented as follows. 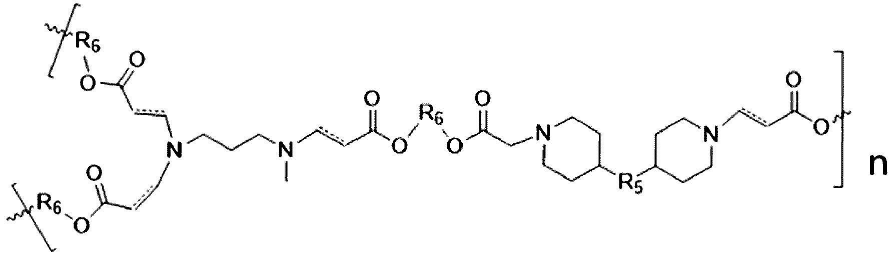 (the definitions of the groups are as defined in relation to Formula 27 above)

In one embodiment, one or both ends of the polymer may be linked to a PEG-containing moiety having a structure of Formula 28 below: 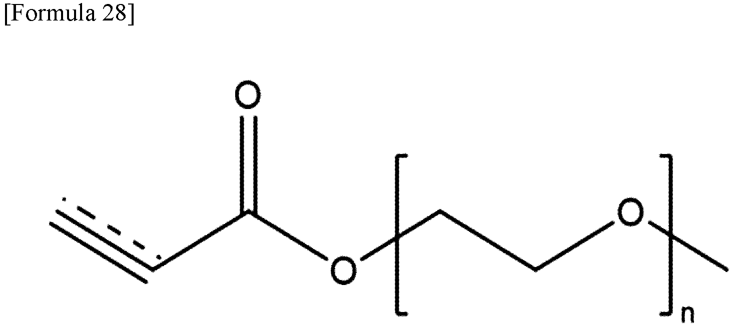 in the formula,
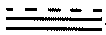 may represent a double bond or a triple bond, and
n may be an integer in the range of 1 to 500.

The present invention provides a polymeric nanoparticle in which the polymer and a nucleic acid molecule form a complex. The polymeric nanoparticle according to the present invention preferably does not include a lipid nanoparticle (LNP).

The nucleic acid molecule that can be included in the polymeric nanoparticle according to the present invention may be RNA. The RNA is preferably mRNA.

The nucleic acid molecule that can be included in the polymeric nanoparticle according to the present invention may be DNA.

The polymeric nanoparticle according to the present invention may be one in which the nucleic acid molecule is isolated in a pH-dependent manner and delivered into cells in the body.

The polymeric nanoparticle according to the present invention may be one in which when delivered into the body, the expression of the nucleic acid molecule is maintained for a longer period of time compared to when the same nucleic acid molecule is delivered into the body in the form of a non-polymeric lipid nanoparticle.

The present invention provides a method for delivering a nucleic acid molecule into a cell using the polymeric nanoparticle. The nucleic acid molecule delivery method according to the present invention preferably does not use a lipid nanoparticle (LNP).

The polymeric nanoparticle according to the present invention may be delivered by parenteral, intramuscular, subcutaneous, or intravenous administration.

The present invention provides a pharmaceutical composition comprising the polymeric nanoparticle. The pharmaceutical composition according to the present invention preferably does not include a lipid nanoparticle (LNP).

The pharmaceutical composition comprising the polymeric nanoparticle according to the present invention may be a gene therapeutic agent.

The pharmaceutical composition comprising the polymeric nanoparticle according to the present invention may be a vaccine.

### Effects of Invention

Since the nucleic acid molecule delivery vehicle based on a polymer having pH sensitivity according to the present invention has a high nucleic acid molecule delivery efficiency, it can be used as an excellent delivery vehicle. In particular, the nucleic acid molecule delivery vehicle according to the present invention can be utilized as a therapeutic gene delivery vehicle and an mRNA vaccine.

In addition, depending on the length of the monomer and polymer, the nucleic acid molecule can be delivered to a desired site, and the nucleic acid molecule can be released in response to a specific stimulus through an additional stimulus-sensitive linker.

In addition, the nucleic acid molecule delivery vehicle according to the present invention can be degraded after releasing the nucleic acid molecule within the cell, so it has low side effects.

### Brief Description of Drawings

Figure 1 is a schematic diagram of the synthesis of the polymer delivery vehicle according to the present invention and a schematic diagram of a polymeric nanoparticle (PNP) comprising the polymer delivery vehicle and mRNA.
Figures 2a to 2e are graphs showing the ¹H-NMR results of polymers 1A to 1E.
Figures 3a to 3e are graphs showing the ¹H-NMR results of polymers 2A to 2E.
Figures 4a to 4e are graphs showing the ¹H-NMR results of polymers 3A to 3E.
Figures 5a to 5e are graphs showing the ¹H-NMR results of polymers 4A to 4E.
Figures 6a to 6e are graphs showing the ¹H-NMR results of polymers 5A to 5E.
Figures 7a to 7e are graphs showing the ¹H-NMR results of polymers 6A to 6E.
Figures 8a to 8e are graphs showing the ¹H-NMR results of polymers 7A to 7E.
Figures 9a to 9e are graphs showing the ¹H-NMR results of polymers 8A to 8E.
Figures 10a to 10e are graphs showing the ¹H-NMR results of polymers 9A to 9E.
Figures 11a to 11e are graphs showing the ¹H-NMR results of polymers 10A to 10E.
Figures 12a to 12e are graphs showing the ¹H-NMR results of polymers 11A to 11E.
Figures 13a and 13b are graphs showing the ¹H-NMR results of polymers PEG-1A and PEG-1B.
Figures 14a and 14b are graphs showing the ¹H-NMR results of polymers PEG-2A and PEG-2B.
Figures 15a and 15b are graphs showing the ¹H-NMR results of polymers PEG-3A and PEG-3B.
Figures 16a and 16b are graphs showing the ¹H-NMR results of polymers PEG-4A and PEG-4B.
Figures 17a to 17c are photographs showing the electrophoresis results of the polymeric nanoparticles (PNP) according to the present invention.
Figure 18 is a graph showing the zeta potential of the polymeric nanoparticles (PNP) according to the present invention.
Figure 19 is a photograph showing the mRNA delivery of the polymeric nanoparticles (PNP) according to the present invention *in vivo.*
Figure 20 is a graph showing the expression duration of mRNA delivered to the polymeric nanoparticles (PNP) according to the present invention.

### Best Mode for Carrying out the Invention

The manufacturing examples and embodiments of the present invention will be described in detail so that those of ordinary skill in the art to which the present invention belongs can easily practice the present invention. However, the present invention may be implemented in various forms and is not limited to the manufacturing examples and embodiments described herein.

Throughout the present specification, when a certain part "includes" a certain component, it means that other components may be further included, rather than excluding other components, unless otherwise specifically stated.

Throughout the present specification, "polymer", "macromolecule," and "polymer" are used interchangeably with each other with the same meaning.

The term "repeating unit" used in describing "polymer" in the present specification refers to a structure in which the same structure appears twice or more repeatedly throughout the length of the polymer, and the polymer may not necessarily be composed of only the repeating unit. For example, the polymer may further comprise branched structures and/or end-capping agents in addition to the given repeating units.

As used herein, the term "substituted", for example "substituted alkyl," means that one or more hydrogen atoms of the alkyl are each independently replaced by a non-hydrogen substituent. The substituent may include, but is not limited to, any of the substituents described herein, for example, halogen, hydroxyl, alkyl, hydroxyalkyl, haloalkyl, cyanoalkyl, alkoxyalkyl, carbonyl (e.g., carboxyl, alkoxycarbonyl, formyl, or acyl), thiocarbonyl (e.g., thioester, thioacetate, or thioformate), alkoxyl, phosphoryl, phosphate, phosphonate, phosphinate, amino, amido, amidine, imine, cyano, nitro, azido, sulfhydryl, alkylthio, sulfate, sulfonate, sulfamoyl, sulfonamido, sulfonyl, heterocyclyl, aralkyl, aryl, or heteroaryl. The substituted moiety on the hydrocarbon chain may itself be substituted, if desired.

In the present invention, an "alkyl group" is a hydrocarbon having substituted or unsubstituted primary, secondary, tertiary and/or quaternary carbon atoms, and includes a saturated aliphatic group which may be straight-chain, branched, cyclic, or a combination thereof. For example, the alkyl group may have 1 to 20 carbon atoms (i.e., a C1-C20 alkyl), 1 to 10 carbon atoms (i.e., a C1-C10 alkyl), or 1 to 6 carbon atoms (i.e., a C1-C6 alkyl). Examples of suitable alkyl groups may include, but are not limited to, methyl (Me, -CH3), ethyl (Et, -CH2CH3), 1-propyl (n-Pr, n-propyl, -CH2CH2CH3), 2-propyl (i-Pr, i-propyl, -CH(CH3)2), 1-butyl (n-Bu, n-butyl, -CH2CH2CH2CH3), 2-methyl-1-propyl (i-Bu, i-butyl, -CH2CH(CH3)2), 2-butyl (s-Bu, s-butyl, -CH(CH3)CH2CH3), 2-methyl-2-propyl (t-Bu, t-butyl, -C(CH3)3), 1-pentyl (n-pentyl, - CH2CH2CH2CH2CH3), 2-pentyl (-CH(CH3)CH2CH2CH3), 3-pentyl (-CH(CH2CH3)2), 2-methyl-2-butyl (-C(CH3)2CH2CH3), 3-methyl-2-butyl (-CH(CH3)CH(CH3)2), 3-methyl-1-butyl (-CH2CH2CH(CH3)2), 2-methyl-1-butyl (-CH2CH(CH3)CH2CH3), 1-hexyl (-CH2CH2CH2CH2CH2CH3), 2-hexyl (-CH(CH3)CH2CH2CH2CH3), 3-hexyl (-CH(CH2CH3)(CH2CH2CH3)), 2-methyl-2-pentyl (-C(CH3)2CH2CH2CH3), 3-methyl-2-pentyl (-CH(CH3)CH(CH3)CH2CH3), 4-methyl-2-pentyl (-CH(CH3)CH2CH(CH3)2), 3-methyl-3-pentyl (-C(CH3)(CH2CH3)2), 2-methyl-3-pentyl (-CH(CH2CH3)CH(CH3)2), 2,3-dimethyl-2-butyl (-C(CH3)2CH(CH3)2), 3,3-dimethyl-2-butyl (-CH(CH3)C(CH3)3), and octyl (-(CH2)7CH3).

Moreover, as used throughout the specification, examples and claims, the term "alkyl" is intended to include both unsubstituted and substituted alkyl groups, the latter of which refers to an alkyl moiety having a substituent that replaces a hydrogen on one or more carbons of the hydrocarbon backbone, which includes a haloalkyl group such as trifluoromethyl.

As used herein, the term "alkylene" refers to a saturated hydrocarbon group having two valences, which may be branched, straight-chain, cyclic, or a combination thereof, and which is derived by removing two hydrogen atoms from the same carbon atom or two different carbon atoms of a parent alkane. For example, an alkylene group may have 1 to 20 carbon atoms, 1 to 10 carbon atoms, or 1 to 6 carbon atoms. Examples of suitable alkylene groups may include, but are not limited to, methylene (-CH2-) and 1,2-ethylene (-CH2-CH2-).

As used herein, the term "heterocycle" refers to a substituted or unsubstituted, monovalent or divalent, saturated or partially saturated non-aromatic ring structure, preferably a 3- to 10-membered ring, more preferably a 3- to 7-membered ring, wherein the ring structure comprises one or more heteroatoms, preferably 1 to 4 heteroatoms, and more preferably 1 to 2 heteroatoms. The terms "heterocyclyl," "heterocycle," "heterocyclic," and "heterocycloalkyl" also include polycyclic ring systems having two or more cyclic rings in which two or more carbons are common to two adjacent rings, wherein at least one of the rings is heterocyclic, for example, the other cyclic rings may be cycloalkyl, cycloalkenyl, cycloalkynyl, aryl, heteroaryl, and/or heterocyclyl. The bicyclic and polycyclic heterocyclic ring systems may be fused, bridged, or spiro ring systems. The substituted heterocycles include heterocyclic rings substituted with any of the substituents disclosed herein, including, for example, a carbonyl group. The heterocyclyl groups include, for example, piperidine, piperazine, pyrrolidine, morpholine, lactones, lactams, and the like. Additionally exemplary heterocycles may include, but are not limited to, dihydropyridyl, dihydroindolyl, tetrahydropyridyl (piperidyl), tetrahydrothiophenyl, sulfur-oxidized tetrahydrothiophenyl, indolenyl, piperidinyl, 4-piperidinyl, pyrrolidinyl, 2-pyrrolidonyl, pyrrolinyl, tetrahydrofuranyl, tetrahydroquinolinyl, tetrahydroisoquinolinyl, decahydroquinolinyl, octahydroisoquinolinyl, 6H-1,2,5-thiadiazinyl, 2H,6H-1,5,2-dithiazinyl, pyranyl, chromenyl, xanthenyl, phenoxathinyl, 2H-pyrrolyl, 3H-indolyl, 4H-quinolizinyl, phthalazinyl, naphthyridinyl, quinoxalinyl, quinazolinyl, cinnolinyl, pteridinyl, 4aH-carbazolyl, carbazolyl, β-carbolinyl, phenanthridinyl, acridinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, quinuclidinyl, morpholinyl, and oxazolidinyl (each of which may be substituted or unsubstituted).

As used herein, the term "carbocycle" refers to a non-aromatic saturated or unsaturated, monovalent or divalent ring which may be monocyclic, bicyclic, or polycyclic and in which each of the ring atoms is carbon. Cycloalkyl groups may have from 3 to 7 carbon atoms as a monocycle, from 7 to 12 carbon atoms as a bicycle, and up to about 20 carbon atoms as a polycycle. Monocyclic cycloalkyls have from 3 to 7 ring atoms, more typically 5 or 6 ring atoms. Bicyclic cycloalkyls may have from 7 to 12 ring atoms and may be fused ring systems, spirocyclic ring systems, or bridged ring systems. In exemplary cycloalkyl groups, the atoms may be arranged in a bicyclo [4,5], [5,5], [5,6], or [6,6] system. In certain embodiments, the cycloalkyl contains from 3 to 20 atoms, or from 3 to 10 atoms, or more preferably from 3 to 7 atoms. Examples of cycloalkyls may include cyclopropyl, cyclobutyl, cyclopentyl, cyclohexyl, cycloheptyl, cyclooctyl, and the like. Unless otherwise specified, cycloalkyl may be substituted by one or more substituents described herein.

As used herein, the term "alkoxyacyl" refers to a group of -C(=O)OR. R is alkyl as defined herein, which may be substituted or unsubstituted.
" - - - - -" as used in the chemical structural formulas herein represents a single bond or a double bond, according to the convention used in the art.
" 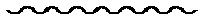 " as used in the chemical structural formulas herein represents a bond connected to another unit, according to the convention used in the art.

As used herein, the term "amine" refers to -NH2, in which the hydrogen atom is unsubstituted or substituted with a substituent such as alkyl or aryl, wherein the substituent such as alkyl or aryl that substitutes the hydrogen atom is as defined herein, and may be substituted or unsubstituted.

As used herein, the term "amine monomer" is a monomer containing an amine functional group, and preferably, may be selected from the group consisting of amine monomers described in Table 1 below.

**[Table 1]**

| No. | Structural formula |
|---|---|
| 1 | 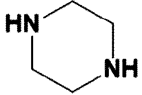 |
| 2 | 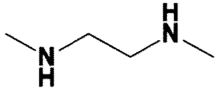 |
| 3 | 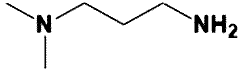 |
| 4 | 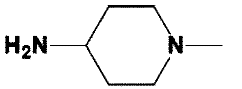 |
| 5 | 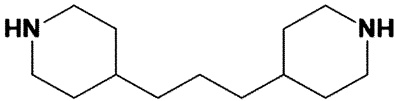 |
| 6 | 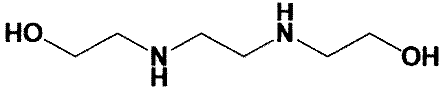 |
| 7 | 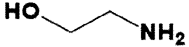 |
| 8 | 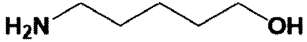 |
| 9 | 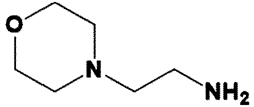 |
| 10 | 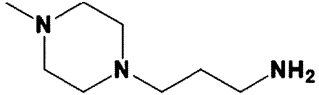 |
| 11 | 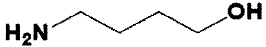 |
| 12 | 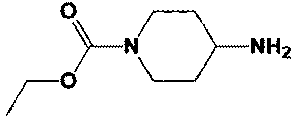 |

As used herein, the term "acrylate monomer" is a monomer in the form of an ester derivative of acrylic acid, and may be selected from the group consisting of acrylate monomers described in Table 2 below.

**[Table 2]**

| No. | Structural formula |
|---|---|
| A | 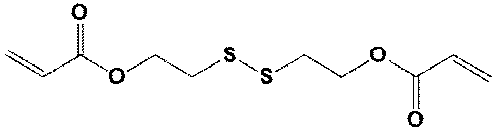 |
| B | 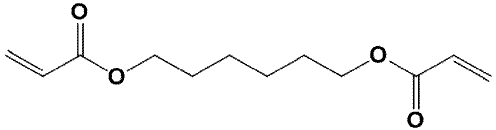 |
| C | 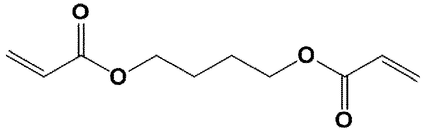 |
| D | 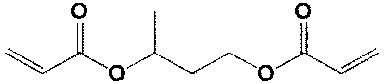 |
| E | 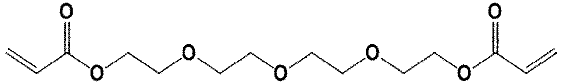 |

As used herein, the term "alkynoate monomer" is a monomer in the form of an ester derivative of alkynoic acid, and may be selected from the group consisting of alkynoate monomers described in Table 3 below.

**[Table 3]**

| No. | Structural formula |
|---|---|
| F | 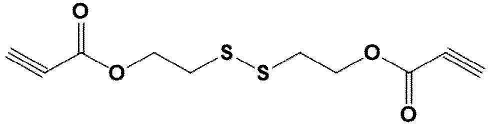 |
| G | 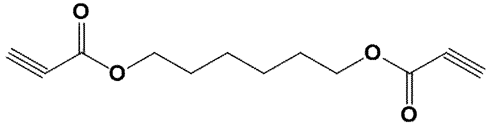 |
| H | 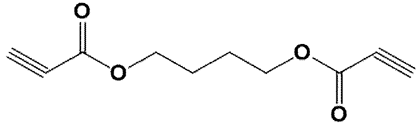 |
| I | 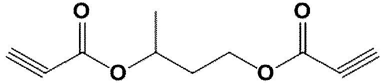 |
| J | 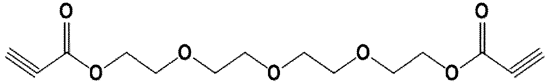 |

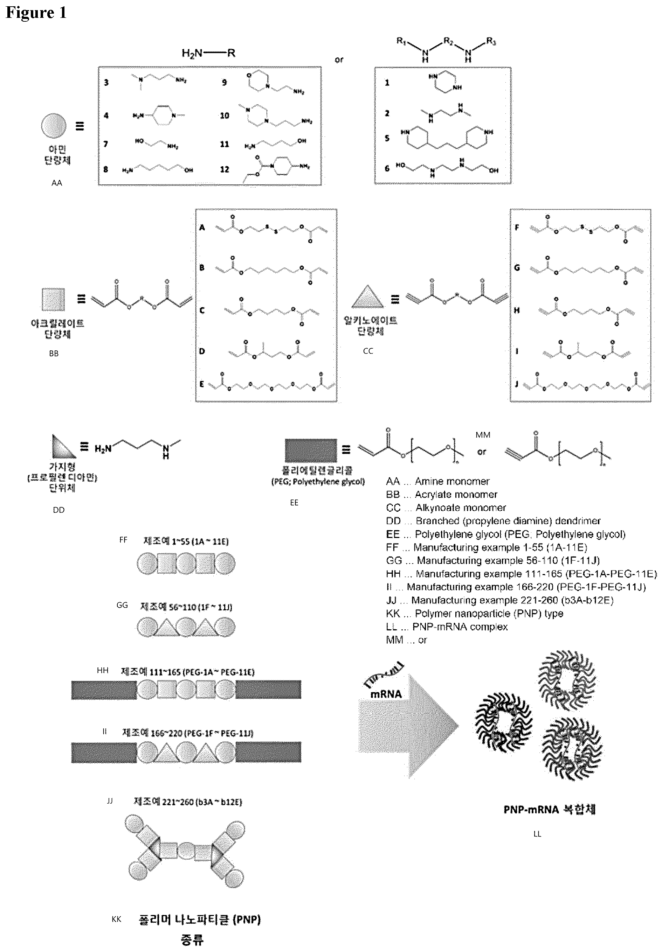

The polymer according to the present invention is characterized by including an amine monomer and an acrylate or alkynoate monomer together, and the two monomers are alternately connected (see schematic diagram of Figure 1).

The polymer according to the present invention is characterized by having an ionizable moiety, where the "ionizable moiety" means a part of the polymer that is ionized depending on the pH. For example, it may be a tertiary amine present in the polymer chemical structure, and those of ordinary skill in the art are well aware of which moiety can be ionized depending on the pH.

Hereinafter, the present invention will be described in more detail through the examples, but the following examples are for illustrative purposes only and are not intended to limit the scope of the present invention.

### [Manufacturing Example 1]

### Preparation of polymer 1A

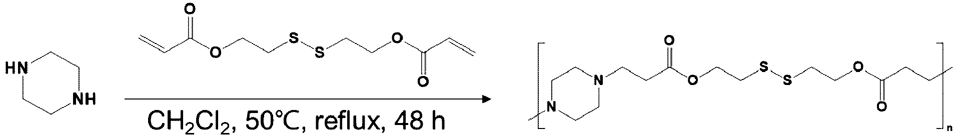

Acrylate monomer A (1000 mg) was placed in a 2-neck round bottom flask, and then amine monomer 1 was added to the flask so that the molar ratio with the acrylate monomer was 1.04. Dichloromethane (CH₂Cl₂, DCM) was added to the flask so that the final concentration was 1000 mg/ml. Thereafter, the reaction was carried out at 50 °C for 48 hours with reflux, and then polymer 1A having the ionizable moiety of the present invention was obtained.

### [Manufacturing Example 2]

### Preparation of polymer 1B

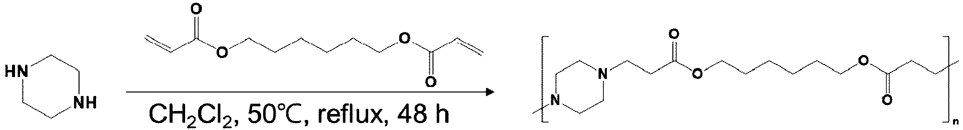

Acrylate monomer B (1000 mg) was placed in a 2-neck round bottom flask, and then amine monomer 1 was added to the flask so that the molar ratio with the acrylate monomer was 1.04. DCM was added to the flask so that the final concentration was 1000 mg/ml. Thereafter, the reaction was carried out at 50 °C for 48 hours with reflux, and then polymer 1B having the ionizable moiety of the present invention was obtained.

### [Manufacturing Example 3]

### Preparation of polymer 1C

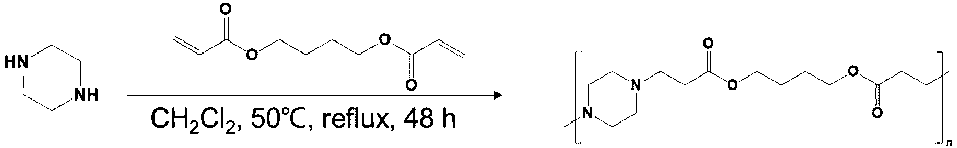

Acrylate monomer C (1000 mg) was placed in a 2-neck round bottom flask, and then amine monomer 1 was added to the flask so that the molar ratio with the acrylate monomer was 1.04. DCM was added to the flask so that the final concentration was 1000 mg/ml. Thereafter, the reaction was carried out at 50 °C for 48 hours with reflux, and then polymer 1C having the ionizable moiety of the present invention was obtained.

### [Manufacturing Example 4]

### Preparation of polymer 1D

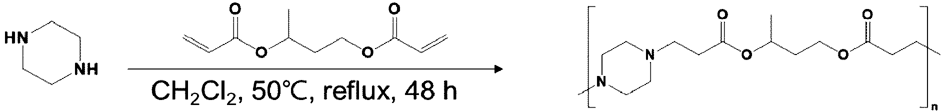

Acrylate monomer D (1000 mg) was placed in a 2-neck round bottom flask, and then amine monomer 1 was added to the flask so that the molar ratio with the acrylate monomer was 1.04. DCM was added to the flask so that the final concentration was 1000 mg/ml. Thereafter, the reaction was carried out at 50 °C for 48 hours with reflux, and then polymer 1D having the ionizable moiety of the present invention was obtained.

### [Manufacturing Example 5]

### Preparation of polymer 1E

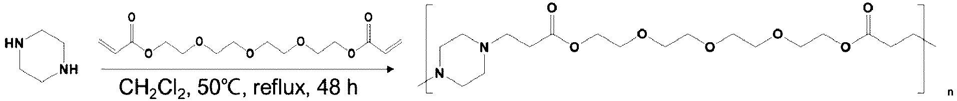

Acrylate monomer E (1000 mg) was placed in a 2-neck round bottom flask, and then amine monomer 1 was added to the flask so that the molar ratio with the acrylate monomer was 1.04. DCM was added to the flask so that the final concentration was 2500 mg/ml. Thereafter, the reaction was carried out at 50 °C for 48 hours with reflux, and then polymer 1E having the ionizable moiety of the present invention was obtained.

### [Manufacturing Example 6]

### Preparation of polymer 2A

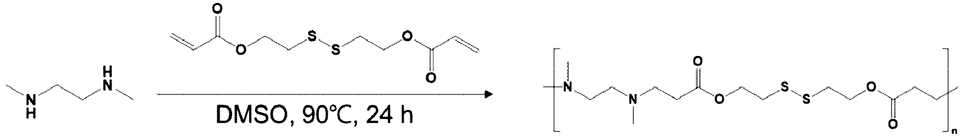

Acrylate monomer A (1000 mg) was placed in a 20 ml vial, and then amine monomer 2 was added to the vial so that the molar ratio with the acrylate monomer was 1.04. Dimethylsulfoxide (DMSO) was added to the vial so that the final concentration was 1000 mg/ml. Thereafter, the reaction was carried out at 90 °C for 24 hours, and then polymer 2A having the ionizable moiety of the present invention was obtained.

### [Manufacturing Example 7]

### Preparation of polymer 2B

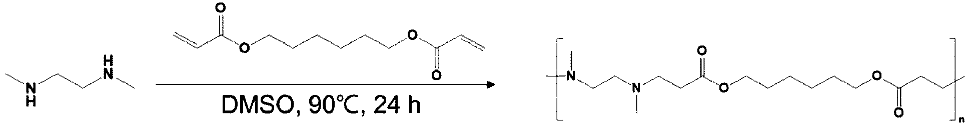

Acrylate monomer B (1000 mg) was placed in a 20 ml vial, and then amine monomer 2 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 2B having the ionizable moiety of the present invention was obtained.

### [Manufacturing Example 8]

### Preparation of polymer 2C

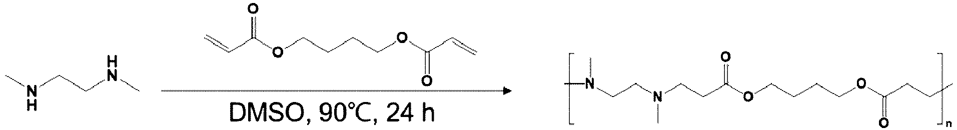

Acrylate monomer C (1000 mg) was placed in a 20 ml vial, and then amine monomer 2 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 2C having the ionizable moiety of the present invention was obtained.

### [Manufacturing Example 9]

### Preparation of polymer 2D

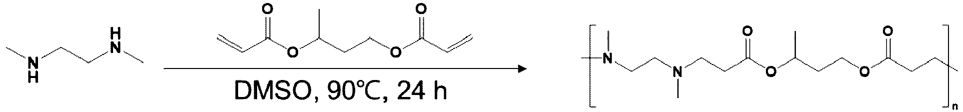

Acrylate monomer D (1000 mg) was placed in a 20 ml vial, and then amine monomer 2 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 2D having the ionizable moiety of the present invention was obtained.

### [Manufacturing Example 10]

### Preparation of polymer 2E

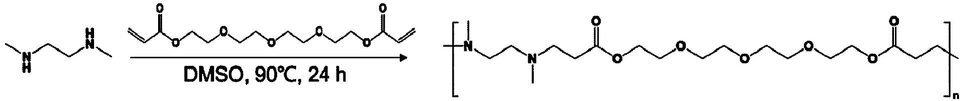

Acrylate monomer E (1000 mg) was placed in a 20 ml vial, and then amine monomer 2 was added to the vial so that the molar ratio with the acrylate monomer was 1.04. DMSO was added to the vial so that the final concentration was 2500 mg/ml. Thereafter, the reaction was carried out at 90 °C for 24 hours, and then polymer 2E having the ionizable moiety of the present invention was obtained.

### [Manufacturing Example 11]

### Preparation of polymer 3A

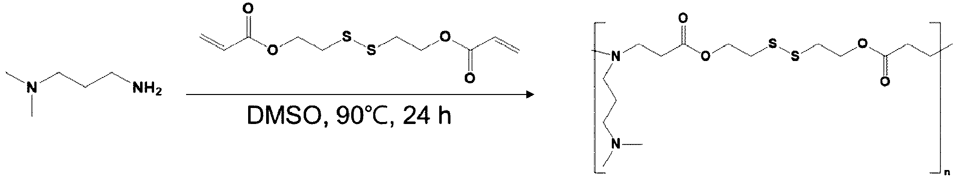

Acrylate monomer A (1000 mg) was placed in a 20 ml vial, and then amine monomer 3 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 3A having the ionizable moiety of the present invention was obtained.

### [Manufacturing Example 12]

### Preparation of polymer 3B

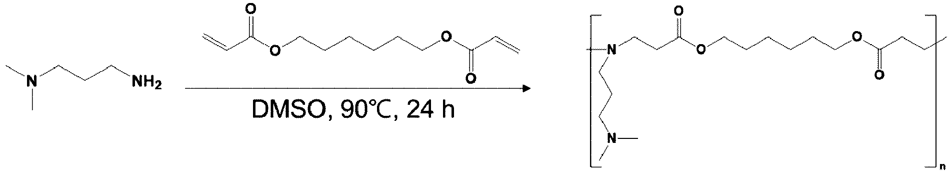

Acrylate monomer B (1000 mg) was placed in a 20 ml vial, and then amine monomer 3 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 3B having the ionizable moiety of the present invention was obtained.

### [Manufacturing Example 13]

### Preparation of polymer 3C

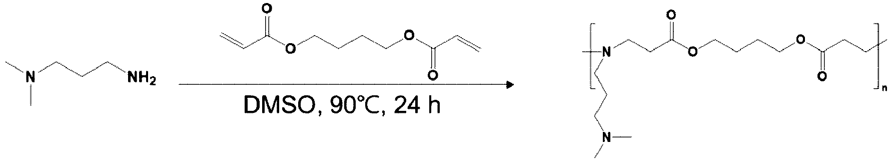

Acrylate monomer C (1000 mg) was placed in a 20 ml vial, and then amine monomer 3 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 3C having the ionizable moiety of the present invention was obtained.

### [Manufacturing Example 14]

### Preparation of polymer 3D

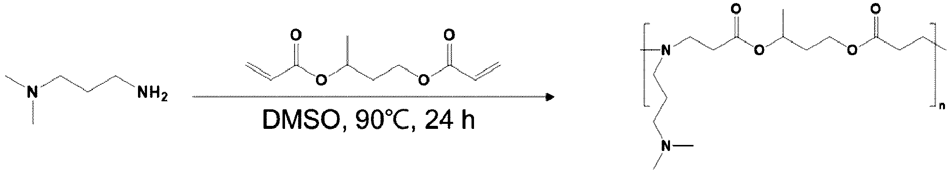

Acrylate monomer D (1000 mg) was placed in a 20 ml vial, and then amine monomer 3 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 3D having the ionizable moiety of the present invention was obtained.

### [Manufacturing Example 15]

### Preparation of polymer 3E

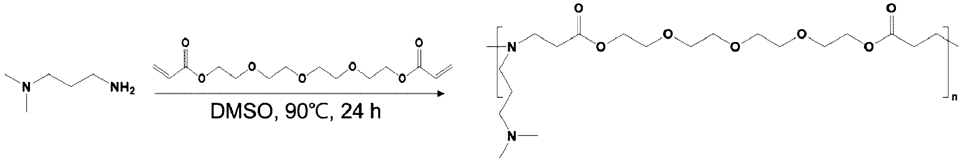

Acrylate monomer E (1000 mg) was placed in a 20 ml vial, and then amine monomer 3 was added to the vial so that the molar ratio with the acrylate monomer was 1.04. DMSO was added to the vial so that the final concentration was 2500 mg/ml. Thereafter, the reaction was carried out at 90 °C for 24 hours, and then polymer 3E having the ionizable moiety of the present invention was obtained.

### [Manufacturing Example 16]

### Preparation of polymer 4A

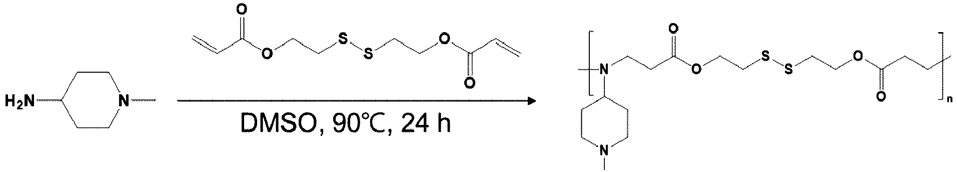

Acrylate monomer A (1000 mg) was placed in a 20 ml vial, and then amine monomer 4 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 4A having the ionizable moiety of the present invention was obtained.

### [Manufacturing Example 17]

### Preparation of polymer 4B

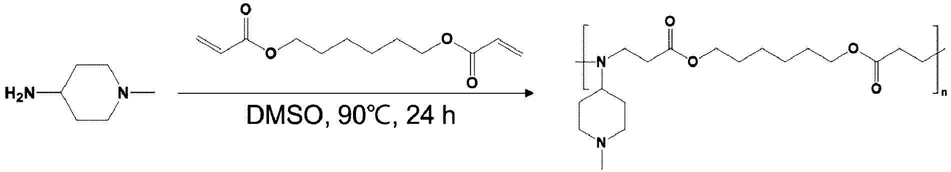

Acrylate monomer B (1000 mg) was placed in a 20 ml vial, and then amine monomer 4 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 4B having the ionizable moiety of the present invention was obtained.

### [Manufacturing Example 18]

### Preparation of polymer 4C

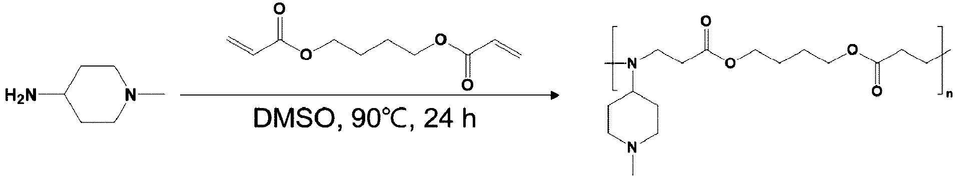

Acrylate monomer C (1000 mg) was placed in a 20 ml vial, and then amine monomer 4 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 4C having the ionizable moiety of the present invention was obtained.

### [Manufacturing Example 19]

### Preparation of polymer 4D

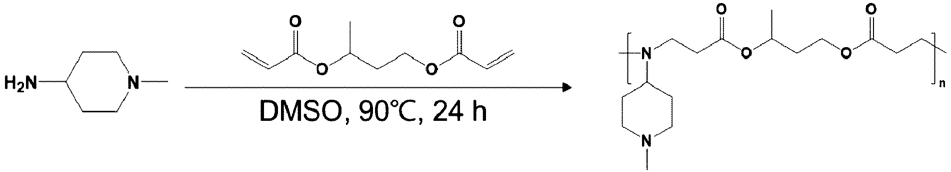

Acrylate monomer D (1000 mg) was placed in a 20 ml vial, and then amine monomer 4 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 4D having the ionizable moiety of the present invention was obtained.

### [Manufacturing Example 20]

### Preparation of polymer 4A

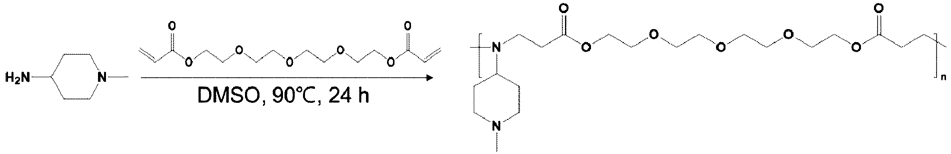

Acrylate monomer E (1000 mg) was placed in a 20 ml vial, and then amine monomer 4 was added to the vial so that the molar ratio with the acrylate monomer was 1.04. DMSO was added to the vial so that the final concentration was 2500 mg/ml. Thereafter, the reaction was carried out at 90 °C for 24 hours, and then polymer 4E having the ionizable moiety of the present invention was obtained.

### [Manufacturing Example 21]

### Preparation of polymer 5A

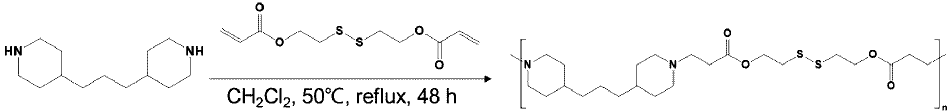

Acrylate monomer A (1000 mg) was placed in a 2-neck round bottom flask, and then amine monomer 5 was added to the flask so that the molar ratio with the acrylate monomer was 1.04. DCM was added to the flask so that the final concentration was 1000 mg/ml. Thereafter, the reaction was carried out at 50 °C for 48 hours with reflux, and then polymer 5A having the ionizable moiety of the present invention was obtained.

### [Manufacturing Example 22]

### Preparation of polymer 5B

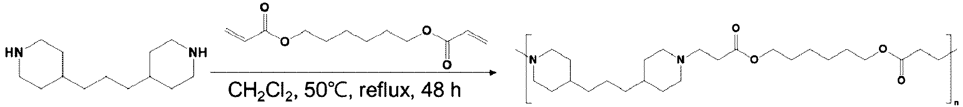

Acrylate monomer B (1000 mg) was placed in a 2-neck round bottom flask, and then amine monomer 5 was added to the flask so that the molar ratio with the acrylate monomer was 1.04. DCM was added to the flask so that the final concentration was 1000 mg/ml. Thereafter, the reaction was carried out at 50 °C for 48 hours with reflux, and then polymer 5B having the ionizable moiety of the present invention was obtained.

### [Manufacturing Example 23]

### Preparation of polymer 5C

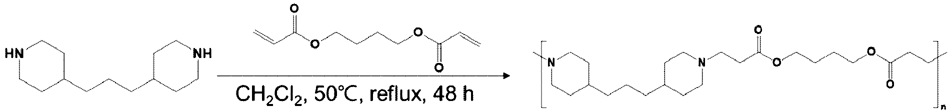

Acrylate monomer C (1000 mg) was placed in a 2-neck round bottom flask, and then amine monomer 5 was added to the flask so that the molar ratio with the acrylate monomer was 1.04. DCM was added to the flask so that the final concentration was 1000 mg/ml. Thereafter, the reaction was carried out at 50 °C for 48 hours with reflux, and then polymer 5C having the ionizable moiety of the present invention was obtained.

### [Manufacturing Example 24]

### Preparation of polymer 5D

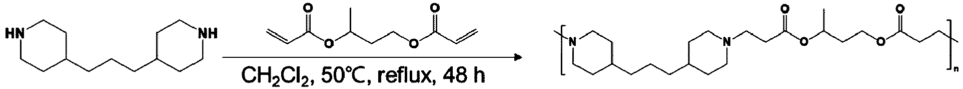

Acrylate monomer D (1000 mg) was placed in a 2-neck round bottom flask, and then amine monomer 5 was added to the flask so that the molar ratio with the acrylate monomer was 1.04. DCM was added to the flask so that the final concentration was 1000 mg/ml. Thereafter, the reaction was carried out at 50 °C for 48 hours with reflux, and then polymer 5D having the ionizable moiety of the present invention was obtained.

### [Manufacturing Example 25]

### Preparation of polymer 5E

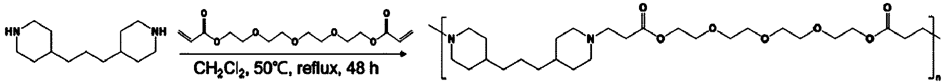

Acrylate monomer E (1000 mg) was placed in a 2-neck round bottom flask, and then amine monomer 5 was added to the flask so that the molar ratio with the acrylate monomer was 1.04. DCM was added to the flask so that the final concentration was 1000 mg/ml. Thereafter, the reaction was carried out at 50 °C for 48 hours with reflux, and then polymer 5E having the ionizable moiety of the present invention was obtained.

### [Manufacturing Example 26]

### Preparation of polymer 6A

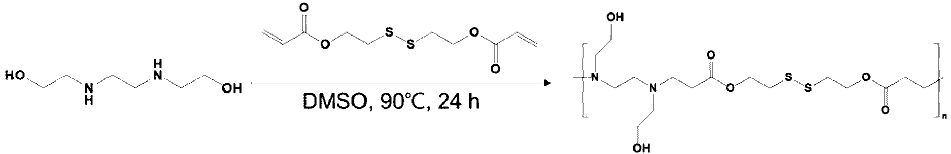

Acrylate monomer A (1000 mg) was placed in a 20 ml vial, and then amine monomer 6 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 6A having the ionizable moiety of the present invention was obtained.

### [Manufacturing Example 27]

### Preparation of polymer 6B

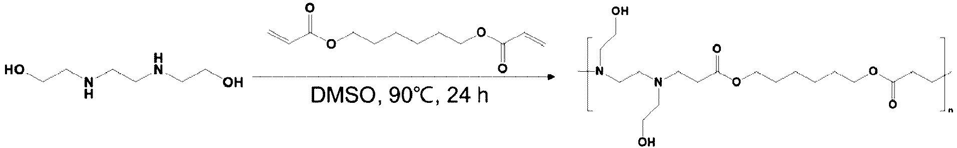

Acrylate monomer B (1000 mg) was placed in a 20 ml vial, and then amine monomer 6 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 6B having the ionizable moiety of the present invention was obtained.

### [Manufacturing Example 28]

### Preparation of polymer 6C

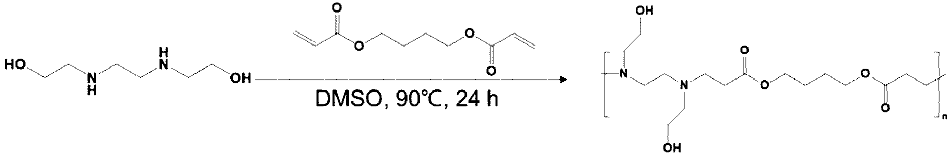

Acrylate monomer C (1000 mg) was placed in a 20 ml vial, and then amine monomer 6 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 6C having the ionizable moiety of the present invention was obtained.

### [Manufacturing Example 29]

### Preparation of polymer 6D

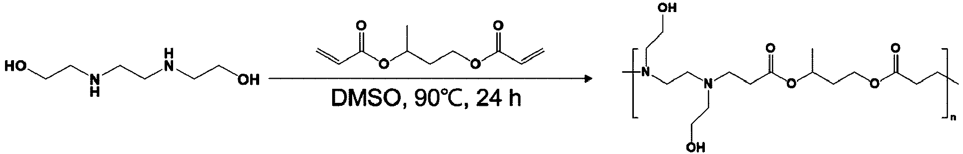

Acrylate monomer D (1000 mg) was placed in a 20 ml vial, and then amine monomer 6 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 6D having the ionizable moiety of the present invention was obtained.

### [Manufacturing Example 30]

### Preparation of polymer 6E

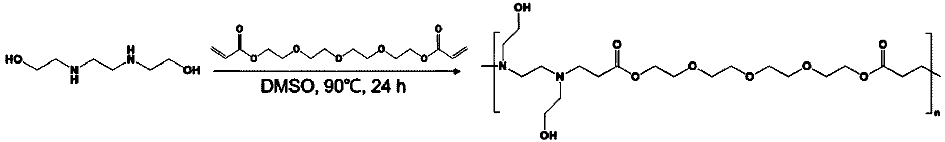

Acrylate monomer E (1000 mg) was placed in a 20 ml vial, and then amine monomer 6 was added to the vial so that the molar ratio with the acrylate monomer was 1.04. DMSO was added to the vial so that the final concentration was 2500 mg/ml. Thereafter, the reaction was carried out at 90 °C for 24 hours, and then polymer 6E having the ionizable moiety of the present invention was obtained.

### [Manufacturing Example 31]

### Preparation of polymer 7A

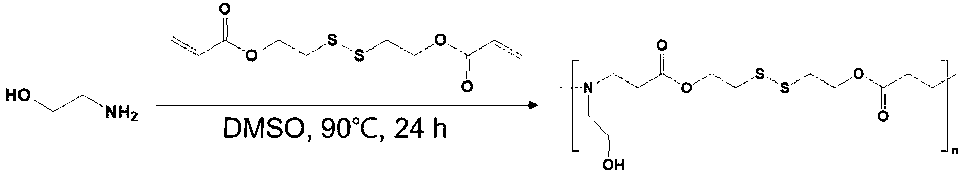

Acrylate monomer A (1000 mg) was placed in a 20 ml vial, and then amine monomer 7 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 7A having the ionizable moiety of the present invention was obtained.

### [Manufacturing Example 32]

### Preparation of polymer 7B

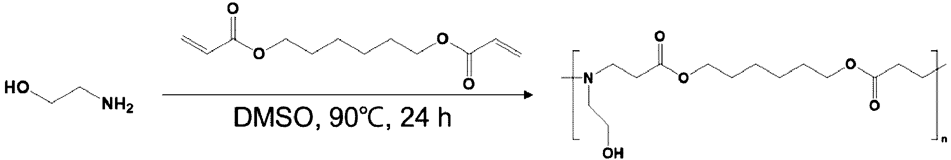

Acrylate monomer B (1000 mg) was placed in a 20 ml vial, and then amine monomer 7 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 7B having the ionizable moiety of the present invention was obtained.

### [Manufacturing Example 33]

### Preparation of polymer 7C

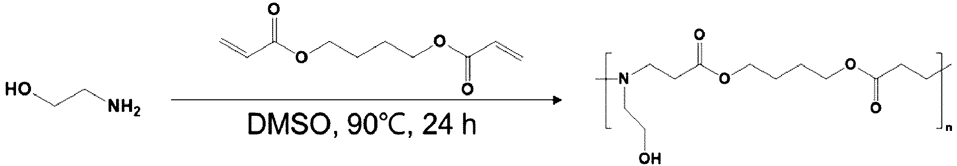

Acrylate monomer C (1000 mg) was placed in a 20 ml vial, and then amine monomer 7 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 7C having the ionizable moiety of the present invention was obtained.

### [Manufacturing Example 34]

### Preparation of polymer 7D

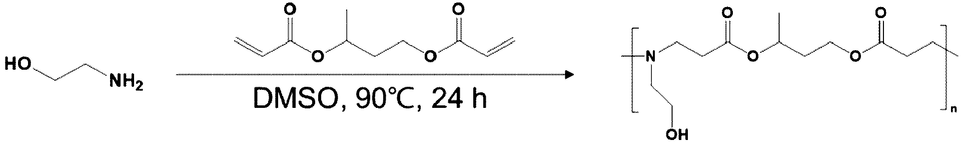

Acrylate monomer D (1000 mg) was placed in a 20 ml vial, and then amine monomer 7 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 7D having the ionizable moiety of the present invention was obtained.

### [Manufacturing Example 35]

### Preparation of polymer 7E

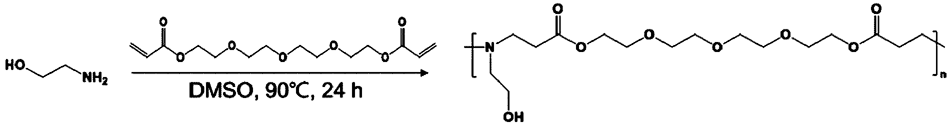

Acrylate monomer E (1000 mg) was placed in a 20 ml vial, and then amine monomer 7 was added to the vial so that the molar ratio with the acrylate monomer was 1.04. DMSO was added to the vial so that the final concentration was 2500 mg/ml. Thereafter, the reaction was carried out at 90 °C for 24 hours, and then polymer 7E having the ionizable moiety of the present invention was obtained.

### [Manufacturing Example 36]

### Preparation of polymer 8A

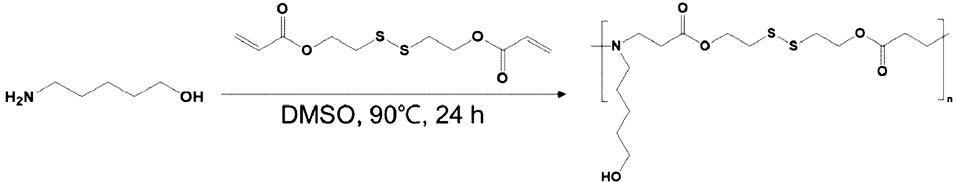

Acrylate monomer A (1000 mg) was placed in a 20 ml vial, and then amine monomer 8 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 8A having the ionizable moiety of the present invention was obtained.

### [Manufacturing Example 37]

### Preparation of polymer 8B

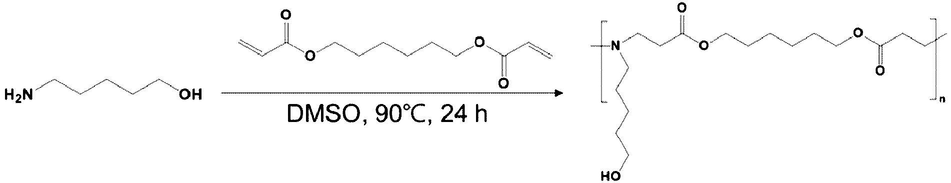

Acrylate monomer B (1000 mg) was placed in a 20 ml vial, and then amine monomer 8 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 8B having the ionizable moiety of the present invention was obtained.

### [Manufacturing Example 38]

### Preparation of polymer 8C

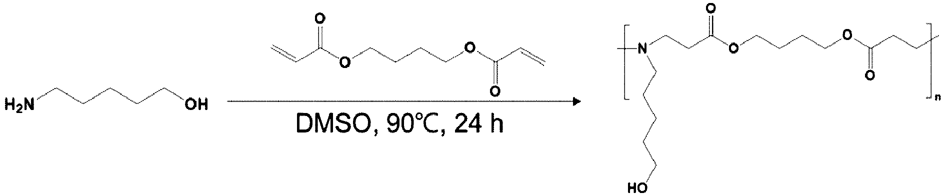

Acrylate monomer C (1000 mg) was placed in a 20 ml vial, and then amine monomer 8 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 8C having the ionizable moiety of the present invention was obtained.

### [Manufacturing Example 39]

### Preparation of polymer 8D

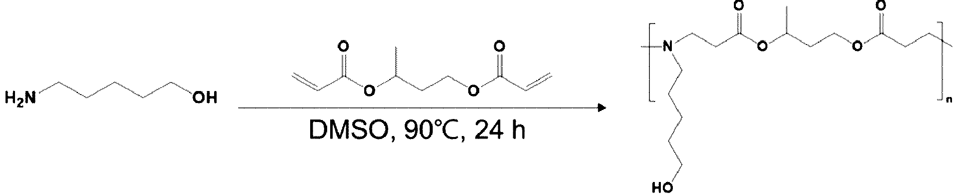

Acrylate monomer D (1000 mg) was placed in a 20 ml vial, and then amine monomer 8 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 8D having the ionizable moiety of the present invention was obtained.

### [Manufacturing Example 40]

### Preparation of polymer 8E

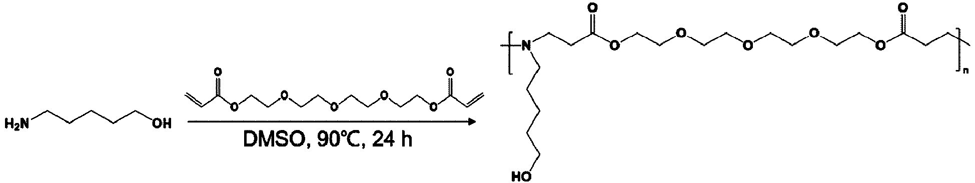

Acrylate monomer E (1000 mg) was placed in a 20 ml vial, and then amine monomer 8 was added to the vial so that the molar ratio with the acrylate monomer was 1.04. DMSO was added to the vial so that the final concentration was 2500 mg/ml. Thereafter, the reaction was carried out at 90 °C for 24 hours, and then polymer 8E having the ionizable moiety of the present invention was obtained.

### [Manufacturing Example 41]

### Preparation of polymer 9A

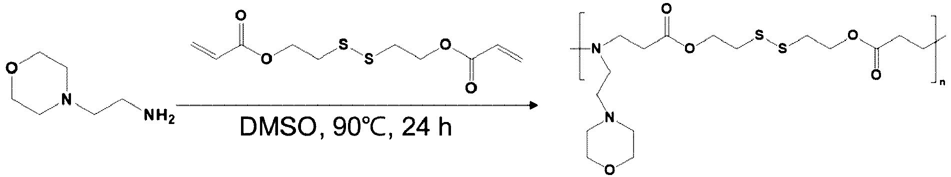

Acrylate monomer A (1000 mg) was placed in a 20 ml vial, and then amine monomer 9 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 9A having the ionizable moiety of the present invention was obtained.

### [Manufacturing Example 42]

### Preparation of polymer 9B

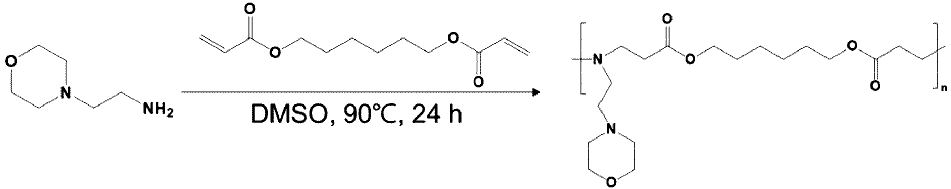

Acrylate monomer B (1000 mg) was placed in a 20 ml vial, and then amine monomer 9 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 9B having the ionizable moiety of the present invention was obtained.

### [Manufacturing Example 43]

### Preparation of polymer 9C

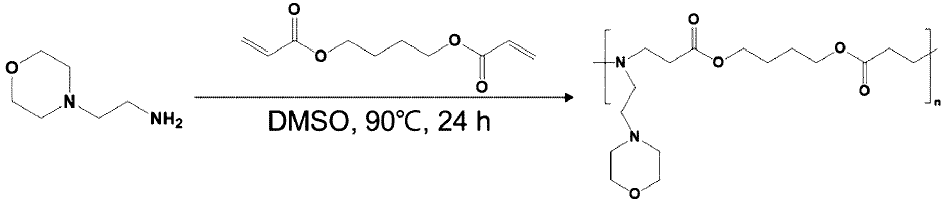

Acrylate monomer C (1000 mg) was placed in a 20 ml vial, and then amine monomer 9 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 9C having the ionizable moiety of the present invention was obtained.

### [Manufacturing Example 44]

### Preparation of polymer 9D

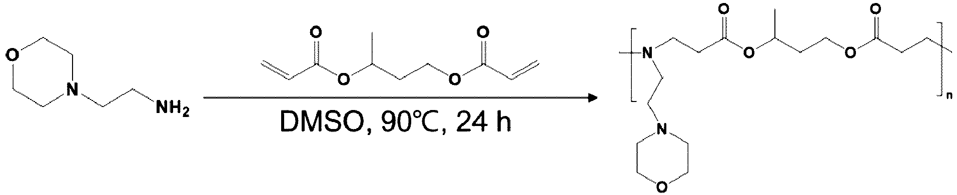

Acrylate monomer D (1000 mg) was placed in a 20 ml vial, and then amine monomer 9 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 9D having the ionizable moiety of the present invention was obtained.

### [Manufacturing Example 45]

### Preparation of polymer 9E

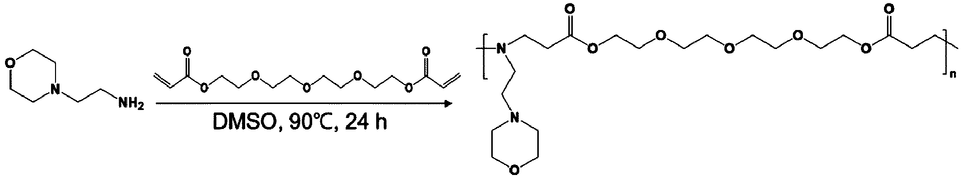

Acrylate monomer E (1000 mg) was placed in a 20 ml vial, and then amine monomer 9 was added to the vial so that the molar ratio with the acrylate monomer was 1.04. DMSO was added to the vial so that the final concentration was 2500 mg/ml. Thereafter, the reaction was carried out at 90 °C for 24 hours, and then polymer 9E having the ionizable moiety of the present invention was obtained.

### [Manufacturing Example 46]

### Preparation of polymer 10A

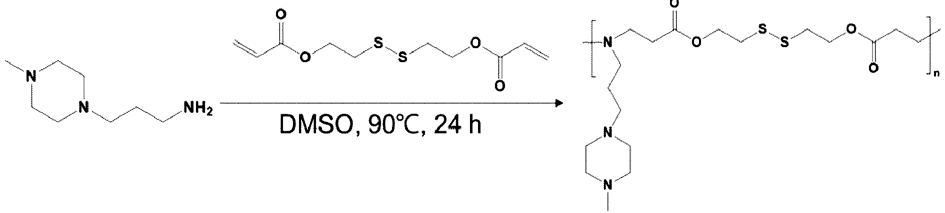

Acrylate monomer A (1000 mg) was placed in a 20 ml vial, and then amine monomer 10 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 10Ahaving the ionizable moiety of the present invention was obtained.

### [Manufacturing Example 47]

### Preparation of polymer 10B

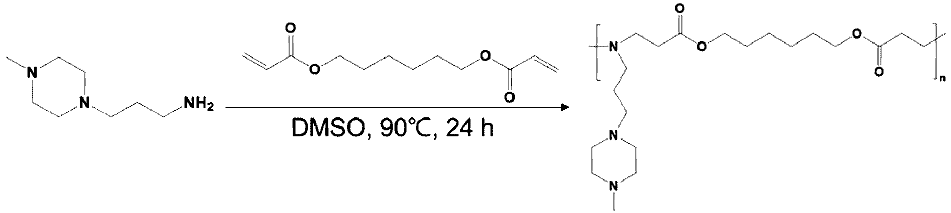

Acrylate monomer B (1000 mg) was placed in a 20 ml vial, and then amine monomer 10 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 10B having the ionizable moiety of the present invention was obtained.

### [Manufacturing Example 48]

### Preparation of polymer 10C

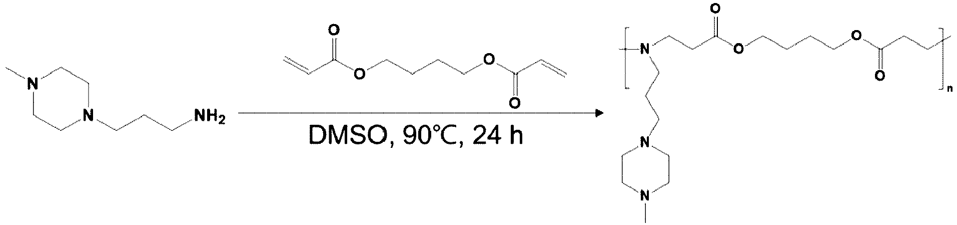

Acrylate monomer C (1000 mg) was placed in a 20 ml vial, and then amine monomer 10 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 10C having the ionizable moiety of the present invention was obtained.

### [Manufacturing Example 49]

### Preparation of polymer 10D

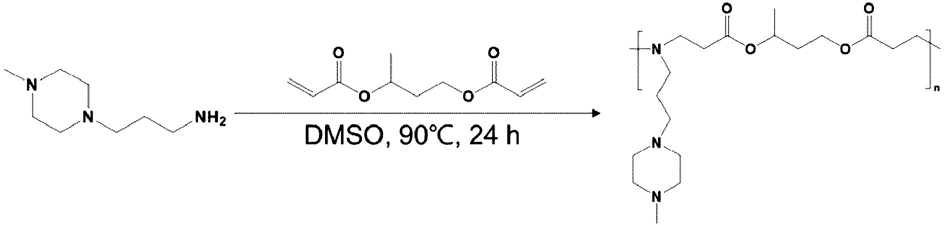

Acrylate monomer D (1000 mg) was placed in a 20 ml vial, and then amine monomer 10 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 10D having the ionizable moiety of the present invention was obtained.

### [Manufacturing Example 50]

### Preparation of polymer 10E

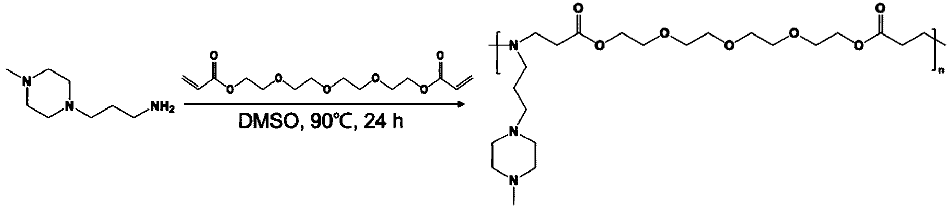

Acrylate monomer E (1000 mg) was placed in a 20 ml vial, and then amine monomer 10 was added to the vial so that the molar ratio with the acrylate monomer was 1.04. DMSO was added to the vial so that the final concentration was 2500 mg/ml. Thereafter, the reaction was carried out at 90 °C for 24 hours, and then polymer 10E having the ionizable moiety of the present invention was obtained.

### [Manufacturing Example 51]

### Preparation of polymer 11A

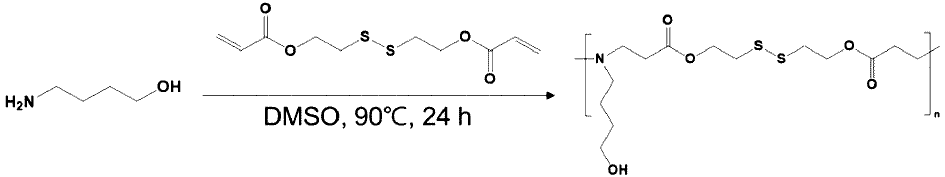

Acrylate monomer A (1000 mg) was placed in a 20 ml vial, and then amine monomer 11 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 11Ahaving the ionizable moiety of the present invention was obtained.

### [Manufacturing Example 52]

### Preparation of polymer 11B

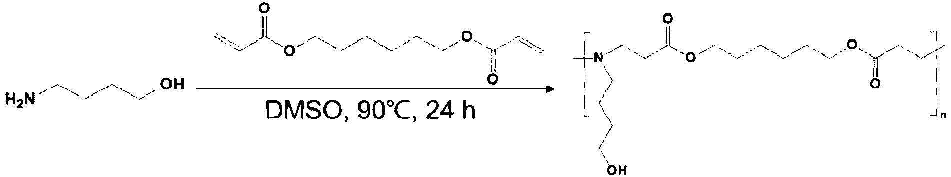

Acrylate monomer B (1000 mg) was placed in a 20 ml vial, and then amine monomer 11 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 11B having the ionizable moiety of the present invention was obtained.

### [Manufacturing Example 53]

### Preparation of polymer 11C

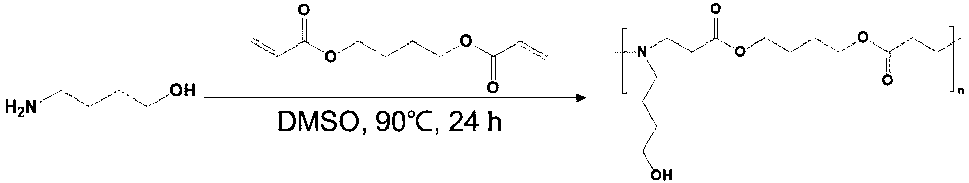

Acrylate monomer C (1000 mg) was placed in a 20 ml vial, and then amine monomer 11 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 11C having the ionizable moiety of the present invention was obtained.

### [Manufacturing Example 54]

### Preparation of polymer 11D

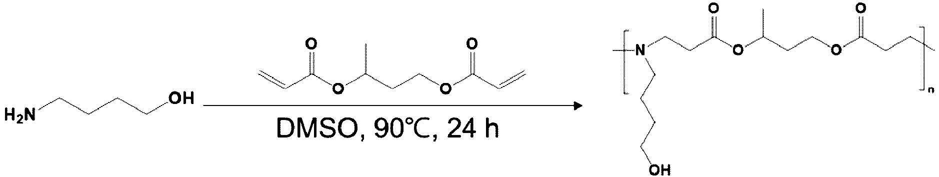

Acrylate monomer D (1000 mg) was placed in a 20 ml vial, and then amine monomer 11 was added to the vial so that the molar ratio with the acrylate monomer was 1.04. DMSO was added to the vial so that the final concentration was 1000 mg/ml. Thereafter, the reaction was carried out at 90 °C for 24 hours, and then polymer 11D having the ionizable moiety of the present invention was obtained.

### [Manufacturing Example 55]

### Preparation of polymer 11E

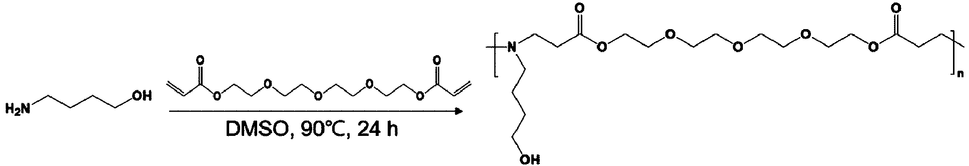

Acrylate monomer E (1000 mg) was placed in a 20 ml vial, and then amine monomer 11 was added to the vial so that the molar ratio with the acrylate monomer was 1.04. DMSO was added to the vial so that the final concentration was 2500 mg/ml. Thereafter, the reaction was carried out at 90 °C for 24 hours, and then polymer 11E having the ionizable moiety of the present invention was obtained.

### [Manufacturing Example 56]

### Preparation of polymer 1F

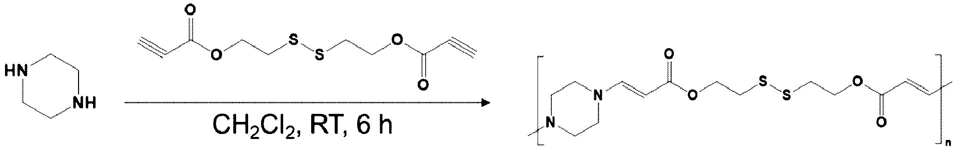

Alkynoate monomer F (500 mg) was placed in a round bottom flask, and then amine monomer 1 was added to the flask so that the molar ratio with the acrylate monomer was 1.04, and 4 mL of dichloromethane was added to the flask. Thereafter, the reaction was carried out at room temperature for 6 hours, and then polymer 1F having the ionizable moiety of the present invention was obtained.

### [Manufacturing Example 57]

### Preparation of polymer 1G

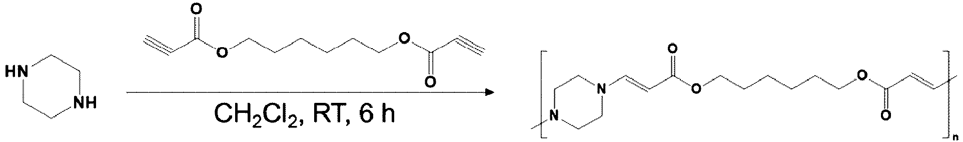

Alkynoate monomer G (500 mg) was placed in a round bottom flask, and then amine monomer 1 was added to the flask so that the molar ratio with the alkynoate monomer was 1.04, and 4 mL of dichloromethane was added to the flask. Thereafter, the reaction was carried out at room temperature for 6 hours, and then polymer 1G having the ionizable moiety of the present invention was obtained.

### [Manufacturing Example 58]

### Preparation of polymer 1H

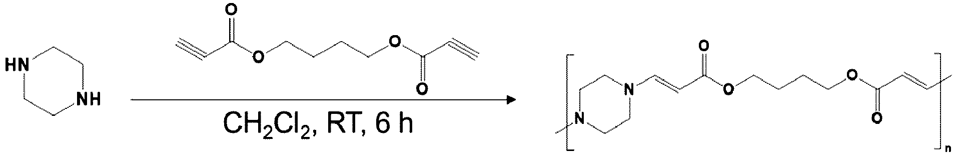

Alkynoate monomer H (500 mg) was placed in a round bottom flask, and then amine monomer 1 was added to the flask so that the molar ratio with the alkynoate monomer was 1.04, and 4 mL of dichloromethane was added to the flask. Thereafter, the reaction was carried out at room temperature for 6 hours, and then polymer 1H having the ionizable moiety of the present invention was obtained.

### [Manufacturing Example 59]

### Preparation of polymer 1I

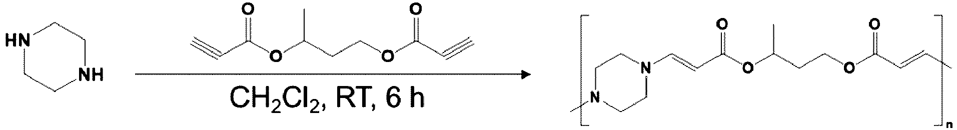

Alkynoate monomer I (500 mg) was placed in a round bottom flask, and then amine monomer 1 was added to the flask so that the molar ratio with the alkynoate monomer was 1.04, and 4 mL of dichloromethane was added to the flask. Thereafter, the reaction was carried out at room temperature for 6 hours, and then polymer 1I having the ionizable moiety of the present invention was obtained.

### [Manufacturing Example 60]

### Preparation of polymer 1J

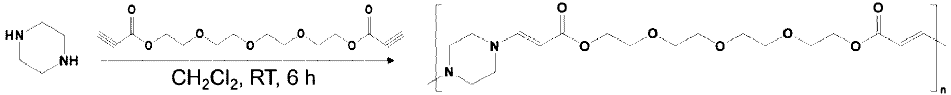

Alkynoate monomer J (500 mg) was placed in a round bottom flask, and then amine monomer 1 was added to the flask so that the molar ratio with the alkynoate monomer was 1.04, and 4 mL of dichloromethane was added to the flask. Thereafter, the reaction was carried out at room temperature for 6 hours, and then polymer 1J having the ionizable moiety of the present invention was obtained.

### [Manufacturing Example 61]

### Preparation of polymer 2F

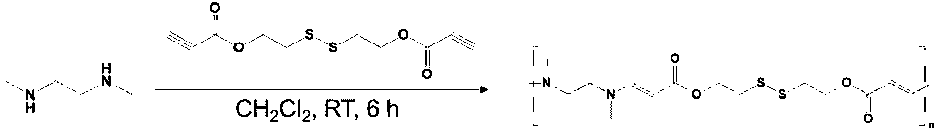

Alkynoate monomer F (500 mg) was placed in a round bottom flask, and then amine monomer 2 was added to the flask so that the molar ratio with the alkynoate monomer was 1.04, and 4 mL of dichloromethane was added to the flask. Thereafter, the reaction was carried out at room temperature for 6 hours, and then polymer 2F having the ionizable moiety of the present invention was obtained.

### [Manufacturing Example 62]

### Preparation of polymer 2G

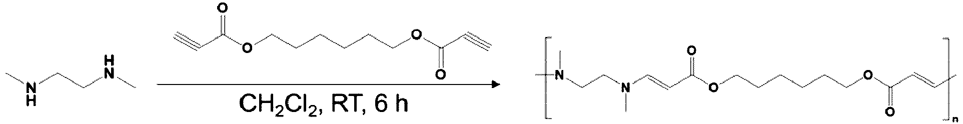

Alkynoate monomer G (500 mg) was placed in a round bottom flask, and then amine monomer 2 was added to the flask so that the molar ratio with the alkynoate monomer was 1.04, and 4 mL of dichloromethane was added to the flask. Thereafter, the reaction was carried out at room temperature for 6 hours, and then polymer 2G having the ionizable moiety of the present invention was obtained.

### [Manufacturing Example 63]

### Preparation of polymer 2H

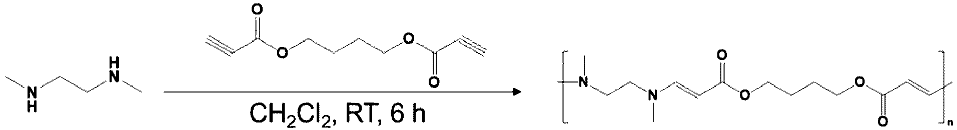

Alkynoate monomer H (500 mg) was placed in a round bottom flask, and then amine monomer 2 was added to the flask so that the molar ratio with the alkynoate monomer was 1.04, and 4 mL of dichloromethane was added to the flask. Thereafter, the reaction was carried out at room temperature for 6 hours, and then polymer 2H having the ionizable moiety of the present invention was obtained.

### [Manufacturing Example 64]

### Preparation of polymer 2I

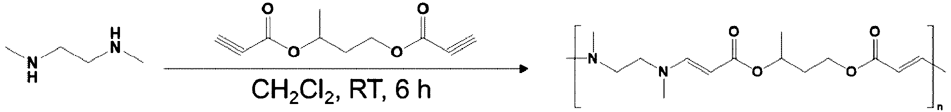

Alkynoate monomer I (500 mg) was placed in a round bottom flask, and then amine monomer 2 was added to the flask so that the molar ratio with the alkynoate monomer was 1.04, and 4 mL of dichloromethane was added to the flask. Thereafter, the reaction was carried out at room temperature for 6 hours, and then polymer 2I having the ionizable moiety of the present invention was obtained.

### [Manufacturing Example 65]

### Preparation of polymer 2J

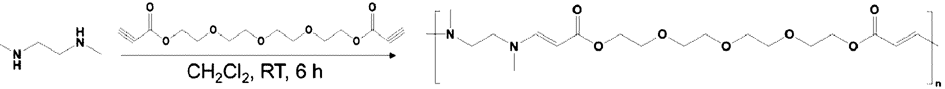

Alkynoate monomer J (500 mg) was placed in a round bottom flask, and then amine monomer 2 was added to the flask so that the molar ratio with the alkynoate monomer was 1.04, and 4 mL of dichloromethane was added to the flask. Thereafter, the reaction was carried out at room temperature for 6 hours, and then polymer 2J having the ionizable moiety of the present invention was obtained.

### [Manufacturing Example 66]

### Preparation of polymer 3F

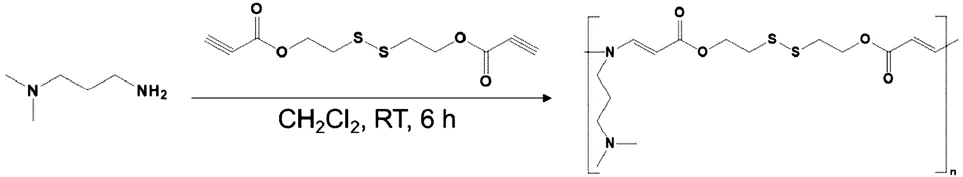

Alkynoate monomer F (500 mg) was placed in a round bottom flask, and then amine monomer 3 was added to the flask so that the molar ratio with the alkynoate monomer was 1.04, and 4 mL of dichloromethane was added to the flask. Thereafter, the reaction was carried out at room temperature for 6 hours, and then polymer 3F having the ionizable moiety of the present invention was obtained.

### [Manufacturing Example 67]

### Preparation of polymer 3G

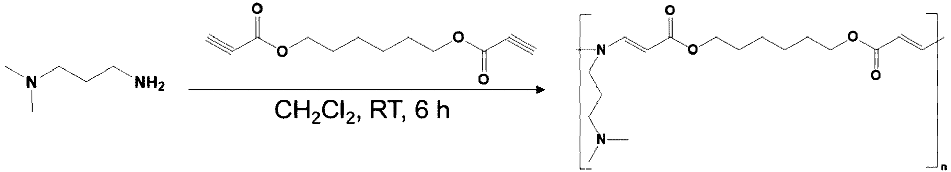

Alkynoate monomer G (500 mg) was placed in a round bottom flask, and then amine monomer 3 was added to the flask so that the molar ratio with the alkynoate monomer was 1.04, and 4 mL of dichloromethane was added to the flask. Thereafter, the reaction was carried out at room temperature for 6 hours, and then polymer 3G having the ionizable moiety of the present invention was obtained.

### [Manufacturing Example 68]

### Preparation of polymer 3H

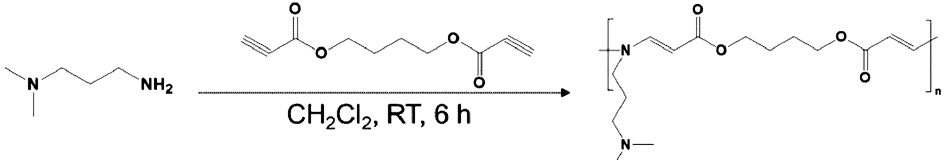

Alkynoate monomer H (500 mg) was placed in a round bottom flask, and then amine monomer 3 was added to the flask so that the molar ratio with the alkynoate monomer was 1.04, and 4 mL of dichloromethane was added to the flask. Thereafter, the reaction was carried out at room temperature for 6 hours, and then polymer 3H having the ionizable moiety of the present invention was obtained.

### [Manufacturing Example 69]

### Preparation of polymer 3I

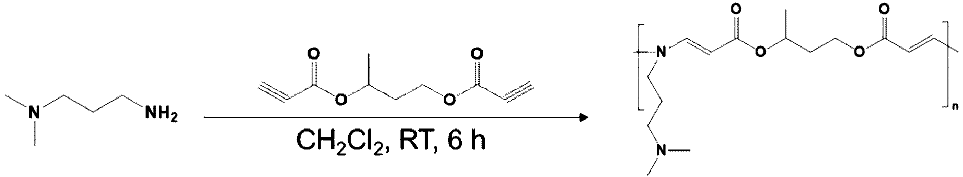

Alkynoate monomer I (500 mg) was placed in a round bottom flask, and then amine monomer 3 was added to the flask so that the molar ratio with the alkynoate monomer was 1.04, and 4 mL of dichloromethane was added to the flask. Thereafter, the reaction was carried out at room temperature for 6 hours, and then polymer 3I having the ionizable moiety of the present invention was obtained.

### [Manufacturing Example 70]

### Preparation of polymer 3J

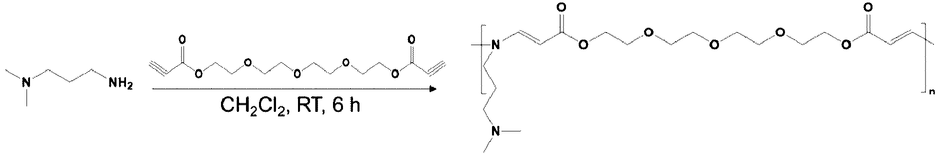

Alkynoate monomer J (500 mg) was placed in a round bottom flask, and then amine monomer 3 was added to the flask so that the molar ratio with the alkynoate monomer was 1.04, and 4 mL of dichloromethane was added to the flask. Thereafter, the reaction was carried out at room temperature for 6 hours, and then polymer 3J having the ionizable moiety of the present invention was obtained.

### [Manufacturing Example 71]

### Preparation of polymer 4F

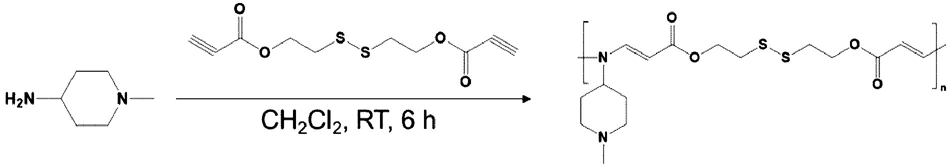

Alkynoate monomer F (500 mg) was placed in a round bottom flask, and then amine monomer 4 was added to the flask so that the molar ratio with the alkynoate monomer was 1.04, and 4 mL of dichloromethane was added to the flask. Thereafter, the reaction was carried out at room temperature for 6 hours, and then polymer 4F having the ionizable moiety of the present invention was obtained.

### [Manufacturing Example 72]

### Preparation of polymer 4G

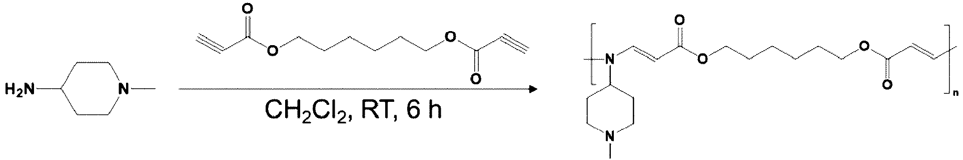

Alkynoate monomer G (500 mg) was placed in a round bottom flask, and then amine monomer 4 was added to the flask so that the molar ratio with the alkynoate monomer was 1.04, and 4 mL of dichloromethane was added to the flask. Thereafter, the reaction was carried out at room temperature for 6 hours, and then polymer 4G having the ionizable moiety of the present invention was obtained.

### [Manufacturing Example 73]

### Preparation of polymer 4H

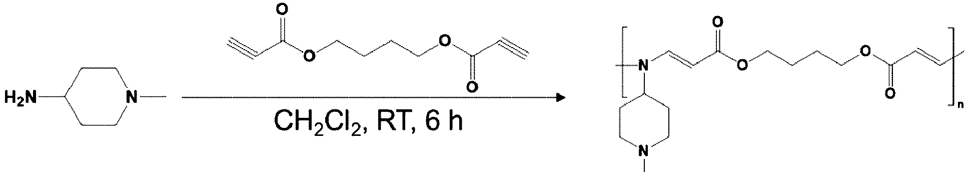

Alkynoate monomer H (500 mg) was placed in a round bottom flask, and then amine monomer 4 was added to the flask so that the molar ratio with the alkynoate monomer was 1.04, and 4 mL of dichloromethane was added to the flask. Thereafter, the reaction was carried out at room temperature for 6 hours, and then polymer 4H having the ionizable moiety of the present invention was obtained.

### [Manufacturing Example 74]

### Preparation of polymer 4I

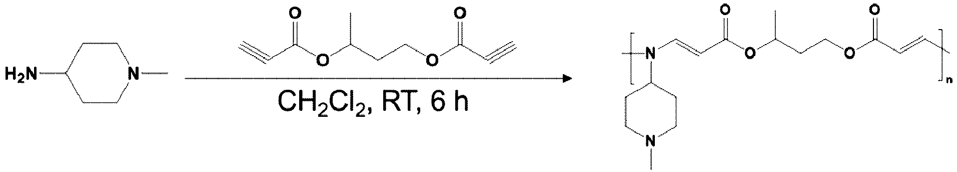

Alkynoate monomer I (500 mg) was placed in a round bottom flask, and then amine monomer 4 was added to the flask so that the molar ratio with the alkynoate monomer was 1.04, and 4 mL of dichloromethane was added to the flask. Thereafter, the reaction was carried out at room temperature for 6 hours, and then polymer 4I having the ionizable moiety of the present invention was obtained.

### [Manufacturing Example 75]

### Preparation of polymer 4J

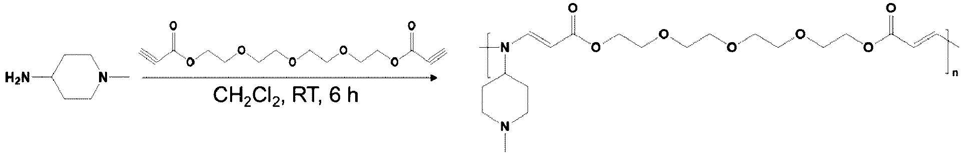

Alkynoate monomer J (500 mg) was placed in a round bottom flask, and then amine monomer 4 was added to the flask so that the molar ratio with the alkynoate monomer was 1.04, and 4 mL of dichloromethane was added to the flask. Thereafter, the reaction was carried out at room temperature for 6 hours, and then polymer 4J having the ionizable moiety of the present invention was obtained.

### [Manufacturing Example 76]

### Preparation of polymer 5F

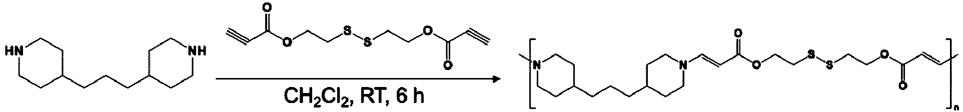

Alkynoate monomer F (500 mg) was placed in a round bottom flask, and then amine monomer 5 was added to the flask so that the molar ratio with the alkynoate monomer was 1.04, and 4 mL of dichloromethane was added to the flask. Thereafter, the reaction was carried out at room temperature for 6 hours, and then polymer 5F having the ionizable moiety of the present invention was obtained.

### [Manufacturing Example 77]

### Preparation of polymer 5G

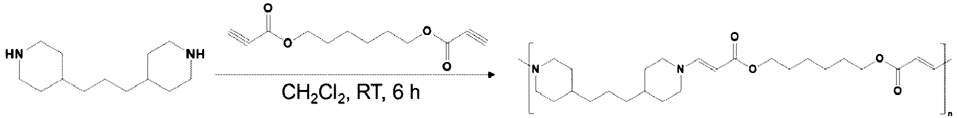

Alkynoate monomer G (500 mg) was placed in a round bottom flask, and then amine monomer 5 was added to the flask so that the molar ratio with the alkynoate monomer was 1.04, and 4 mL of dichloromethane was added to the flask. Thereafter, the reaction was carried out at room temperature for 6 hours, and then polymer 5G having the ionizable moiety of the present invention was obtained.

### [Manufacturing Example 78]

### Preparation of polymer 5H

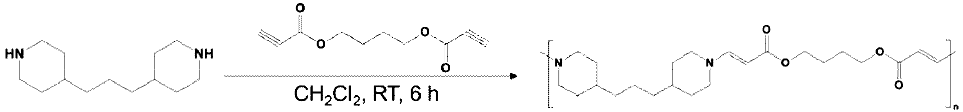

Alkynoate monomer H (500 mg) was placed in a round bottom flask, and then amine monomer 5 was added to the flask so that the molar ratio with the alkynoate monomer was 1.04, and 4 mL of dichloromethane was added to the flask. Thereafter, the reaction was carried out at room temperature for 6 hours, and then polymer 5H having the ionizable moiety of the present invention was obtained.

### [Manufacturing Example 79]

### Preparation of polymer 5I

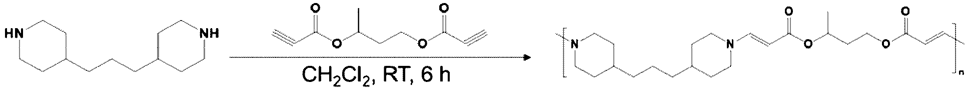

Alkynoate monomer I (500 mg) was placed in a round bottom flask, and then amine monomer 5 was added to the flask so that the molar ratio with the alkynoate monomer was 1.04, and 4 mL of dichloromethane was added to the flask. Thereafter, the reaction was carried out at room temperature for 6 hours, and then polymer 5I having the ionizable moiety of the present invention was obtained.

### [Manufacturing Example 80]

### Preparation of polymer 5J

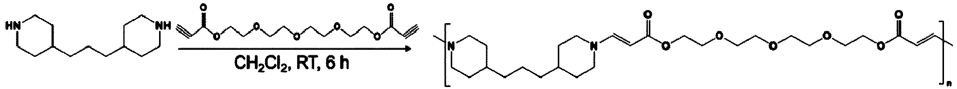

Alkynoate monomer J (500 mg) was placed in a round bottom flask, and then amine monomer 5 was added to the flask so that the molar ratio with the alkynoate monomer was 1.04, and 4 mL of dichloromethane was added to the flask. Thereafter, the reaction was carried out at room temperature for 6 hours, and then polymer 5J having the ionizable moiety of the present invention was obtained.

### [Manufacturing Example 81]

### Preparation of polymer 6F

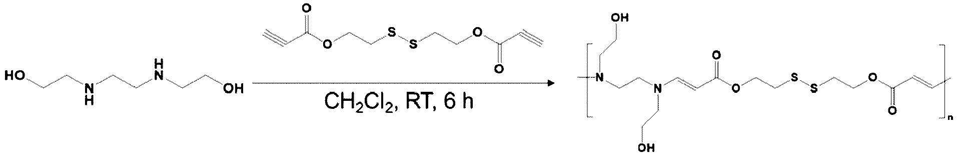

Alkynoate monomer F (500 mg) was placed in a round bottom flask, and then amine monomer 6 was added to the flask so that the molar ratio with the alkynoate monomer was 1.04, and 4 mL of dichloromethane was added to the flask. Thereafter, the reaction was carried out at room temperature for 6 hours, and then polymer 6F having the ionizable moiety of the present invention was obtained.

### [Manufacturing Example 82]

### Preparation of polymer 6G

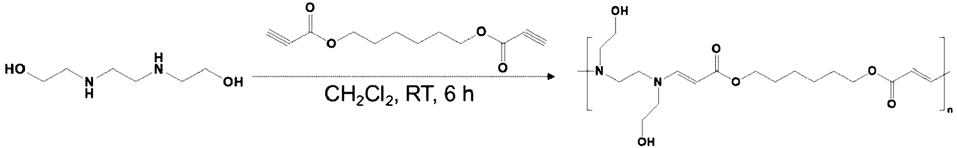

Alkynoate monomer G (500 mg) was placed in a round bottom flask, and then amine monomer 6 was added to the flask so that the molar ratio with the alkynoate monomer was 1.04, and 4 mL of dichloromethane was added to the flask. Thereafter, the reaction was carried out at room temperature for 6 hours, and then polymer 6G having the ionizable moiety of the present invention was obtained.

### [Manufacturing Example 83]

### Preparation of polymer 6H

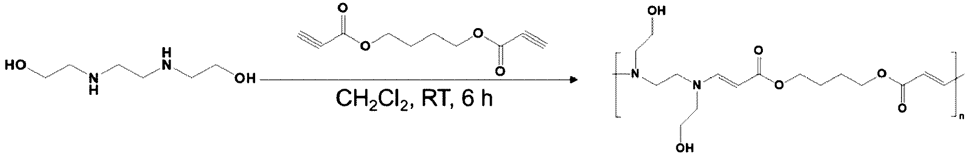

Alkynoate monomer H (500 mg) was placed in a round bottom flask, and then amine monomer 6 was added to the flask so that the molar ratio with the alkynoate monomer was 1.04, and 4 mL of dichloromethane was added to the flask. Thereafter, the reaction was carried out at room temperature for 6 hours, and then polymer 6H having the ionizable moiety of the present invention was obtained.

### [Manufacturing Example 84]

### Preparation of polymer 6I

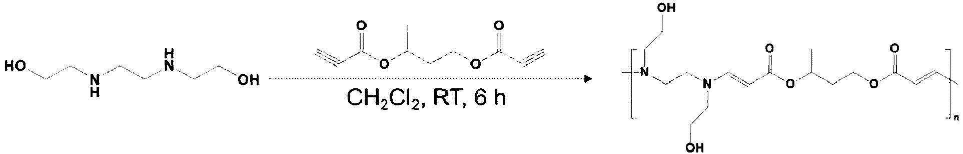

Alkynoate monomer I (500 mg) was placed in a round bottom flask, and then amine monomer 6 was added to the flask so that the molar ratio with the alkynoate monomer was 1.04, and 4 mL of dichloromethane was added to the flask. Thereafter, the reaction was carried out at room temperature for 6 hours, and then polymer 6I having the ionizable moiety of the present invention was obtained.

### [Manufacturing Example 85]

### Preparation of polymer 6J

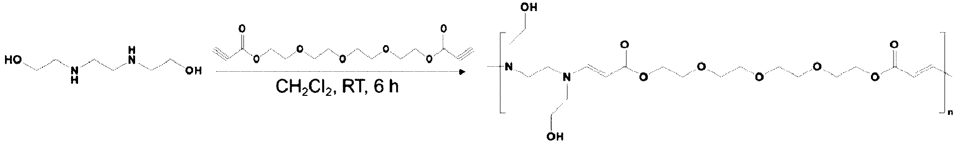

Alkynoate monomer J (500 mg) was placed in a round bottom flask, and then amine monomer 6 was added to the flask so that the molar ratio with the alkynoate monomer was 1.04, and 4 mL of dichloromethane was added to the flask. Thereafter, the reaction was carried out at room temperature for 6 hours, and then polymer 6J having the ionizable moiety of the present invention was obtained.

### [Manufacturing Example 86]

### Preparation of polymer 7F

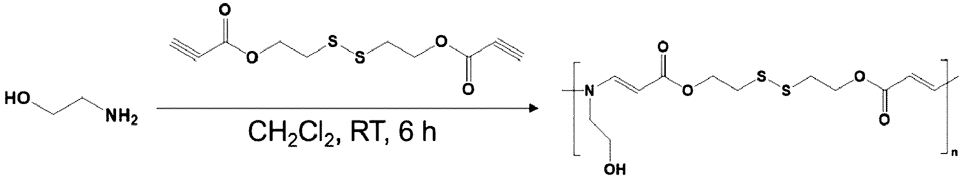

Alkynoate monomer F (500 mg) was placed in a round bottom flask, and then amine monomer 7 was added to the flask so that the molar ratio with the alkynoate monomer was 1.04, and 4 mL of dichloromethane was added to the flask. Thereafter, the reaction was carried out at room temperature for 6 hours, and then polymer 7F having the ionizable moiety of the present invention was obtained.

### [Manufacturing Example 87]

### Preparation of polymer 7G

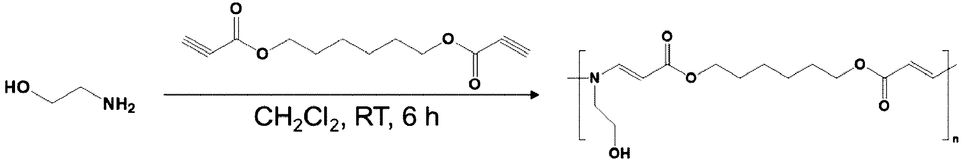

Alkynoate monomer G (500 mg) was placed in a round bottom flask, and then amine monomer 7 was added to the flask so that the molar ratio with the alkynoate monomer was 1.04, and 4 mL of dichloromethane was added to the flask. Thereafter, the reaction was carried out at room temperature for 6 hours, and then polymer 7G having the ionizable moiety of the present invention was obtained.

### [Manufacturing Example 88]

### Preparation of polymer 7H

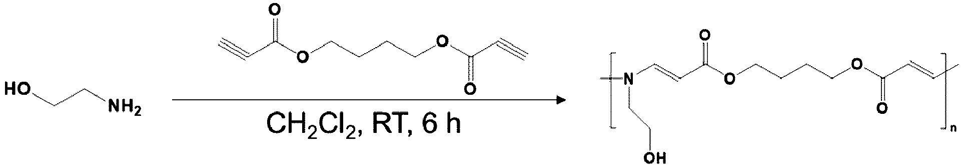

Alkynoate monomer H (500 mg) was placed in a round bottom flask, and then amine monomer 7 was added to the flask so that the molar ratio with the alkynoate monomer was 1.04, and 4 mL of dichloromethane was added to the flask. Thereafter, the reaction was carried out at room temperature for 6 hours, and then polymer 7H having the ionizable moiety of the present invention was obtained.

### [Manufacturing Example 89]

### Preparation of polymer 71

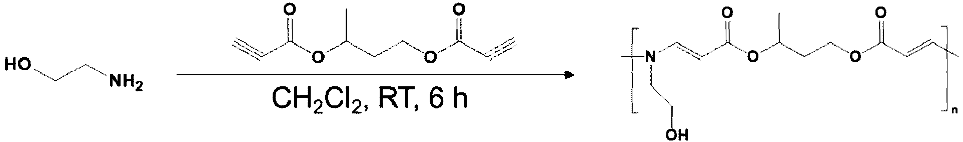

Alkynoate monomer I (500 mg) was placed in a round bottom flask, and then amine monomer 7 was added to the flask so that the molar ratio with the alkynoate monomer was 1.04, and 4 mL of dichloromethane was added to the flask. Thereafter, the reaction was carried out at room temperature for 6 hours, and then polymer 7I having the ionizable moiety of the present invention was obtained.

### [Manufacturing Example 90]

### Preparation of polymer 7J

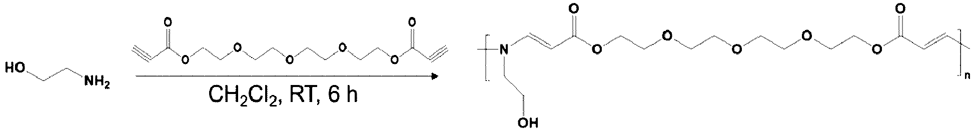

Alkynoate monomer J (500 mg) was placed in a round bottom flask, and then amine monomer 7 was added to the flask so that the molar ratio with the alkynoate monomer was 1.04, and 4 mL of dichloromethane was added to the flask. Thereafter, the reaction was carried out at room temperature for 6 hours, and then polymer 7J having the ionizable moiety of the present invention was obtained.

### [Manufacturing Example 91]

### Preparation of polymer 8F

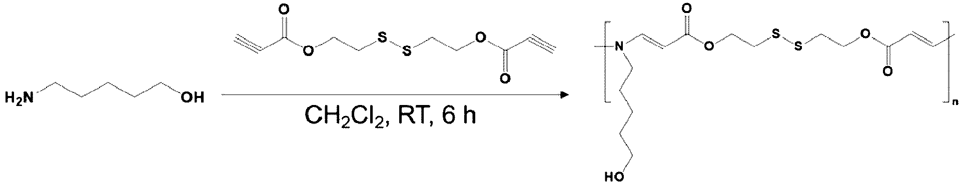

Alkynoate monomer F (500 mg) was placed in a round bottom flask, and then amine monomer 8 was added to the flask so that the molar ratio with the alkynoate monomer was 1.04, and 4 mL of dichloromethane was added to the flask. Thereafter, the reaction was carried out at room temperature for 6 hours, and then polymer 8F having the ionizable moiety of the present invention was obtained.

### [Manufacturing Example 92]

### Preparation of polymer 8G

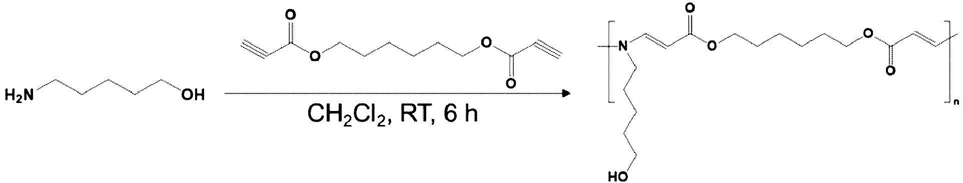

Alkynoate monomer G (500 mg) was placed in a round bottom flask, and then amine monomer 8 was added to the flask so that the molar ratio with the alkynoate monomer was 1.04, and 4 mL of dichloromethane was added to the flask. Thereafter, the reaction was carried out at room temperature for 6 hours, and then polymer 8G having the ionizable moiety of the present invention was obtained.

### [Manufacturing Example 93]

### Preparation of polymer 8H

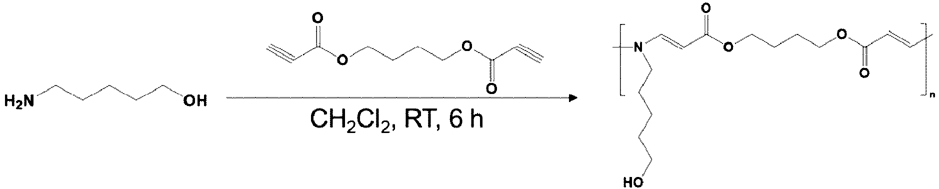

Alkynoate monomer H (500 mg) was placed in a round bottom flask, and then amine monomer 8 was added to the flask so that the molar ratio with the alkynoate monomer was 1.04, and 4 mL of dichloromethane was added to the flask. Thereafter, the reaction was carried out at room temperature for 6 hours, and then polymer 8H having the ionizable moiety of the present invention was obtained.

### [Manufacturing Example 94]

### Preparation of polymer 8I

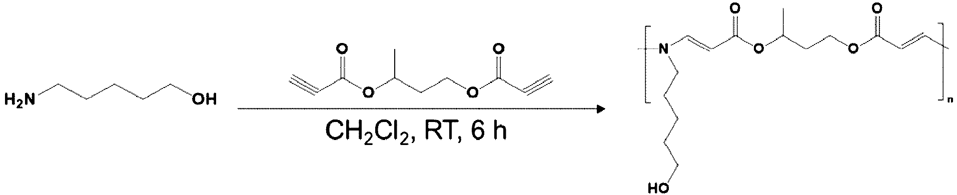

Alkynoate monomer I (500 mg) was placed in a round bottom flask, and then amine monomer 8 was added to the flask so that the molar ratio with the alkynoate monomer was 1.04, and 4 mL of dichloromethane was added to the flask. Thereafter, the reaction was carried out at room temperature for 6 hours, and then polymer 8I having the ionizable moiety of the present invention was obtained.

### [Manufacturing Example 95]

### Preparation of polymer 8J

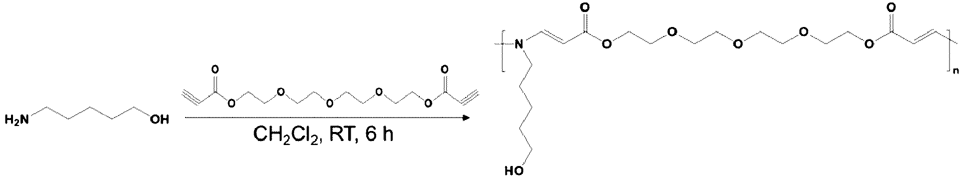

Alkynoate monomer J (500 mg) was placed in a round bottom flask, and then amine monomer 8 was added to the flask so that the molar ratio with the alkynoate monomer was 1.04, and 4 mL of dichloromethane was added to the flask. Thereafter, the reaction was carried out at room temperature for 6 hours, and then polymer 8J having the ionizable moiety of the present invention was obtained.

### [Manufacturing Example 96]

### Preparation of polymer 9F

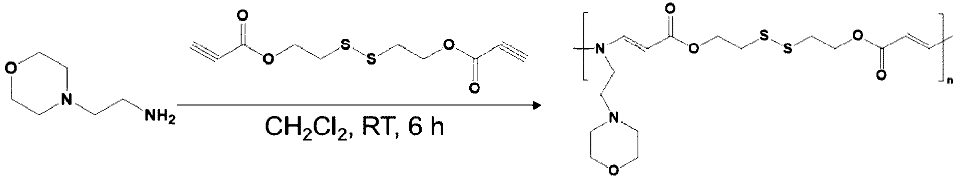

Alkynoate monomer F (500 mg) was placed in a round bottom flask, and then amine monomer 9 was added to the flask so that the molar ratio with the alkynoate monomer was 1.04, and 4 mL of dichloromethane was added to the flask. Thereafter, the reaction was carried out at room temperature for 6 hours, and then polymer 9F having the ionizable moiety of the present invention was obtained.

### [Manufacturing Example 97]

### Preparation of polymer 9G

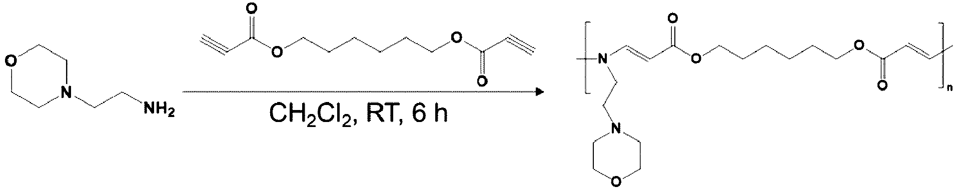

Alkynoate monomer G (500 mg) was placed in a round bottom flask, and then amine monomer 9 was added to the flask so that the molar ratio with the alkynoate monomer was 1.04, and 4 mL of dichloromethane was added to the flask. Thereafter, the reaction was carried out at room temperature for 6 hours, and then polymer 9G having the ionizable moiety of the present invention was obtained.

### [Manufacturing Example 98]

### Preparation of polymer 9H

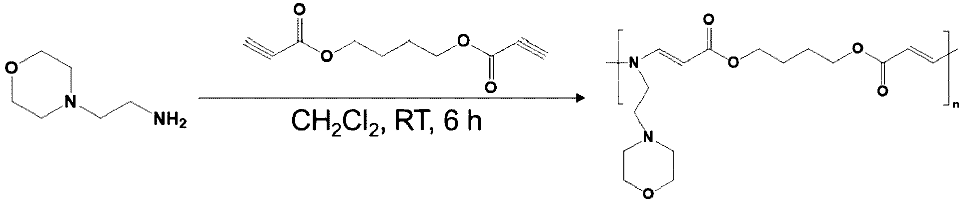

Alkynoate monomer H (500 mg) was placed in a round bottom flask, and then amine monomer 9 was added to the flask so that the molar ratio with the alkynoate monomer was 1.04, and 4 mL of dichloromethane was added to the flask. Thereafter, the reaction was carried out at room temperature for 6 hours, and then polymer 9H having the ionizable moiety of the present invention was obtained.

### [Manufacturing Example 99]

### Preparation of polymer 9I

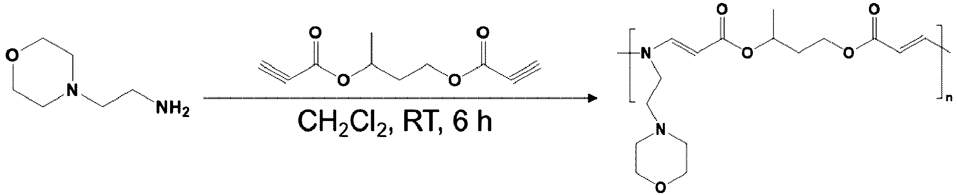

Alkynoate monomer I (500 mg) was placed in a round bottom flask, and then amine monomer 9 was added to the flask so that the molar ratio with the alkynoate monomer was 1.04, and 4 mL of dichloromethane was added to the flask. Thereafter, the reaction was carried out at room temperature for 6 hours, and then polymer 9I having the ionizable moiety of the present invention was obtained.

### [Manufacturing Example 100]

### Preparation of polymer 9J

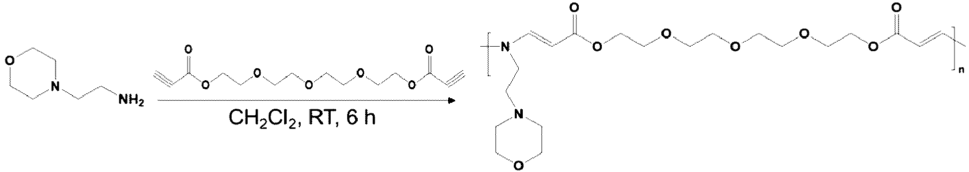

Alkynoate monomer J (500 mg) was placed in a round bottom flask, and then amine monomer 9 was added to the flask so that the molar ratio with the alkynoate monomer was 1.04, and 4 mL of dichloromethane was added to the flask. Thereafter, the reaction was carried out at room temperature for 6 hours, and then polymer 9J having the ionizable moiety of the present invention was obtained.

### [Manufacturing Example 101]

### Preparation of polymer 10F

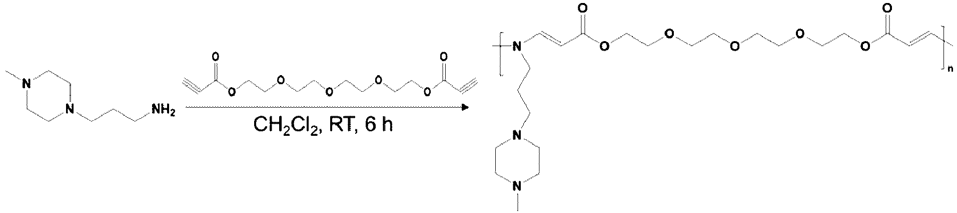

Alkynoate monomer F (500 mg) was placed in a round bottom flask, and then amine monomer 10 was added to the flask so that the molar ratio with the alkynoate monomer was 1.04, and 4 mL of dichloromethane was added to the flask. Thereafter, the reaction was carried out at room temperature for 6 hours, and then polymer 10F having the ionizable moiety of the present invention was obtained.

### [Manufacturing Example 102]

### Preparation of polymer 10G

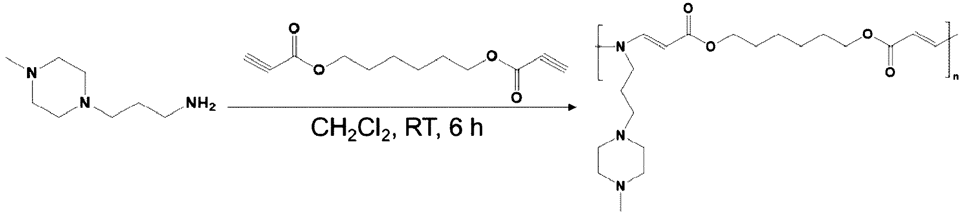

Alkynoate monomer G (500 mg) was placed in a round bottom flask, and then amine monomer 10 was added to the flask so that the molar ratio with the alkynoate monomer was 1.04, and 4 mL of dichloromethane was added to the flask. Thereafter, the reaction was carried out at room temperature for 6 hours, and then polymer 10G having the ionizable moiety of the present invention was obtained.

### [Manufacturing Example 103]

### Preparation of polymer 10H

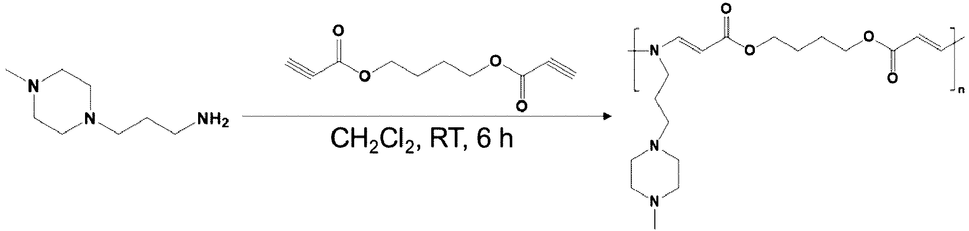

Alkynoate monomer H (500 mg) was placed in a round bottom flask, and then amine monomer 10 was added to the flask so that the molar ratio with the alkynoate monomer was 1.04, and 4 mL of dichloromethane was added to the flask. Thereafter, the reaction was carried out at room temperature for 6 hours, and then polymer 10H having the ionizable moiety of the present invention was obtained.

### [Manufacturing Example 104]

### Preparation of polymer 10I

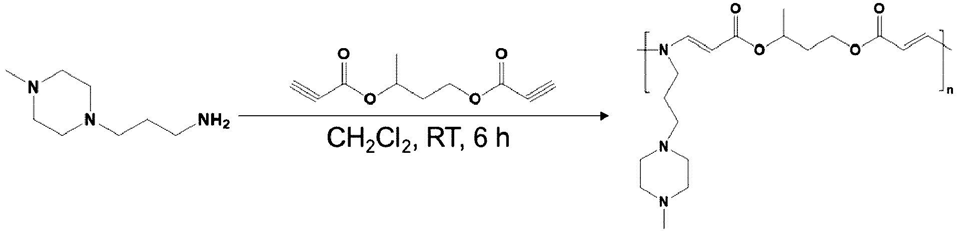

Alkynoate monomer I (500 mg) was placed in a round bottom flask, and then amine monomer 10 was added to the flask so that the molar ratio with the alkynoate monomer was 1.04, and 4 mL of dichloromethane was added to the flask. Thereafter, the reaction was carried out at room temperature for 6 hours, and then polymer 10I having the ionizable moiety of the present invention was obtained.

### [Manufacturing Example 105]

### Preparation of polymer 10J

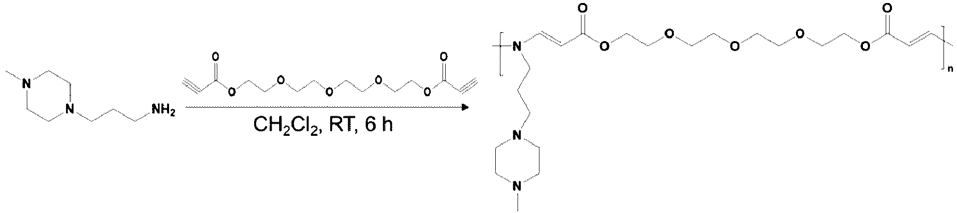

Alkynoate monomer J (500 mg) was placed in a round bottom flask, and then amine monomer 10 was added to the flask so that the molar ratio with the alkynoate monomer was 1.04, and 4 mL of dichloromethane was added to the flask. Thereafter, the reaction was carried out at room temperature for 6 hours, and then polymer 10J having the ionizable moiety of the present invention was obtained.

### [Manufacturing Example 106]

### Preparation of polymer 11F

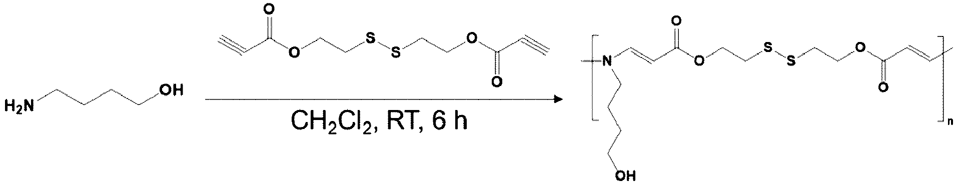

Alkynoate monomer F (500 mg) was placed in a round bottom flask, and then amine monomer 11 was added to the flask so that the molar ratio with the alkynoate monomer was 1.04, and 4 mL of dichloromethane was added to the flask. Thereafter, the reaction was carried out at room temperature for 6 hours, and then polymer 11F having the ionizable moiety of the present invention was obtained.

### [Manufacturing Example 107]

### Preparation of polymer 11G

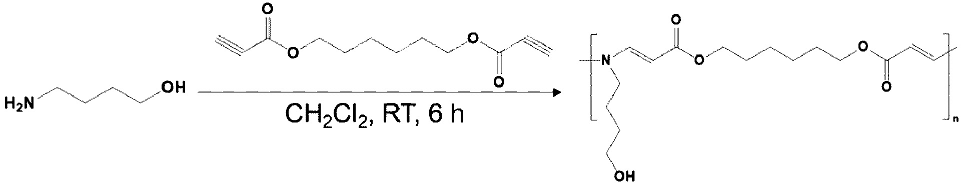

Alkynoate monomer G (500 mg) was placed in a round bottom flask, and then amine monomer 11 was added to the flask so that the molar ratio with the alkynoate monomer was 1.04, and 4 mL of dichloromethane was added to the flask. Thereafter, the reaction was carried out at room temperature for 6 hours, and then polymer 11G having the ionizable moiety of the present invention was obtained.

### [Manufacturing Example 108]

### Preparation of polymer 11H

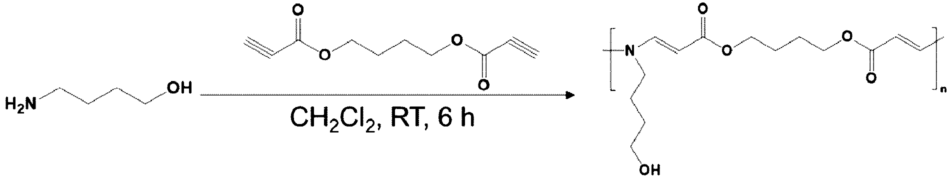

Alkynoate monomer H (500 mg) was placed in a round bottom flask, and then amine monomer 11 was added to the flask so that the molar ratio with the alkynoate monomer was 1.04, and 4 mL of dichloromethane was added to the flask. Thereafter, the reaction was carried out at room temperature for 6 hours, and then polymer 11H having the ionizable moiety of the present invention was obtained.

### [Manufacturing Example 109]

### Preparation of polymer 11I

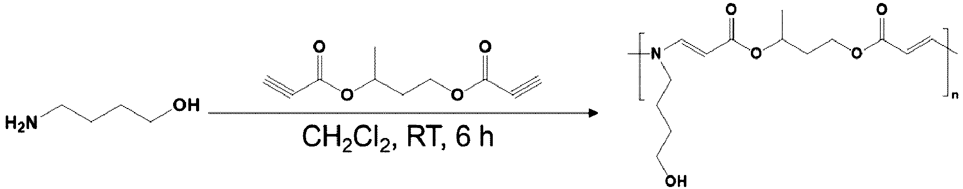

Alkynoate monomer I (500 mg) was placed in a round bottom flask, and then amine monomer 11 was added to the flask so that the molar ratio with the alkynoate monomer was 1.04, and 4 mL of dichloromethane was added to the flask. Thereafter, the reaction was carried out at room temperature for 6 hours, and then polymer 11I having the ionizable moiety of the present invention was obtained.

### [Manufacturing Example 110]

### Preparation of polymer 11J

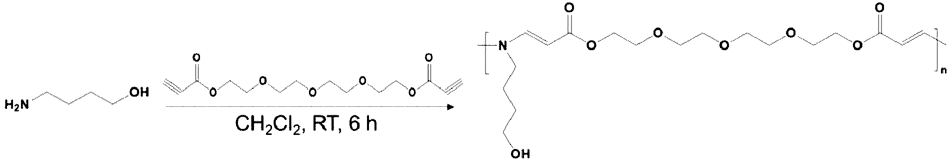

Alkynoate monomer J (500 mg) was placed in a round bottom flask, and then amine monomer 11 was added to the flask so that the molar ratio with the alkynoate monomer was 1.04, and 4 mL of dichloromethane was added to the flask. Thereafter, the reaction was carried out at room temperature for 6 hours, and then polymer 11J having the ionizable moiety of the present invention was obtained.

### [Manufacturing Example 111]

### Preparation of polymer PEG-1A

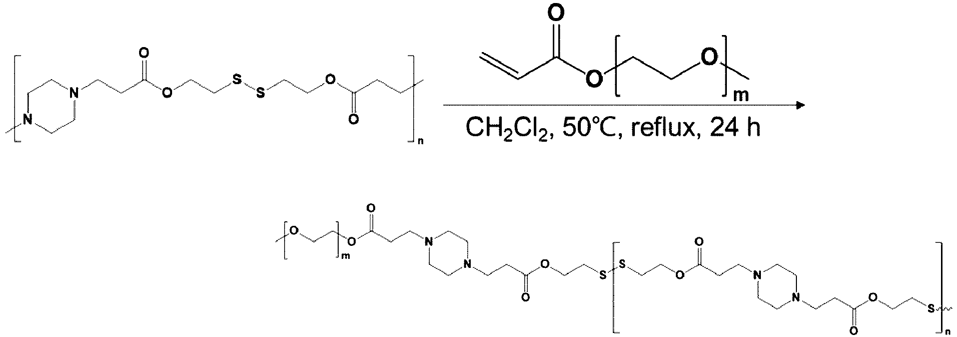

Polymer 1A prepared in the same manner as in Manufacturing Example 1 above and 648.8 mg of PEG-acrylate were dissolved in CH₂Cl₂ to a concentration of 1000 mg/ml and added to a flask. The reaction was carried out at 50 °C for 24 hours with reflux, and then PEG-1A was obtained.

### [Manufacturing Example 112]

### Preparation of polymer PEG-1B

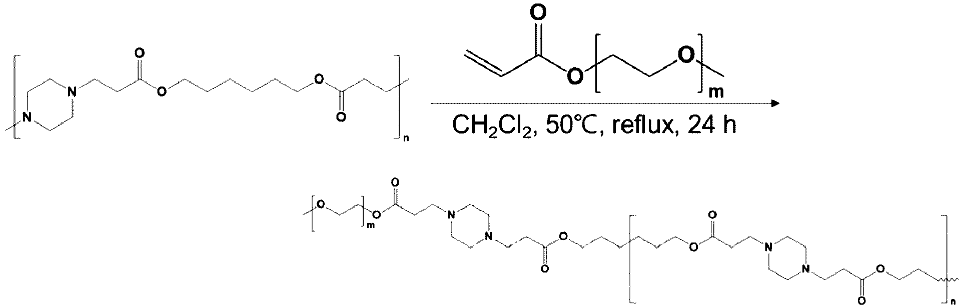

Polymer 1B prepared in the same manner as in Manufacturing Example 2 above and 752.3 mg of PEG-acrylate were dissolved in CH₂Cl₂ to a concentration of 1000 mg/ml and added to a flask. The reaction was carried out at 50 °C for 24 hours with reflux, and then PEG-1B was obtained.

### [Manufacturing Example 113]

### Preparation of polymer PEG-1C

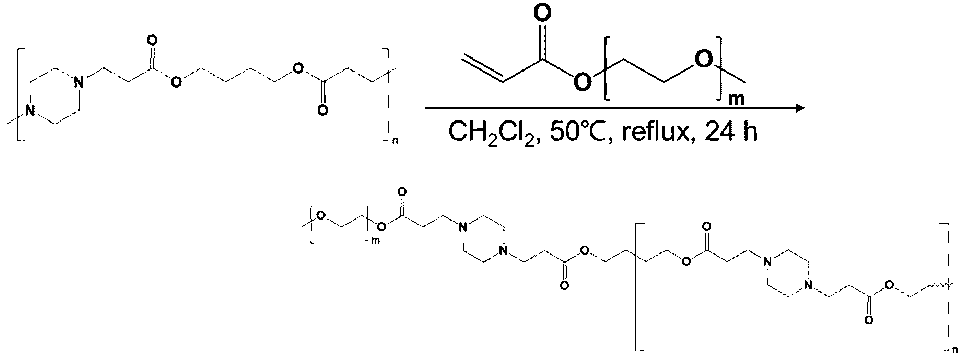

Polymer 1C prepared in the same manner as in Manufacturing Example 3 above and 858.7 mg of PEG-acrylate were dissolved in CH₂Cl₂ to a concentration of 1000 mg/ml and added to a flask. The reaction was carried out at 50 °C for 24 hours with reflux, and then PEG-1C was obtained.

### [Manufacturing Example 114]

### Preparation of polymer PEG-1D

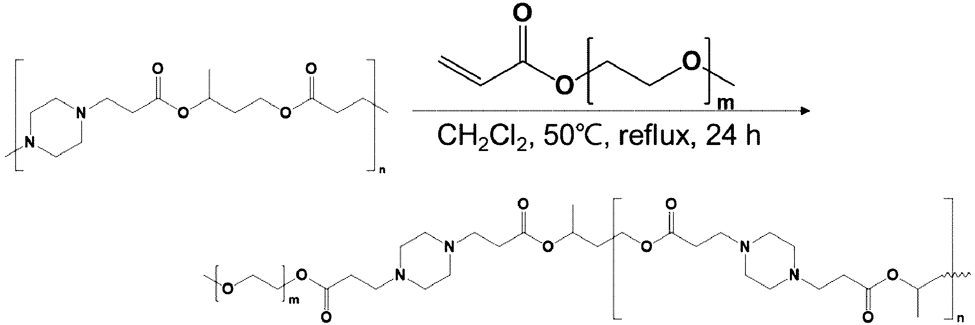

Polymer 1D prepared in the same manner as in Manufacturing Example 4 above and 858.7 mg of PEG-acrylate were dissolved in CH₂Cl₂ to a concentration of 1000 mg/ml and added to a flask. The reaction was carried out at 50 °C for 24 hours with reflux, and then PEG-1D was obtained.

### [Manufacturing Example 115]

### Preparation of polymer PEG-1E

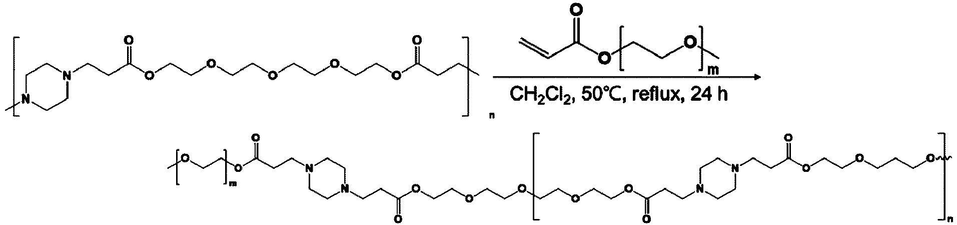

Polymer 1E prepared in the same manner as in Manufacturing Example 5 above and 563.0 mg of PEG-acrylate were dissolved in CH₂Cl₂ to a concentration of 2500 mg/ml and added to a flask. The reaction was carried out at 50 °C for 24 hours with reflux, and then PEG-1E was obtained.

### [Manufacturing Example 116]

### Preparation of polymer PEG-2A

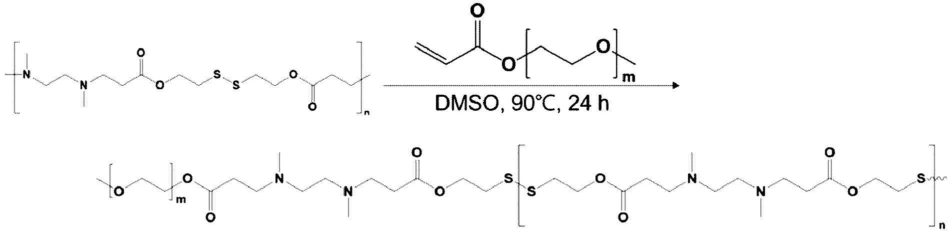

Polymer 2A prepared in the same manner as in Manufacturing Example 6 above and 648.8 mg of PEG-acrylate were dissolved in DMSO to a concentration of 1000 mg/ml and added to a flask. The reaction was carried out at 90 °C for 24 hours, and then PEG-2A was obtained.

### [Manufacturing Example 117]

### Preparation of polymer PEG-2B

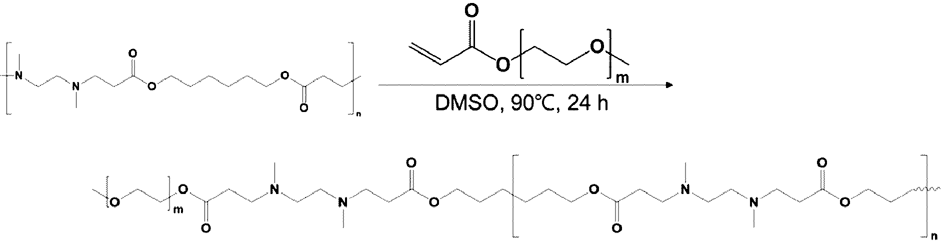

Polymer 2B prepared in the same manner as in Manufacturing Example 7 above and 752.3 mg of PEG-acrylate were dissolved in DMSO to a concentration of 1000 mg/ml and added to a flask. The reaction was carried out at 90 °C for 24 hours, and then PEG-2B was obtained.

### [Manufacturing Example 118]

### Preparation of polymer PEG-2C

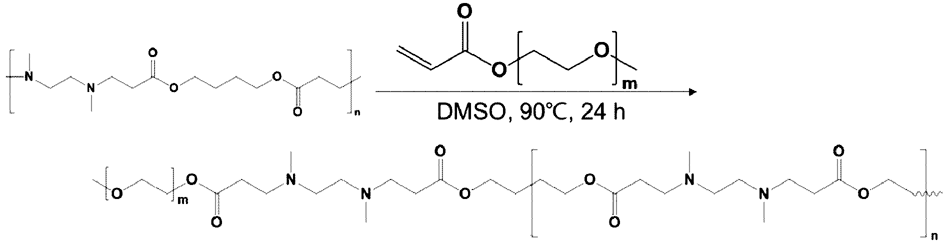

Polymer 2C prepared in the same manner as in Manufacturing Example 8 above and 858.7 mg of PEG-acrylate were dissolved in DMSO to a concentration of 1000 mg/ml and added to a flask. The reaction was carried out at 90 °C for 24 hours, and then PEG-2C was obtained.

### [Manufacturing Example 119]

### Preparation of polymer PEG-2D

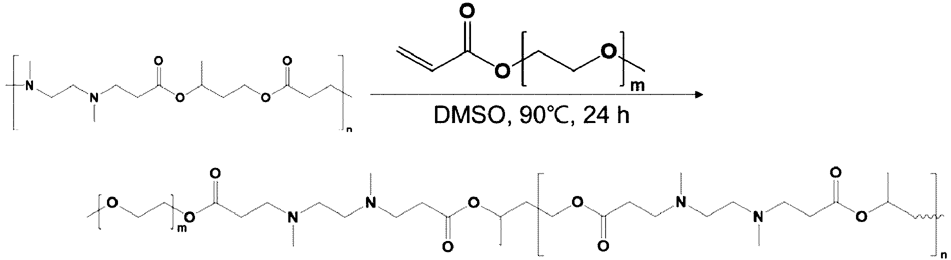

Polymer 2D prepared in the same manner as in Manufacturing Example 9 above and 858.7 mg of PEG-acrylate were dissolved in DMSO to a concentration of 1000 mg/ml and added to a flask. The reaction was carried out at 90 °C for 24 hours, and then PEG-2D was obtained.

### [Manufacturing Example 120]

### Preparation of polymer PEG-2E

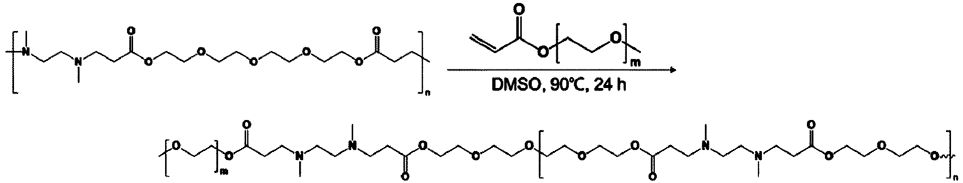

Polymer 2E prepared in the same manner as in Manufacturing Example 10 above and 563.0 mg of PEG-acrylate were dissolved in DMSO to a concentration of 2500 mg/ml and added to a flask. The reaction was carried out at 90 °C for 24 hours, and then PEG-2E was obtained.

### [Manufacturing Example 121]

### Preparation of polymer PEG-3A

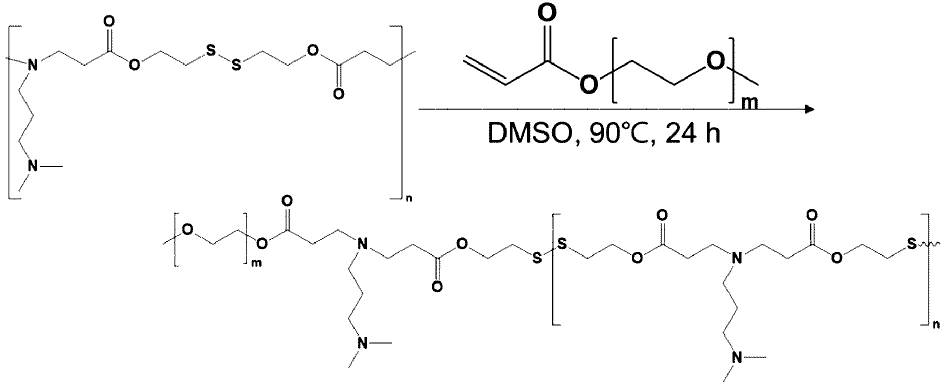

Polymer 3A prepared in the same manner as in Manufacturing Example 11 above and 648.8 mg of PEG-acrylate were dissolved in DMSO to a concentration of 1000 mg/ml and added to a flask. The reaction was carried out at 90 °C for 24 hours, and then PEG-3A was obtained.

### [Manufacturing Example 122]

### Preparation of polymer PEG-3B

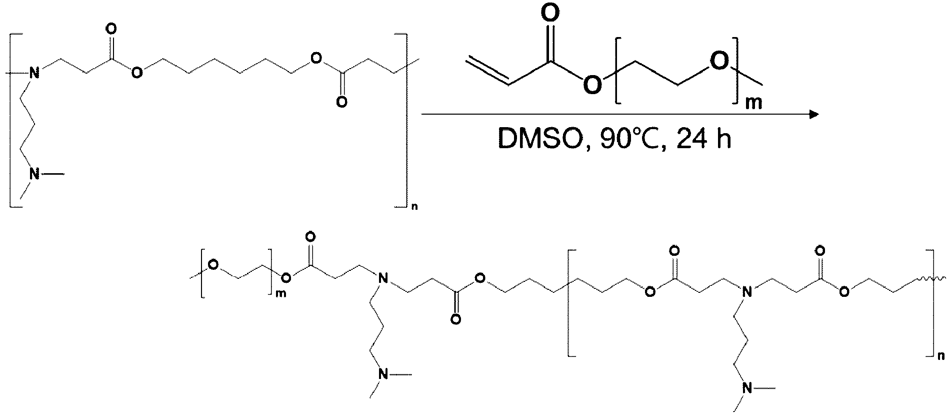

Polymer 3B prepared in the same manner as in Manufacturing Example 12 above and 752.3 mg of PEG-acrylate were dissolved in DMSO to a concentration of 1000 mg/ml and added to a flask. The reaction was carried out at 90 °C for 24 hours, and then PEG-3B was obtained.

### [Manufacturing Example 123]

### Preparation of polymer PEG-3C

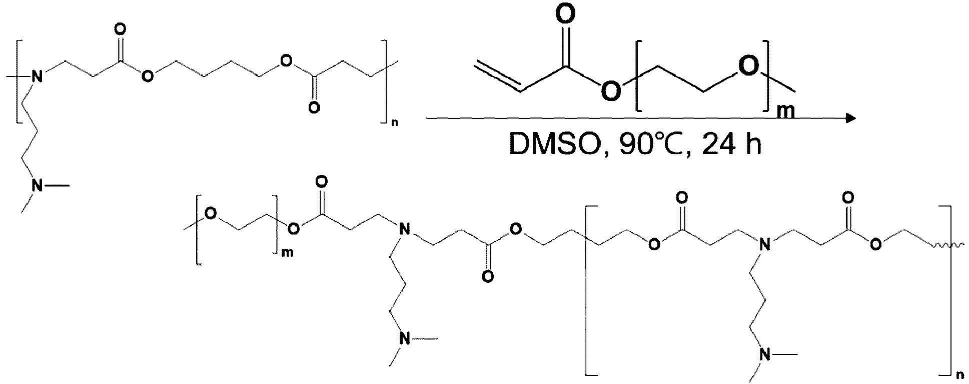

Polymer 3C prepared in the same manner as in Manufacturing Example 13 above and 858.7 mg of PEG-acrylate were dissolved in DMSO to a concentration of 1000 mg/ml and added to a flask. The reaction was carried out at 90 °C for 24 hours, and then PEG-3C was obtained.

### [Manufacturing Example 124]

### Preparation of polymer PEG-3D

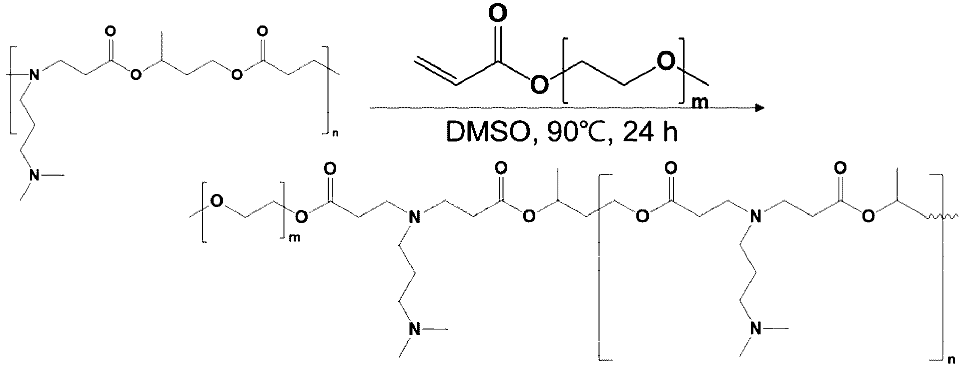

Polymer 3D prepared in the same manner as in Manufacturing Example 14 above and 858.7 mg of PEG-acrylate were dissolved in DMSO to a concentration of 1000 mg/ml and added to a flask. The reaction was carried out at 90 °C for 24 hours, and then PEG-3D was obtained.

### [Manufacturing Example 125]

### Preparation of polymer PEG-3E

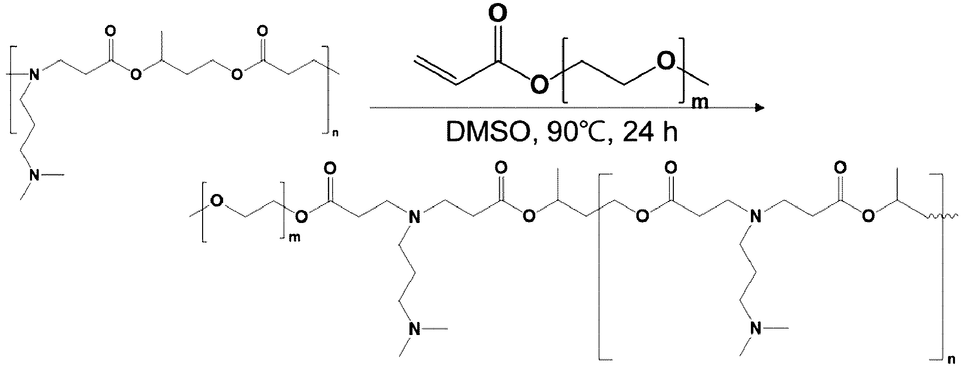

Polymer 3E prepared in the same manner as in Manufacturing Example 15 above and 563.0 mg of PEG-acrylate were dissolved in DMSO to a concentration of 2500 mg/ml and added to a flask. The reaction was carried out at 90 °C for 24 hours, and then PEG-3E was obtained.

### [Manufacturing Example 126]

### Preparation of polymer PEG-4A

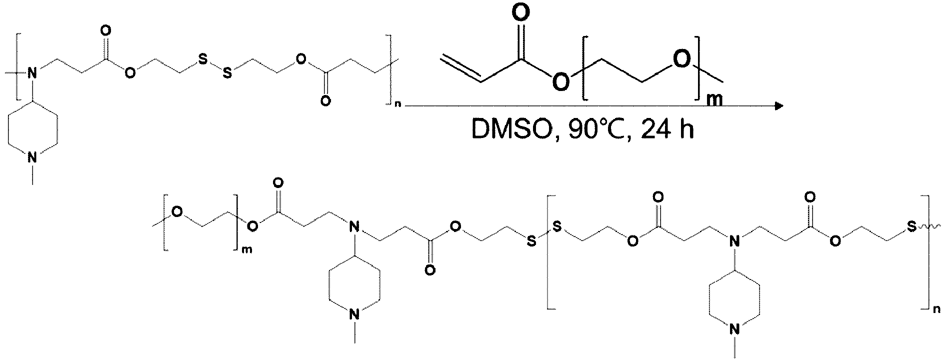

Polymer 4A prepared in the same manner as in Manufacturing Example 16 above and 648.8 mg of PEG-acrylate were dissolved in DMSO to a concentration of 1000 mg/ml and added to a flask. The reaction was carried out at 90 °C for 24 hours, and then PEG-4A was obtained.

### [Manufacturing Example 127]

### Preparation of polymer PEG-4B

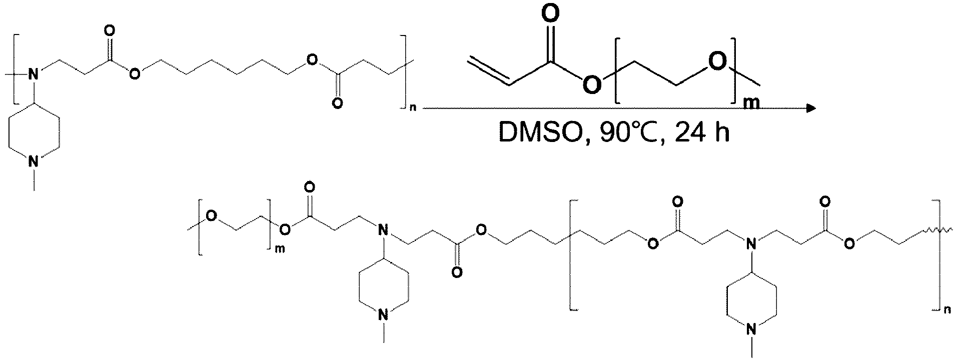

Polymer 4B prepared in the same manner as in Manufacturing Example 17 above and 752.3 mg of PEG-acrylate were dissolved in DMSO to a concentration of 1000 mg/ml and added to a flask. The reaction was carried out at 90 °C for 24 hours, and then PEG-4B was obtained.

### [Manufacturing Example 128]

### Preparation of polymer PEG-4C

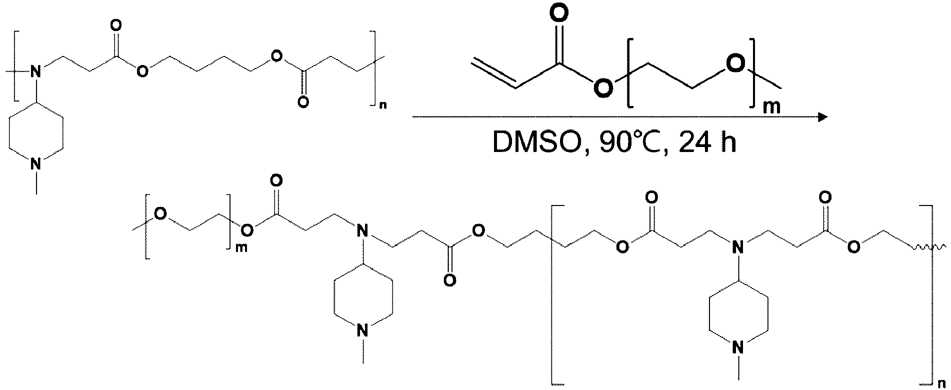

Polymer 4C prepared in the same manner as in Manufacturing Example 18 above and 858.7 mg of PEG-acrylate were dissolved in DMSO to a concentration of 1000 mg/ml and added to a flask. The reaction was carried out at 90 °C for 24 hours, and then PEG-4C was obtained.

### [Manufacturing Example 129]

### Preparation of polymer PEG-4D

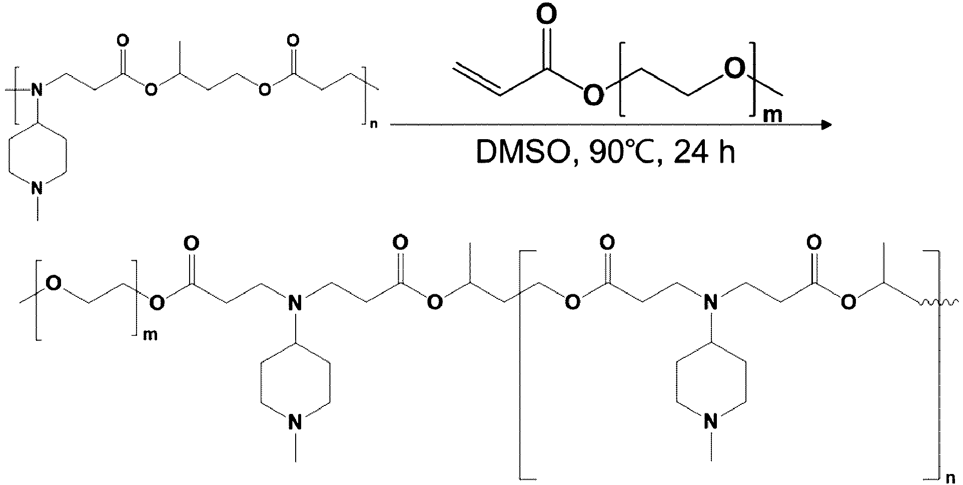

Polymer 4D prepared in the same manner as in Manufacturing Example 19 above and 858.7 mg of PEG-acrylate were dissolved in DMSO to a concentration of 1000 mg/ml and added to a flask. The reaction was carried out at 90 °C for 24 hours, and then PEG-4D was obtained.

### [Manufacturing Example 130]

### Preparation of polymer PEG-4E

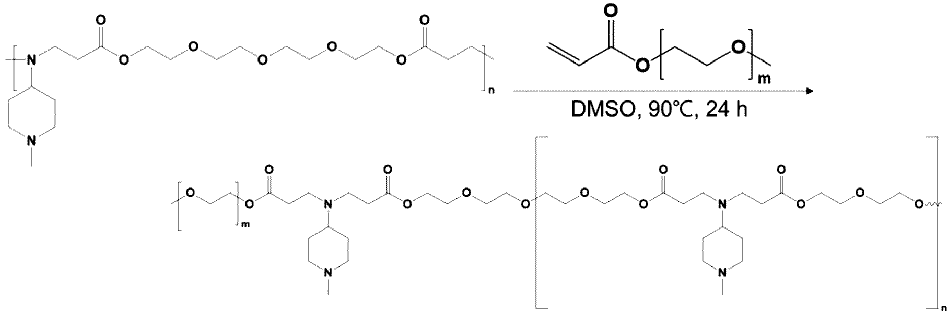

Polymer 4E prepared in the same manner as in Manufacturing Example 20 above and 563.0 mg of PEG-acrylate were dissolved in DMSO to a concentration of 2500 mg/ml and added to a flask. The reaction was carried out at 90 °C for 24 hours, and then PEG-4E was obtained.

### [Manufacturing Example 131]

### Preparation of polymer PEG-5A

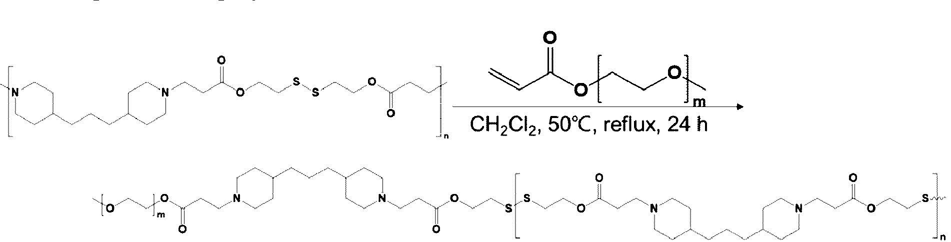

Polymer 5A prepared in the same manner as in Manufacturing Example 21 above and 648.8 mg of PEG-acrylate were dissolved in CH₂Cl₂ to a concentration of 1000 mg/ml and added to a flask. The reaction was carried out at 50 °C for 24 hours with reflux, and then PEG-5A was obtained.

### [Manufacturing Example 132]

### Preparation of polymer PEG-5B

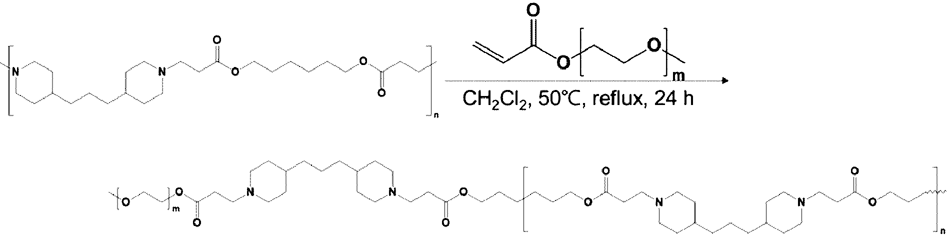

Polymer 5B prepared in the same manner as in Manufacturing Example 22 above and 752.3 mg of PEG-acrylate were dissolved in CH₂Cl₂ to a concentration of 1000 mg/ml and added to a flask. The reaction was carried out at 50 °C for 24 hours with reflux, and then PEG-5B was obtained.

### [Manufacturing Example 133]

### Preparation of polymer PEG-5C

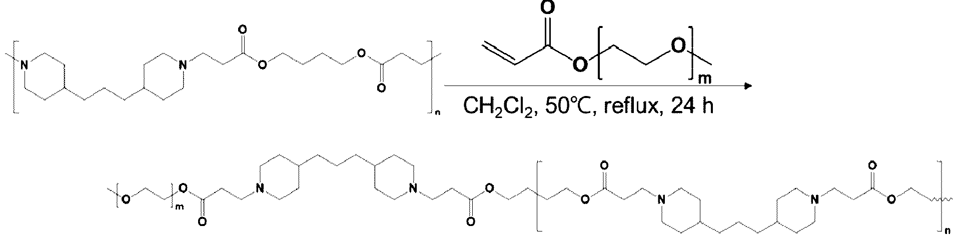

Polymer 5C prepared in the same manner as in Manufacturing Example 23 above and 858.7 mg of PEG-acrylate were dissolved in CH₂Cl₂ to a concentration of 1000 mg/ml and added to a flask. The reaction was carried out at 50 °C for 24 hours with reflux, and then PEG-5C was obtained.

### [Manufacturing Example 134]

### Preparation of polymer PEG-5D

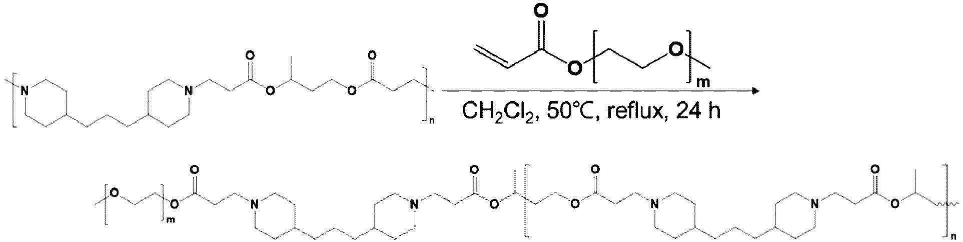

Polymer 5D prepared in the same manner as in Manufacturing Example 24 above and 858.7 mg of PEG-acrylate were dissolved in CH₂Cl₂ to a concentration of 1000 mg/ml and added to a flask. The reaction was carried out at 50 °C for 24 hours with reflux, and then PEG-5D was obtained.

### [Manufacturing Example 135]

### Preparation of polymer PEG-5E

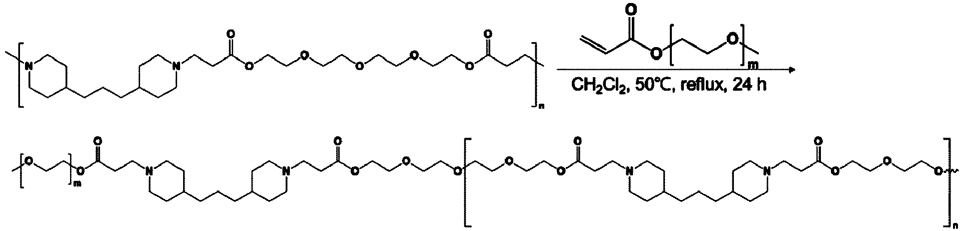

Polymer 5E prepared in the same manner as in Manufacturing Example 25 above and 563.0 mg of PEG-acrylate were dissolved in CH₂Cl₂ to a concentration of 2500 mg/ml and added to a flask. The reaction was carried out at 50 °C for 24 hours with reflux, and then PEG-5E was obtained.

### [Manufacturing Example 136]

### Preparation of polymer PEG-6A

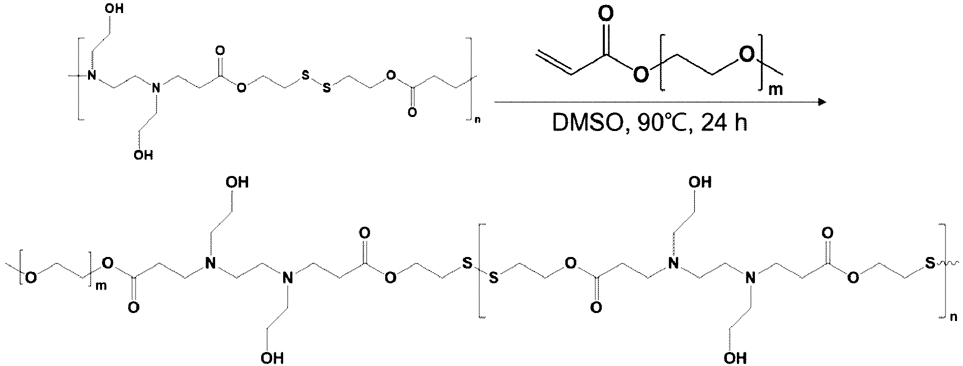

Polymer 6A prepared in the same manner as in Manufacturing Example 26 above and 648.8 mg of PEG-acrylate were dissolved in DMSO to a concentration of 1000 mg/ml and added to a flask. The reaction was carried out at 90 °C for 24 hours, and then PEG-6A was obtained.

### [Manufacturing Example 137]

### Preparation of polymer PEG-6B

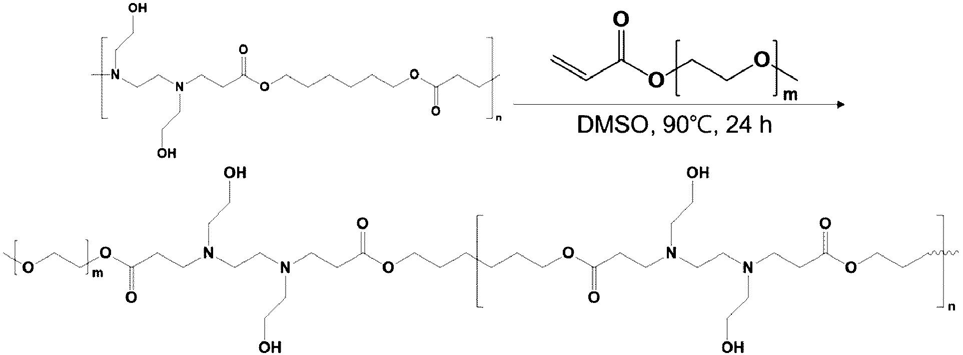

Polymer 6B prepared in the same manner as in Manufacturing Example 27 above and 752.3 mg of PEG-acrylate were dissolved in DMSO to a concentration of 1000 mg/ml and added to a flask. The reaction was carried out at 90 °C for 24 hours, and then PEG-6B was obtained.

### [Manufacturing Example 138]

### Preparation of polymer PEG-6C

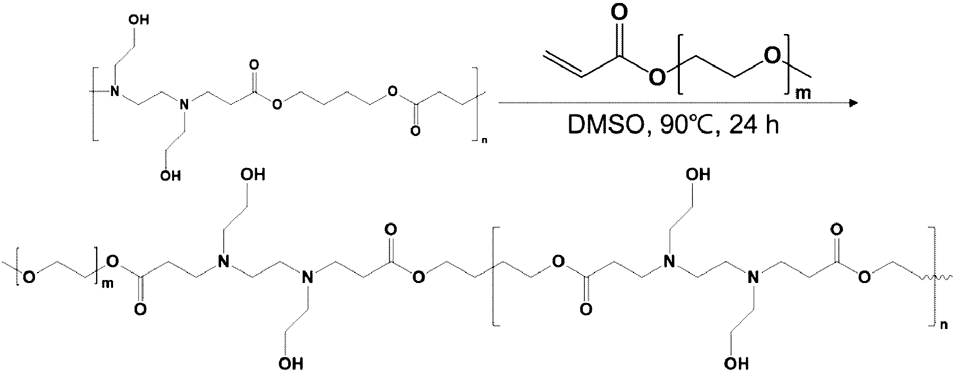

Polymer 6C prepared in the same manner as in Manufacturing Example 28 above and 858.7 mg of PEG-acrylate were dissolved in DMSO to a concentration of 1000 mg/ml and added to a flask. The reaction was carried out at 90 °C for 24 hours, and then PEG-6C was obtained.

### [Manufacturing Example 139]

### Preparation of polymer PEG-6D

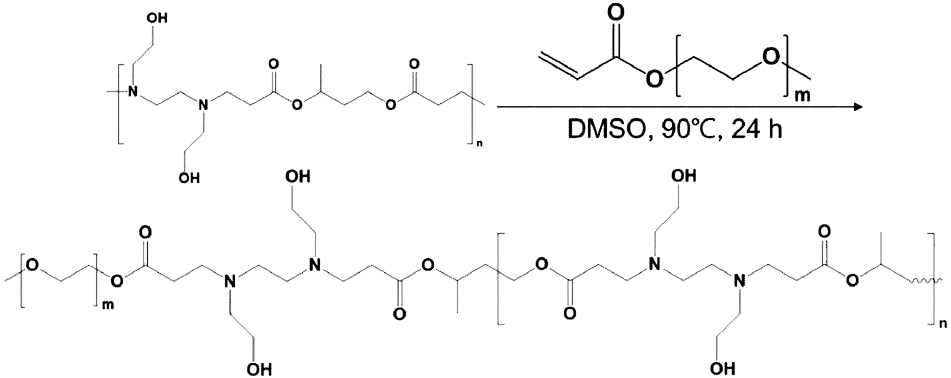

Polymer 6D prepared in the same manner as in Manufacturing Example 29 above and 858.7 mg of PEG-acrylate were dissolved in DMSO to a concentration of 1000 mg/ml and added to a flask. The reaction was carried out at 90 °C for 24 hours, and then PEG-6D was obtained.

### [Manufacturing Example 140]

### Preparation of polymer PEG-6E

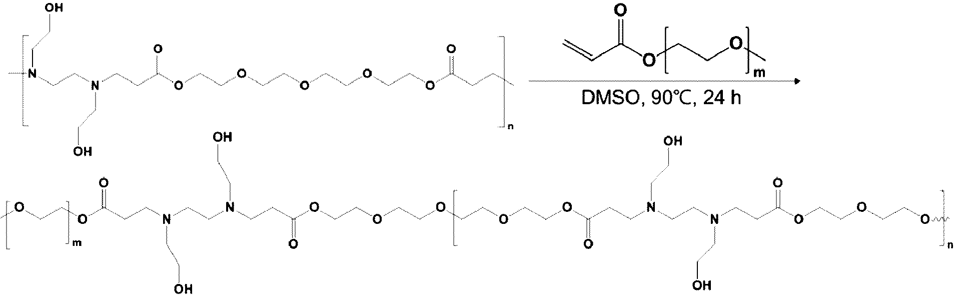

Polymer 6E prepared in the same manner as in Manufacturing Example 30 above and 563.0 mg of PEG-acrylate were dissolved in DMSO to a concentration of 2500 mg/ml and added to a flask. The reaction was carried out at 90 °C for 24 hours, and then PEG-6E was obtained.

### [Manufacturing Example 141]

### Preparation of polymer PEG-7A

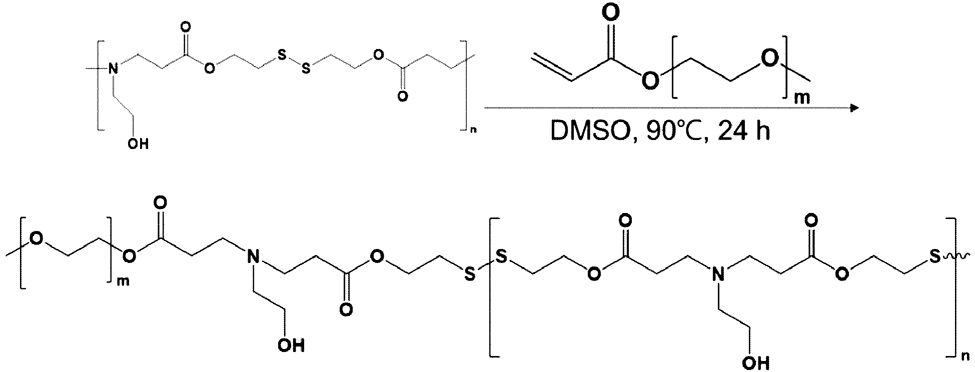

Polymer 7A prepared in the same manner as in Manufacturing Example 31 above and 648.8 mg of PEG-acrylate were dissolved in DMSO to a concentration of 1000 mg/ml and added to a flask. The reaction was carried out at 90 °C for 24 hours, and then PEG-7A was obtained.

### [Manufacturing Example 142]

### Preparation of polymer PEG-7B

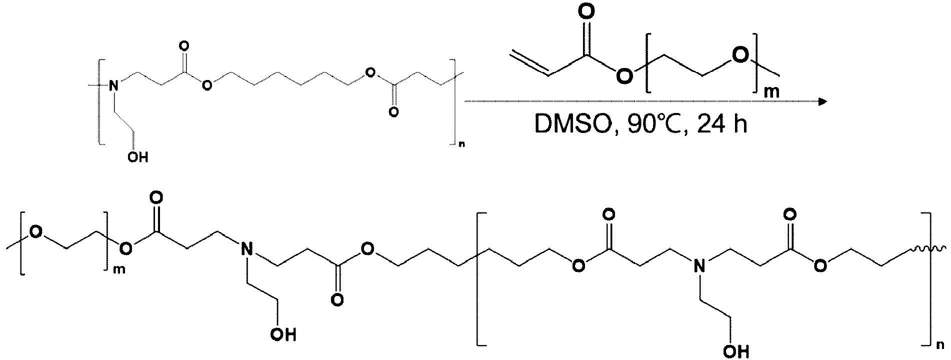

Polymer 7B prepared in the same manner as in Manufacturing Example 32 above and 752.3 mg of PEG-acrylate were dissolved in DMSO to a concentration of 1000 mg/ml and added to a flask. The reaction was carried out at 90 °C for 24 hours, and then PEG-7B was obtained.

### [Manufacturing Example 143]

### Preparation of polymer PEG-7C

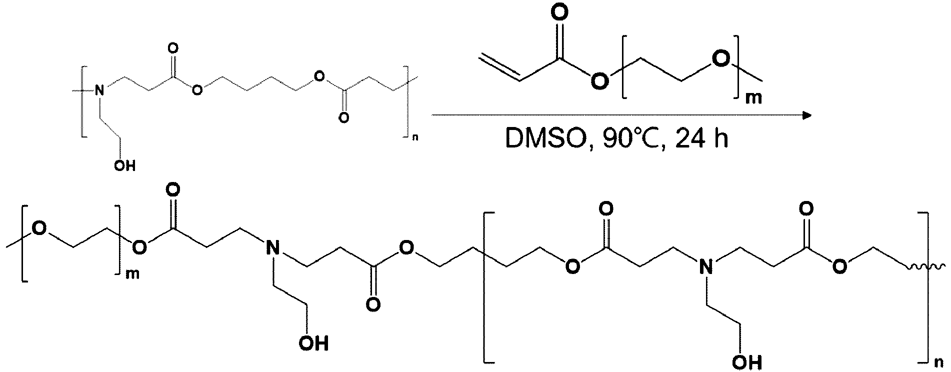

Polymer 7C prepared in the same manner as in Manufacturing Example 33 above and 858.7 mg of PEG-acrylate were dissolved in DMSO to a concentration of 1000 mg/ml and added to a flask. The reaction was carried out at 90 °C for 24 hours, and then PEG-7C was obtained.

### [Manufacturing Example 144]

### Preparation of polymer PEG-7D

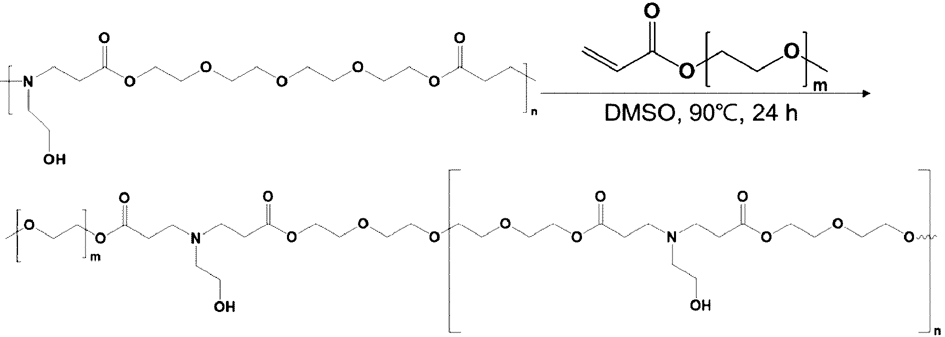

Polymer 7D prepared in the same manner as in Manufacturing Example 34 above and 858.7 mg of PEG-acrylate were dissolved in DMSO to a concentration of 1000 mg/ml and added to a flask. The reaction was carried out at 90 °C for 24 hours, and then PEG-7D was obtained.

### [Manufacturing Example 145]

### Preparation of polymer PEG-7E

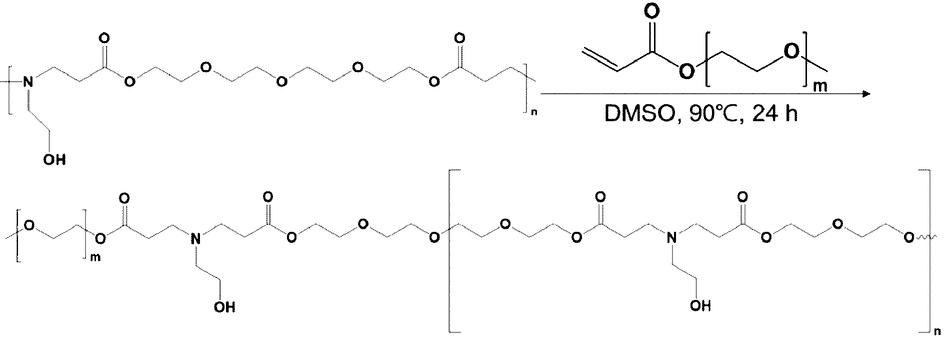

Polymer 7E prepared in the same manner as in Manufacturing Example 35 above and 563.0 mg of PEG-acrylate were dissolved in DMSO to a concentration of 2500 mg/ml and added to a flask. The reaction was carried out at 90 °C for 24 hours, and then PEG-7E was obtained.

### [Manufacturing Example 146]

### Preparation of polymer PEG-8A

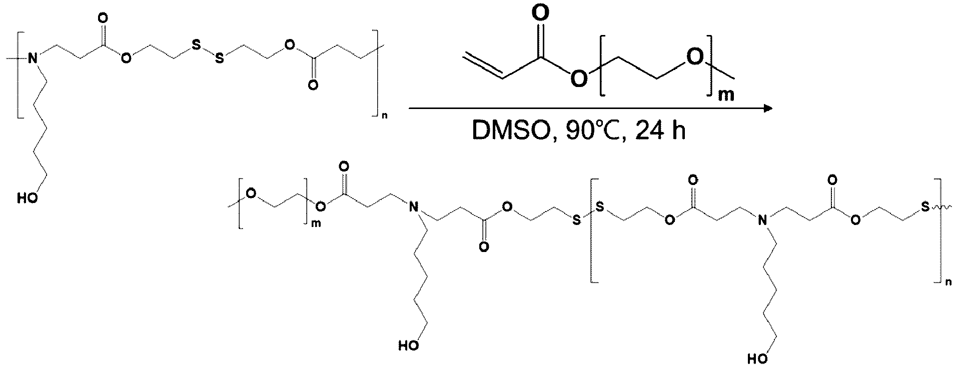

Polymer 8A prepared in the same manner as in Manufacturing Example 36 above and 648.8 mg of PEG-acrylate were dissolved in DMSO to a concentration of 1000 mg/ml and added to a flask. The reaction was carried out at 90 °C for 24 hours, and then PEG-8A was obtained.

### [Manufacturing Example 147]

### Preparation of polymer PEG-8B

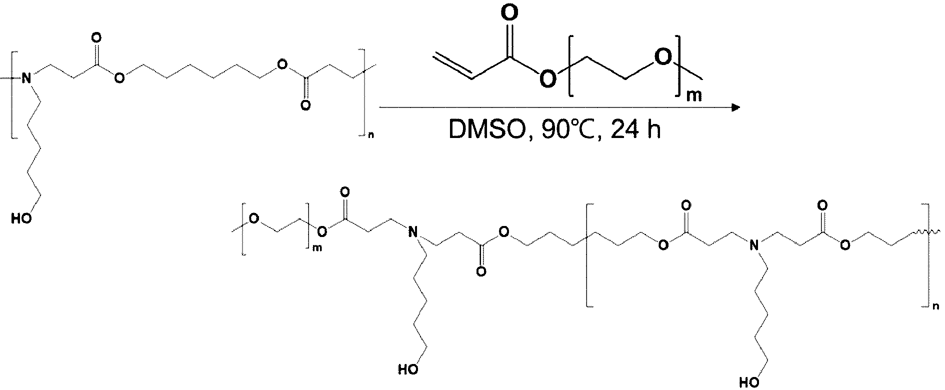

Polymer 8B prepared in the same manner as in Manufacturing Example 37 above and 752.3 mg of PEG-acrylate were dissolved in DMSO to a concentration of 1000 mg/ml and added to a flask. The reaction was carried out at 90 °C for 24 hours, and then PEG-8B was obtained.

### [Manufacturing Example 148]

### Preparation of polymer PEG-8C

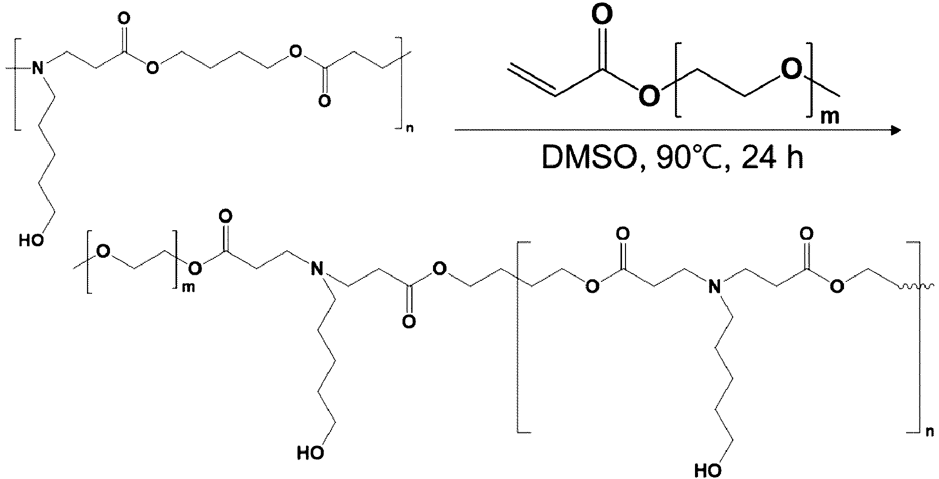

Polymer 8C prepared in the same manner as in Manufacturing Example 38 above and 858.7 mg of PEG-acrylate were dissolved in DMSO to a concentration of 1000 mg/ml and added to a flask. The reaction was carried out at 90 °C for 24 hours, and then PEG-8C was obtained.

### [Manufacturing Example 149]

### Preparation of polymer PEG-8D

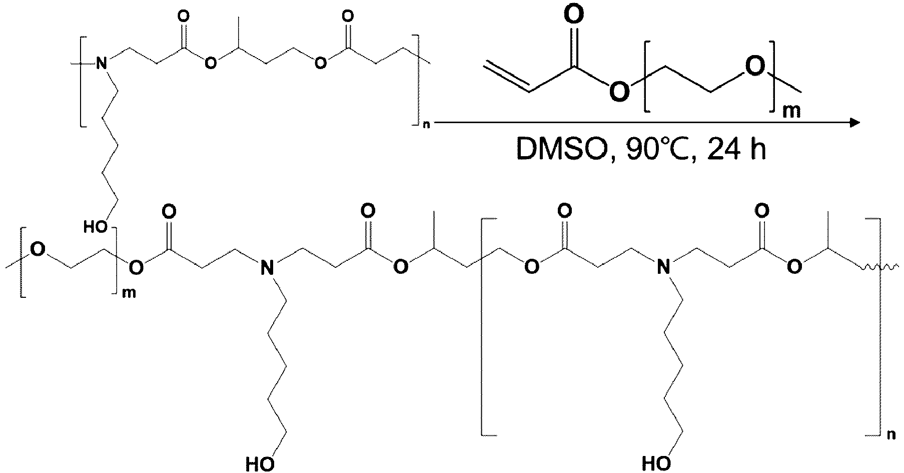

Polymer 8D prepared in the same manner as in Manufacturing Example 39 above and 858.7 mg of PEG-acrylate were dissolved in DMSO to a concentration of 1000 mg/ml and added to a flask. The reaction was carried out at 90 °C for 24 hours, and then PEG-8D was obtained.

### [Manufacturing Example 150]

### Preparation of polymer PEG-8E

Polymer 8E prepared in the same manner as in Manufacturing Example 40 above and 563.0 mg of PEG-acrylate were dissolved in DMSO to a concentration of 2500 mg/ml and added to a flask. The reaction was carried out at 90 °C for 24 hours, and then PEG-8E was obtained.

### [Manufacturing Example 151]

### Preparation of polymer PEG-9A

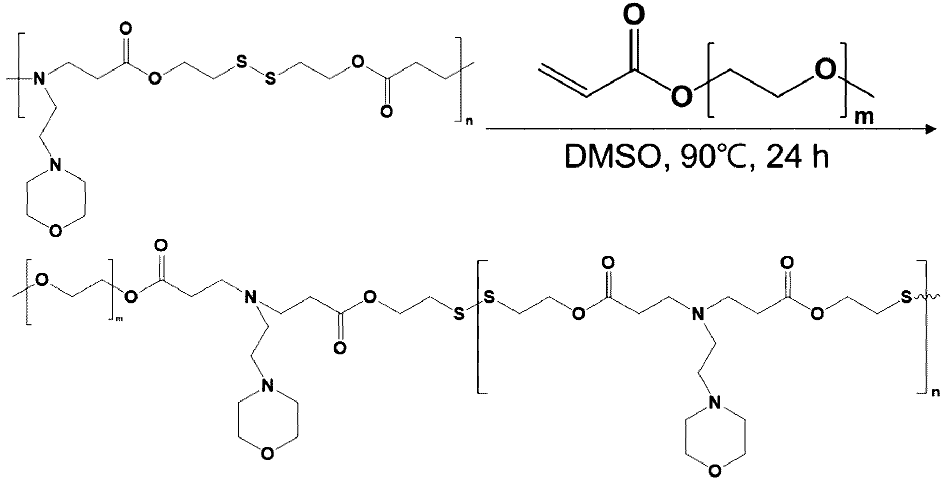

Polymer 9A prepared in the same manner as in Manufacturing Example 41 above and 648.8 mg of PEG-acrylate were dissolved in DMSO to a concentration of 1000 mg/ml and added to a flask. The reaction was carried out at 90 °C for 24 hours, and then PEG-9A was obtained.

### [Manufacturing Example 152]

### Preparation of polymer PEG-9B

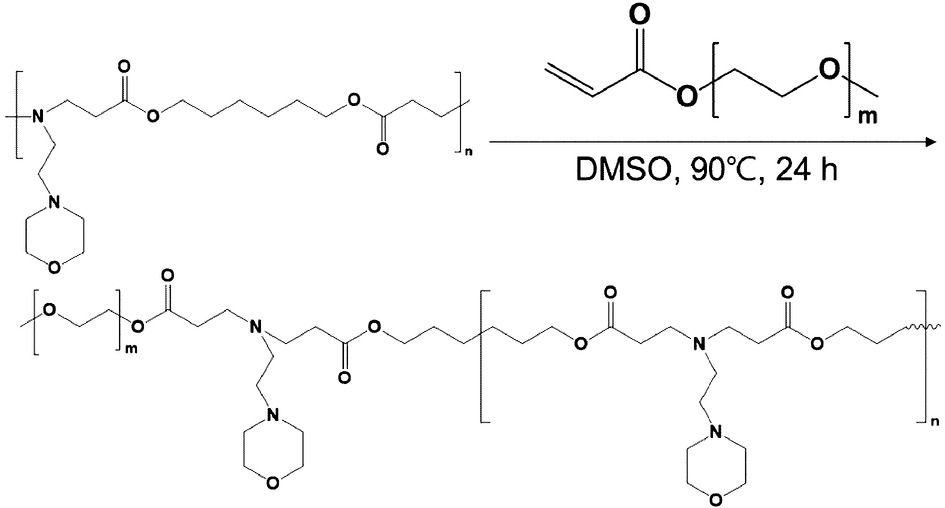

Polymer 9B prepared in the same manner as in Manufacturing Example 42 above and 752.3 mg of PEG-acrylate were dissolved in DMSO to a concentration of 1000 mg/ml and added to a flask. The reaction was carried out at 90 °C for 24 hours, and then PEG-9B was obtained.

### [Manufacturing Example 153]

### Preparation of polymer PEG-9C

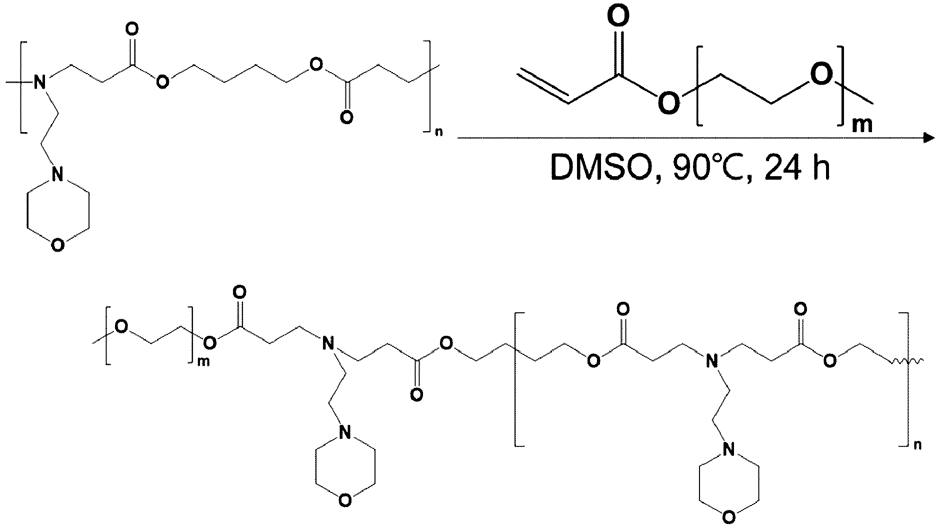

Polymer 9C prepared in the same manner as in Manufacturing Example 43 above and 858.7 mg of PEG-acrylate were dissolved in DMSO to a concentration of 1000 mg/ml and added to a flask. The reaction was carried out at 90 °C for 24 hours, and then PEG-9C was obtained.

### [Manufacturing Example 154]

### Preparation of polymer PEG-9D

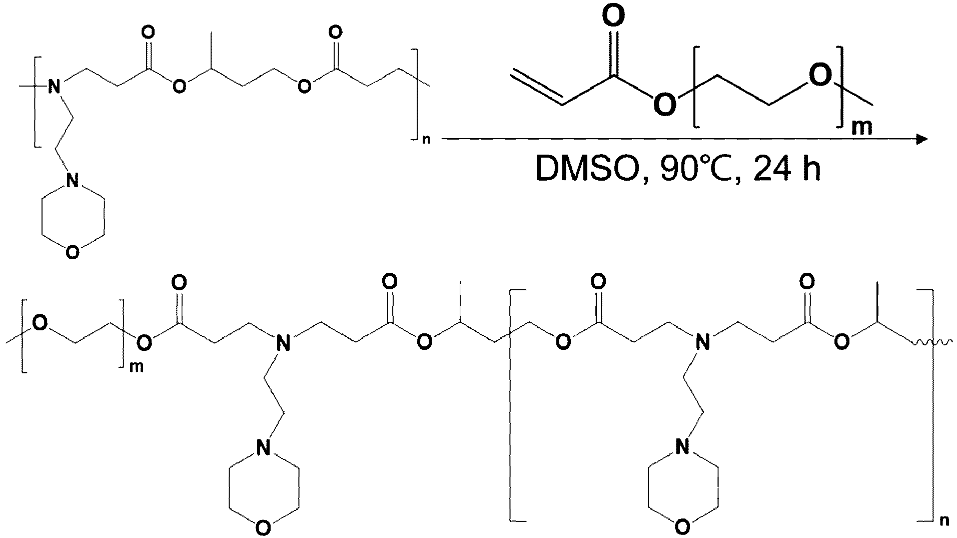

Polymer 9D prepared in the same manner as in Manufacturing Example 44 above and 858.7 mg of PEG-acrylate were dissolved in DMSO to a concentration of 1000 mg/ml and added to a flask. The reaction was carried out at 90 °C for 24 hours, and then PEG-9D was obtained.

### [Manufacturing Example 155]

### Preparation of polymer PEG-9E

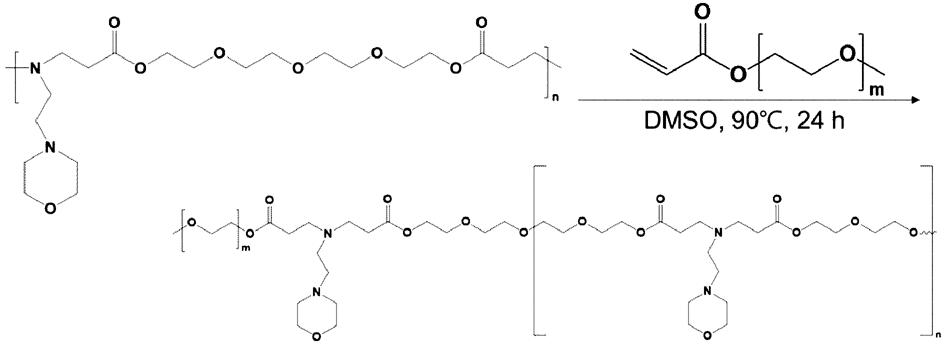

Polymer 9E prepared in the same manner as in Manufacturing Example 45 above and 563.0 mg of PEG-acrylate were dissolved in DMSO to a concentration of 2500 mg/ml and added to a flask. The reaction was carried out at 90 °C for 24 hours, and then PEG-9E was obtained.

### [Manufacturing Example 156]

### Preparation of polymer PEG-10A

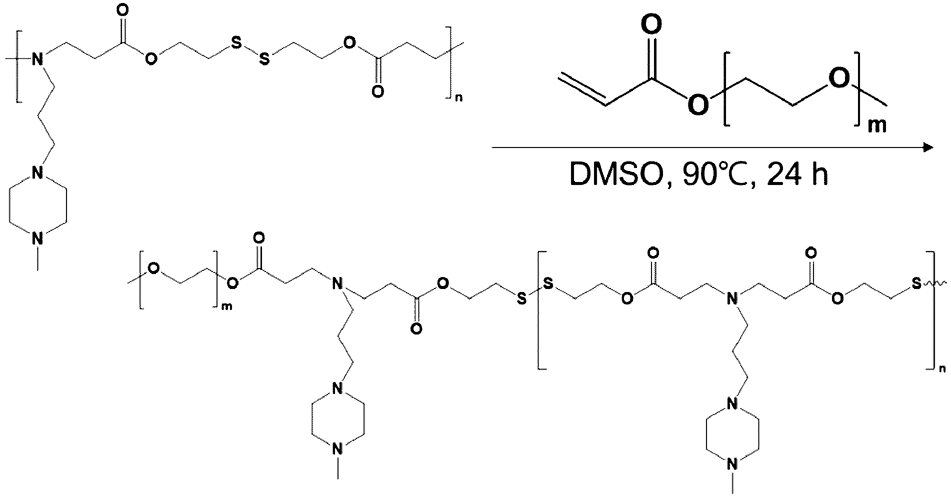

Polymer 10A prepared in the same manner as in Manufacturing Example 46 above and 648.8 mg of PEG-acrylate were dissolved in DMSO to a concentration of 1000 mg/ml and added to a flask. The reaction was carried out at 90 °C for 24 hours, and then PEG-10A was obtained.

### [Manufacturing Example 157]

### Preparation of polymer PEG-10B

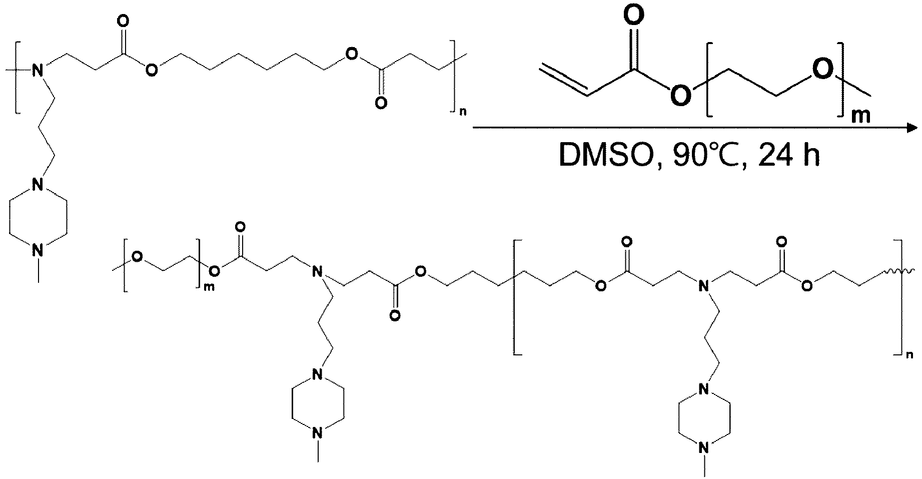

Polymer 10B prepared in the same manner as in Manufacturing Example 47 above and 752.3 mg of PEG-acrylate were dissolved in DMSO to a concentration of 1000 mg/ml and added to a flask. The reaction was carried out at 90 °C for 24 hours, and then PEG-10B was obtained.

### [Manufacturing Example 158]

### Preparation of polymer PEG-10C

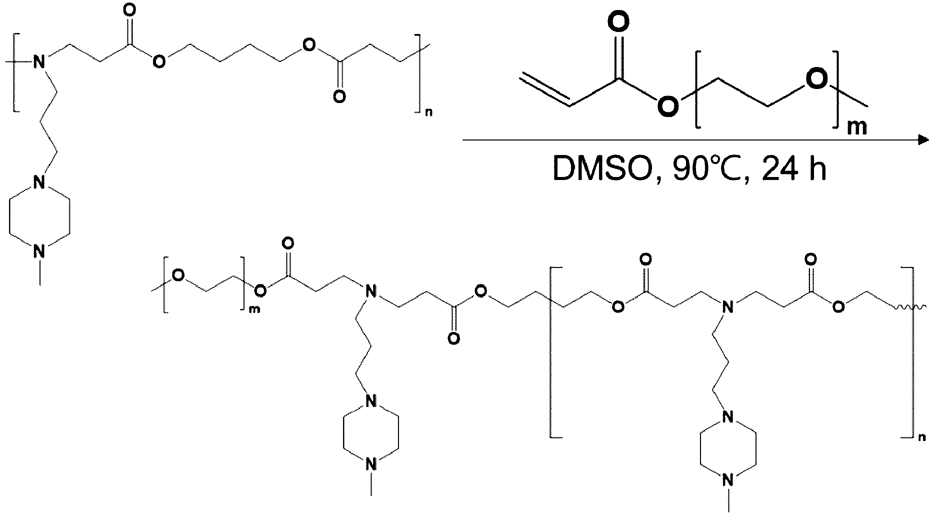

Polymer 10C prepared in the same manner as in Manufacturing Example 48 above and 858.7 mg of PEG-acrylate were dissolved in DMSO to a concentration of 1000 mg/ml and added to a flask. The reaction was carried out at 90 °C for 24 hours, and then PEG-10C was obtained.

### [Manufacturing Example 159]

### Preparation of polymer PEG-10D

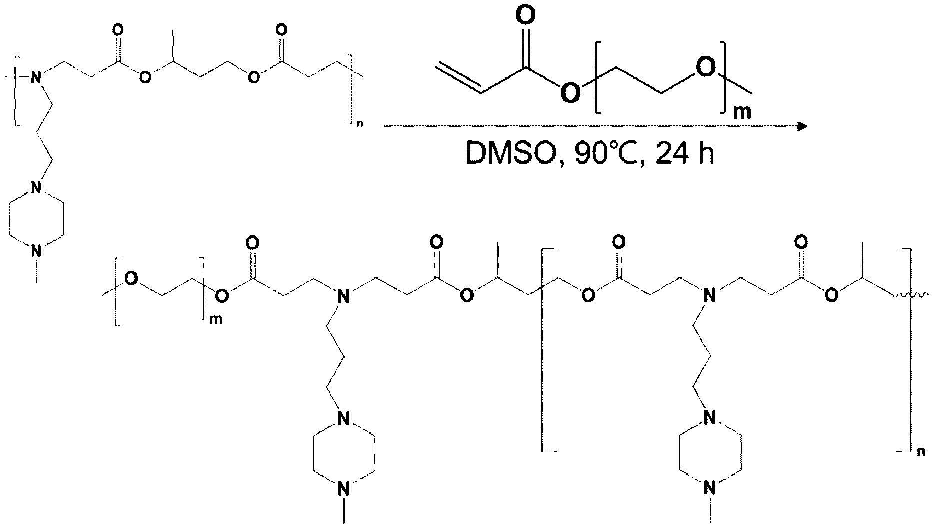

Polymer 10D prepared in the same manner as in Manufacturing Example 49 above and 858.7 mg of PEG-acrylate were dissolved in DMSO to a concentration of 1000 mg/ml and added to a flask. The reaction was carried out at 90 °C for 24 hours, and then PEG-10D was obtained.

### [Manufacturing Example 160]

### Preparation of polymer PEG-10E

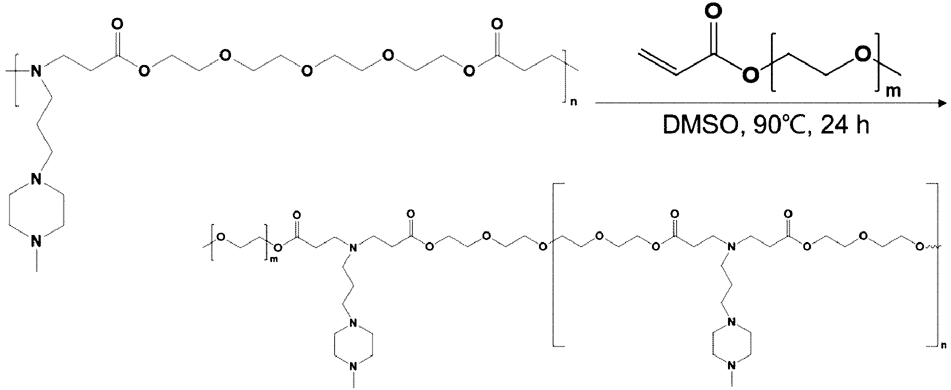

Polymer 10E prepared in the same manner as in Manufacturing Example 50 above and 563.0 mg of PEG-acrylate were dissolved in DMSO to a concentration of 2500 mg/ml and added to a flask. The reaction was carried out at 90 °C for 24 hours, and then PEG-10E was obtained.

### [Manufacturing Example 161]

### Preparation of polymer PEG-11A

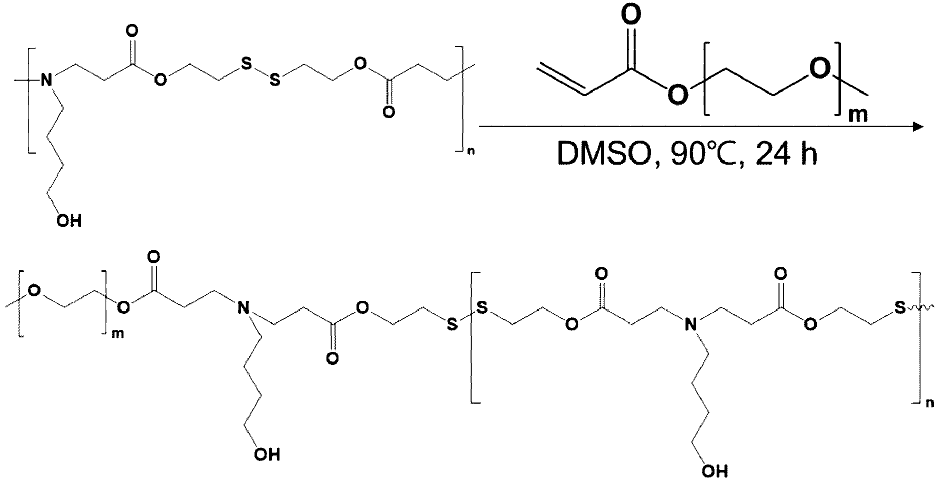

Polymer 11A prepared in the same manner as in Manufacturing Example 51 above and 648.8 mg of PEG-acrylate were dissolved in DMSO to a concentration of 1000 mg/ml and added to a flask. The reaction was carried out at 90 °C for 24 hours, and then PEG-11A was obtained.

### [Manufacturing Example 162]

### Preparation of polymer PEG-11B

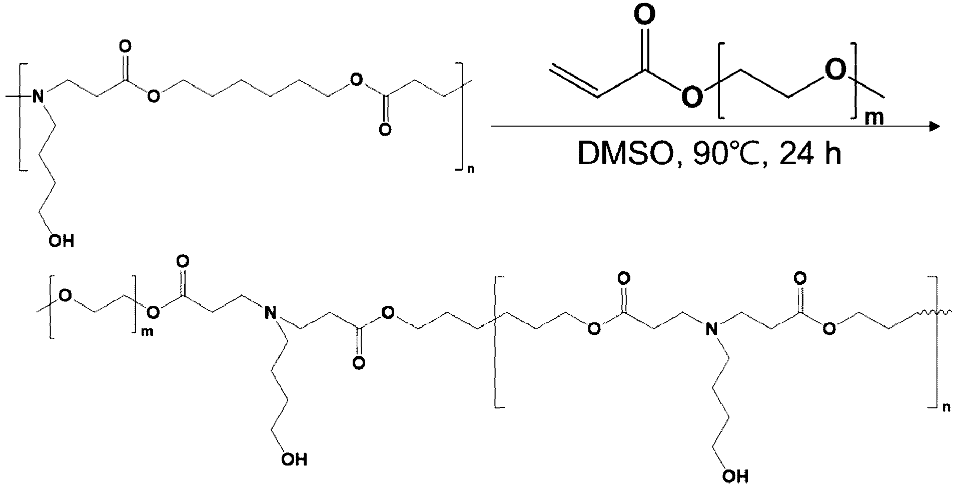

Polymer 11B prepared in the same manner as in Manufacturing Example 52 above and 752.3 mg of PEG-acrylate were dissolved in DMSO to a concentration of 1000 mg/ml and added to a flask. The reaction was carried out at 90 °C for 24 hours, and then PEG-11B was obtained.

### [Manufacturing Example 163]

### Preparation of polymer PEG-11C

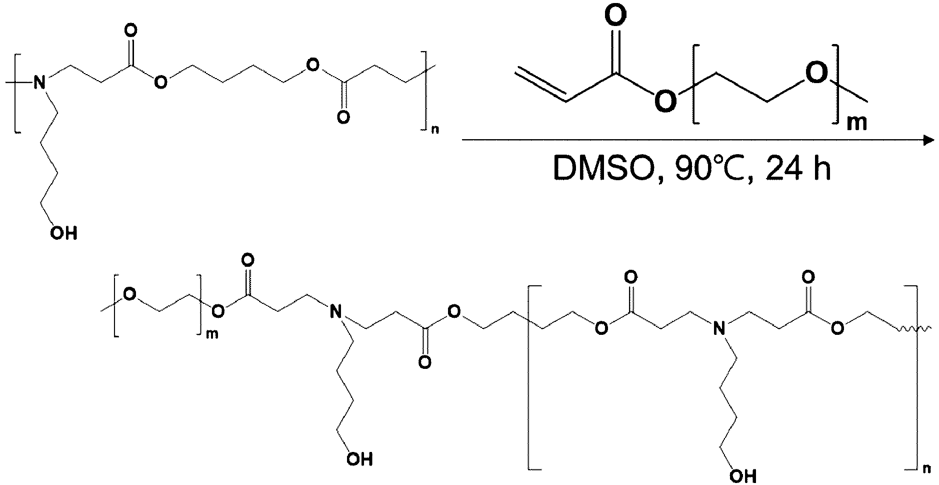

Polymer 11C prepared in the same manner as in Manufacturing Example 53 above and 858.7 mg of PEG-acrylate were dissolved in DMSO to a concentration of 1000 mg/ml and added to a flask. The reaction was carried out at 90 °C for 24 hours, and then PEG-11C was obtained.

### [Manufacturing Example 164]

### Preparation of polymer PEG-11D

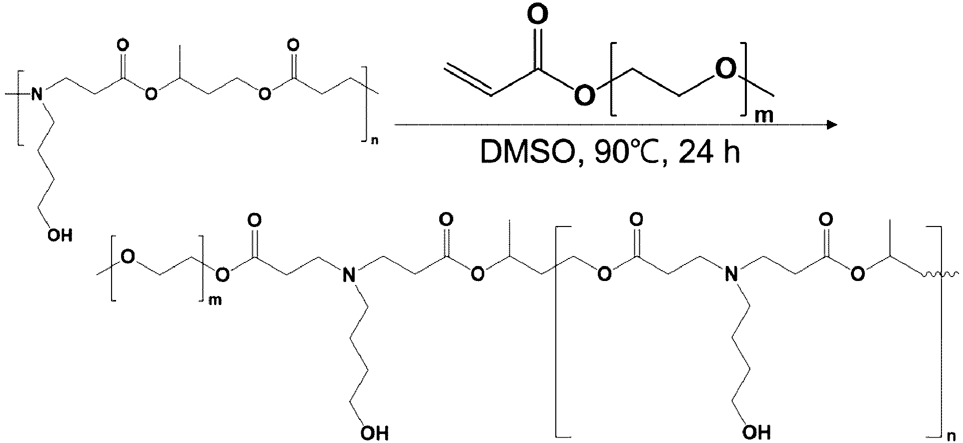

Polymer 11D prepared in the same manner as in Manufacturing Example 54 above and 858.7 mg of PEG-acrylate were dissolved in DMSO to a concentration of 1000 mg/ml and added to **a flask. The reaction was carried out at 90 °C for 24** hours, and then PEG-11D was obtained.

### [Manufacturing Example 165]

### Preparation of polymer PEG-11E

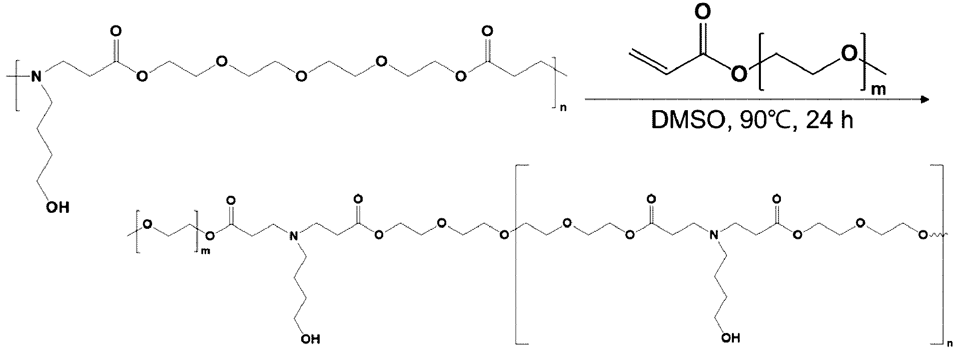

Polymer 11E prepared in the same manner as in Manufacturing Example 55 above and 563.0 mg of PEG-acrylate were dissolved in DMSO to a concentration of 2500 mg/ml and added to a flask. The reaction was carried out at 90 °C for 24 hours, and then PEG-11E was obtained.

### [Manufacturing Example 166]

### Preparation of polymer PEG-1F

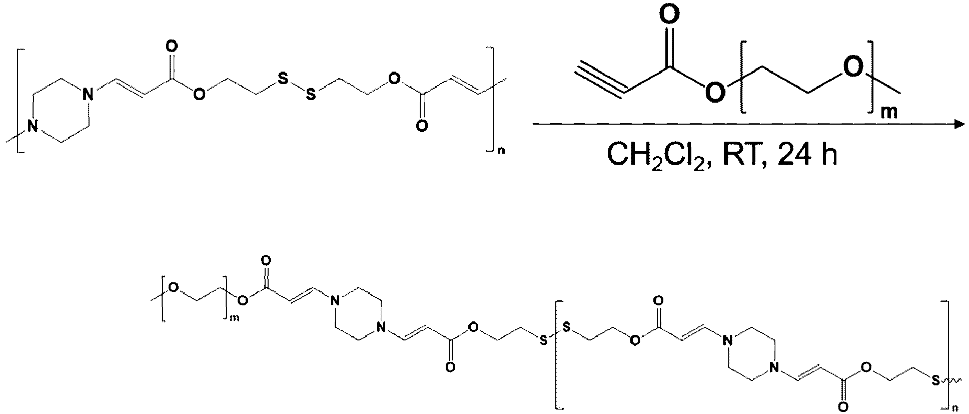

Polymer 1F prepared in the same manner as in Manufacturing Example 56 above and 329.5 mg of PEG-alkynoate were dissolved in 4 mL of CH₂Cl₂ and added to a flask. The reaction was carried out at room temperature for 24 hours, and then PEG-1F was obtained.

### [Manufacturing Example 167]

### Preparation of polymer PEG-1G

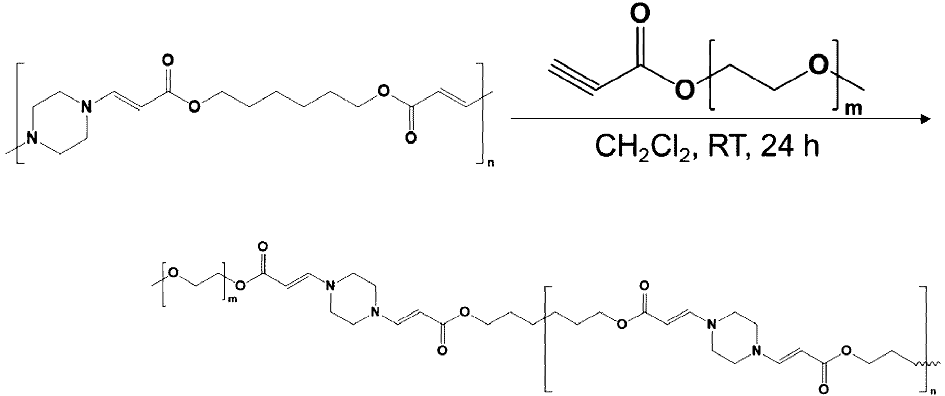

Polymer 1G prepared in the same manner as in Manufacturing Example 57 above and 383.0 mg of PEG-alkynoate were dissolved in 4 mL of CH₂Cl₂ and added to a flask. The reaction was carried out at room temperature for 24 hours, and then PEG-1G was obtained.

### [Manufacturing Example 168]

### Preparation of polymer PEG-1H

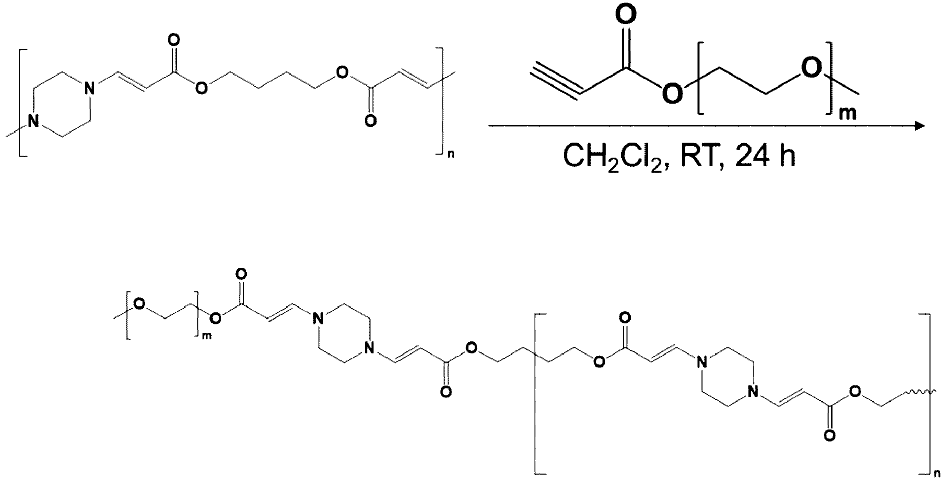

Polymer 1H prepared in the same manner as in Manufacturing Example 58 above and 438.3 mg of PEG-alkynoate were dissolved in 4 mL of CH₂Cl₂ and added to a flask. The reaction was carried out at room temperature for 24 hours, and then PEG-1H was obtained.

### [Manufacturing Example 169]

### Preparation of polymer PEG-11

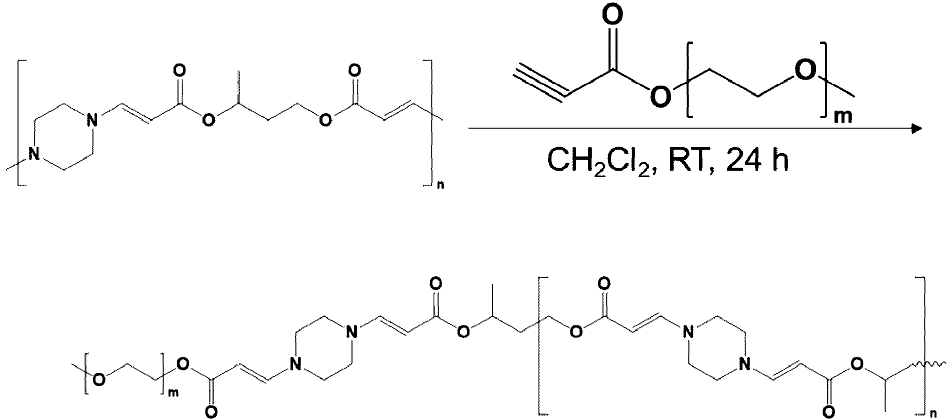

Polymer 1I prepared in the same manner as in Manufacturing Example 59 above and 438.3 mg of PEG-alkynoate were dissolved in 4 mL of CH₂Cl₂ and added to a flask. The reaction was carried out at room temperature for 24 hours, and then PEG-1I was obtained.

### [Manufacturing Example 170]

### Preparation of polymer PEG-1J

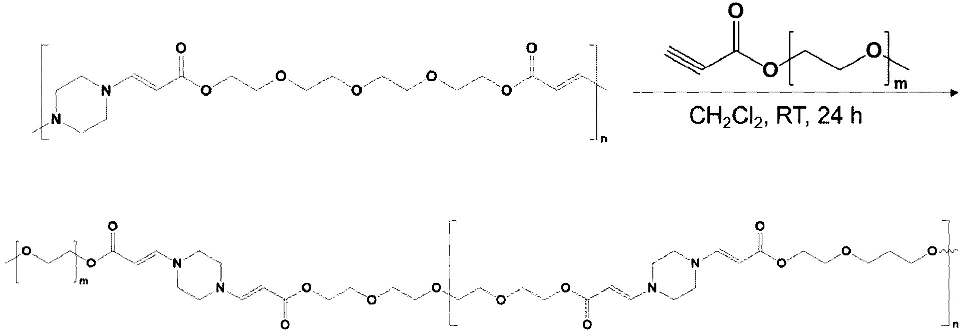

Polymer 1J prepared in the same manner as in Manufacturing Example 60 above and 285.3 mg of PEG-alkynoate were dissolved in 4 mL of CH₂Cl₂ and added to a flask. The reaction was carried out at room temperature for 24 hours, and then PEG-1J was obtained.

### [Manufacturing Example 171]

### Preparation of polymer PEG-2F

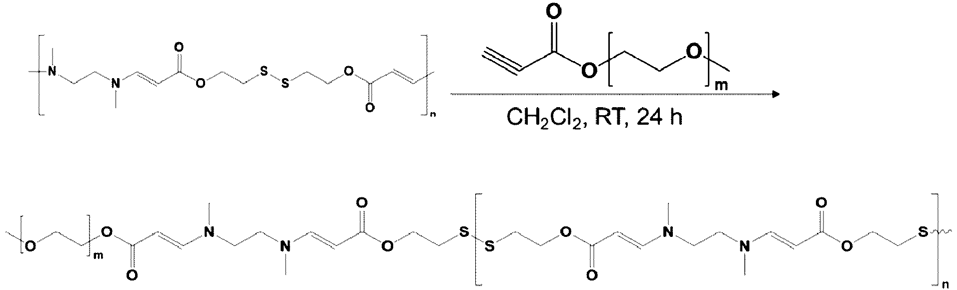

Polymer 2F prepared in the same manner as in Manufacturing Example 61 above and 329.5 mg of PEG-alkynoate were dissolved in 4 mL of CH₂Cl₂ and added to a flask. The reaction was carried out at room temperature for 24 hours, and then PEG-2F was obtained.

### [Manufacturing Example 172]

### Preparation of polymer PEG-2G

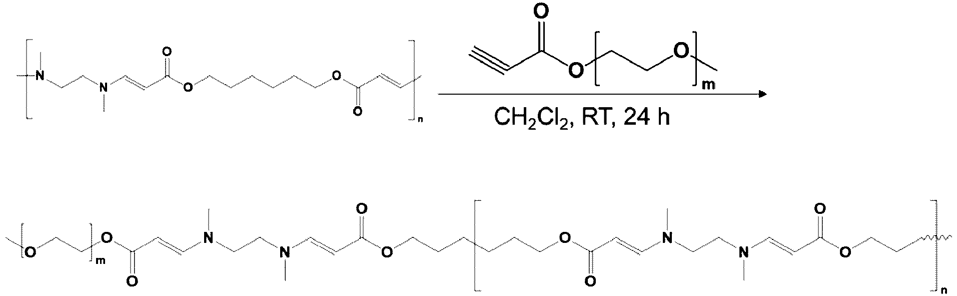

Polymer 2G prepared in the same manner as in Manufacturing Example 62 above and 383.0 mg of PEG-alkynoate were dissolved in 4 mL of CH₂Cl₂ and added to a flask. The reaction was carried out at room temperature for 24 hours, and then PEG-2G was obtained.

### [Manufacturing Example 173]

### Preparation of polymer PEG-2H

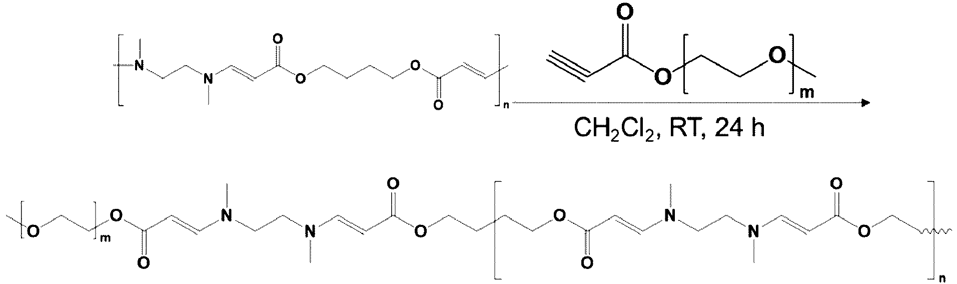

Polymer 2H prepared in the same manner as in Manufacturing Example 63 above and 438.3 mg of PEG-alkynoate were dissolved in 4 mL of CH₂Cl₂ and added to a flask. The reaction was carried out at room temperature for 24 hours, and then PEG-2H was obtained.

### [Manufacturing Example 174]

### Preparation of polymer PEG-21

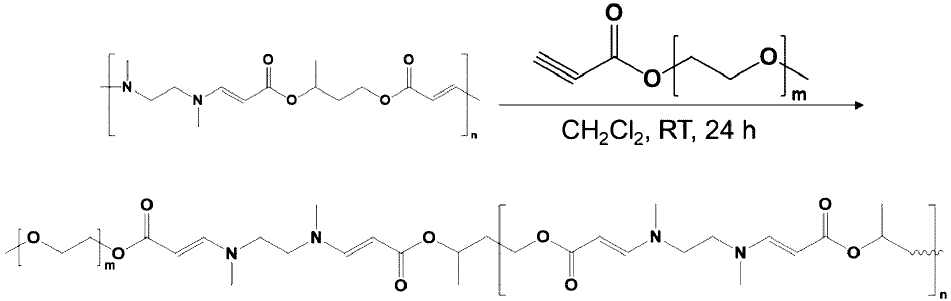

Polymer 2I prepared in the same manner as in Manufacturing Example 64 above and 438.3 mg of PEG-alkynoate were dissolved in 4 mL of CH₂Cl₂ and added to a flask. The reaction was carried out at room temperature for 24 hours, and then PEG-2I was obtained.

### [Manufacturing Example 175]

### Preparation of polymer PEG-2J

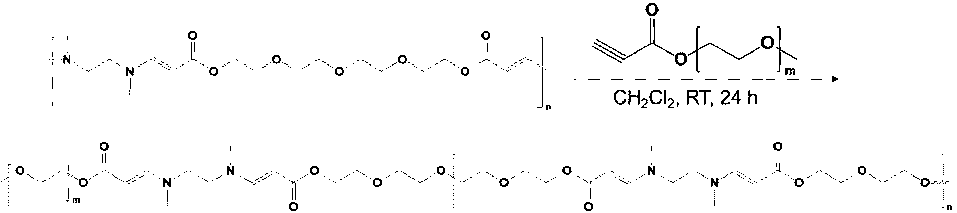

Polymer 2J prepared in the same manner as in Manufacturing Example 65 above and 285.3 mg of PEG-alkynoate were dissolved in 4 mL of CH₂Cl₂ and added to a flask. The reaction was carried out at room temperature for 24 hours, and then PEG-2J was obtained.

### [Manufacturing Example 176]

### Preparation of polymer PEG-3F

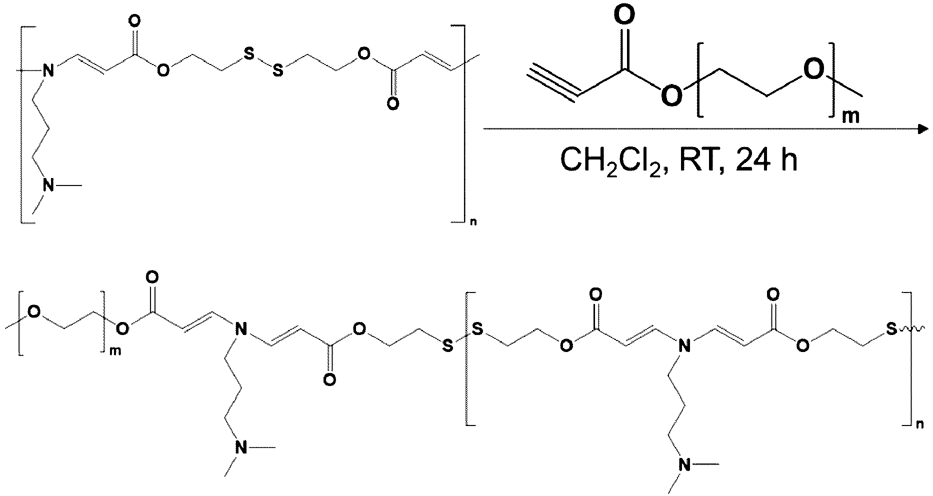

Polymer 3F prepared in the same manner as in Manufacturing Example 66 above and 329.5 mg of PEG-alkynoate were dissolved in 4 mL of CH₂Cl₂ and added to a flask. The reaction was carried out at room temperature for 24 hours, and then PEG-3F was obtained.

### [Manufacturing Example 177]

### Preparation of polymer PEG-3G

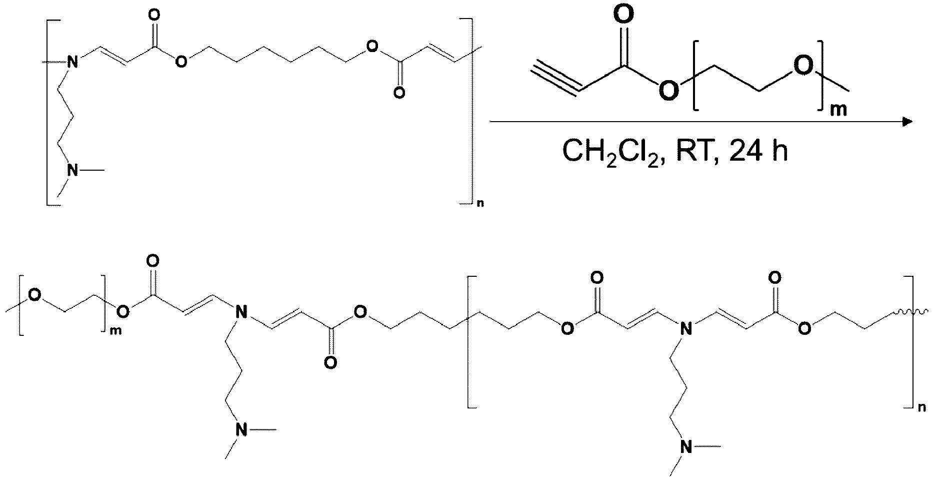

Polymer 3G prepared in the same manner as in Manufacturing Example 67 above and 383.0 mg of PEG-alkynoate were dissolved in 4 mL of CH₂Cl₂ and added to a flask. The reaction was carried out at room temperature for 24 hours, and then PEG-3G was obtained.

### [Manufacturing Example 178]

### Preparation of polymer PEG-3H

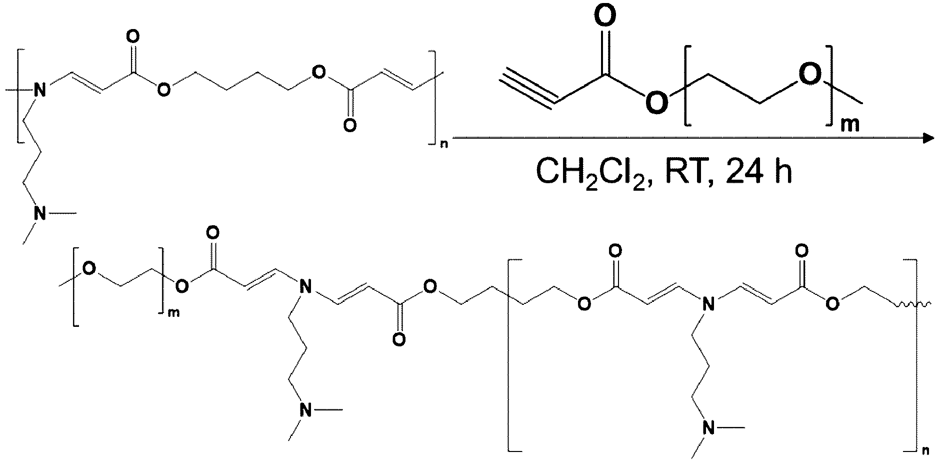

Polymer 3H prepared in the same manner as in Manufacturing Example 68 above and 438.3 mg of PEG-alkynoate were dissolved in 4 mL of CH₂Cl₂ and added to a flask. The reaction was carried out at room temperature for 24 hours, and then PEG-3H was obtained.

### [Manufacturing Example 179]

### Preparation of polymer PEG-3I

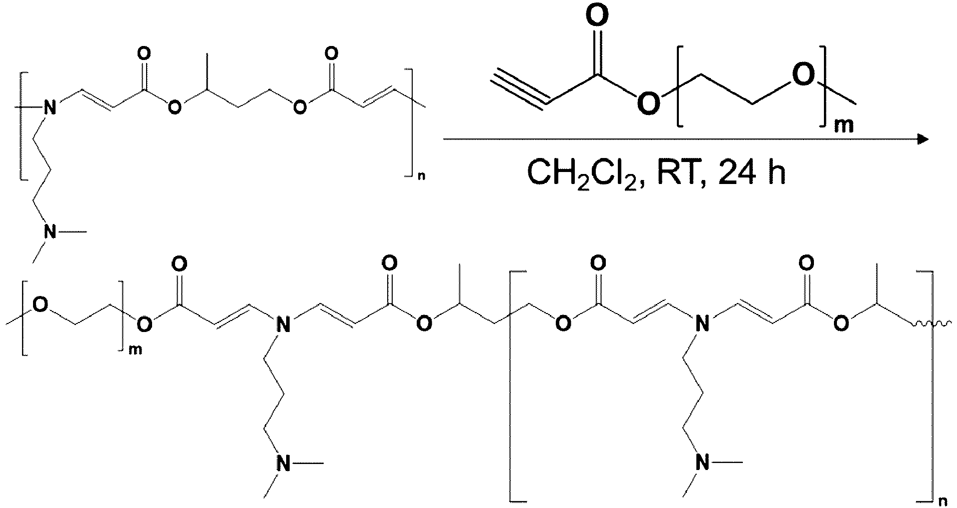

Polymer 3I prepared in the same manner as in Manufacturing Example 69 above and 438.3 mg of PEG-alkynoate were dissolved in 4 mL of CH₂Cl₂ and added to a flask. The reaction was carried out at room temperature for 24 hours, and then PEG-3I was obtained.

### [Manufacturing Example 180]

### Preparation of polymer PEG-3J

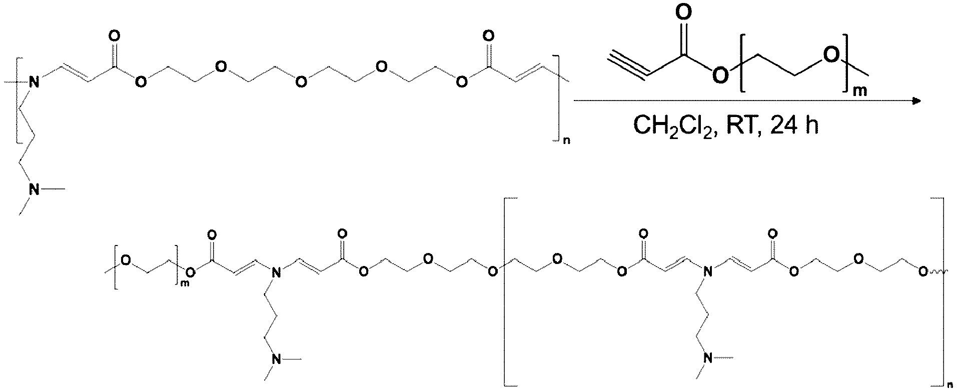

Polymer 3J prepared in the same manner as in Manufacturing Example 70 above and 285.3 mg of PEG-alkynoate were dissolved in 4 mL of CH₂Cl₂ and added to a flask. The reaction was carried out at room temperature for 24 hours, and then PEG-3J was obtained.

### [Manufacturing Example 181]

### Preparation of polymer PEG-4F

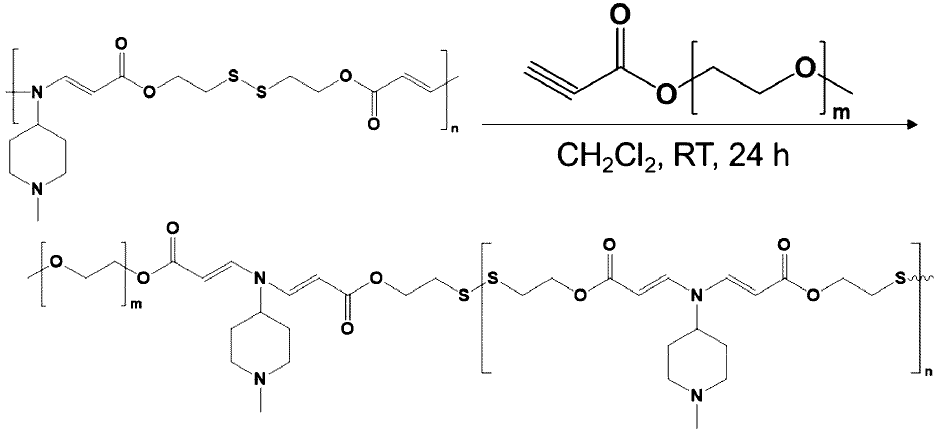

Polymer 4F prepared in the same manner as in Manufacturing Example 71 above and 329.5 mg of PEG-alkynoate were dissolved in 4 mL of CH₂Cl₂ and added to a flask. The reaction was carried out at room temperature for 24 hours, and then PEG-4F was obtained.

### [Manufacturing Example 182]

### Preparation of polymer PEG-4G

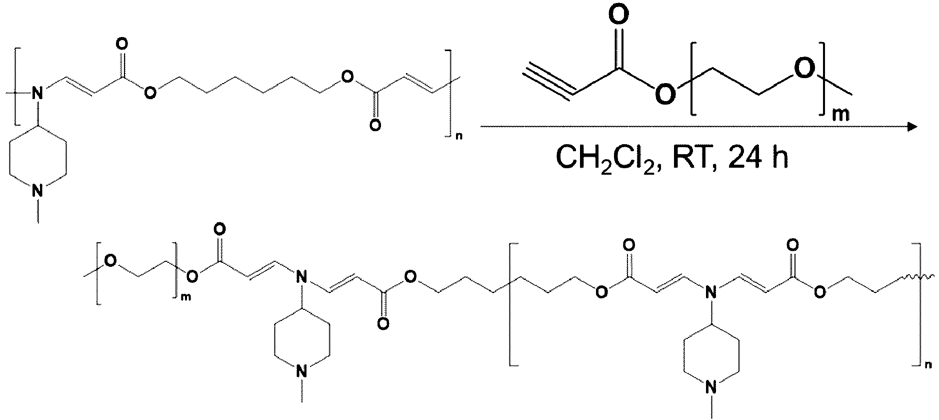

Polymer 4G prepared in the same manner as in Manufacturing Example 72 above and 383.0 mg of PEG-alkynoate were dissolved in 4 mL of CH₂Cl₂ and added to a flask. The reaction was carried out at room temperature for 24 hours, and then PEG-4G was obtained.

### [Manufacturing Example 183]

### Preparation of polymer PEG-4H

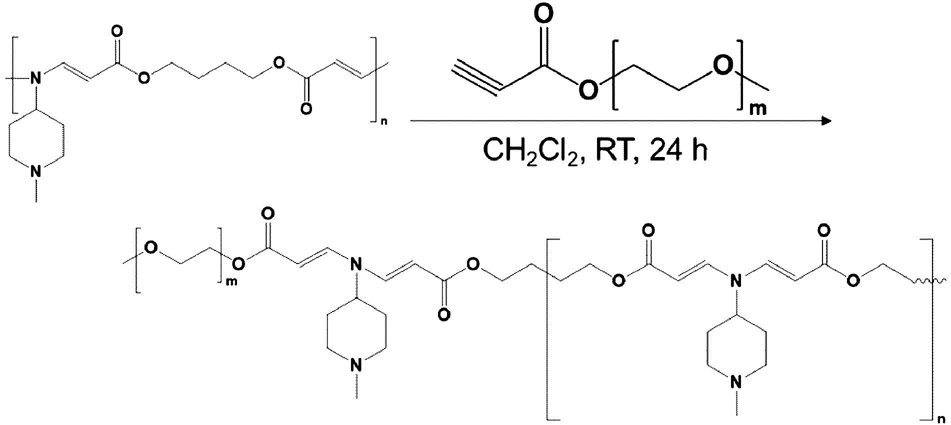

Polymer 4H prepared in the same manner as in Manufacturing Example 73 above and 438.3 mg of PEG-alkynoate were dissolved in 4 mL of CH₂Cl₂ and added to a flask. The reaction was carried out at room temperature for 24 hours, and then PEG-4H was obtained.

### [Manufacturing Example 184]

### Preparation of polymer PEG-4I

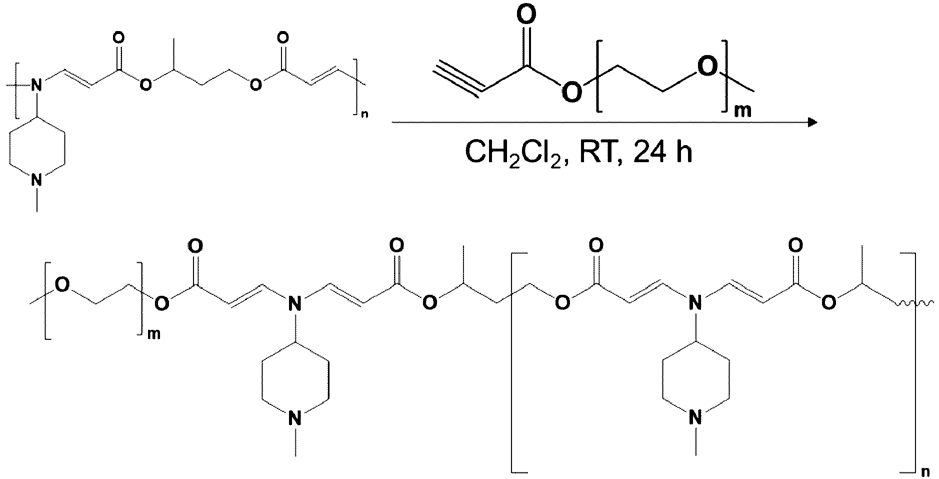

Polymer 4I prepared in the same manner as in Manufacturing Example 74 above and 438.3 mg of PEG-alkynoate were dissolved in 4 mL of CH₂Cl₂ and added to a flask. The reaction was carried out at room temperature for 24 hours, and then PEG-4I was obtained.

### [Manufacturing Example 185]

### Preparation of polymer PEG-4J

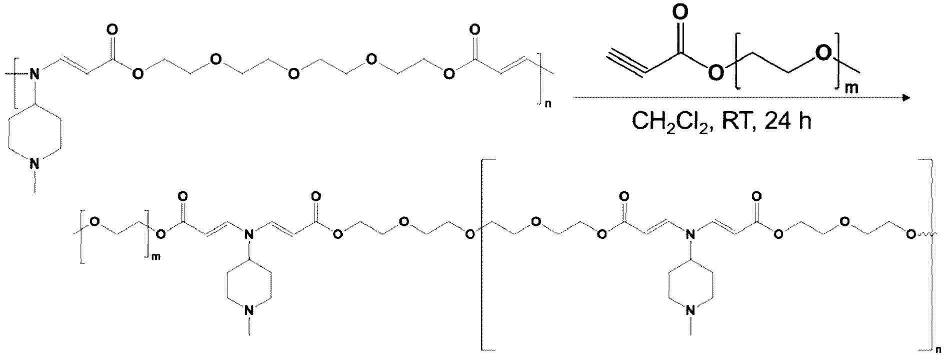

Polymer 4J prepared in the same manner as in Manufacturing Example 75 above and 285.3 mg of PEG-alkynoate were dissolved in 4 mL of CH₂Cl₂ and added to a flask. The reaction was carried out at room temperature for 24 hours, and then PEG-4J was obtained.

### [Manufacturing Example 186]

### Preparation of polymer PEG-5F

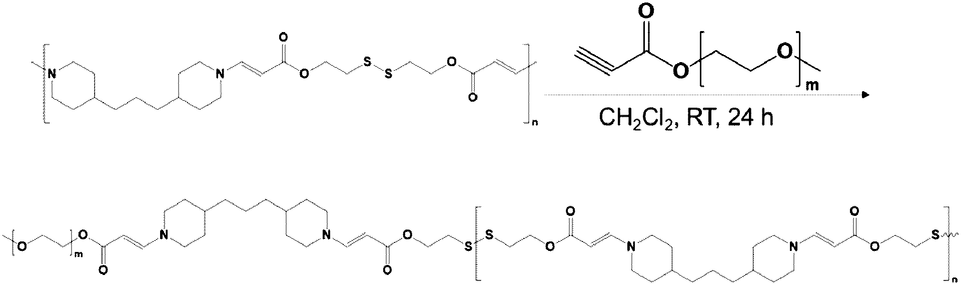

Polymer 5F prepared in the same manner as in Manufacturing Example 76 above and 329.5 mg of PEG-alkynoate were dissolved in 4 mL of CH₂Cl₂ and added to a flask. The reaction was carried out at room temperature for 24 hours, and then PEG-5F was obtained.

### [Manufacturing Example 187]

### Preparation of polymer PEG-5G

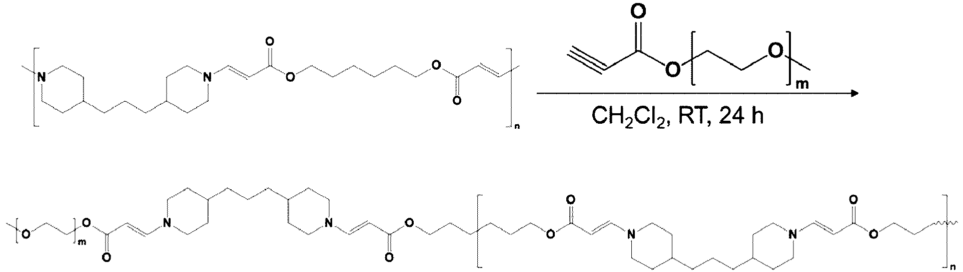

Polymer 5G prepared in the same manner as in Manufacturing Example 77 above and 383.0 mg of PEG-alkynoate were dissolved in 4 mL of CH₂Cl₂ and added to a flask. The reaction was carried out at room temperature for 24 hours, and then PEG-5G was obtained.

### [Manufacturing Example 188]

### Preparation of polymer PEG-5H

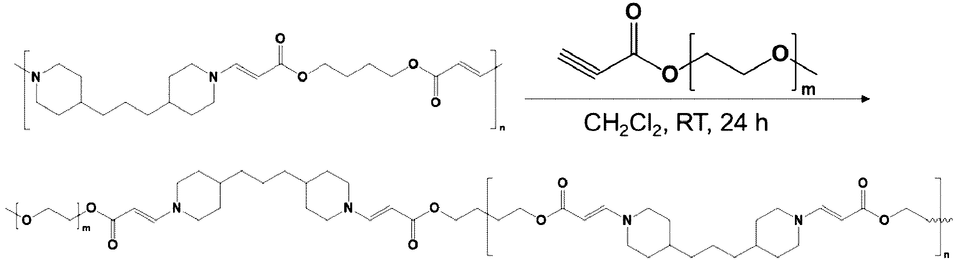

Polymer 5H prepared in the same manner as in Manufacturing Example 78 above and 438.3 mg of PEG-alkynoate were dissolved in 4 mL of CH₂Cl₂ and added to a flask. The reaction was carried out at room temperature for 24 hours, and then PEG-5H was obtained.

### [Manufacturing Example 189]

### Preparation of polymer PEG-51

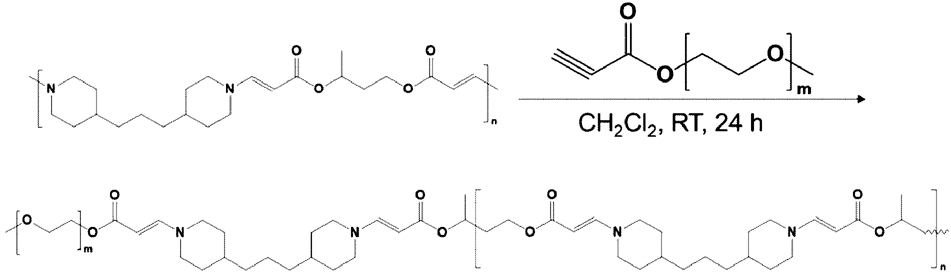

Polymer 5I prepared in the same manner as in Manufacturing Example 79 above and 438.3 mg of PEG-alkynoate were dissolved in 4 mL of CH₂Cl₂ and added to a flask. The reaction was carried out at room temperature for 24 hours, and then PEG-5I was obtained.

### [Manufacturing Example 190]

### Preparation of polymer PEG-5J

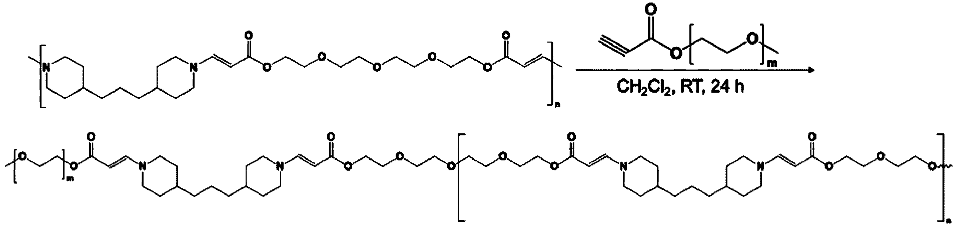

Polymer 5J prepared in the same manner as in Manufacturing Example 80 above and 285.3 mg of PEG-alkynoate were dissolved in 4 mL of CH₂Cl₂ and added to a flask. The reaction was carried out at room temperature for 24 hours, and then PEG-5J was obtained.

### [Manufacturing Example 191]

### Preparation of polymer PEG-6F

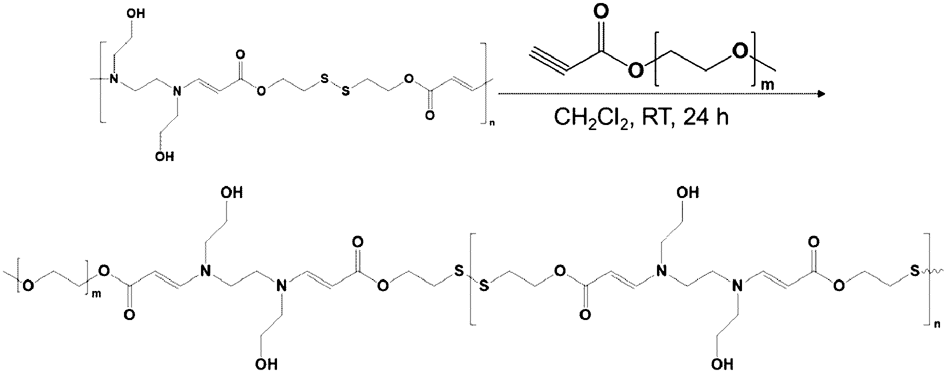

Polymer 6F prepared in the same manner as in Manufacturing Example 81 above and 329.5 mg of PEG-alkynoate were dissolved in 4 mL of CH₂Cl₂ and added to a flask. The reaction was carried out at room temperature for 24 hours, and then PEG-6F was obtained.

### [Manufacturing Example 192]

### Preparation of polymer PEG-6G

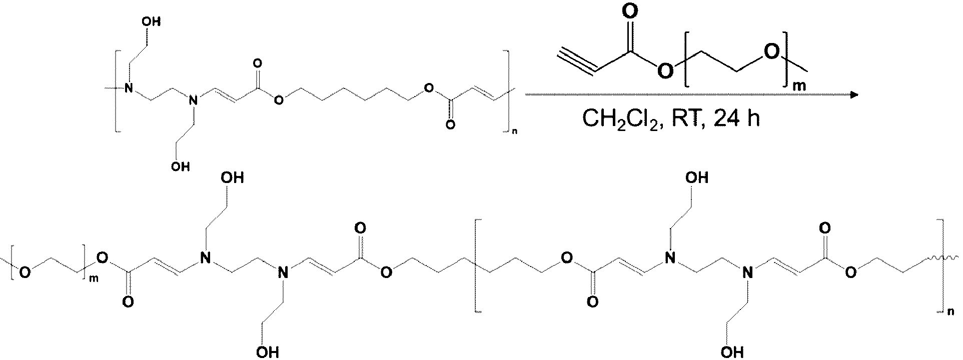

Polymer 6G prepared in the same manner as in Manufacturing Example 82 above and 383.0 mg of PEG-alkynoate were dissolved in 4 mL of CH₂Cl₂ and added to a flask. The reaction was carried out at room temperature for 24 hours, and then PEG-6G was obtained.

### [Manufacturing Example 193]

### Preparation of polymer PEG-6H

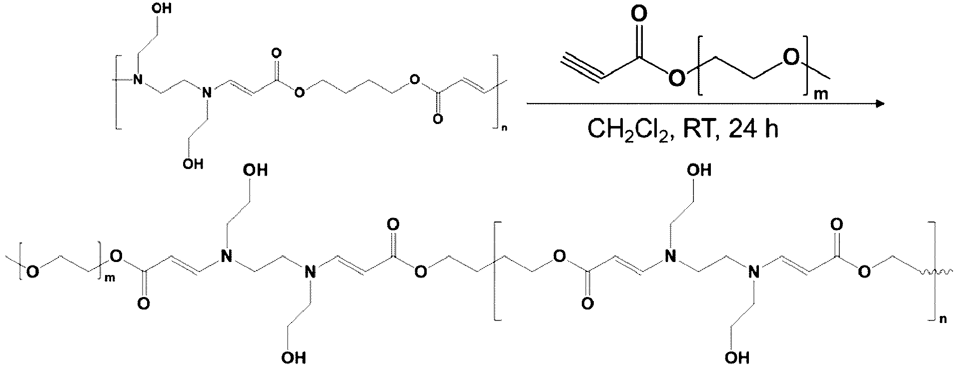

Polymer 6H prepared in the same manner as in Manufacturing Example 83 above and 438.3 mg of PEG-alkynoate were dissolved in 4 mL of CH₂Cl₂ and added to a flask. The reaction was carried out at room temperature for 24 hours, and then PEG-6H was obtained.

### [Manufacturing Example 194]

### Preparation of polymer PEG-6I

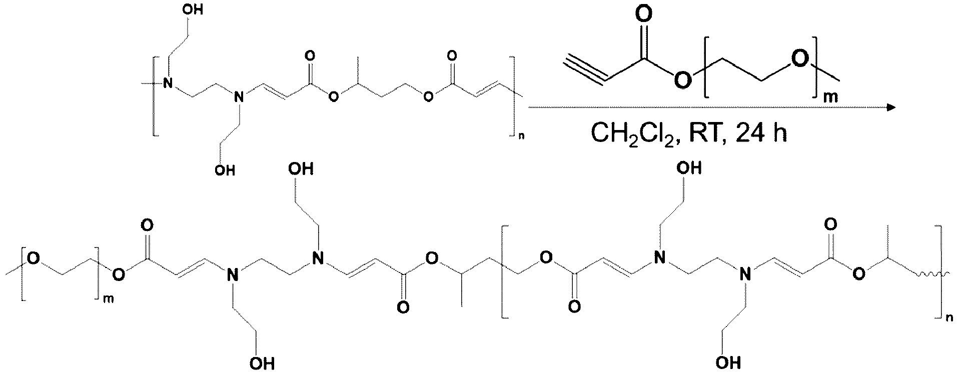

Polymer 6I prepared in the same manner as in Manufacturing Example 84 above and 438.3 mg of PEG-alkynoate were dissolved in 4 mL of CH₂Cl₂ and added to a flask. The reaction was carried out at room temperature for 24 hours, and then PEG-6I was obtained.

### [Manufacturing Example 195]

### Preparation of polymer PEG-6J

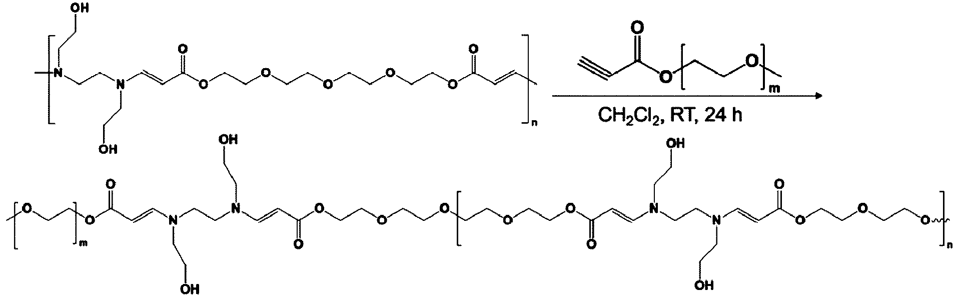

Polymer 6J prepared in the same manner as in Manufacturing Example 85 above and 285.3 mg of PEG-alkynoate were dissolved in 4 mL of CH₂Cl₂ and added to a flask. The reaction was carried out at room temperature for 24 hours, and then PEG-6J was obtained.

### [Manufacturing Example 196]

### Preparation of polymer PEG-7F

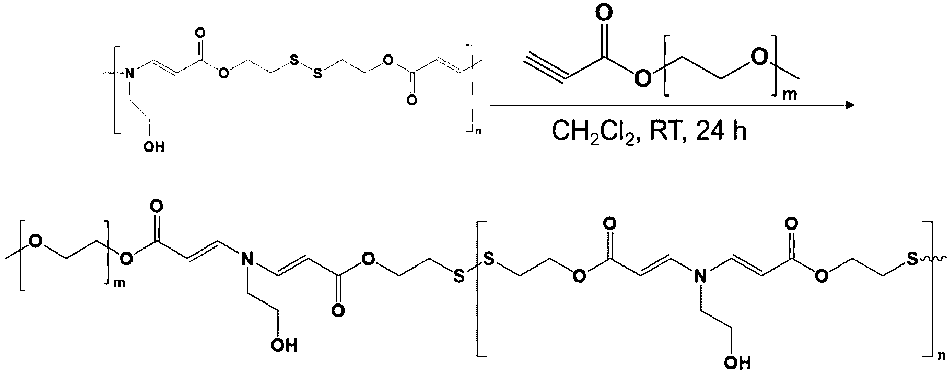

Polymer 7F prepared in the same manner as in Manufacturing Example 86 above and 329.5 mg of PEG-alkynoate were dissolved in 4 mL of CH₂Cl₂ and added to a flask. The reaction was carried out at room temperature for 24 hours, and then PEG-7F was obtained.

### [Manufacturing Example 197]

### Preparation of polymer PEG-7G

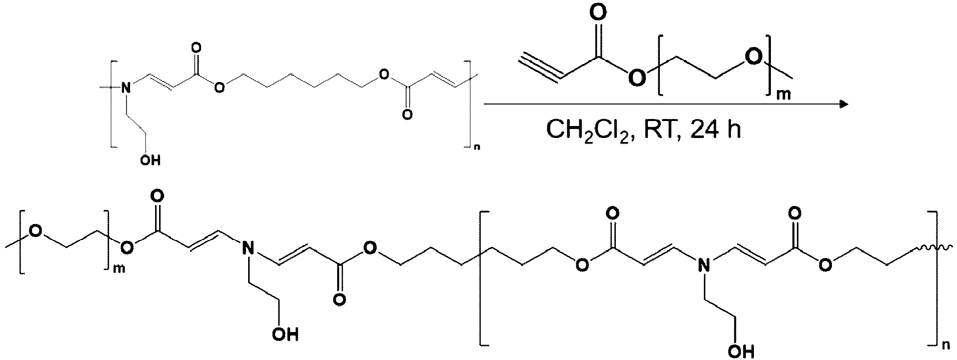

Polymer 7G prepared in the same manner as in Manufacturing Example 87 above and 383.0 mg of PEG-alkynoate were dissolved in 4 mL of CH₂Cl₂ and added to a flask. The reaction was carried out at room temperature for 24 hours, and then PEG-7G was obtained.

### [Manufacturing Example 198]

### Preparation of polymer PEG-7H

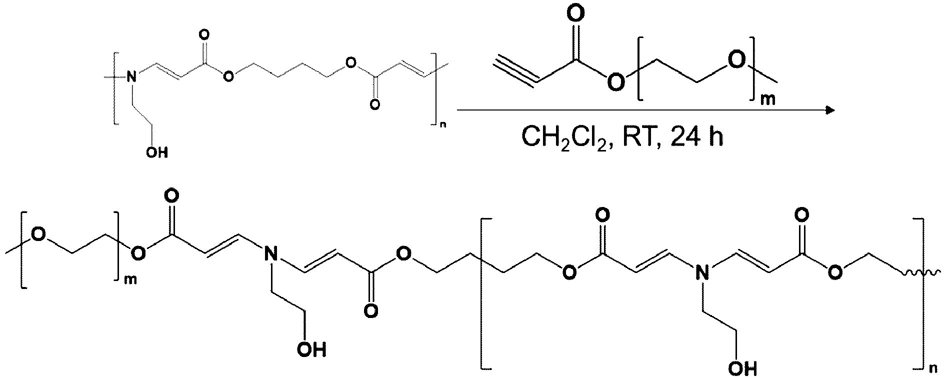

Polymer 7H prepared in the same manner as in Manufacturing Example 88 above and 438.3 mg of PEG-alkynoate were dissolved in 4 mL of CH₂Cl₂ and added to a flask. The reaction was carried out at room temperature for 24 hours, and then PEG-7H was obtained.

### [Manufacturing Example 199]

### Preparation of polymer PEG-71

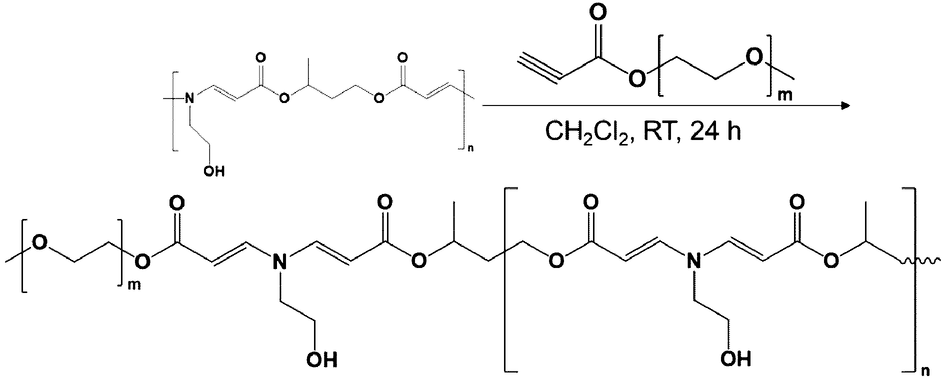

Polymer 7I prepared in the same manner as in Manufacturing Example 89 above and 438.3 mg of PEG-alkynoate were dissolved in 4 mL of CH₂Cl₂ and added to a flask. The reaction was carried out at room temperature for 24 hours, and then PEG-7I was obtained.

### [Manufacturing Example 200]

### Preparation of polymer PEG-7J

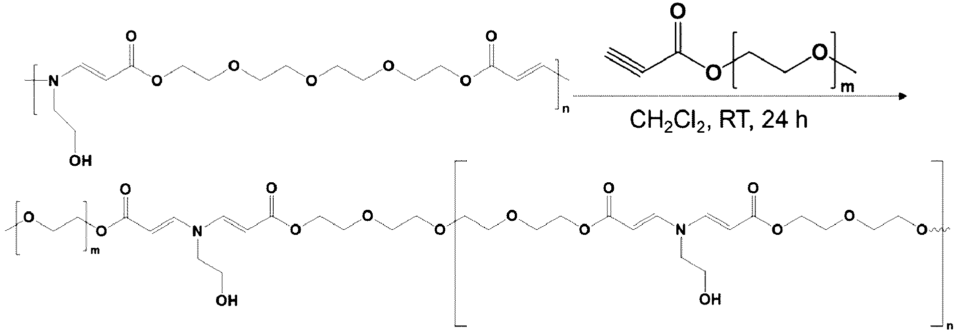

Polymer 7J prepared in the same manner as in Manufacturing Example 90 above and 285.3 mg of PEG-alkynoate were dissolved in 4 mL of CH₂Cl₂ and added to a flask. The reaction was carried out at room temperature for 24 hours, and then PEG-7J was obtained.

### [Manufacturing Example 201]

### Preparation of polymer PEG-8F

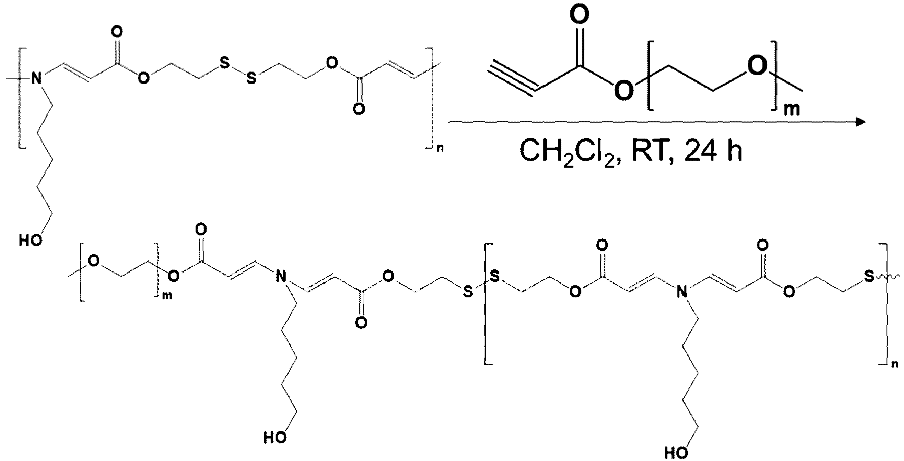

Polymer 8F prepared in the same manner as in Manufacturing Example 91 above and 329.5 mg of PEG-alkynoate were dissolved in 4 mL of CH₂Cl₂ and added to a flask. The reaction was carried out at room temperature for 24 hours, and then PEG-8F was obtained.

### [Manufacturing Example 202]

### Preparation of polymer PEG-8G

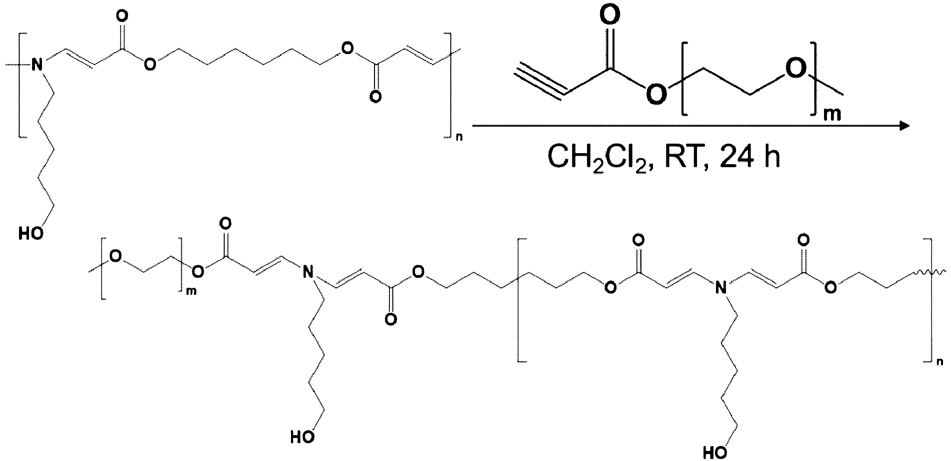

Polymer 8G prepared in the same manner as in Manufacturing Example 92 above and 383.0 mg of PEG-alkynoate were dissolved in 4 mL of CH₂Cl₂ and added to a flask. The reaction was carried out at room temperature for 24 hours, and then PEG-8G was obtained.

### [Manufacturing Example 203]

### Preparation of polymer PEG-8H

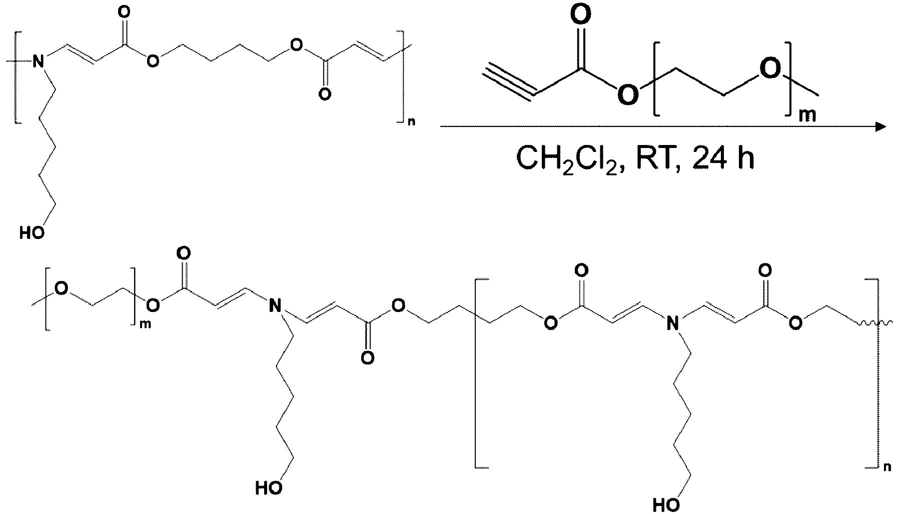

Polymer 8H prepared in the same manner as in Manufacturing Example 93 above and 438.3 mg of PEG-alkynoate were dissolved in 4 mL of CH₂Cl₂ and added to a flask. The reaction was carried out at room temperature for 24 hours, and then PEG-8H was obtained.

### [Manufacturing Example 204]

### Preparation of polymer PEG-8I

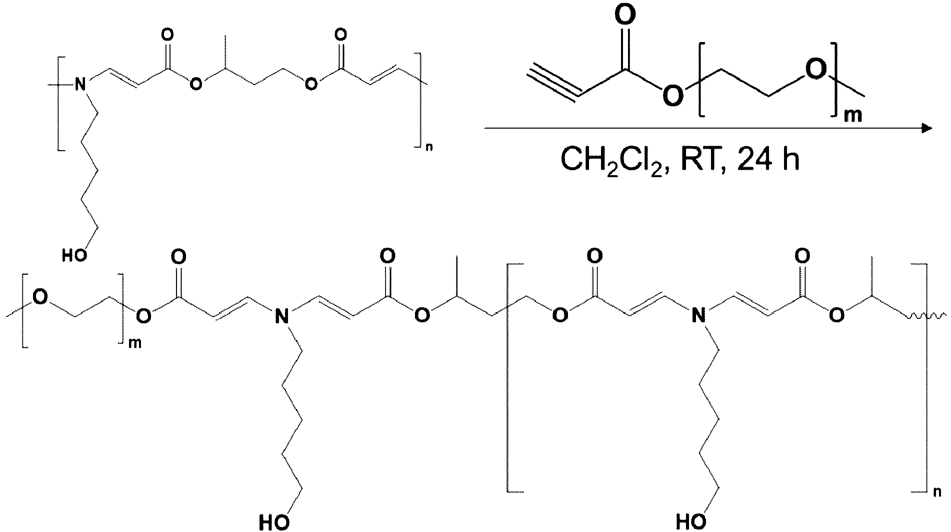

Polymer 8I prepared in the same manner as in Manufacturing Example 94 above and 438.3 mg of PEG-alkynoate were dissolved in 4 mL of CH₂Cl₂ and added to a flask. The reaction was carried out at room temperature for 24 hours, and then PEG-8I was obtained.

### [Manufacturing Example 205]

### Preparation of polymer PEG-8J

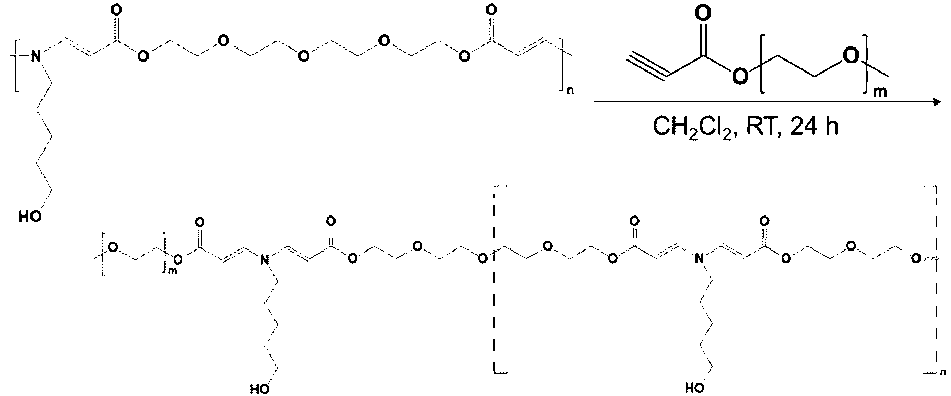

Polymer 8J prepared in the same manner as in Manufacturing Example 95 above and 285.3 mg of PEG-alkynoate were dissolved in 4 mL of CH₂Cl₂ and added to a flask. The reaction was carried out at room temperature for 24 hours, and then PEG-8J was obtained.

### [Manufacturing Example 206]

### Preparation of polymer PEG-9F

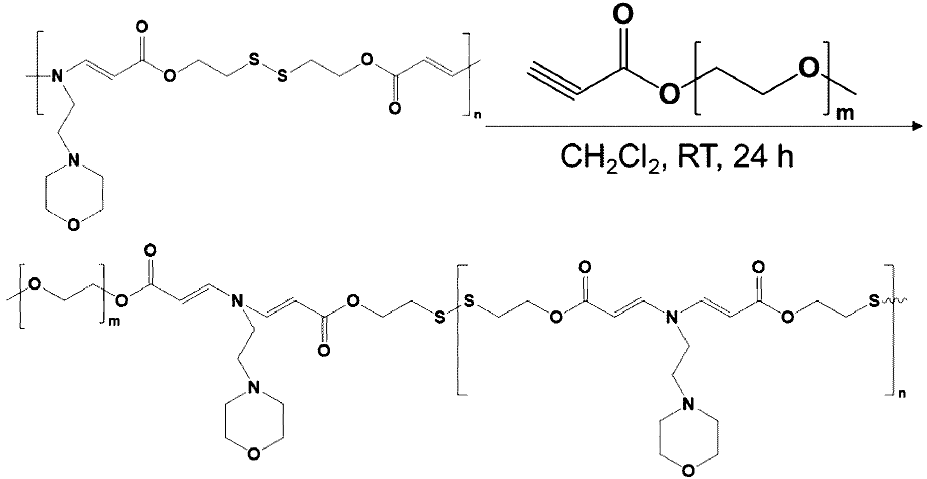

Polymer 9F prepared in the same manner as in Manufacturing Example 96 above and 329.5 mg of PEG-alkynoate were dissolved in 4 mL of CH₂Cl₂ and added to a flask. The reaction was carried out at room temperature for 24 hours, and then PEG-9F was obtained.

### [Manufacturing Example 207]

### Preparation of polymer PEG-9G

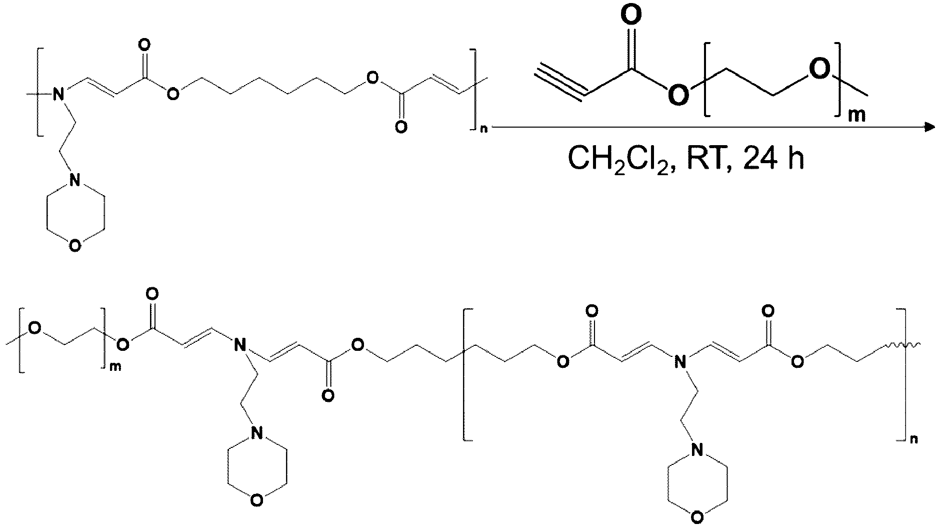

Polymer 9G prepared in the same manner as in Manufacturing Example 97 above and 383.0 mg of PEG-alkynoate were dissolved in 4 mL of CH₂Cl₂ and added to a flask. The reaction was carried out at room temperature for 24 hours, and then PEG-9G was obtained.

### [Manufacturing Example 208]

### Preparation of polymer PEG-9H

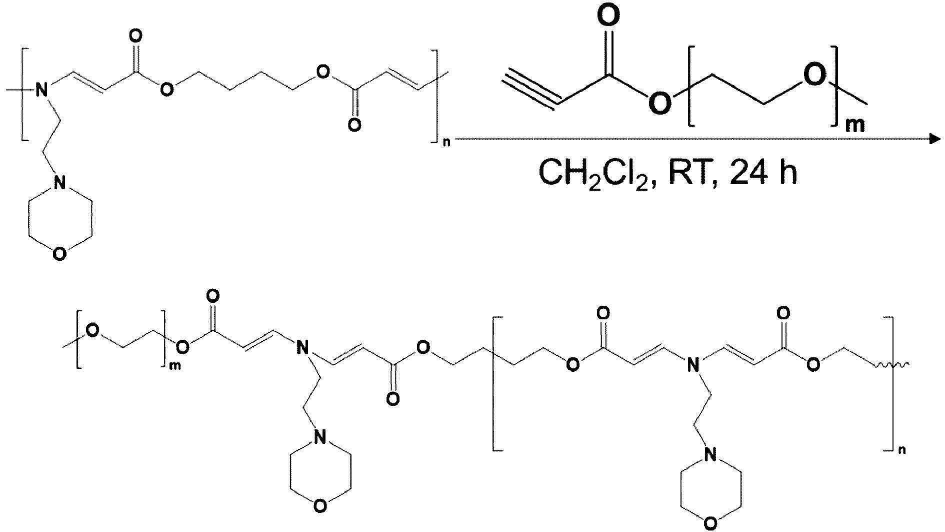

Polymer 9H prepared in the same manner as in Manufacturing Example 98 above and 438.3 mg of PEG-alkynoate were dissolved in 4 mL of CH₂Cl₂ and added to a flask. The reaction was carried out at room temperature for 24 hours, and then PEG-9H was obtained.

### [Manufacturing Example 209]

### Preparation of polymer PEG-9I

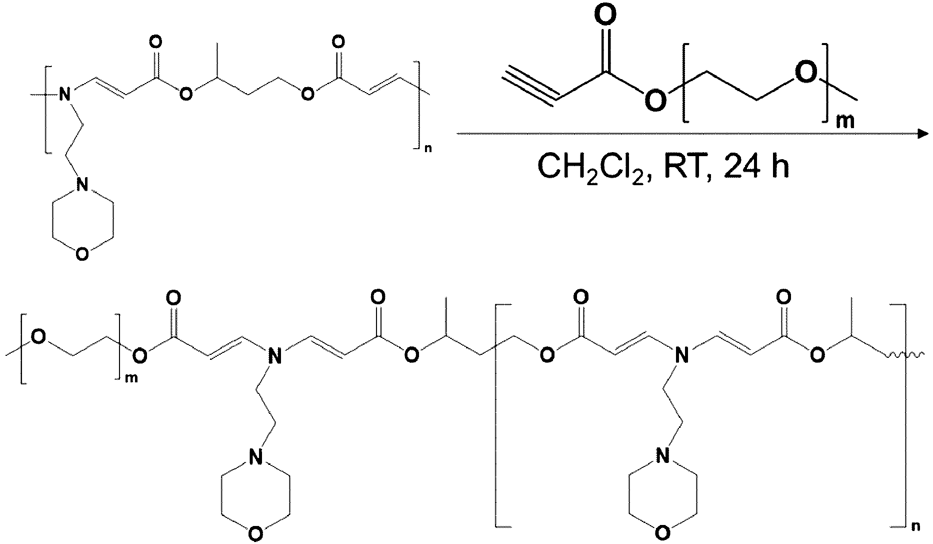

Polymer 91 prepared in the same manner as in Manufacturing Example 99 above and 438.3 mg of PEG-alkynoate were dissolved in 4 mL of CH₂Cl₂ and added to a flask. The reaction was carried out at room temperature for 24 hours, and then PEG-9I was obtained.

### [Manufacturing Example 210]

### Preparation of polymer PEG-9J

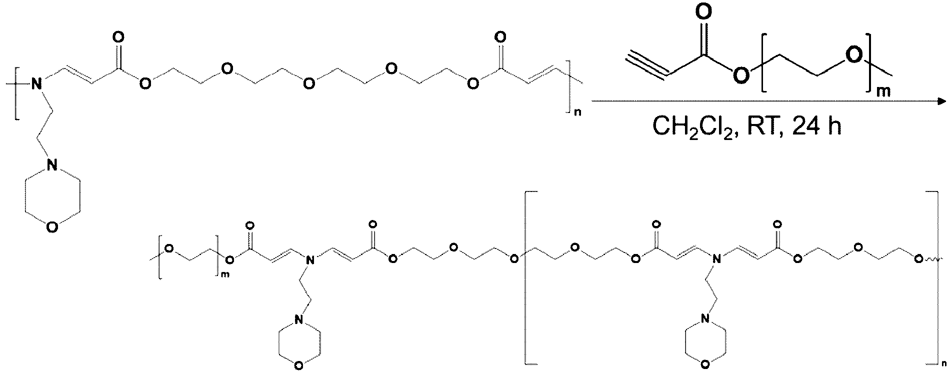

Polymer 9J prepared in the same manner as in Manufacturing Example 100 above and 285.3 mg of PEG-alkynoate were dissolved in 4 mL of CH₂Cl₂ and added to a flask. The reaction was carried out at room temperature for 24 hours, and then PEG-9J was obtained.

### [Manufacturing Example 211]

### Preparation of polymer PEG-10F

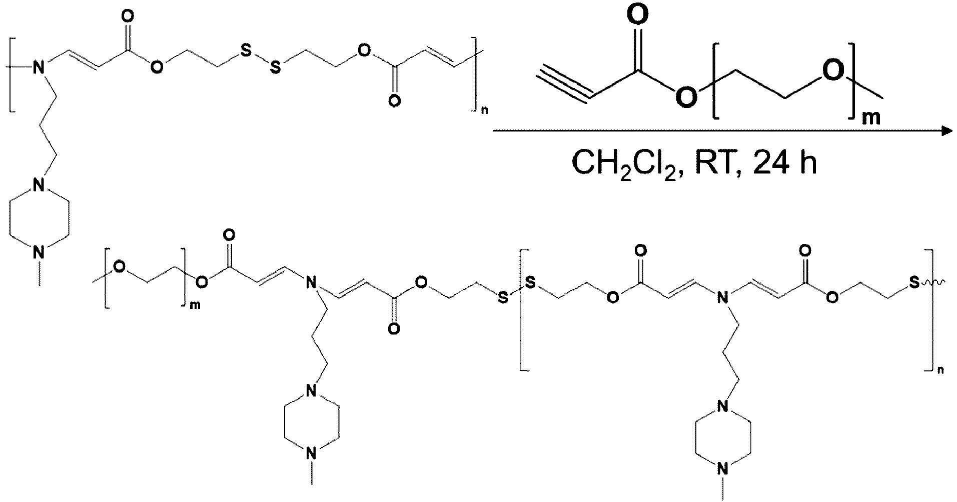

Polymer 10F prepared in the same manner as in Manufacturing Example 101 above and 329.5 mg of PEG-alkynoate were dissolved in 4 mL of CH₂Cl₂ and added to a flask. The reaction was carried out at room temperature for 24 hours, and then PEG-10F was obtained.

### [Manufacturing Example 212]

### Preparation of polymer PEG-10G

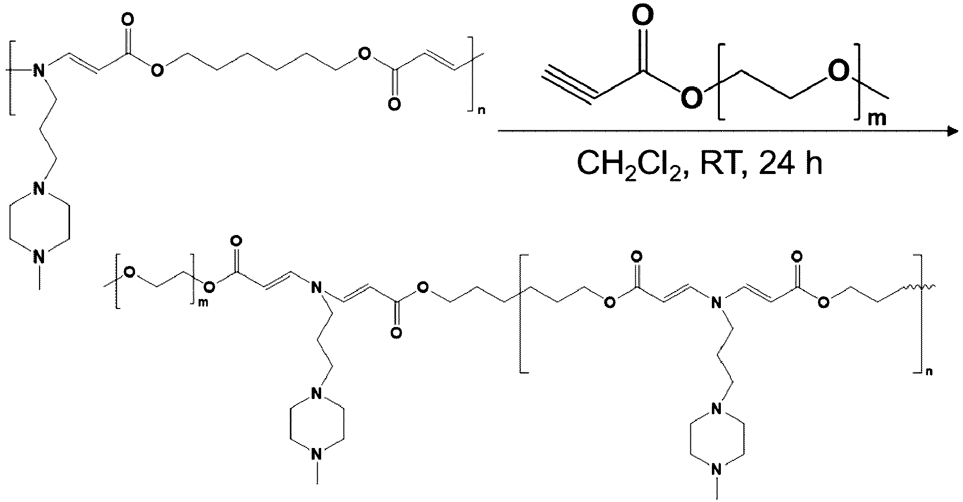

Polymer 10G prepared in the same manner as in Manufacturing Example 102 above and 383.0 mg of PEG-alkynoate were dissolved in 4 mL of CH₂Cl₂ and added to a flask. The reaction was carried out at room temperature for 24 hours, and then PEG-10G was obtained.

### [Manufacturing Example 213]

### Preparation of polymer PEG-10H

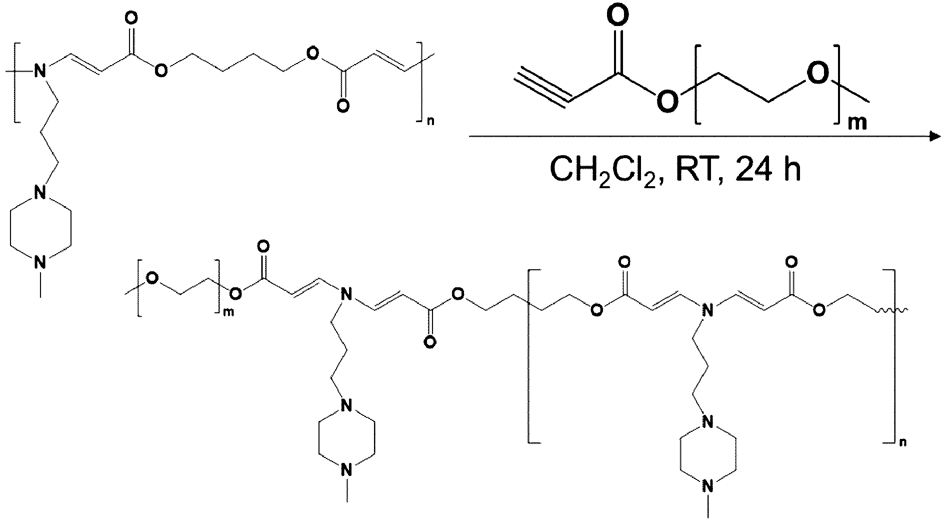

Polymer 10H prepared in the same manner as in Manufacturing Example 103 above and 438.3 mg of PEG-alkynoate were dissolved in 4 mL of CH₂Cl₂ and added to a flask. The reaction was carried out at room temperature for 24 hours, and then PEG-10H was obtained.

### [Manufacturing Example 214]

### Preparation of polymer PEG-101

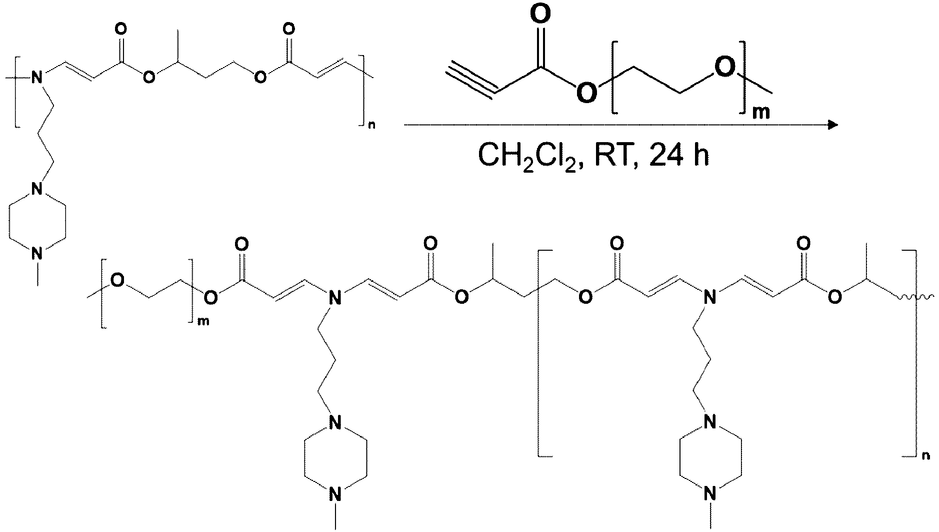

Polymer 101 prepared in the same manner as in Manufacturing Example 104 above and 438.3 mg of PEG-alkynoate were dissolved in 4 mL of CH₂Cl₂ and added to a flask. The reaction was carried out at room temperature for 24 hours, and then PEG-101 was obtained.

### [Manufacturing Example 215]

### Preparation of polymer PEG-10J

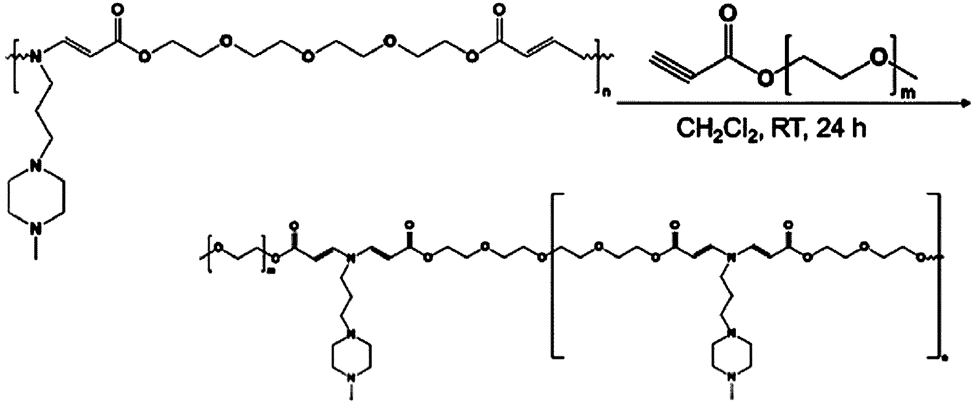

Polymer 10J prepared in the same manner as in Manufacturing Example 105 above and 285.3 mg of PEG-alkynoate were dissolved in 4 mL of CH₂Cl₂ and added to a flask. The reaction was carried out at room temperature for 24 hours, and then PEG-10J was obtained.

### [Manufacturing Example 216]

### Preparation of polymer PEG-11F

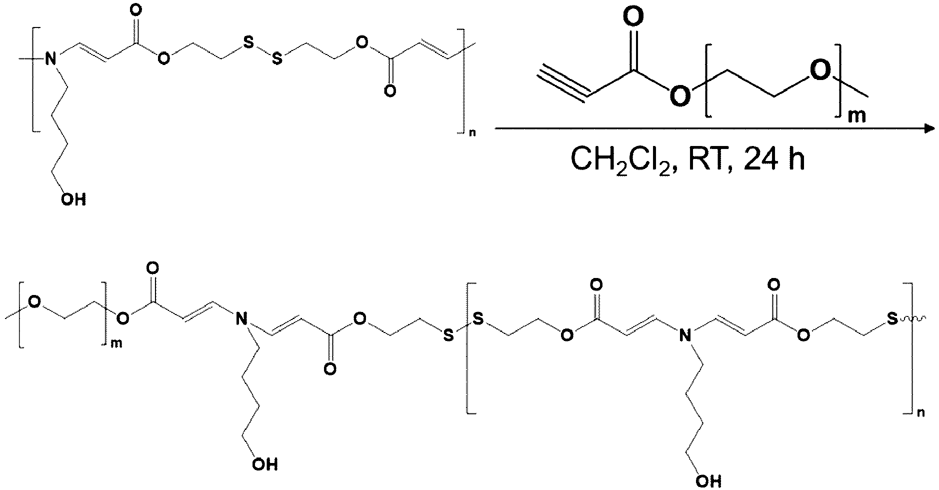

Polymer 11F prepared in the same manner as in Manufacturing Example 106 above and 329.5 mg of PEG-alkynoate were dissolved in 4 mL of CH₂Cl₂ and added to a flask. The reaction was carried out at room temperature for 24 hours, and then PEG-11F was obtained.

### [Manufacturing Example 217]

### Preparation of polymer PEG-11G

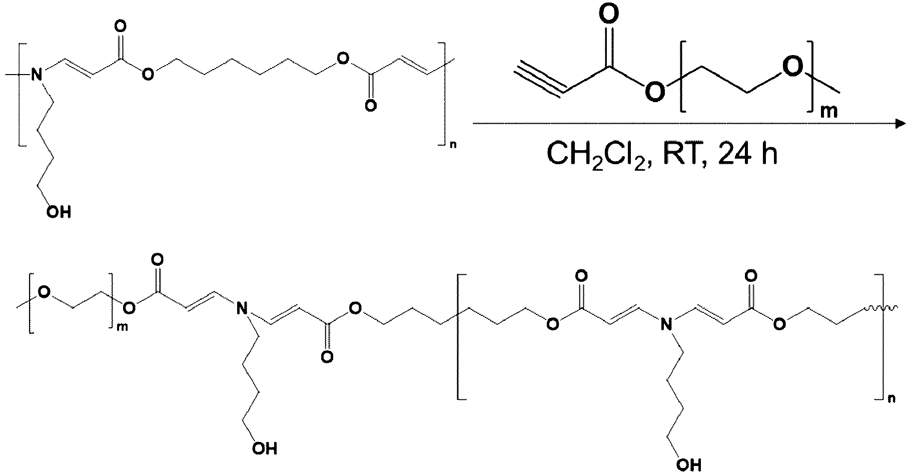

Polymer 11G prepared in the same manner as in Manufacturing Example 107 above and 383.0 mg of PEG-alkynoate were dissolved in 4 mL of CH₂Cl₂ and added to a flask. The reaction was carried out at room temperature for 24 hours, and then PEG-11G was obtained.

### [Manufacturing Example 218]

### Preparation of polymer PEG-11H

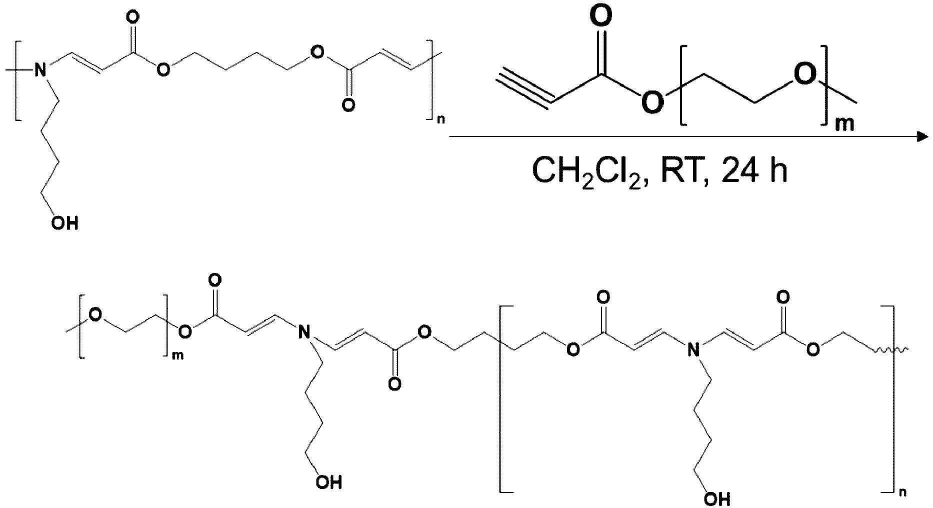

Polymer 11H prepared in the same manner as in Manufacturing Example 108 above and 438.3 mg of PEG-alkynoate were dissolved in 4 mL of CH₂Cl₂ and added to a flask. The reaction was carried out at room temperature for 24 hours, and then PEG-11H was obtained.

### [Manufacturing Example 219]

### Preparation of polymer PEG-11I

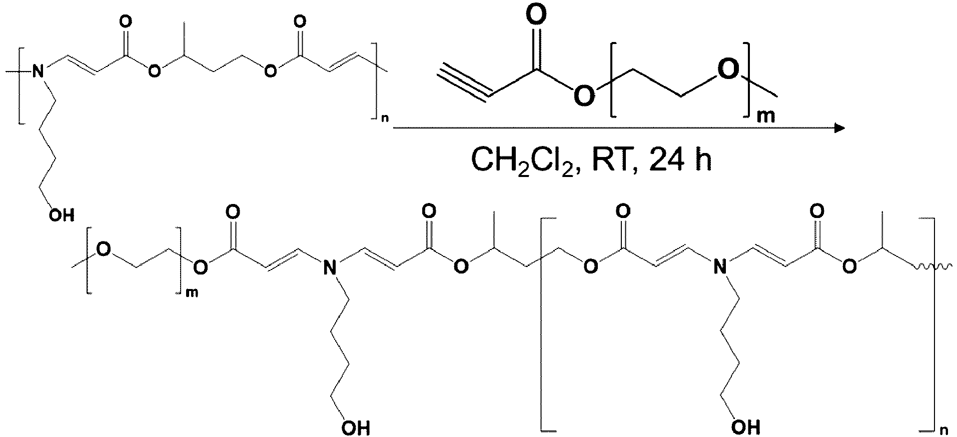

Polymer 11I prepared in the same manner as in Manufacturing Example 109 above and 438.3 mg of PEG-alkynoate were dissolved in 4 mL of CH₂Cl₂ and added to a flask. The reaction was carried out at room temperature for 24 hours, and then PEG-11I was obtained.

### [Manufacturing Example 220]

### Preparation of polymer PEG-11J

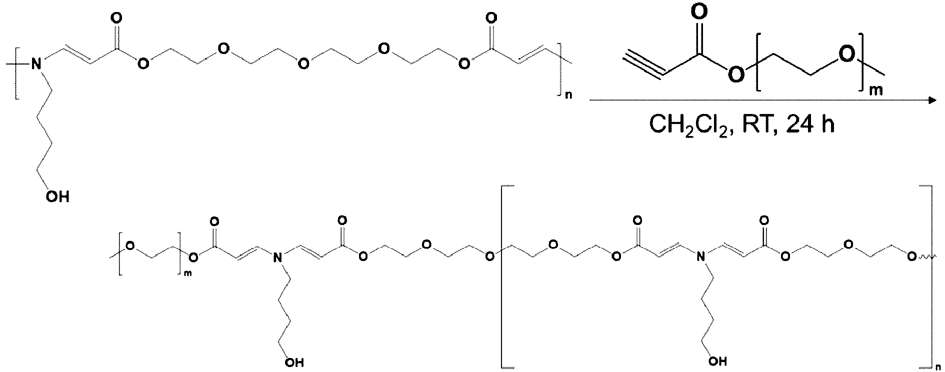

Polymer 11J prepared in the same manner as in Manufacturing Example 110 above and 285.3 mg of PEG-alkynoate were dissolved in 4 mL of CH₂Cl₂ and added to a flask. The reaction was carried out at room temperature for 24 hours, and then PEG-11J was obtained.

### [Manufacturing Example 221]

### Preparation of polymer b3A

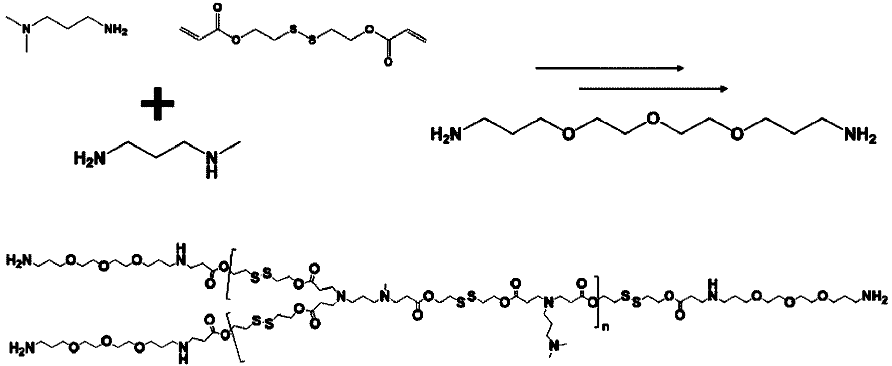

Amine monomer 3, acrylate monomer A, and branched dendrimer were added to a round bottom flask in a molar ratio of 0.4:1:0.2. Thereafter, dimethylformamide (N,N-dimethylformamide, DMF) was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b3A was obtained 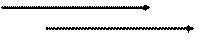 used in the reaction scheme in the present specification is a notation that is well known to those of ordinary skill in the art, indicating that a multi-step reaction occurs and that intermediates are omitted.

### [Manufacturing Example 222]

### Preparation of polymer b3B

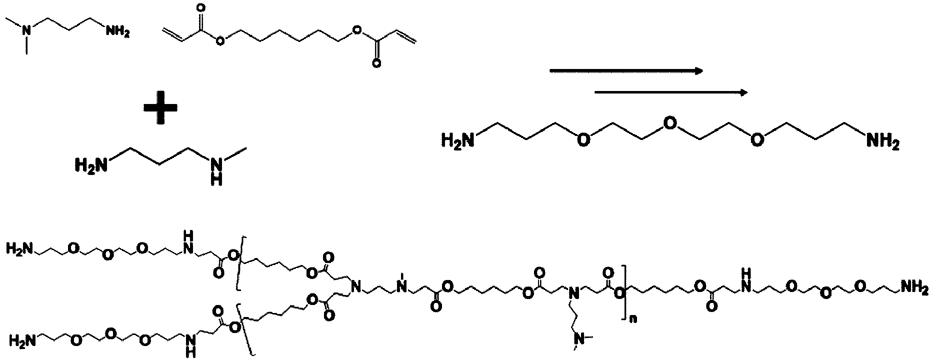

Amine monomer 3, acrylate monomer B, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b3B was obtained.

### [Manufacturing Example 223]

### Preparation of polymer b3C

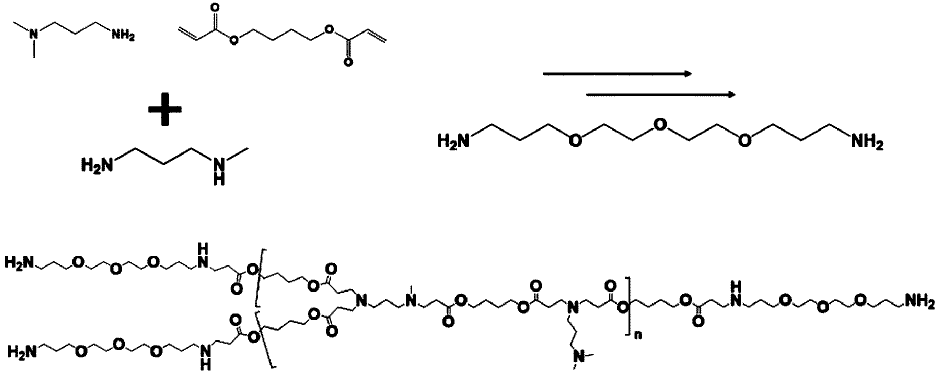

Amine monomer 3, acrylate monomer C, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b3C was obtained.

### [Manufacturing Example 224]

### Preparation of polymer b3D

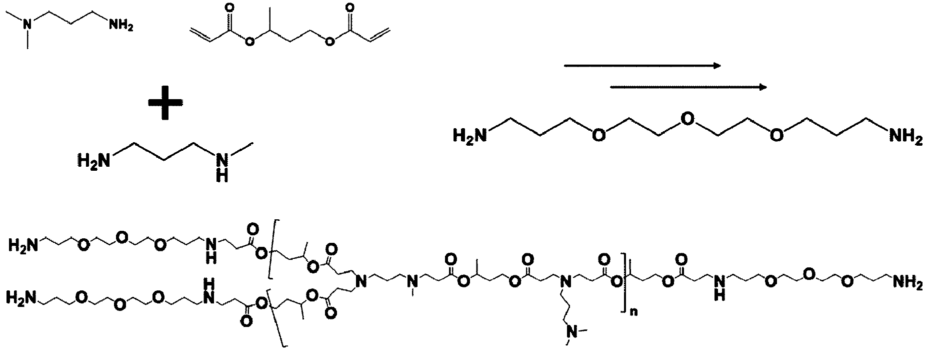

Amine monomer 3, acrylate monomer D, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b3D was obtained.

### [Manufacturing Example 225]

### Preparation of polymer b3E

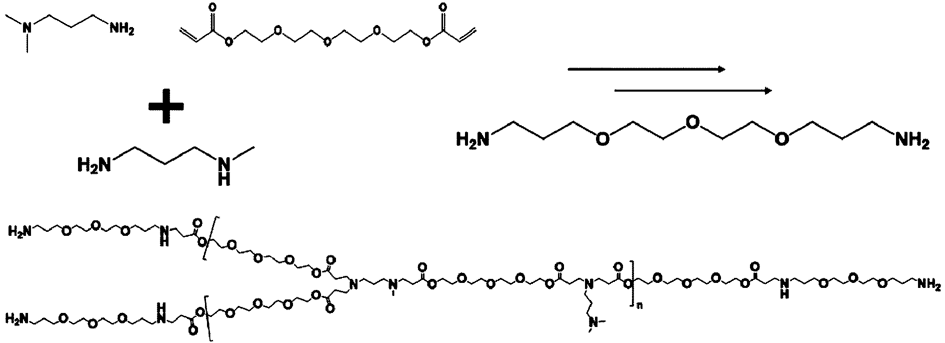

Amine monomer 3, acrylate monomer E, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b3E was obtained.

### [Manufacturing Example 226]

### Preparation of polymer b4A

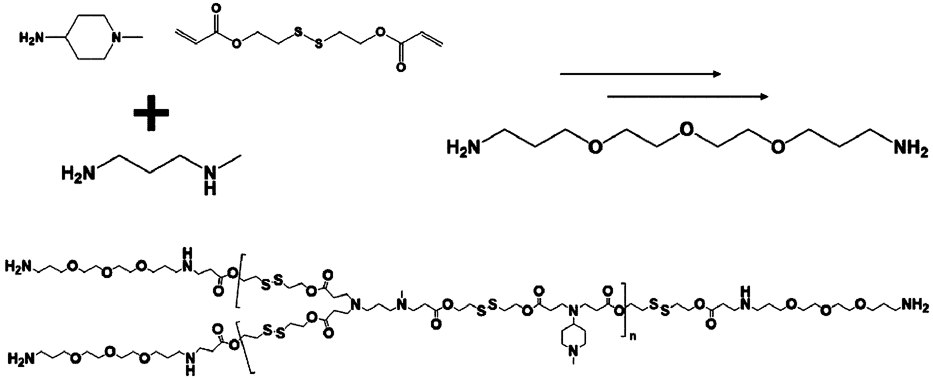

Amine monomer 4, acrylate monomer A, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b4A was obtained.

### [Manufacturing Example 227]

### Preparation of polymer b4B

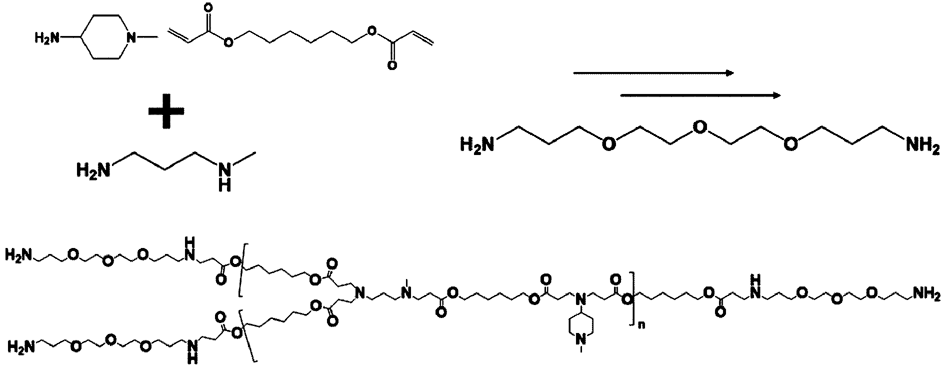

Amine monomer 4, acrylate monomer B, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b4B was obtained.

### [Manufacturing Example 228]

### Preparation of polymer b4C

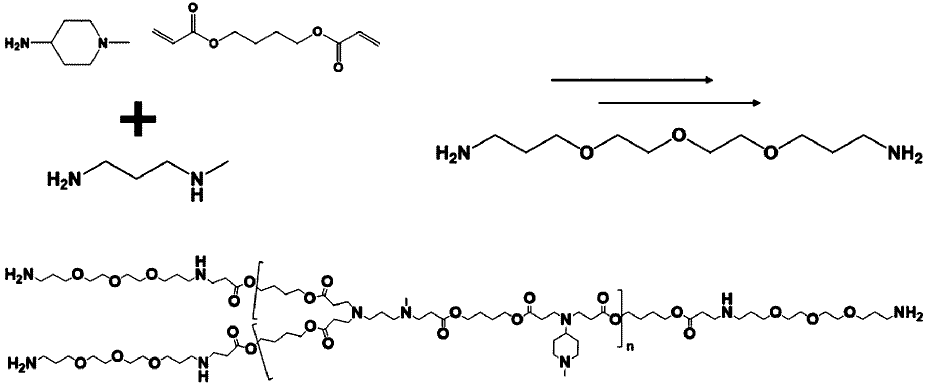

Amine monomer 4, acrylate monomer C, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b4C was obtained.

### [Manufacturing Example 229]

### Preparation of polymer b4D

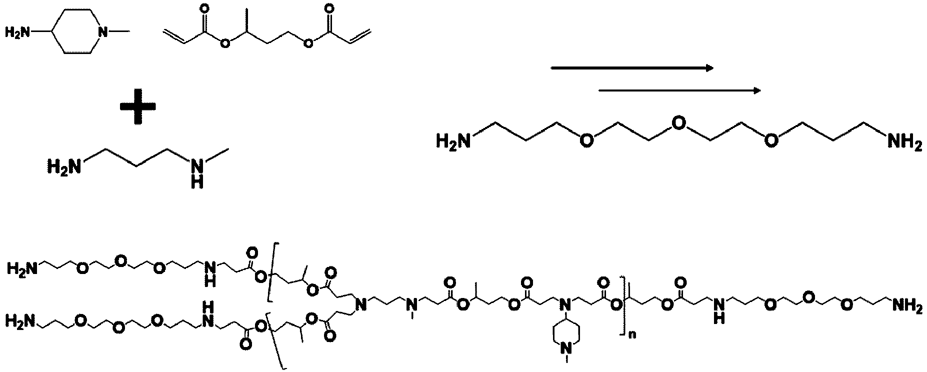

Amine monomer 4, acrylate monomer D, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b4D was obtained.

### [Manufacturing Example 230]

### Preparation of polymer b4E

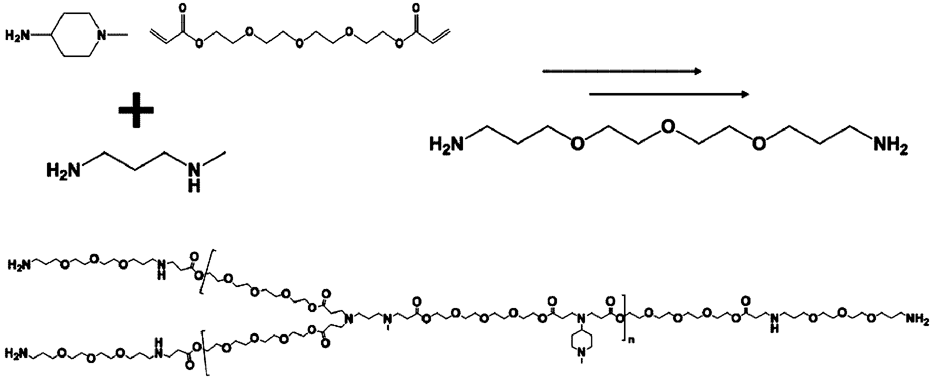

Amine monomer 4, acrylate monomer E, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b4E was obtained.

### [Manufacturing Example 231]

### Preparation of polymer b7A

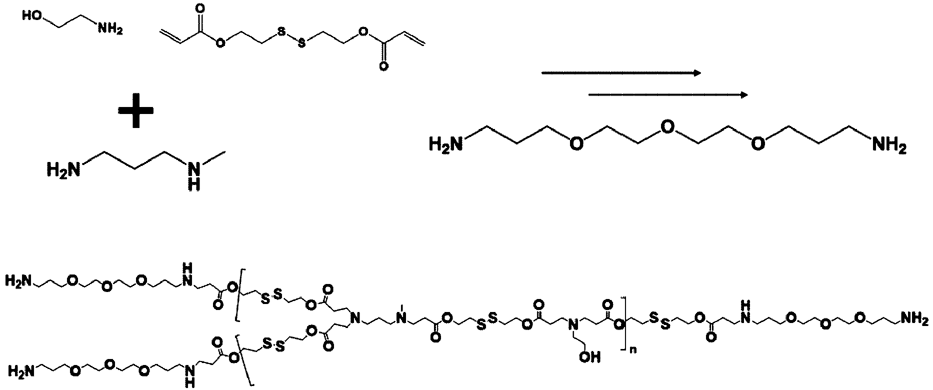

Amine monomer 7, acrylate monomer A, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b7A was obtained.

### [Manufacturing Example 232]

### Preparation of polymer b7B

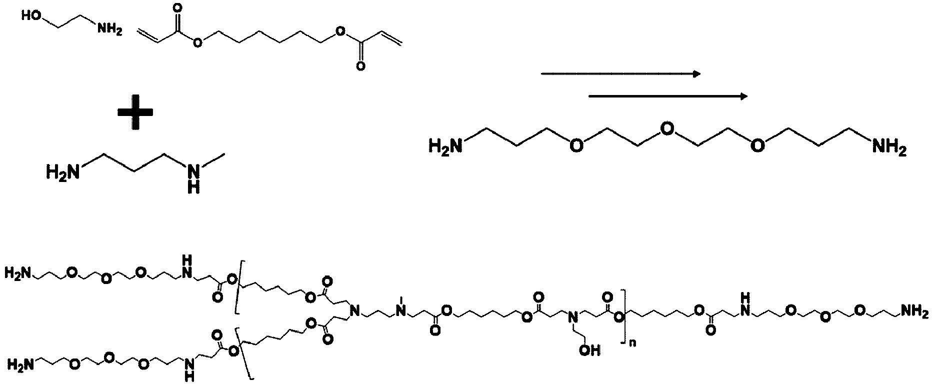

Amine monomer 7, acrylate monomer B, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b7B was obtained.

### [Manufacturing Example 233]

### Preparation of polymer b7C

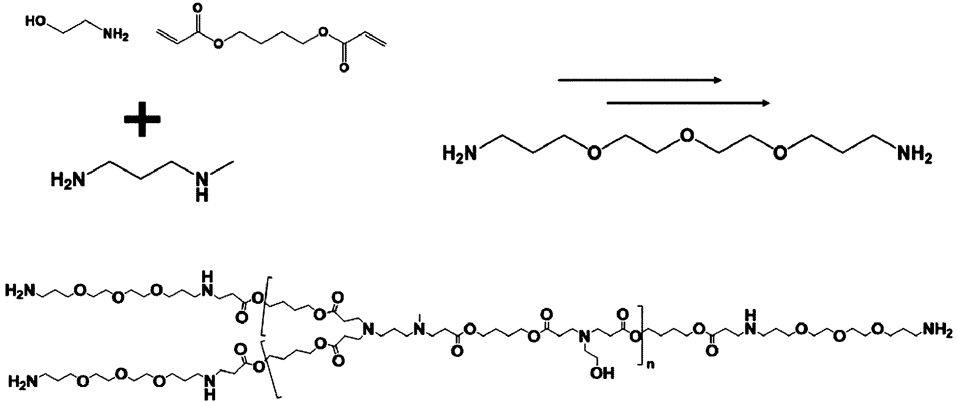

Amine monomer 7, acrylate monomer C, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b7C was obtained.

### [Manufacturing Example 234]

### Preparation of polymer b7D

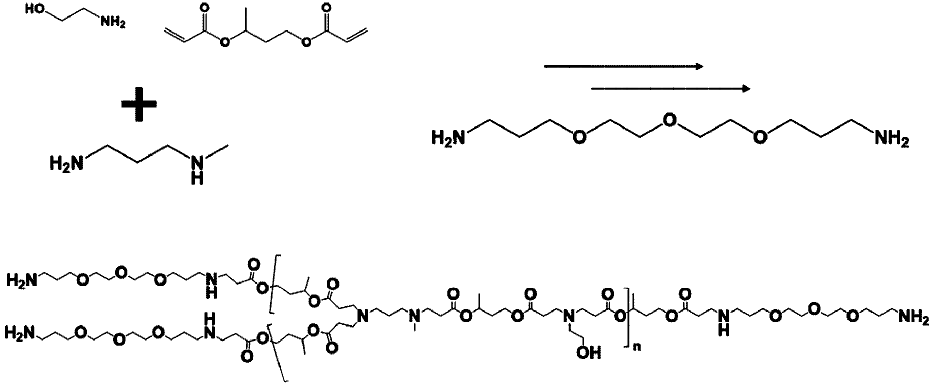

Amine monomer 7, acrylate monomer D, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b7D was obtained.

### [Manufacturing Example 235]

### Preparation of polymer b7E

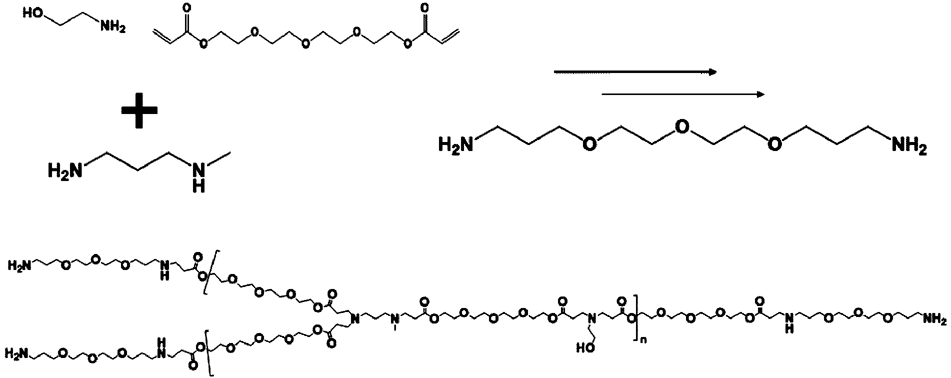

Amine monomer 7, acrylate monomer E, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b7E was obtained.

### [Manufacturing Example 236]

### Preparation of polymer b8A

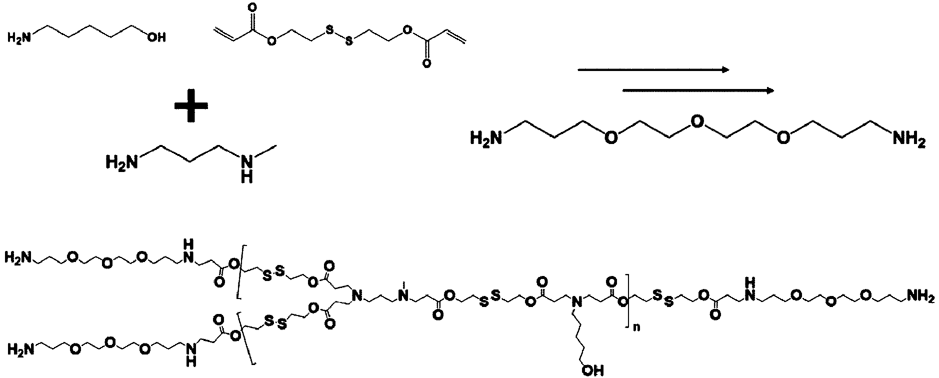

Amine monomer 8, acrylate monomer A, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b8A was obtained.

### [Manufacturing Example 237]

### Preparation of polymer b8B

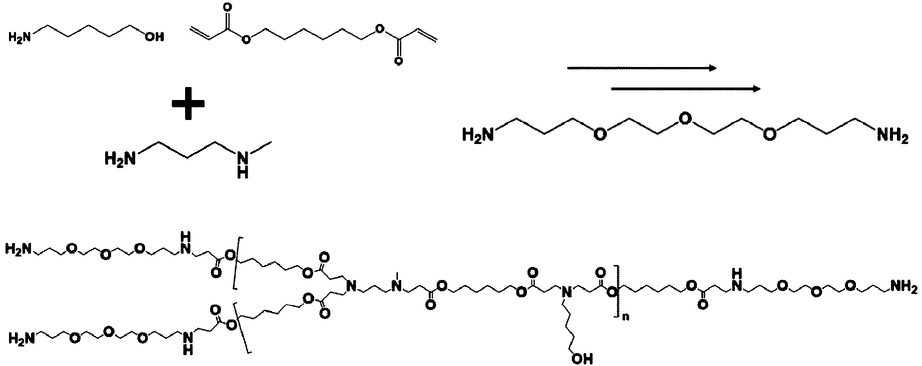

Amine monomer 8, acrylate monomer B, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b8B was obtained.

### [Manufacturing Example 238]

### Preparation of polymer b8C

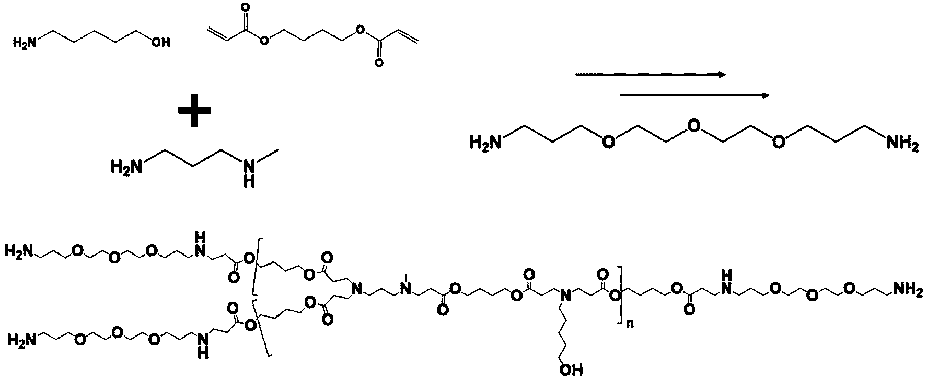

Amine monomer 8, acrylate monomer C, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b8C was obtained.

### [Manufacturing Example 239]

### Preparation of polymer b8D

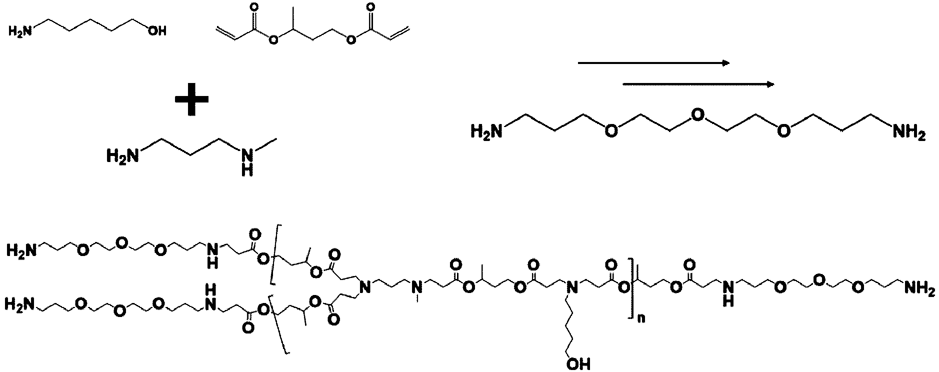

Amine monomer 8, acrylate monomer D, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b8D was obtained.

### [Manufacturing Example 240]

### Preparation of polymer b8E

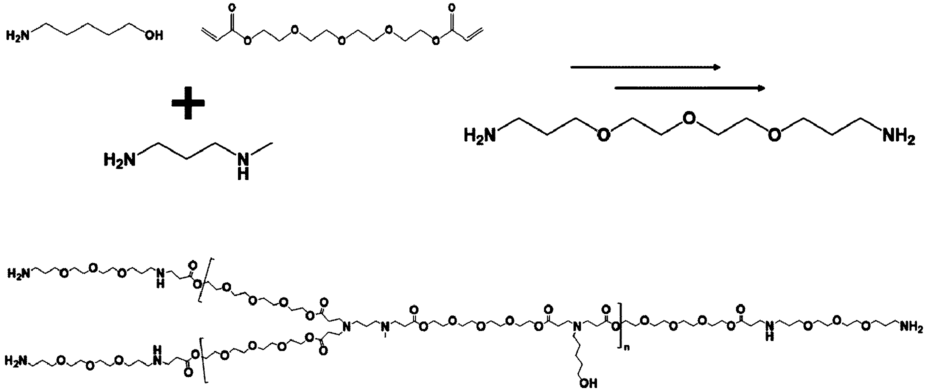

Amine monomer 8, acrylate monomer E, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b8E was obtained.

### [Manufacturing Example 241]

### Preparation of polymer b9A

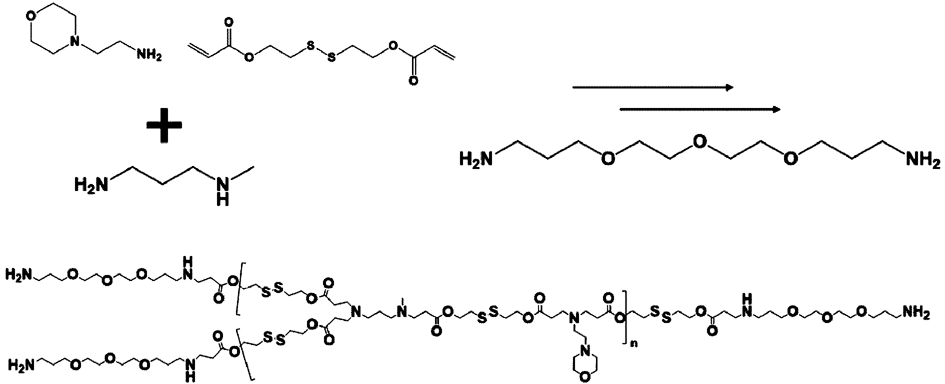

Amine monomer 9, acrylate monomer A, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b9A was obtained.

### [Manufacturing Example 242]

### Preparation of polymer b9B

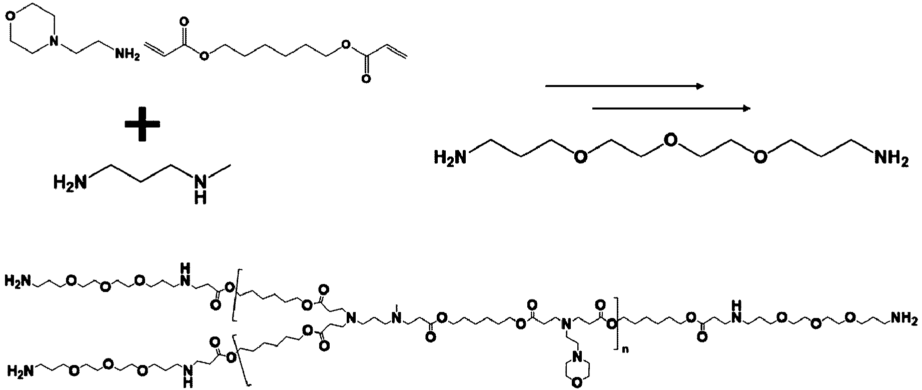

Amine monomer 9, acrylate monomer B, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b9B was obtained.

### [Manufacturing Example 243]

### Preparation of polymer b9C

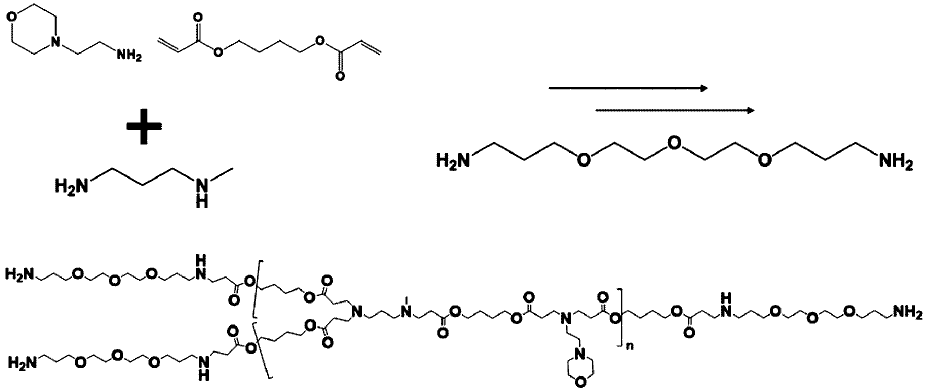

Amine monomer 9, acrylate monomer C, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b9C was obtained.

### [Manufacturing Example 244]

### Preparation of polymer b9D

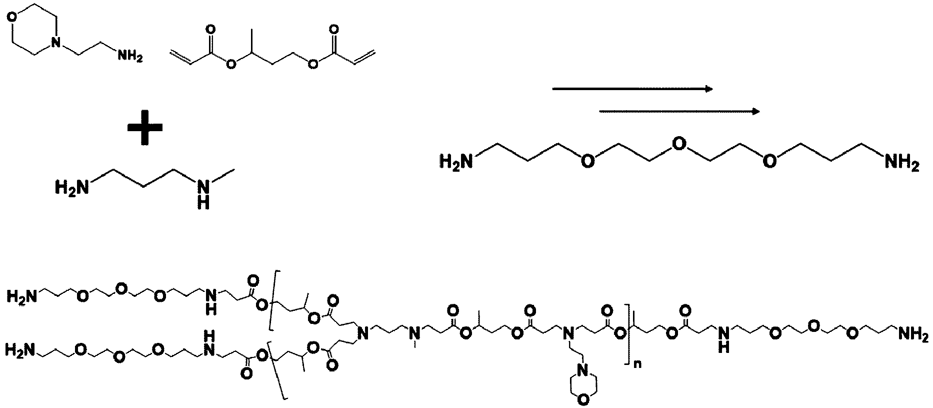

Amine monomer 9, acrylate monomer D, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b9D was obtained.

### [Manufacturing Example 245]

### Preparation of polymer b9E

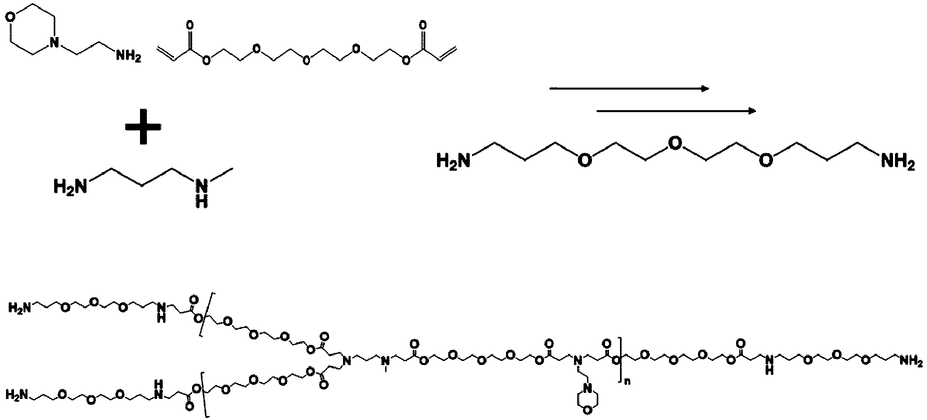

Amine monomer 9, acrylate monomer E, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b9E was obtained.

### [Manufacturing Example 246]

### Preparation of polymer b10A

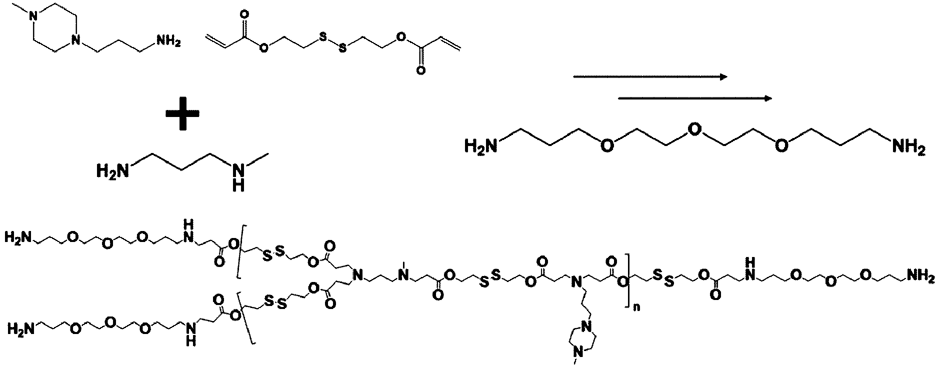

Amine monomer 10, acrylate monomer A, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b10A was obtained.

### [Manufacturing Example 247]

### Preparation of polymer b10B

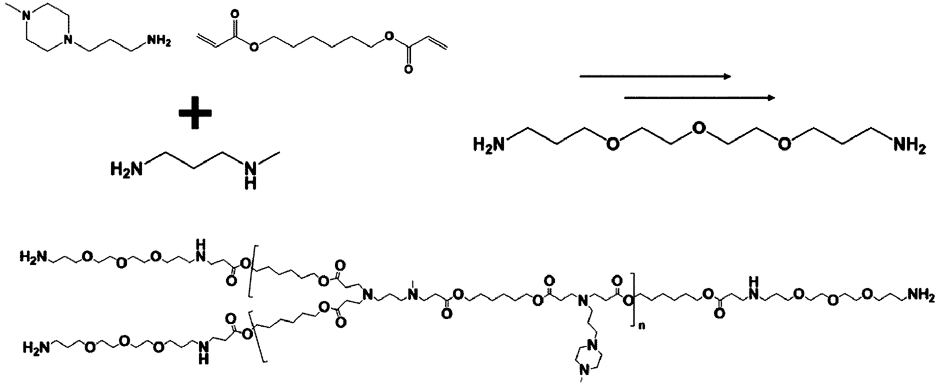

Amine monomer 10, acrylate monomer B, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b10B was obtained.

### [Manufacturing Example 248]

### Preparation of polymer b10C

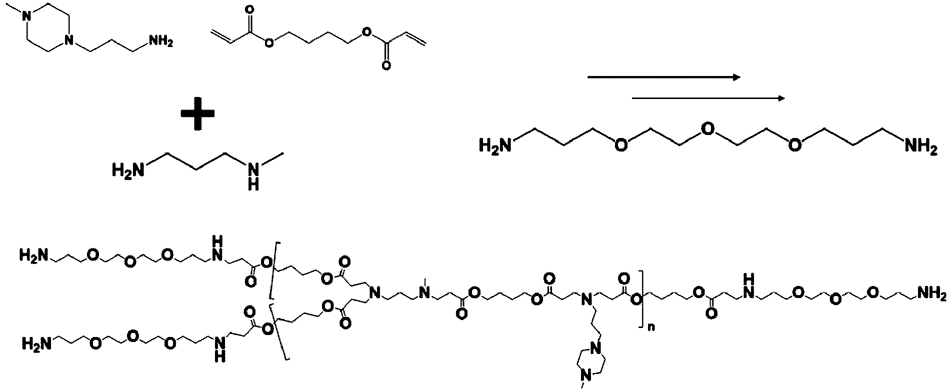

Amine monomer 10, acrylate monomer C, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b10C was obtained.

### [Manufacturing Example 249]

### Preparation of polymer b10D

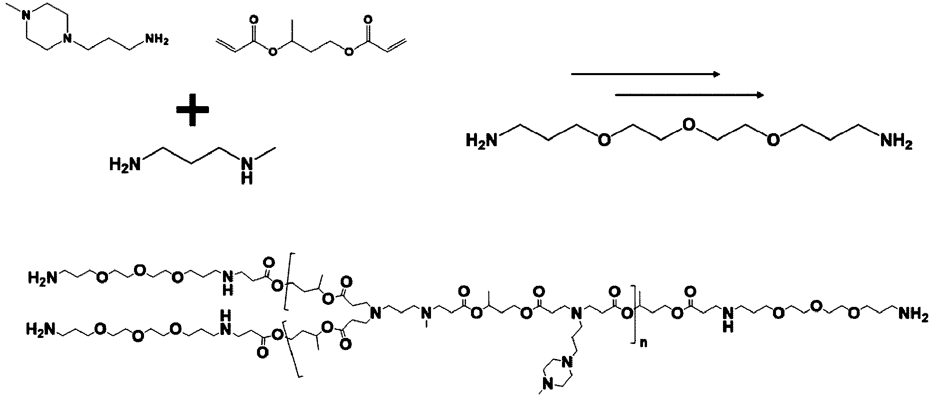

Amine monomer 10, acrylate monomer D, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b10D was obtained.

### [Manufacturing Example 250]

### Preparation of polymer b10E

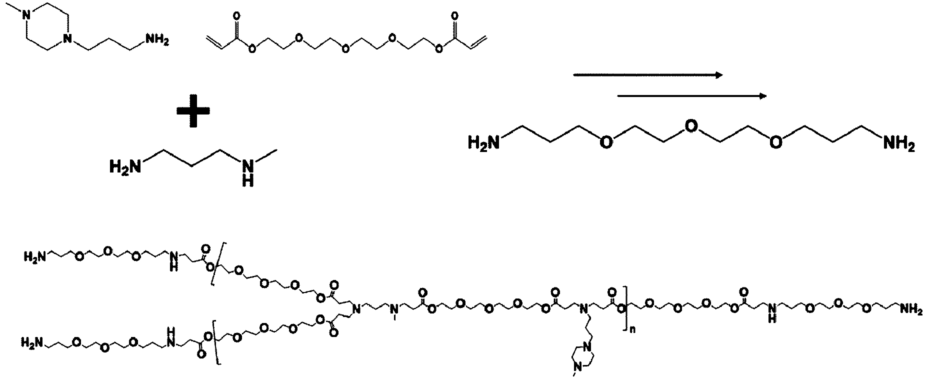

Amine monomer 10, acrylate monomer E, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b10E was obtained.

### [Manufacturing Example 251]

### Preparation of polymer b11A

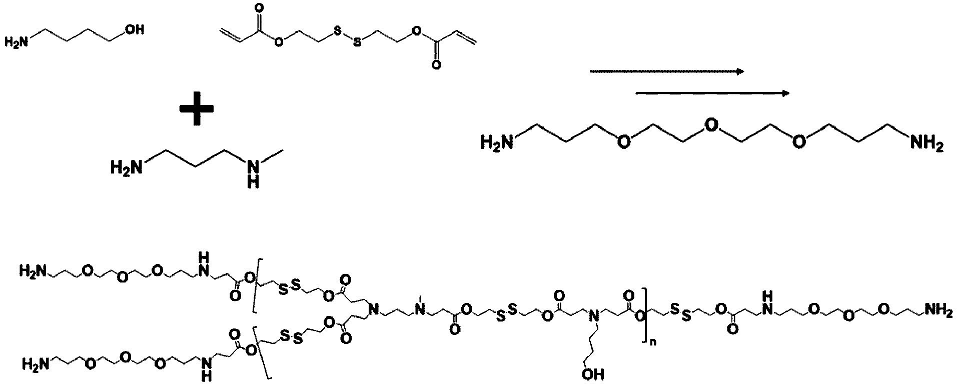

Amine monomer 11, acrylate monomer A, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b11A was obtained.

### [Manufacturing Example 252]

### Preparation of polymer b11B

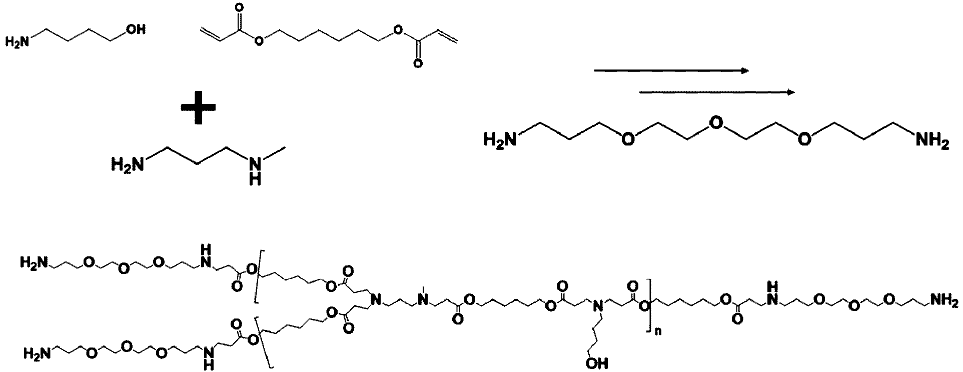

Amine monomer 11, acrylate monomer B, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b 11B was obtained.

### [Manufacturing Example 253]

### Preparation of polymer b11C

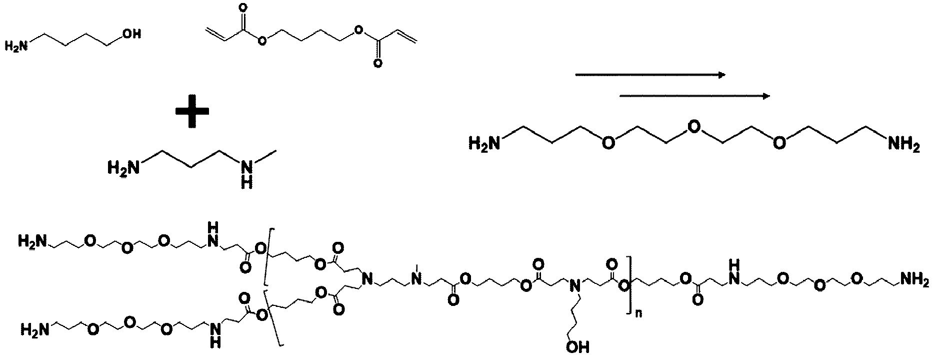

Amine monomer 11, acrylate monomer C, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b11C was obtained.

### [Manufacturing Example 254]

### Preparation of polymer b11D

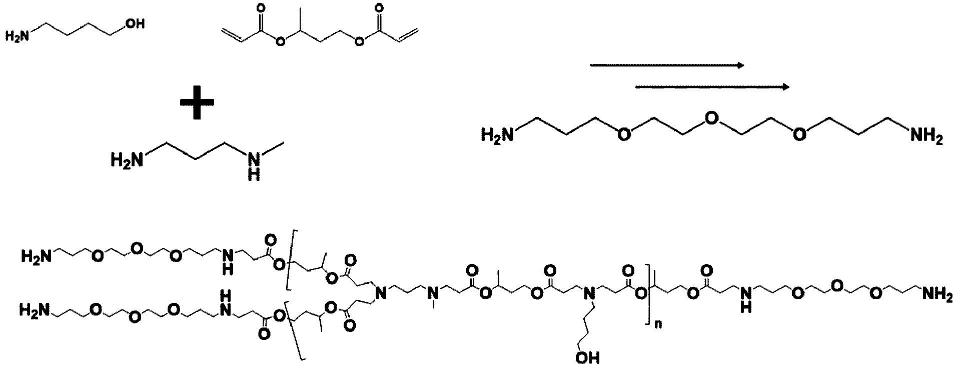

Amine monomer 11, acrylate monomer D, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b11D was obtained.

### [Manufacturing Example 255]

### Preparation of polymer b11e

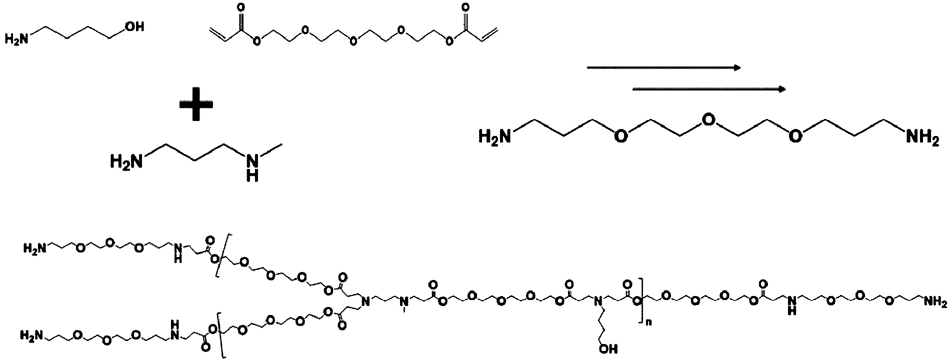

Amine monomer 11, acrylate monomer E, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b11E was obtained.

### [Manufacturing Example 256]

### Preparation of polymer b12A

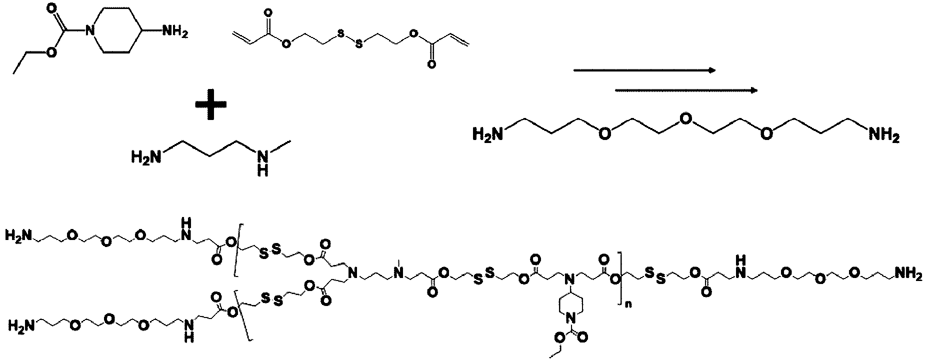

Amine monomer 12, acrylate monomer A, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b12A was obtained.

### [Manufacturing Example 257]

### Preparation of polymer b12B

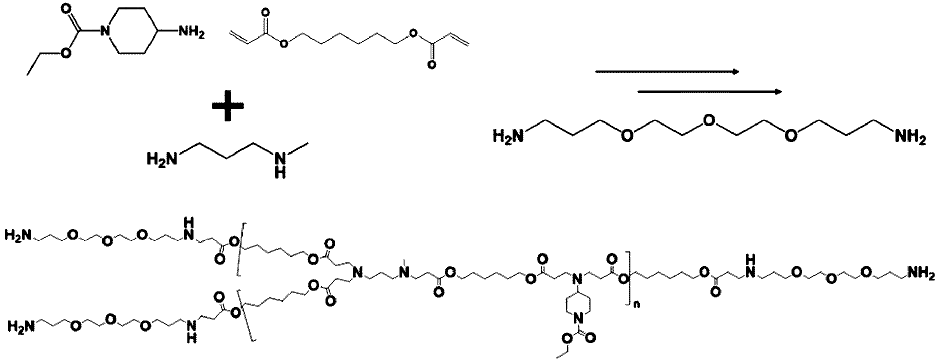

Amine monomer 12, acrylate monomer B, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b12B was obtained.

### [Manufacturing Example 258]

### Preparation of polymer b12C

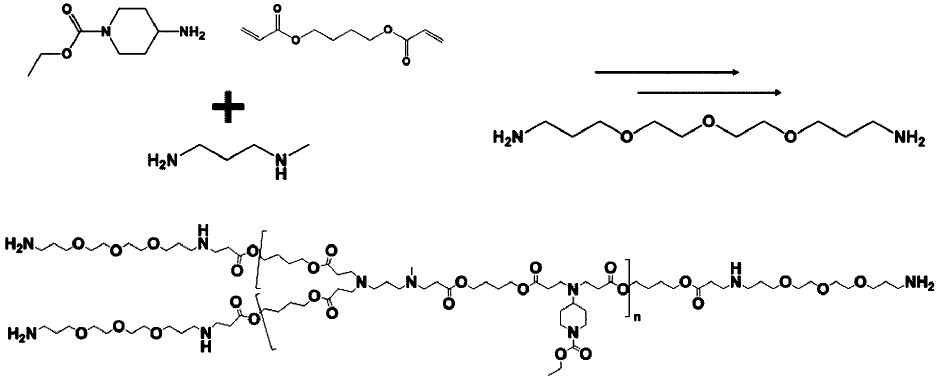

Amine monomer 12, acrylate monomer C, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b12C was obtained.

### [Manufacturing Example 259]

### Preparation of polymer b12D

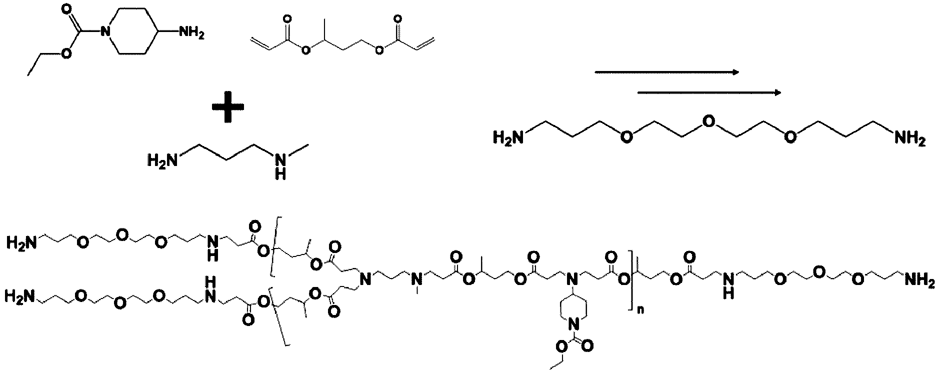

Amine monomer 12, acrylate monomer D, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b12D was obtained.

### [Manufacturing Example 260]

### Preparation of polymer b12E

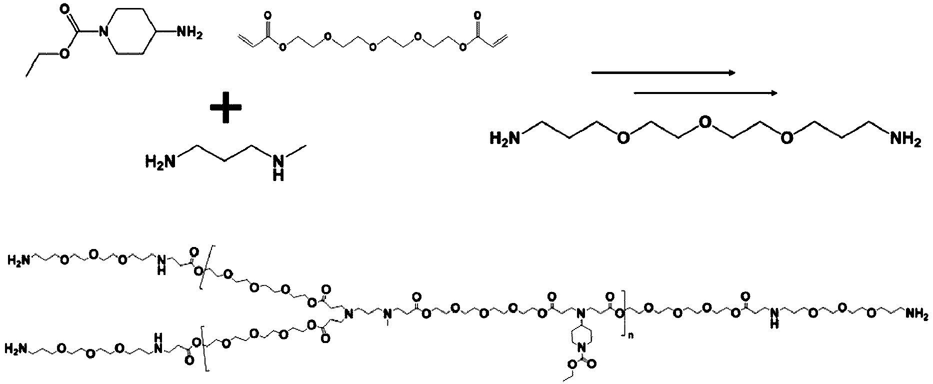

Amine monomer 12, acrylate monomer E, and branched dendrimer were added to a round bottom flask in a molar ratio of 0.4:1:0.2. Thereafter, DMF was added to the flask so that the concentration was 150 mg/ml, and the reaction was carried out at 90 °C for 48 hours. Thereafter, the mixture was cooled to room temperature, and then an end-capping agent was dissolved in DMF to a concentration of 150 mg/ml and added in a molar ratio of 2, and the reaction was carried out for 24 hours at room temperature, and then b12E was obtained.

### [Example 1]

### Preparation of polymeric nanoparticles (PNP)

mRNA was added to the polymer prepared by any one of Manufacturing Examples 1 to 260 above, and polymeric nanoparticles (PNP), which are nucleic acid delivery vehicles of the present invention, were prepared by the following method.

The polymer prepared by any one of Manufacturing Example 1 to 260 above was diluted in a pH 5.0, 25 mM sodium acetate buffer at a ratio for each polymer. Thereafter, 6 µl of the polymer solution and 6 µl of the mRNA solution having a concentration of 1 mg/ml were mixed and then stabilized at 4 °C for 30 minutes. Thereafter, the mixture was diluted with 38 µl of PBS to make a total volume of 50 µl, and used in the following test examples.

### [Test Example 1]

### Analysis of synthesized polymer

The polymers obtained by the methods of Manufacturing Example 1 to 260 above were analyzed by the following methods.

### 1.1 ¹H-NMR

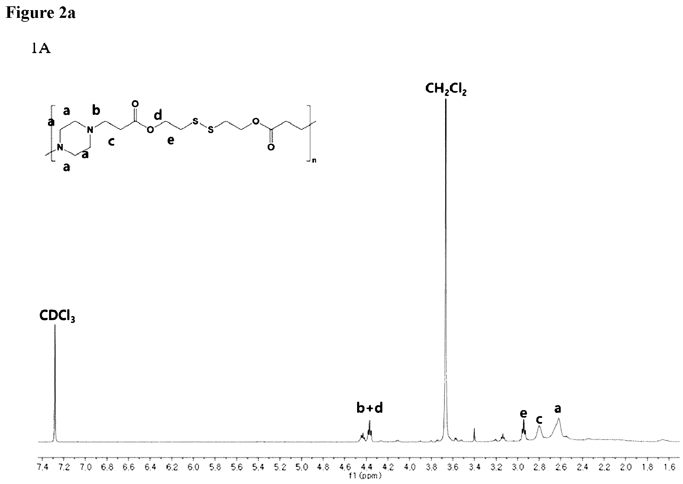

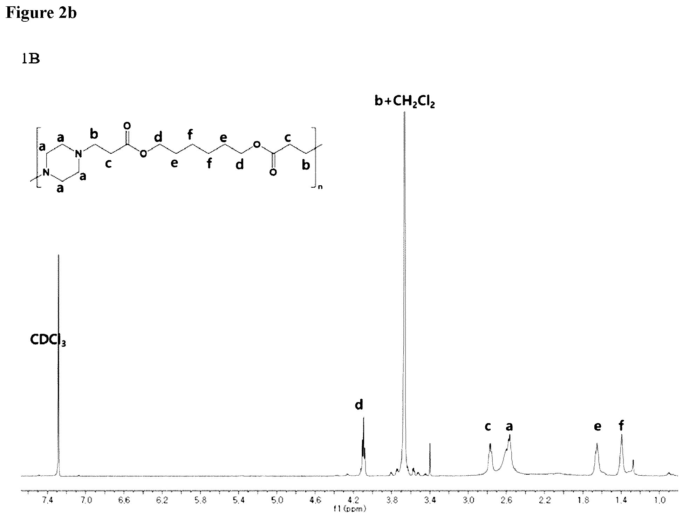

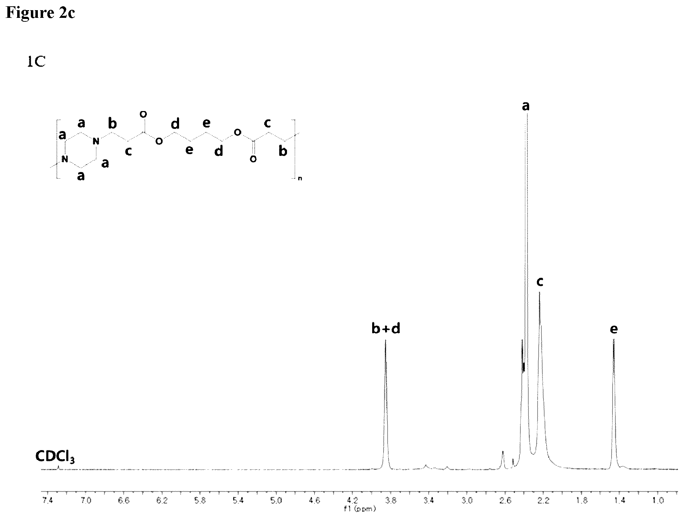

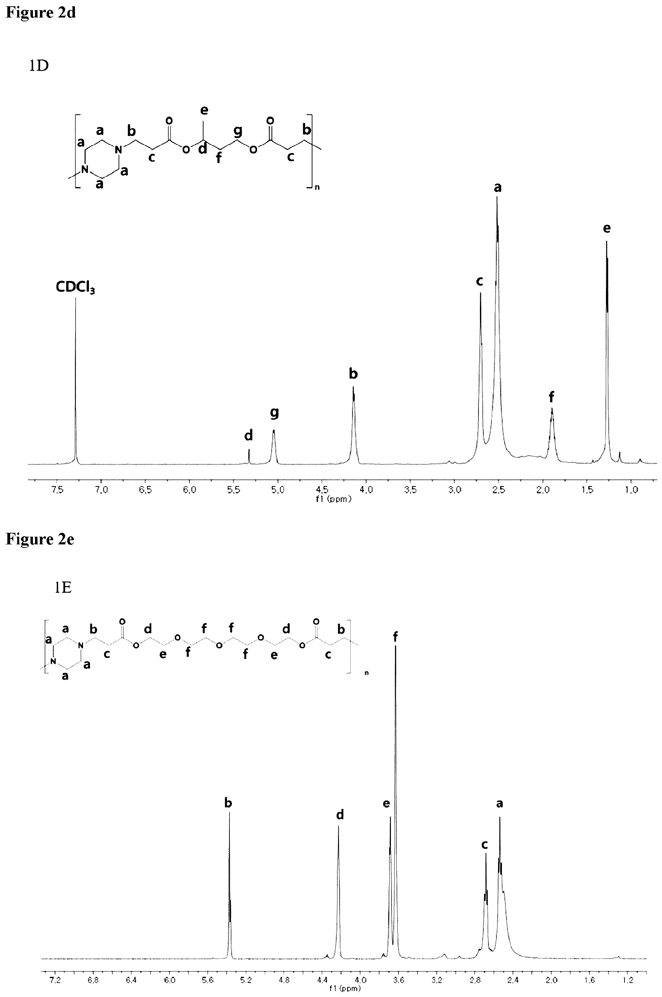

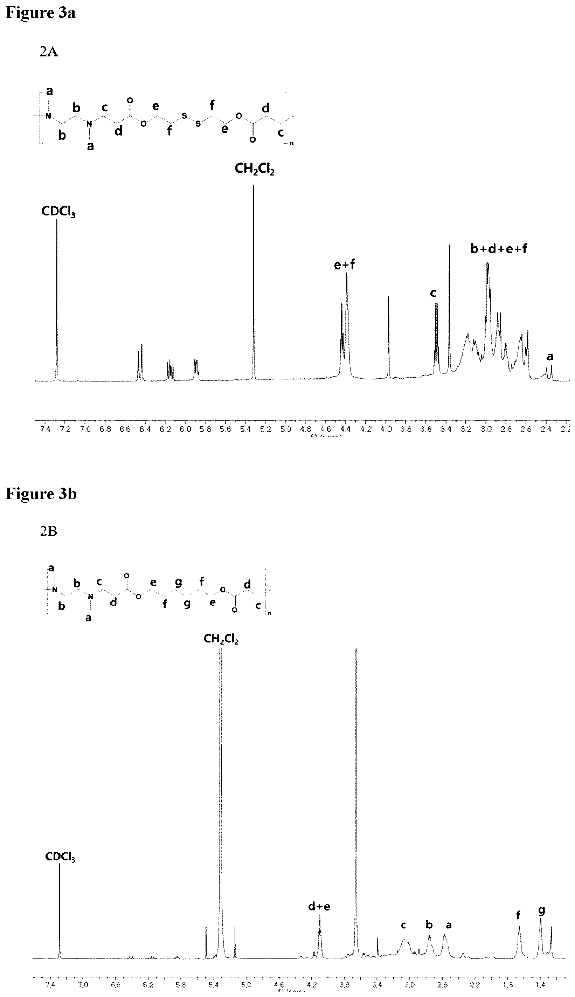

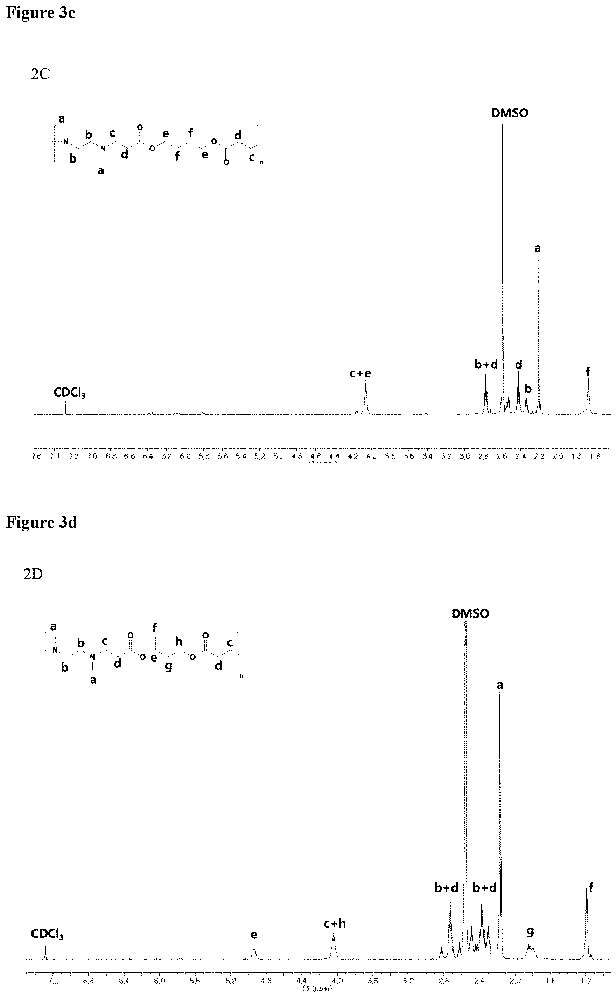

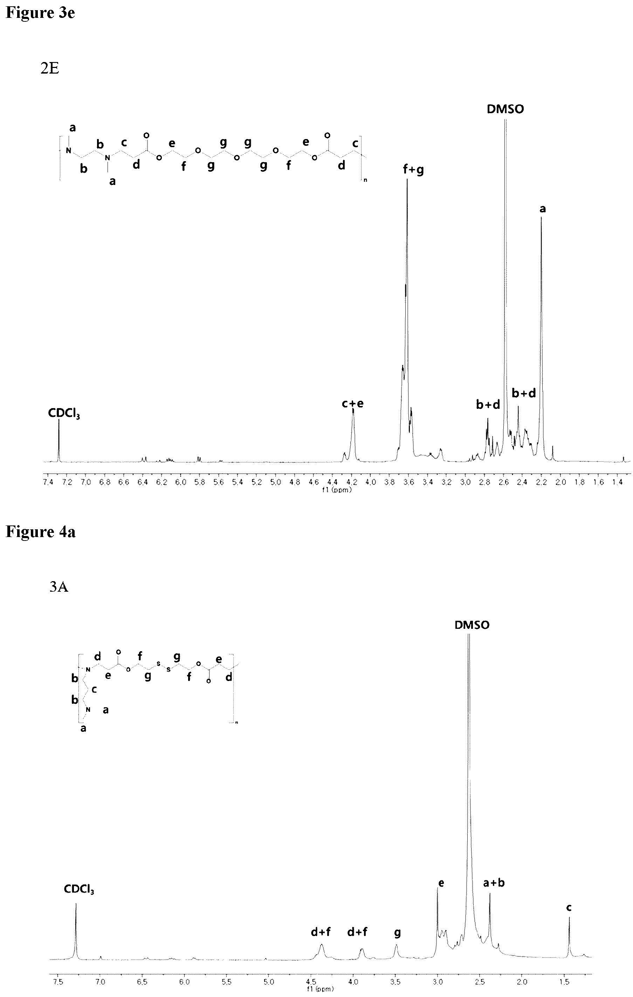

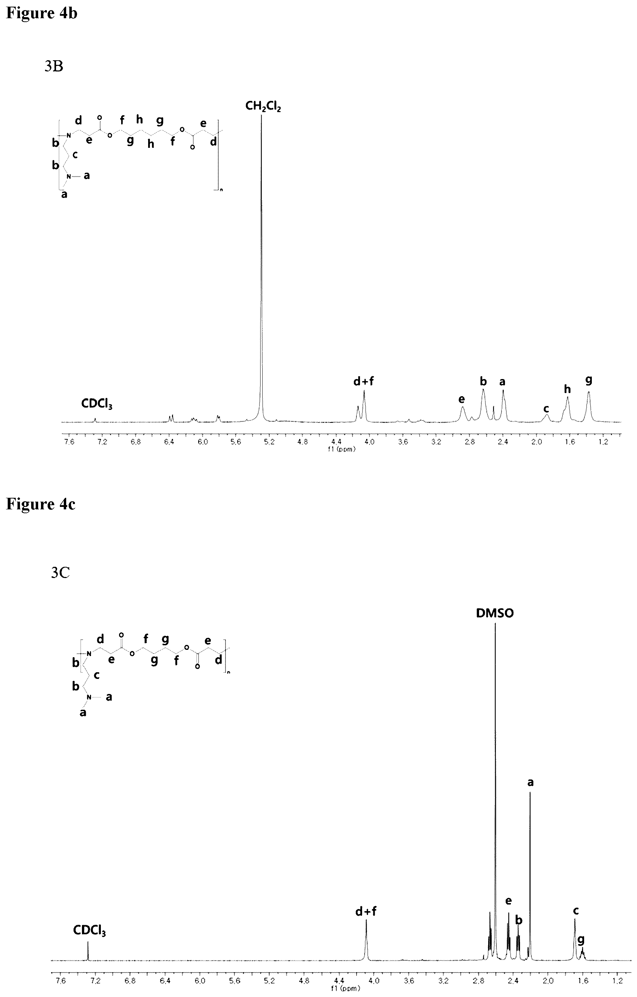

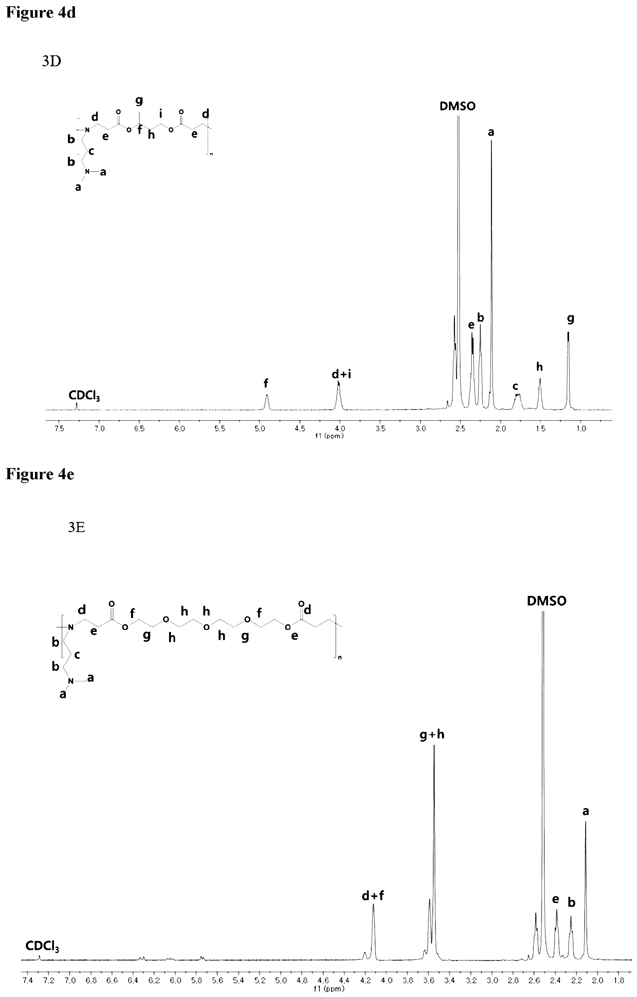

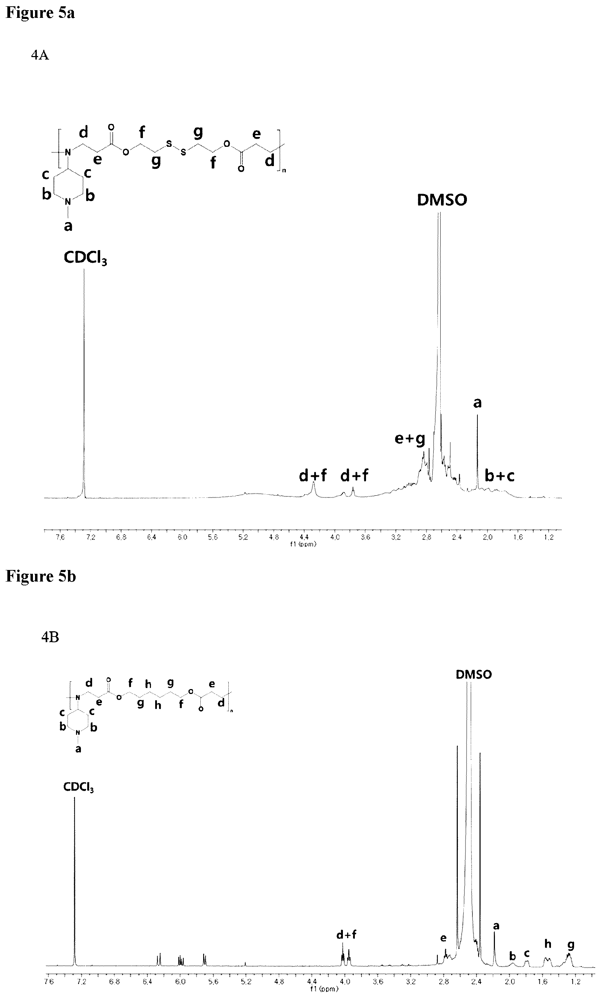

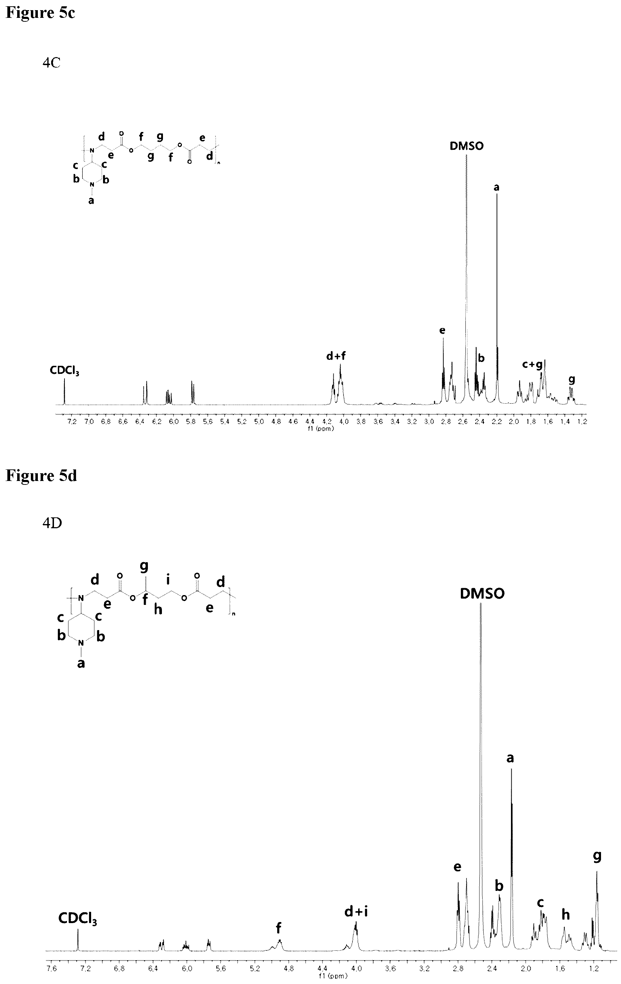

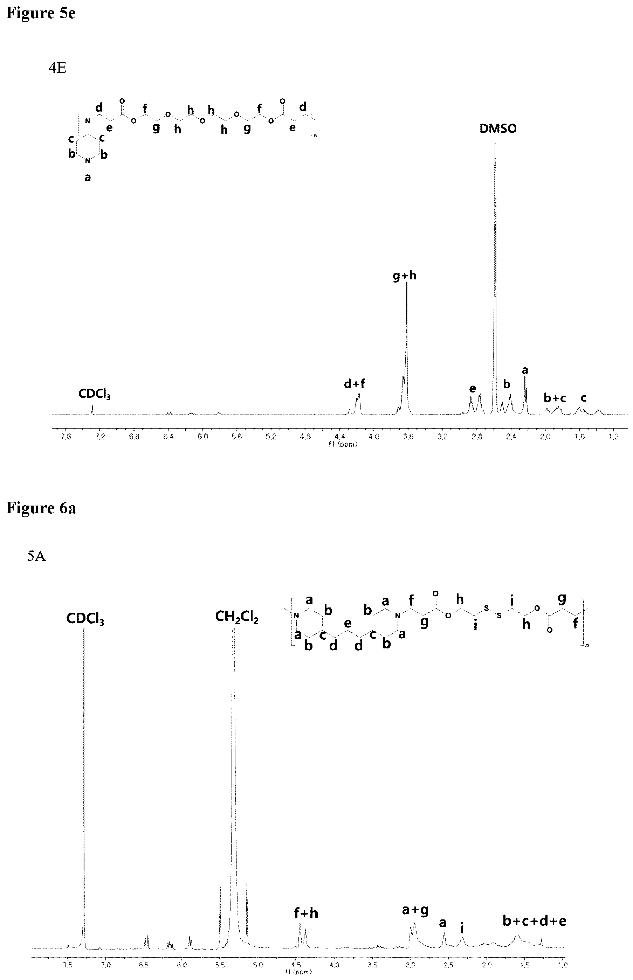

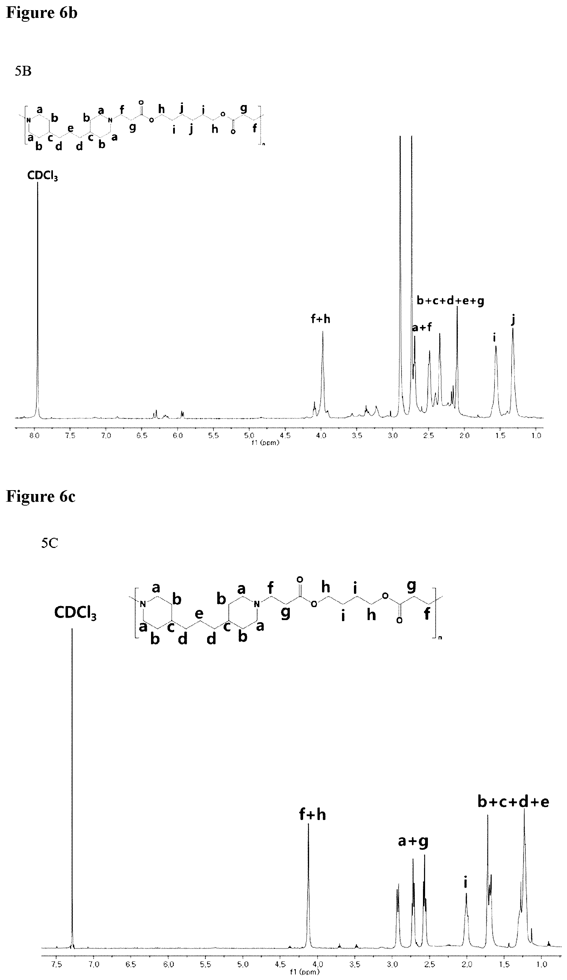

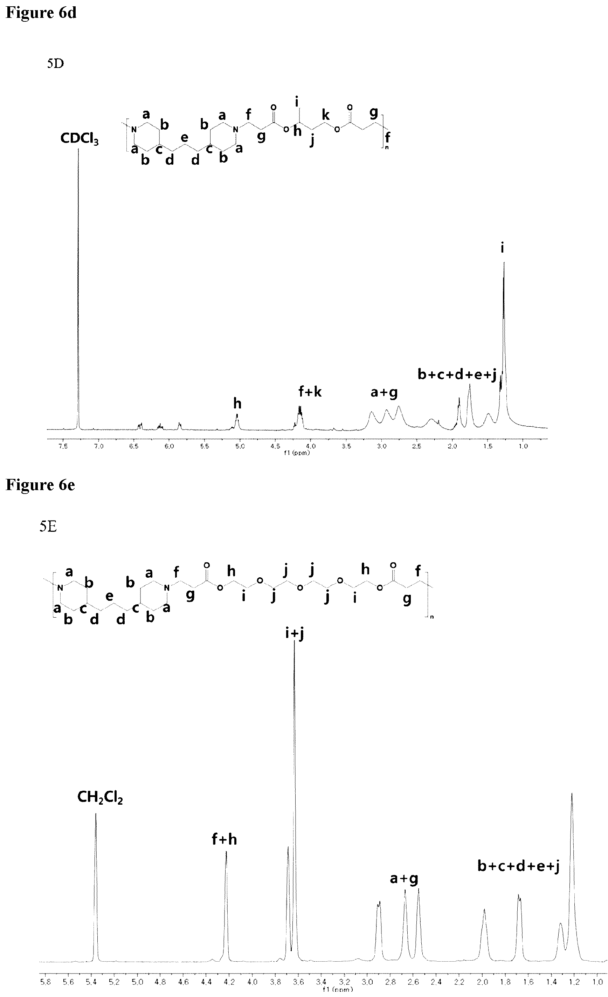

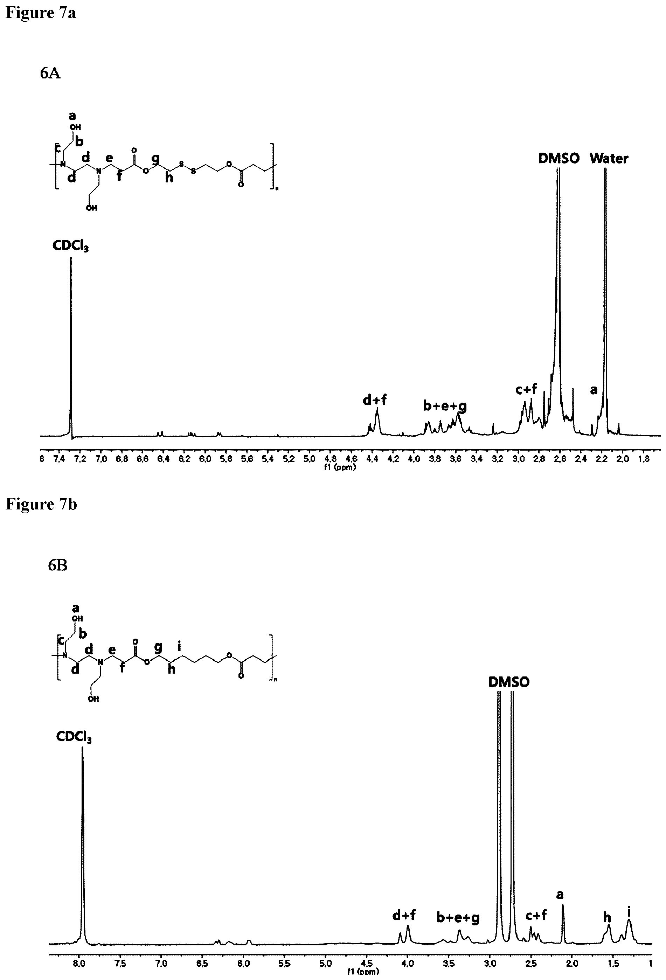

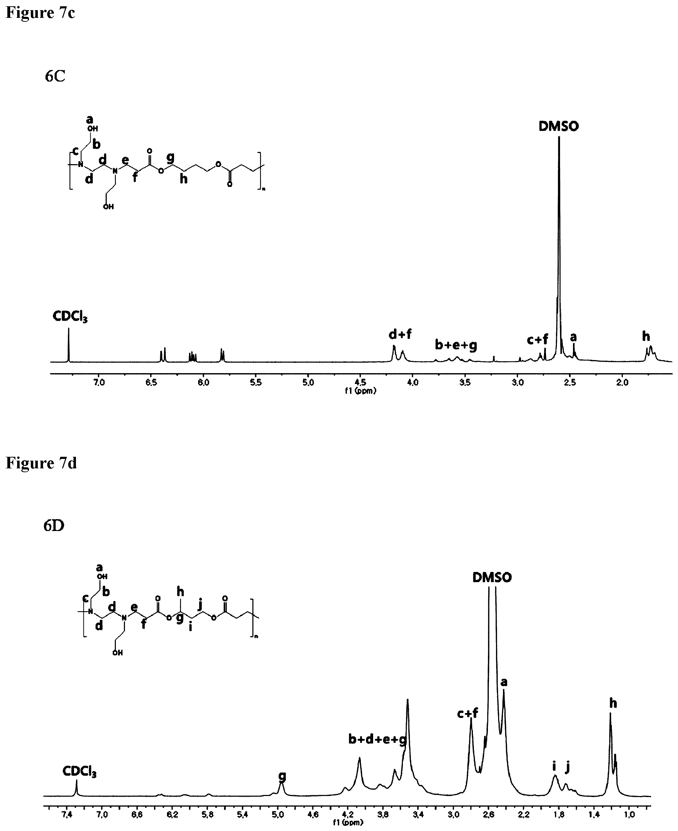

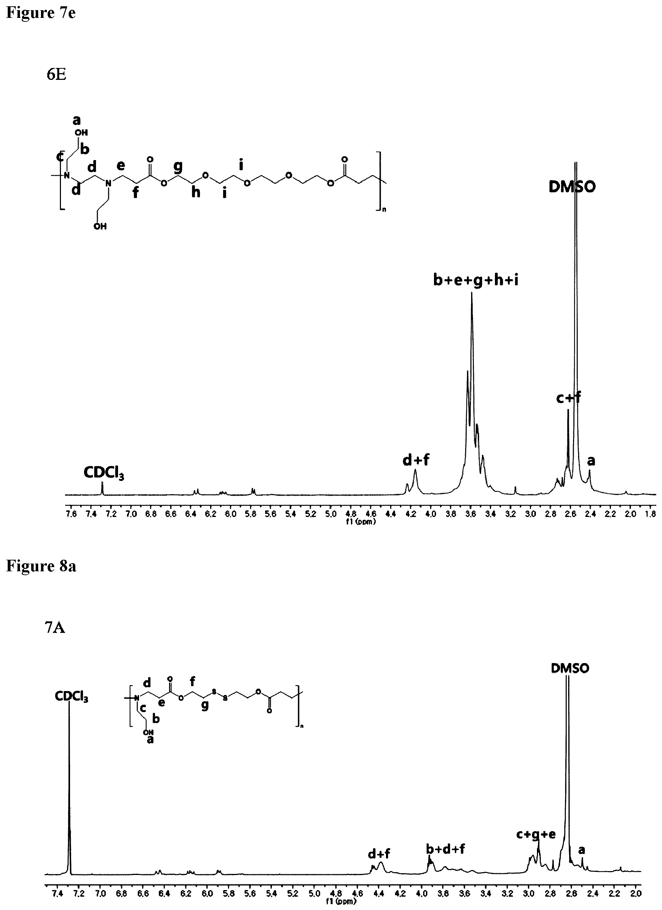

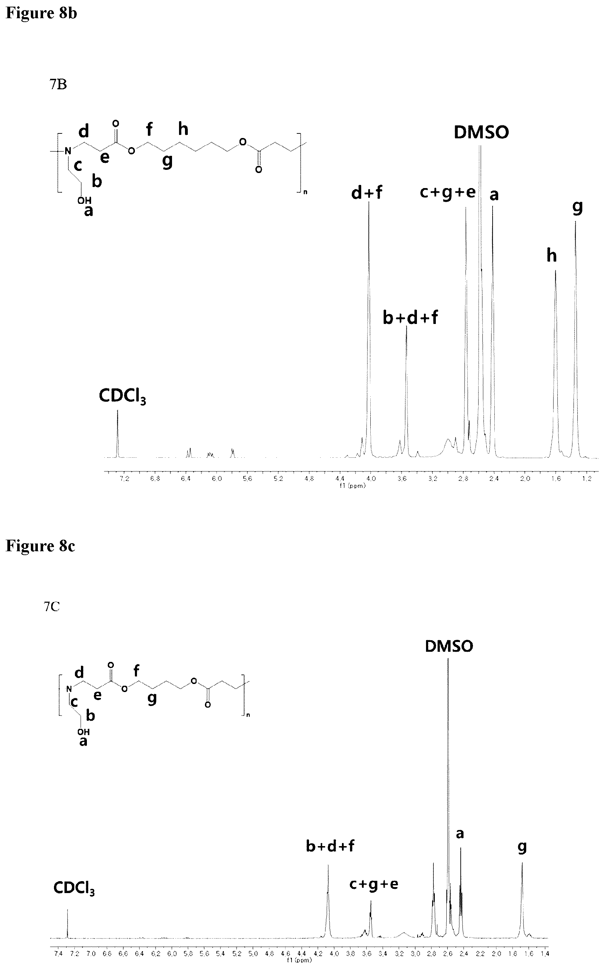

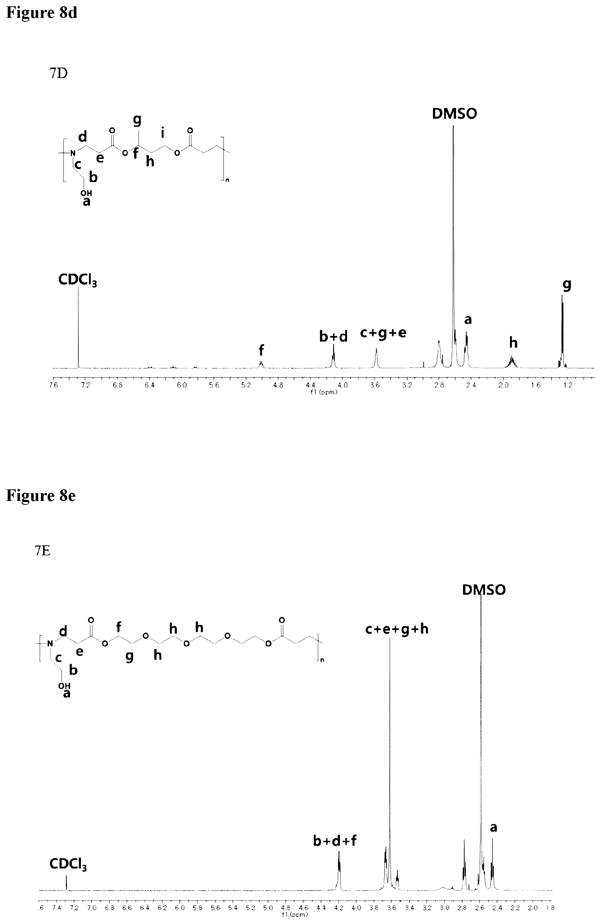

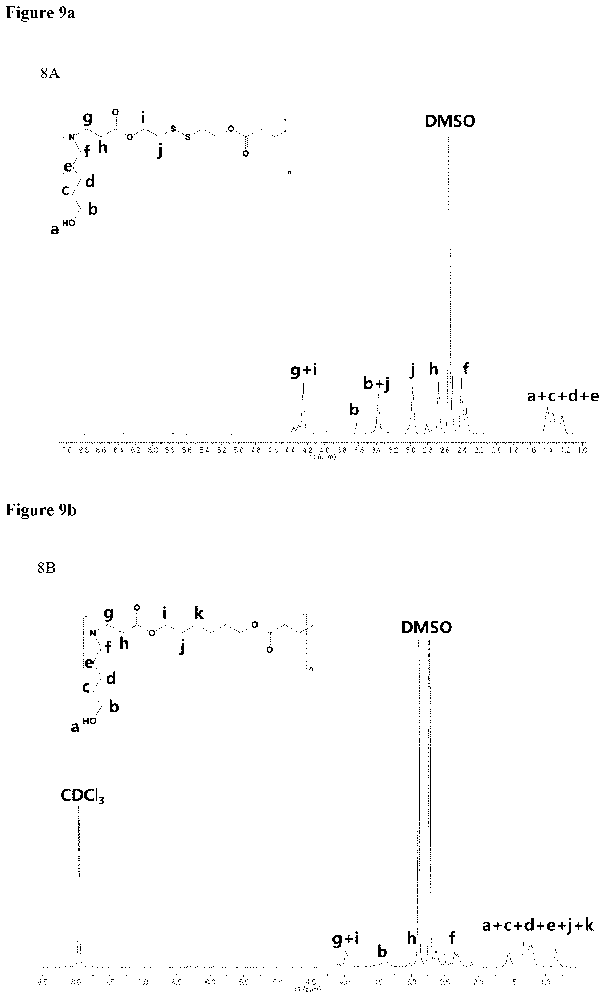

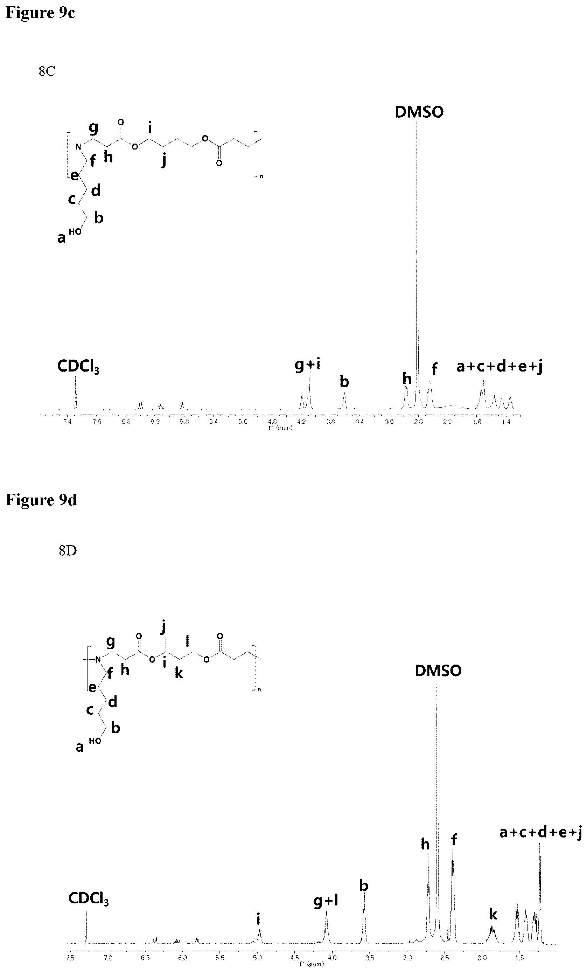

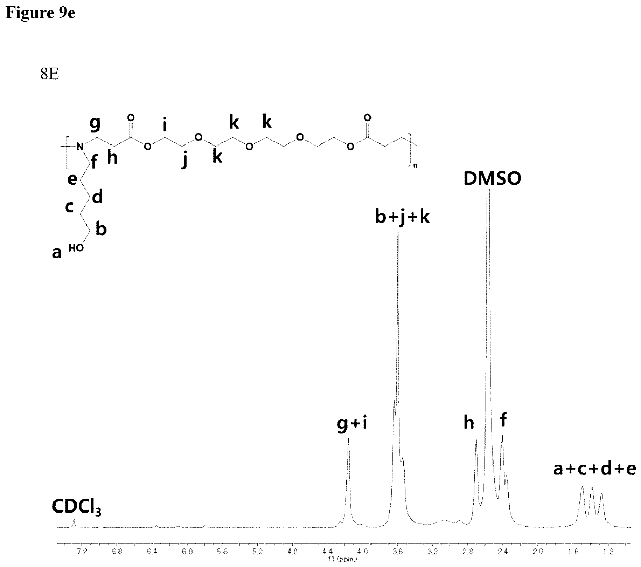

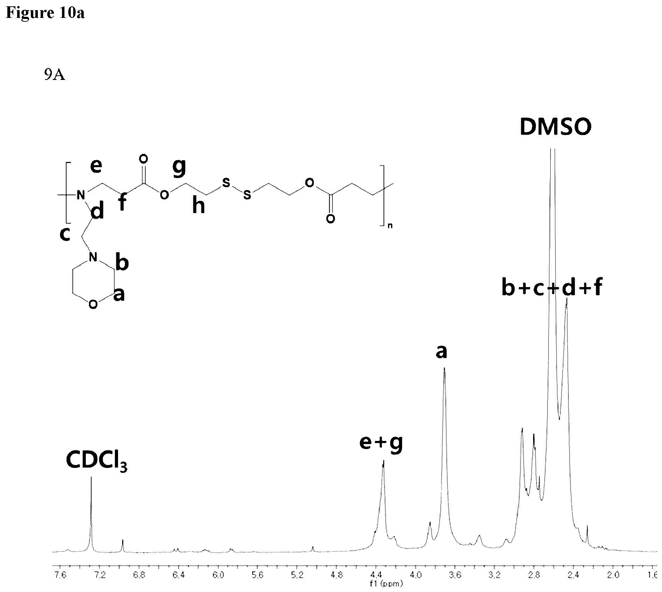

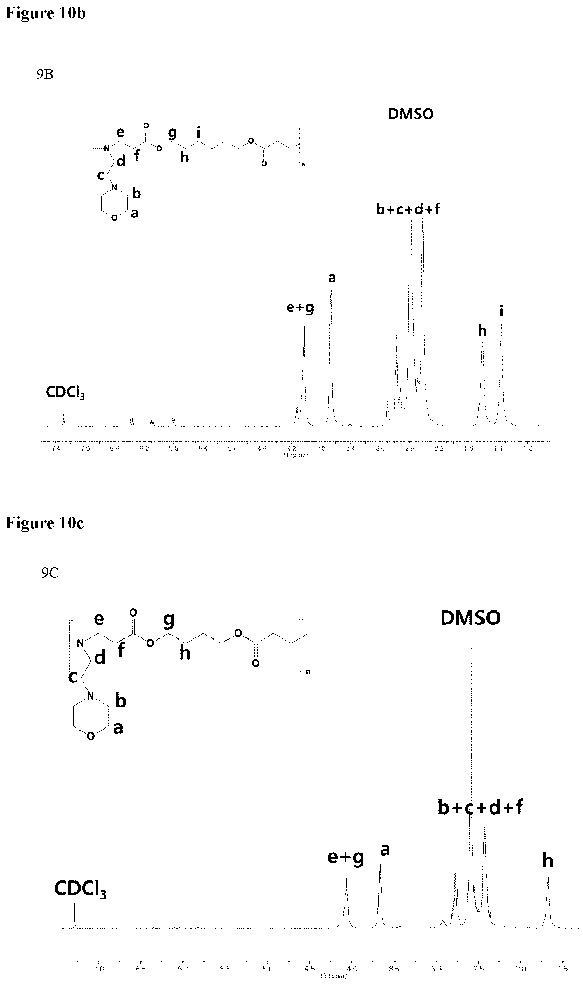

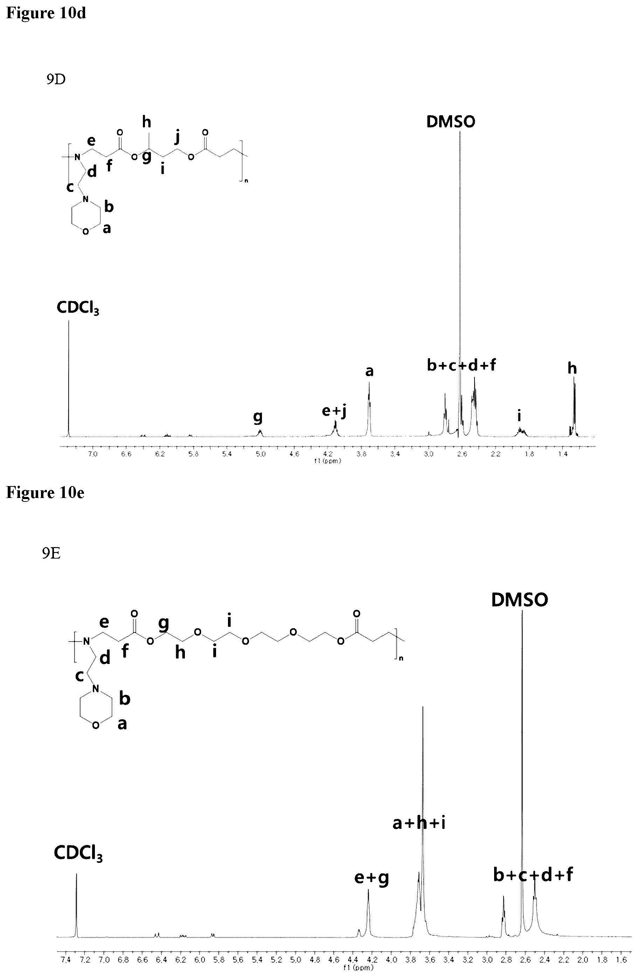

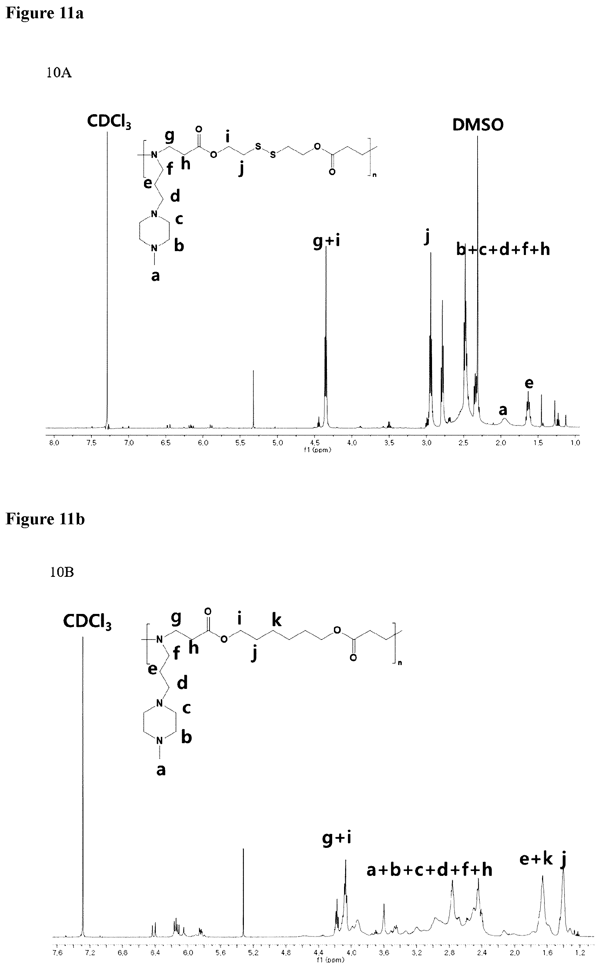

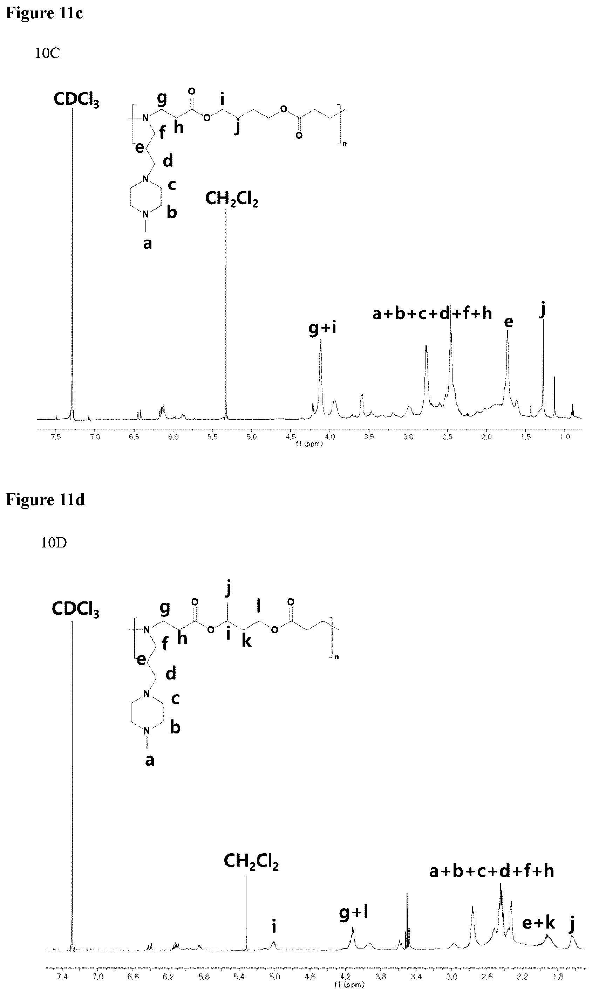

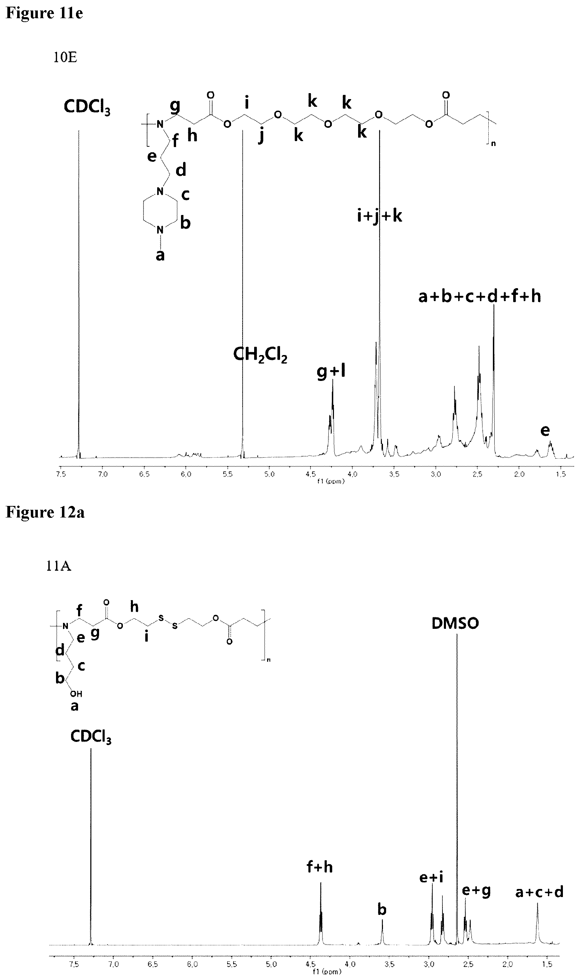

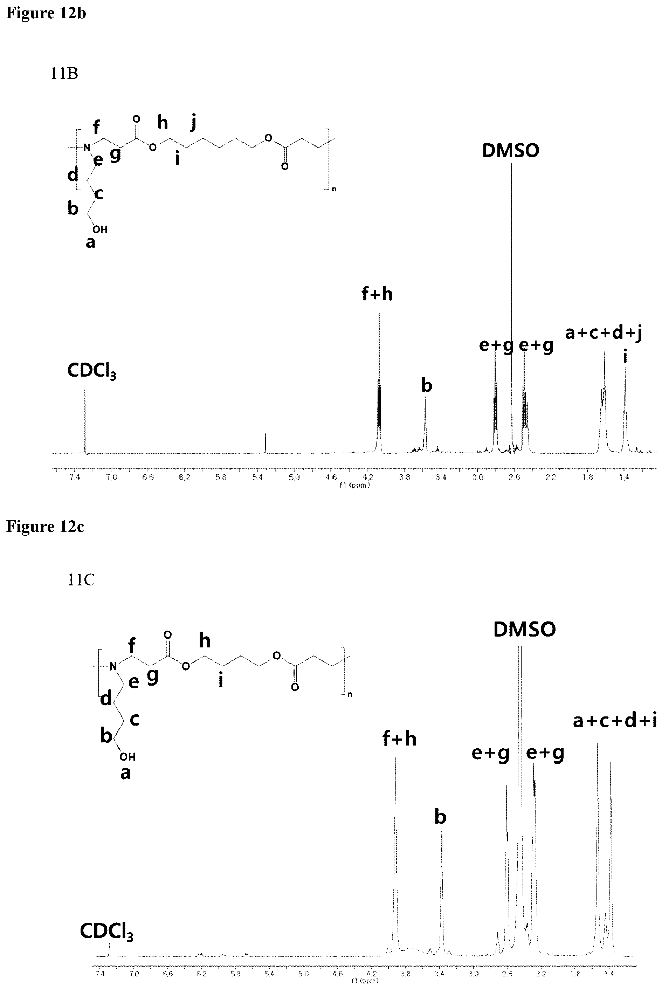

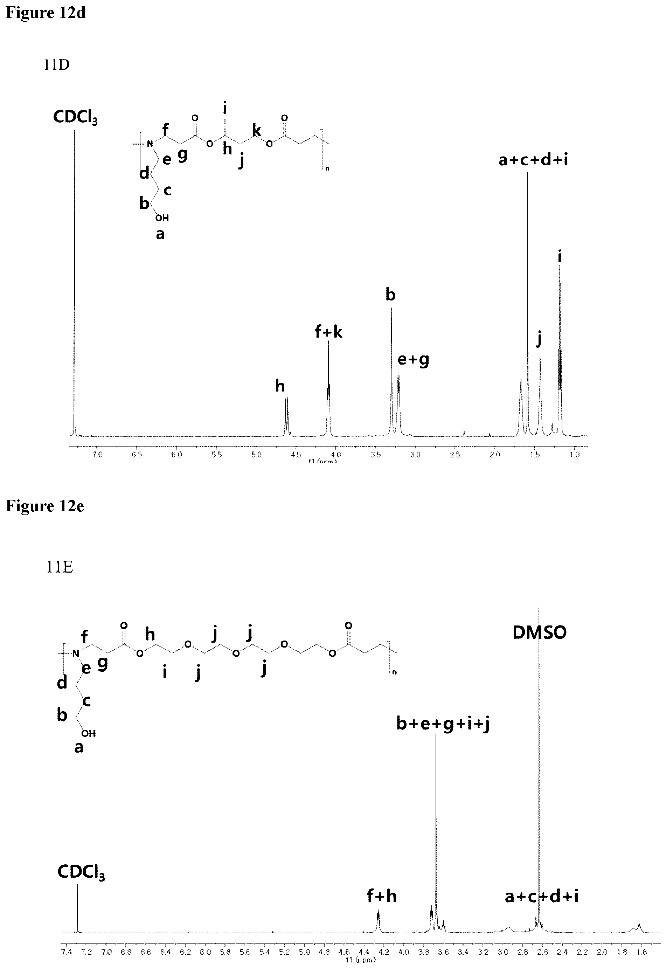

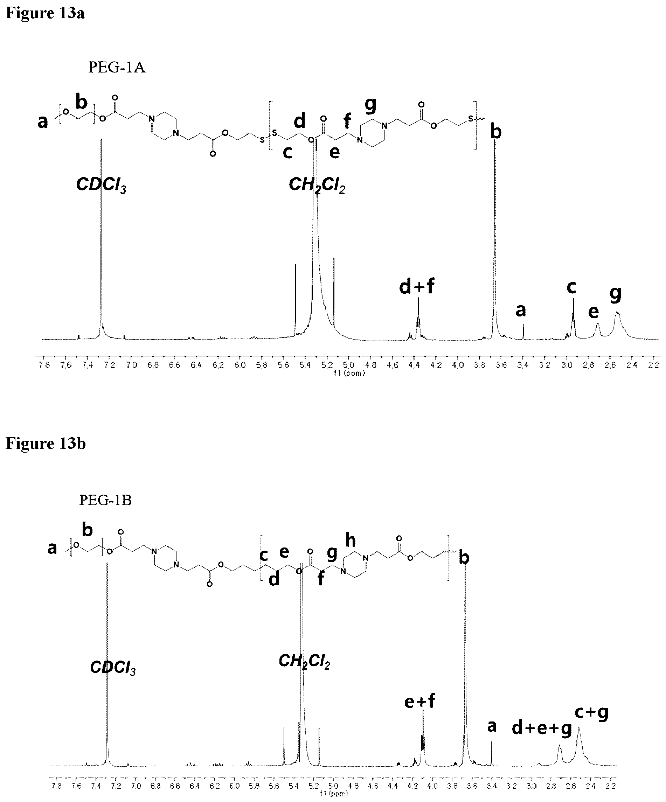

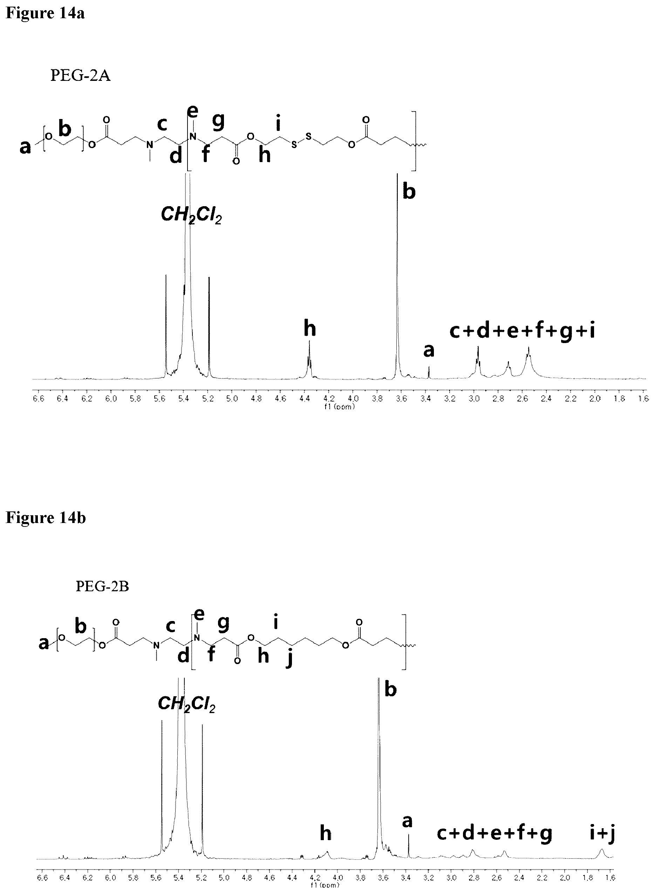

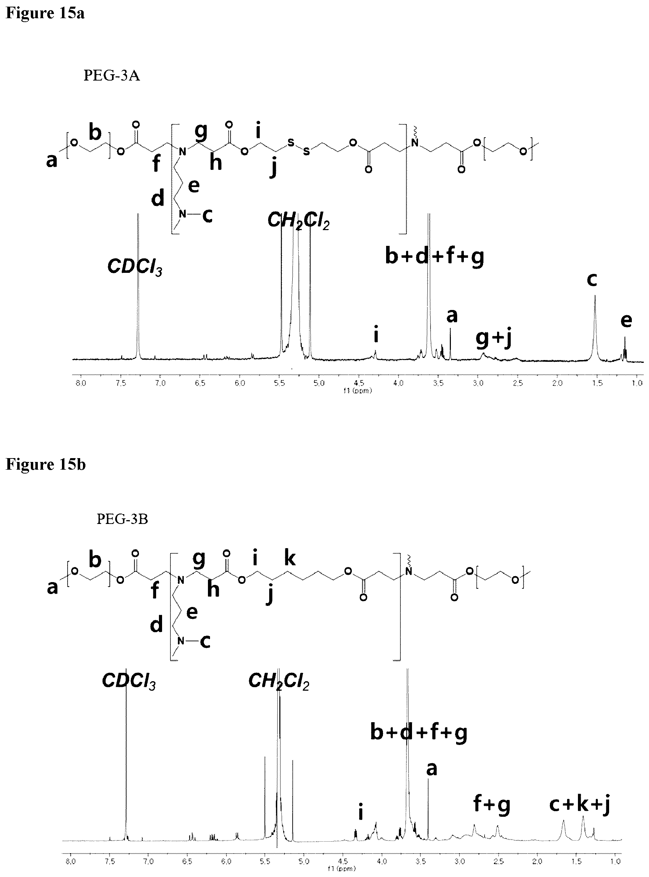

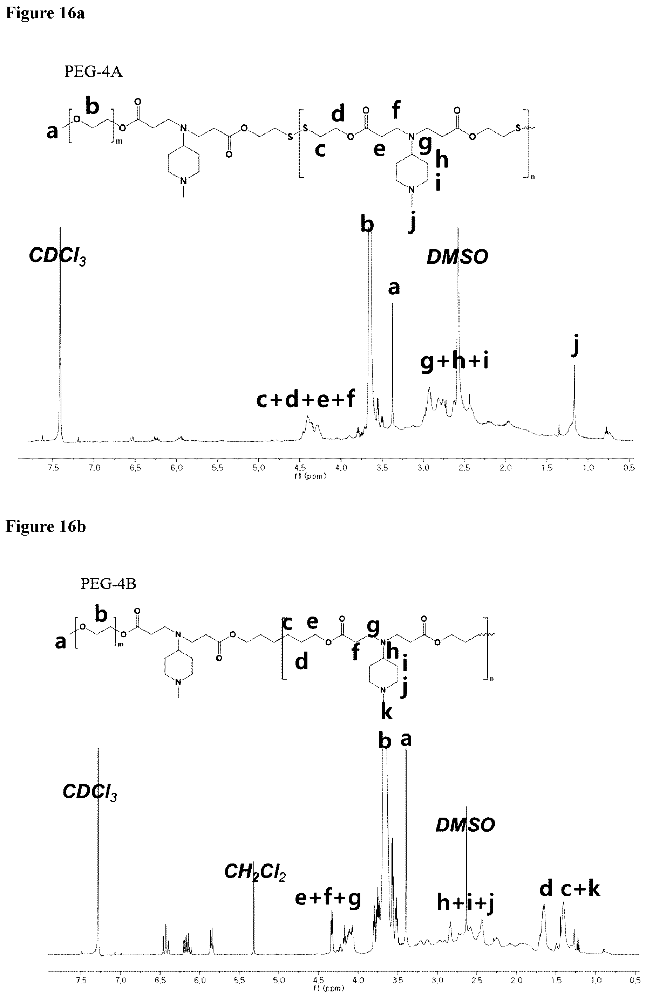

¹H-NMR analysis was performed on the synthesized polymer using deuterated chloroform (CDCl3) solvent, and the results are shown in Figures 2 to 16. As shown in Figures 2 to 16, it was confirmed that all polymers were prepared normally.

### 1.2 Hydrophobic gel permeable chromatography

The solvent was removed from the synthesized polymer, and then analysis was performed using hydrophobic gel permeable chromatography (hydrophobic GPC) by dissolving the polymer in tetrahydrofuran (THF) solvent at a concentration of 1 to 3 mg/ml.

### [Test Example 2]

### Analysis of polymeric nanoparticle (PNP) delivery vehicle

The polymeric nanoparticle (PNP) prepared in the same manner as in Example 1 above was analyzed by the following method.

### 2.1 Agarose gel electrophoresis

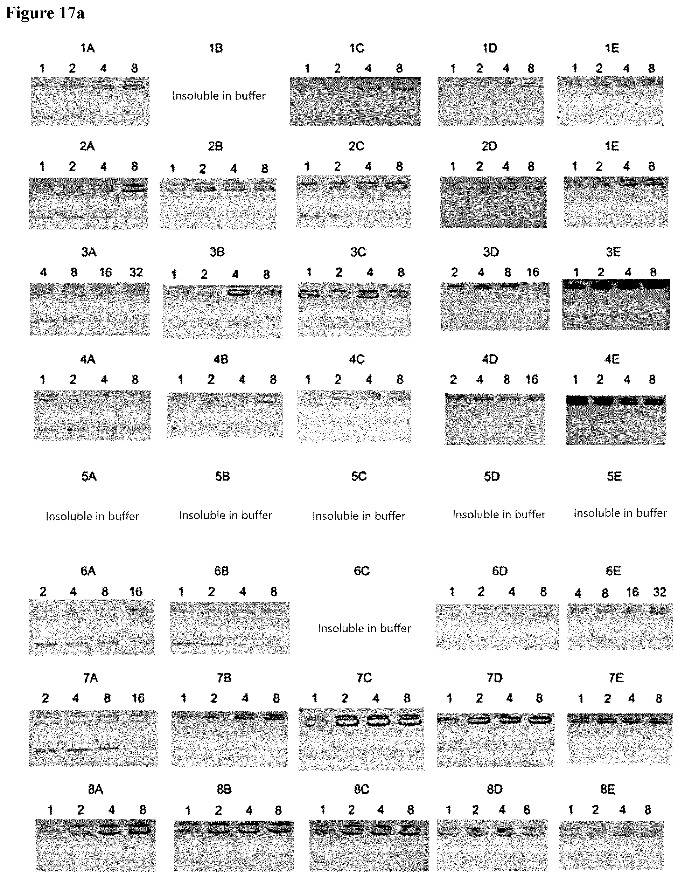

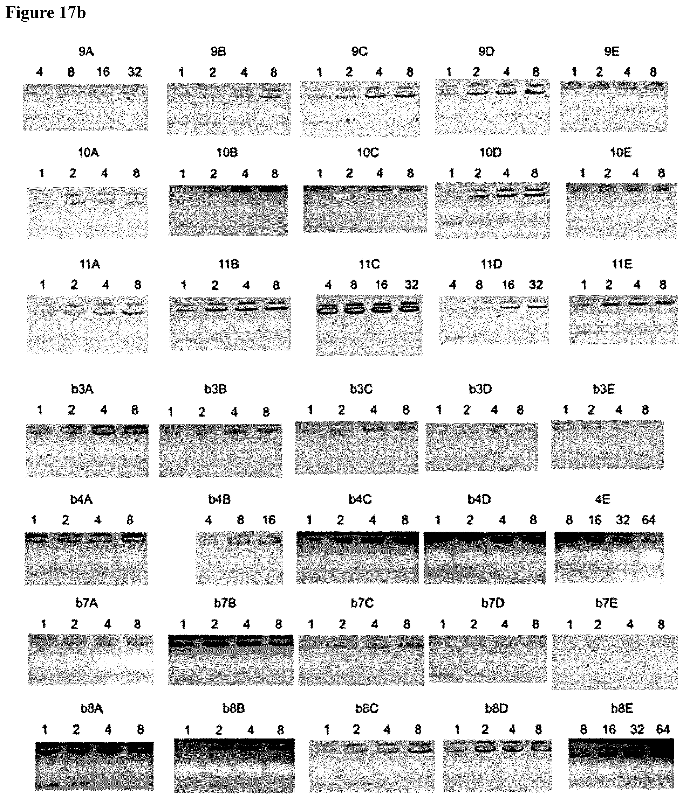

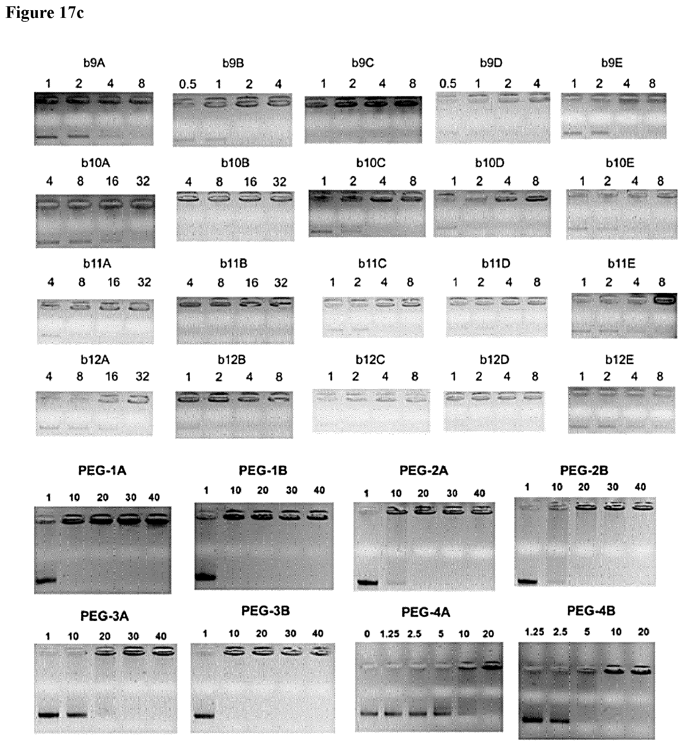

Agarose gel electrophoresis was performed on the polymers having pH sensitivity by adjusting the N/P ratio (the ratio of amine; N contained in the polymer and the ratio of phosphate; P contained in mRNA) of mRNA and polymer. The pH of the 25 mM NaOAc buffer was set to 5.0, and 5 µl of the solution in which the polymer was dissolved according to the N/P ratio was mixed with 5 µl of mRNA at a concentration of 200 ng/5 µl, and then gently vortexed. Thereafter, the mixture was stabilized at 4 °C for 30 minutes, loaded onto a 1 w/v% agarose gel containing GelRed, and electrophoresed at 100 V for 10 minutes. As a result of the experiment, as shown in Figures 17a to 17c, when a complex is formed at different N/P (Polymer/mRNA) ratios for each polymer, it can be confirmed that the mRNA band is not formed in the gel but is caught in the inlet. For example, the 6B polymer completely formed a complex starting from an N/P ratio of 4.

### 2.2 Zeta potential and dynamic light scattering measurement

For the polymers having pH sensitivity, the N/P ratio for each polymer was adjusted, and 6 µl of the solution dissolved in a pH 5.0,25 mM NaOAc buffer was mixed with 6 µl of luciferase mRNA (FLuc) of SEQ ID NO: 1 at a concentration of 1 mg/ml, and then gently vortexed. Thereafter, the mixture was stabilized at 4 °C for 30 minutes, and 38 µl of PBS was added to make a final volume of 50 µl.

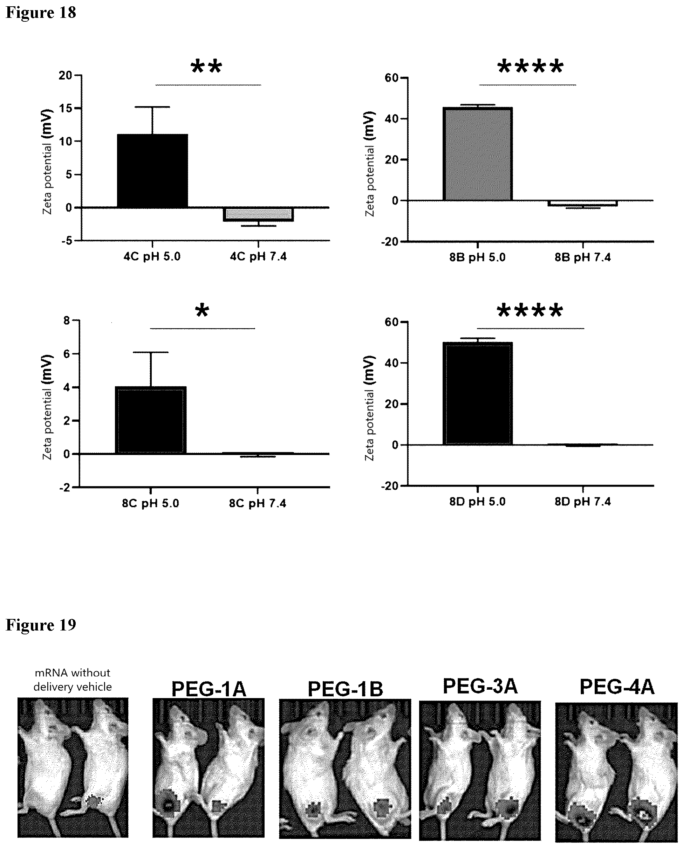

The zeta potential and size of the polymer-mRNA complex were measured by diluting the complex solution described above 10 times in distilled water and using a zeta potential meter and a dynamic light scattering (DLS) device. As shown in Figure 18, it was confirmed that the zeta potential changed depending on the pH. Through this, it was confirmed that the mRNA complex was formed due to a positive charge at low pH, and it was confirmed that it could have low toxicity and high mRNA delivery efficiency by changing to neutral in the pH in the body.

### [Test Example 3]

### Confirmation of mRNA expression in vivo of polymeric nanoparticles (PNP)

The polymeric nanoparticles prepared according to Example 1 above were injected intramuscularly into animals to confirm the expression of mRNA.

### 3.1 Confirmation of in vivo mRNA expression

A representative polymer was synthesized according to the method described in the above manufacturing example, and polymeric nanoparticles were prepared according to Manufacturing Example 261, and mRNA expression level screening was performed. The method for forming the polymer-mRNA complex used for in vivo injection to confirm the mRNA expression is as follows. The weight ratio of each polymer having pH sensitivity and a micelle structure was adjusted, and 6 µl of the solution dissolved in a pH 5.0,25 mM NaOAc buffer was mixed with 6 µl of FLuc of SEQ ID NO: 1 at a concentration of 1 mg/ml, and then gently vortexed. Thereafter, the mixture was stabilized at 4 °C for 30 minutes, and 38 µl of PBS was added to make a final volume of 50 µl. Regarding the preparation of lipid nanoparticles (LNPs) used as a comparative group, the following preparation method was used. The molar ratio of ionizable lipid (SM-102):DSPC:cholesterol:PEG-lipid was set to 50:10:38.5:1.5 and dissolved in ethanol. Thereafter, the mRNA diluted in a 25 mM, pH 5.0 sodium acetate buffer was mixed with the mixture dissolved in ethanol so that the volume ratio was 3:1 and the N/P ratio was 5.67. Thereafter, Centrifugation was performed at least three times at 1000 g for 5 minutes with PBS added using a 100 kDa Amicon. Finally, the mRNA was diluted with PBS so that the concentration was 6 ug/50 ul.

**[Table 4]**

| SEQ ID NO | RNA sequence |
|---|---|
| 1 | ATGGAGGACGCCAAGAACATCAAGAAGGGCCCCGCCCCCTTCTACCCCCTGGAGG ACGGCACCGCCGGCGAGCAGCTGCACAAGGCCATGAAGCGGTACGCCCTGGTGC CCGGCACCATCGCCTTCACCGACGCCCACATCGAGGTGGACATCACCTACGCCGA GTACTTCGAGATGAGCGTGCGGCTGGCCGAGGCCATGAAGCGGTACGGCCTGAAC ACCAACCACCGGATCGTGGTGTGCAGCGAGAACAGCCTGCAGTTCTTCATGCCCG TGCTGGGCGCCCTGTTCATCGGCGTGGCCGTGGCCCCCGCCAACGACATCTACAA CGAGCGGGAGCTGCTGAACAGCATGGGCATCAGCCAGCCCACCGTGGTGTTCGTG AGCAAGAAGGGCCTGCAGAAGATCCTGAACGTGCAGAAGAAGCTGCCCATCATC CAGAAGATCATCATCATGGACAGCAAGACCGACTACCAGGGCTTCCAGAGCATGT ACACCTTCGTGACCAGCCACCTGCCCCCCGGCTTCAACGAGTACGACTTCGTGCCC GAGAGCTTCGACCGGGACAAGACCATCGCCCTGATCATGAACAGCAGCGGCAGC ACCGGCCTGCCCAAGGGCGTGGCCCTGCCCCACCGGACCGCCTGCGTGCGGTTCA GCCACGCCCGGGACCCCATCTTCGGCAACCAGATCATCCCCGACACCGCCATCCT GAGCGTGGTGCCCTTCCACCACGGCTTCGGCATGTTCACCACCCTGGGCTACCTGA TCTGCGGCTTCCGGGTGGTGCTGATGTACCGGTTCGAGGAGGAGCTGTTCCTGCG GAGCCTGCAGGACTACAAGATCCAGAGCGCCCTGCTGGTGCCCACCCTGTTCAGC TTCTTCGCCAAGAGCACCCTGATCGACAAGTACGACCTGAGCAACCTGCACGAGA TCGCCAGCGGCGGCGCCCCCCTGAGCAAGGAGGTGGGCGAGGCCGTGGCCAAGC GGTTCCACCTGCCCGGCATCCGGCAGGGCTACGGCCTGACCGAGACCACCAGCGC CATCCTGATCACCCCCGAGGGCGACGACAAGCCCGGCGCCGTGGGCAAGGTGGT GCCCTTCTTCGAGGCCAAGGTGGTGGACCTGGACACCGGCAAGACCCTGGGCGTG AACCAGCGGGGCGAGCTGTGCGTGCGGGGCCCCATGATCATGAGCGGCTACGTG AACAACCCCGAGGCCACCAACGCCCTGATCGACAAGGACGGCTGGCTGCACAGC GGCGACATCGCCTACTGGGACGAGGACGAGCACTTCTTCATCGTGGACCGGCTGA AGAGCCTGATCAAGTACAAGGGCTACCAGGTGGCCCCCGCCGAGCTGGAGAGCA TCCTGCTGCAGCACCCCAACATCTTCGACGCCGGCGTGGCCGGCCTGCCCGACGA CGACGCCGGCGAGCTGCCCGCCGCCGTGGTGGTGCTGGAGCACGGCAAGACCAT GACCGAGAAGGAGATCGTGGACTACGTGGCCAGCCAGGTGACCACCGCCAAGAA GCTGCGGGGCGGCGTGGTGTTCGTGGACGAGGTGCCCAAGGGCCTGACCGGCAA GCTGGACGCCCGGAAGATCCGGGAGATCCTGATCAAGGCCAAGAAGGGCGGCAA GATCGCCGTGTGA |

In order to confirm in vivo mRNA expression using the composition above, luciferase mRNA (FLuc) of SEQ ID NO: 1 was injected into the gastrocnemius muscle of the mouse hind leg at a concentration of 6 µg/50 µl. Thereafter, mRNA expression was confirmed after 24 hours. 200 µl of luciferin was injected intraperitoneally into each individual at a concentration of 15 mg/ml. 10 minutes after luciferin injection, mRNA expression was confirmed with an exposure time of 15 seconds.

As a result, as shown in Table 5 below, when delivering mRNA using the polymers according to the present invention, it was confirmed that mRNA expression was equivalent or higher than that of free mRNA (in the present specification, free mRNA refers to mRNA without a delivery vehicle), and it was confirmed that among them, 22 polymers showed higher mRNA expression than lipid nanoparticle (LNP) delivery vehicles. In addition, experiments using representative branched polymers also showed a high mRNA expression rate compared to free mRNA. Therefore, it can be seen that the delivery vehicle of the present invention can be used as an mRNA delivery vehicle.

**[Table 5]**

| Manufacturing Example | Item No. | mRNA expression efficiency |
|---|---|---|
| 1 | 1A | 3.61 |
| 3 | 1C | 9.56 |
| 4 | 1D | 6.39 |
| 5 | 1E | 5.39 |
| 6 | 2A | 2.02 |
| 9 | 2D | 8.37 |
| 10 | 2E | 27.79 |
| 11 | 3A | 6.58 |
| 13 | 3C | 17.81 |
| 14 | 3D | 1.07 |
| 15 | 3E | 10.19 |
| 16 | 4A | 1.76 |
| 17 | 4B | 23.72 |
| 18 | 4C | 58.63 |
| 19 | 4D | 38.72 |
| 20 | 4E | 16.00 |
| 21 | 5A | 2.24 |
| 27 | 6B | 41.22 |
| 29 | 6D | 21.58 |
| 30 | 6E | 67.60 |
| 31 | 7A | 36.43 |
| 32 | 7B | 37.83 |
| 33 | 7C | 11.28 |
| 34 | 7D | 62.72 |
| 35 | 7E | 6.29 |
| 36 | 8A | 29.36 |
| 37 | 8B | 78.62 |
| 38 | 8C | 333.17 |
| 39 | 8D | 50.90 |
| 40 | 8E | 32.18 |
| 41 | 9A | 2.12 |
| 42 | 9B | 27.33 |
| 43 | 9C | 92.24 |
| 44 | 9D | 160.44 |
| 45 | 9E | 18.38 |
| 50 | 10E | 2.54 |
| 51 | 11A | 36.90 |
| 53 | 11C | 6.14 |
| 55 | 11E | 2.43 |
| 222 | b3B | 13.47 |
| 223 | b3C | 1.45 |
| 225 | b3E | 1.24 |
| 227 | b4B | 658.79 |
| 228 | b4C | 100.52 |
| 229 | b4D | 115.61 |
| 235 | b7E | 18.36 |
| 238 | b8C | 218.05 |
| 239 | b8D | 24.59 |
| 242 | b9B | 45.64 |
| 243 | b9C | 3.80 |
| 244 | b9D | 105.24 |
| 247 | b10B | 72.51 |
| 248 | b10C | 18.37 |
| 249 | b10D | 19.68 |
| 250 | b10E | 182.42 |
| 252 | b11B | 867.09 |
| 253 | b11C | 67.04 |
| 255 | b12E | 10.02 |
| 256 | b12A | 98.05 |
| 258 | b12C | 80.08 |
| 256 | b12D | 10.26 |
| Free mRNA | comparative group | 1 |
| Lipid nanoparticle | comparative group | 12.27 |

### [Test Example 4]

### Confirmation of in vivo mRNA expression of polymeric nanoparticles (PNP) comprising PEG

The mRNA expression efficiency using polymeric nanoparticles (PNP) comprising PEG at both ends was confirmed.

The polymeric nanoparticles prepared according to Example 1 above were injected intramuscularly into animals to confirm the expression rate of mRNA.

Eight polymers (PEG-1A, PEG-1B, PEG-2A, PEG-2B, PEG-3A, PEG-3B, PEG-4A, PEG-4B) prepared in the same manner as in the above manufacturing examples was screened for the in vivo mRNA expression efficiency. The method for forming the polymer-mRNA complex used for in vivo injection to confirm the mRNA expression is as follows. The weight ratio of each polymer was adjusted, and 6 µl of the solution dissolved in a pH 5.0, 25 mM NaOAc buffer was mixed with 6 µl of luciferase mRNA (FLuc) of SEQ ID NO: 1 at a concentration of 1 mg/ml, and then gently vortexed. Thereafter, the mixture was stabilized at 4 °C for 30 minutes, and 38 µl of PBS was added to make a final volume of 50 µl.

In order to confirm in vivo mRNA expression using the composition above, it was injected into the gastrocnemius muscle of the mouse hind leg. Thereafter, mRNA expression was confirmed 24 hours after intramuscular injection. 200 µl of luciferin was injected intraperitoneally into each individual at a concentration of 15 mg/ml. 10 minutes after luciferin injection, mRNA expression was confirmed with an exposure time of 15 seconds.

As a result, as shown in Figure 19, PEG was included at both ends of the polymers prepared in Manufacturing Examples 111 to 220 above, and at this time, the mRNA expression of representative polymers was confirmed. As a result, it was confirmed that the expression rate was equal to or higher than that of free mRNA. In addition, it was confirmed that 1B prepared by Manufacturing Example 2 had poor solubility, but PEG-1B, which had PEG attached to both ends, had improved solubility and mRNA expression was significantly increased. Therefore, it can be seen that the delivery vehicle of the present invention can be effectively used as an mRNA delivery vehicle.

### [Test Example 5]

### Confirmation of in vivo expression retention time of polymeric nanoparticles (PNP) comprising PEG

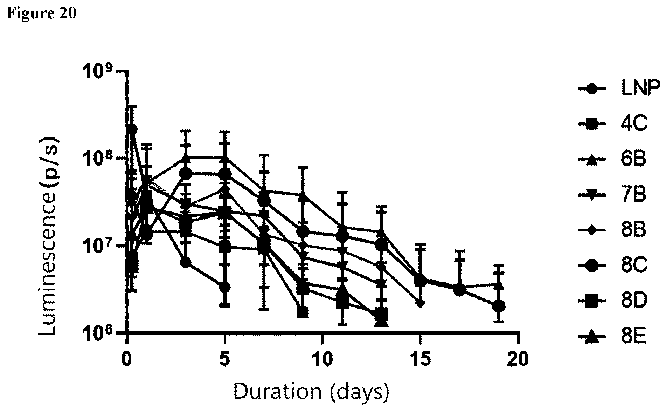

In order to confirm the expression retention time of mRNA, polymer-mRNA complexes were prepared using representative polymers of the manufacturing examples in the same manner as in Test Example 3, and then injected into the hind legs of mice, and measured for up to 20 days. The results are shown in Figure 20.

As shown in Figure 20, it was confirmed that in the case of lipid nanoparticle-mRNA complex (LNP-mRNA), expression reached its peak at 4 hours and then dropped below the baseline value at about 48 hours, whereas in the case of all polymeric nanoparticle-mRNA complexes used in the test, the expression time was maintained longer than that of lipid nanoparticle-mRNA complexes. In particular, it was confirmed that in the case of 6B and 8C polymer complexes, mRNA expression was maintained for more than 10 days, which means that the administration frequency can be drastically reduced compared to the existing method in the development of a gene therapeutic agent.

## Claims

1. A polymer suitable for intracellular nucleic acid delivery, comprising a repeating unit of Formula 1 below: 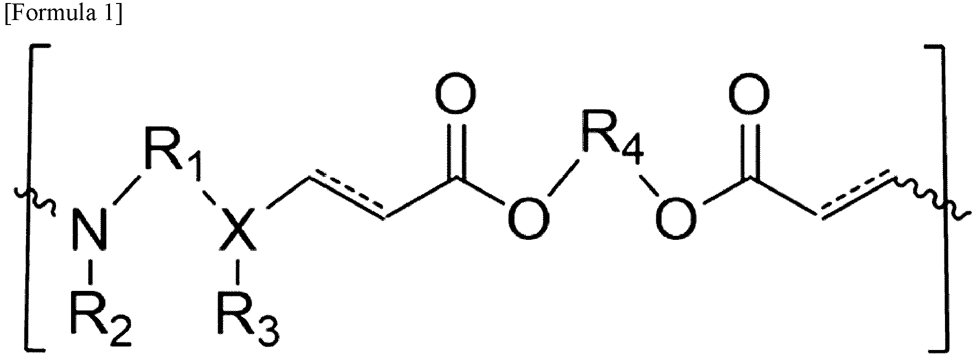 in the formula,
R1 is a bond or a C1-6 alkylene,
R2 and R3 are each independently (i) hydrogen, (ii) C1-6 alkyl which may be substituted with alkylamine or hydroxy, (iii) a 5- or 6-membered carbocycle or heterocycle, or (iv) C1-6 alkyl substituted with a 5- or 6-membered carbocycle or heterocycle,
said R2 and R3 may be linked together to form a C1-6 alkylene,
said 5- or 6-membered carbocycle or heterocycle may be substituted with C1-3 alkyl or C1-3 alkoxyacyl,
X is a nitrogen atom or a bond, and when X is a bond, said R3 is absent,
R4 is a linear or branched C1-15 alkylene, 1 to 5 carbon atoms of said alkylene may be replaced by oxygen or sulfur, and
- - - - - represents a single bond or a double bond.

2. A polymer suitable for intracellular nucleic acid delivery, comprising a repeating unit of Formula 2 below: 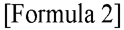 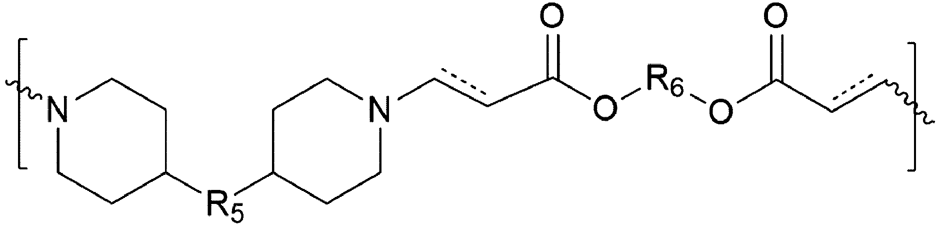 in the formula,
R5 is a C1-6 alkylene,
R6 is a linear or branched C1-15 alkylene, 1 to 5 carbon atoms of said alkylene may be replaced by oxygen or sulfur, and
- - - - - represents a single bond or a double bond.

3. The polymer according to claim 1, wherein the repeating unit of Formula 1 is formed by a reaction between any one of the monomers of Formulas 3 to 13 below and any one of the monomers of Formulas 14 to 23 below.
| Formula | Structure |
|---|---|
| 3 | 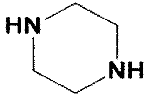 |
| 4 | 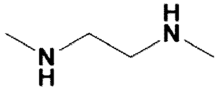 |
| 5 | 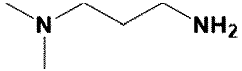 |
| 6 | 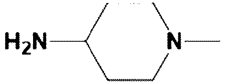 |
| 7 | 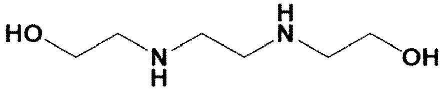 |
| 8 | 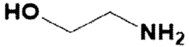 |
| 9 | 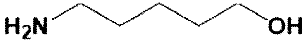 |
| 10 | 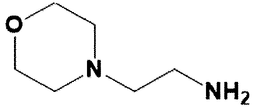 |
| 11 | 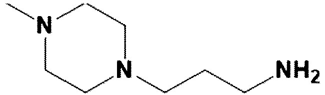 |
| 12 | 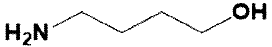 |
| 13 | 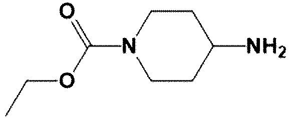 |
| 14 | 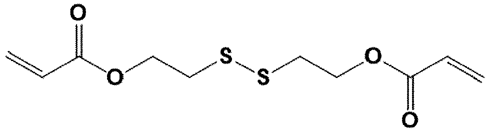 |
| 15 | 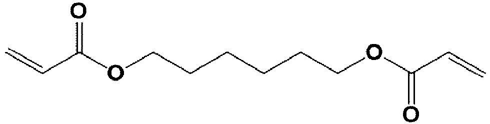 |
| 16 | 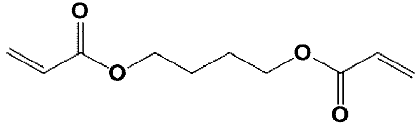 |
| 17 | 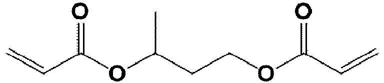 |
| 18 | 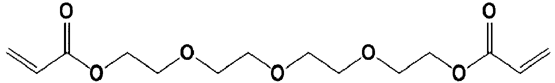 |
| 19 | 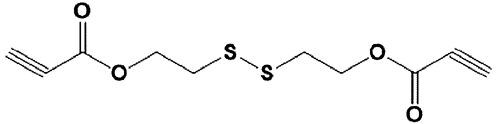 |
| 20 | 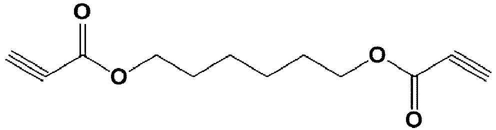 |
| 21 | 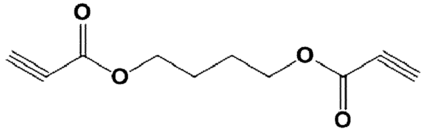 |
| 22 | 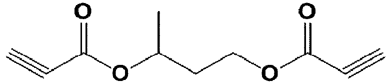 |
| 23 | 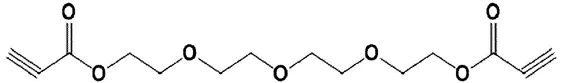 |

4. The polymer according to claim 2, wherein the repeating unit of Formula 2 is formed by a reaction between a monomer of Formula 24 below and any one of the monomers of Formulas 14 to 23 below.
| Formula | Structure |
|---|---|
| 14 | 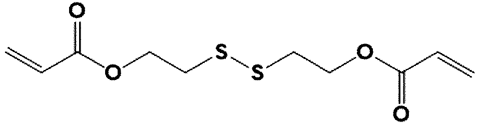 |
| 15 | 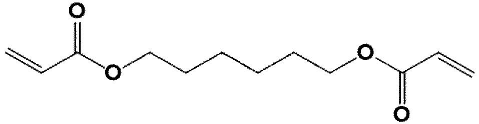 |
| 16 | 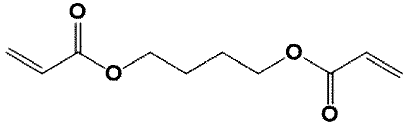 |
| 17 | 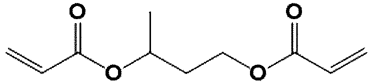 |
| 18 | 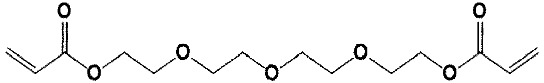 |
| 19 | 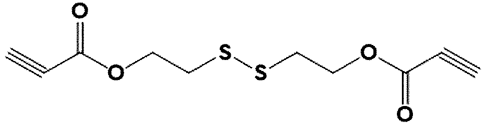 |
| 20 | 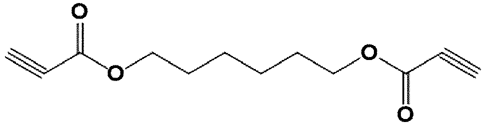 |
| 21 | 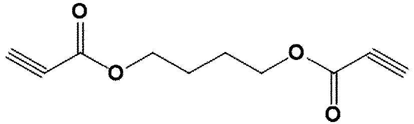 |
| 22 | 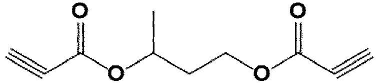 |
| 23 | 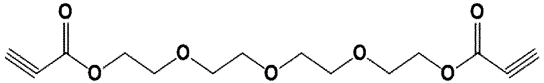 |
| 24 | 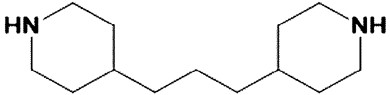 |

5. A polymer comprising a repeating unit selected from the group consisting of repeating units of the chemical structures set forth in the table below.

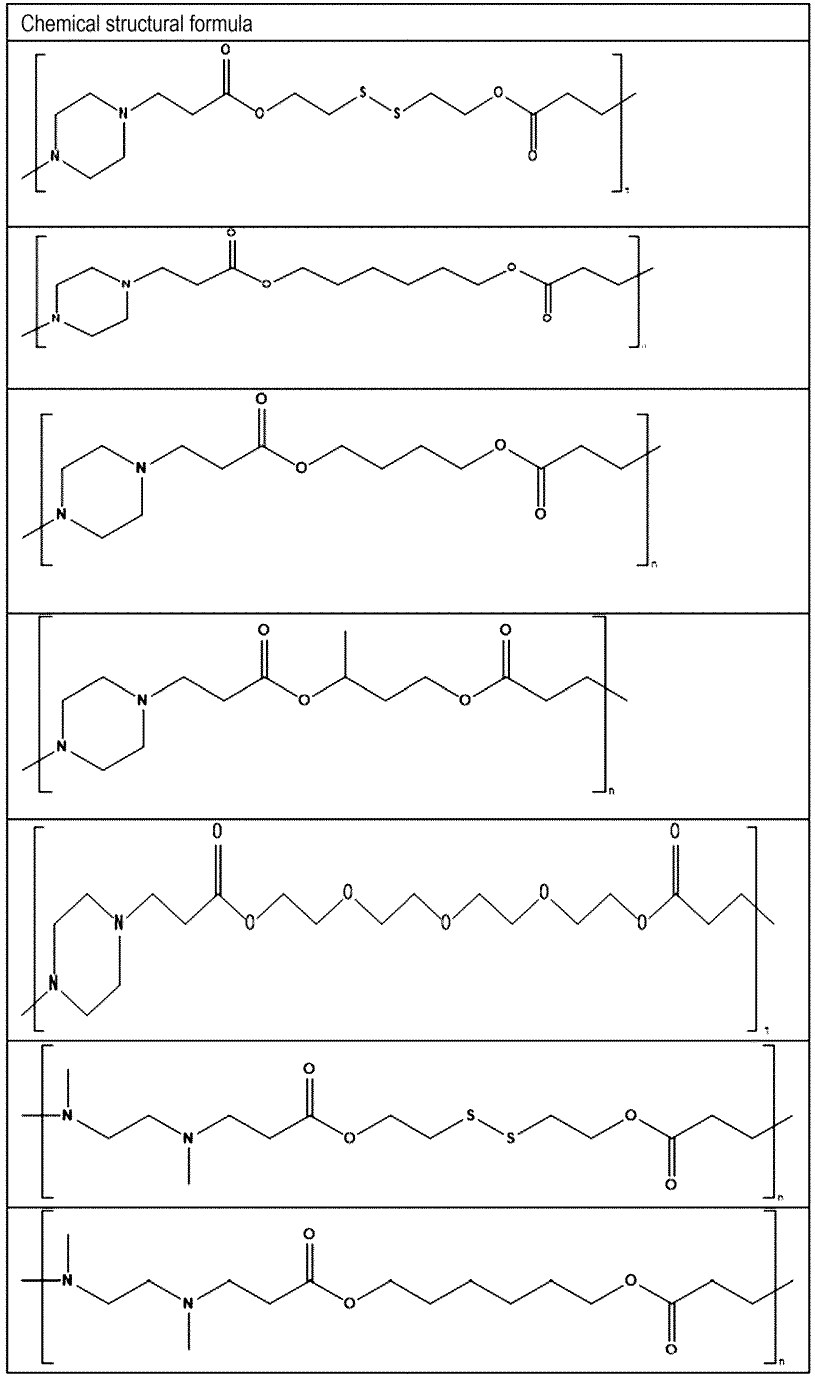

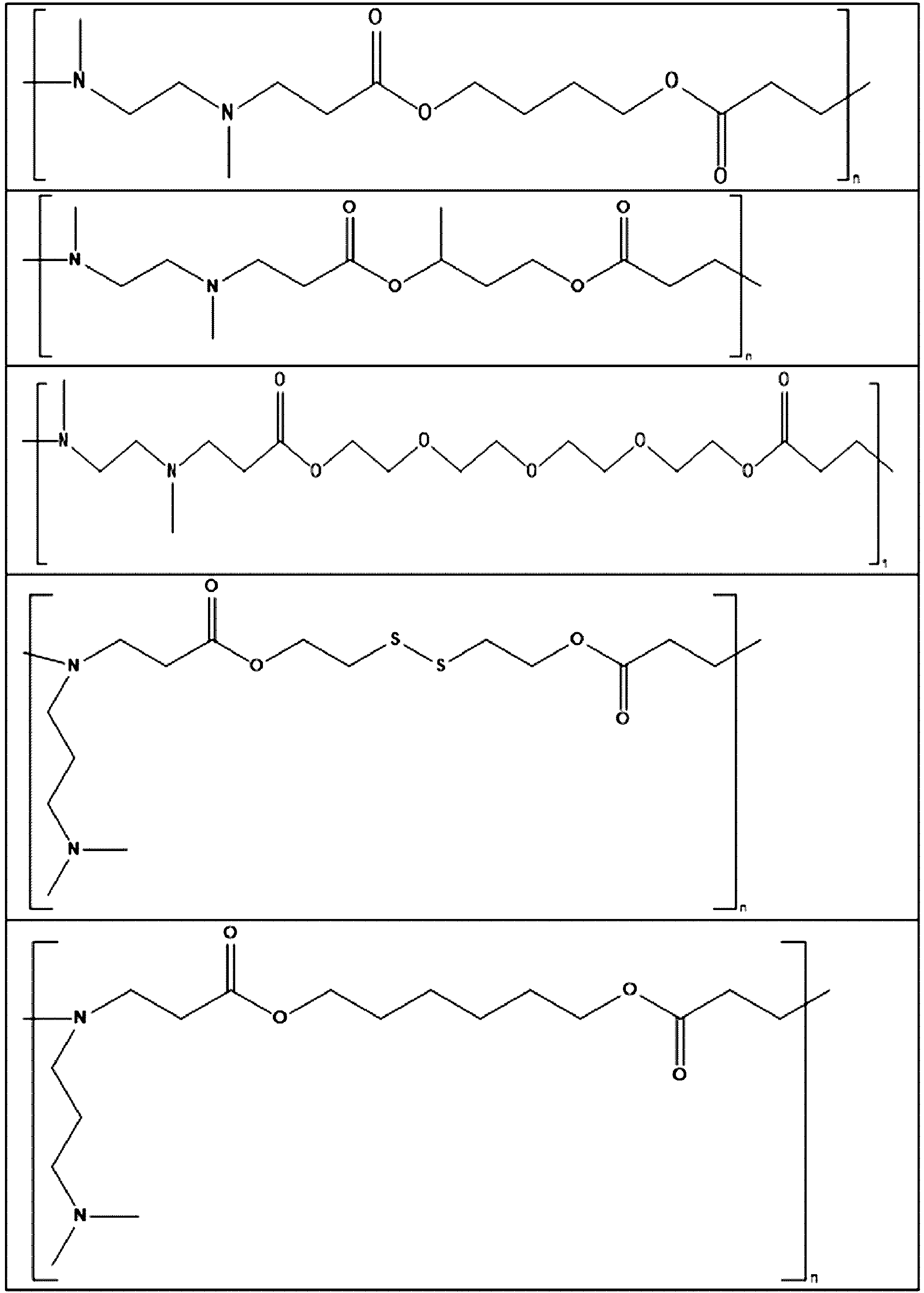

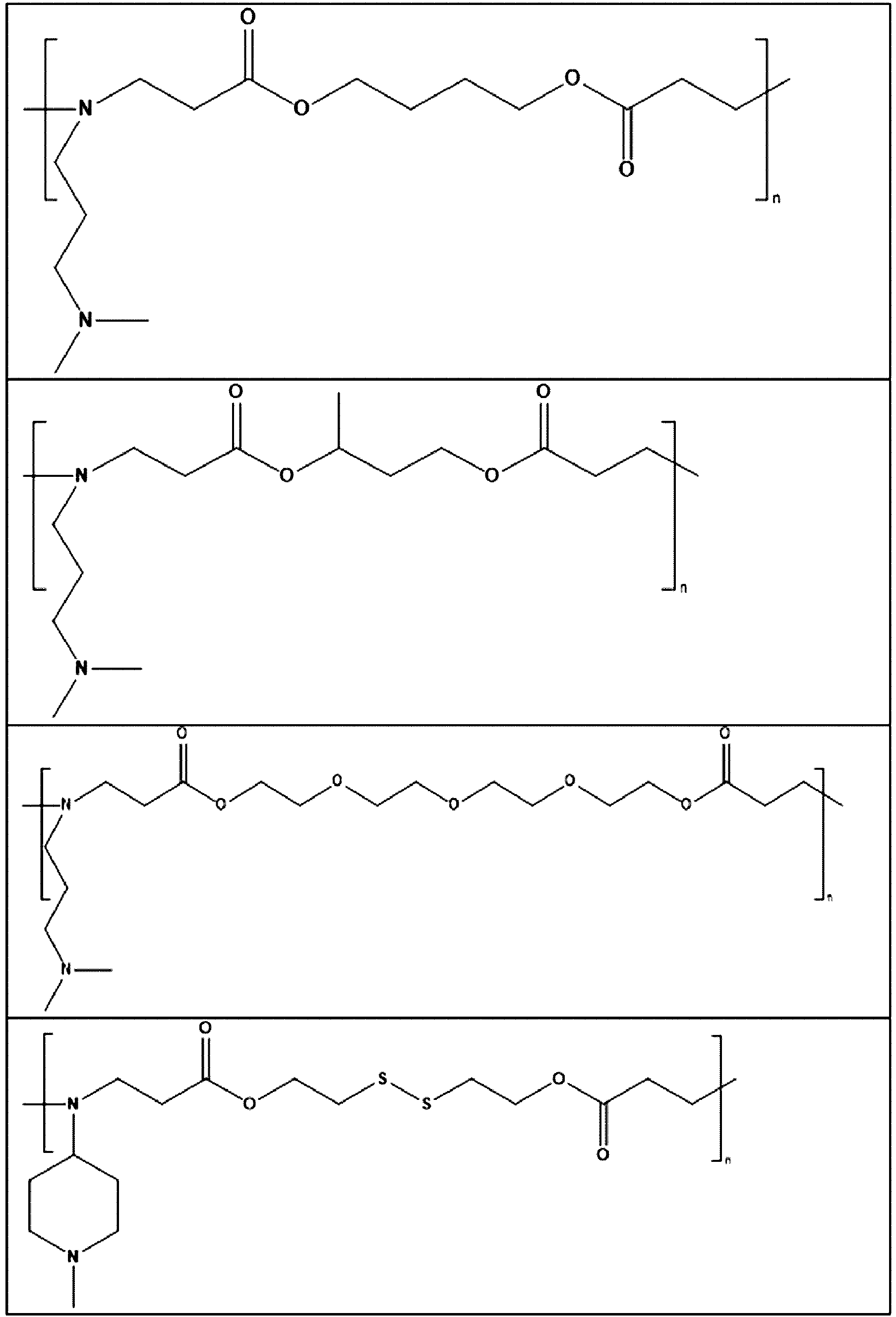

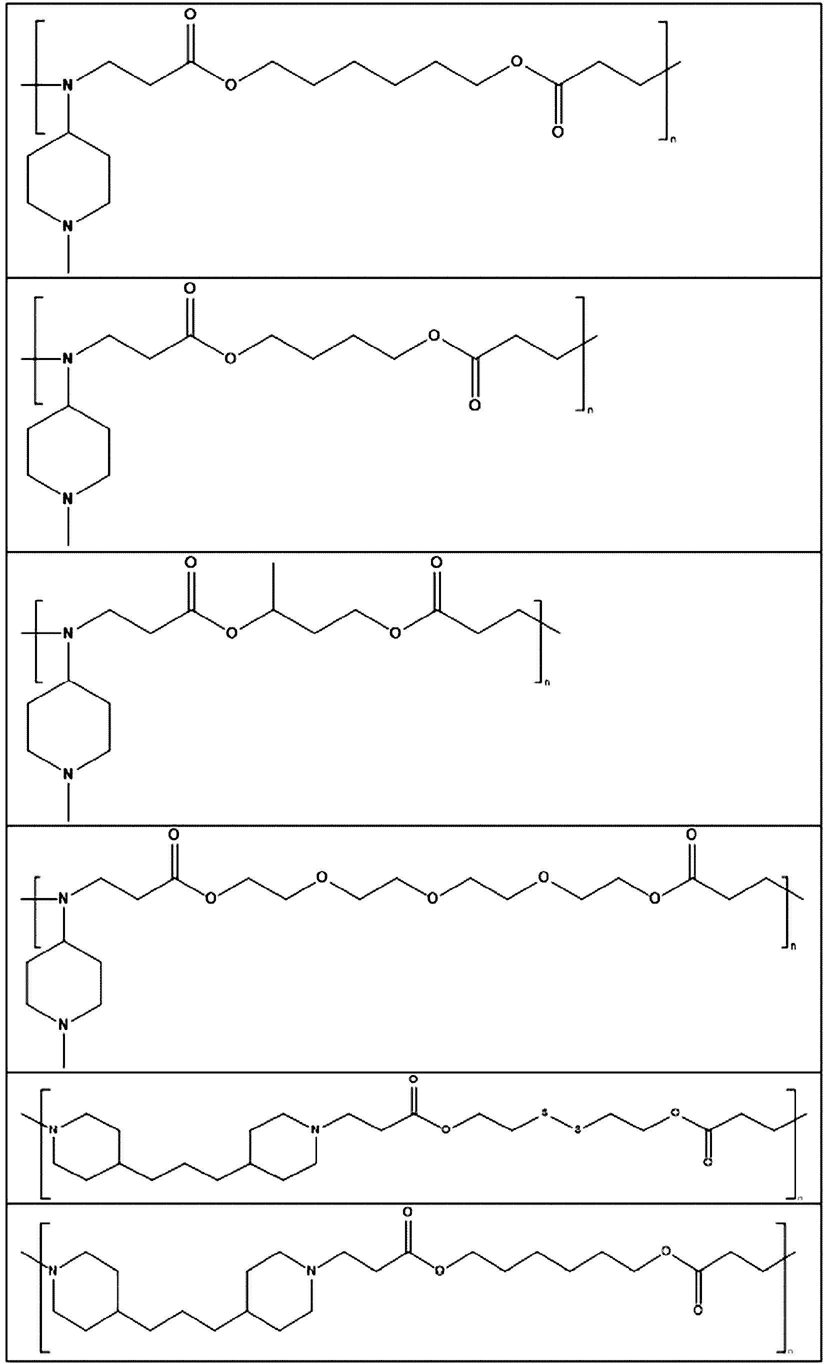

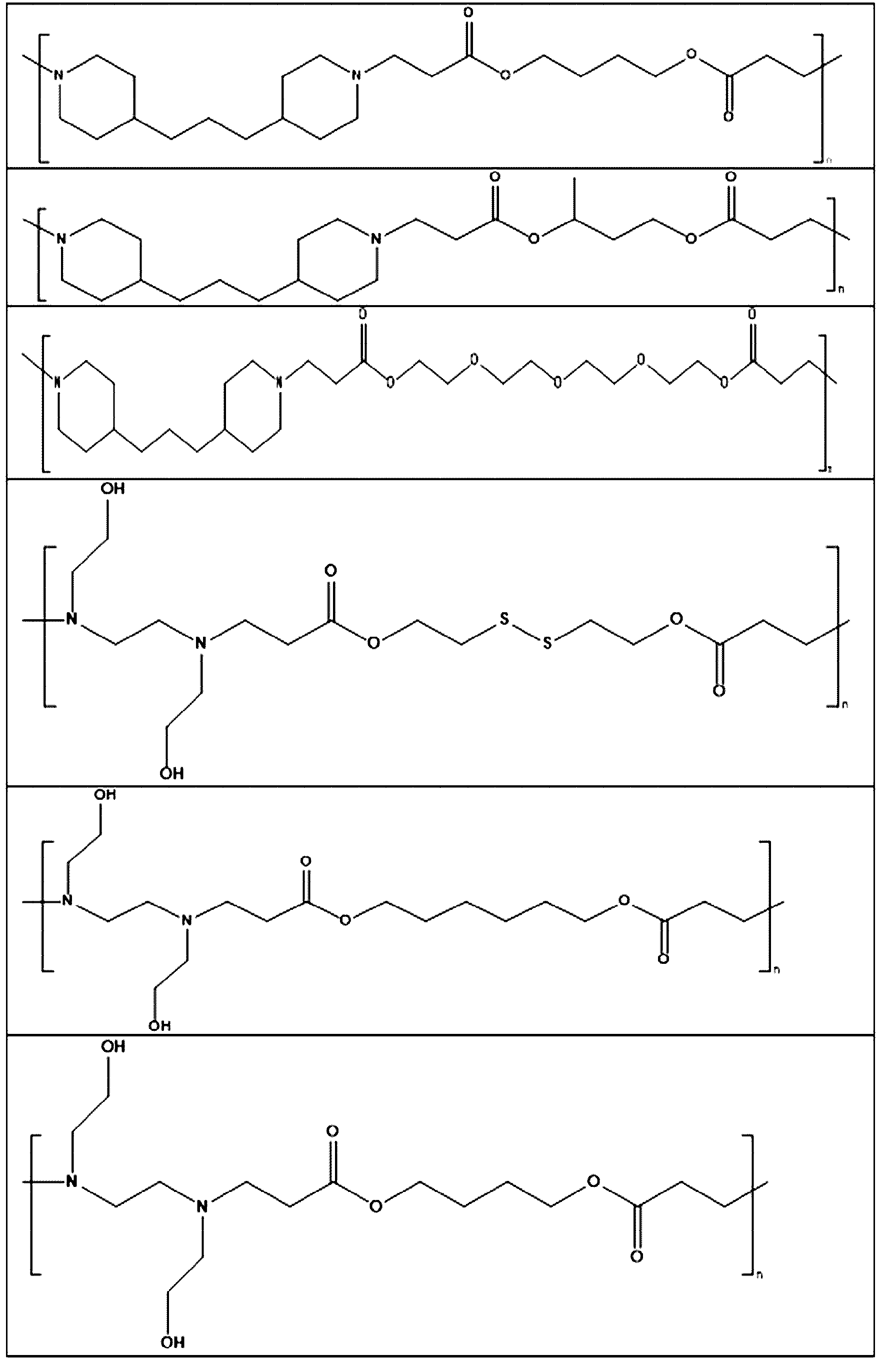

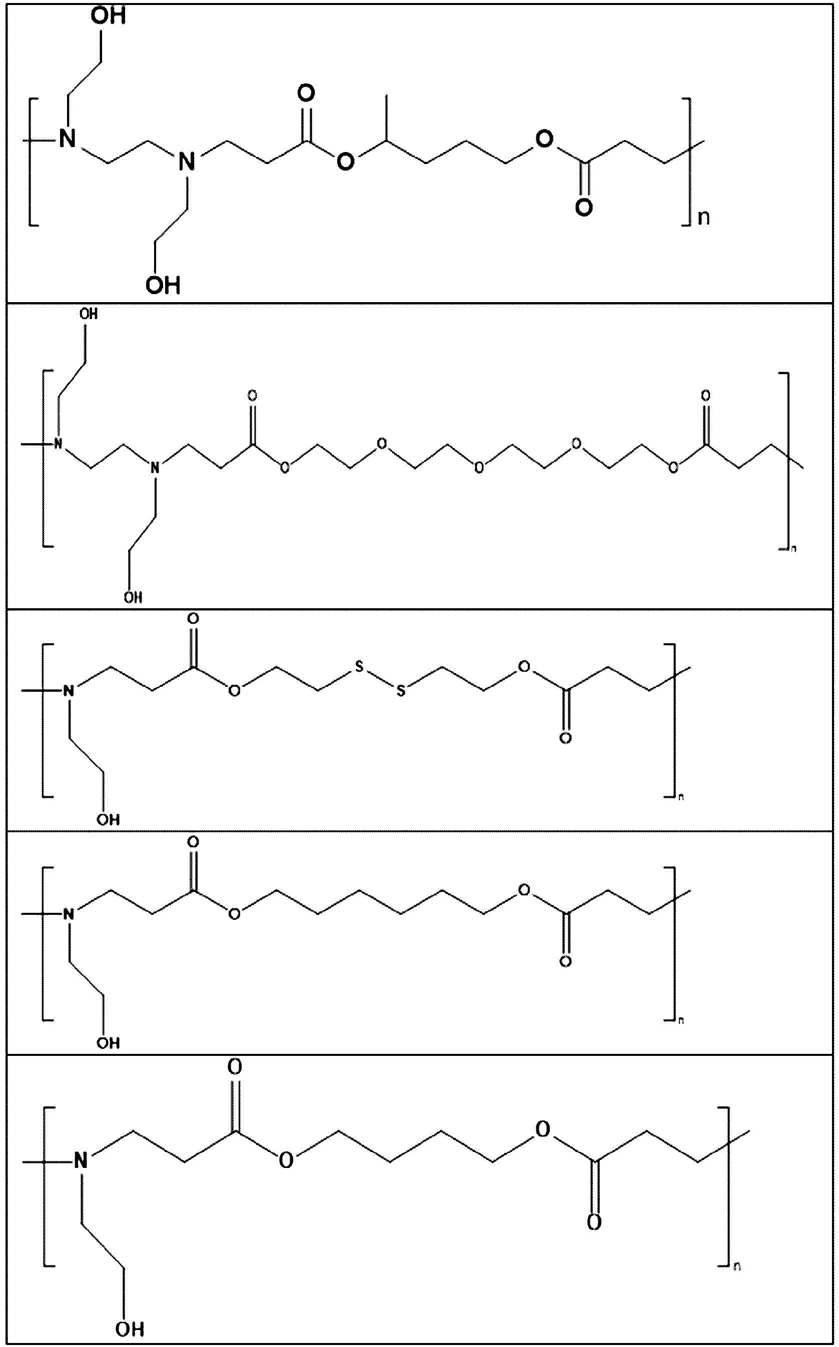

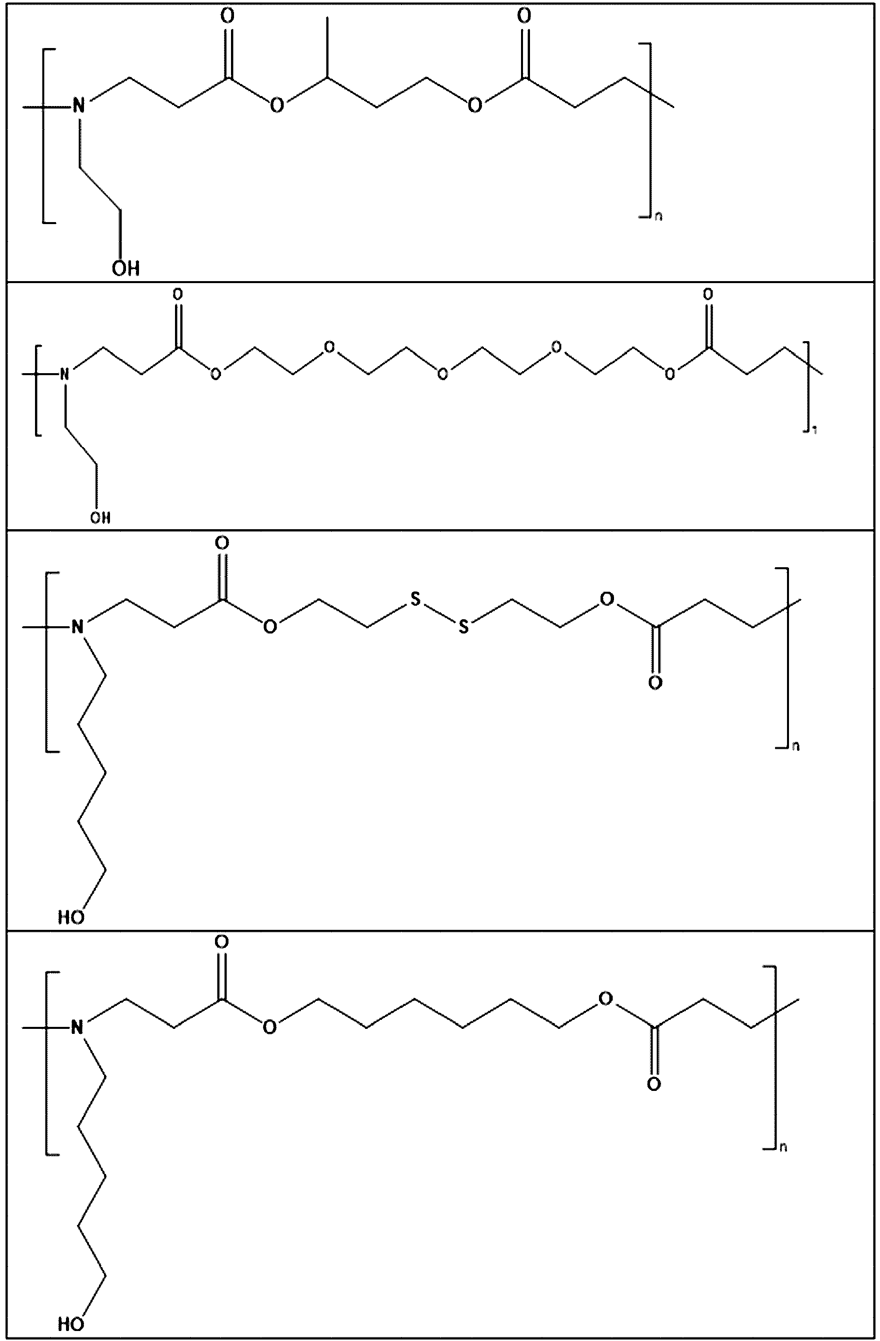

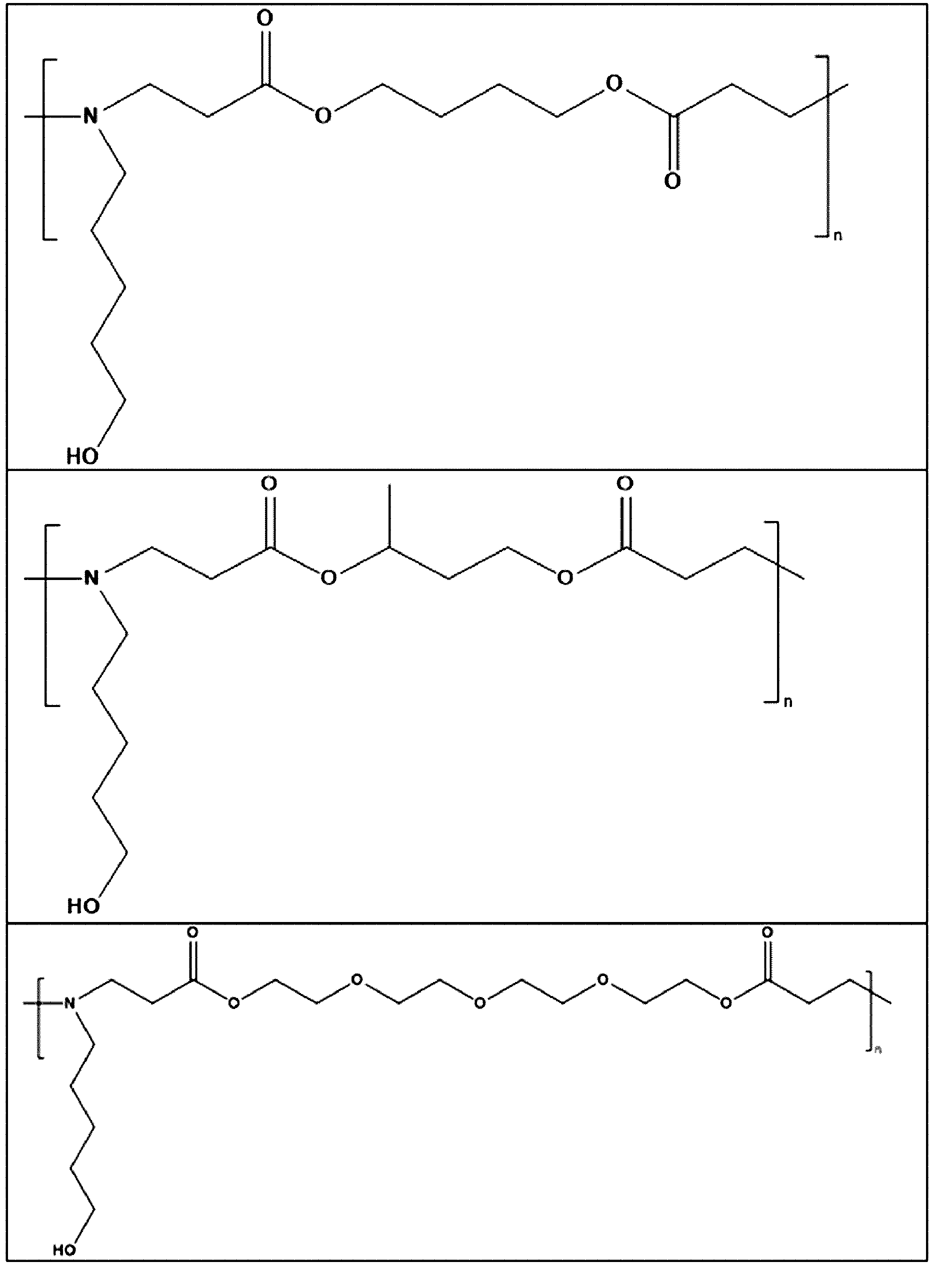

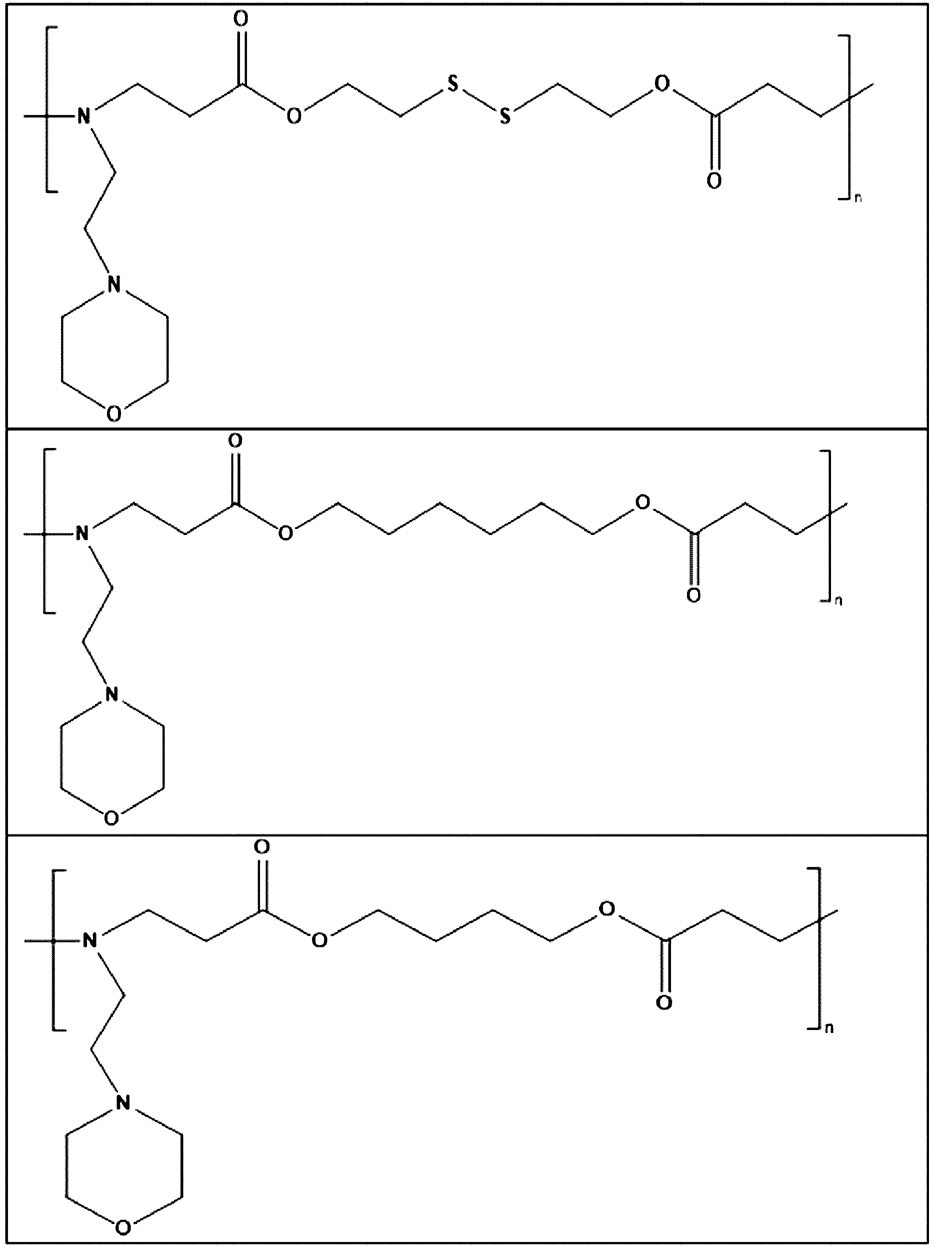

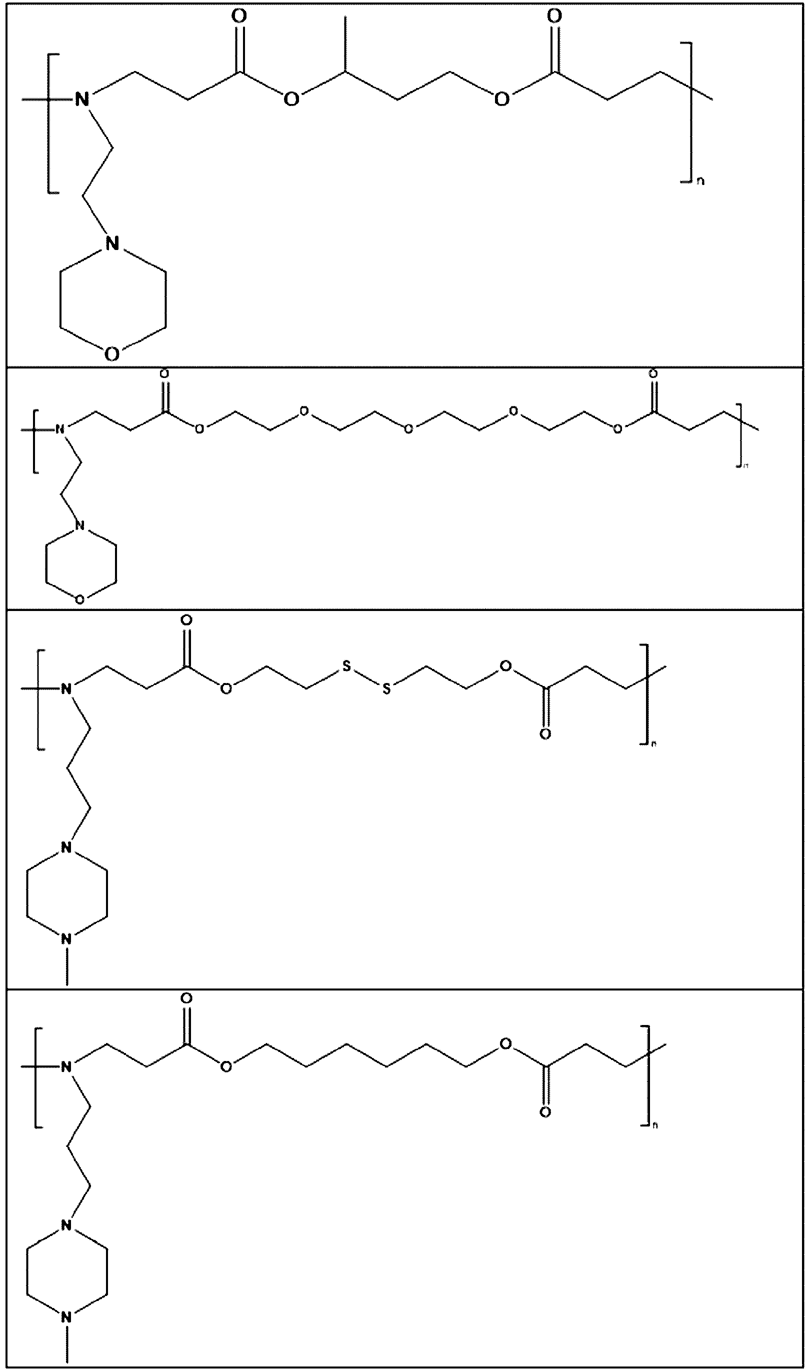

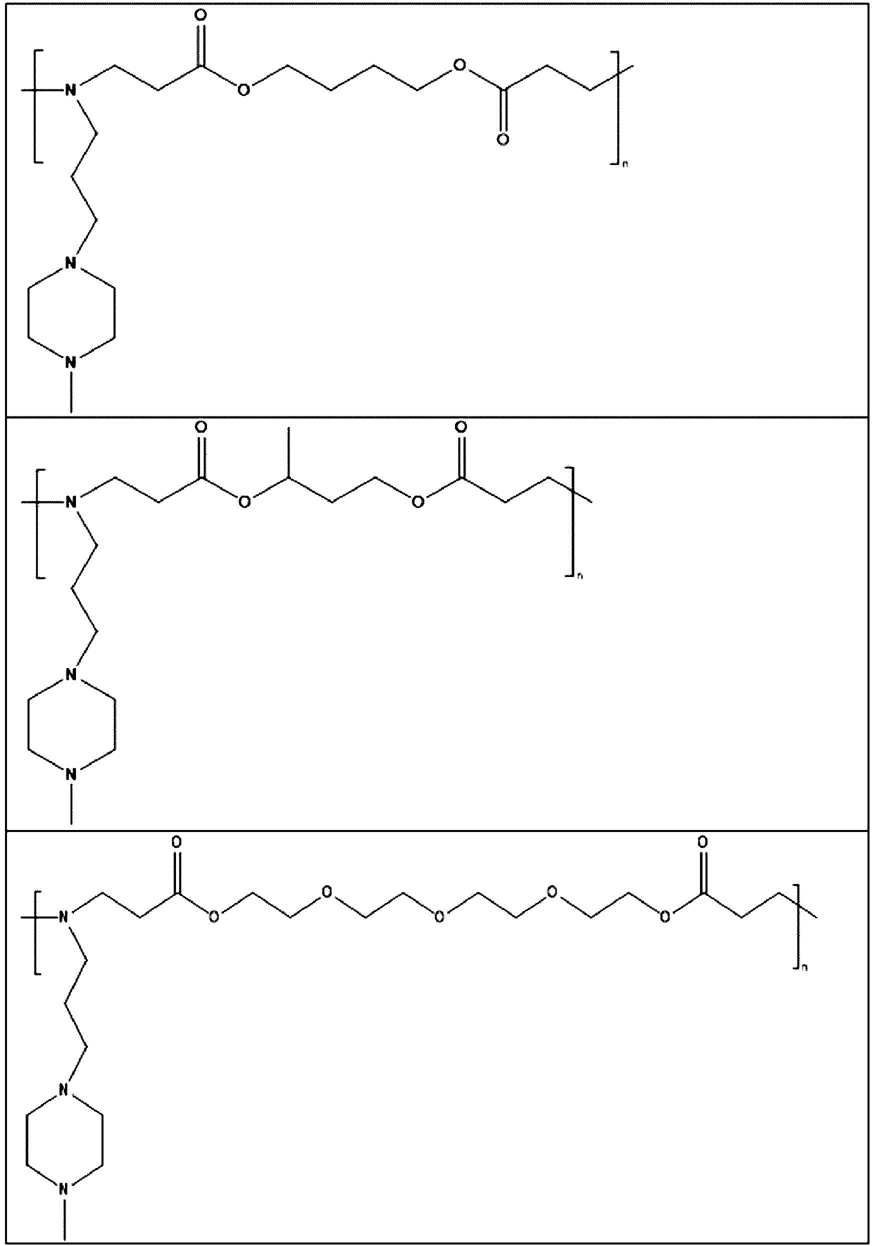

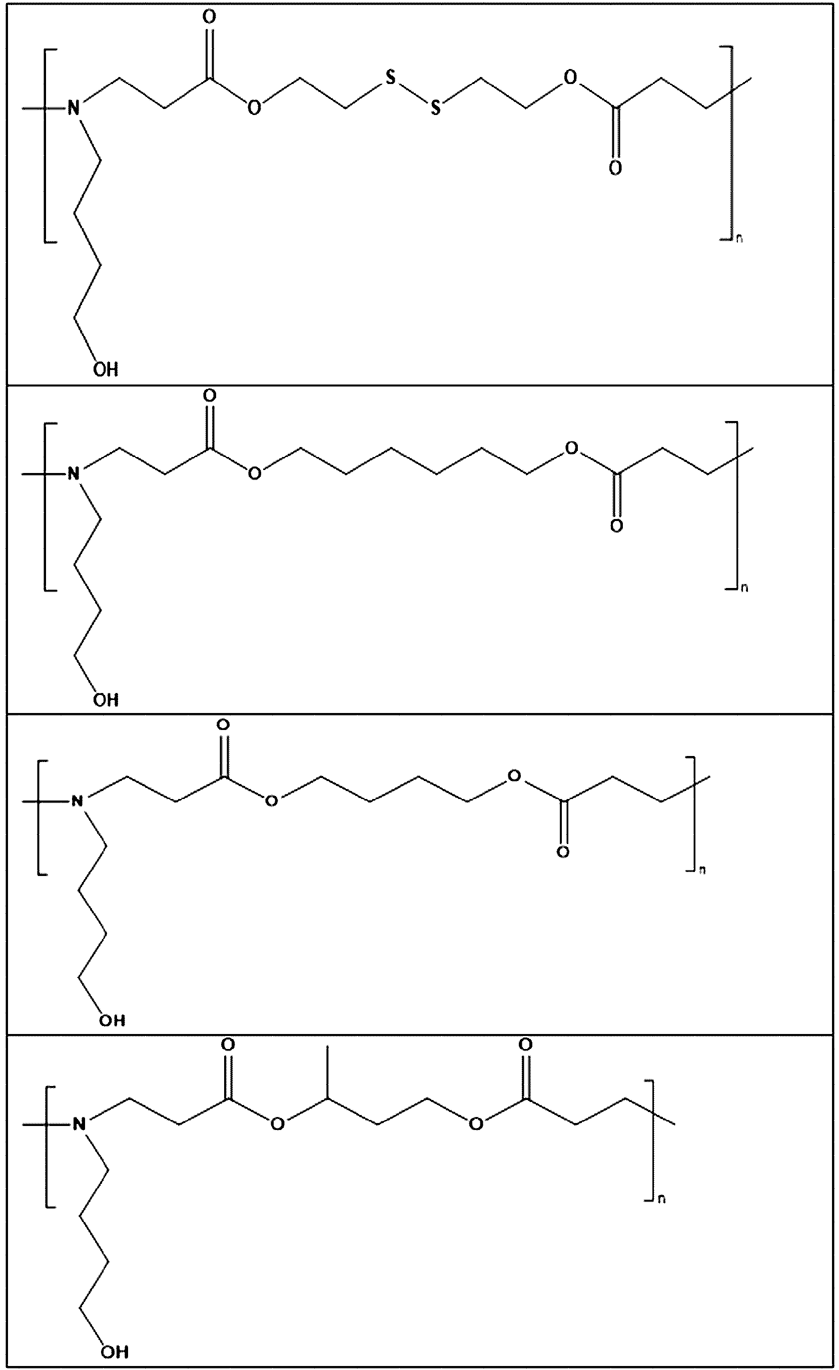

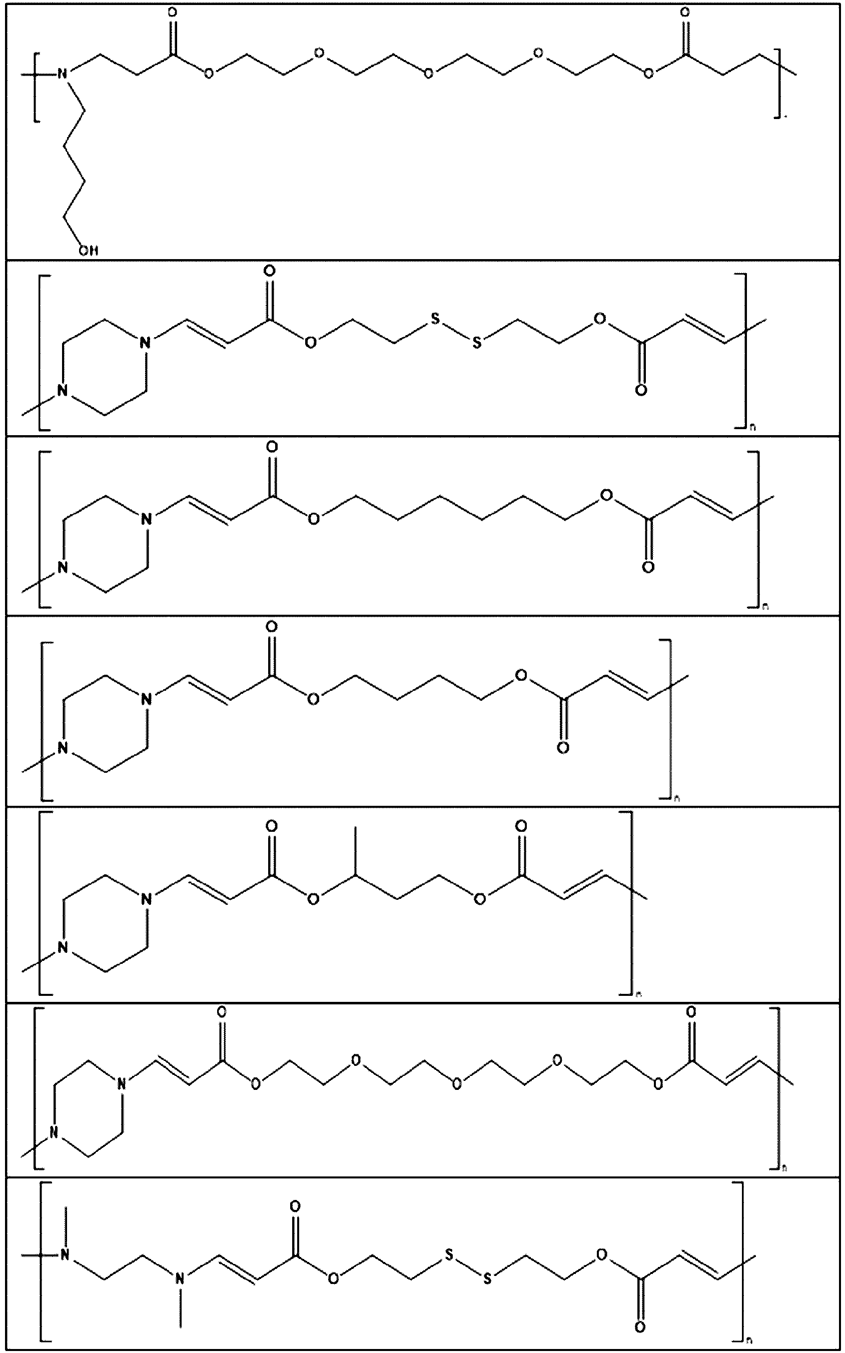

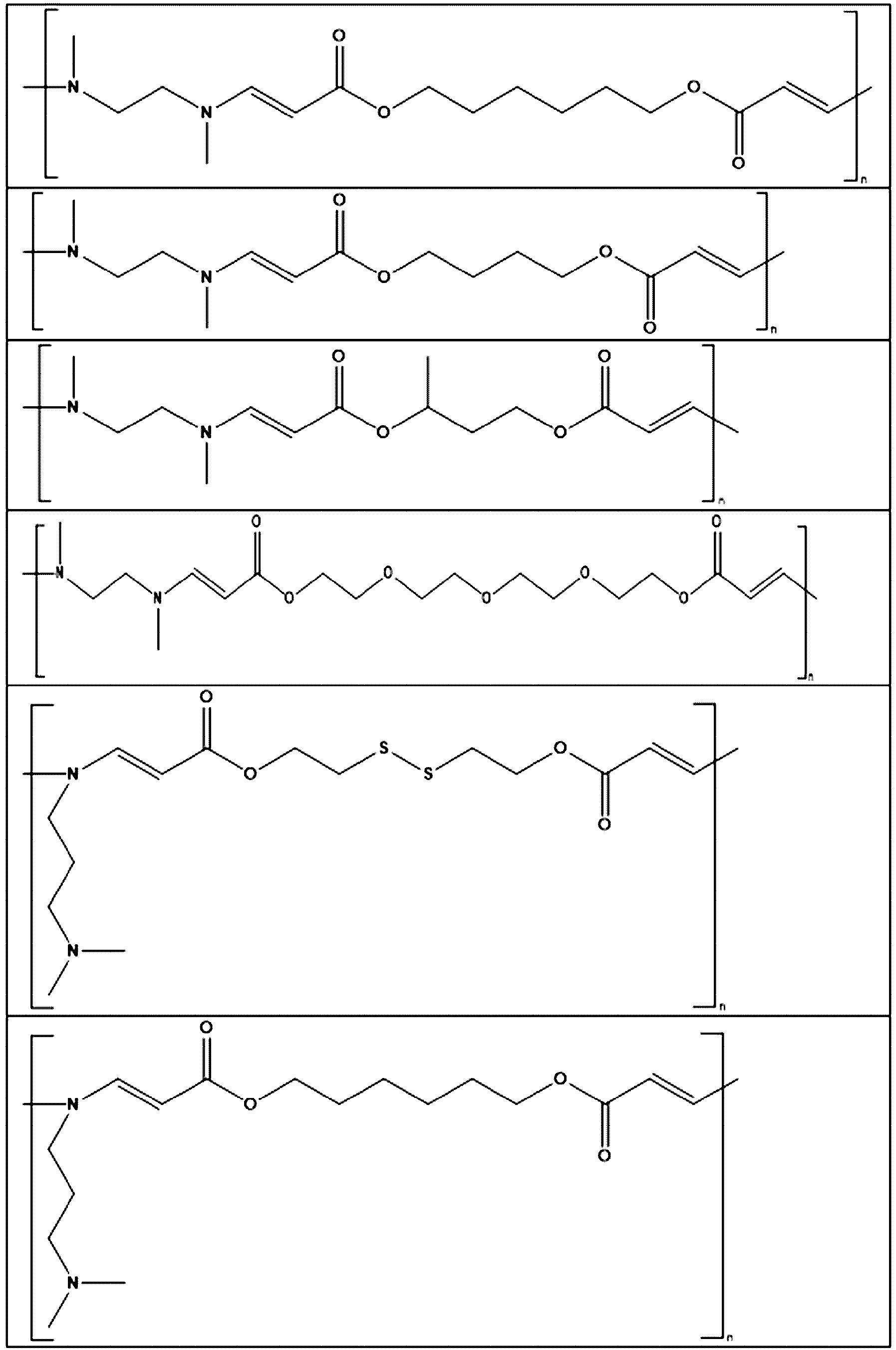

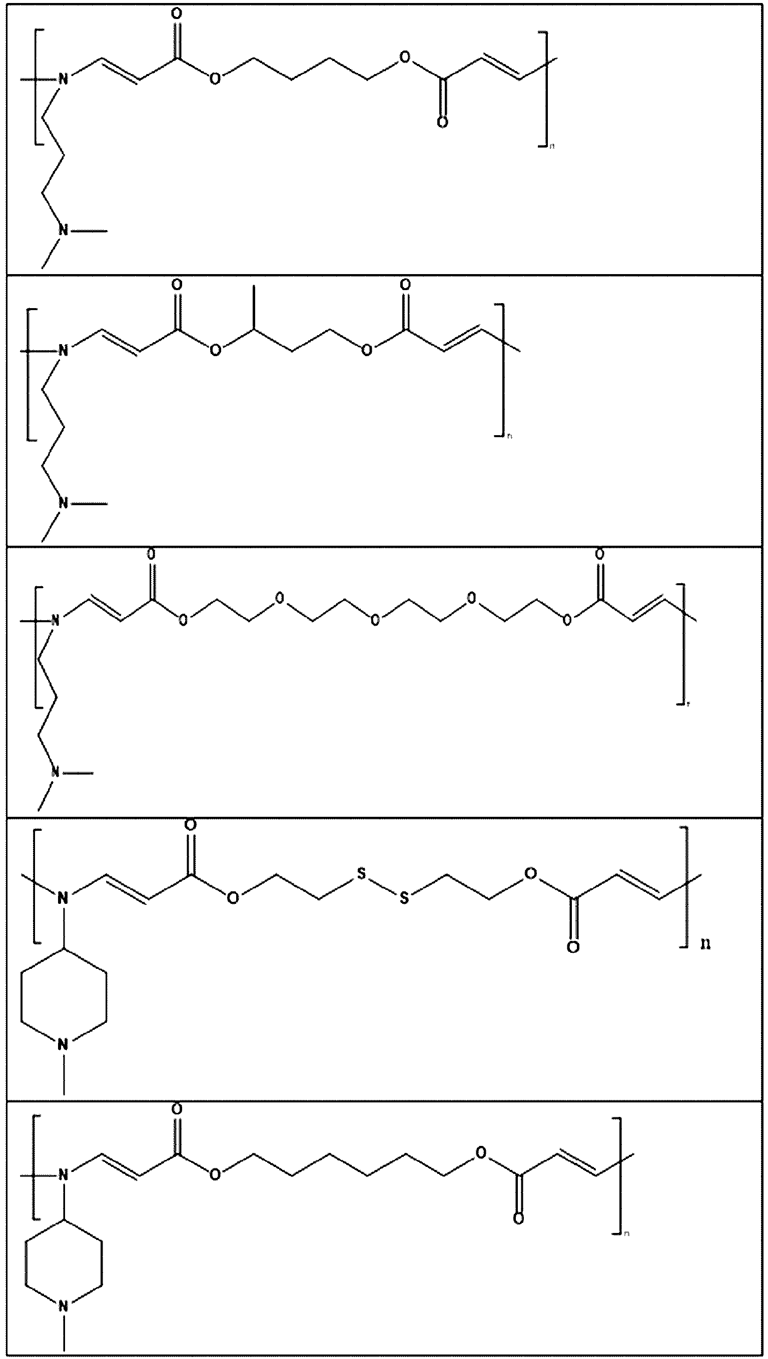

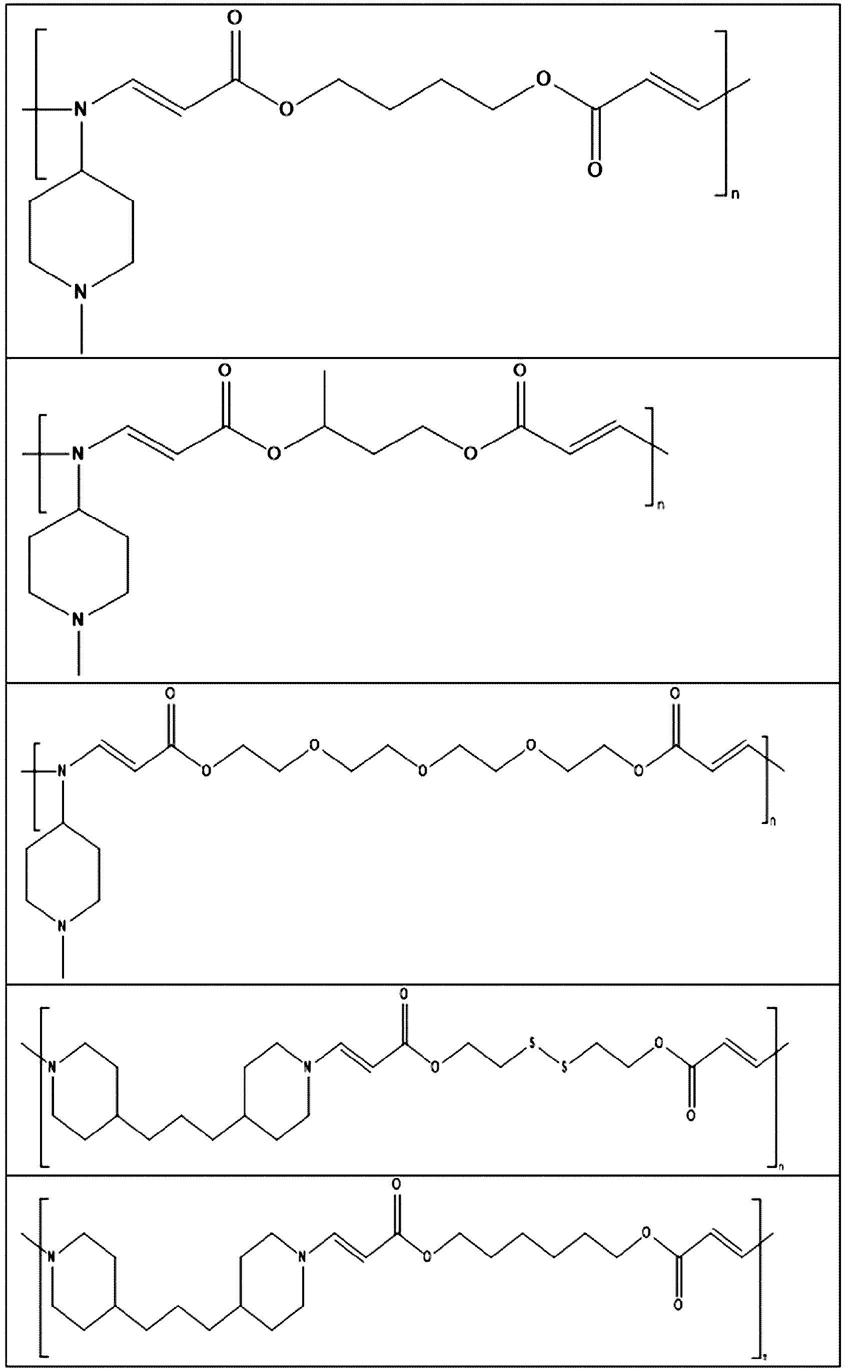

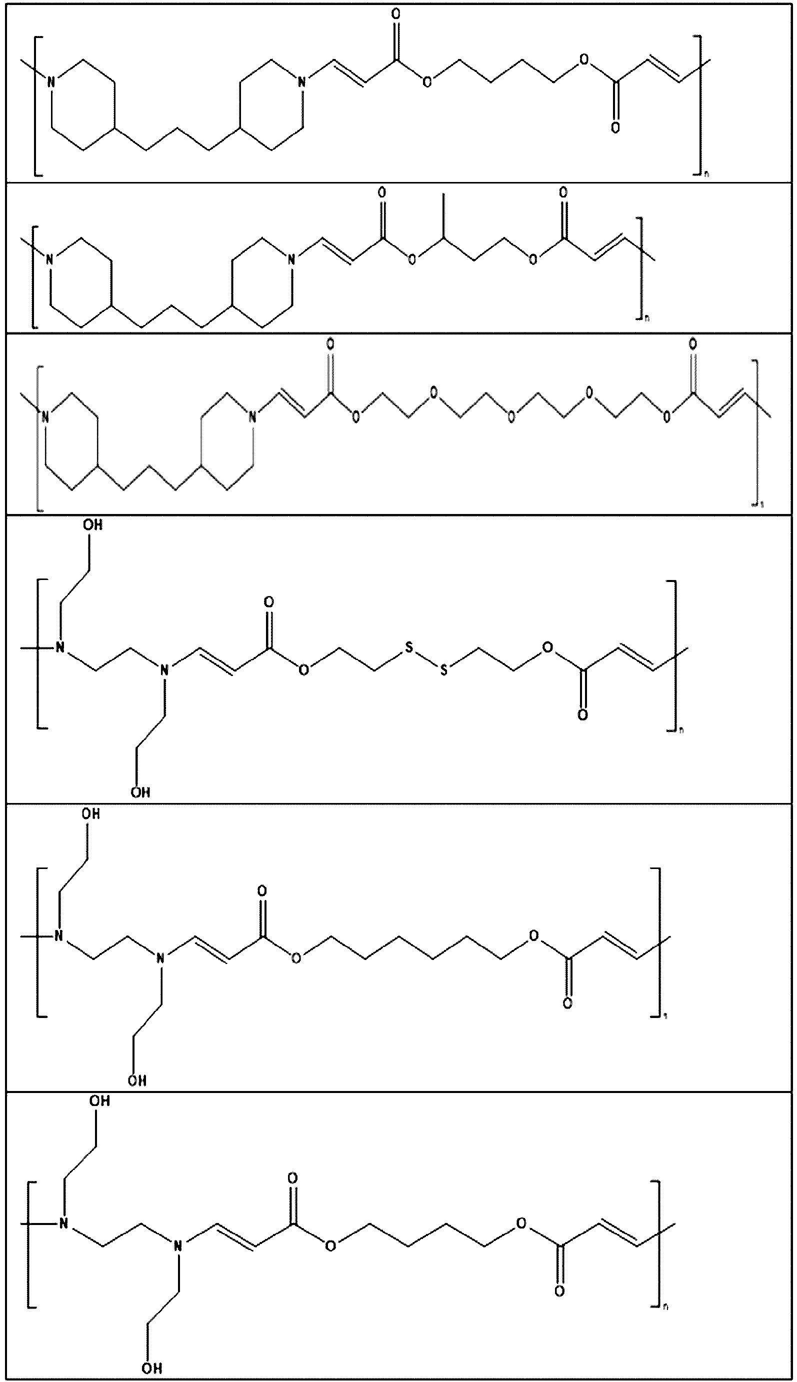

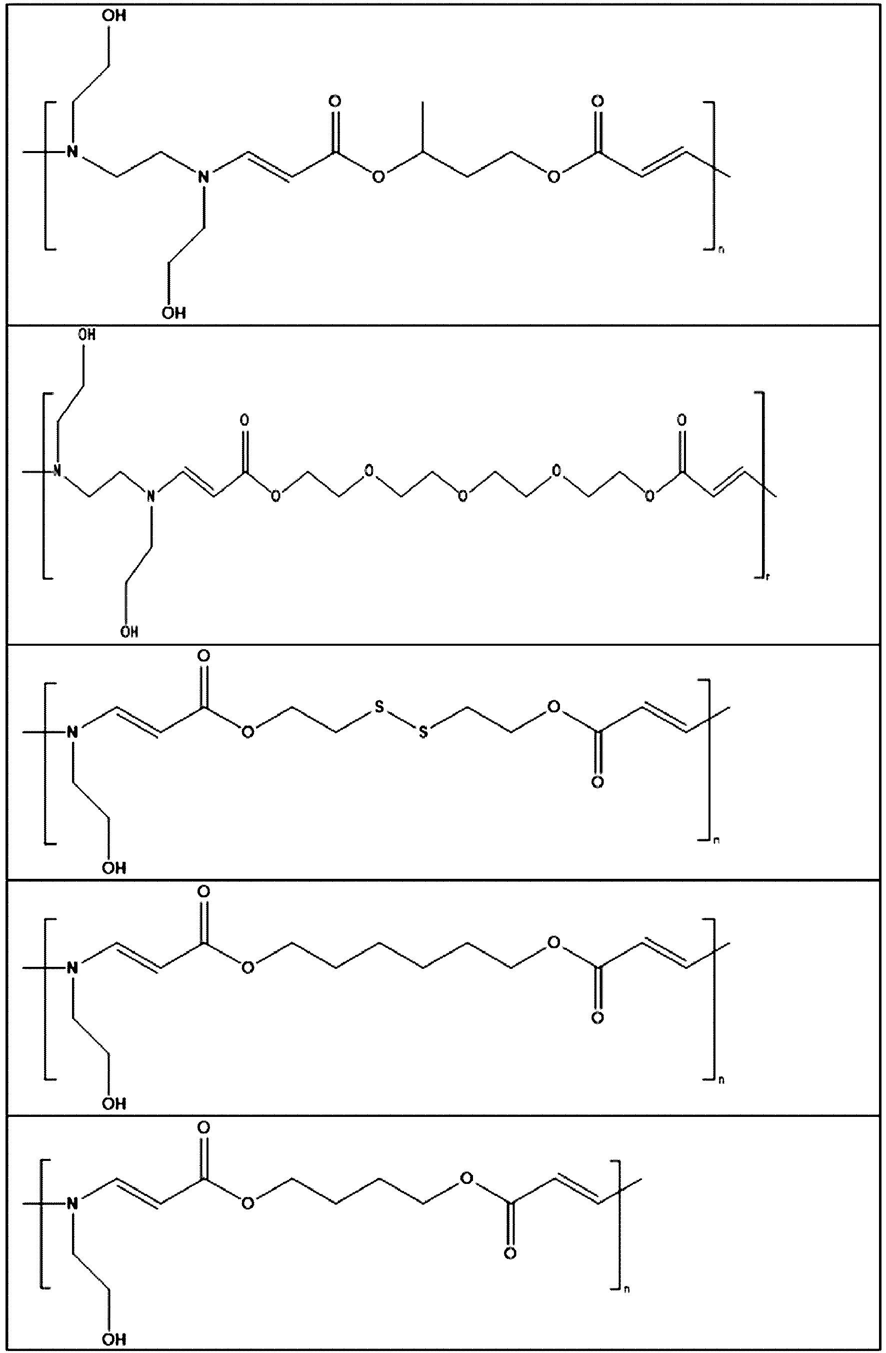

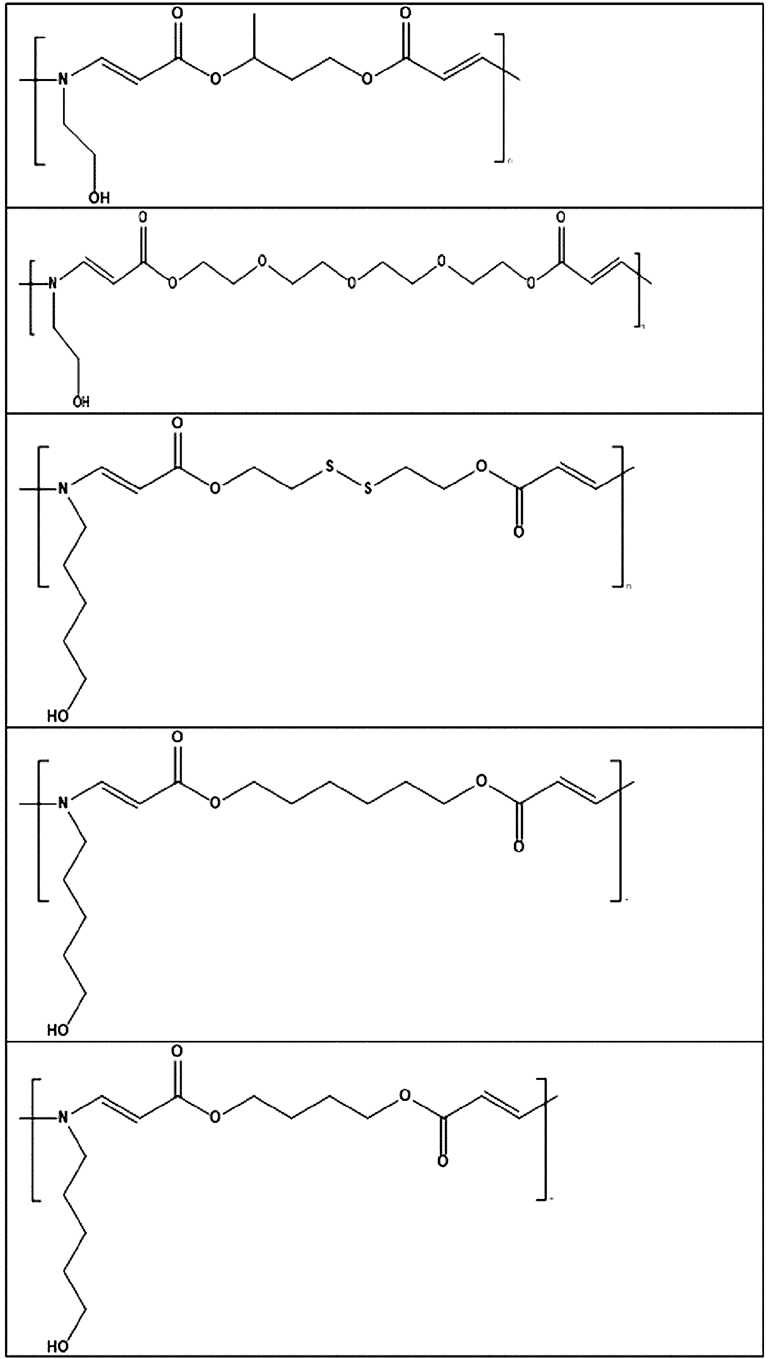

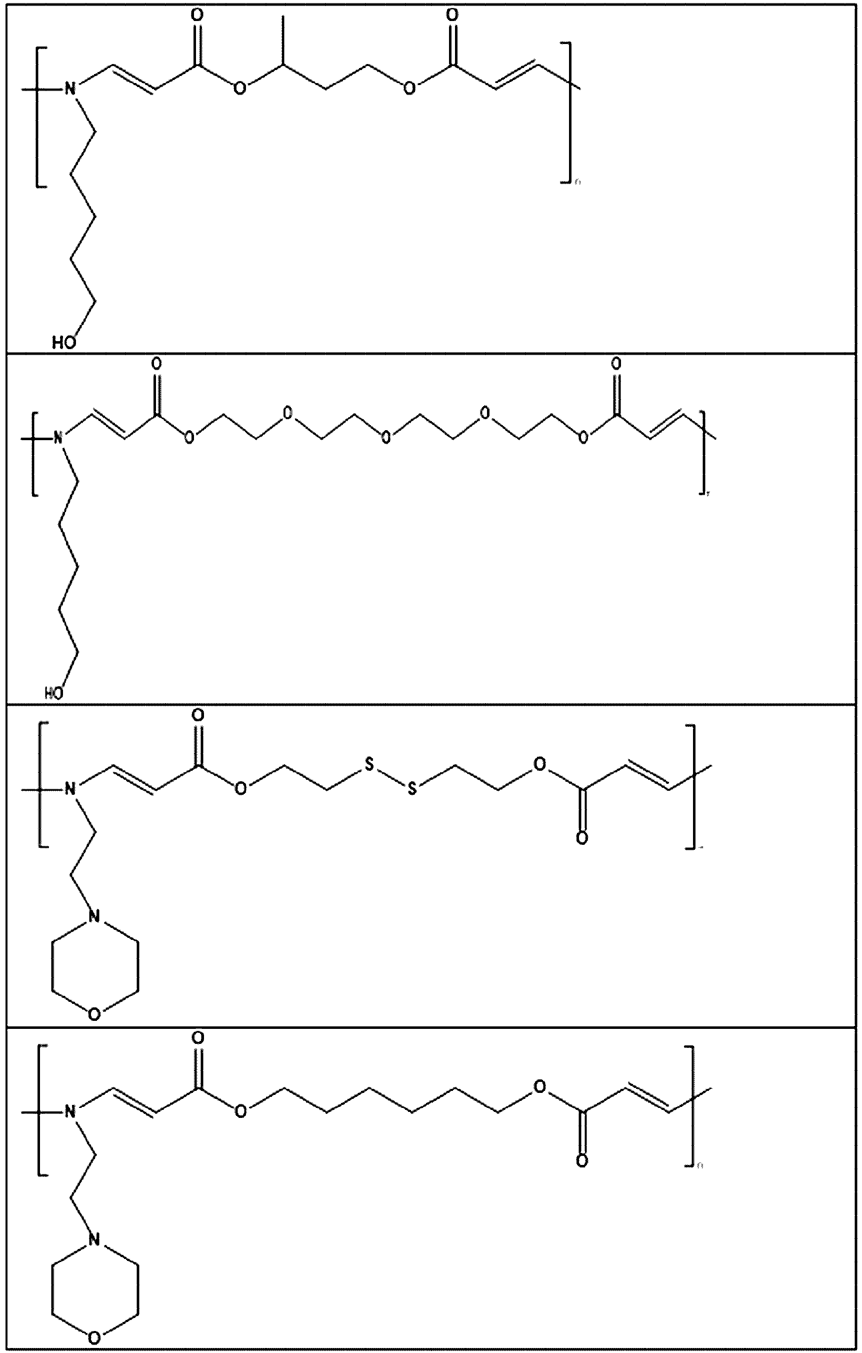

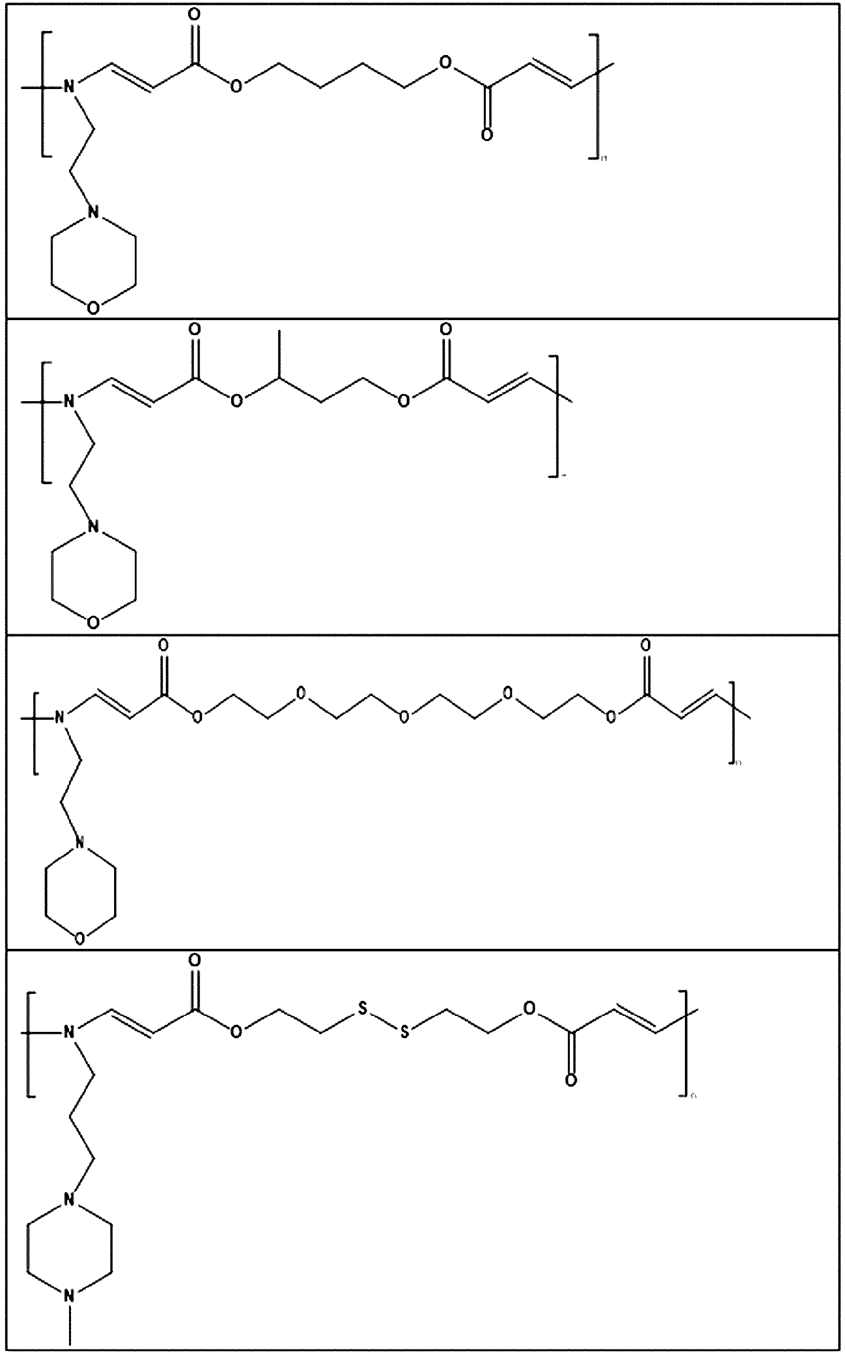

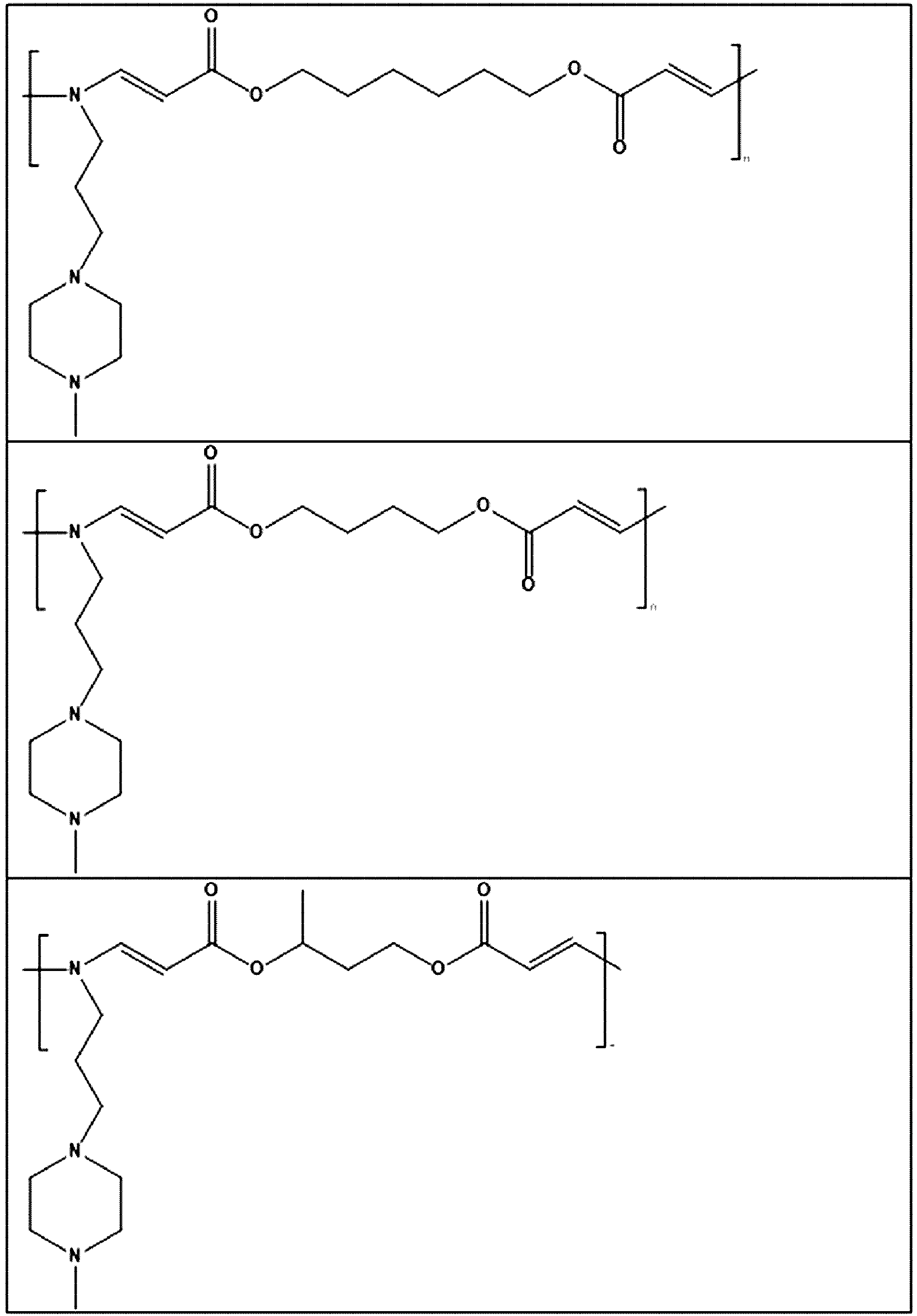

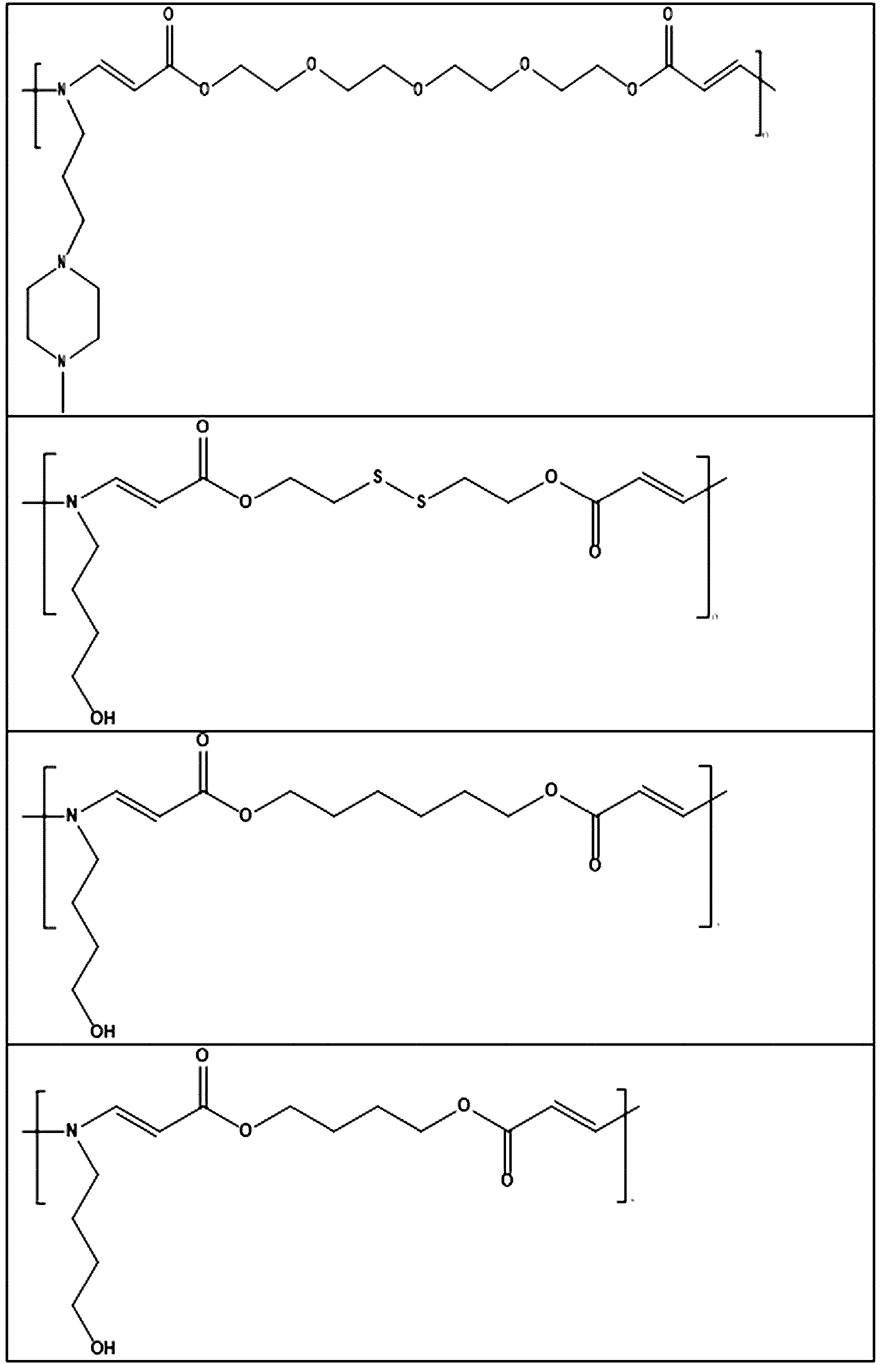

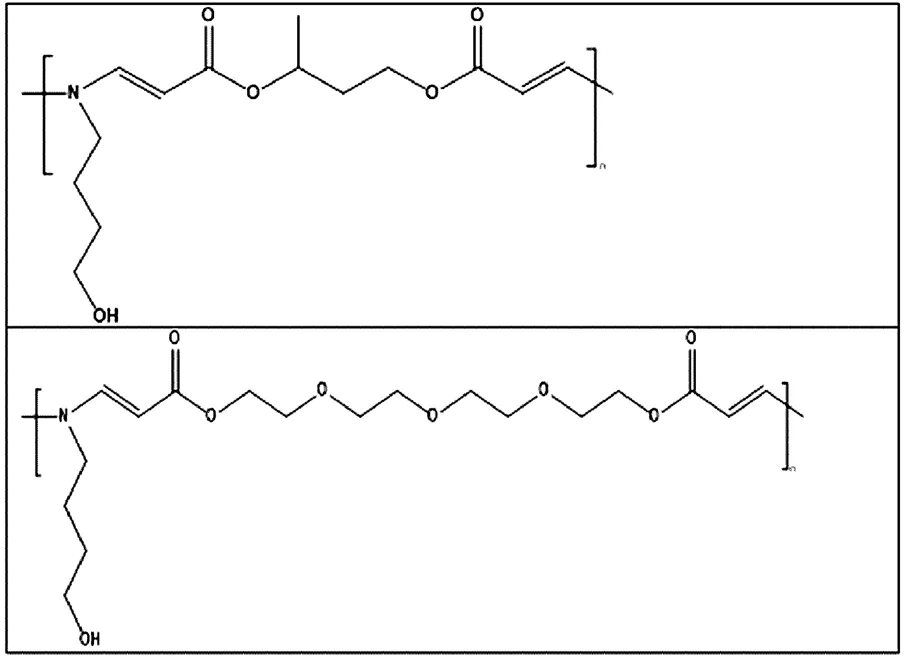

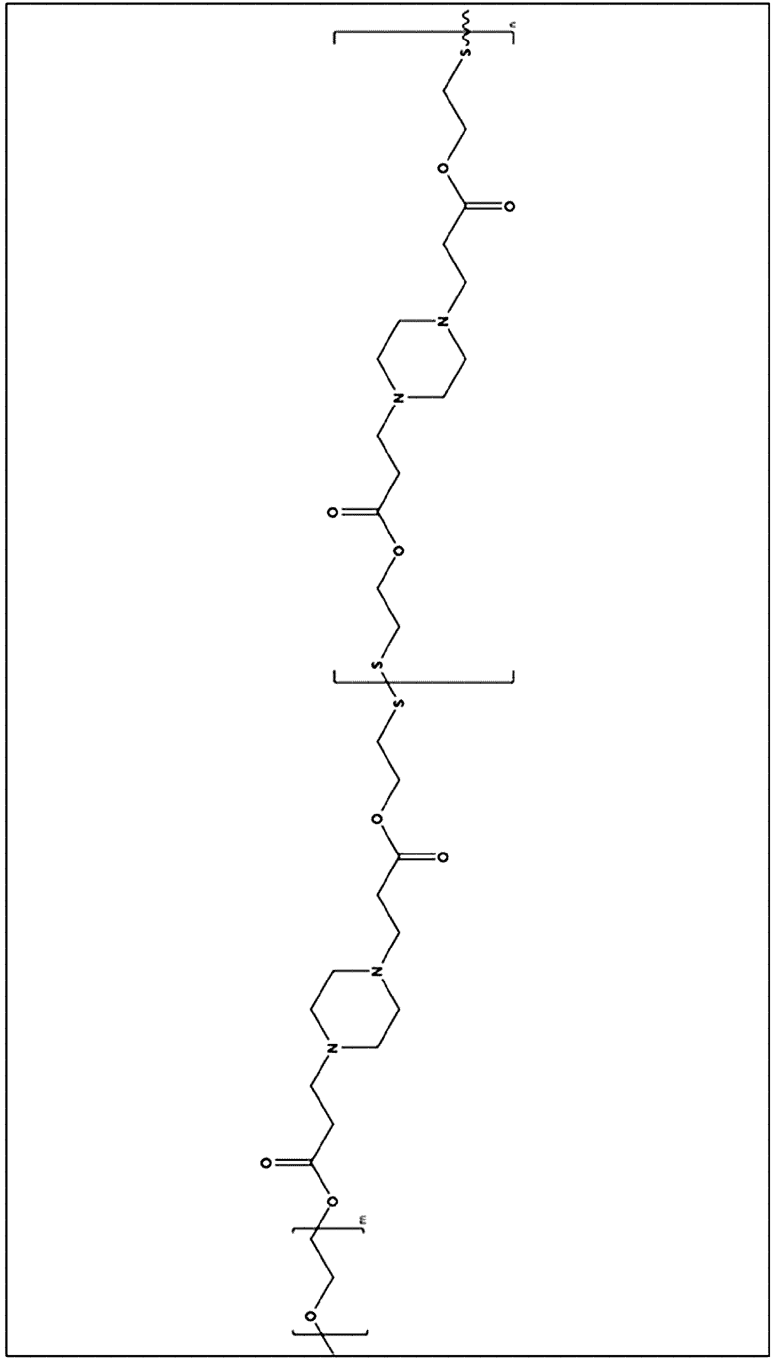

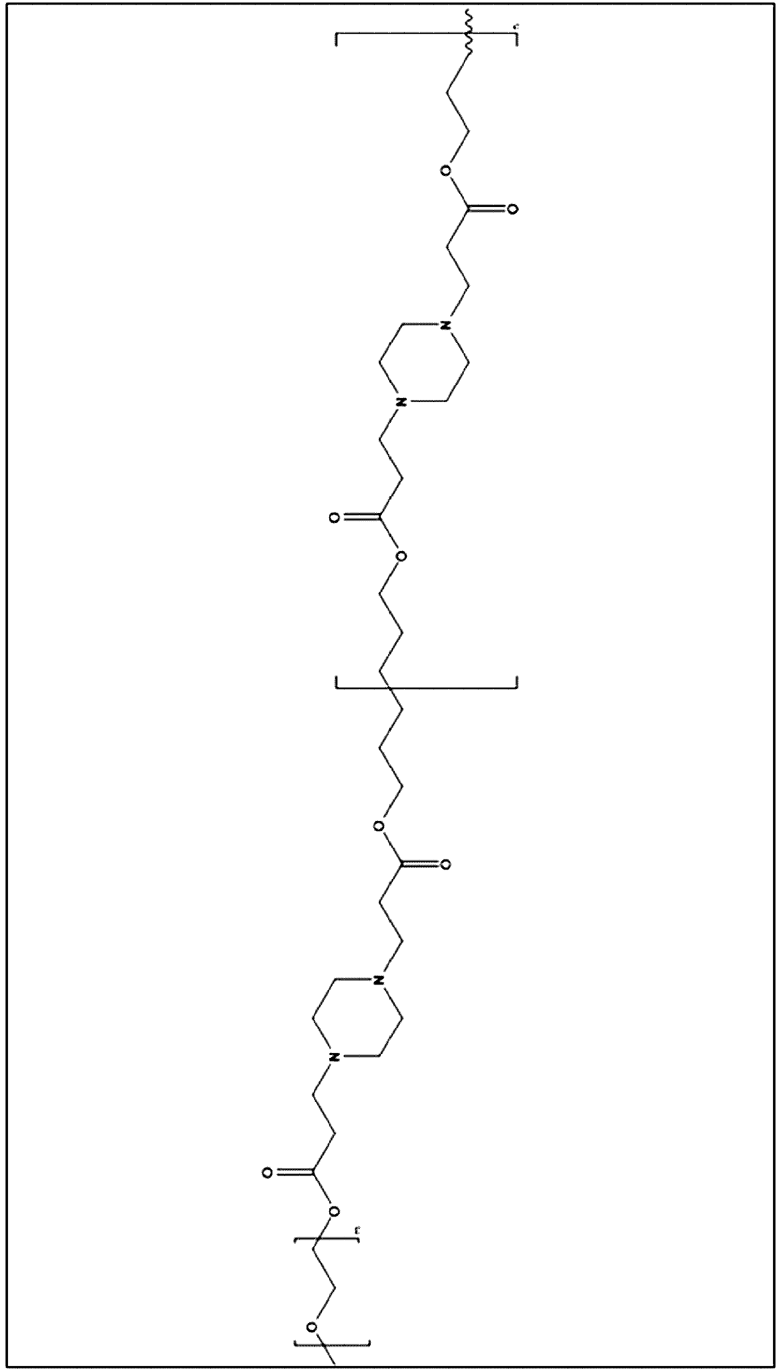

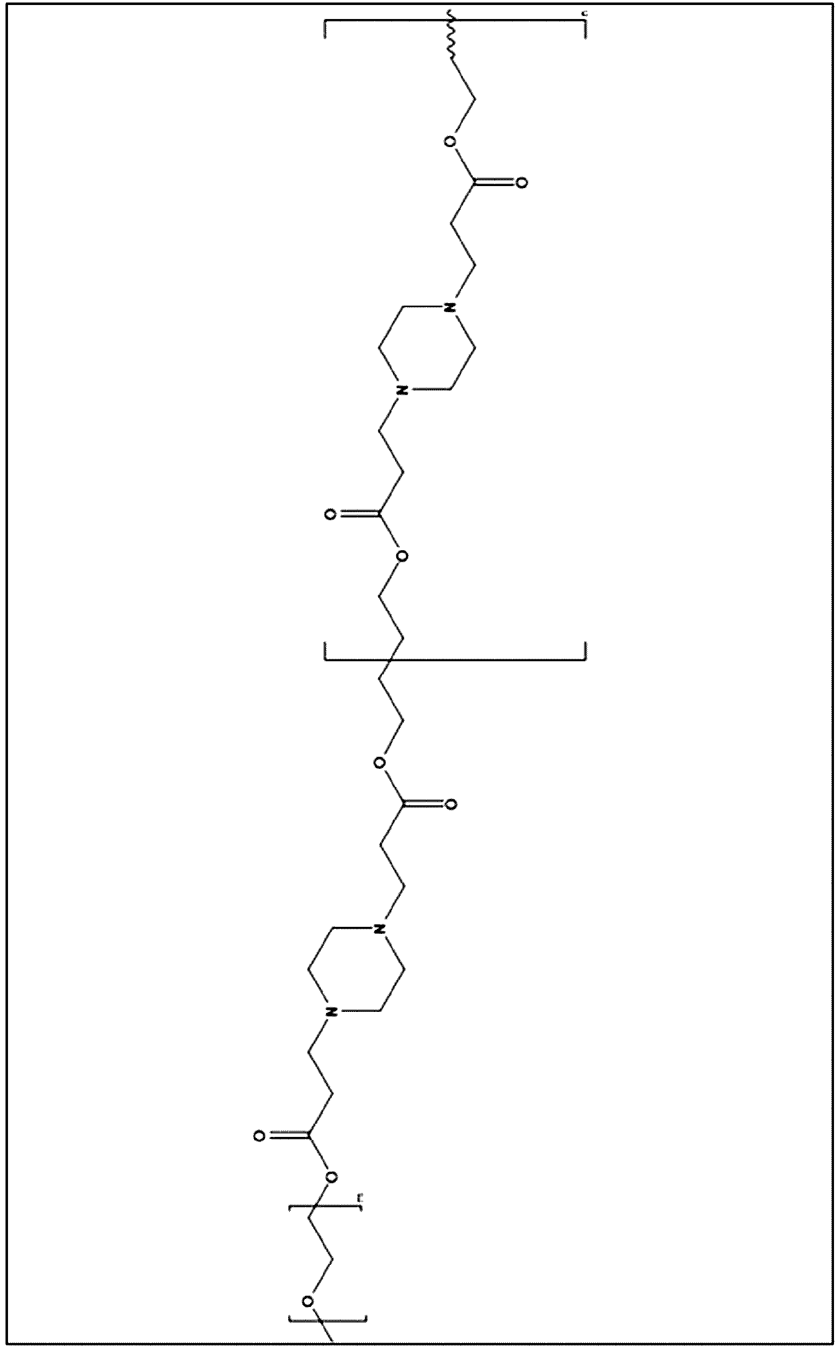

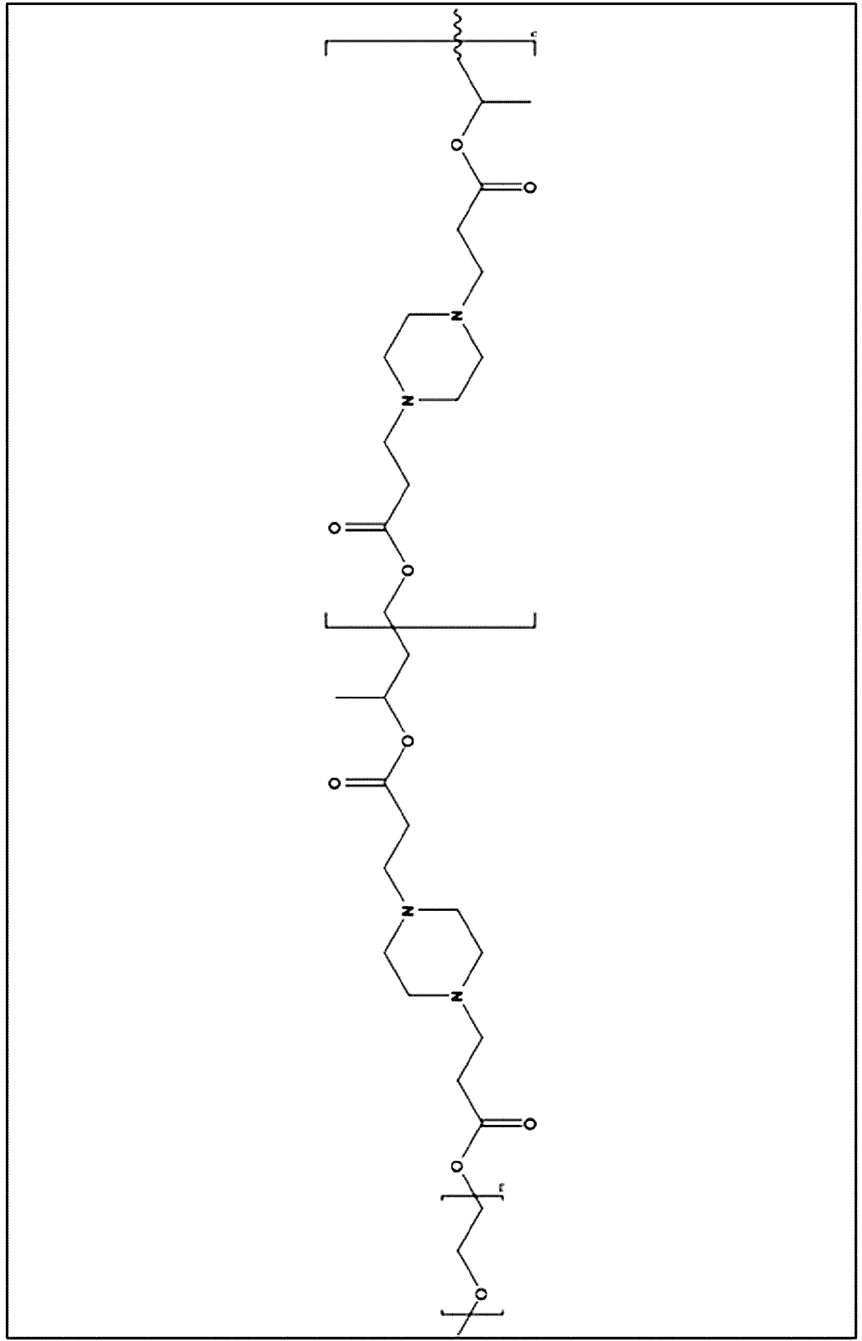

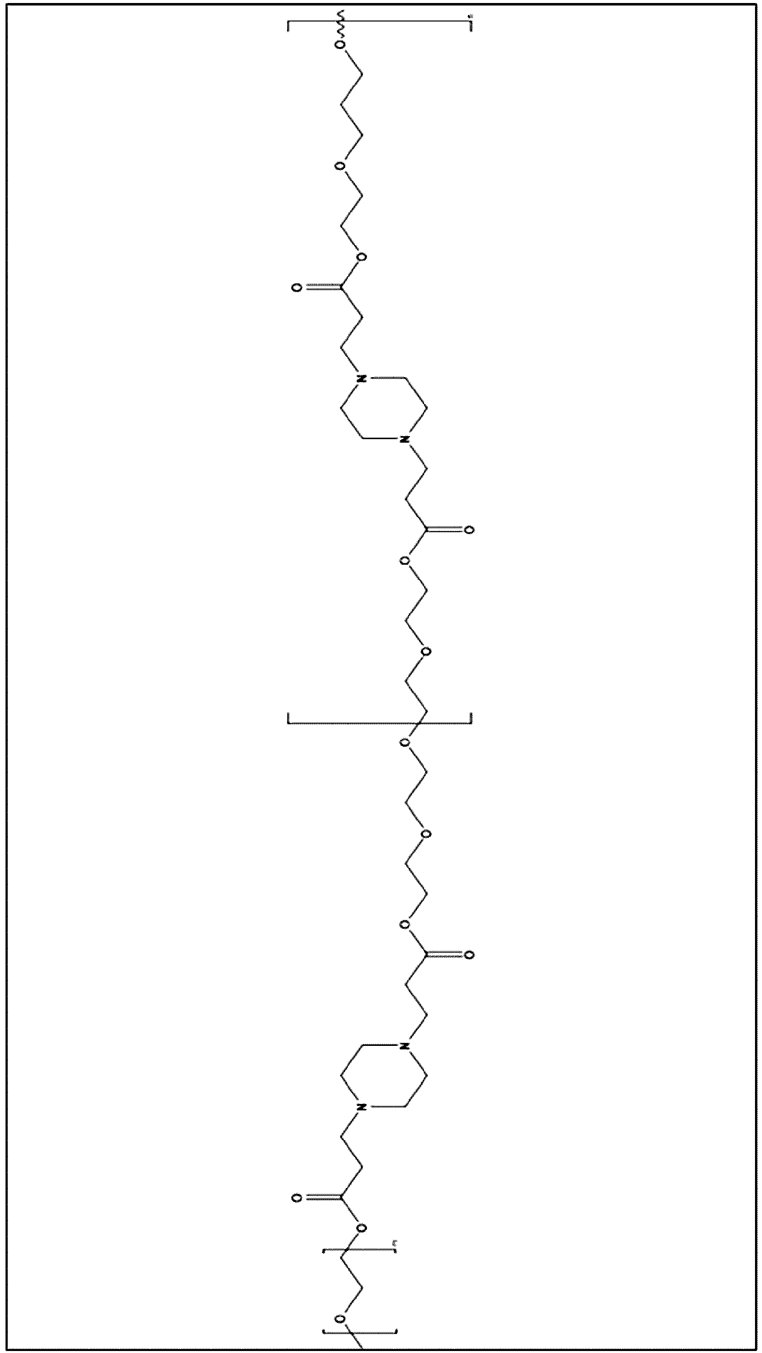

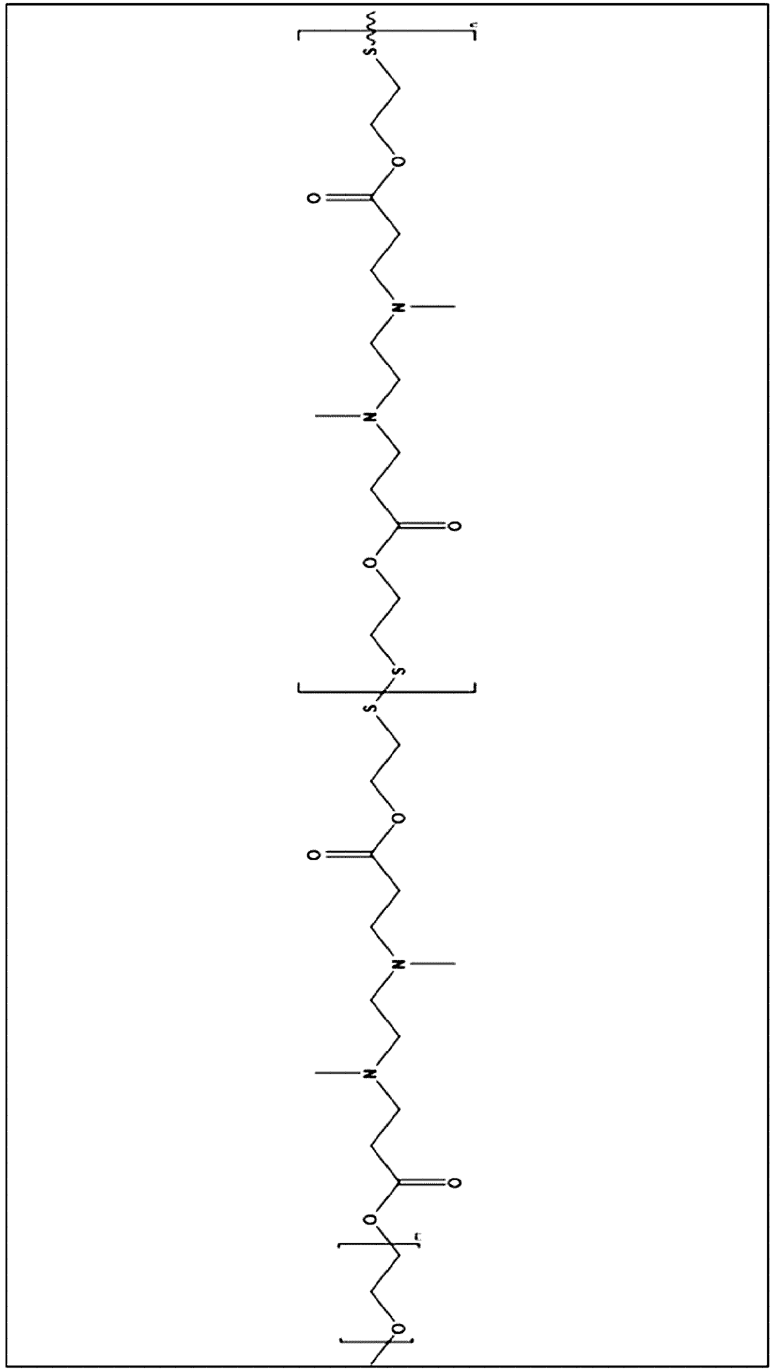

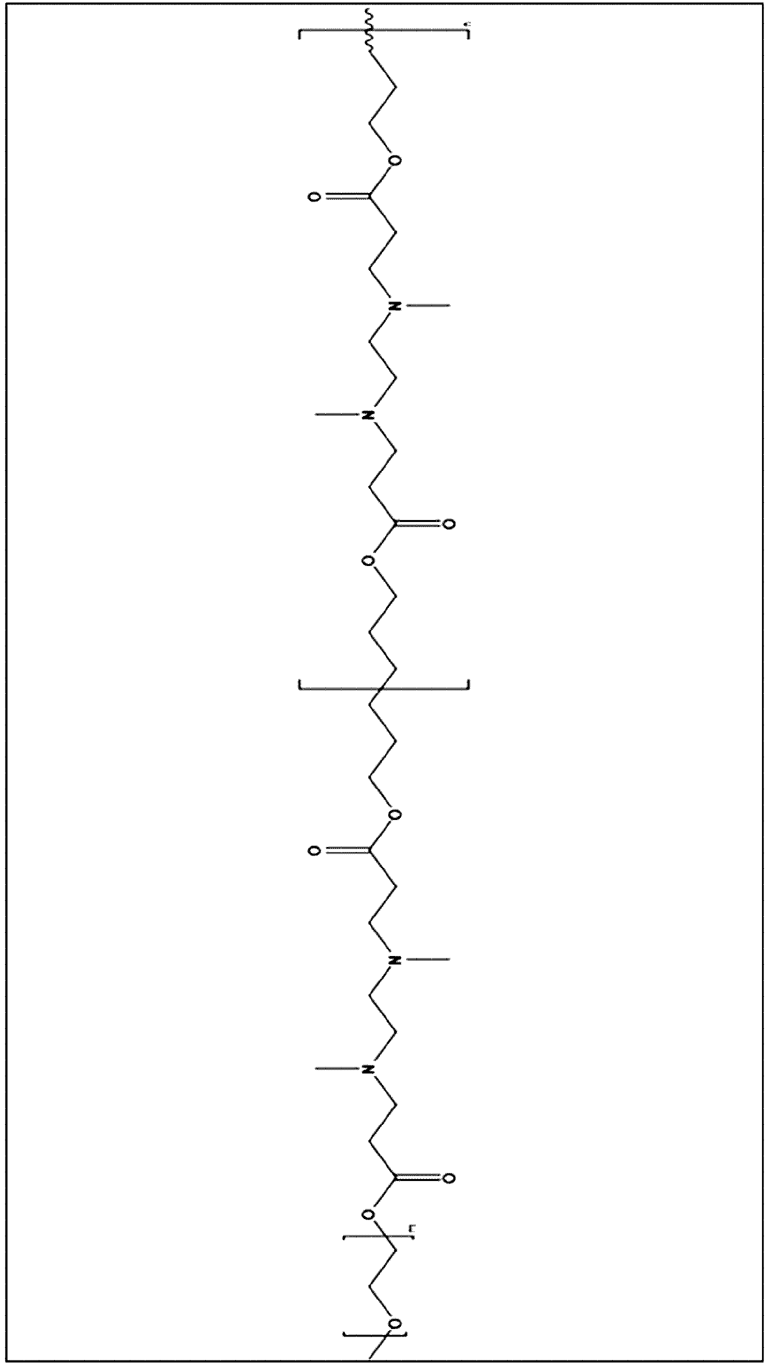

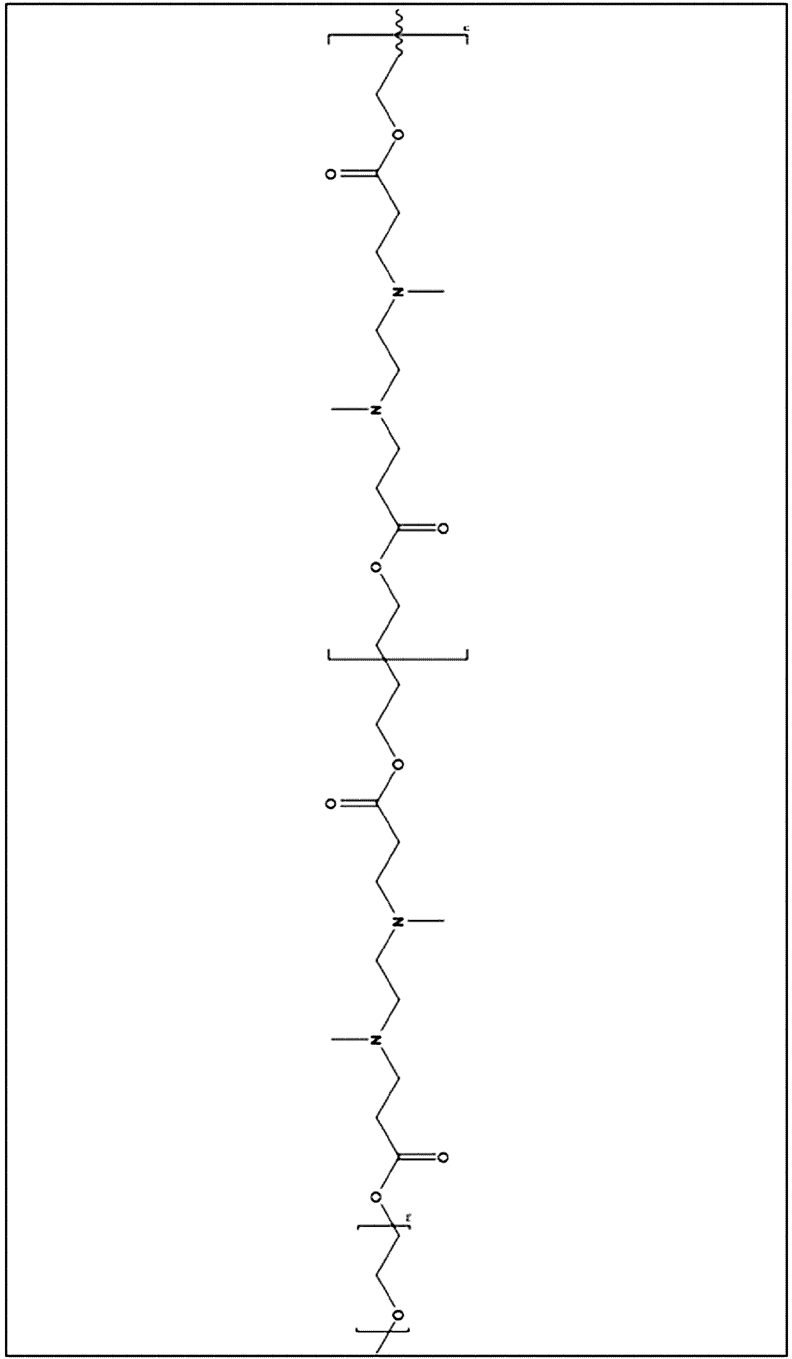

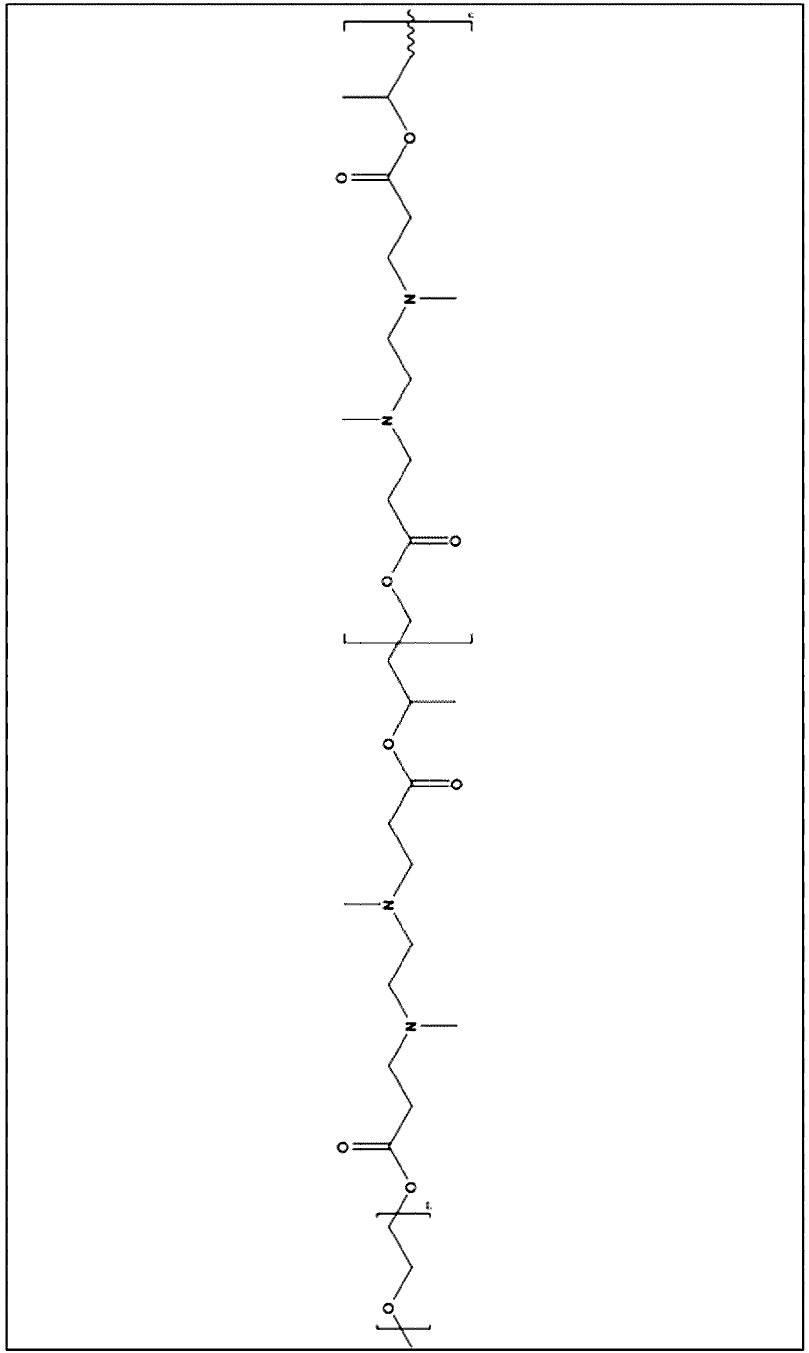

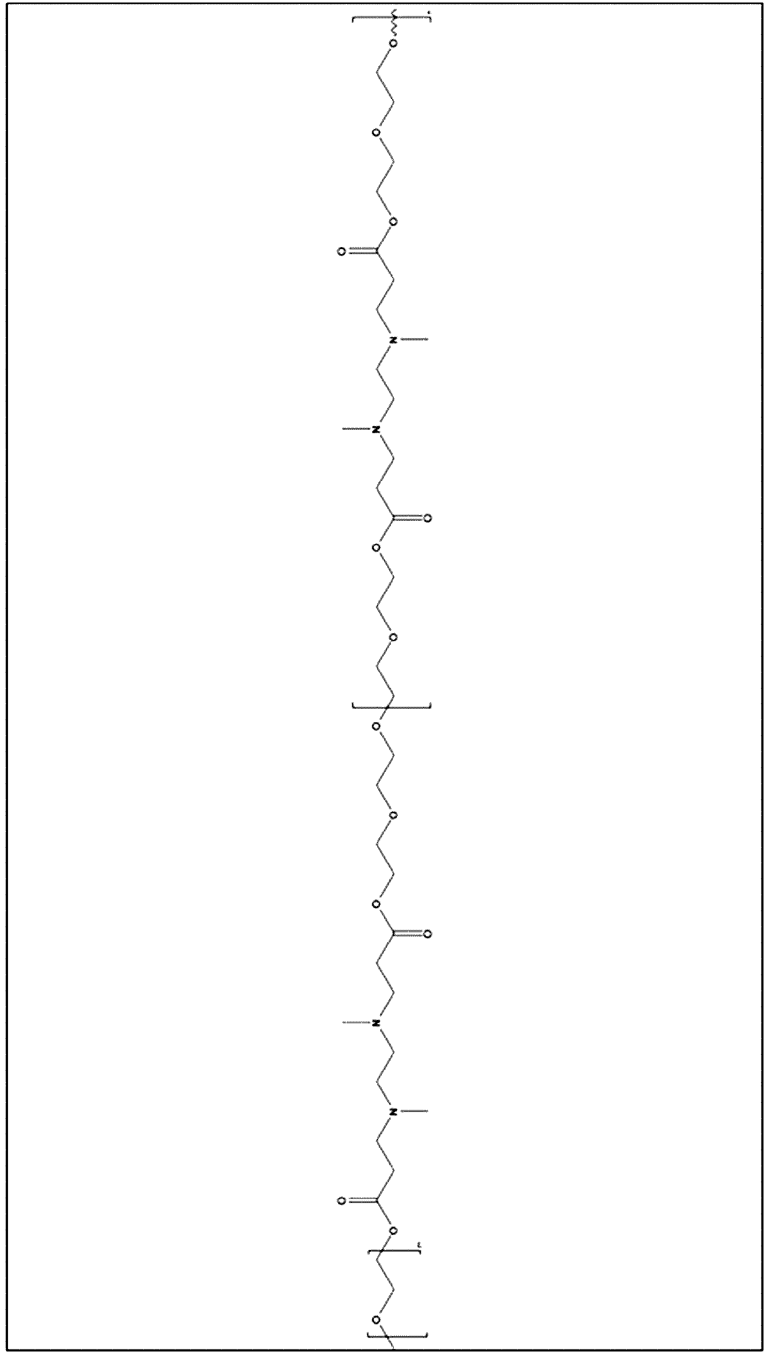

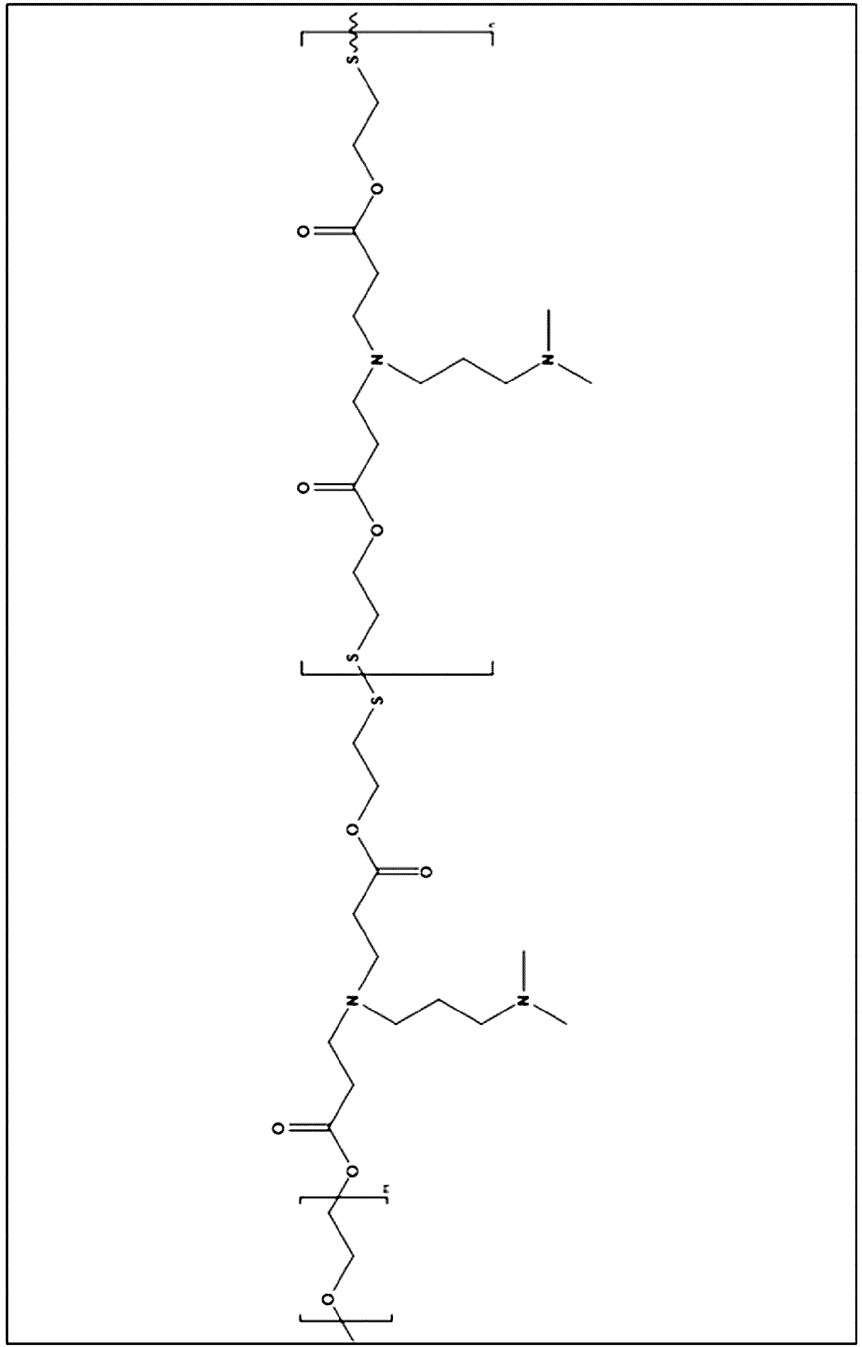

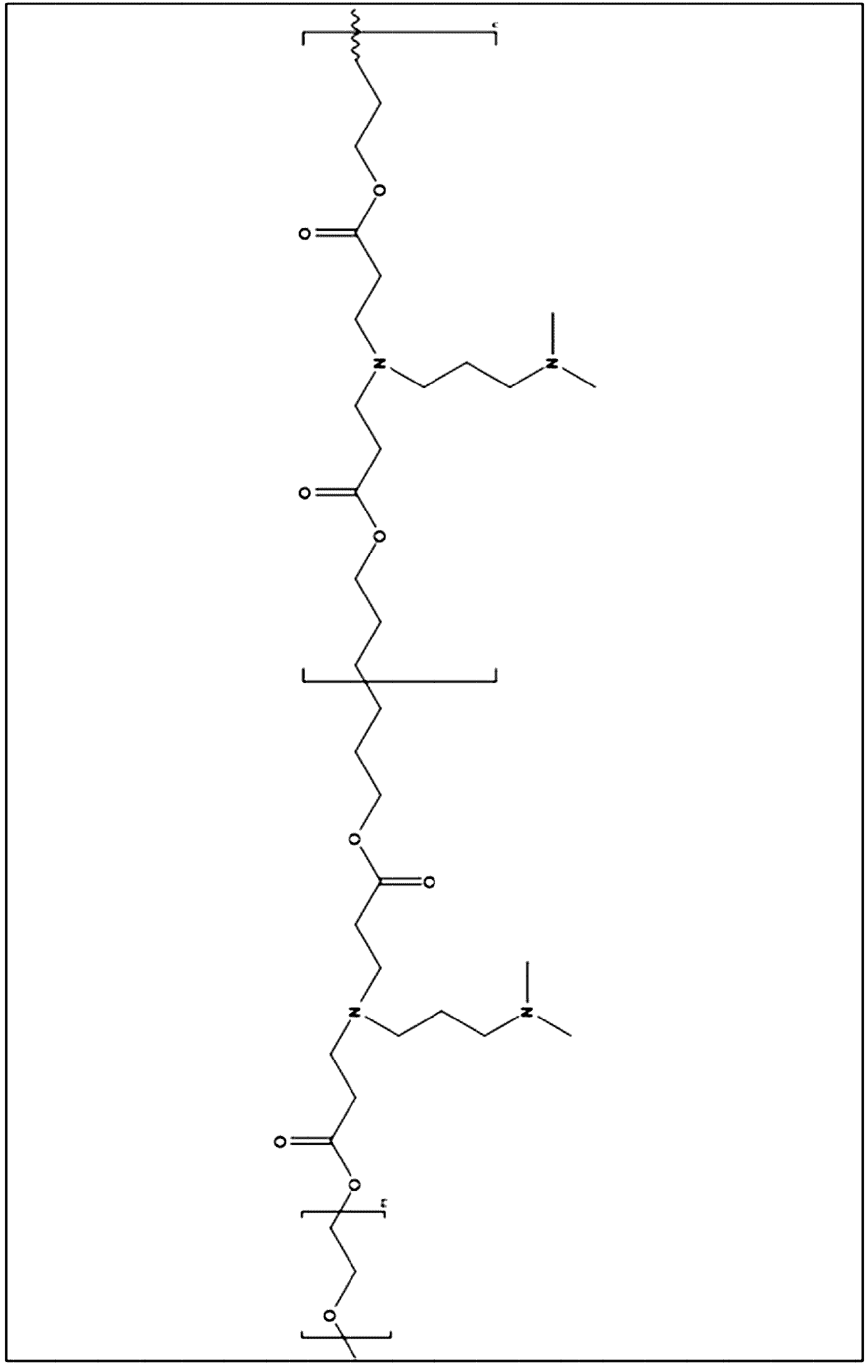

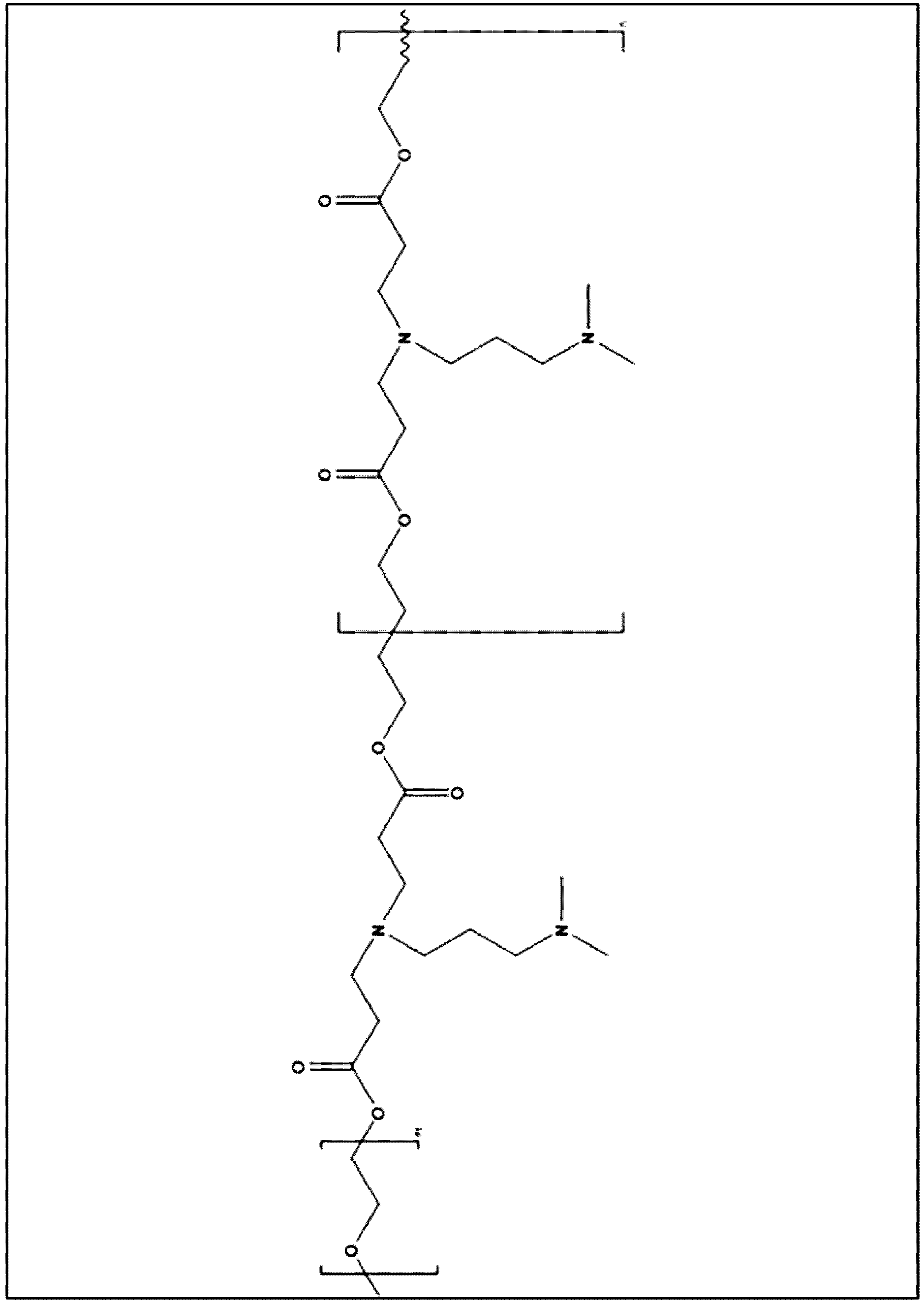

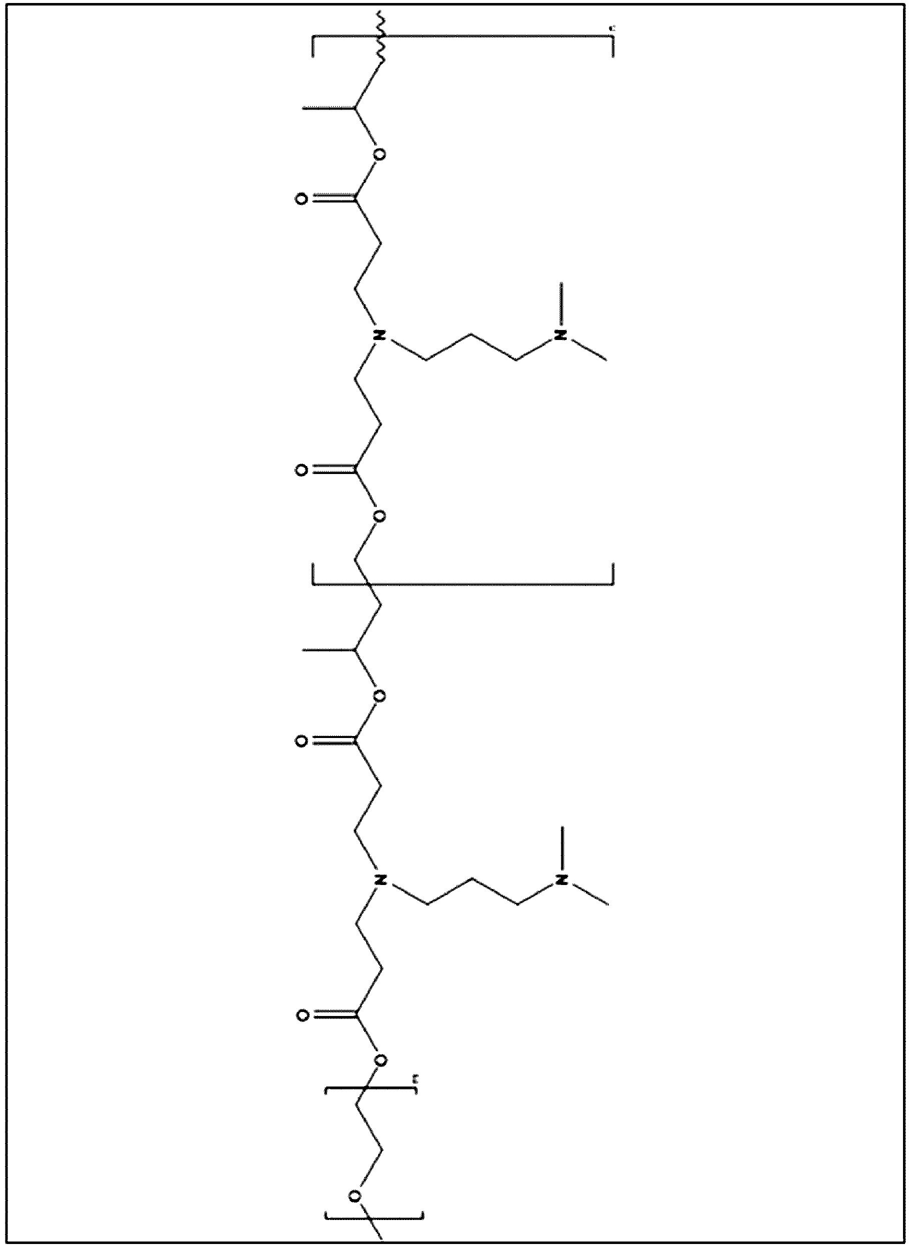

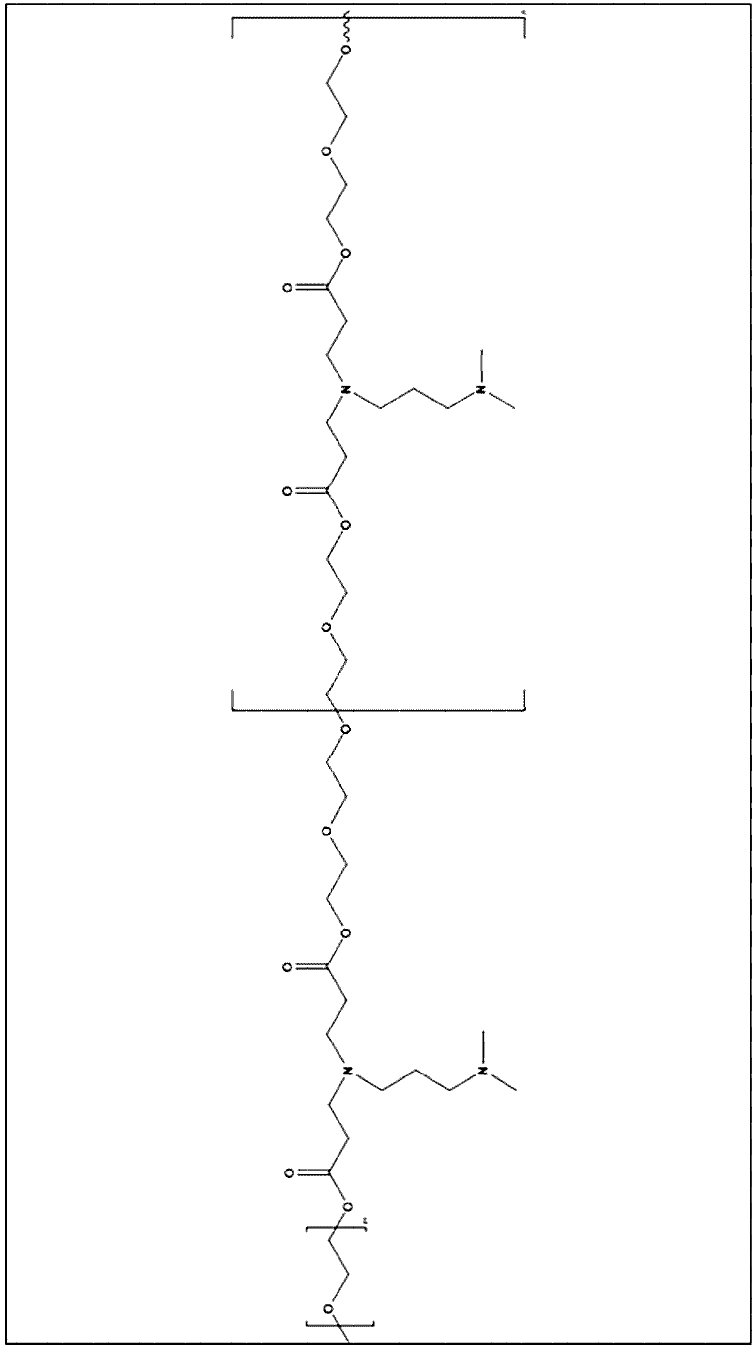

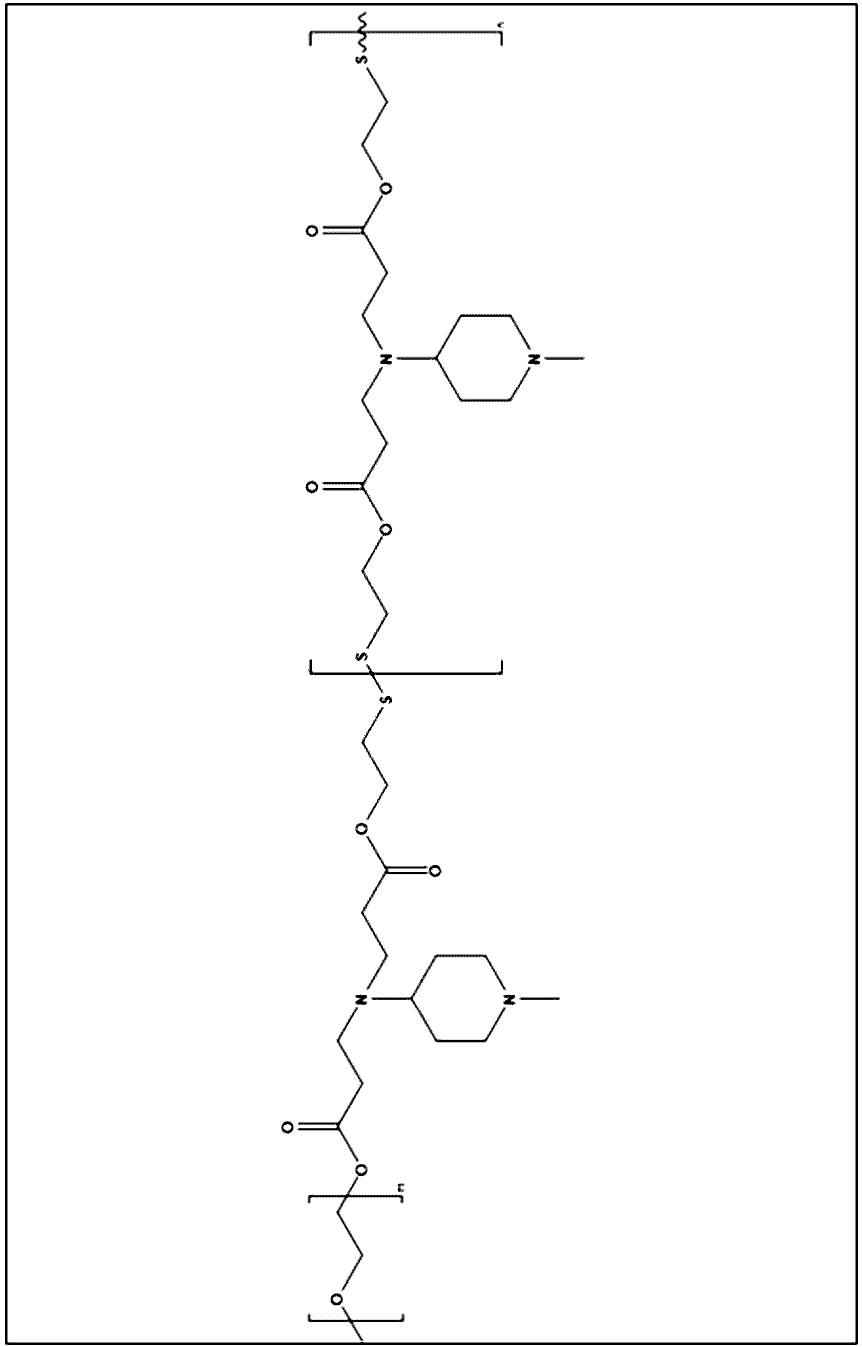

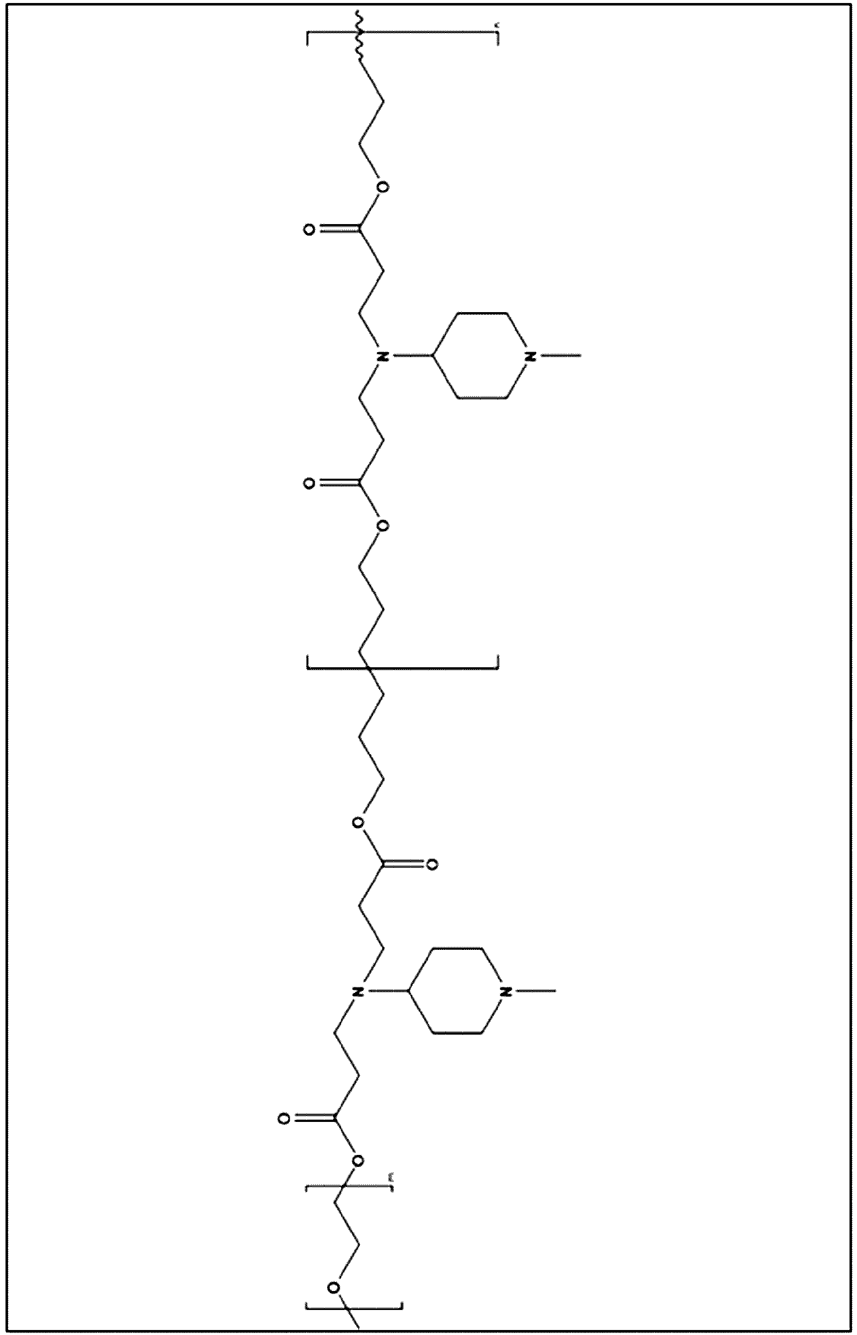

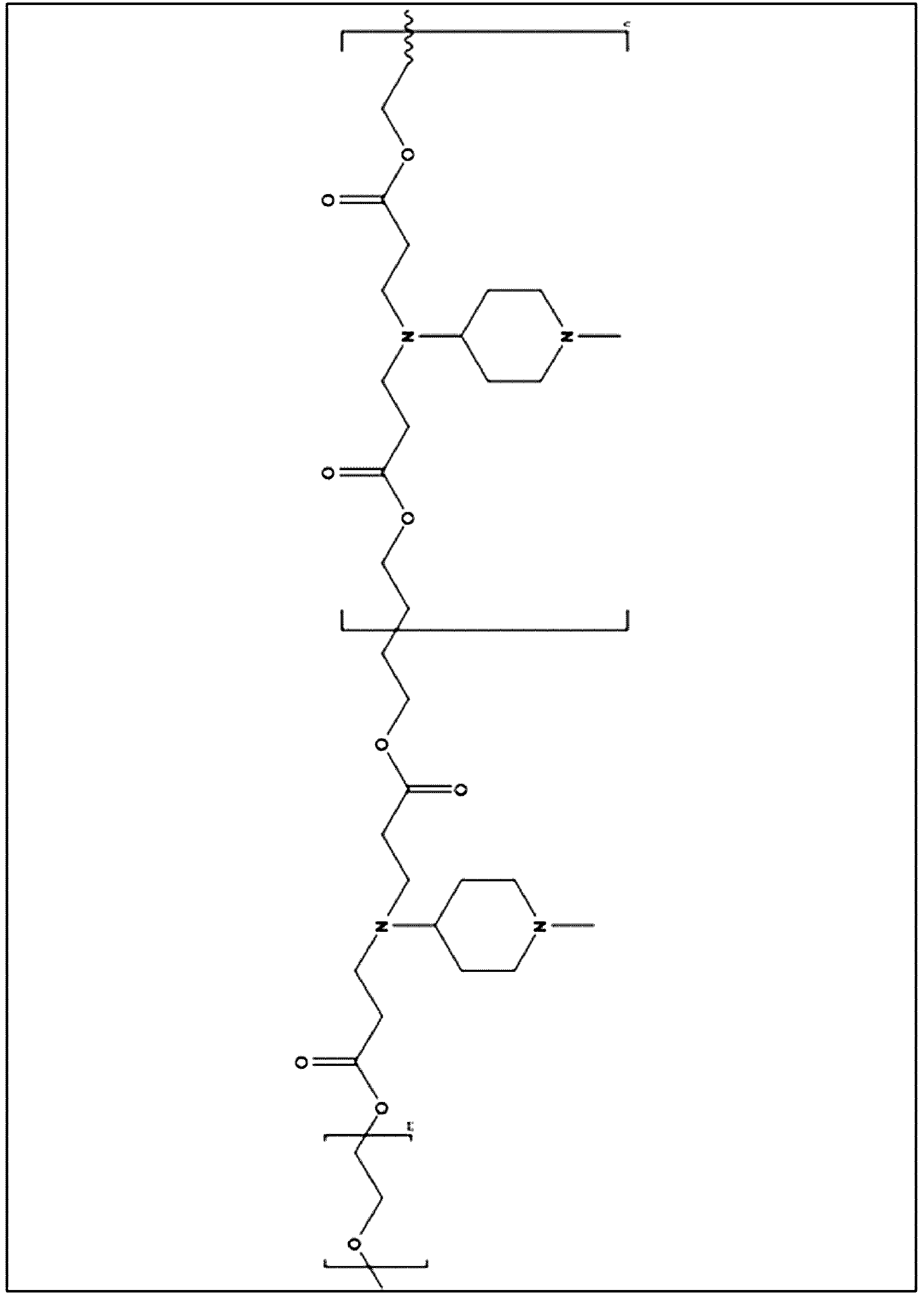

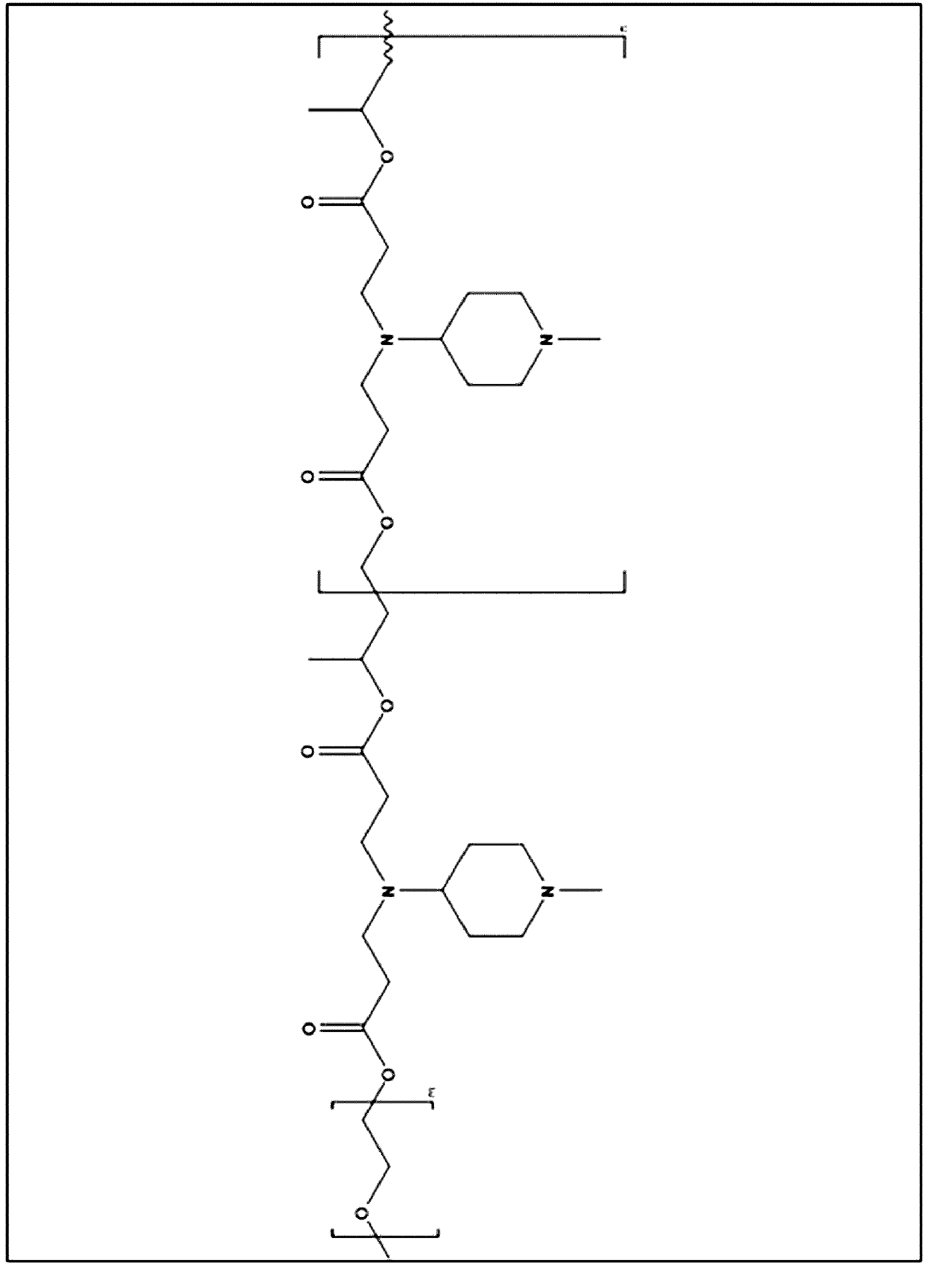

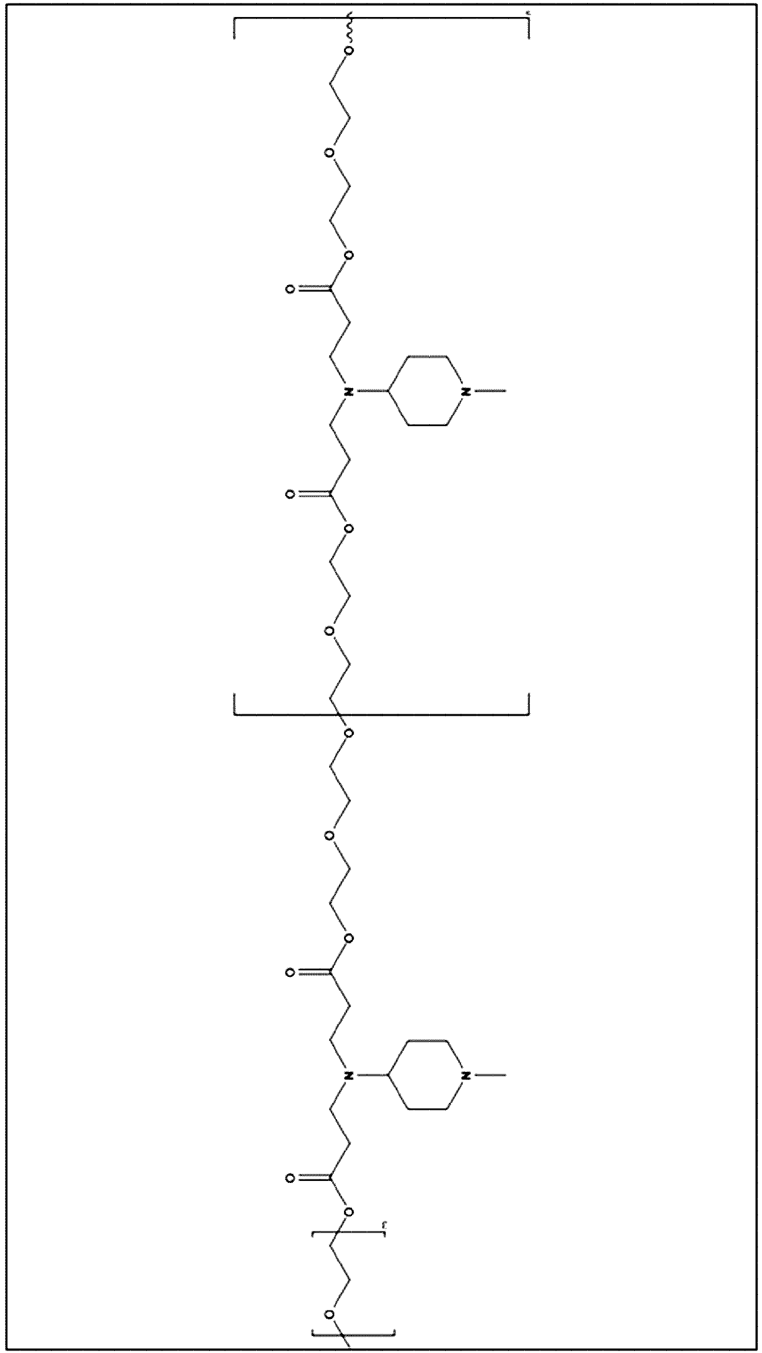

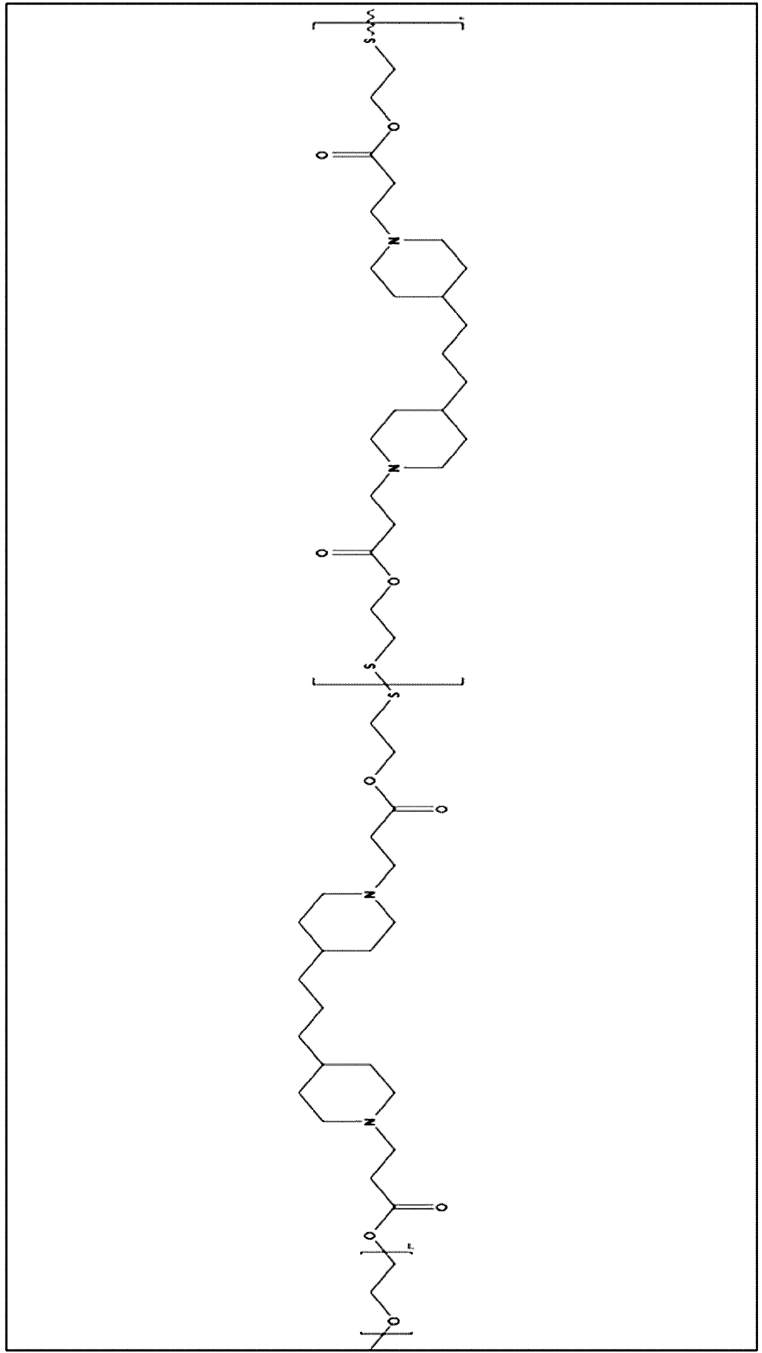

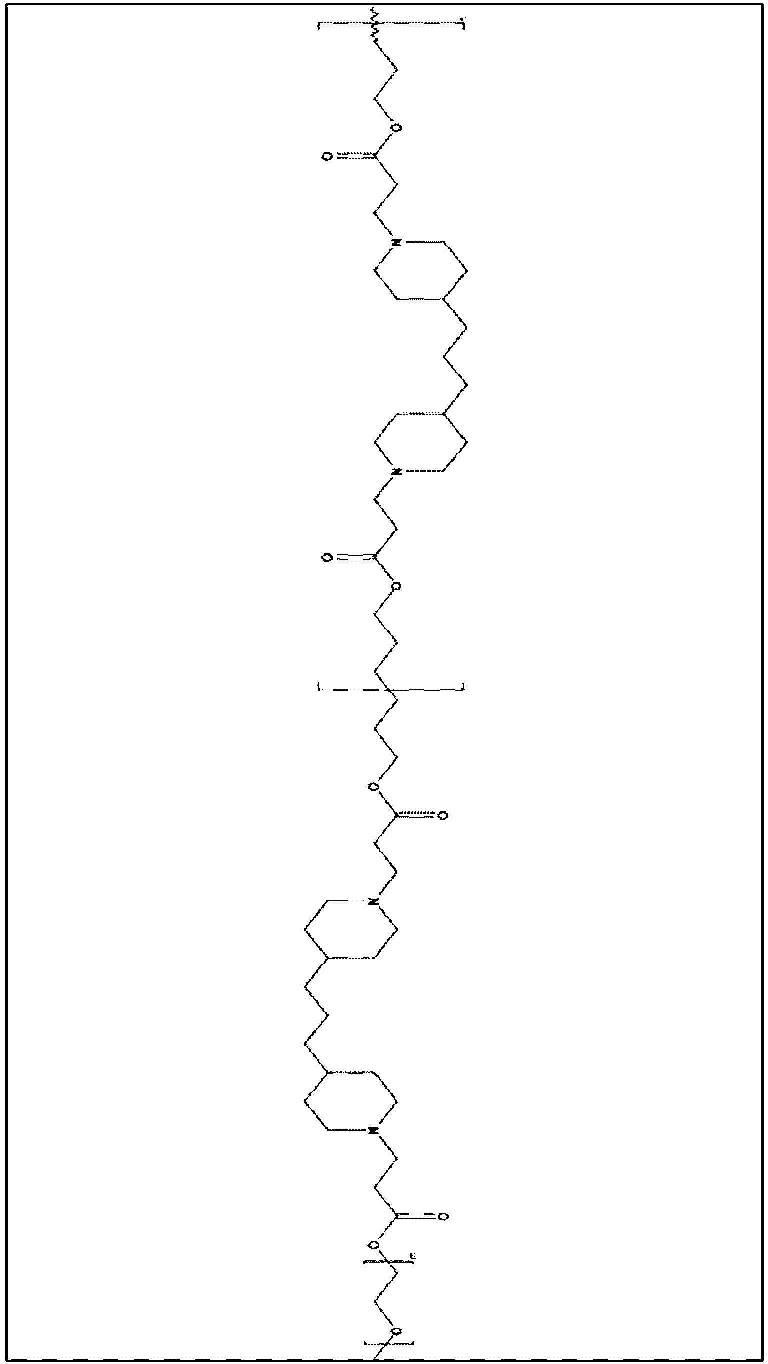

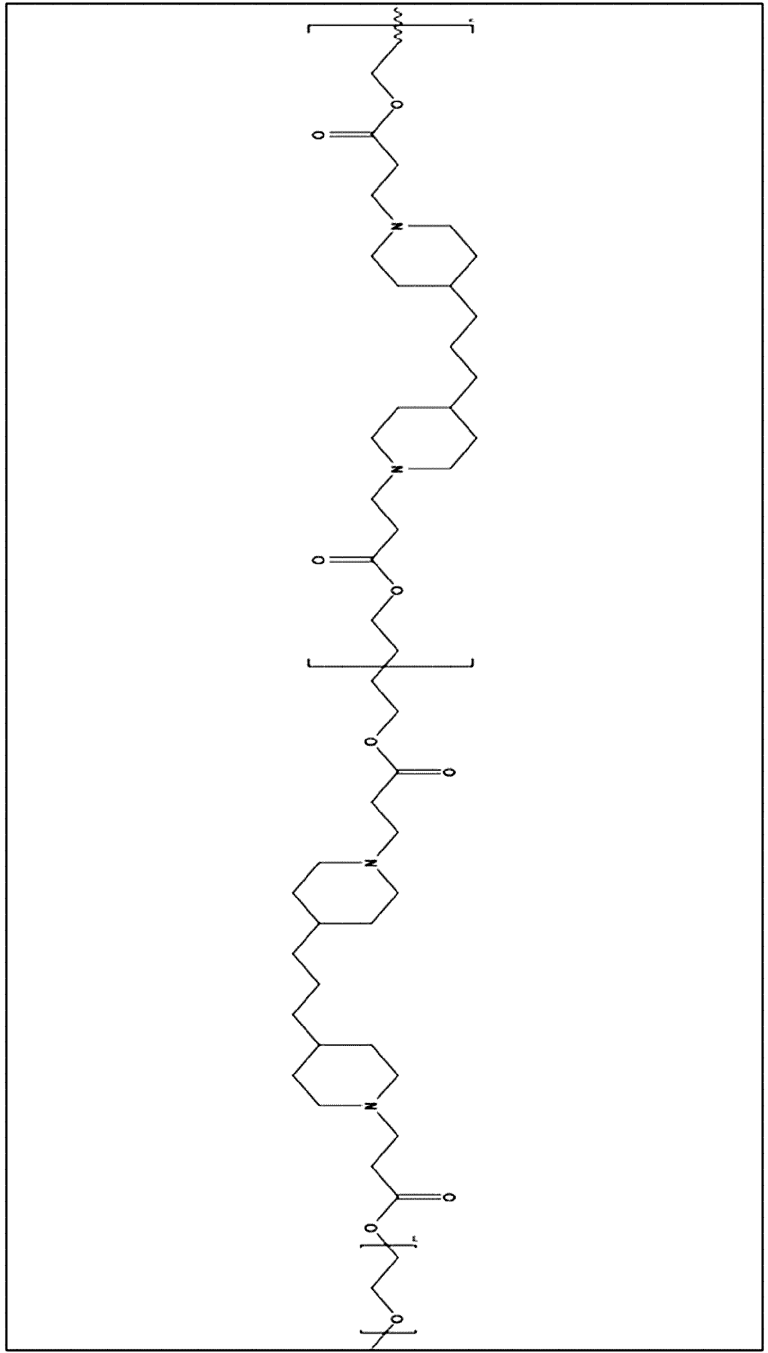

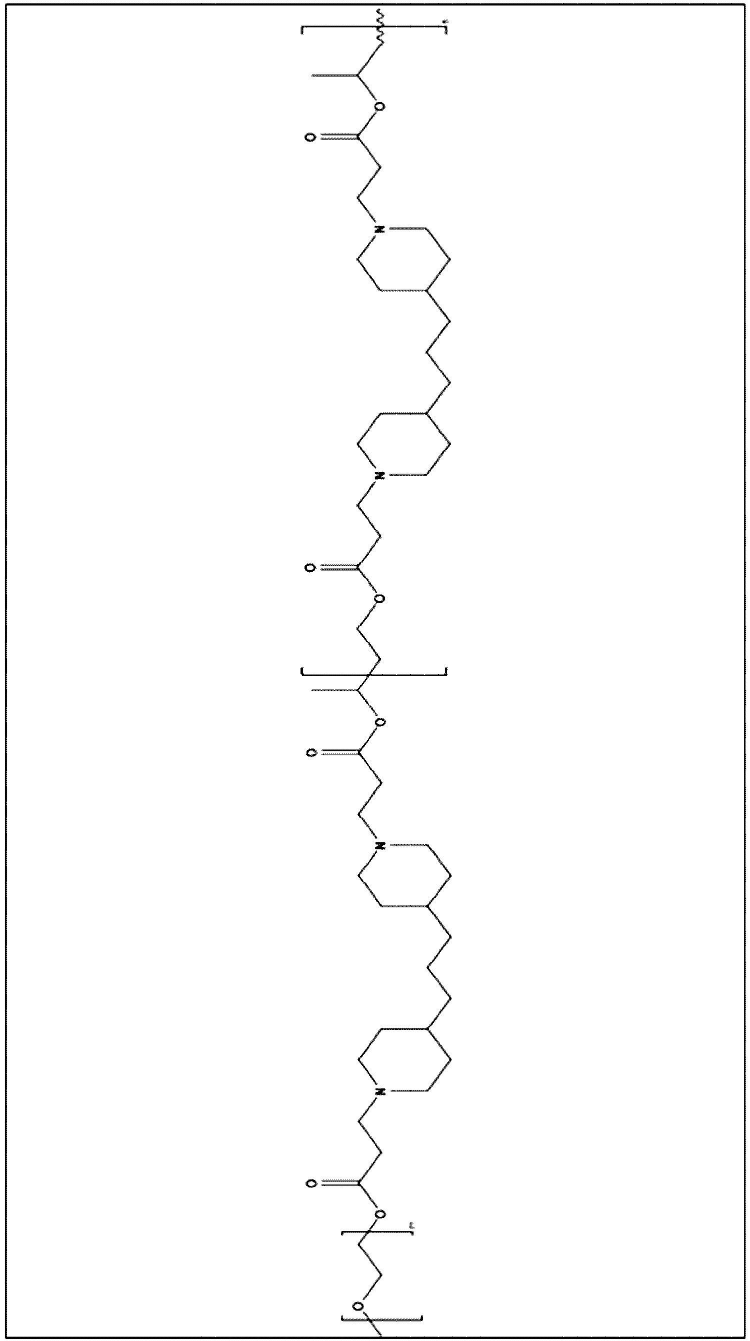

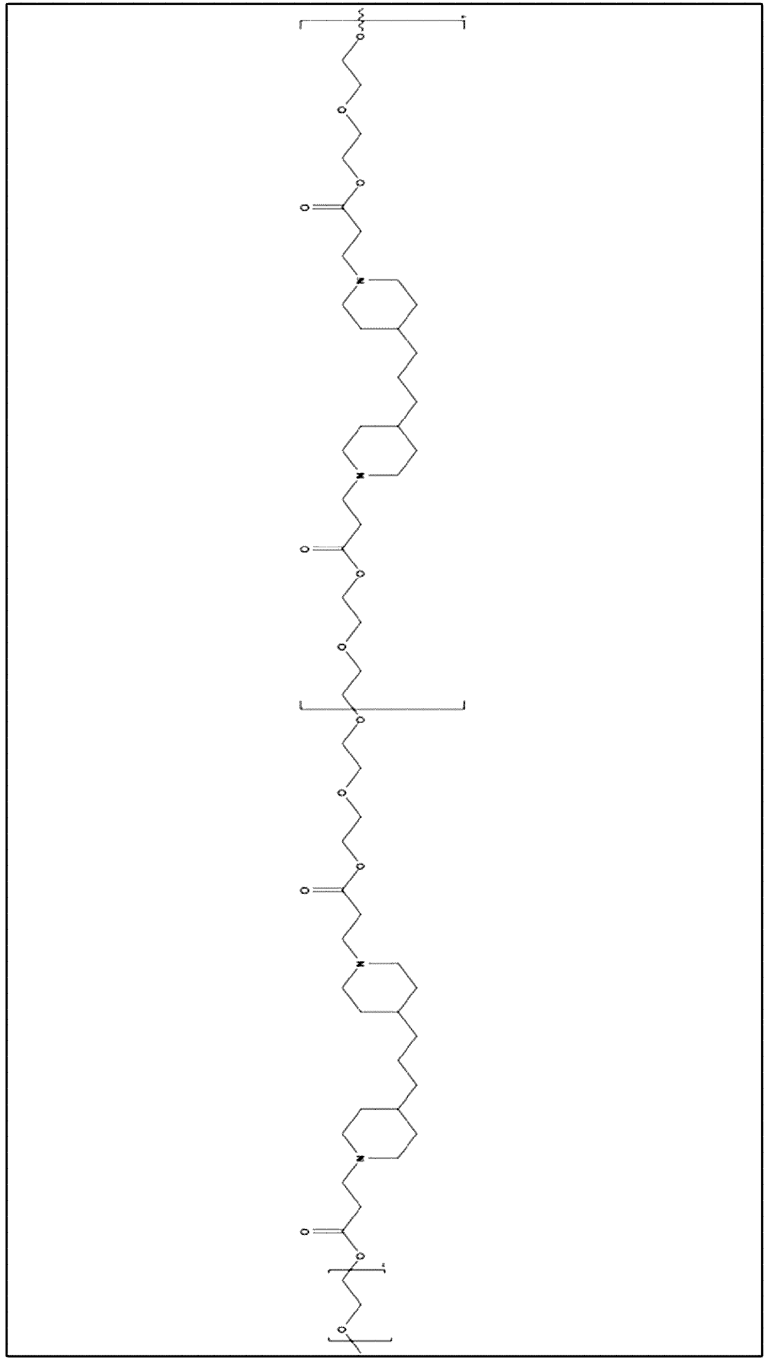

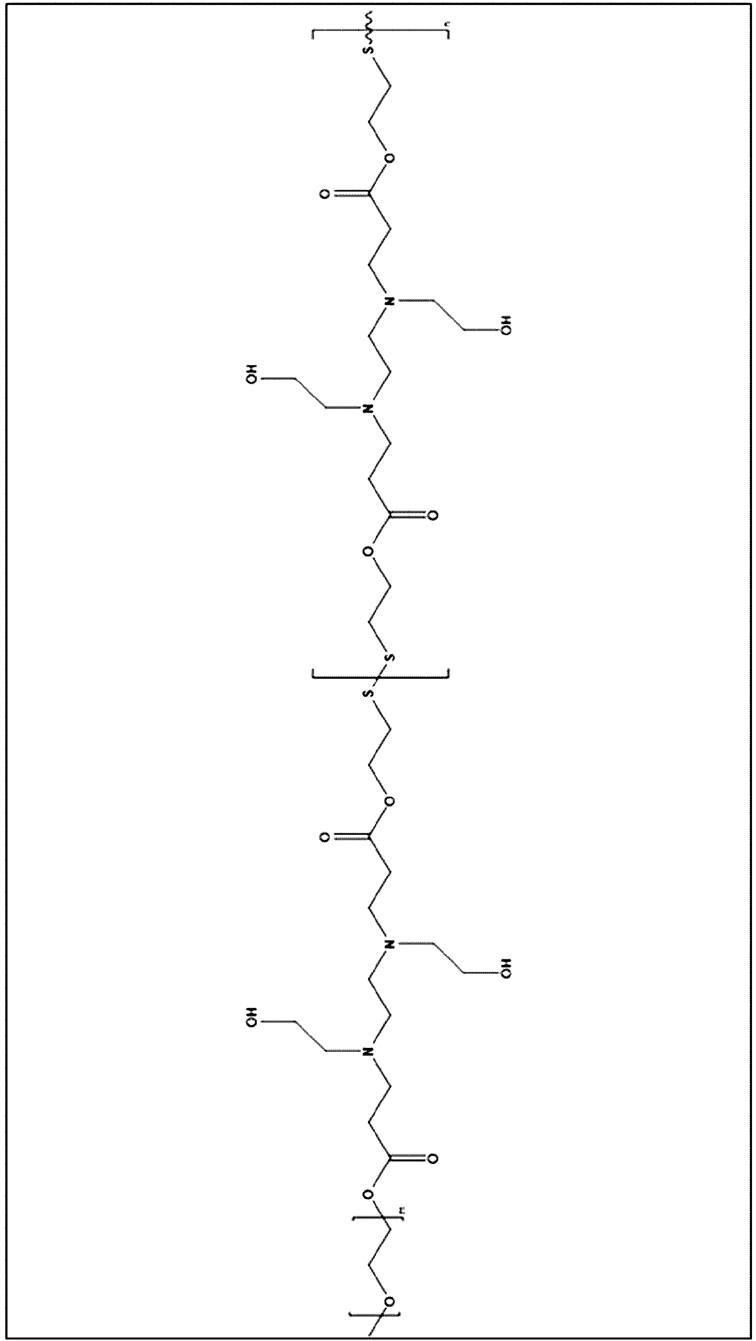

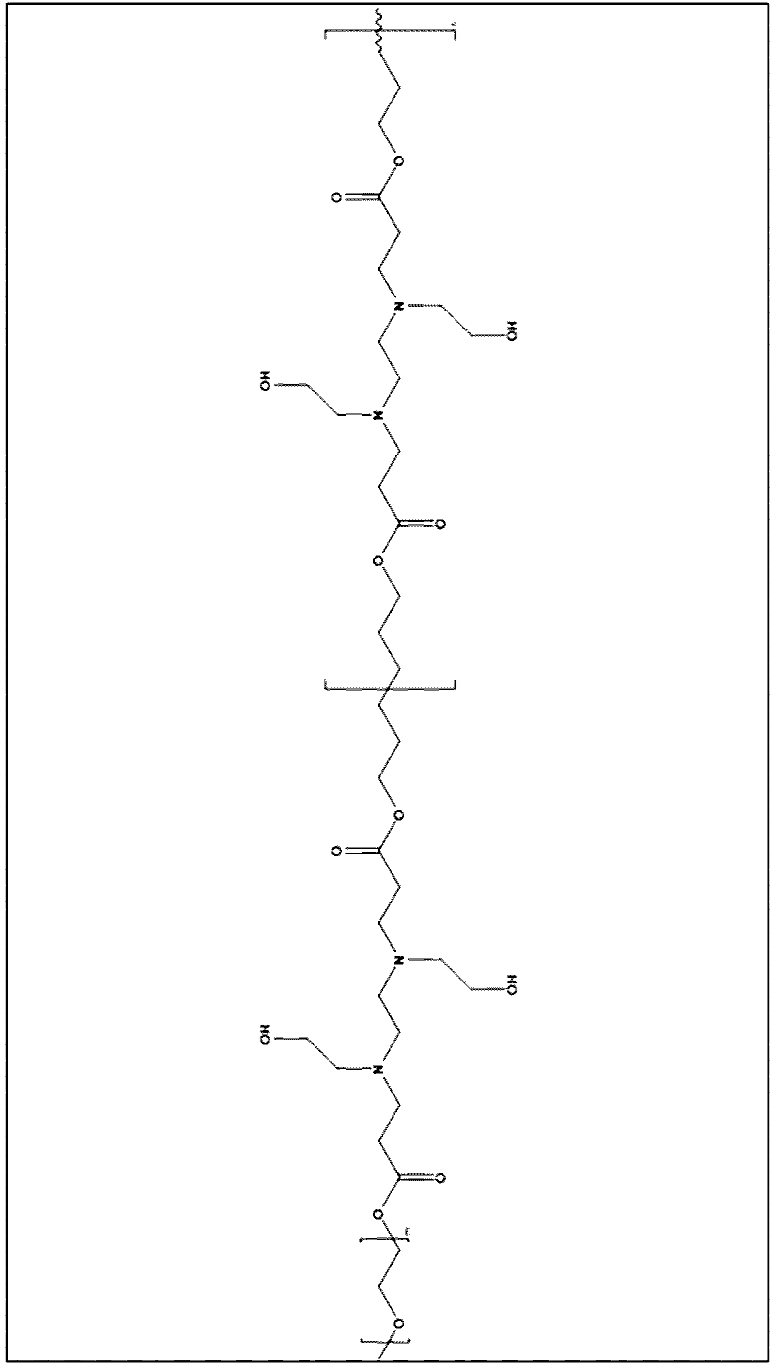

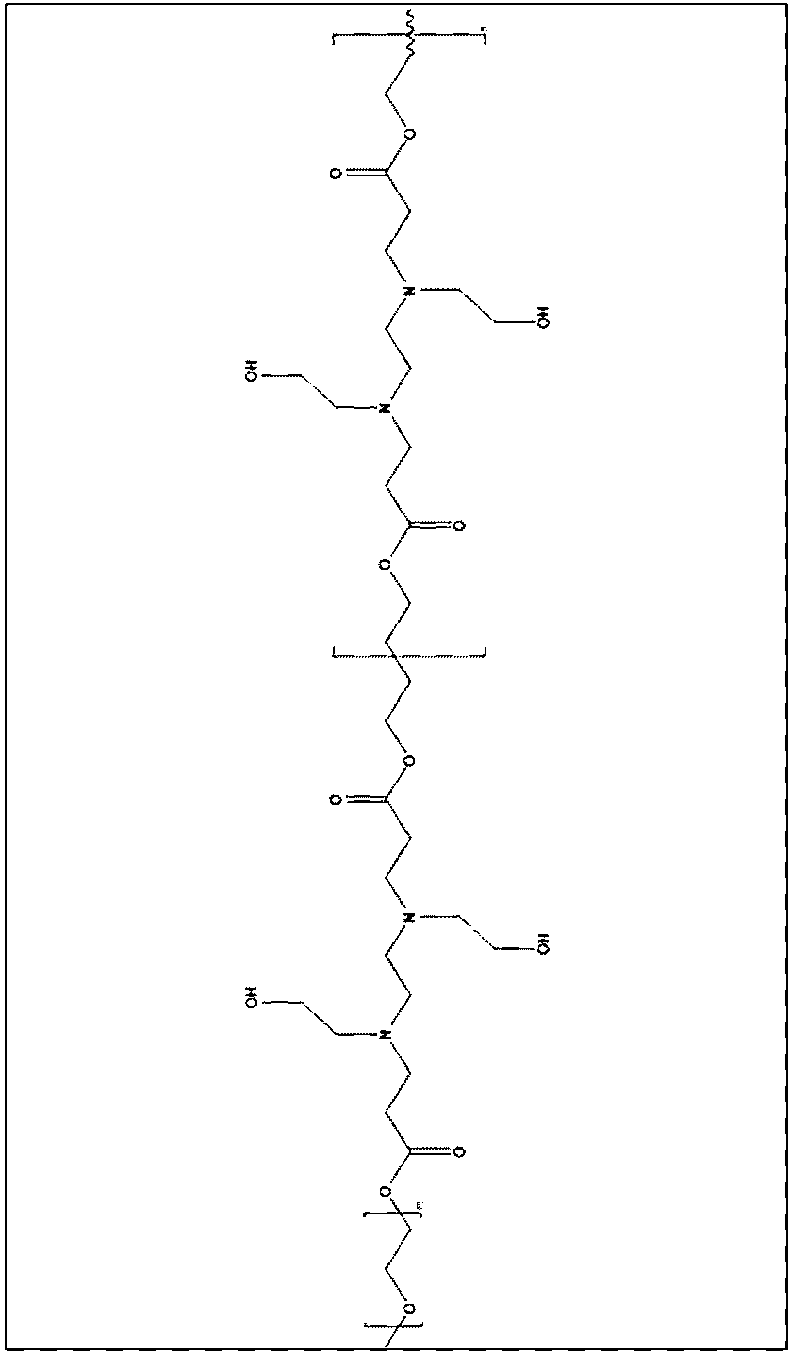

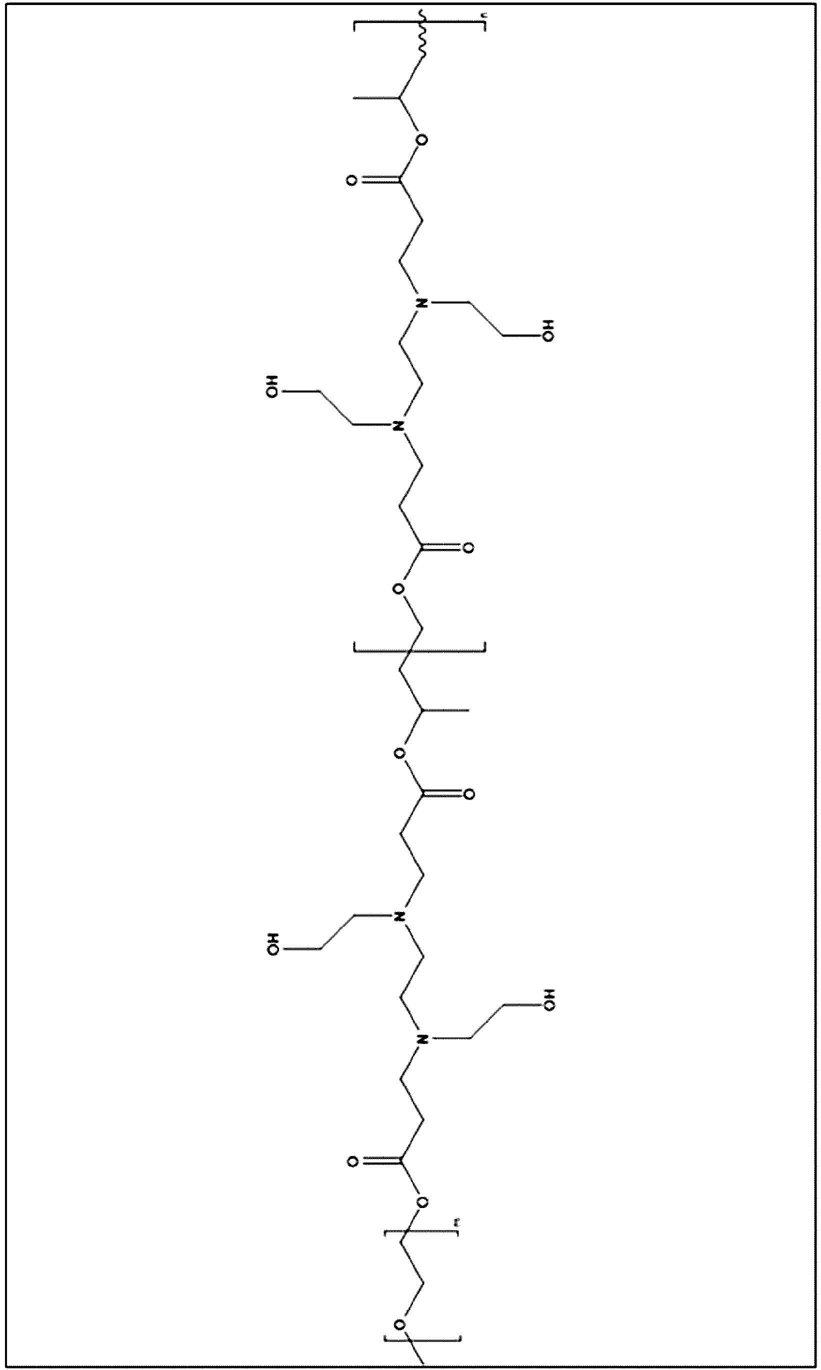

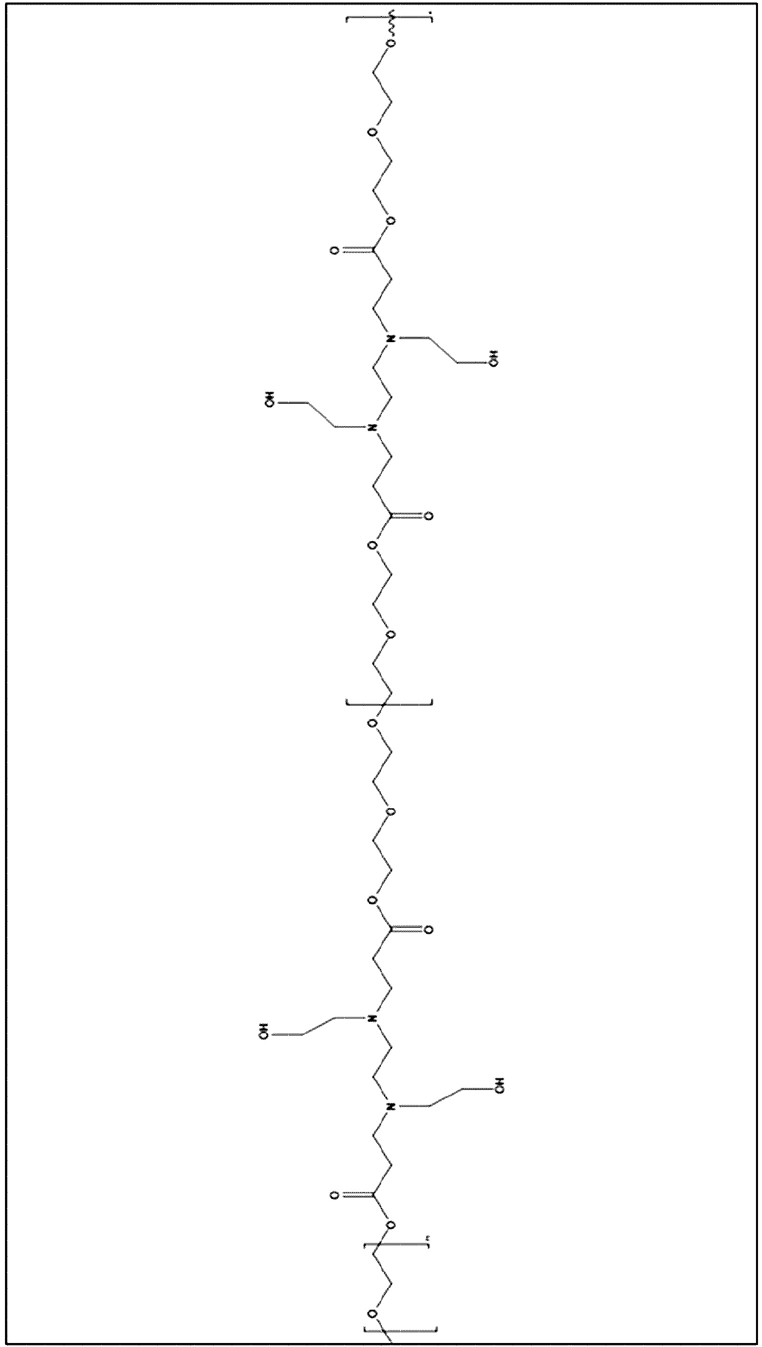

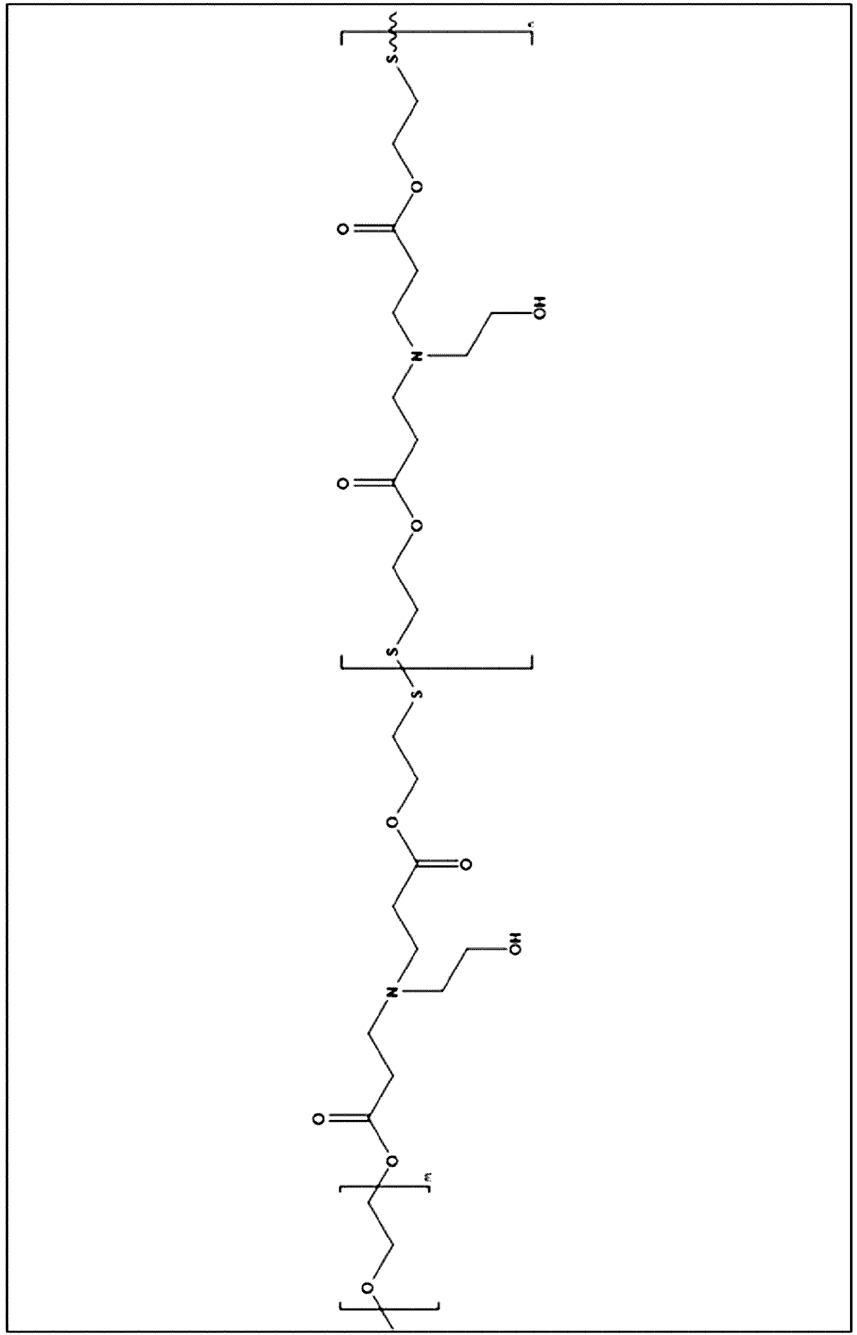

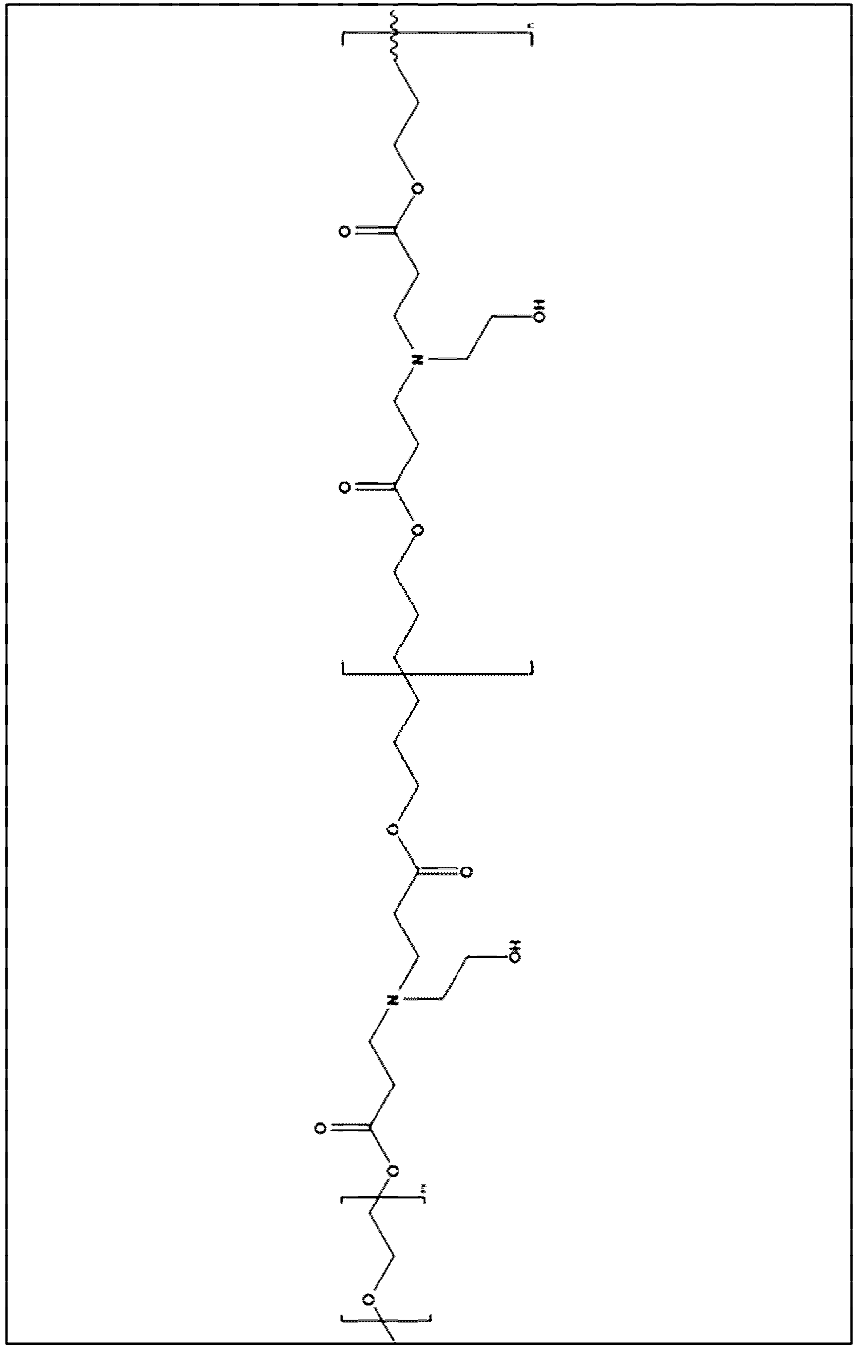

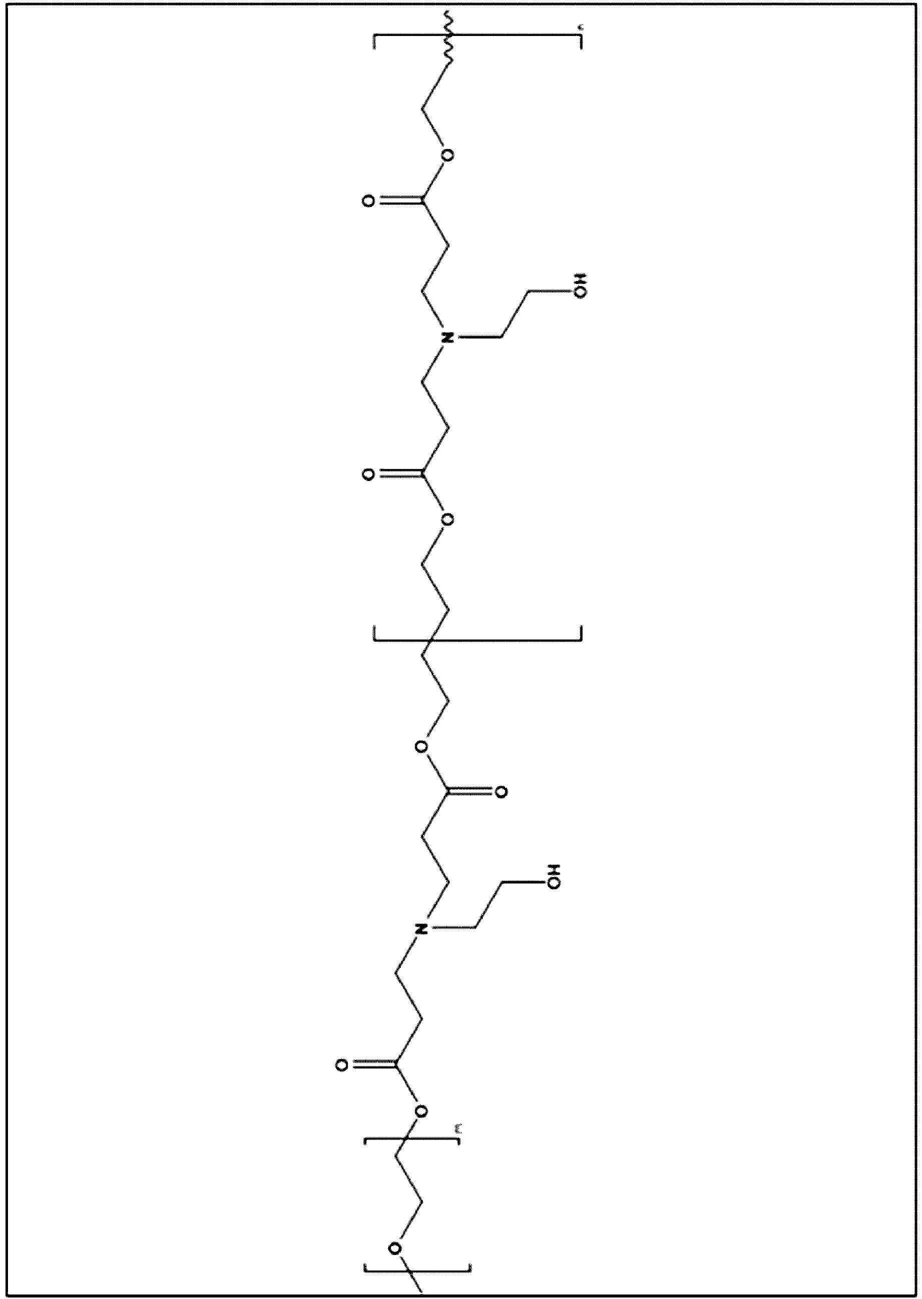

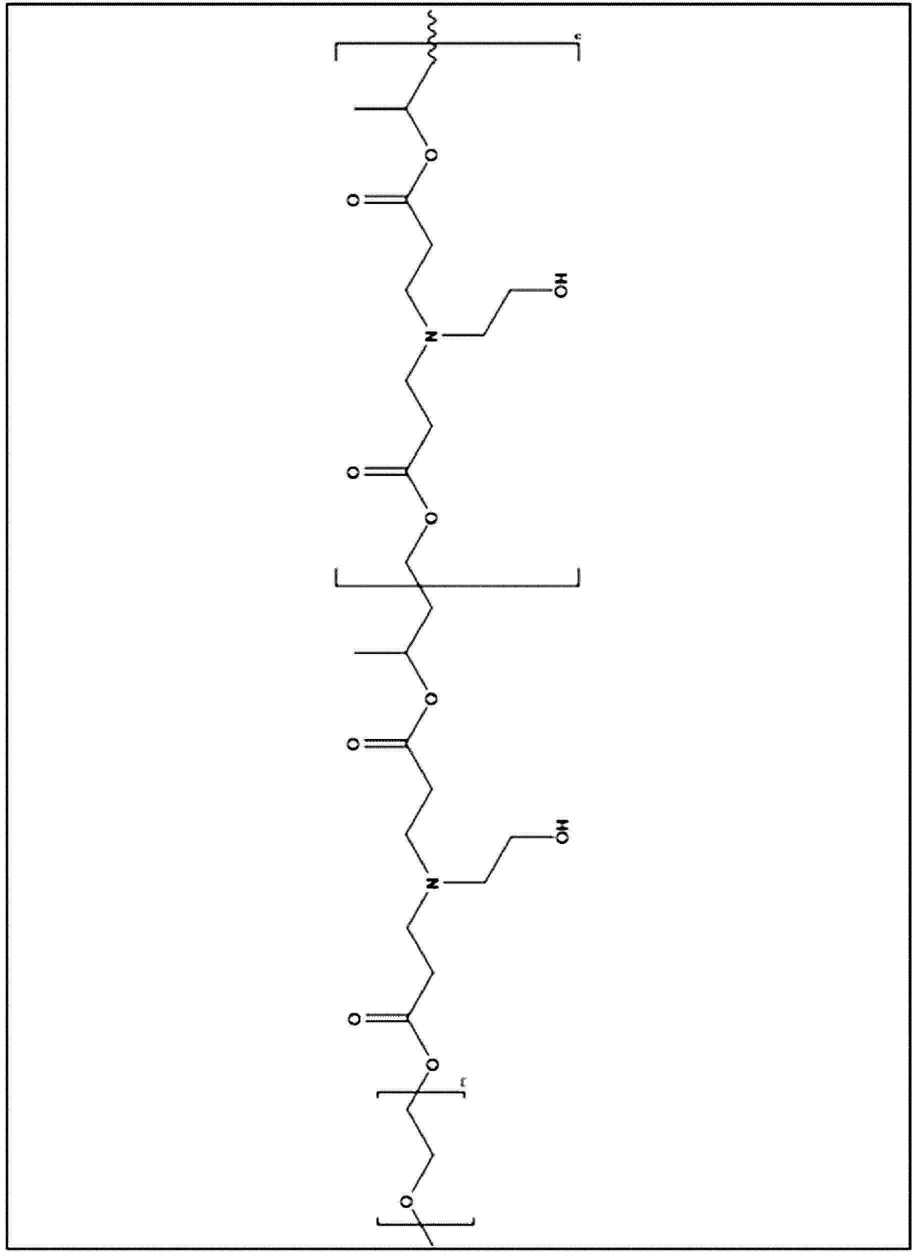

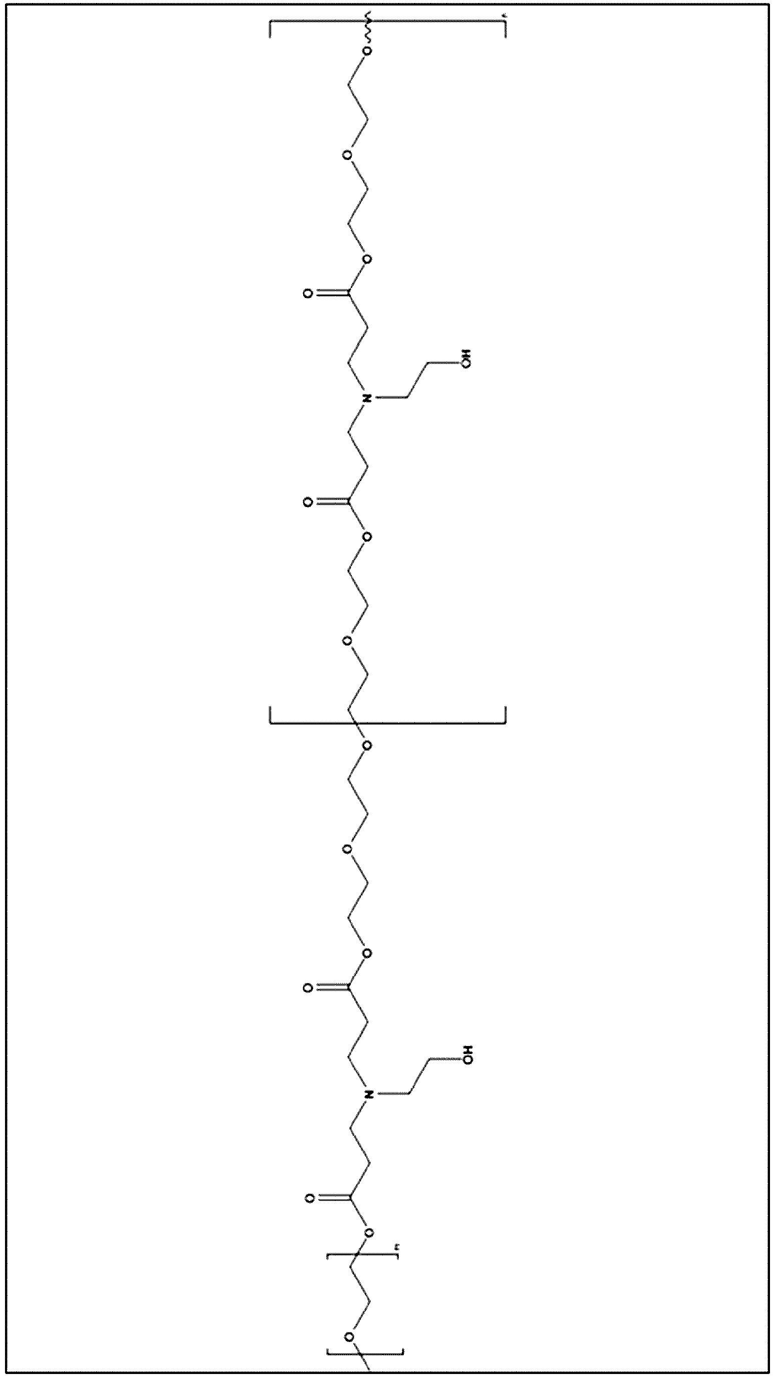

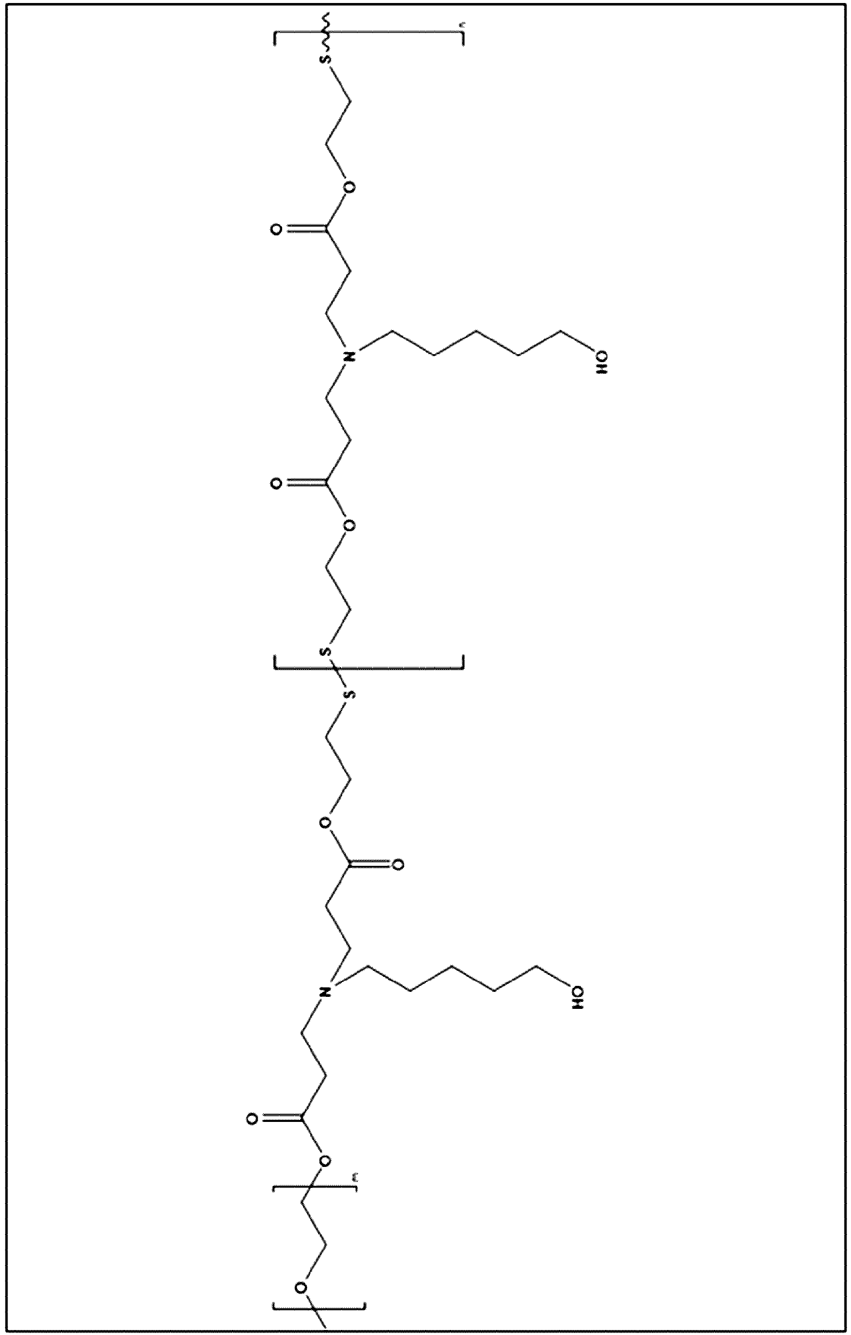

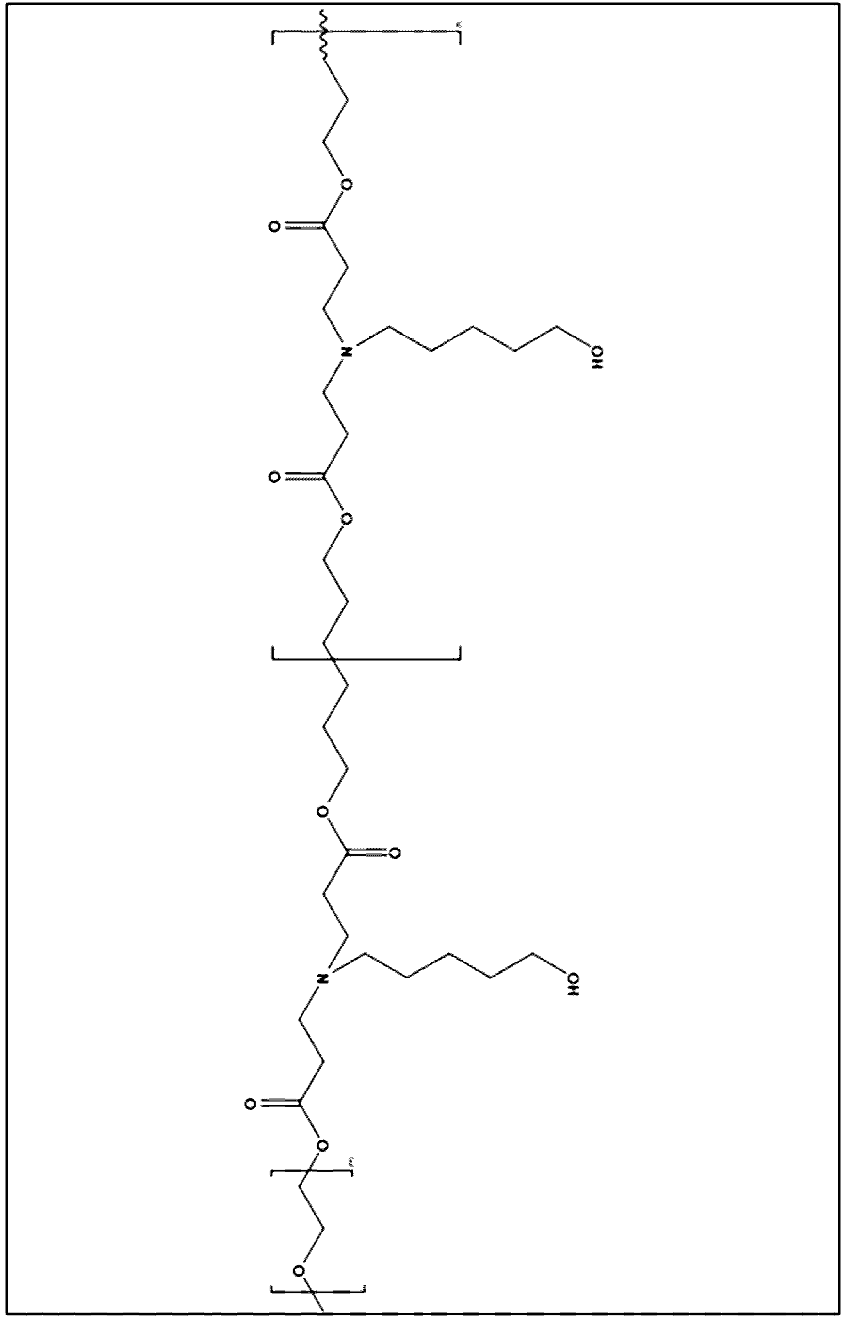

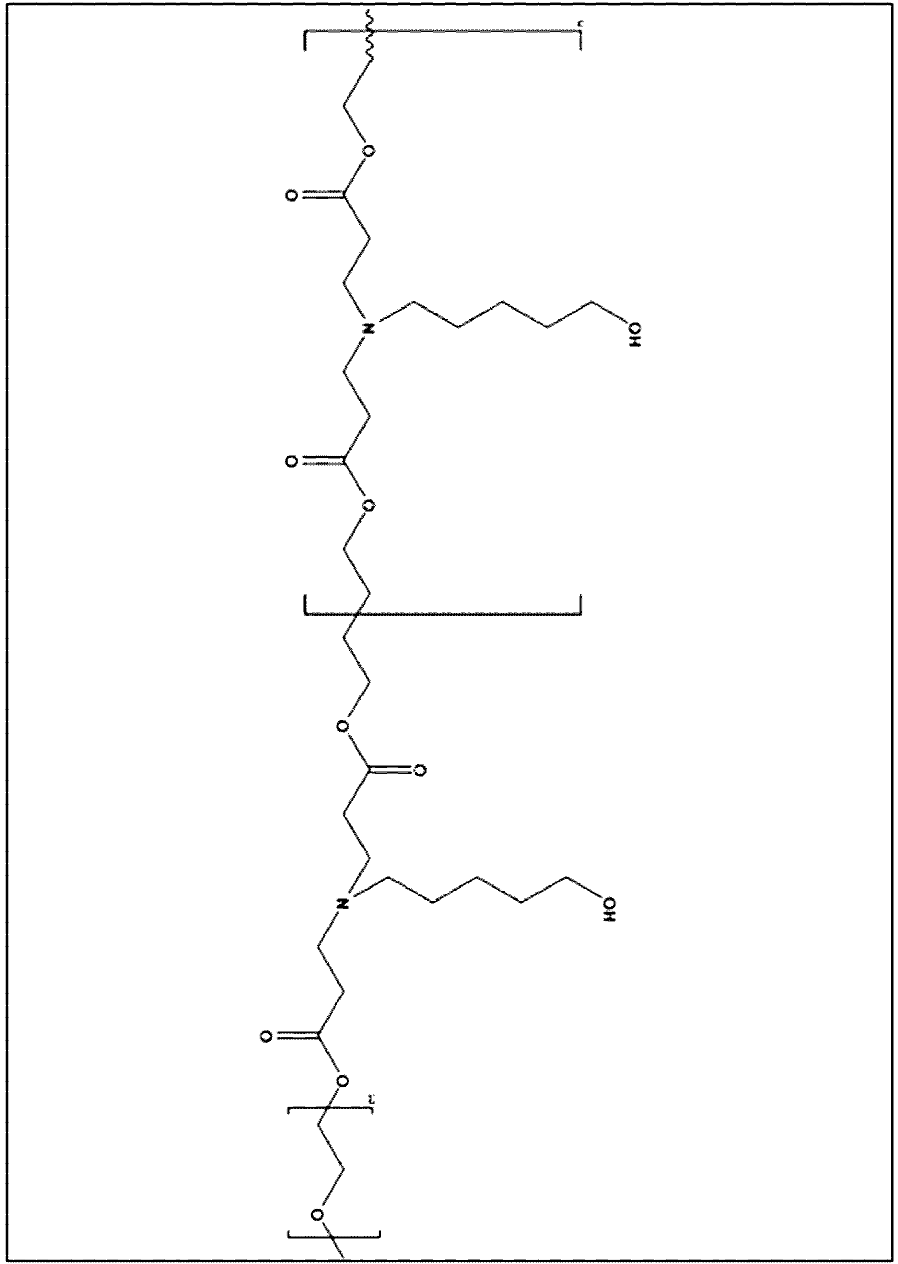

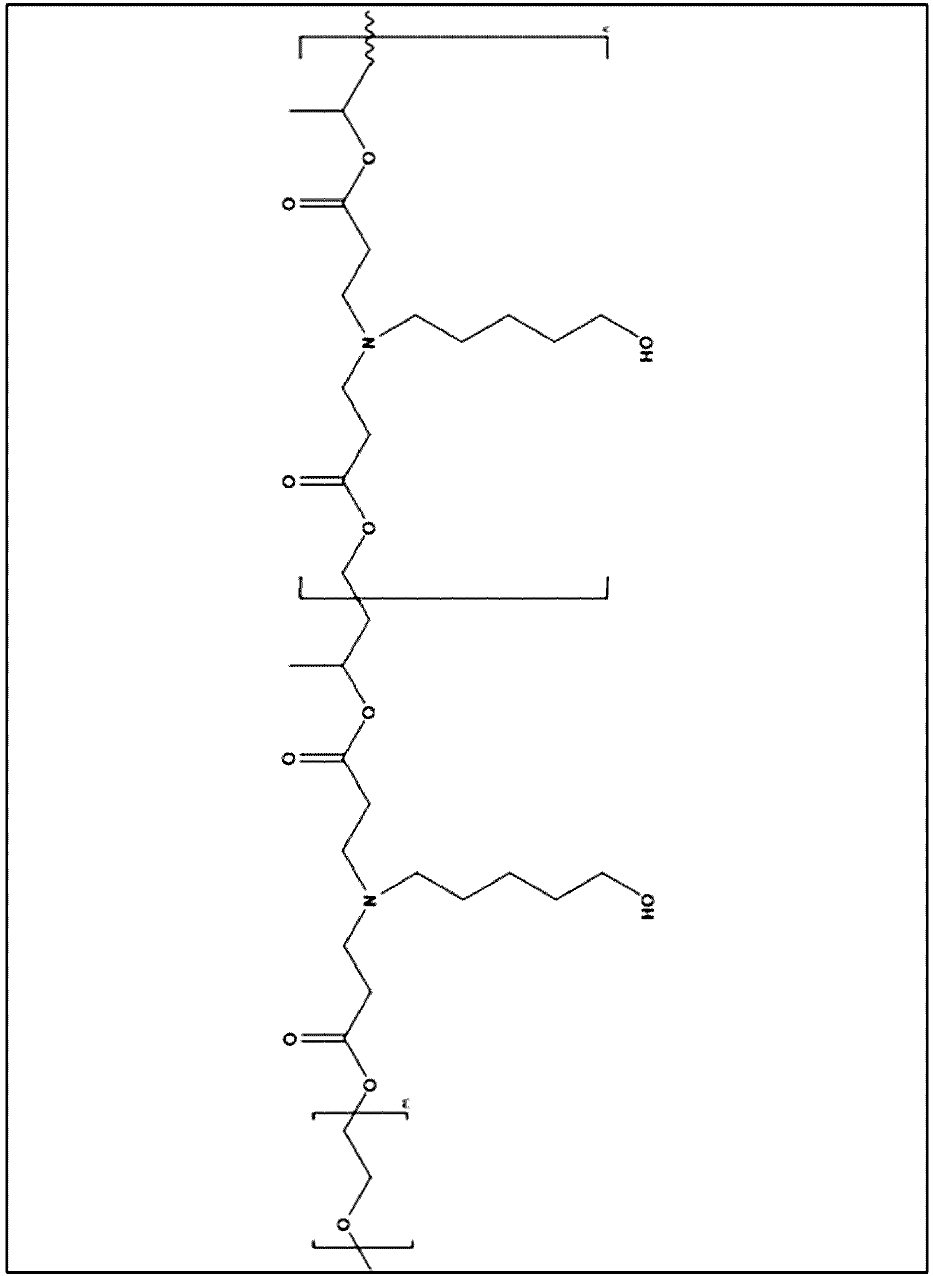

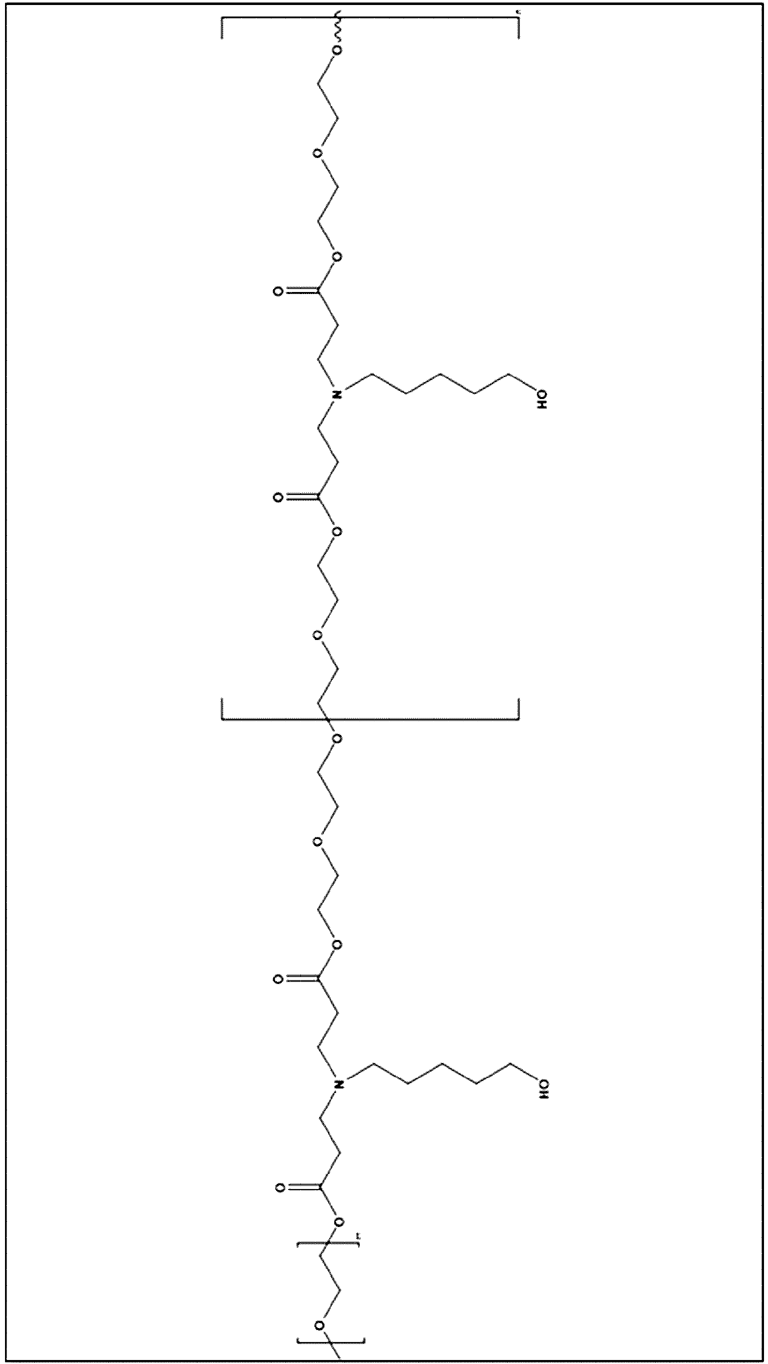

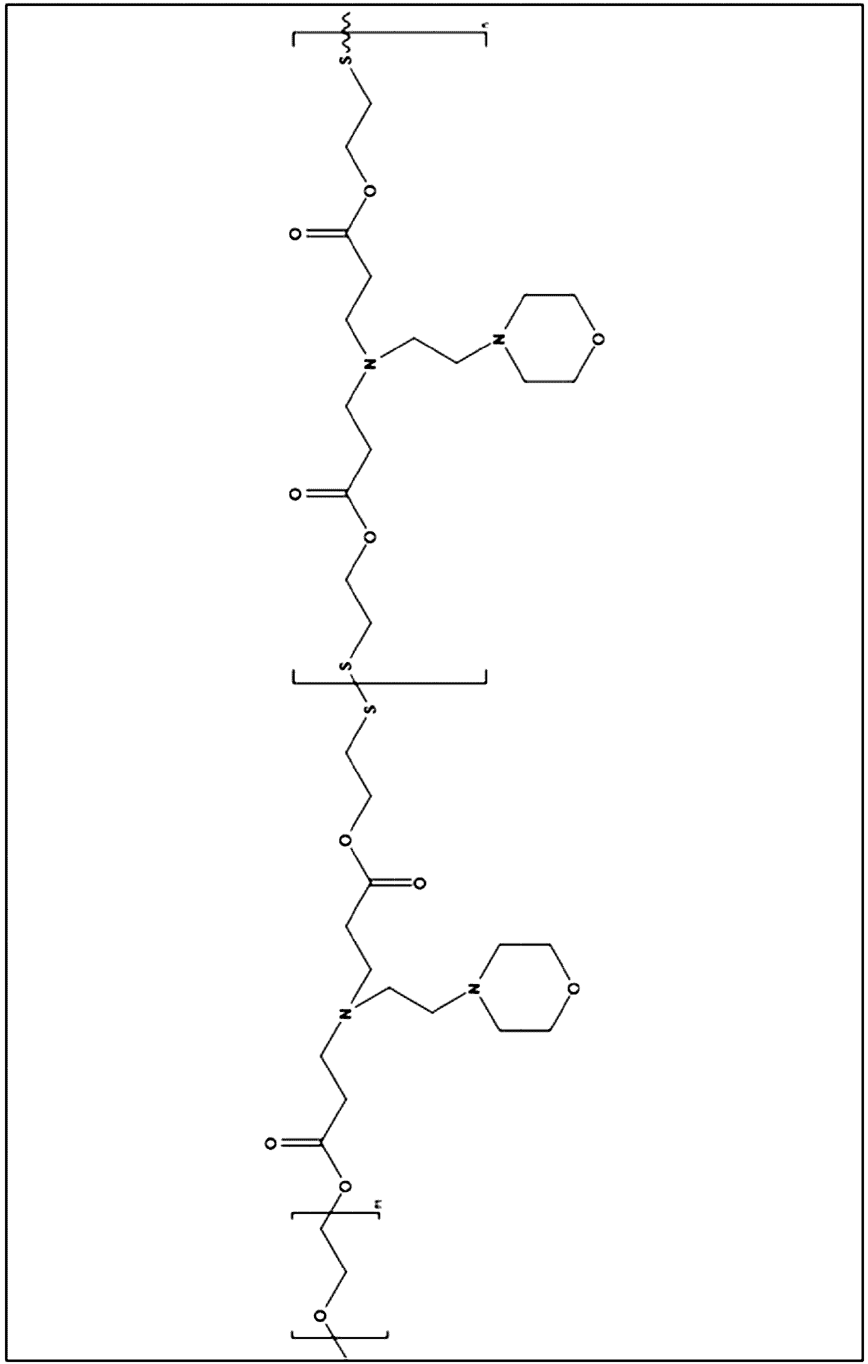

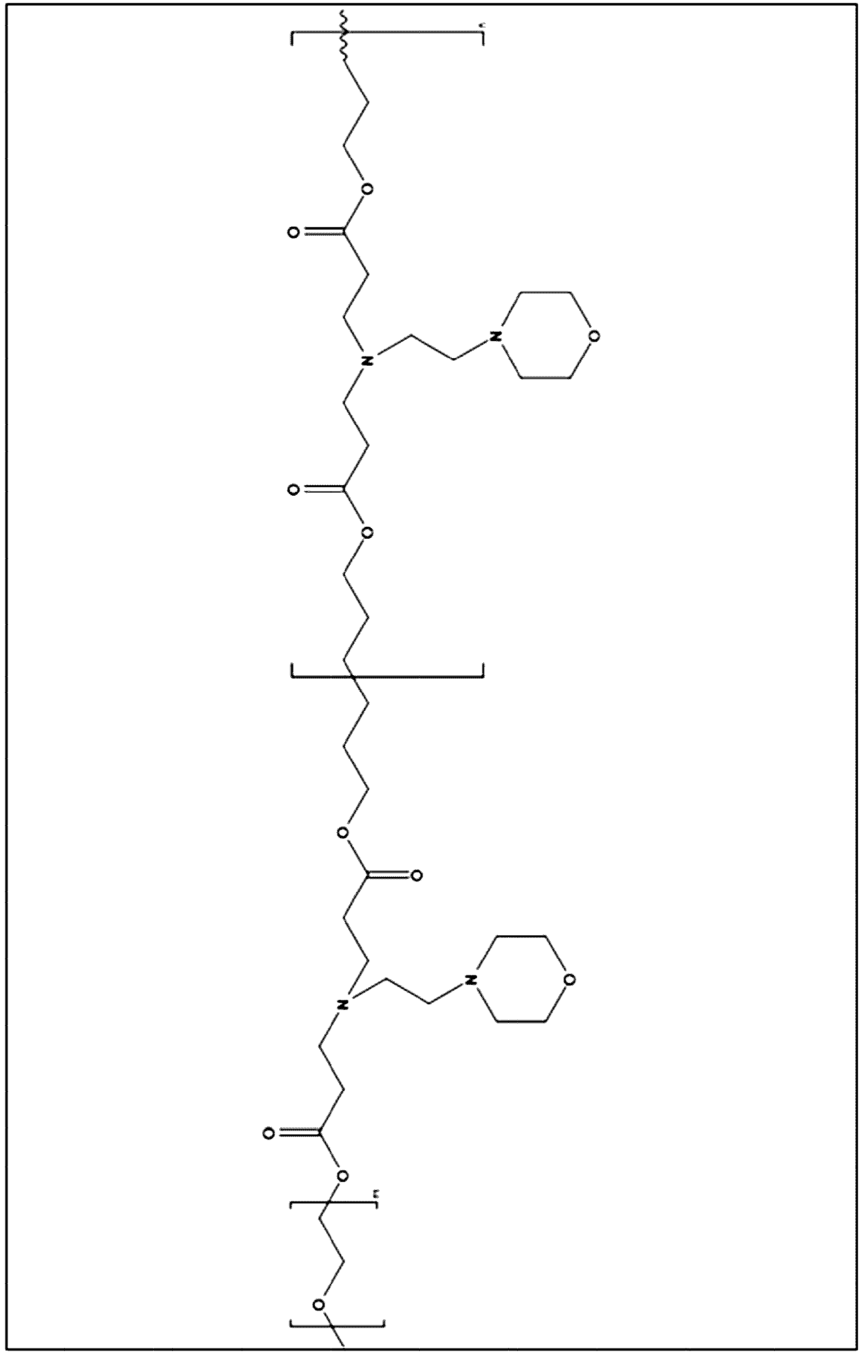

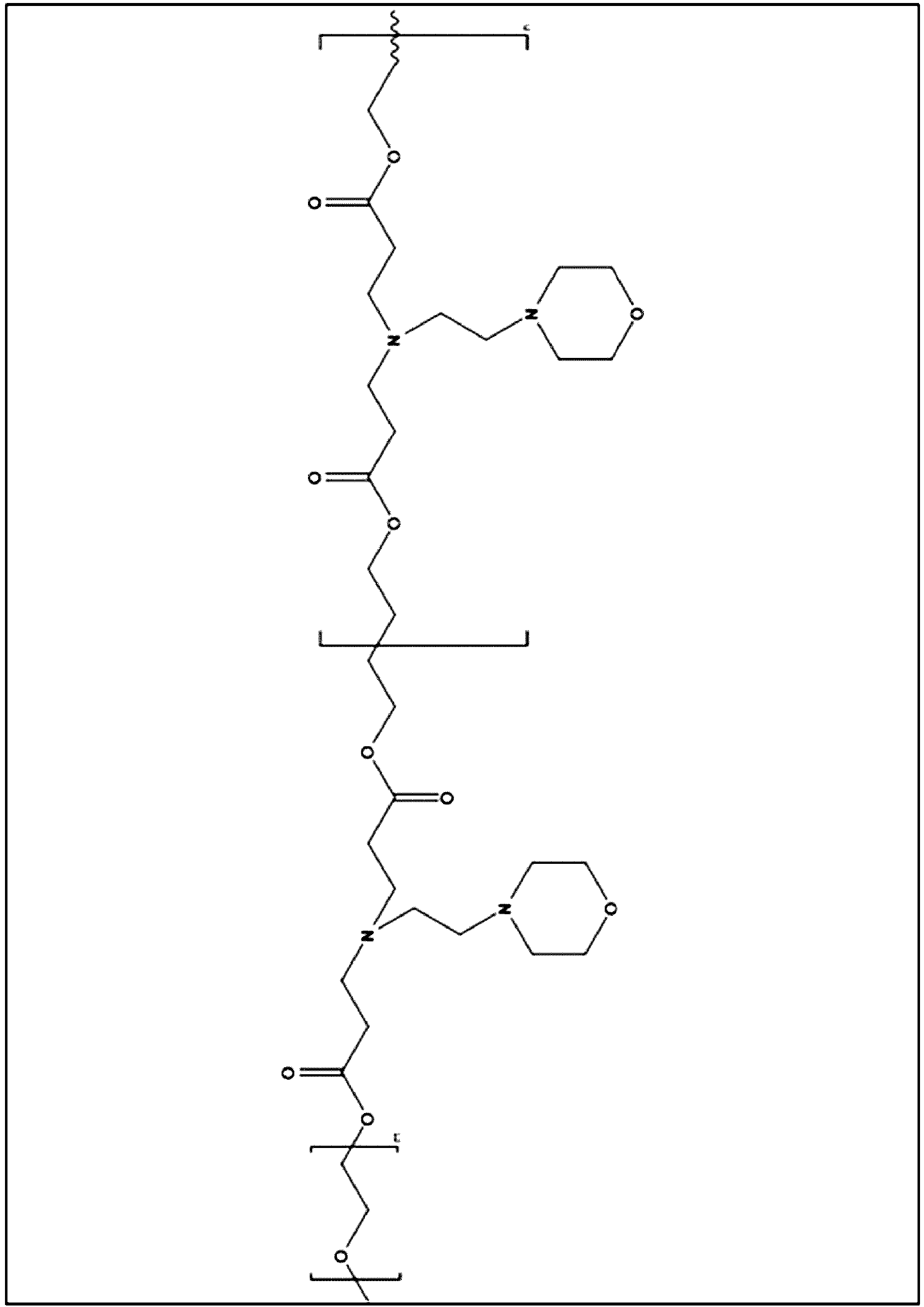

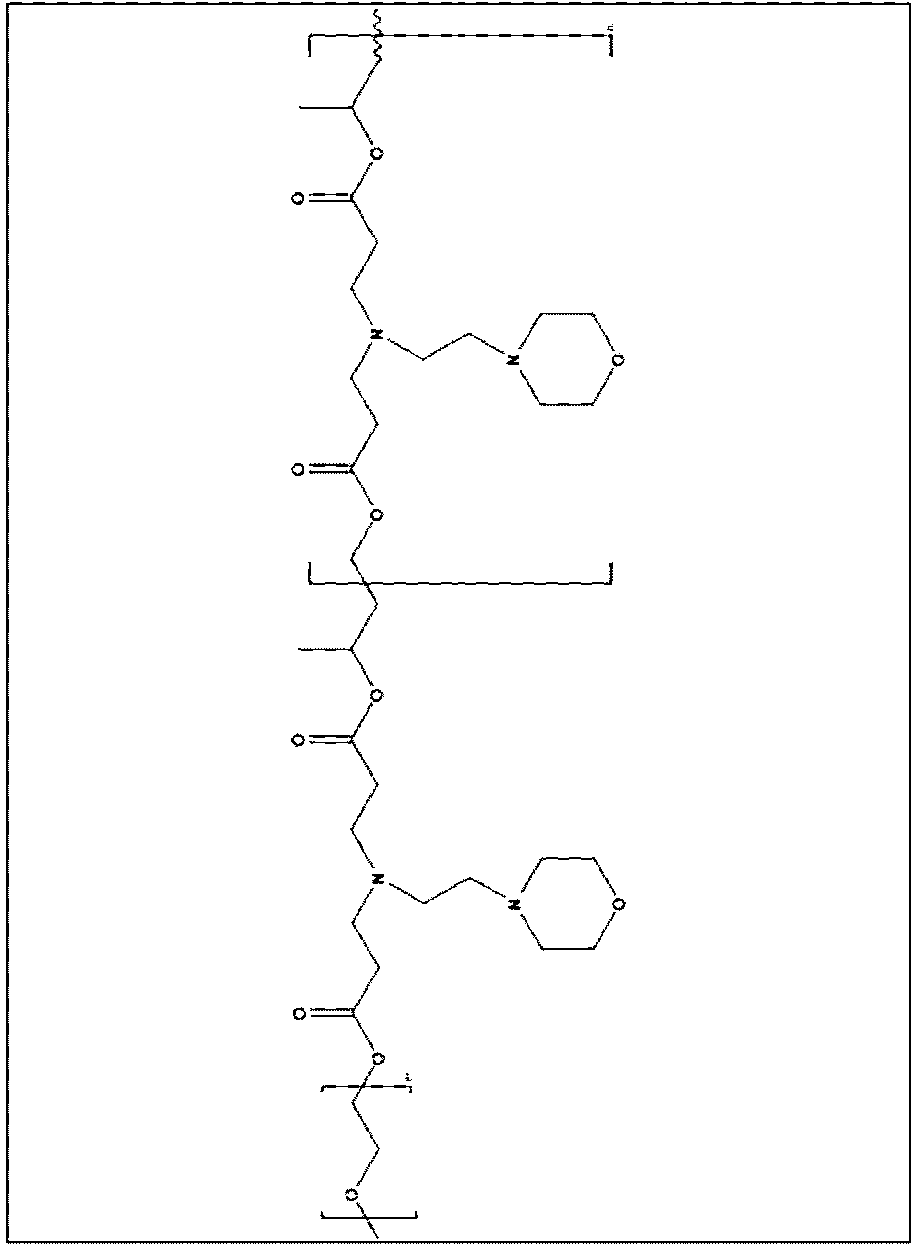

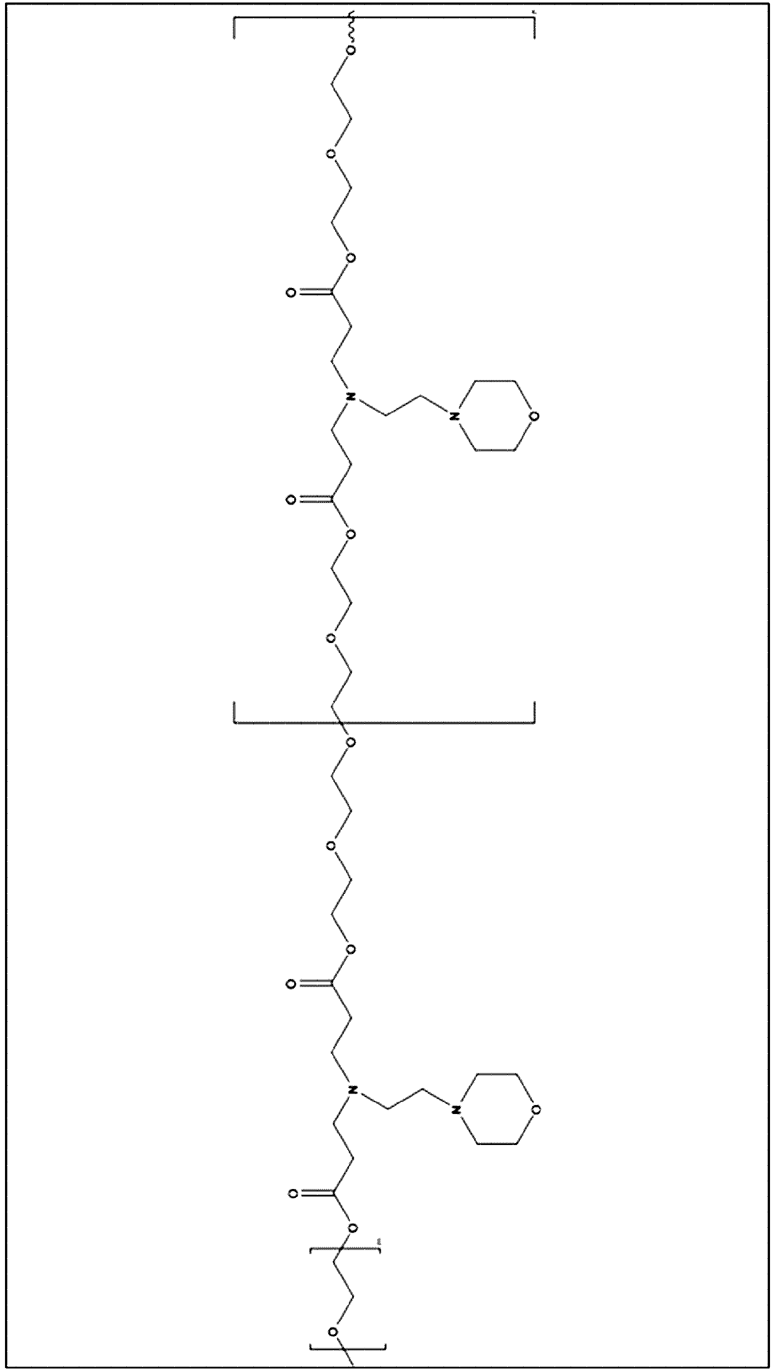

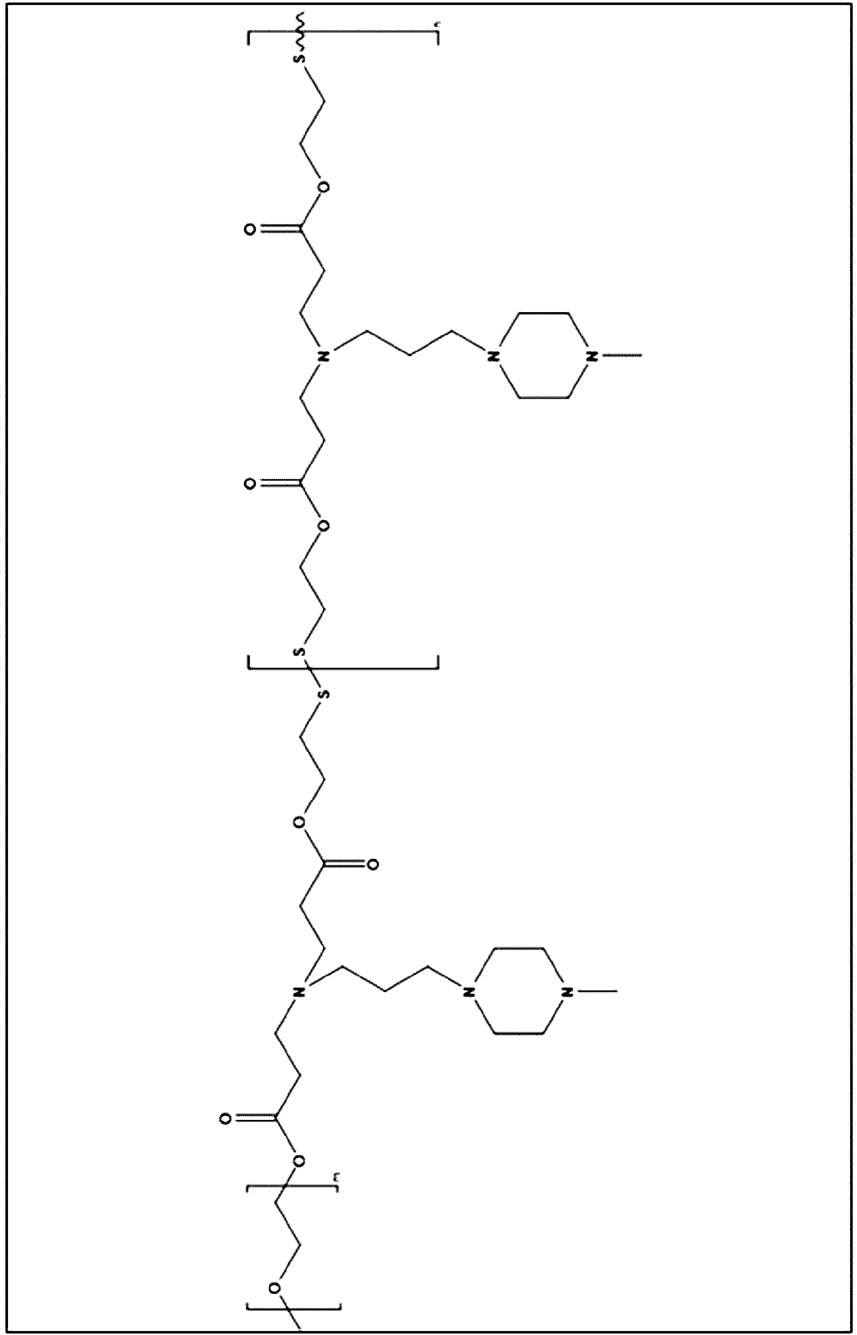

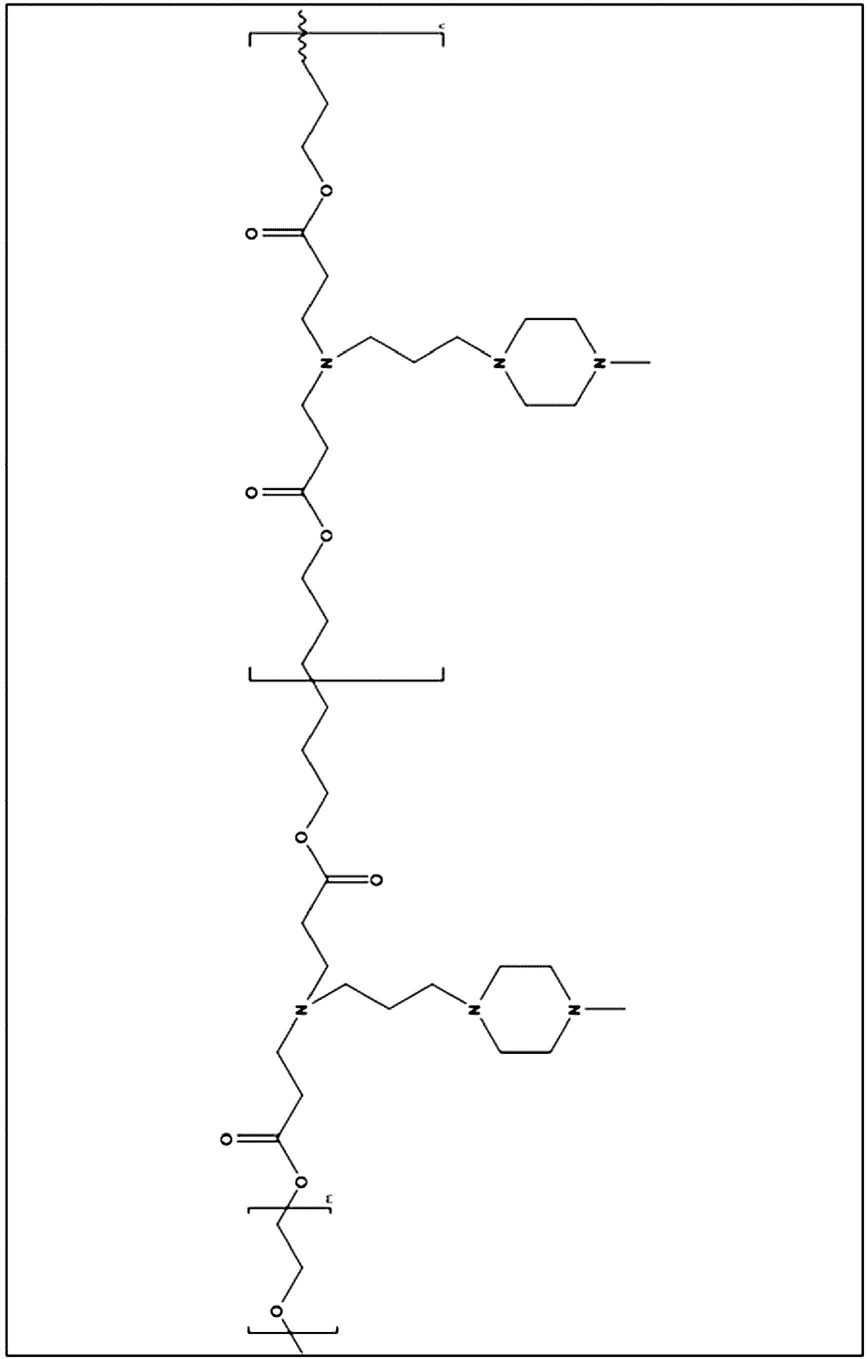

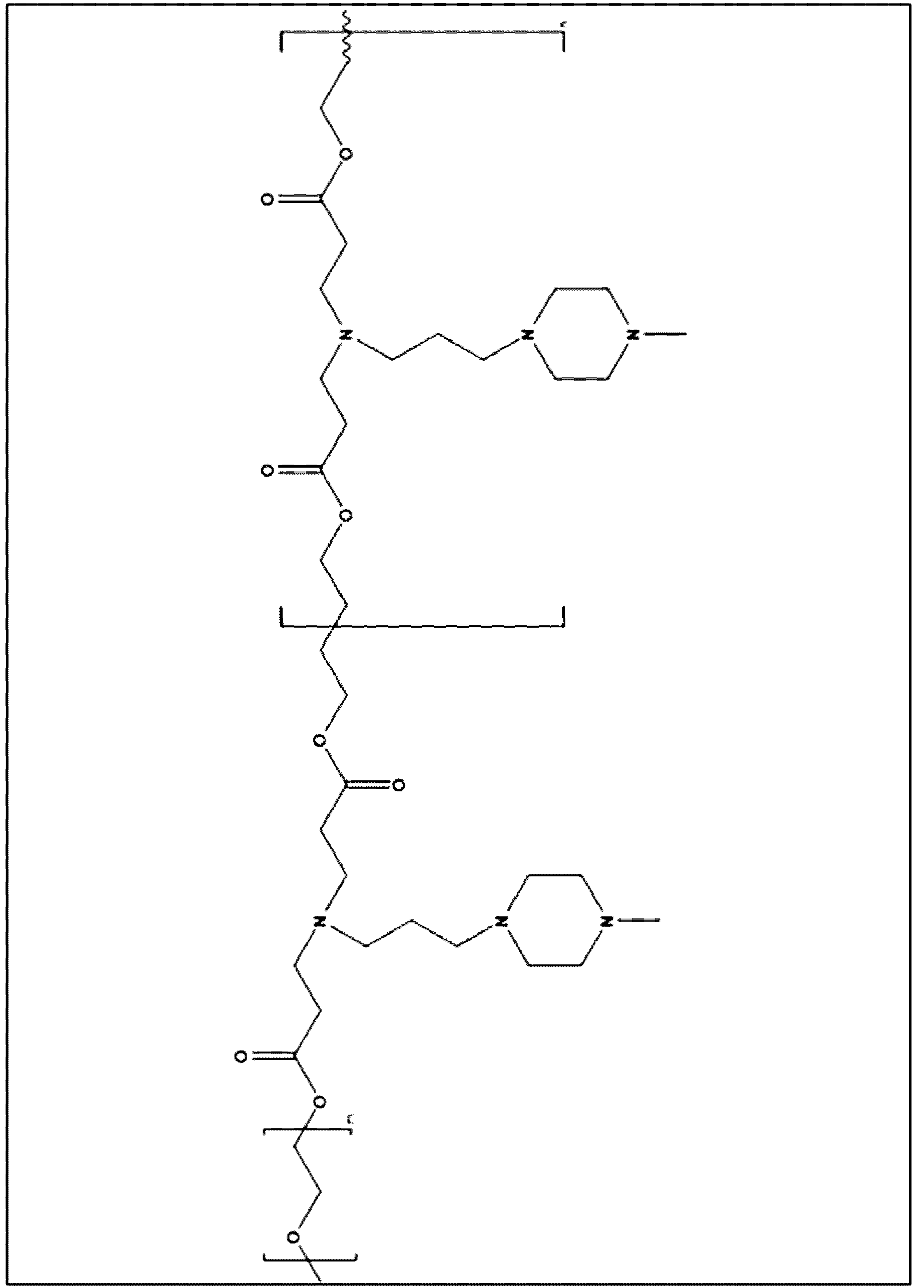

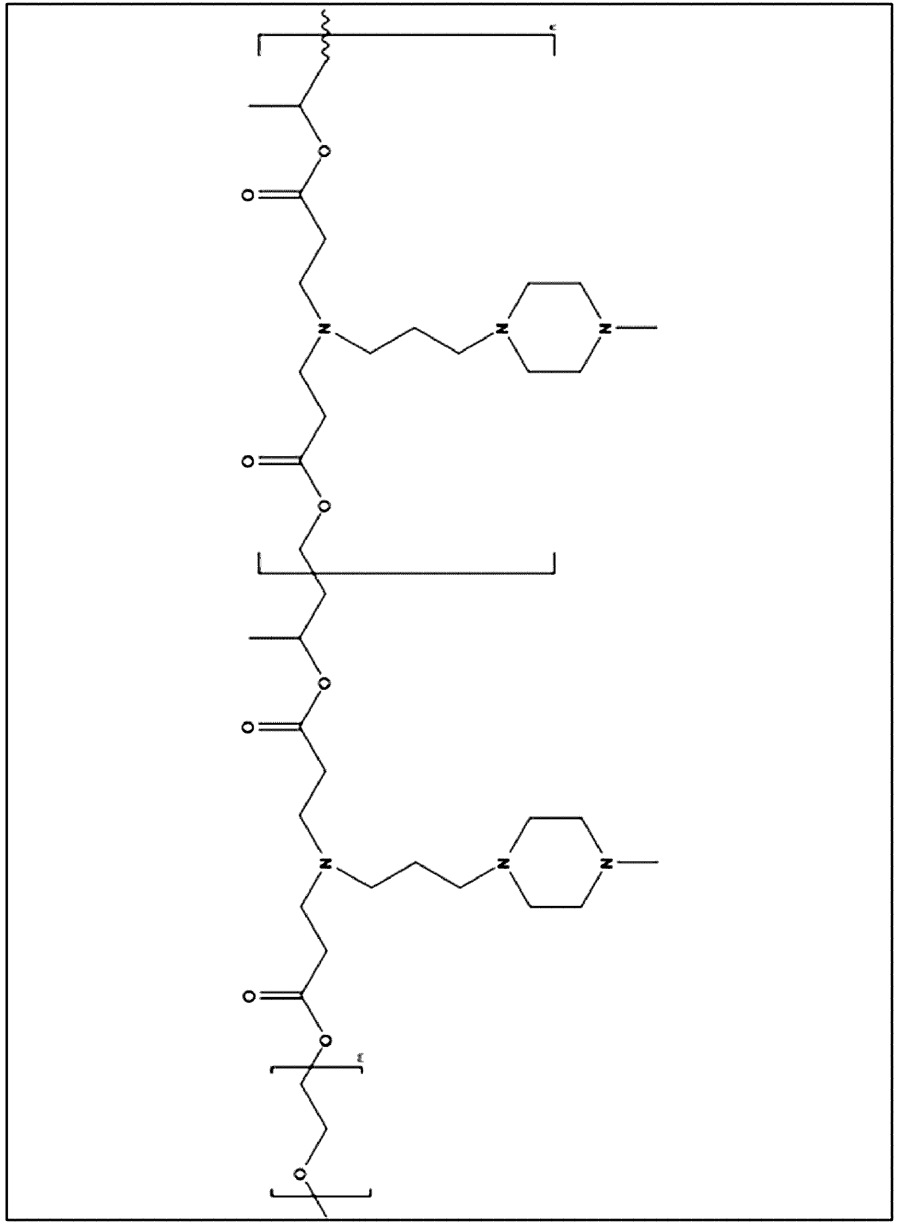

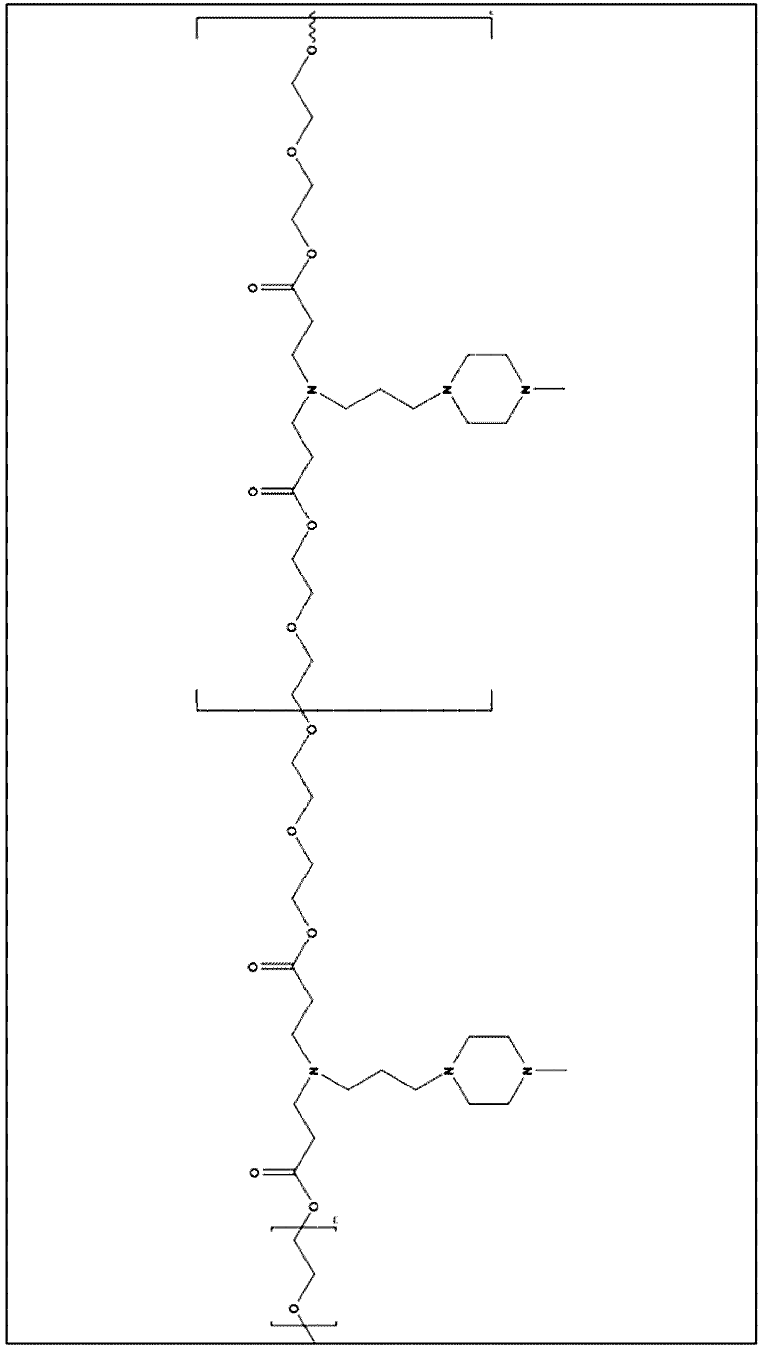

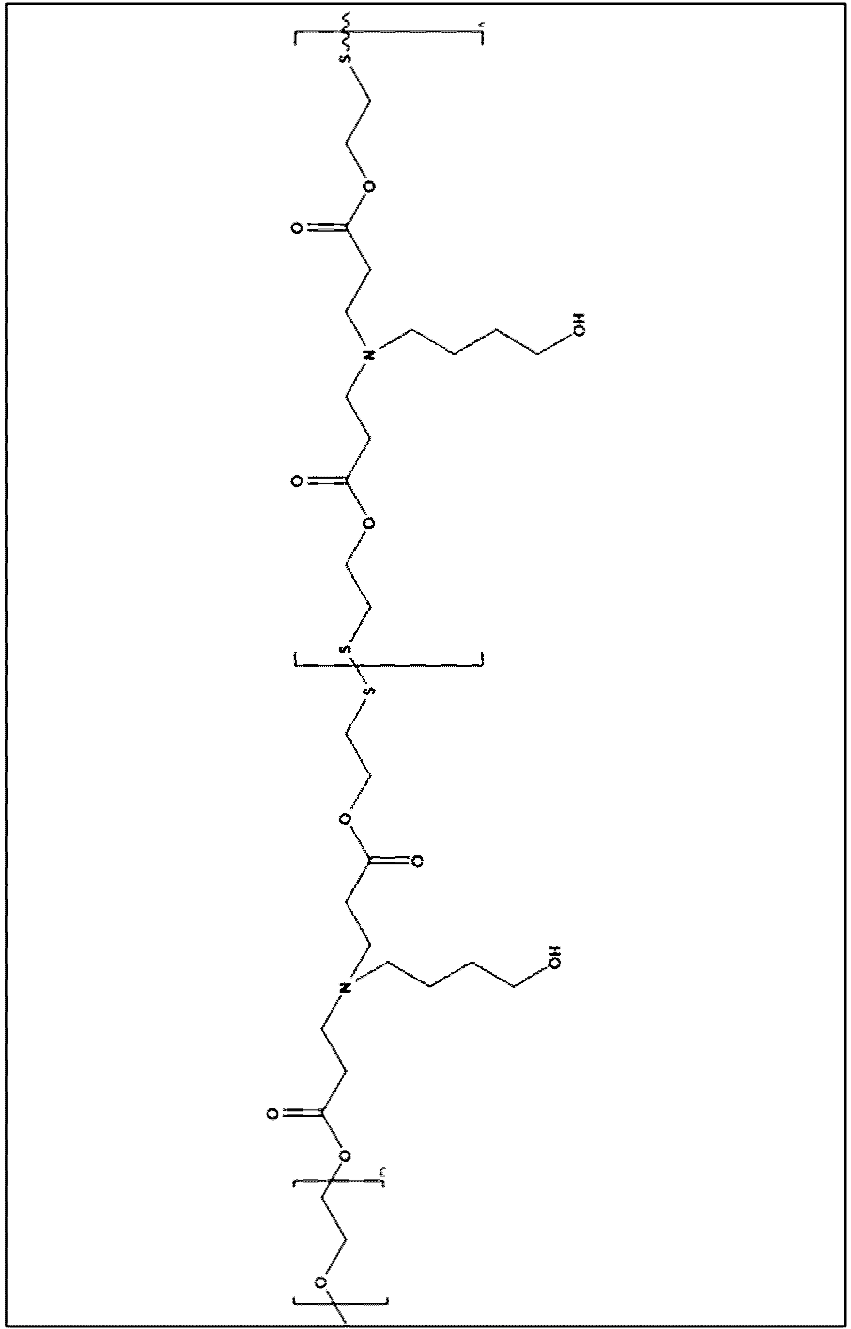

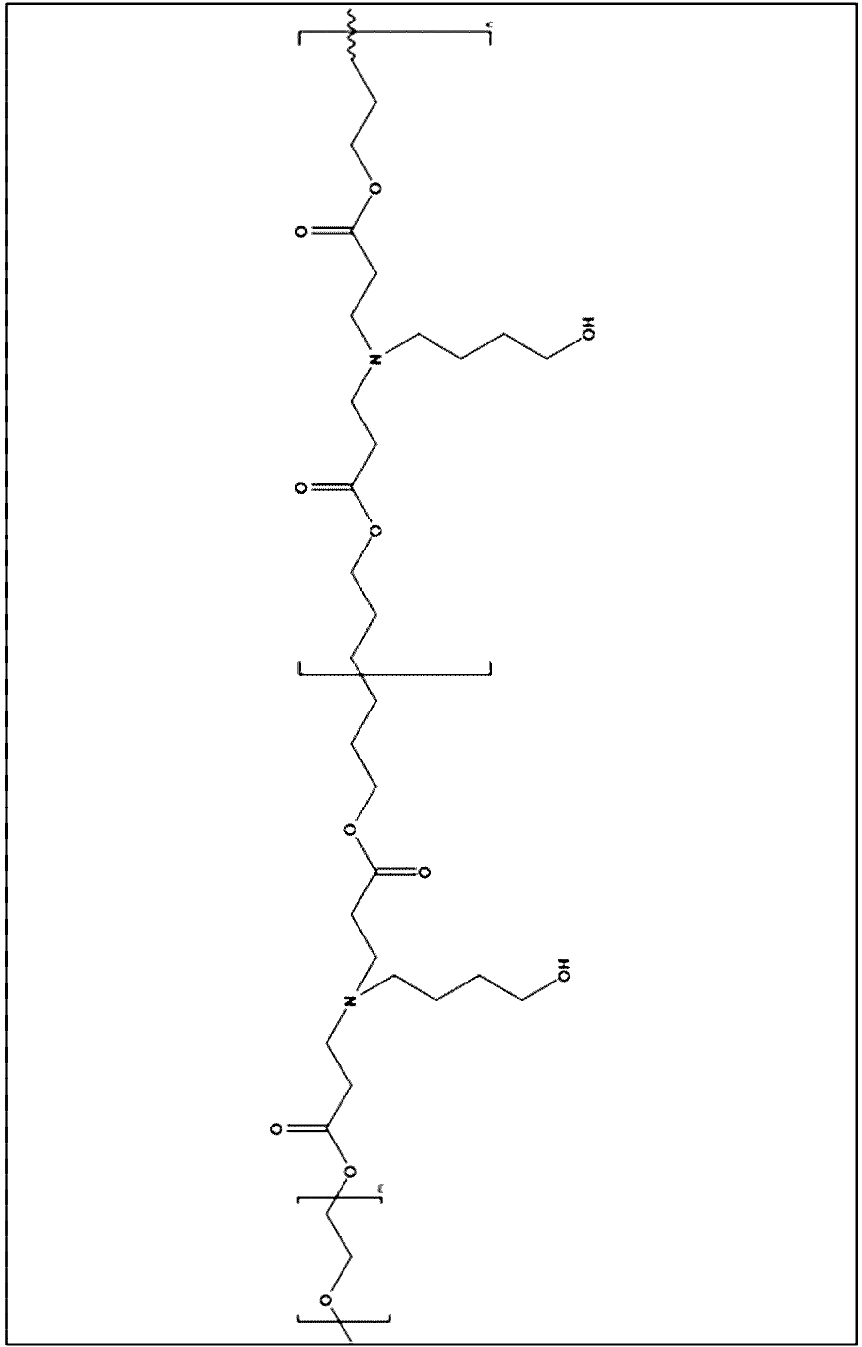

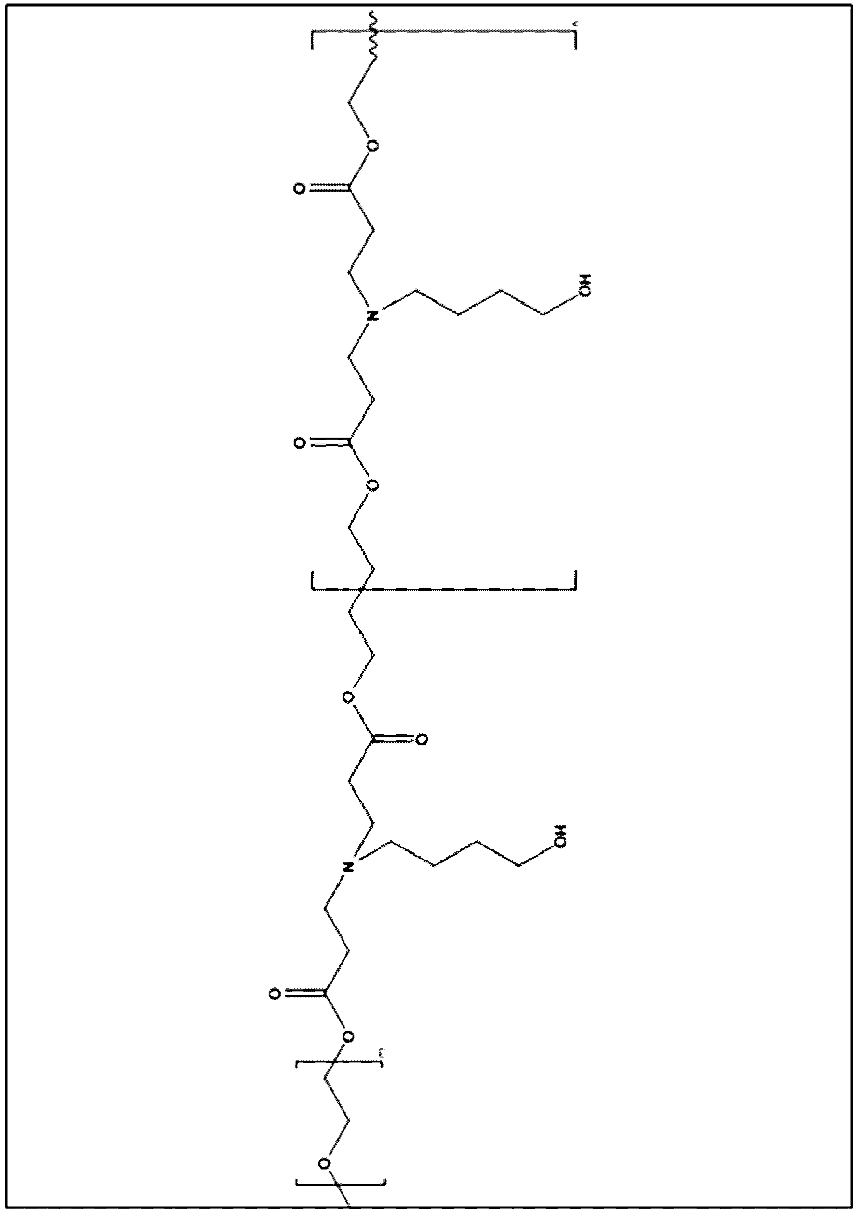

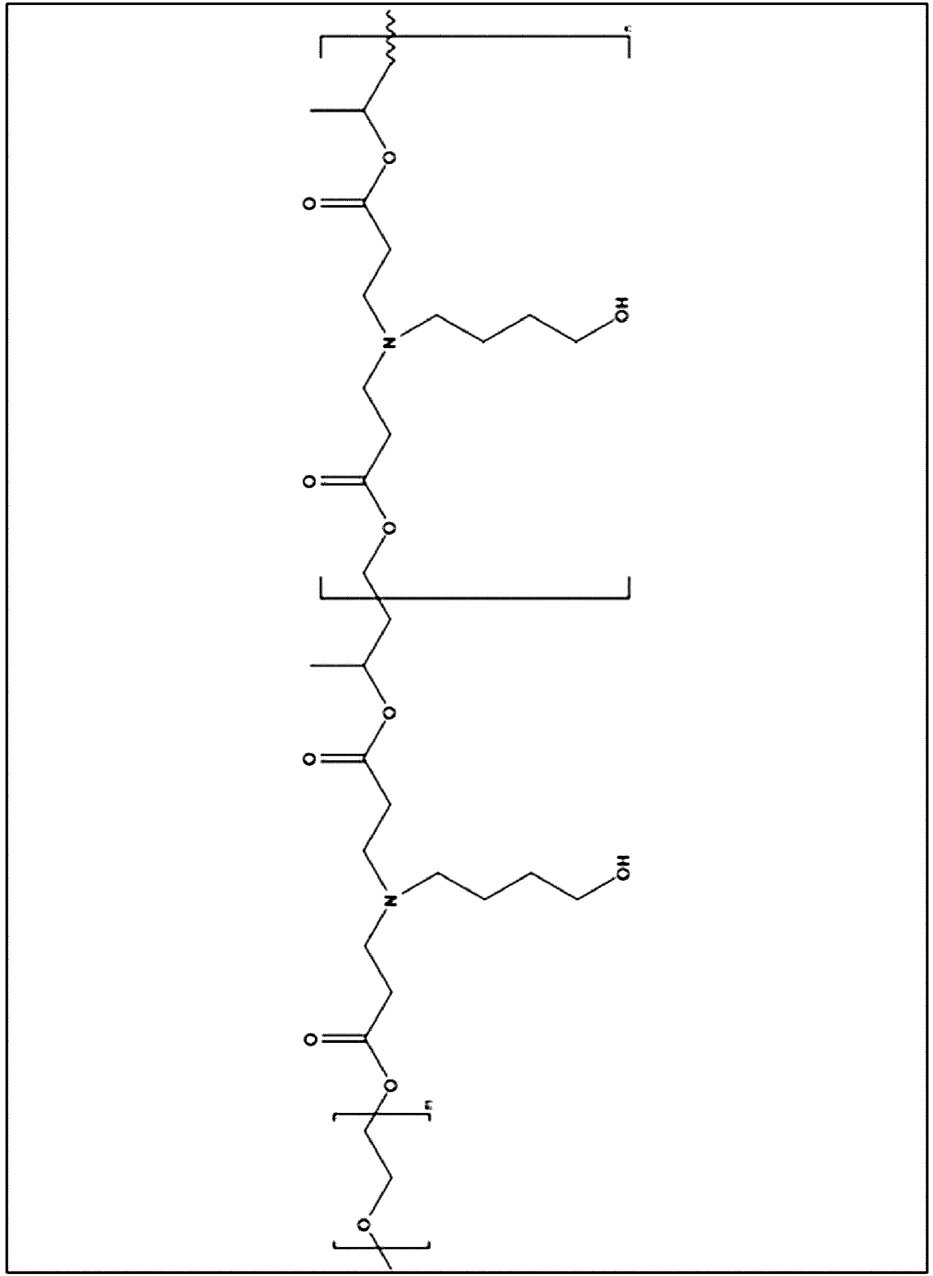

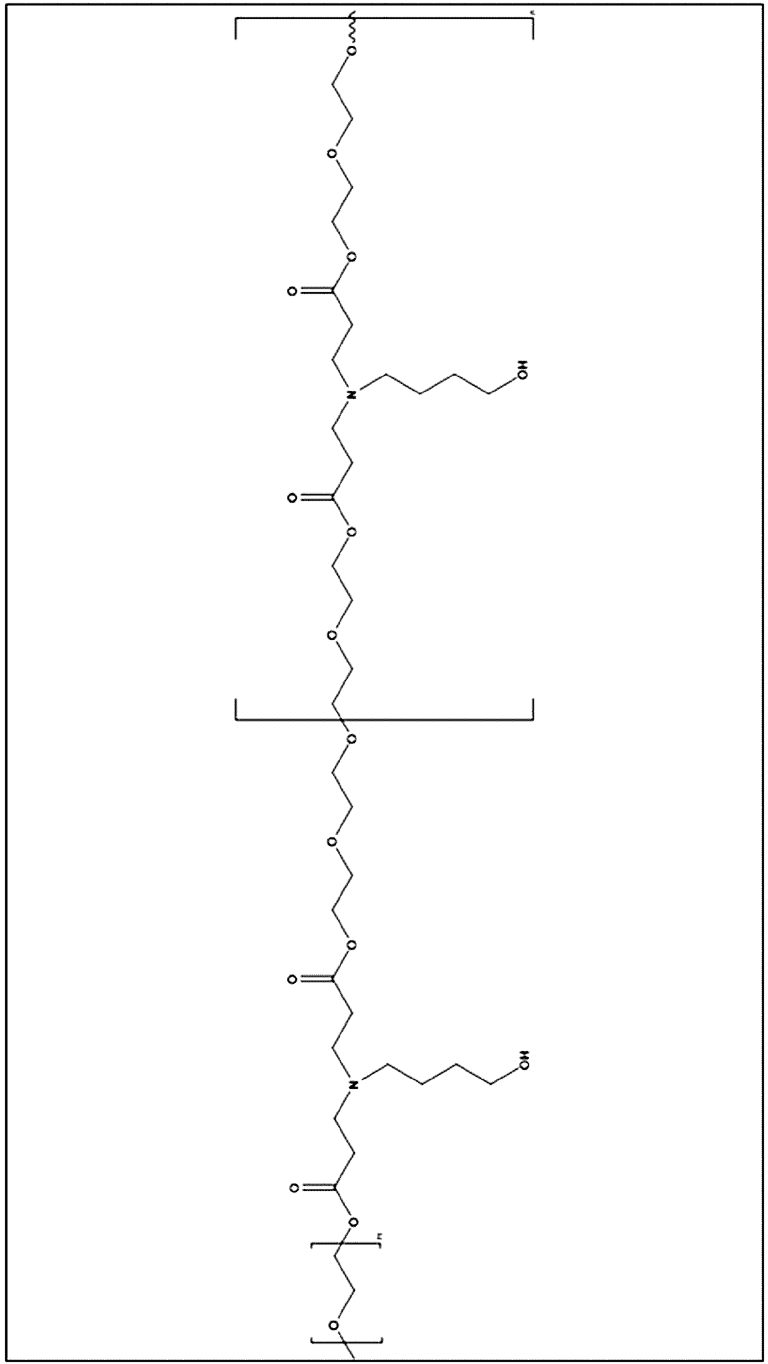

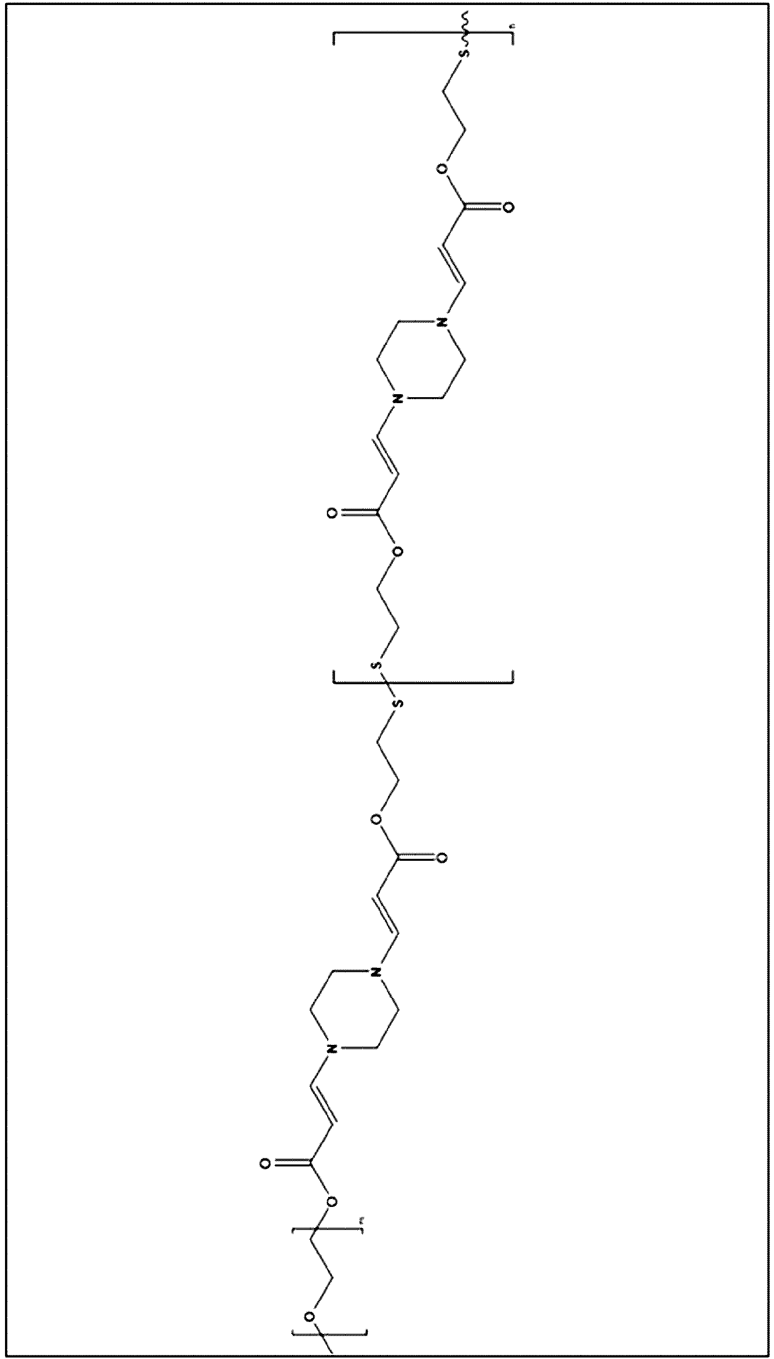

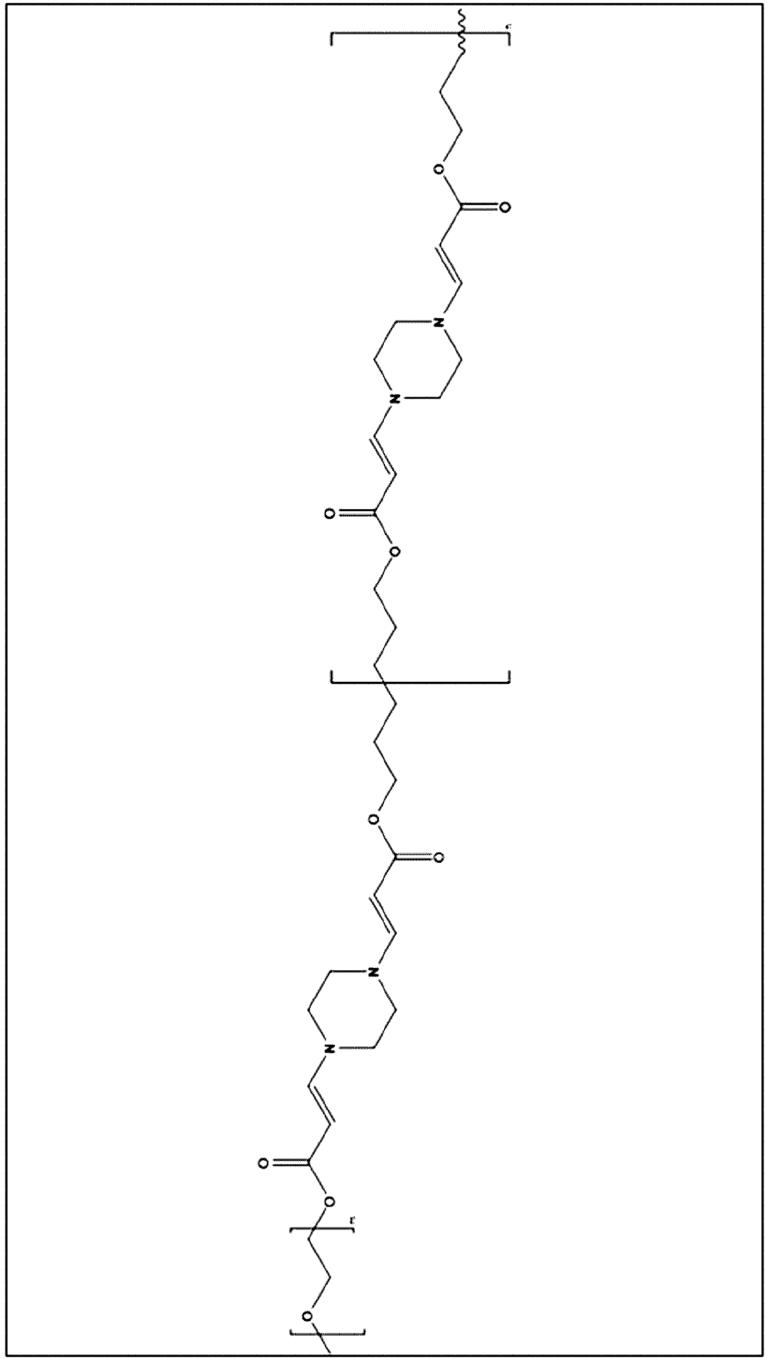

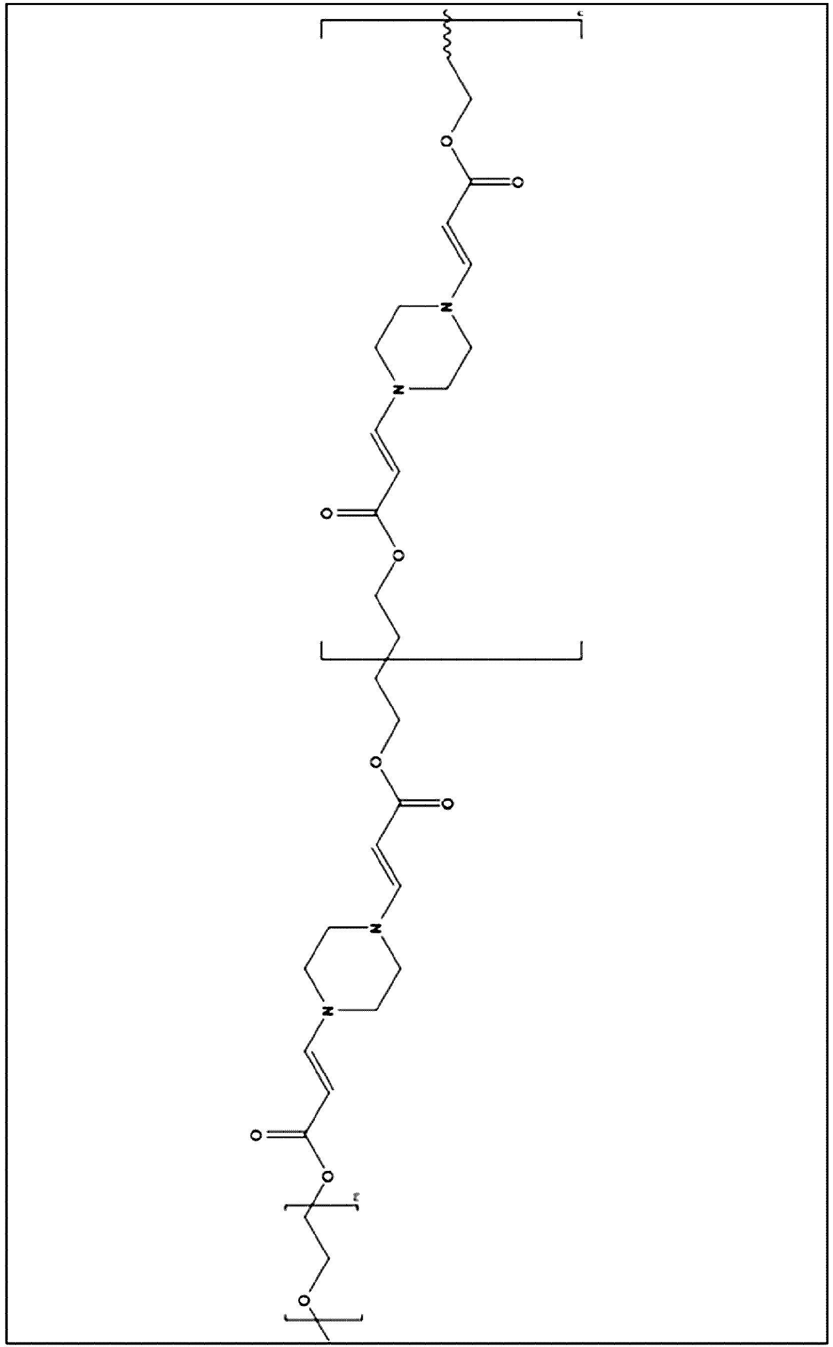

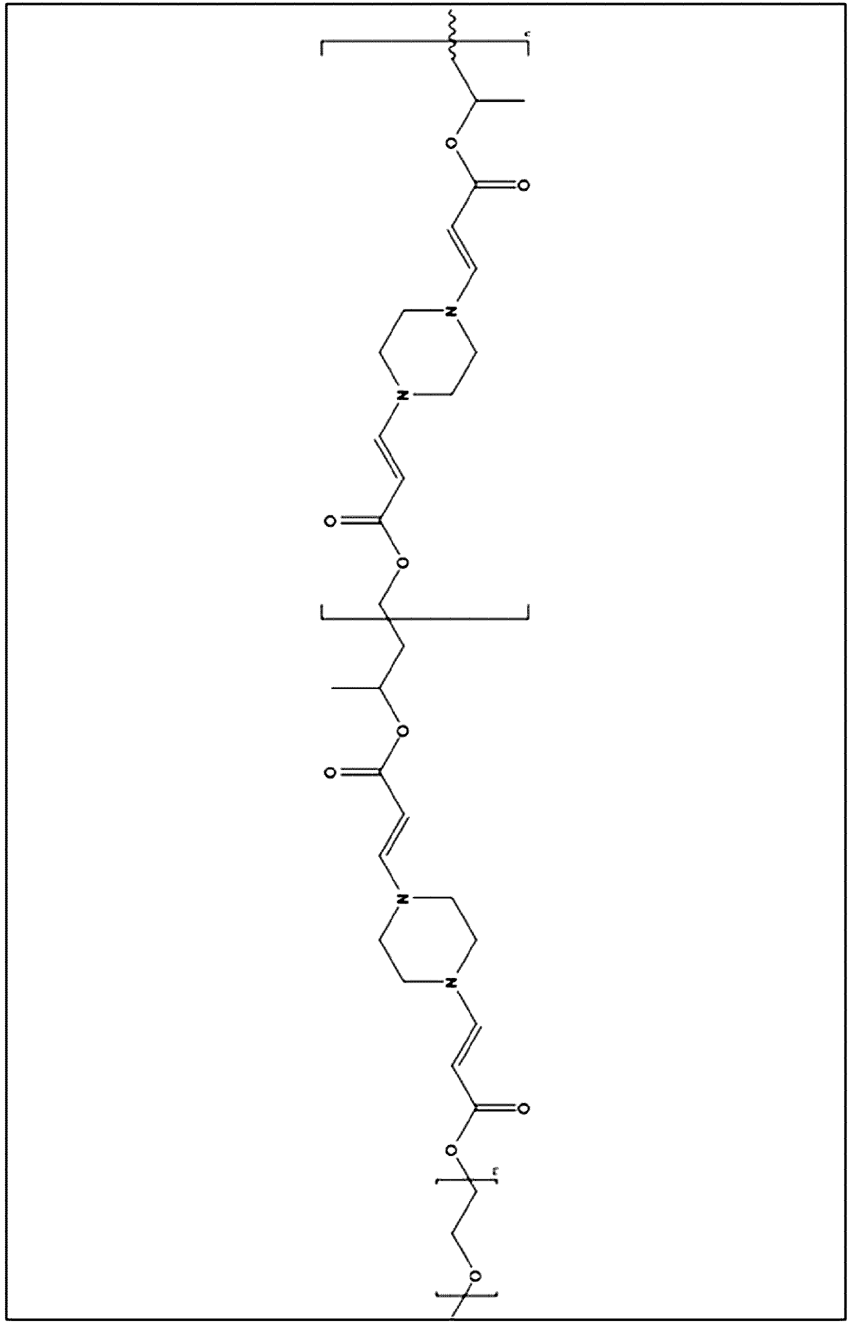

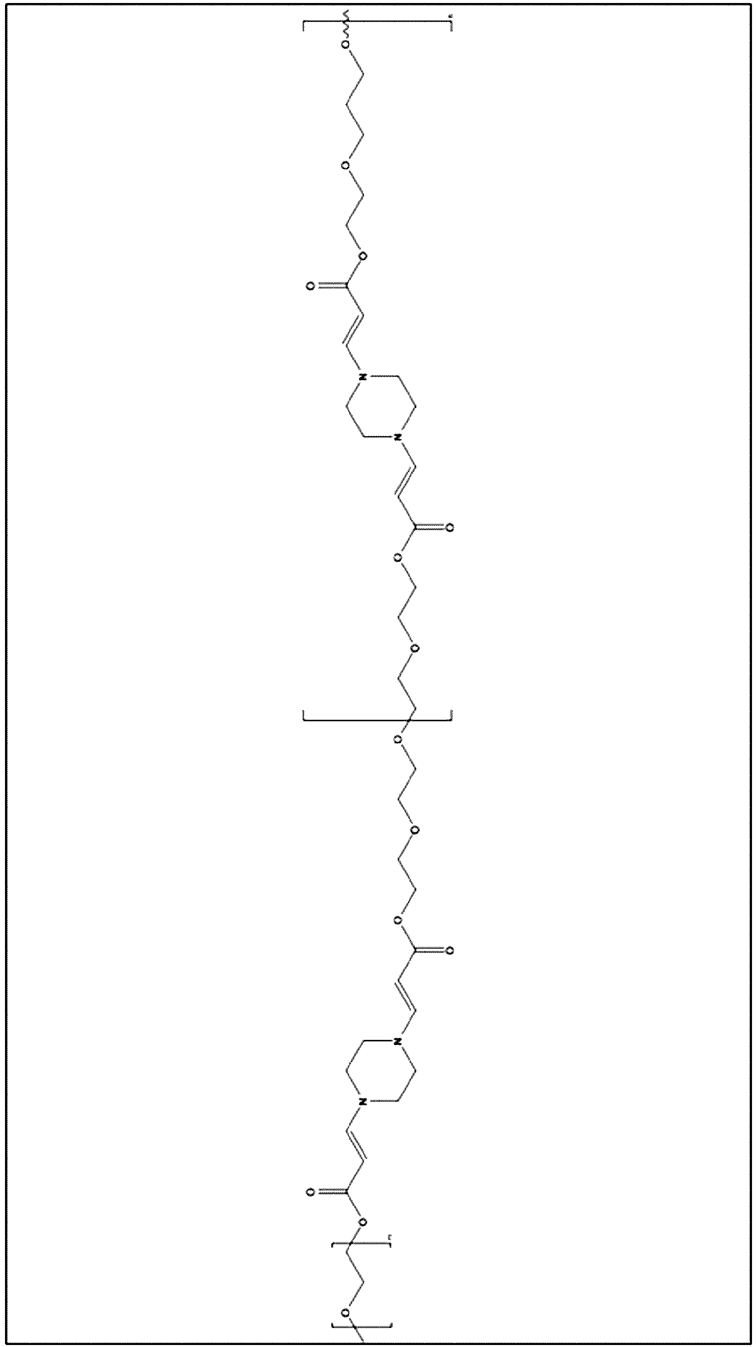

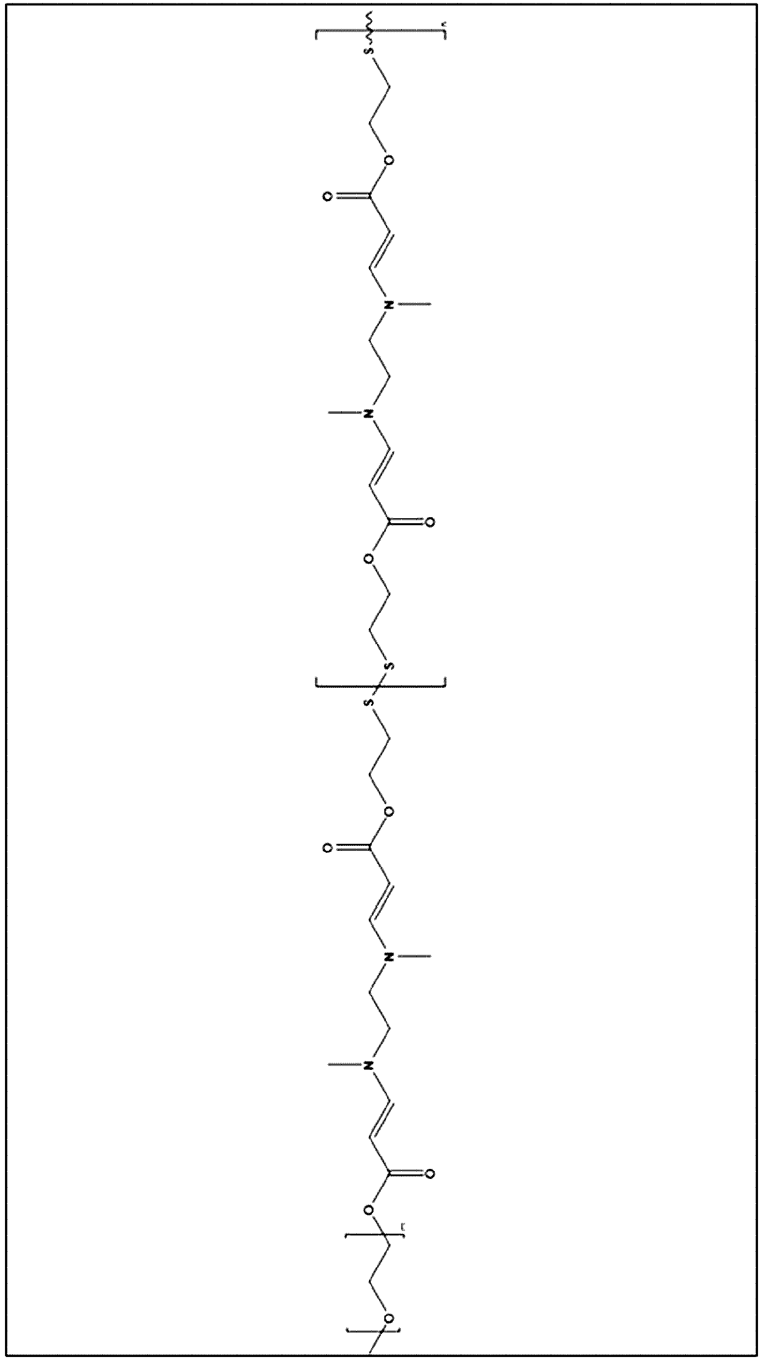

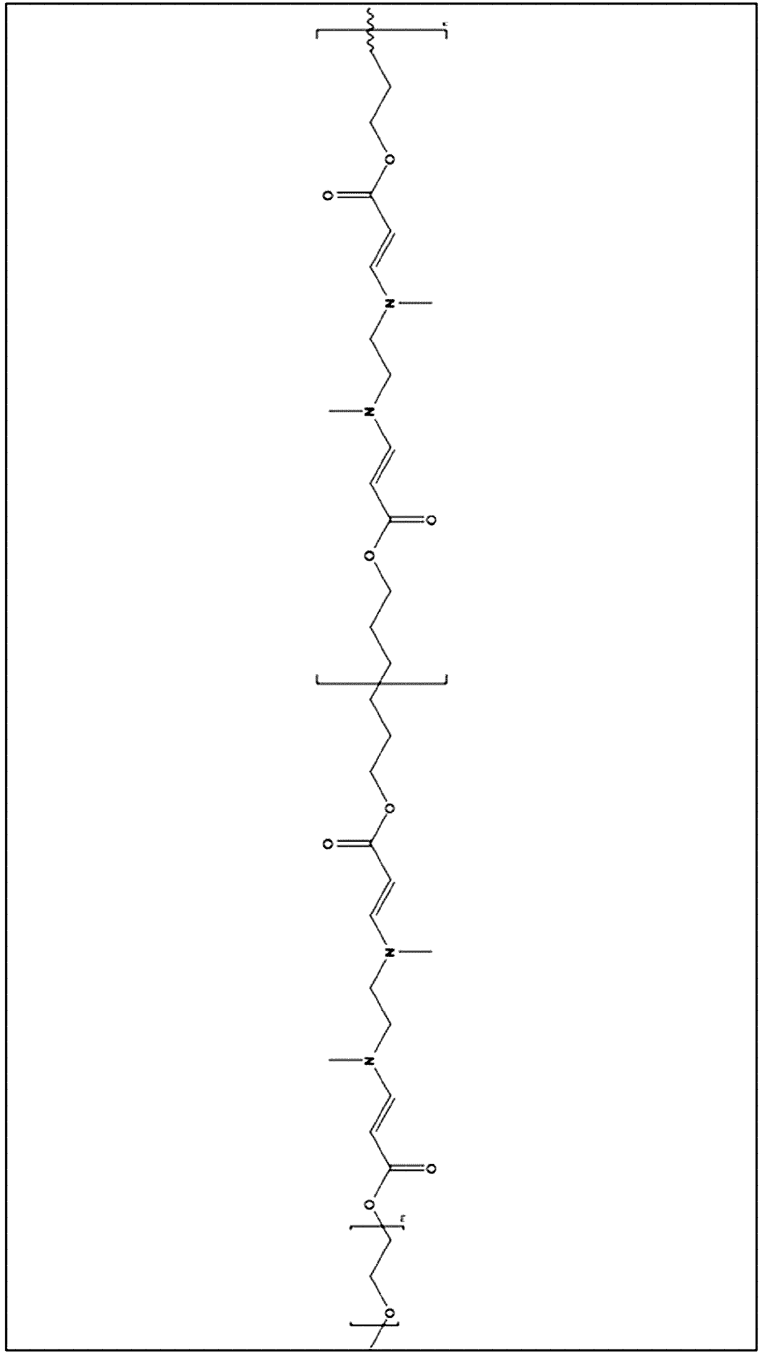

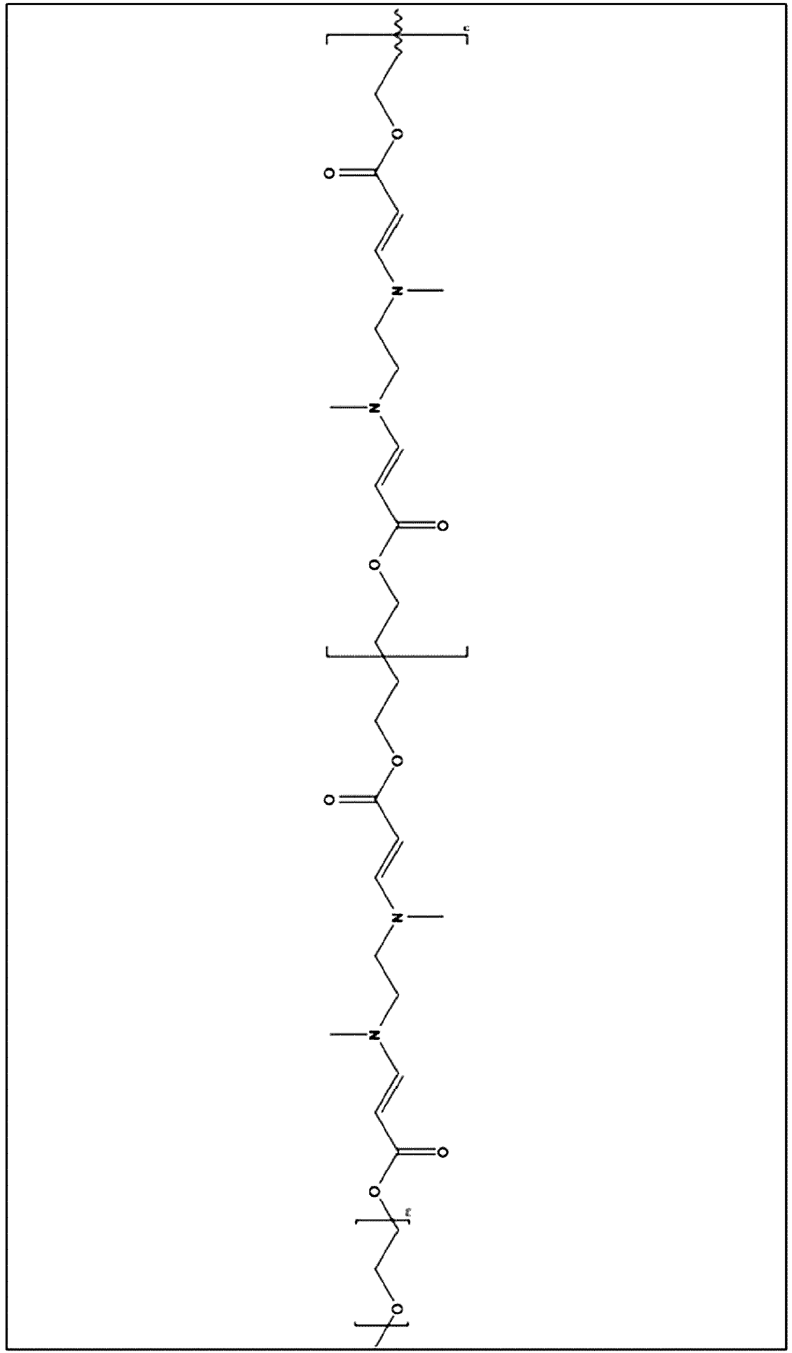

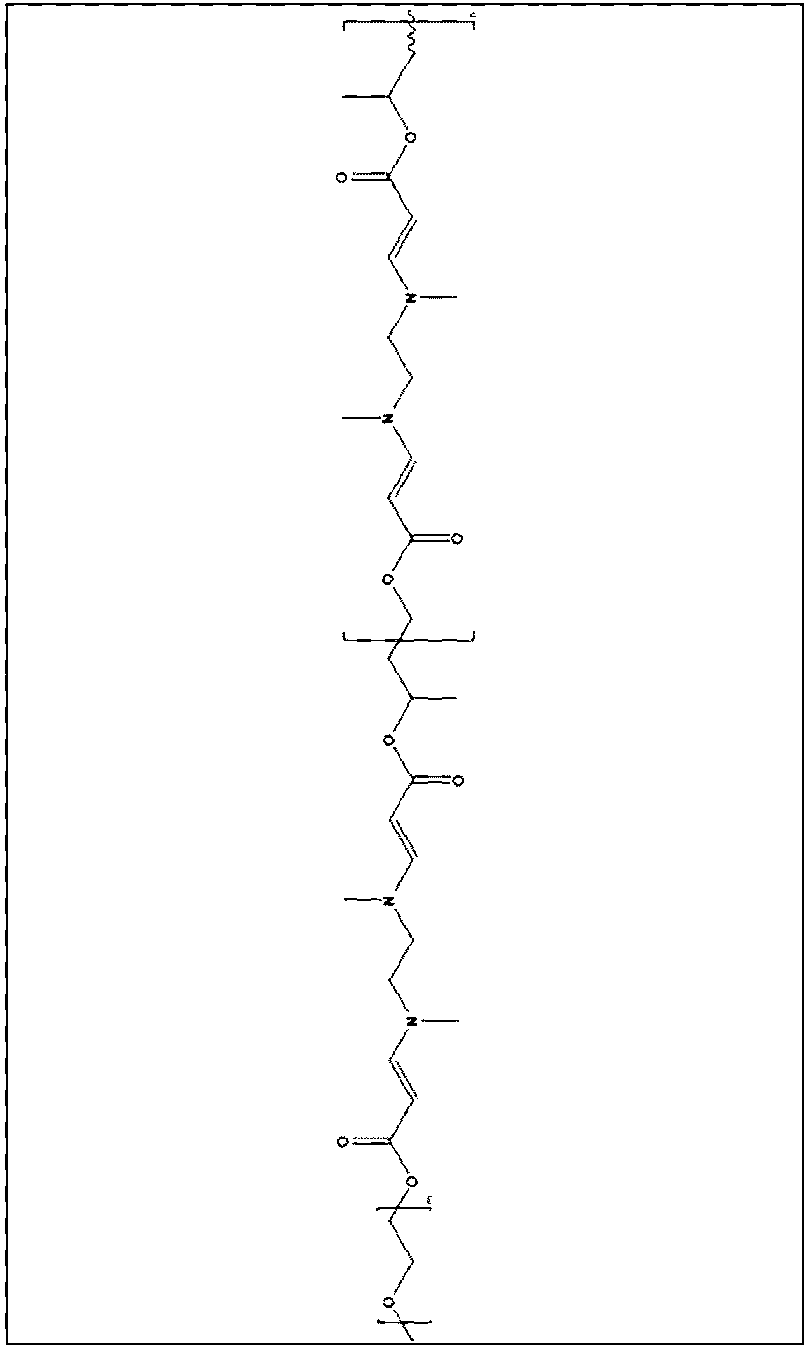

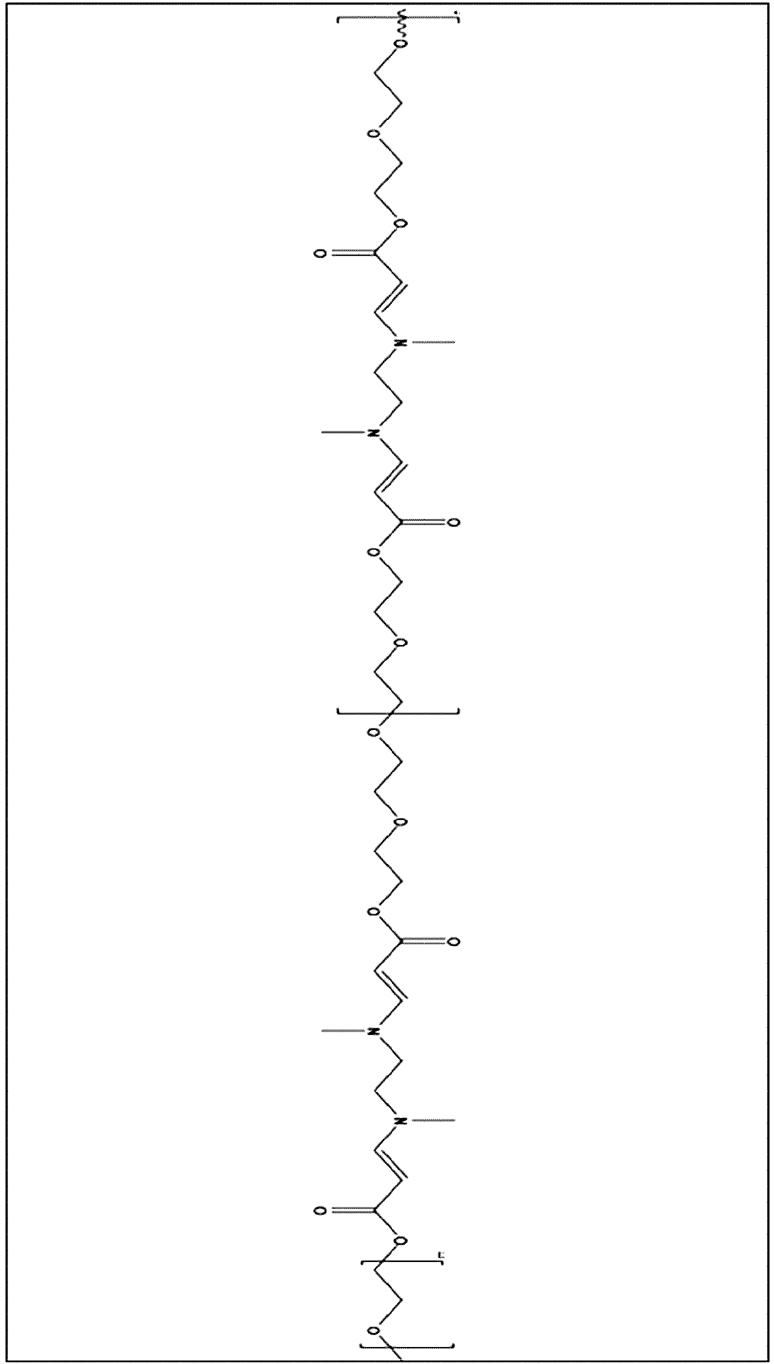

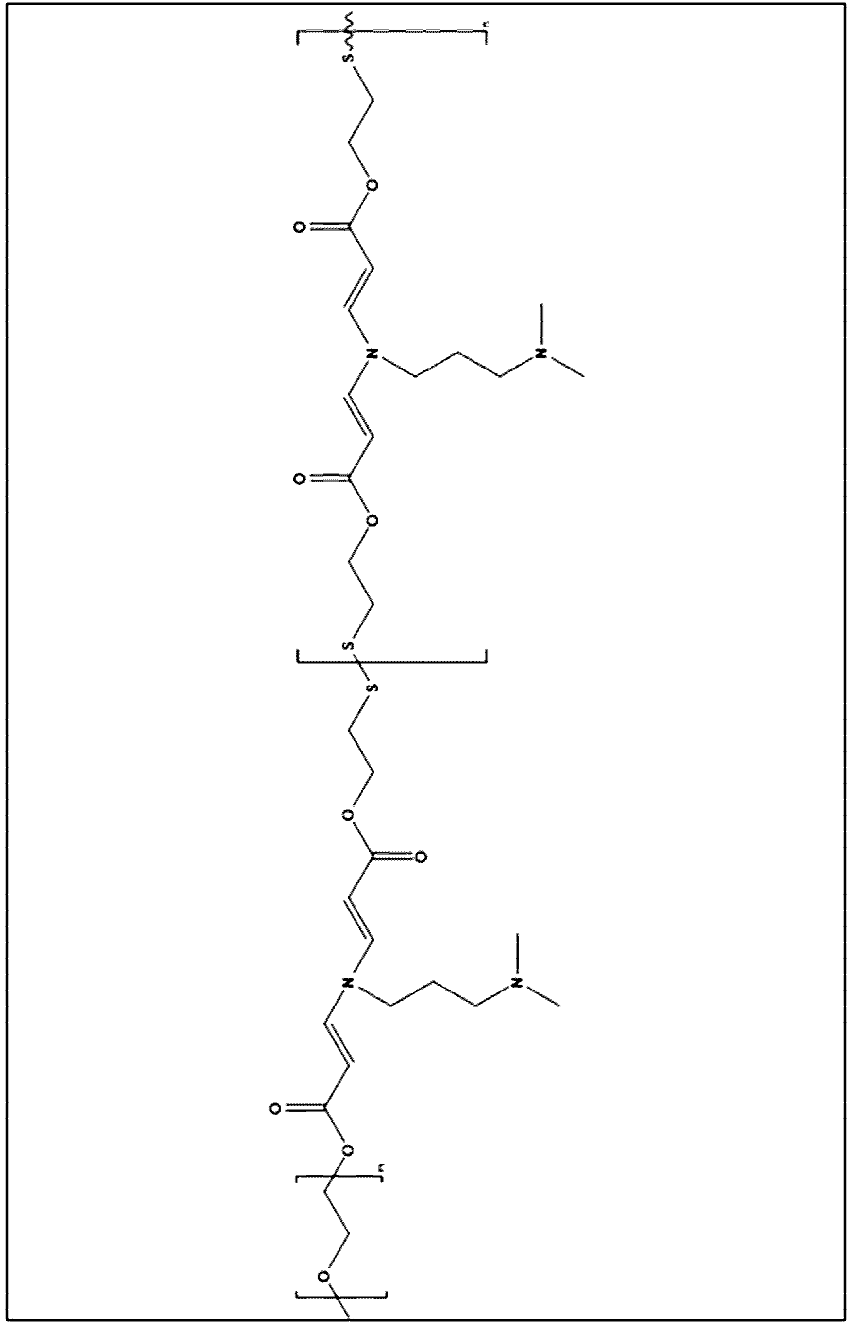

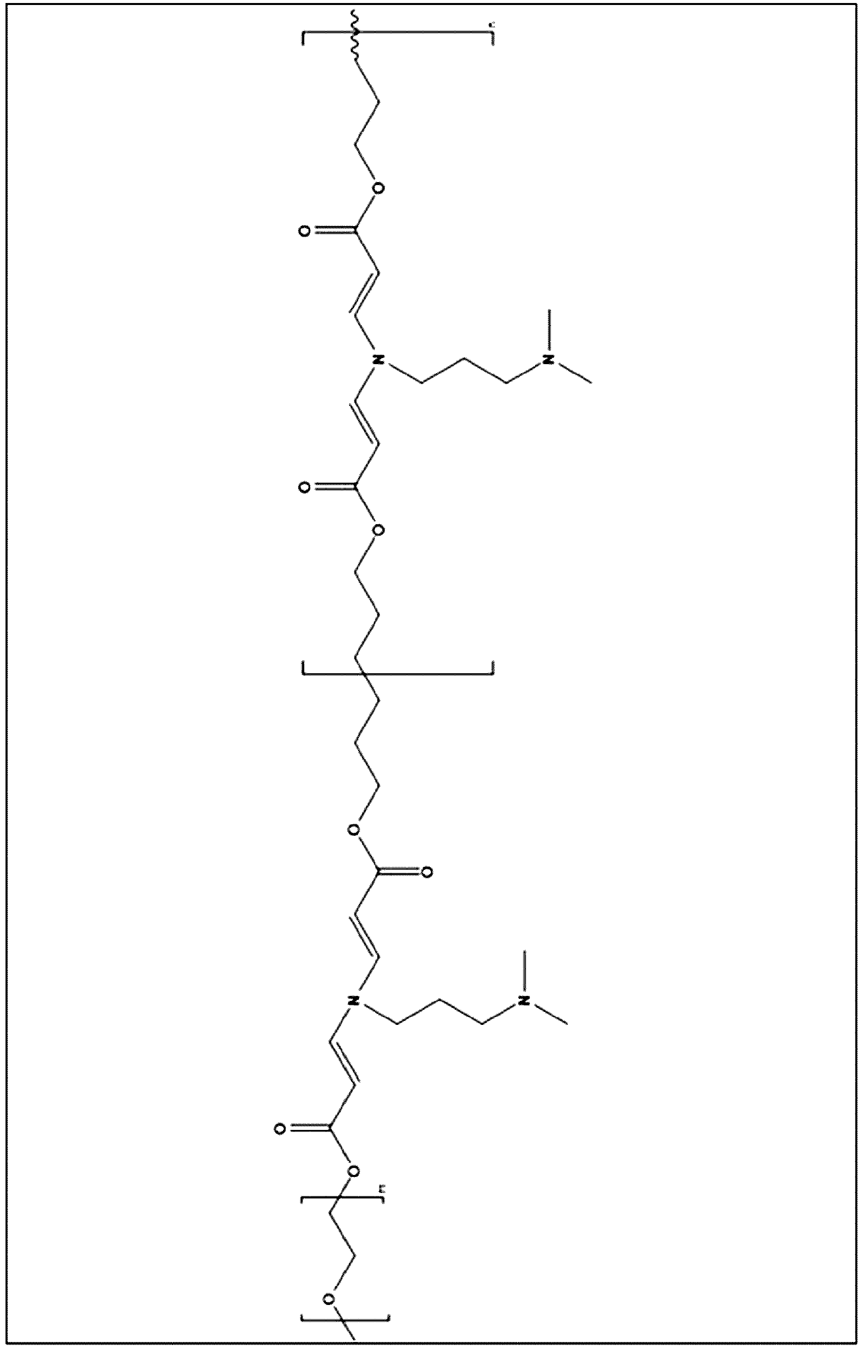

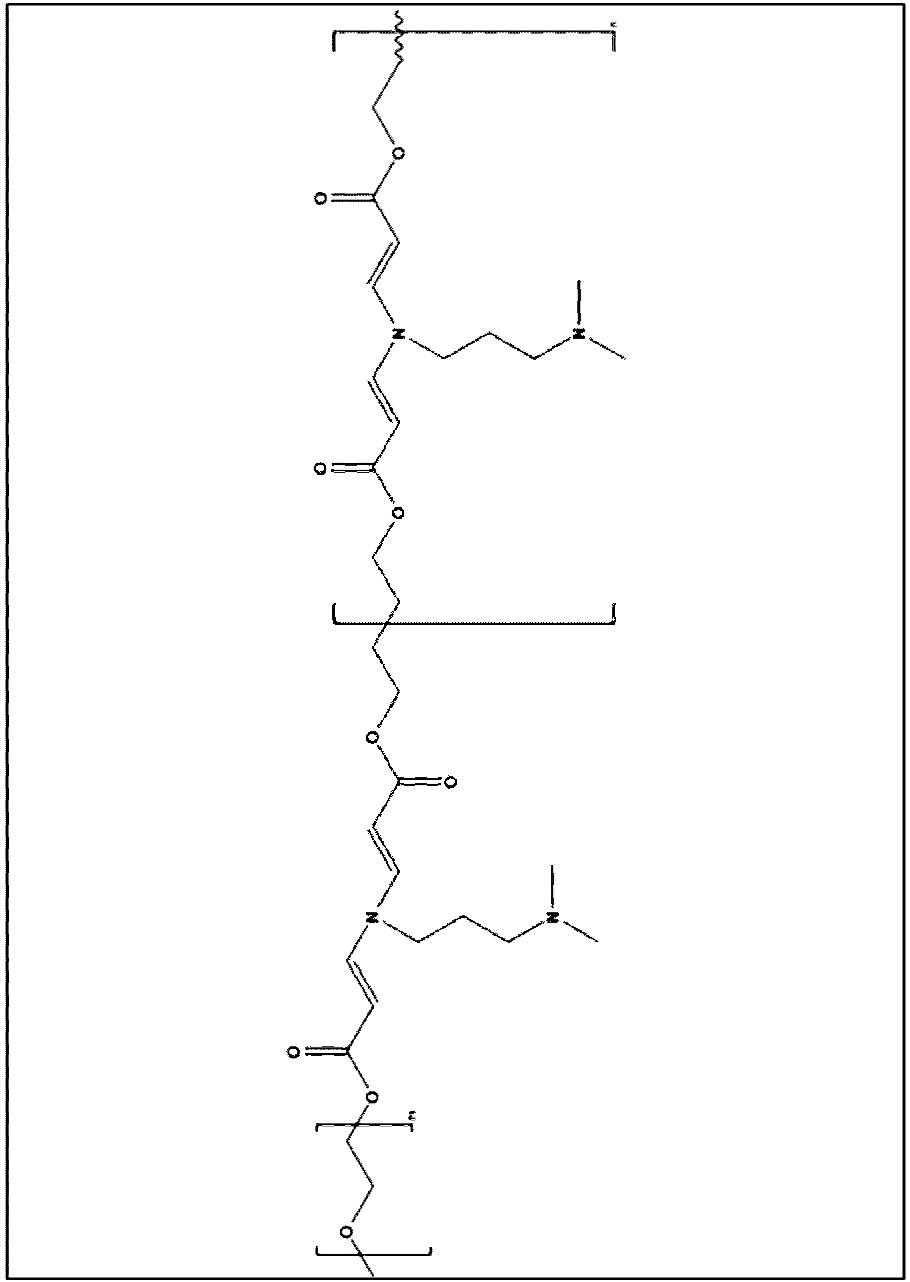

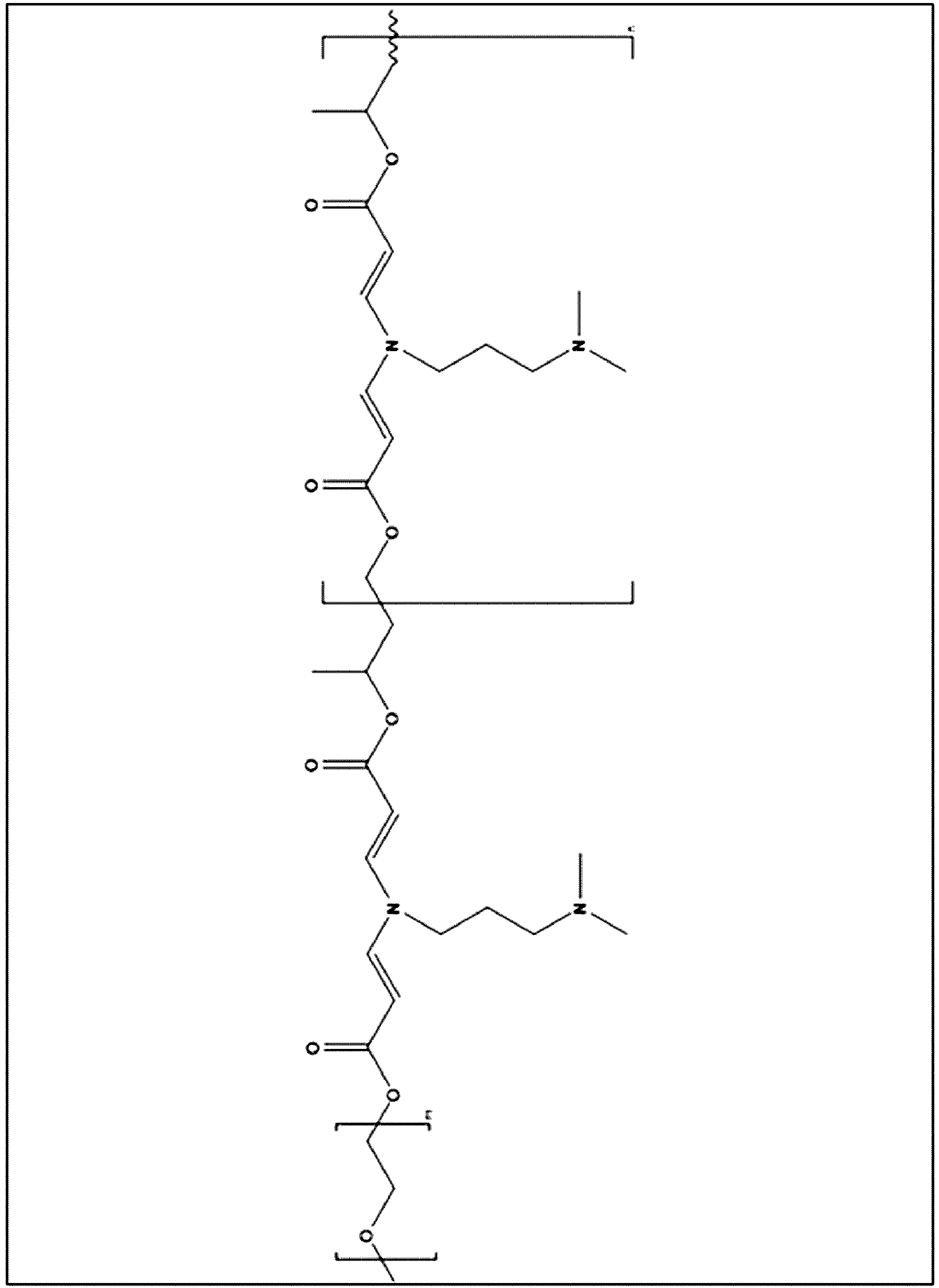

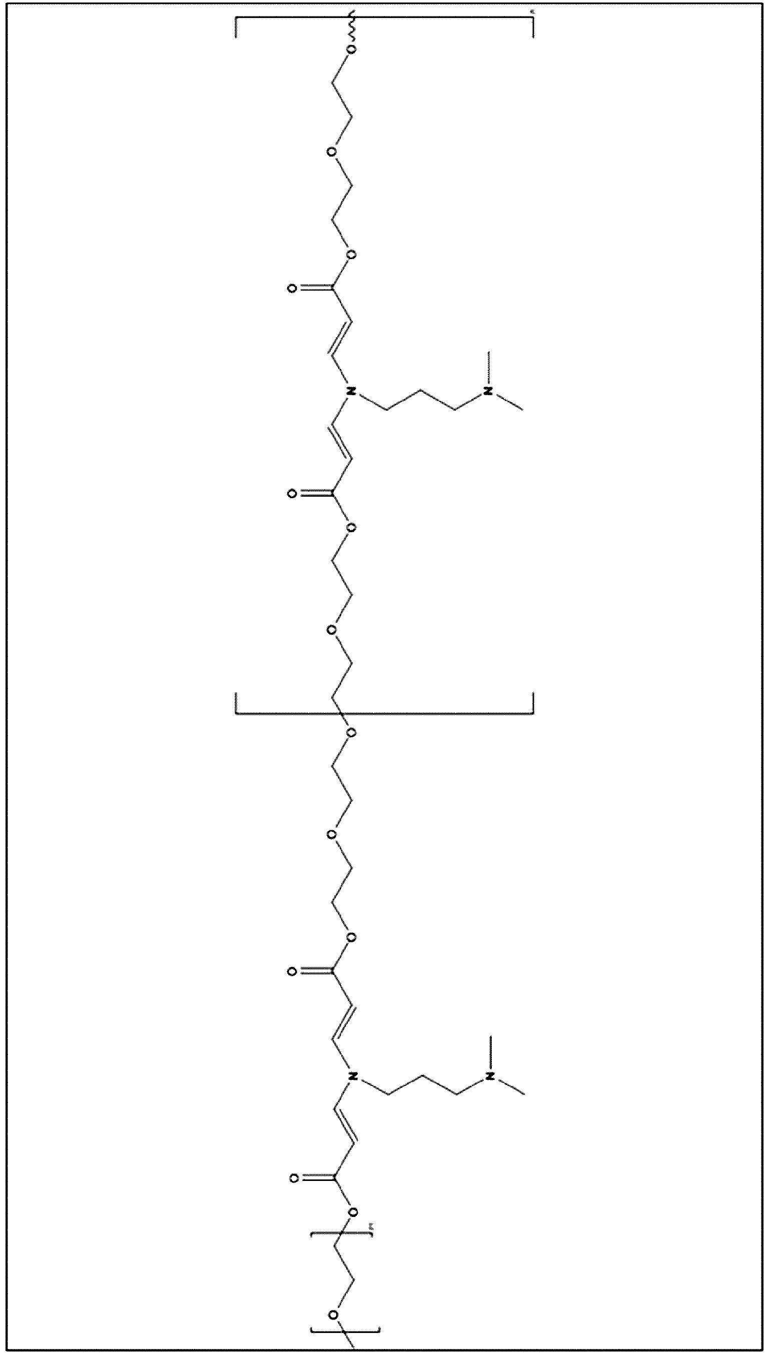

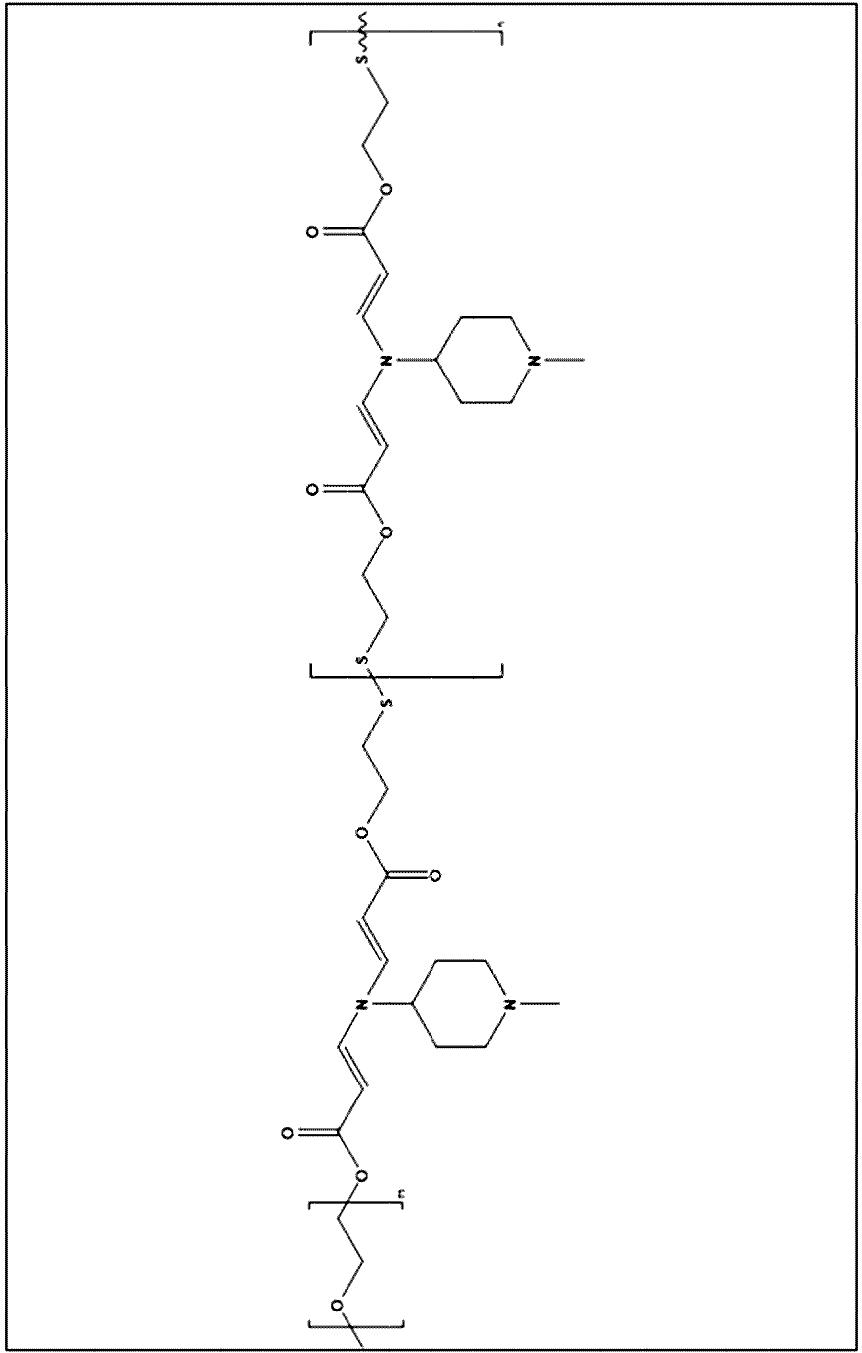

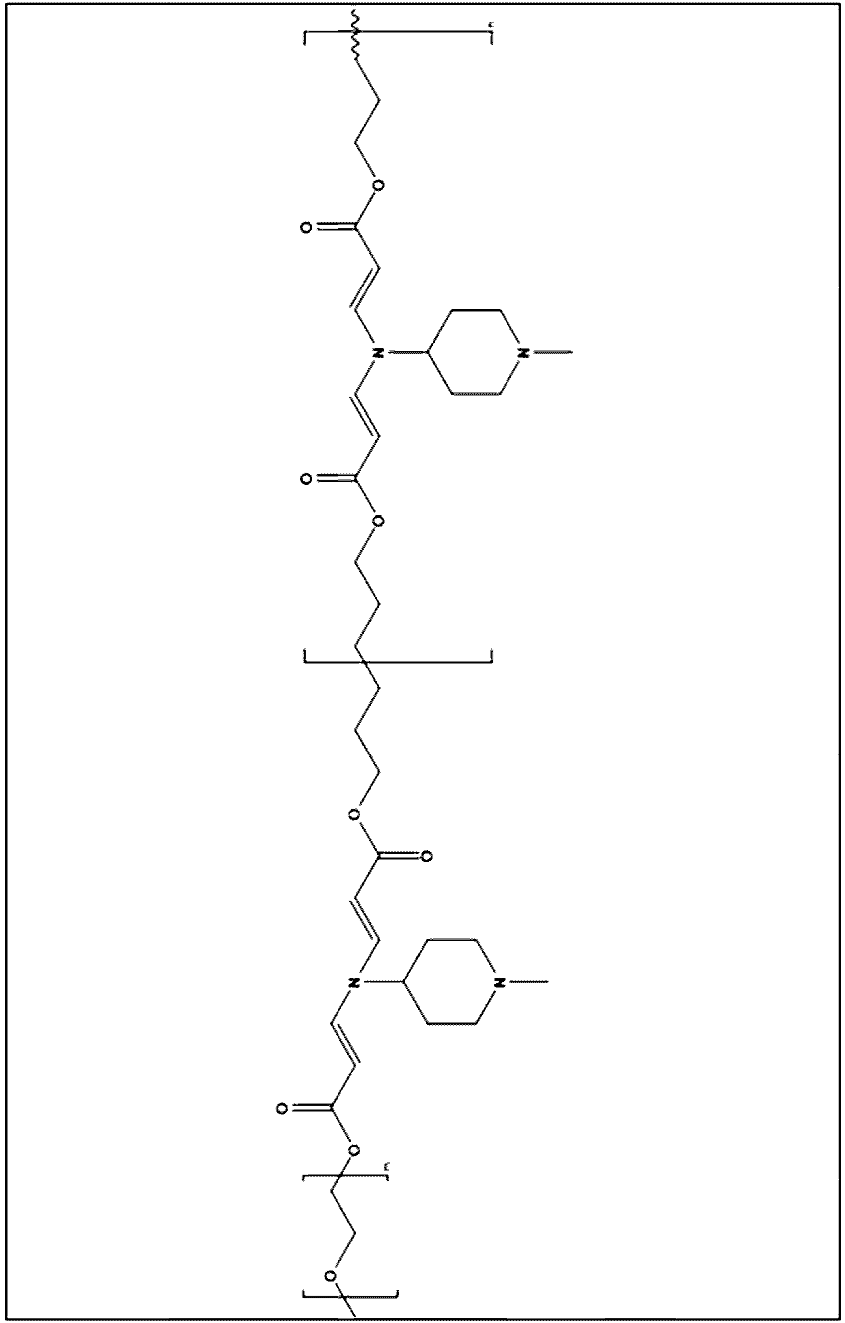

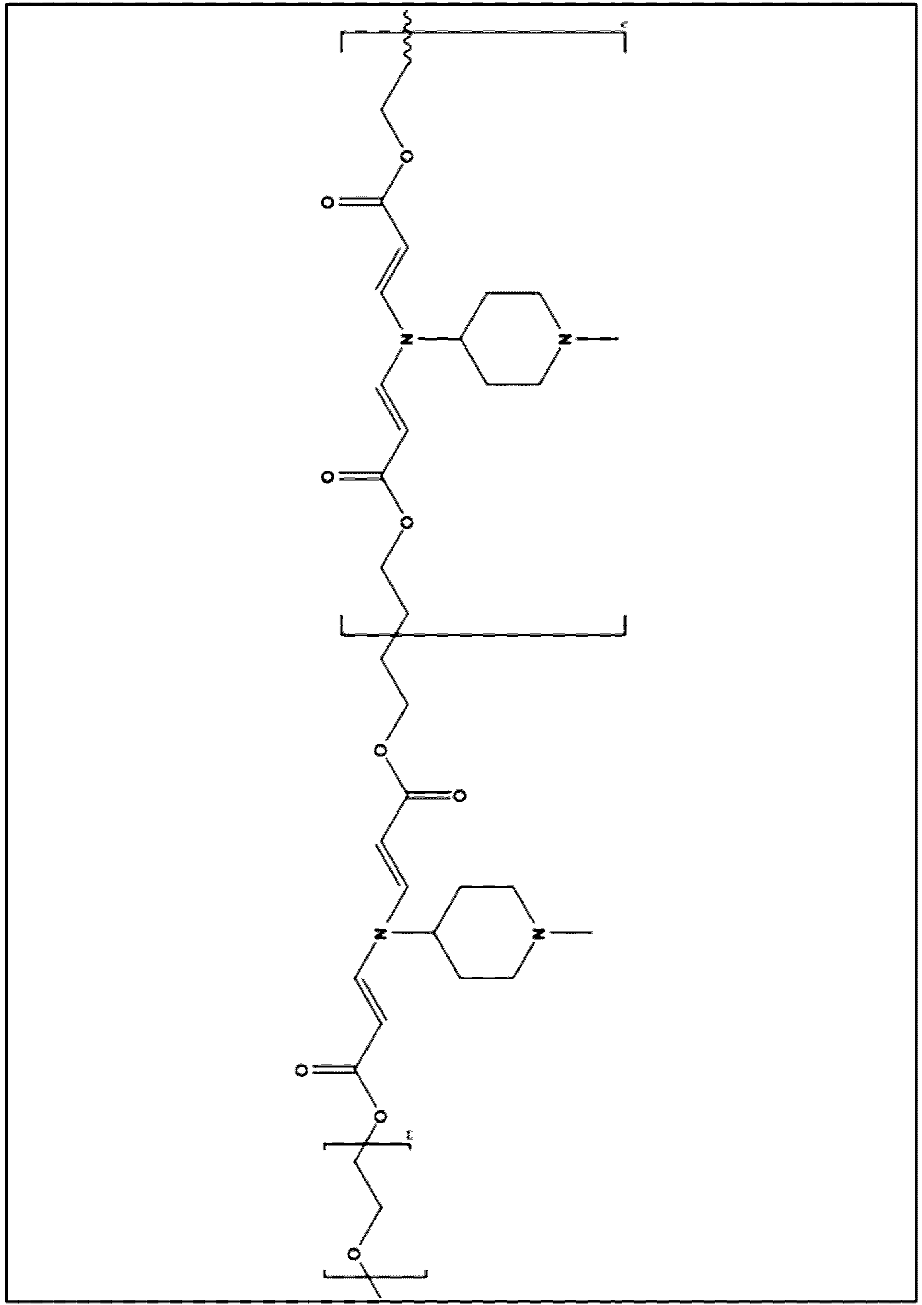

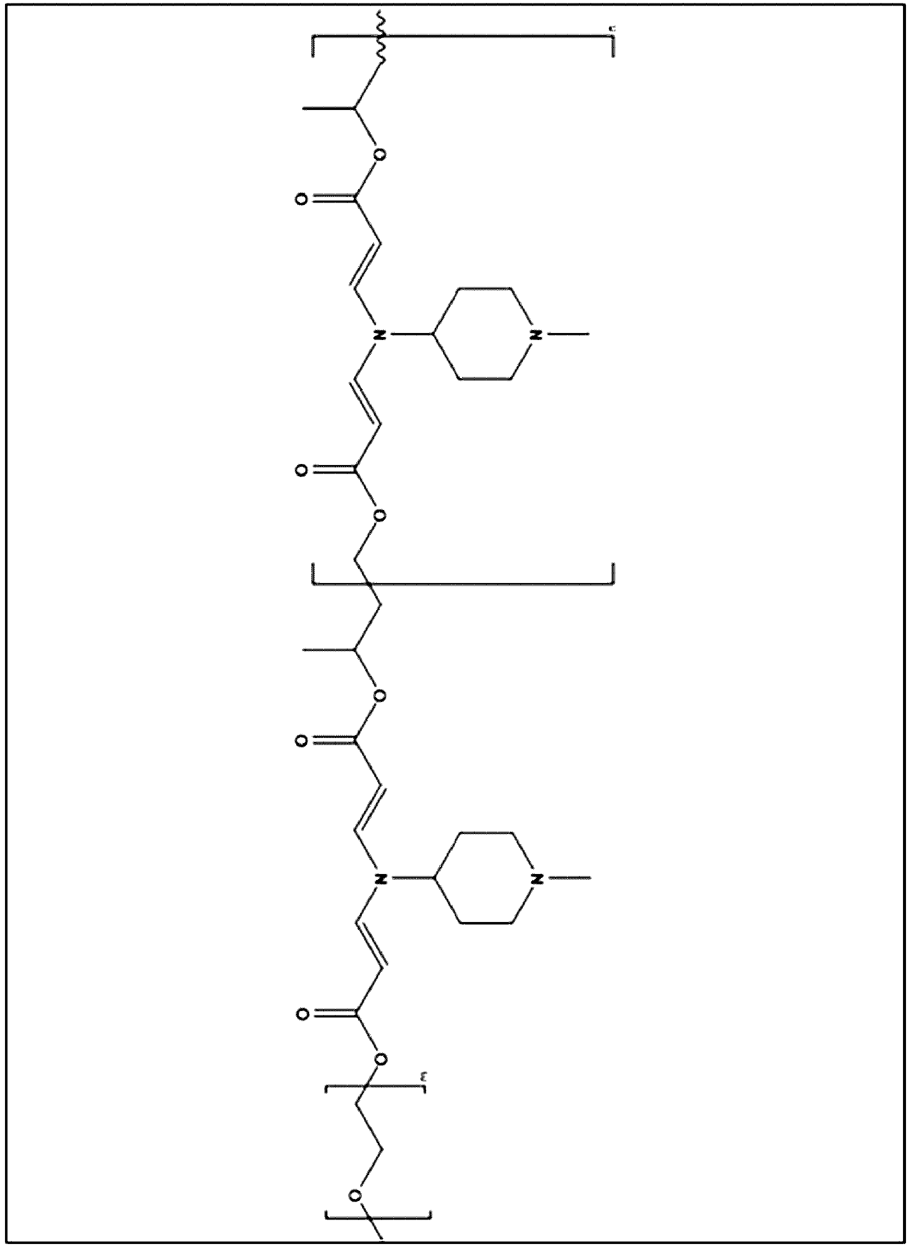

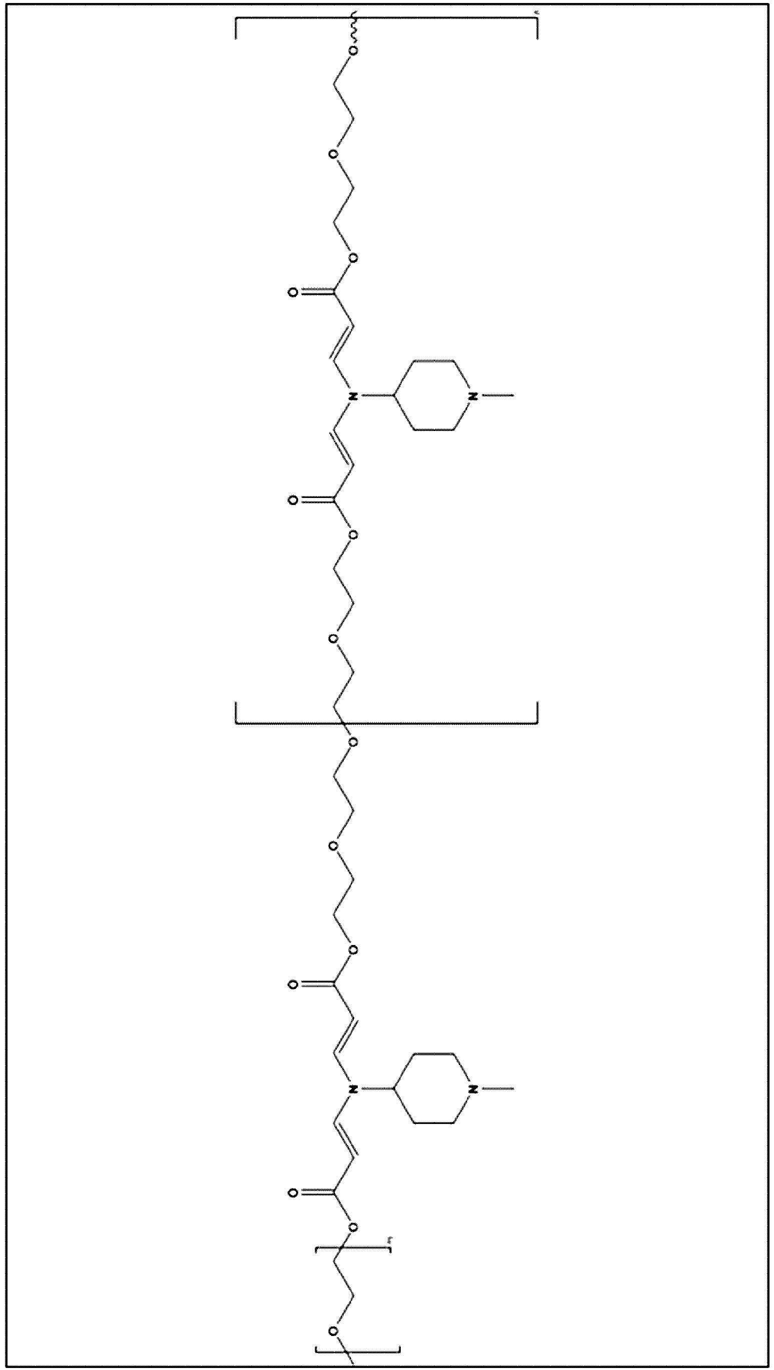

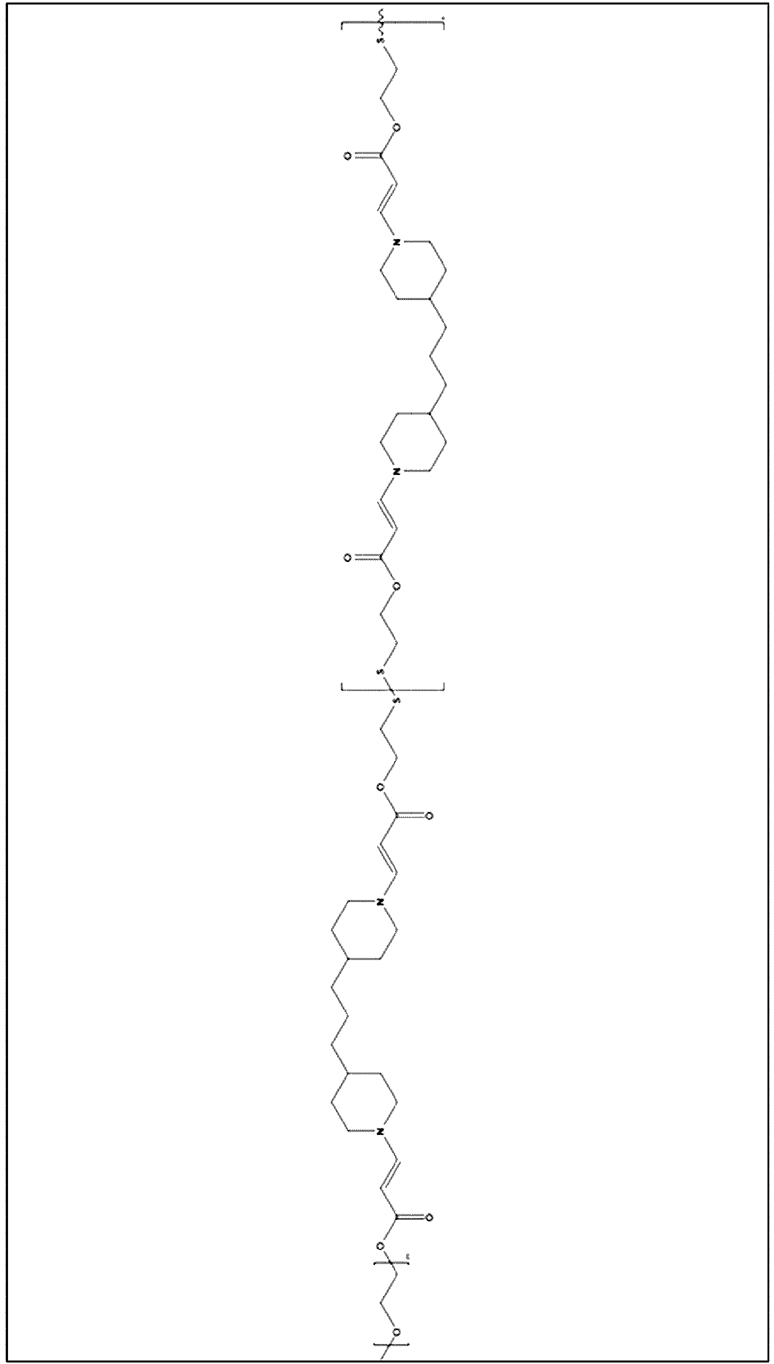

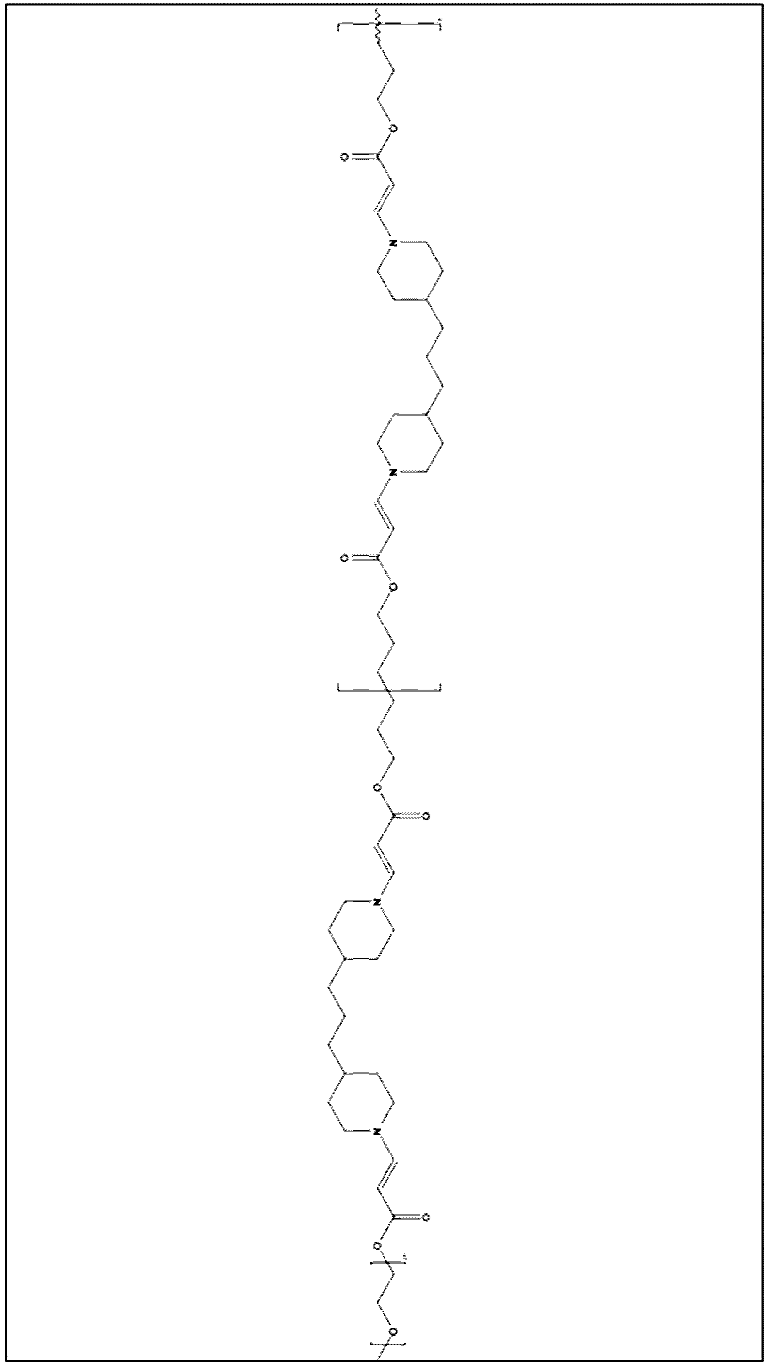

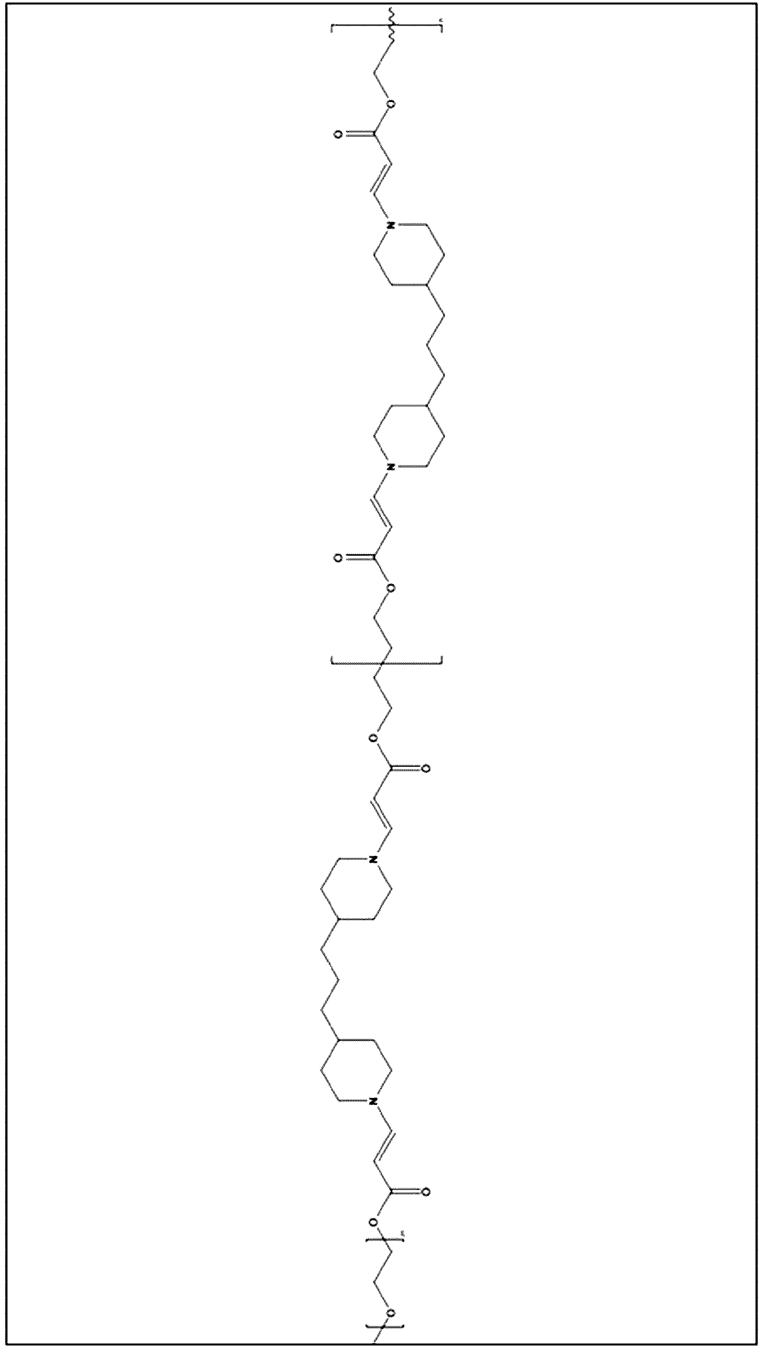

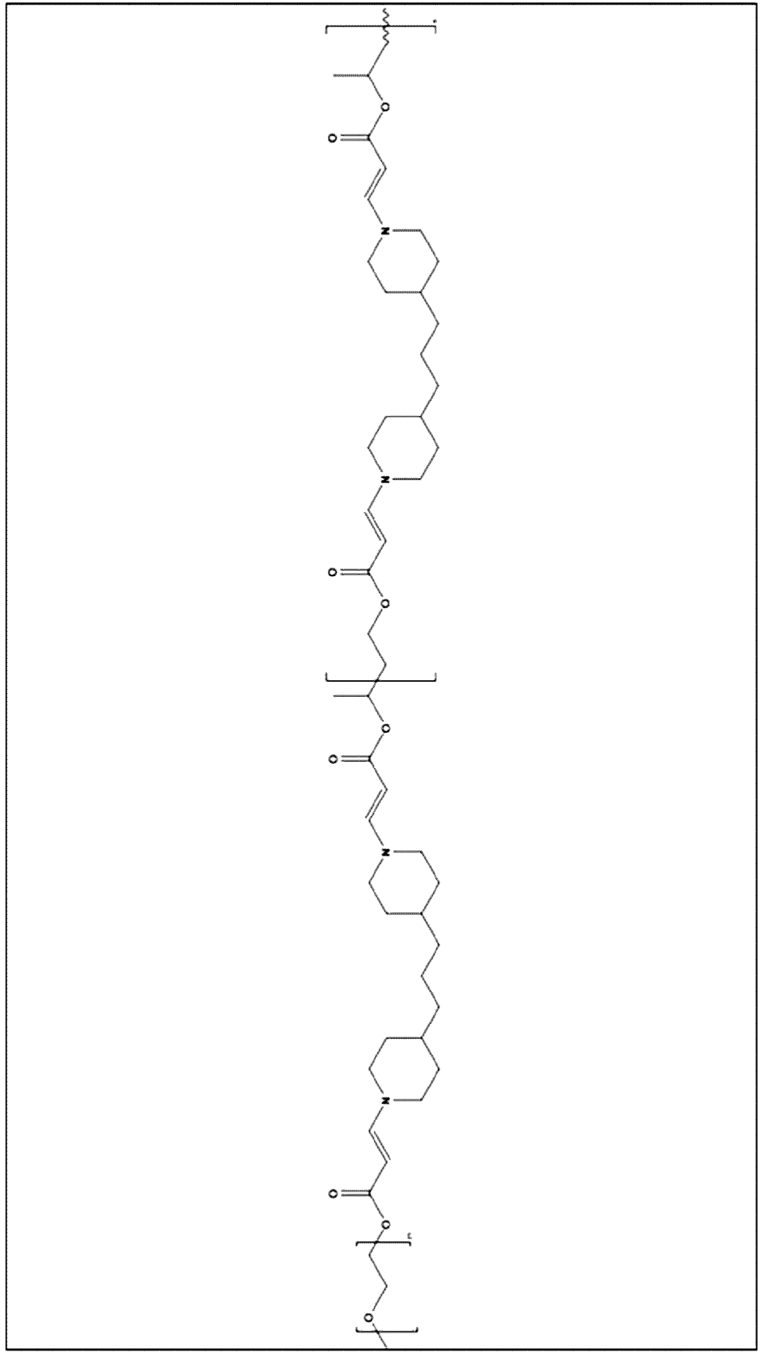

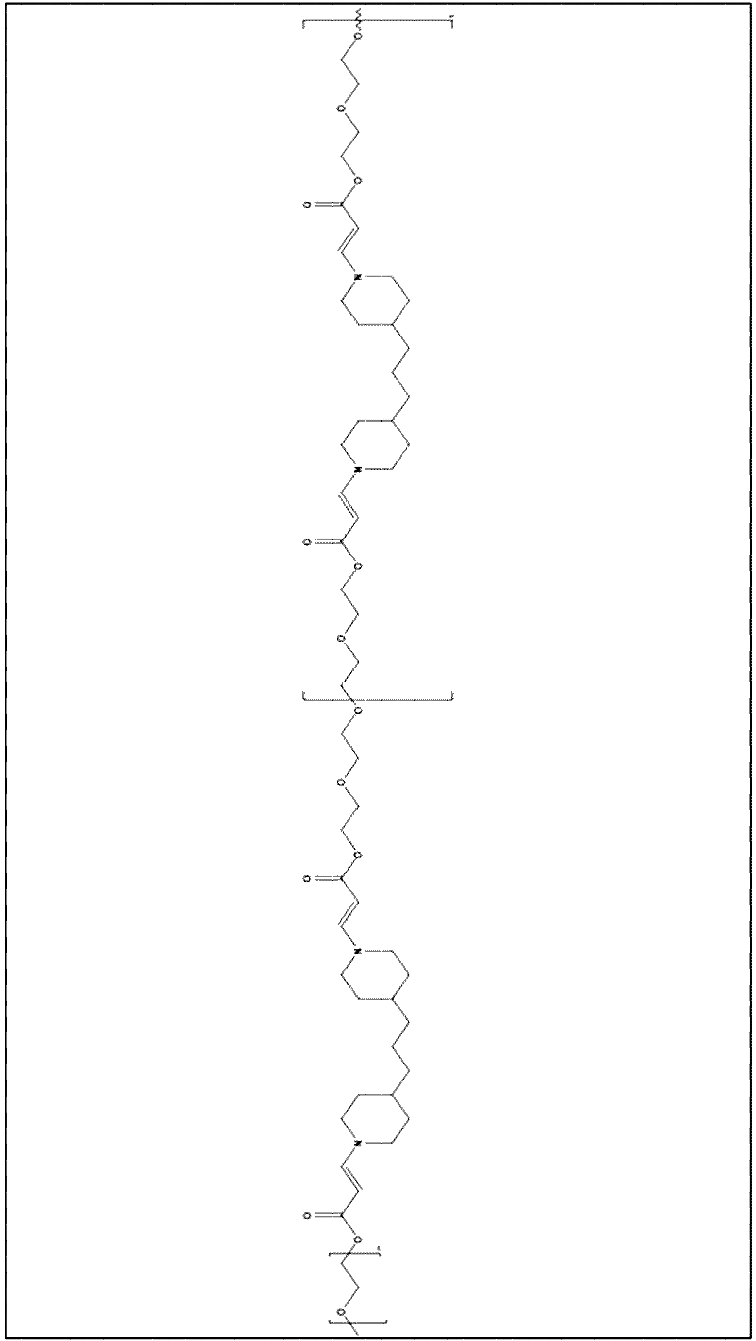

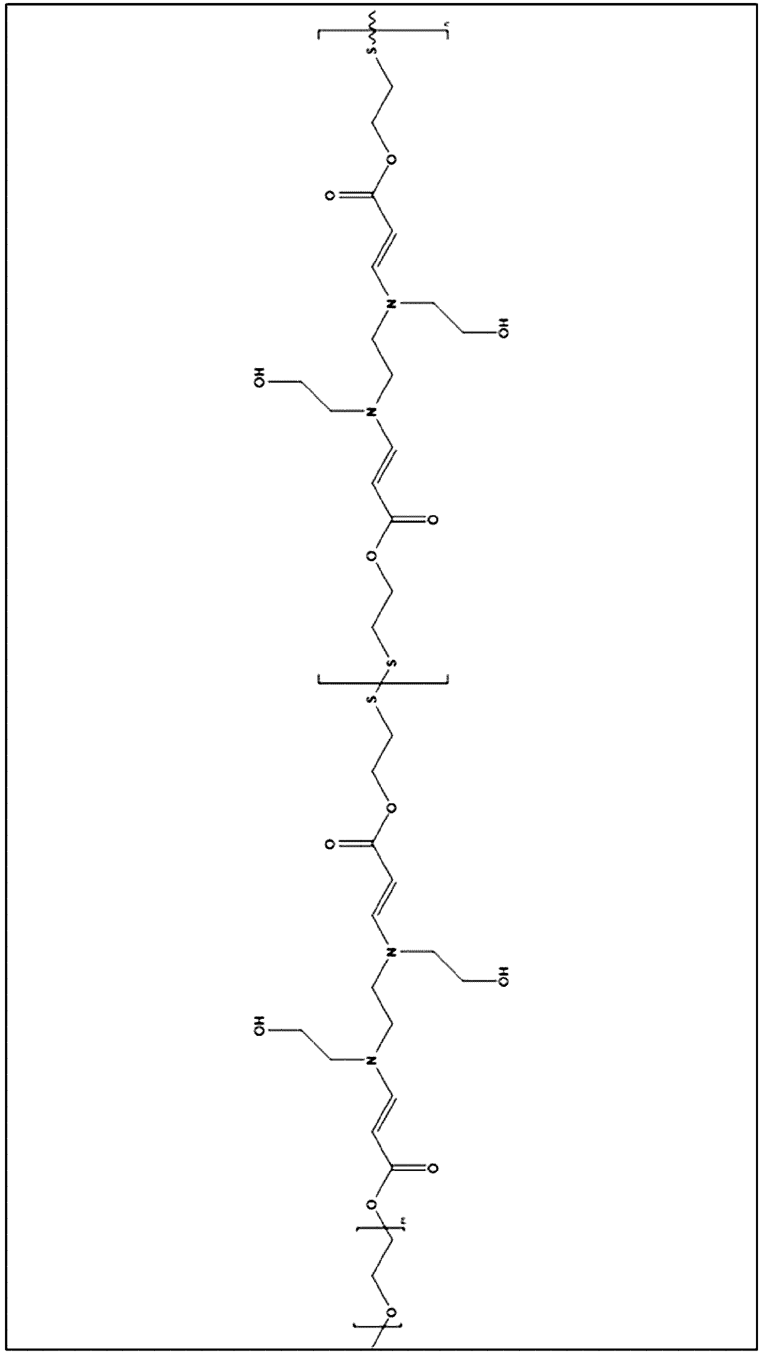

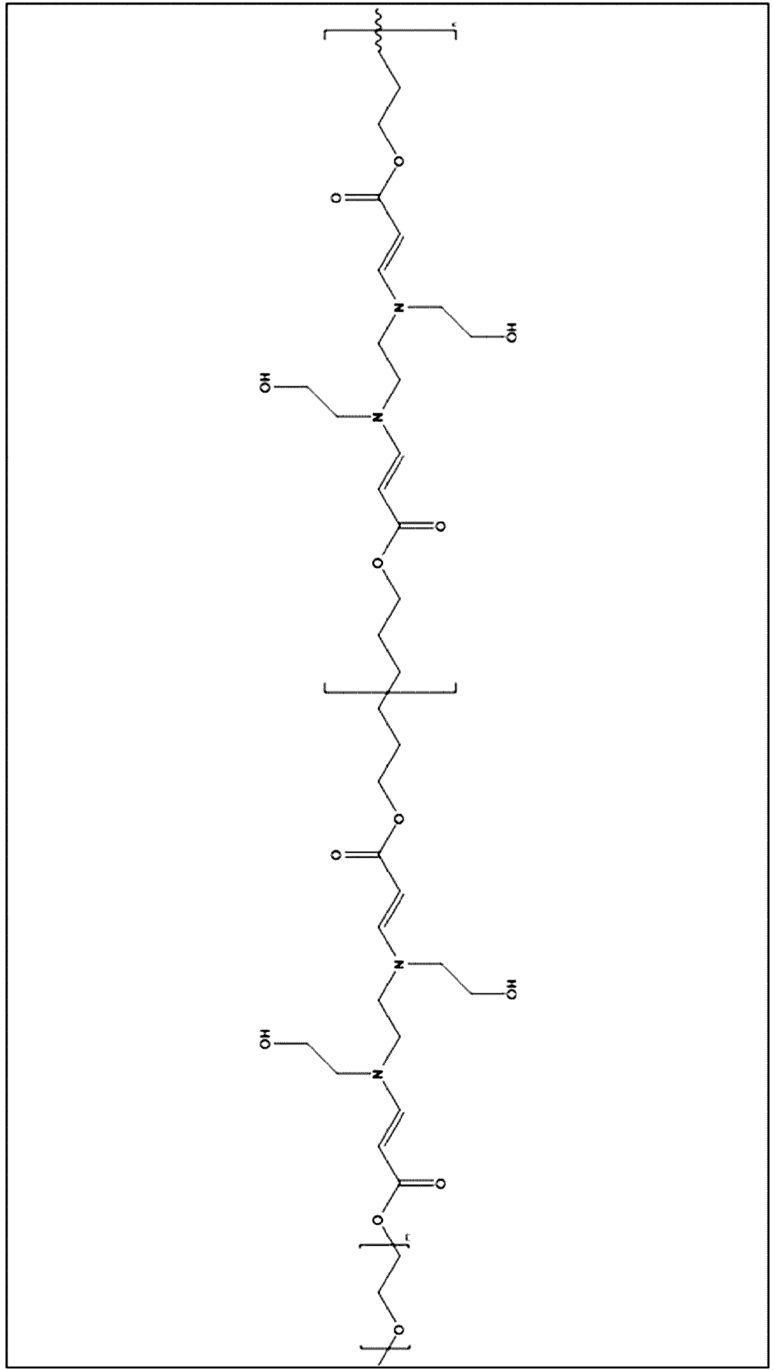

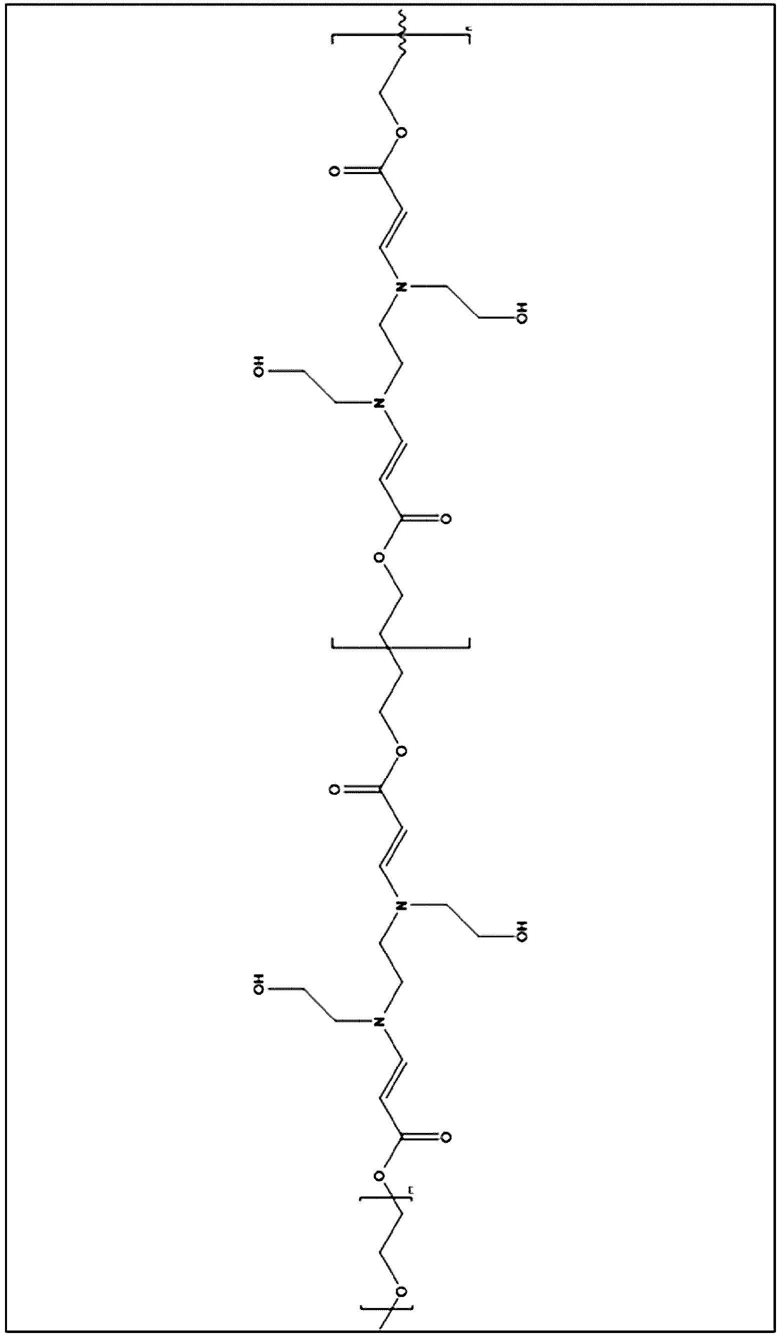

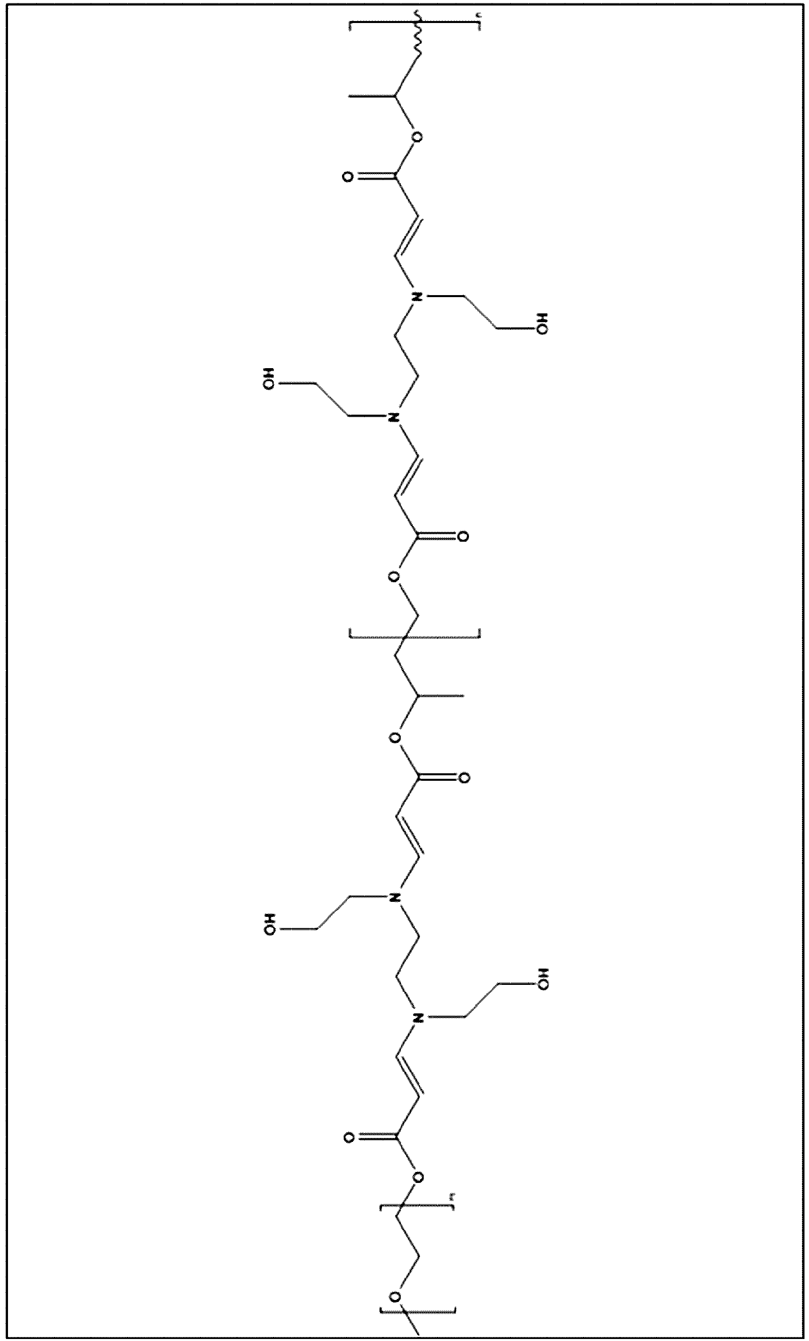

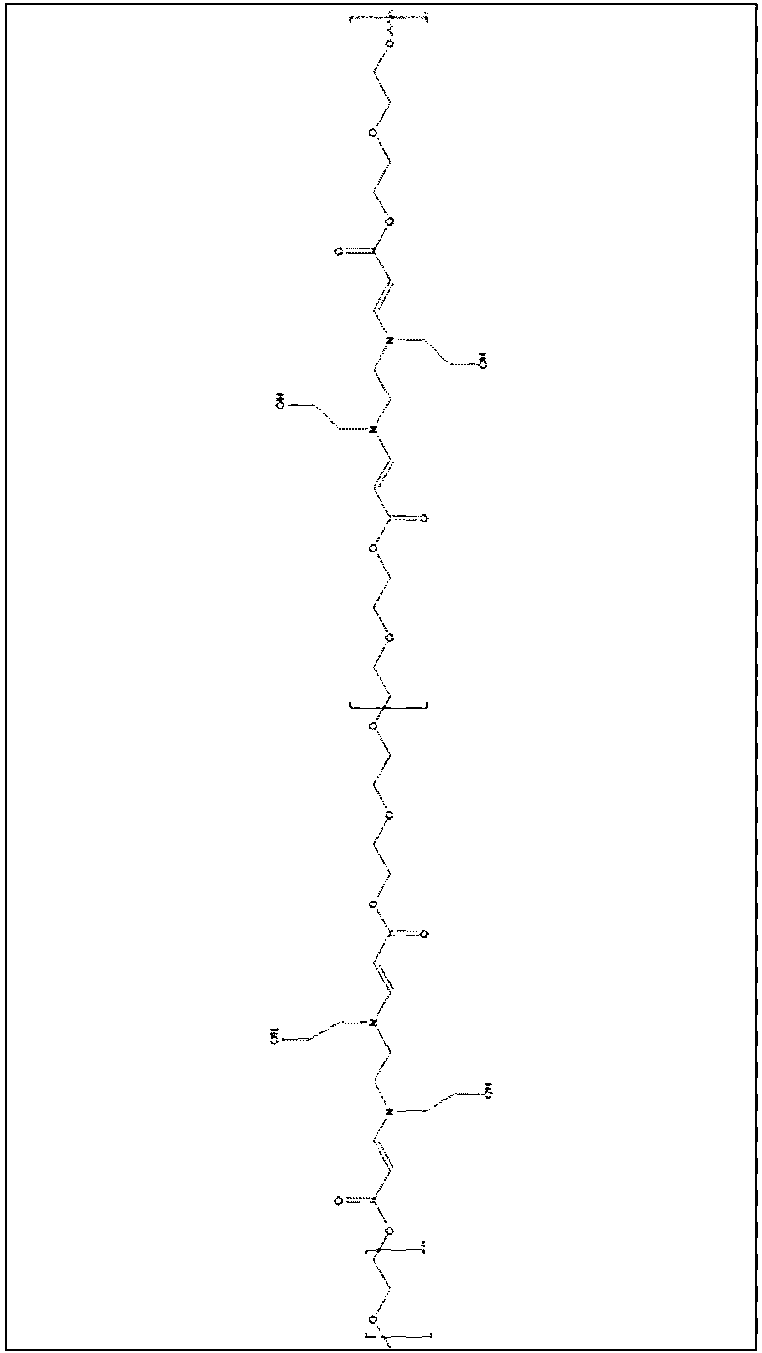

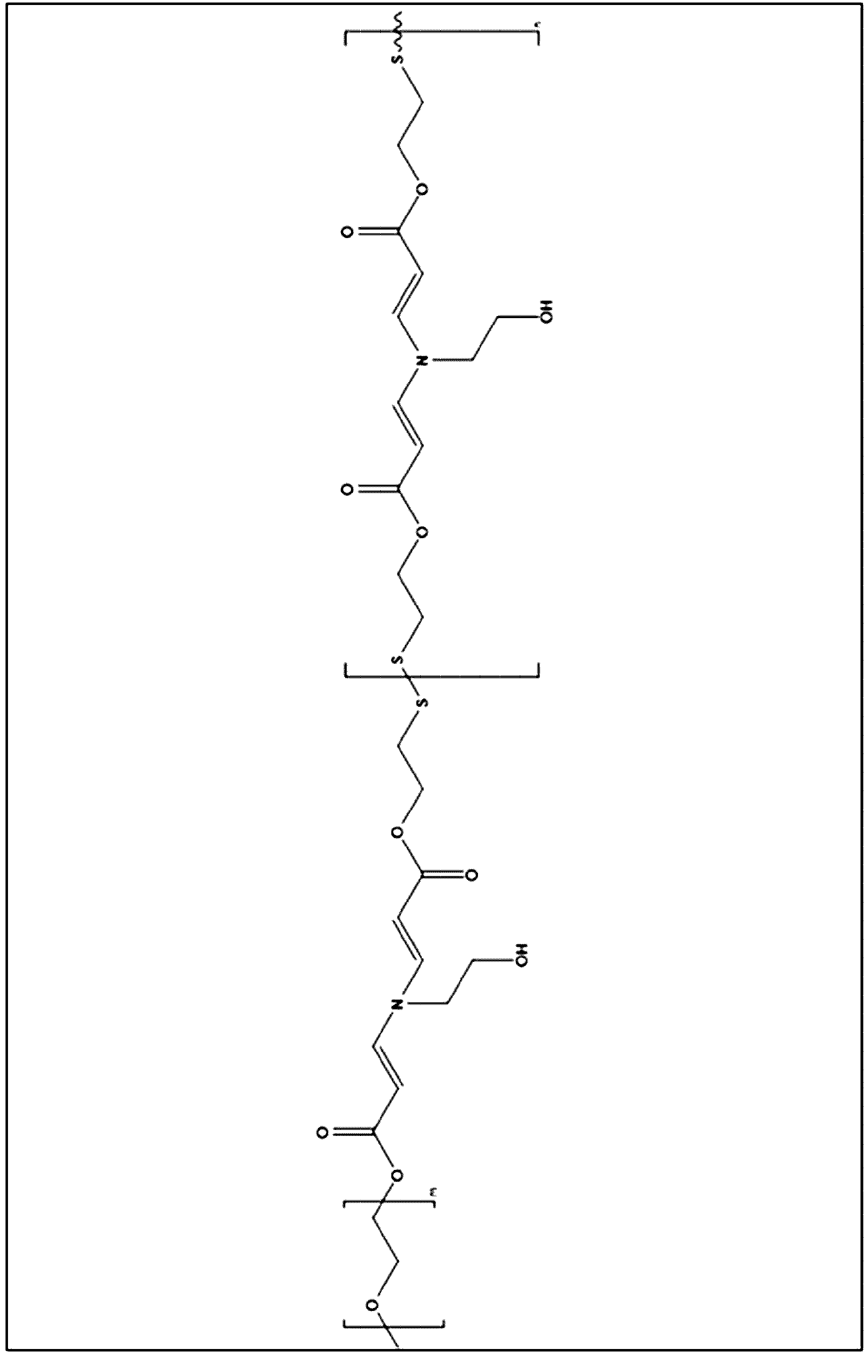

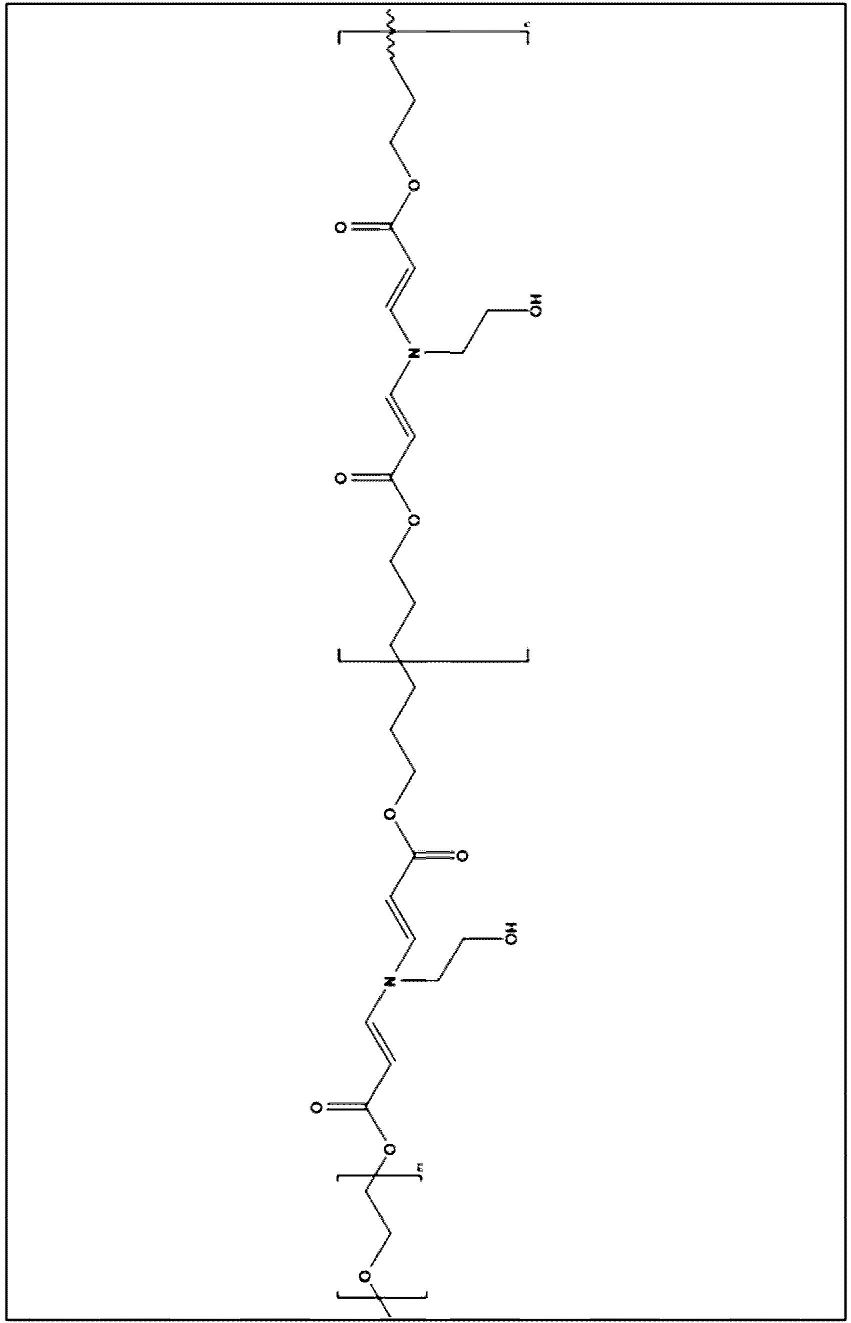

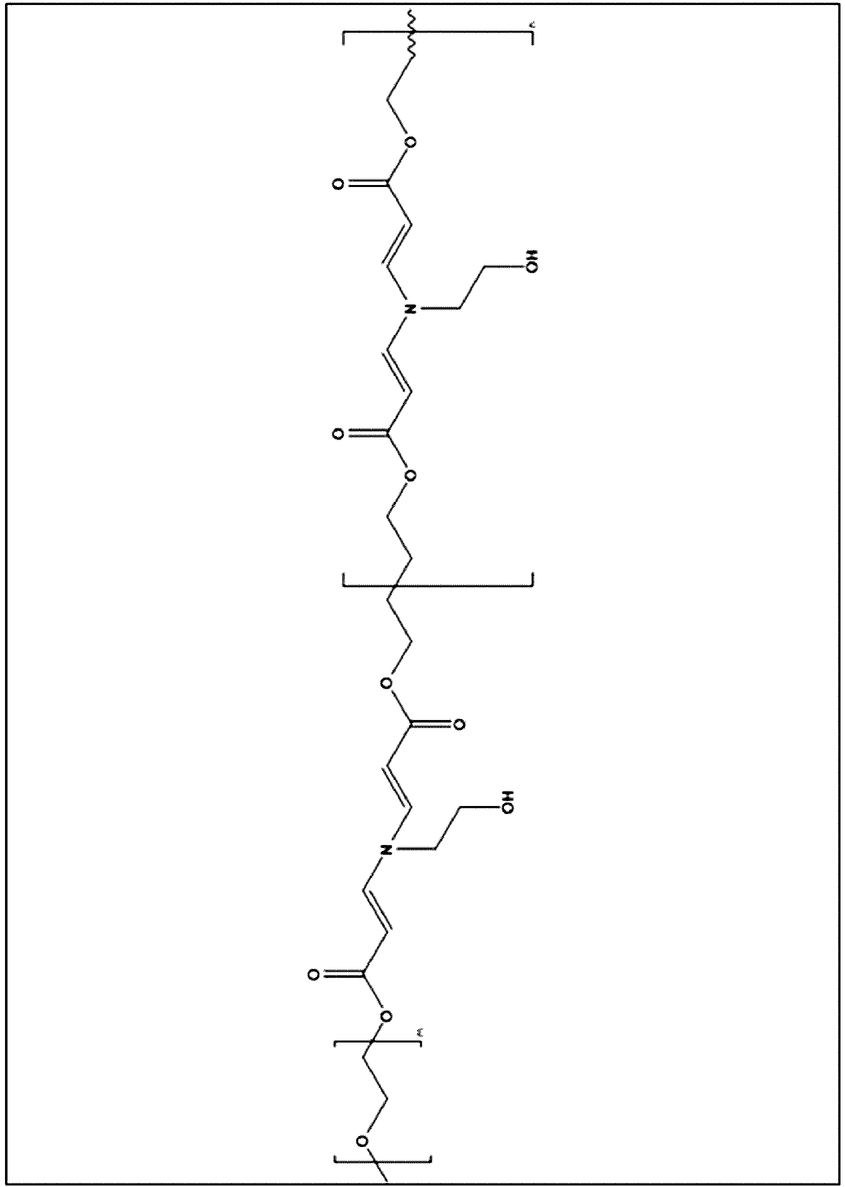

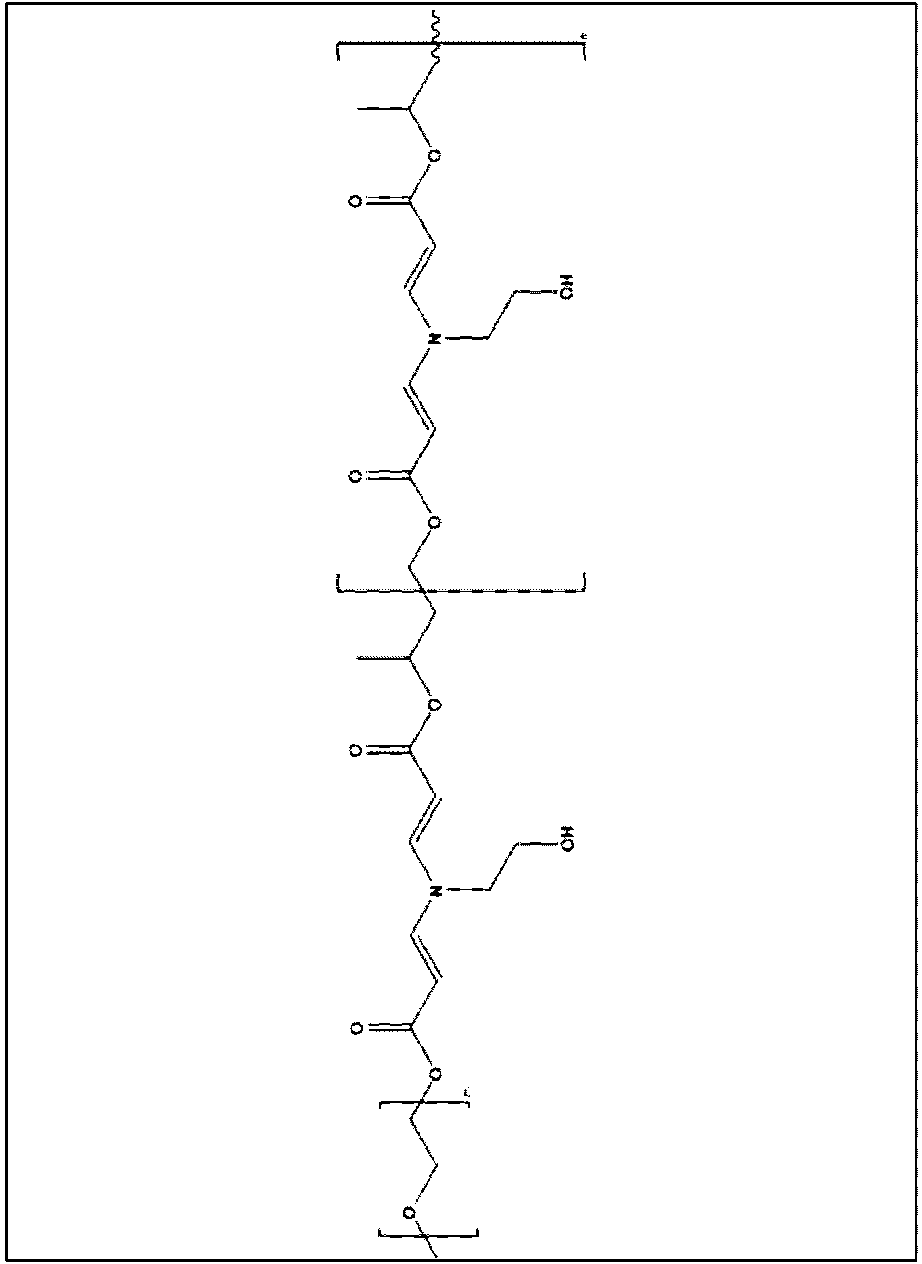

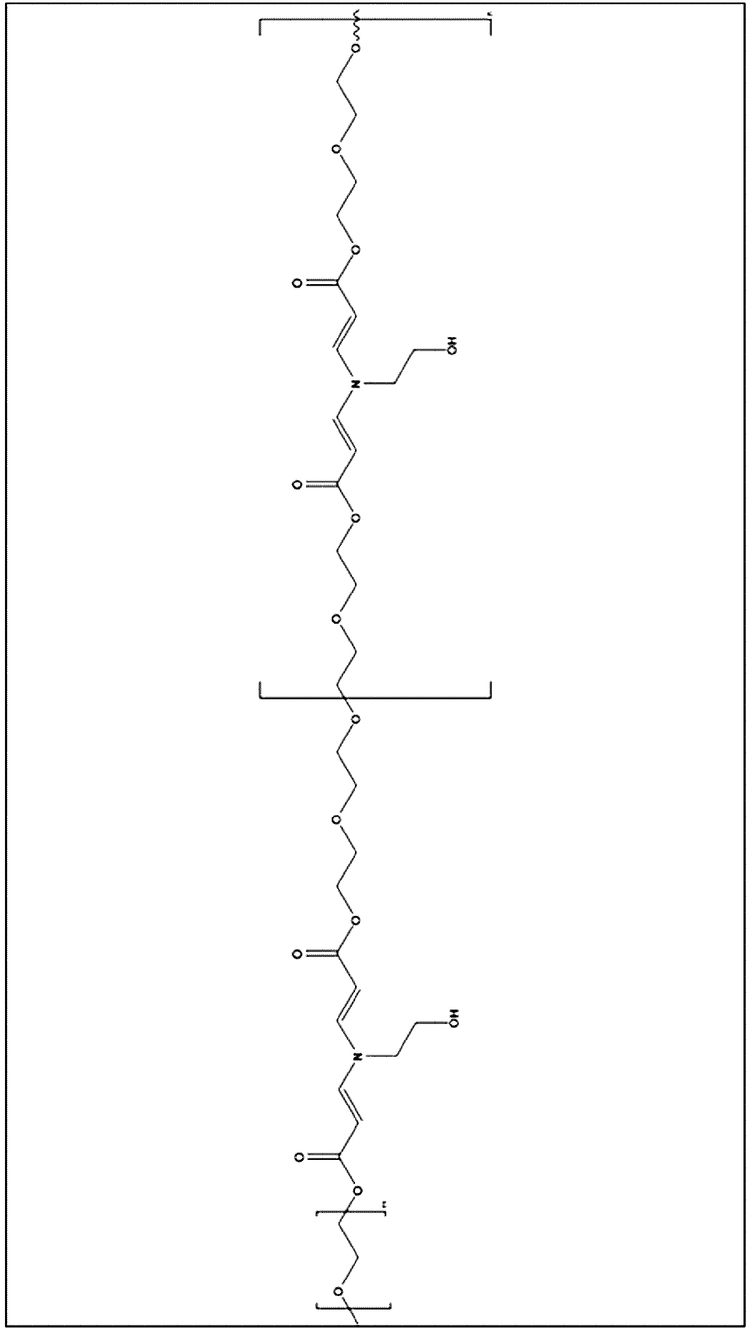

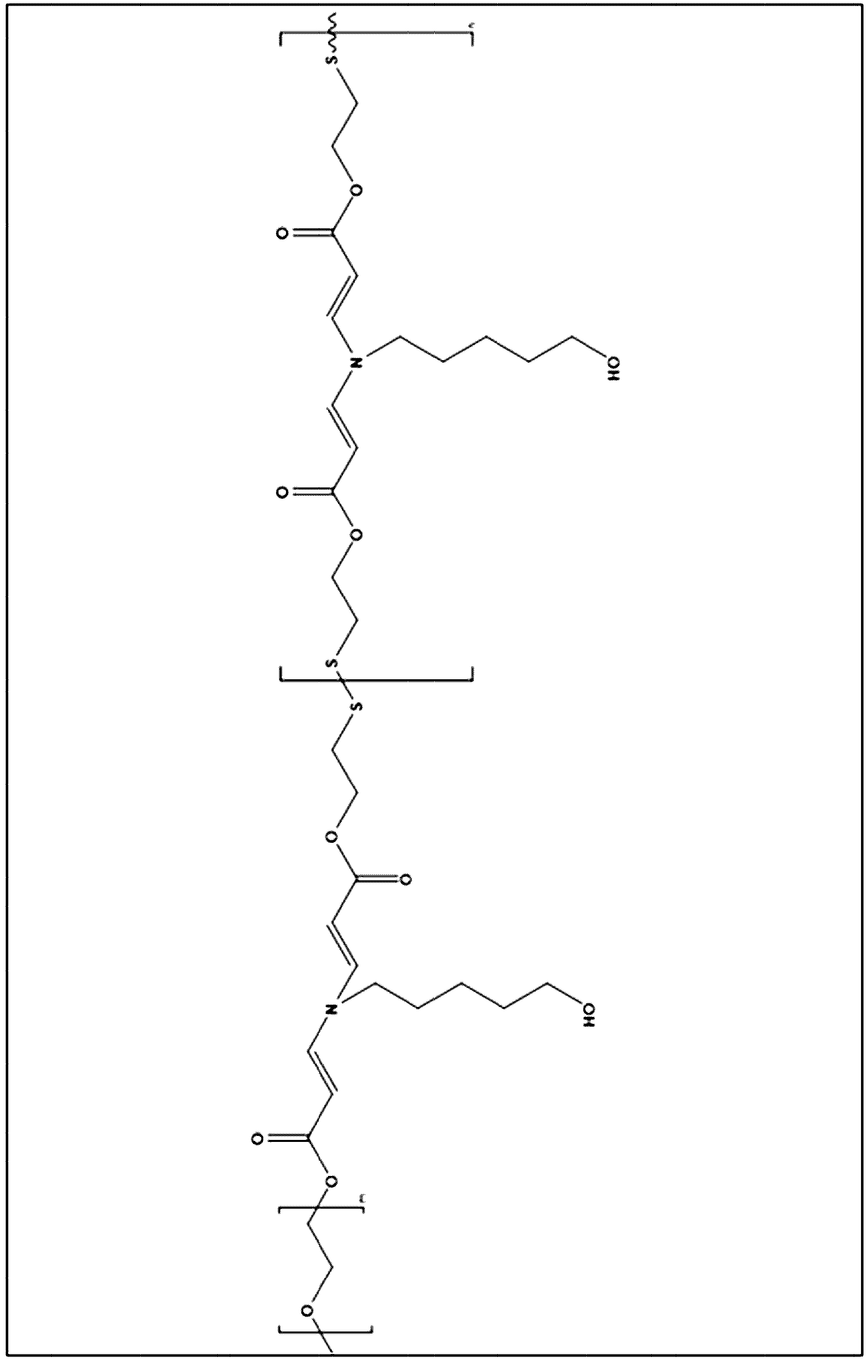

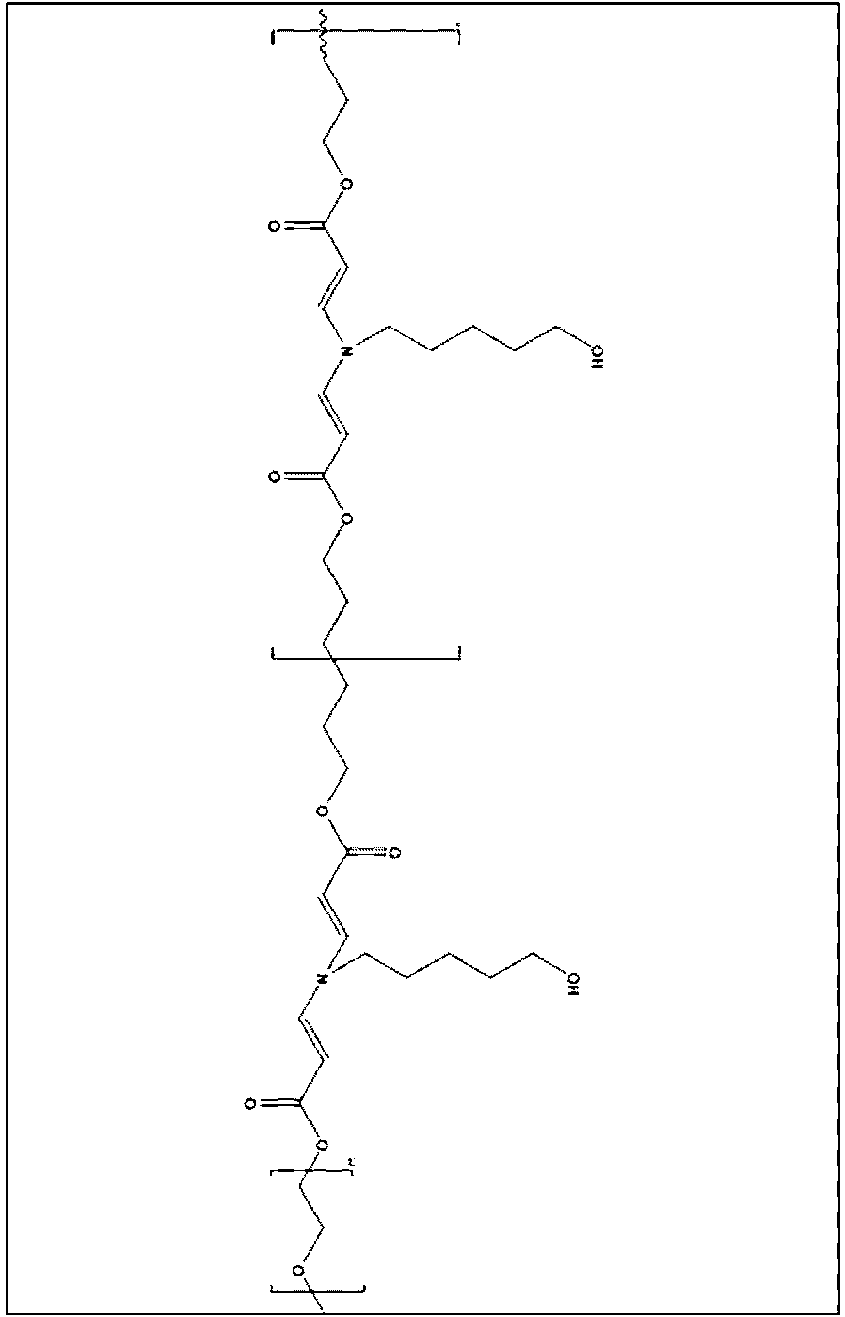

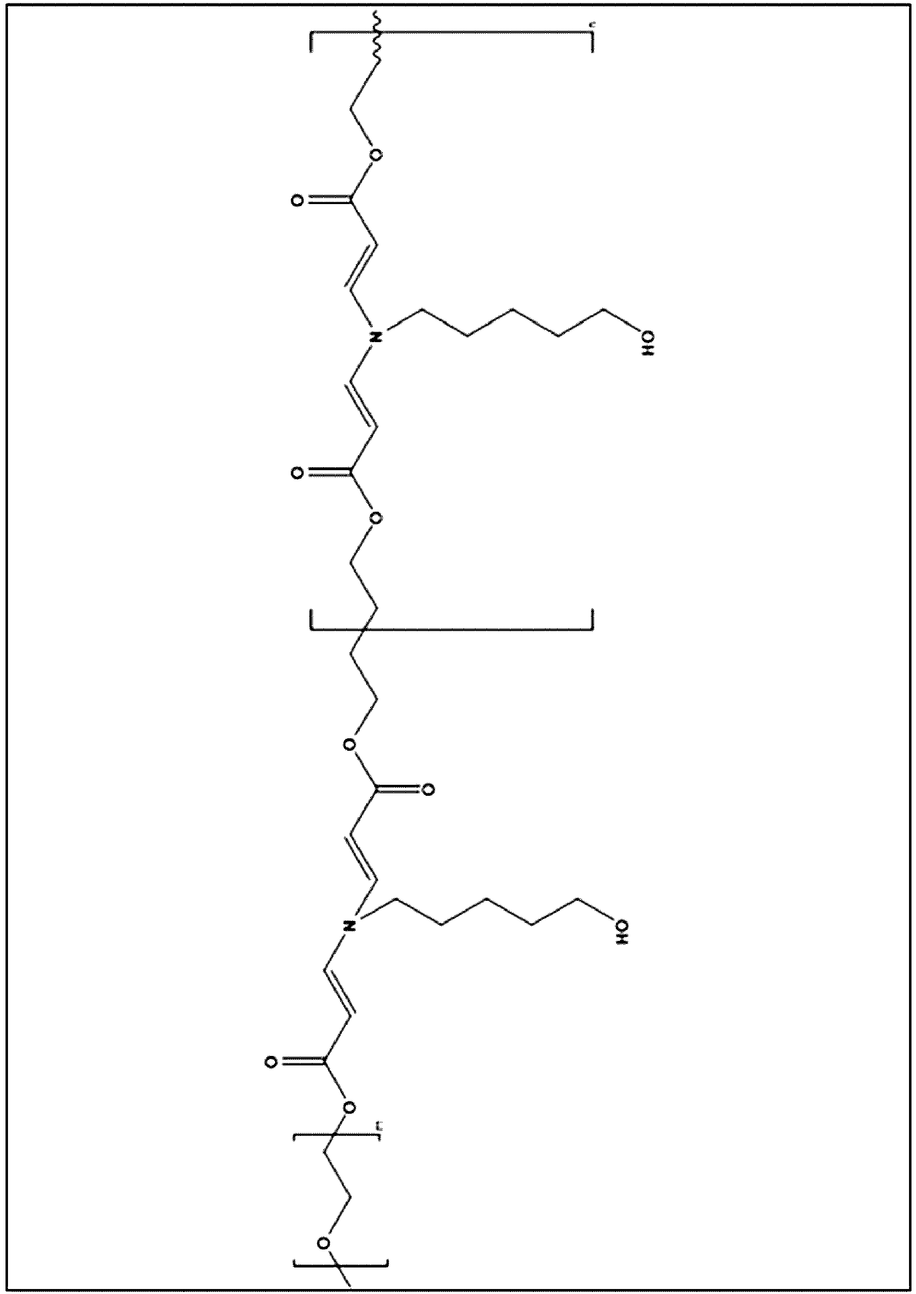

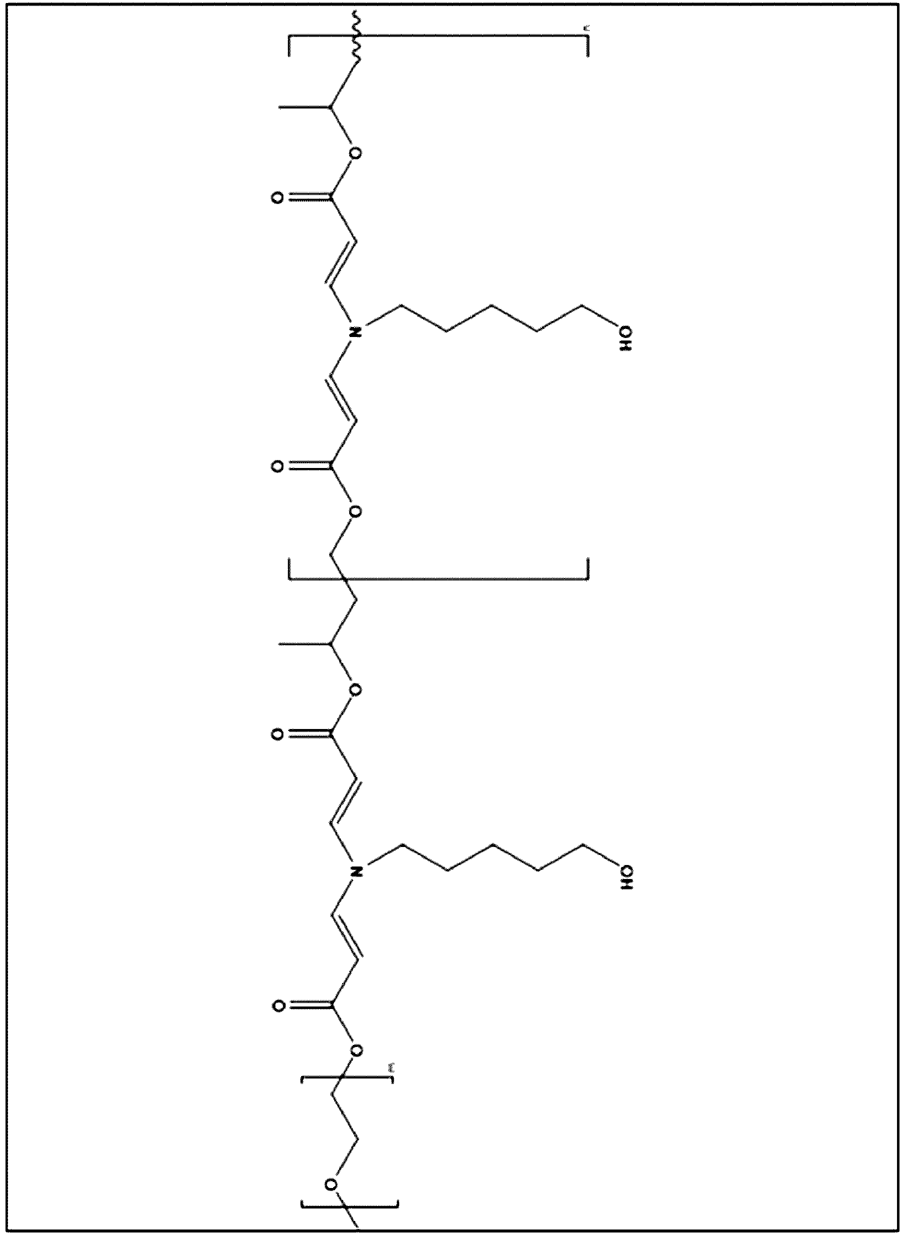

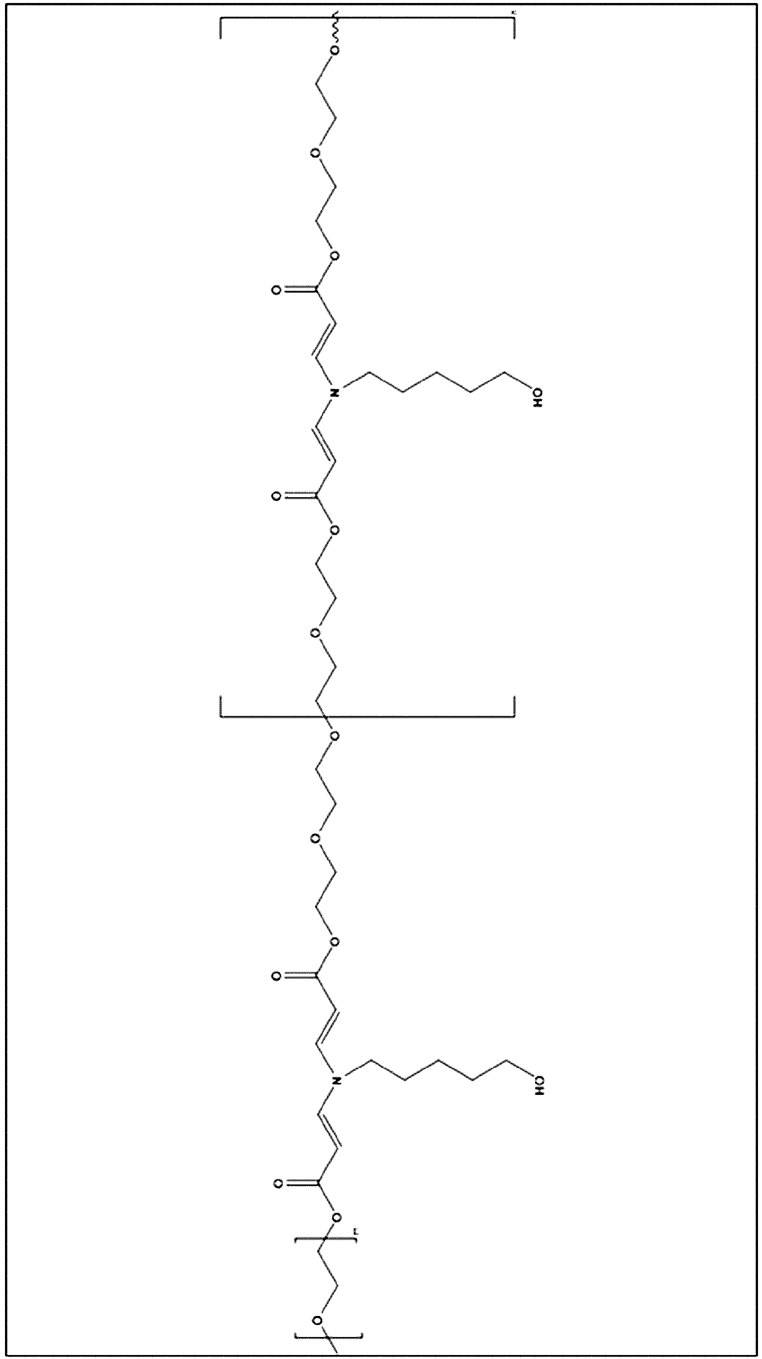

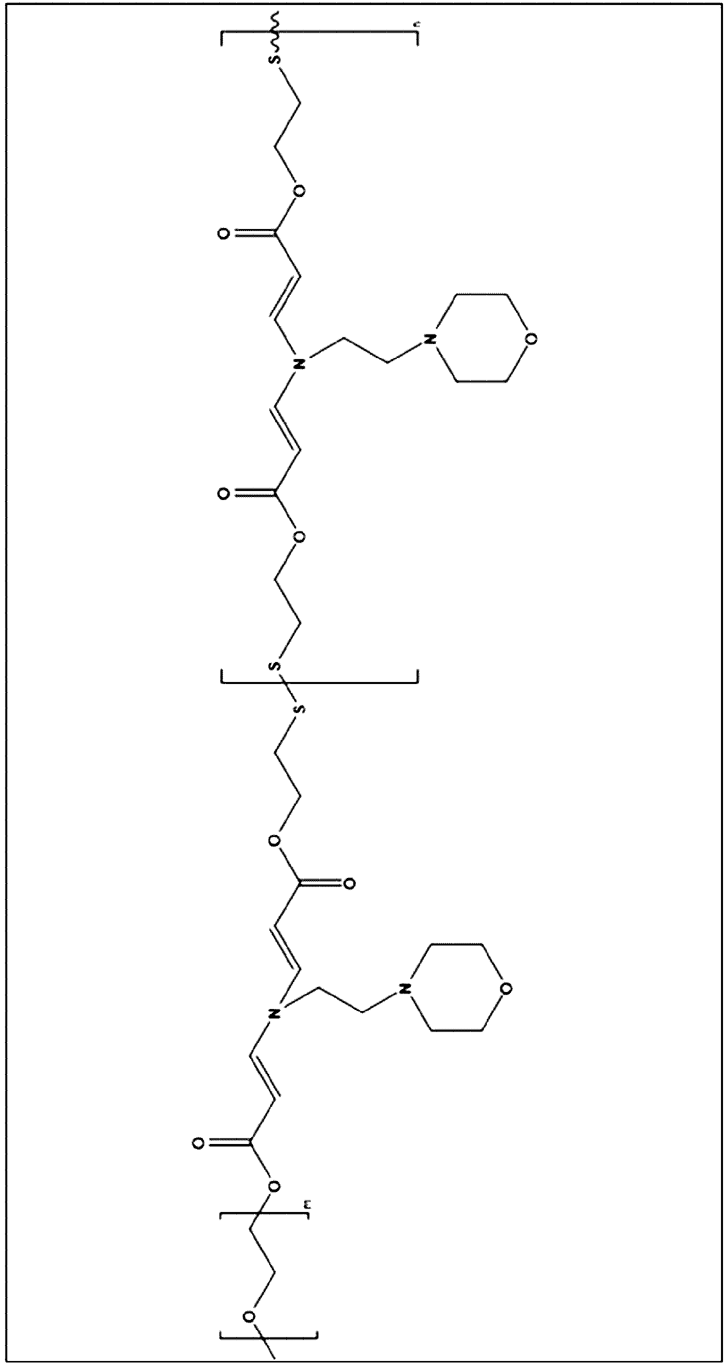

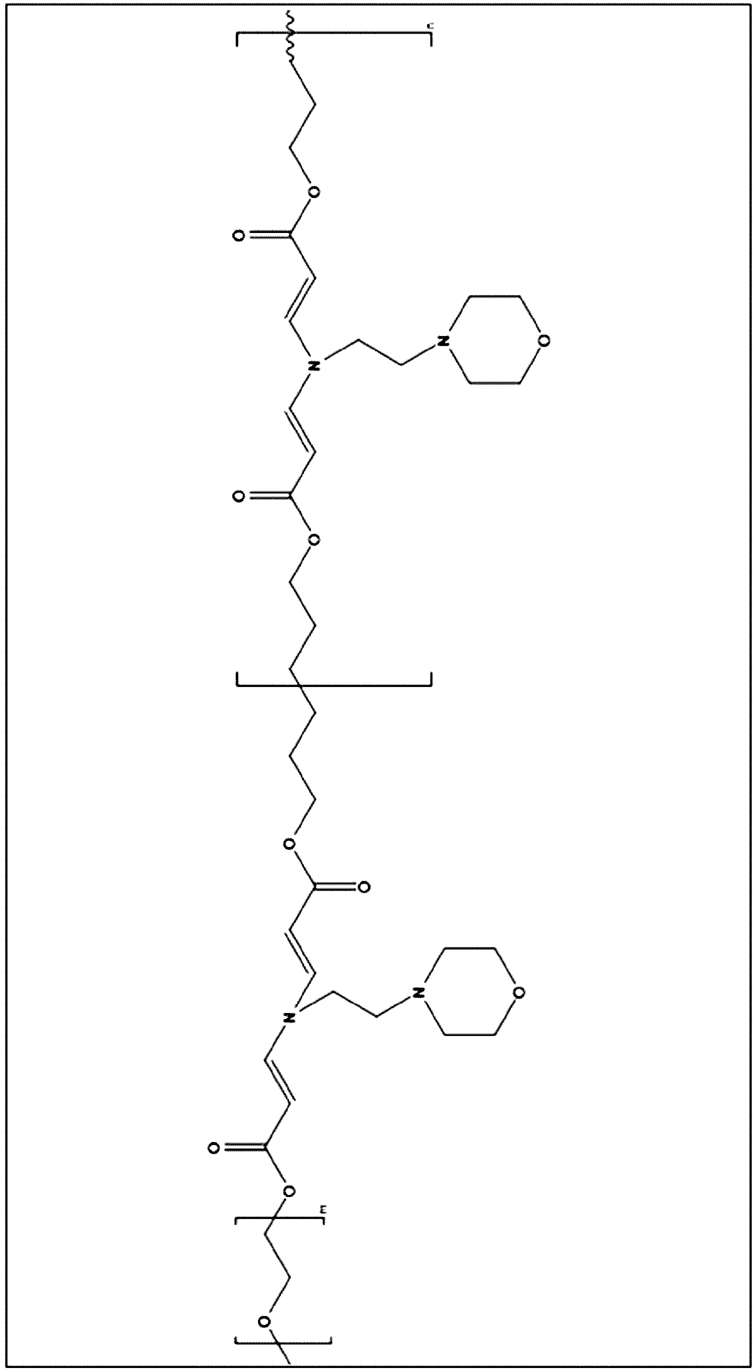

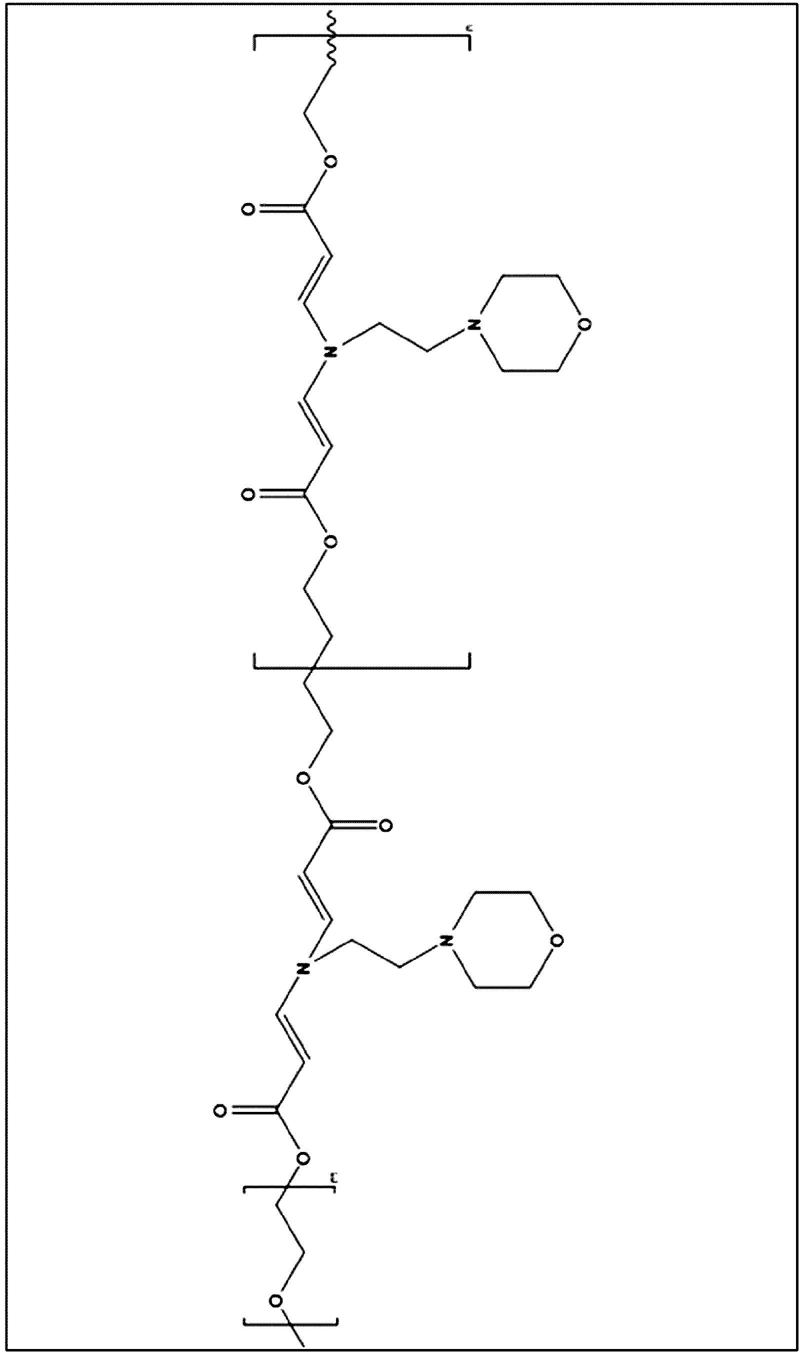

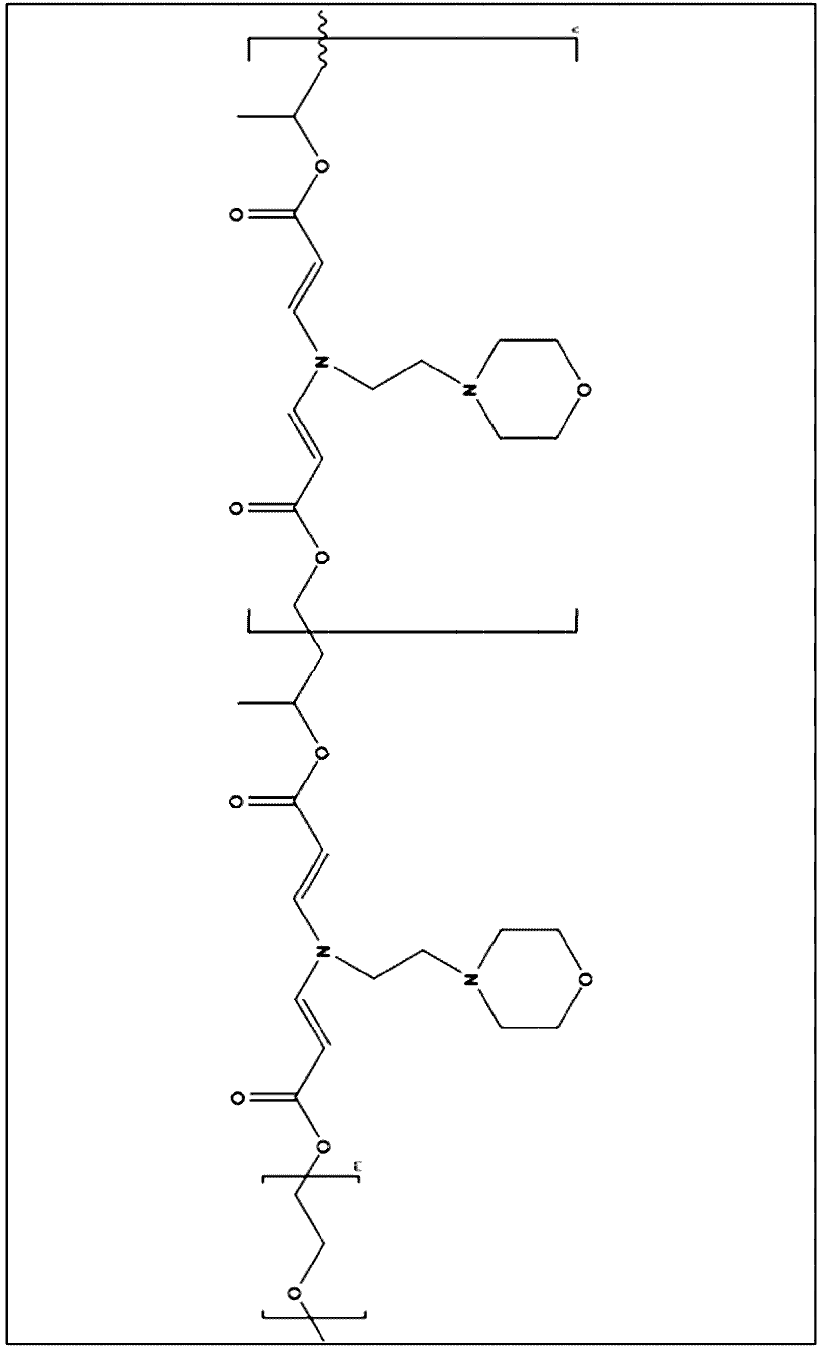

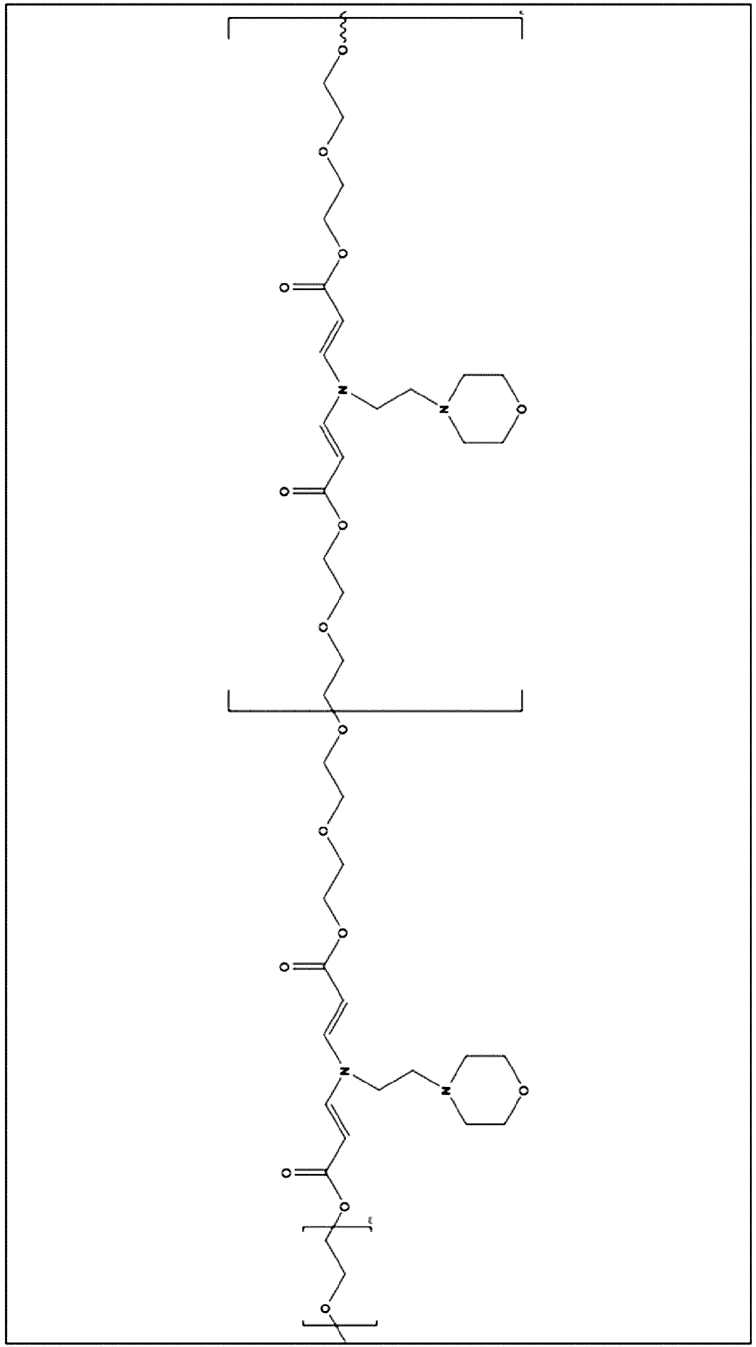

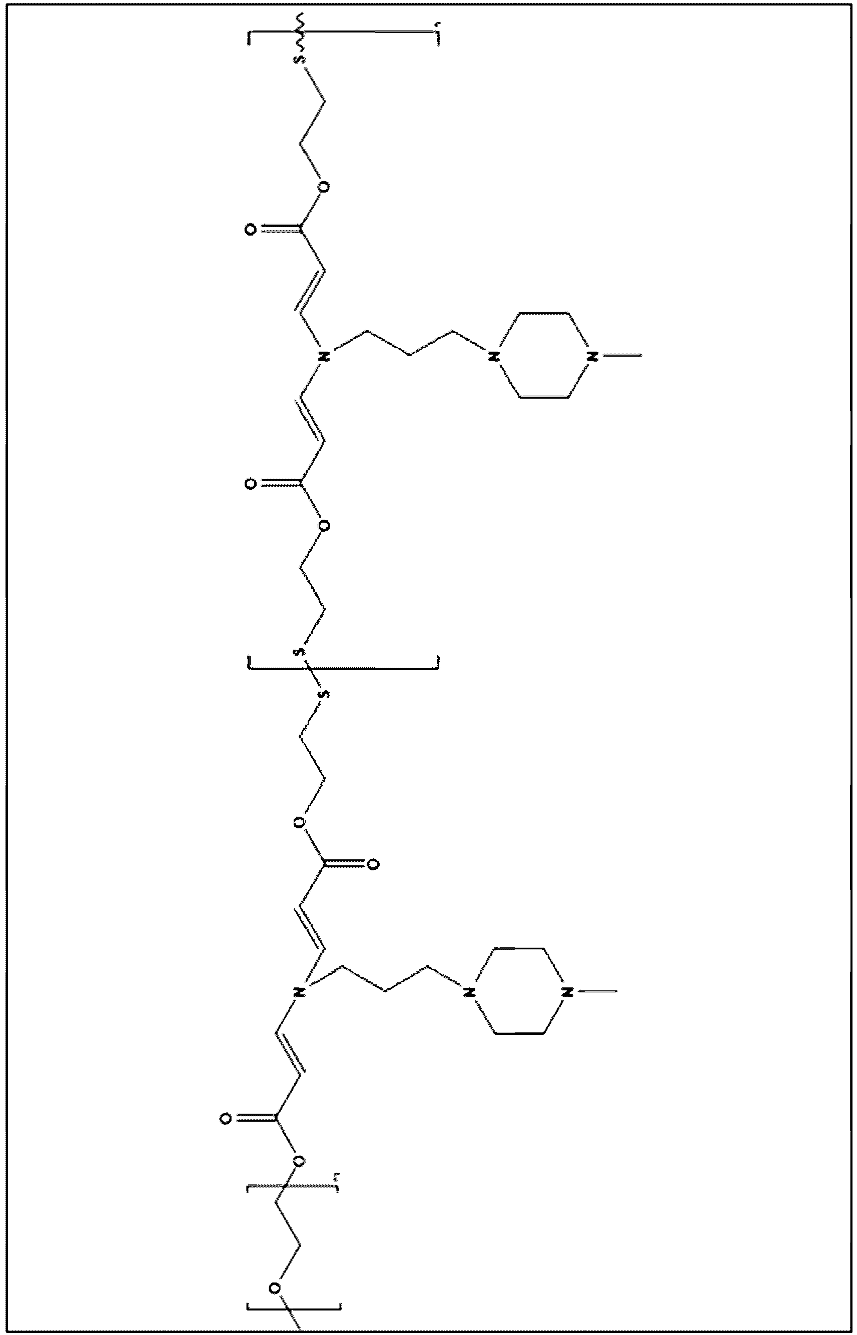

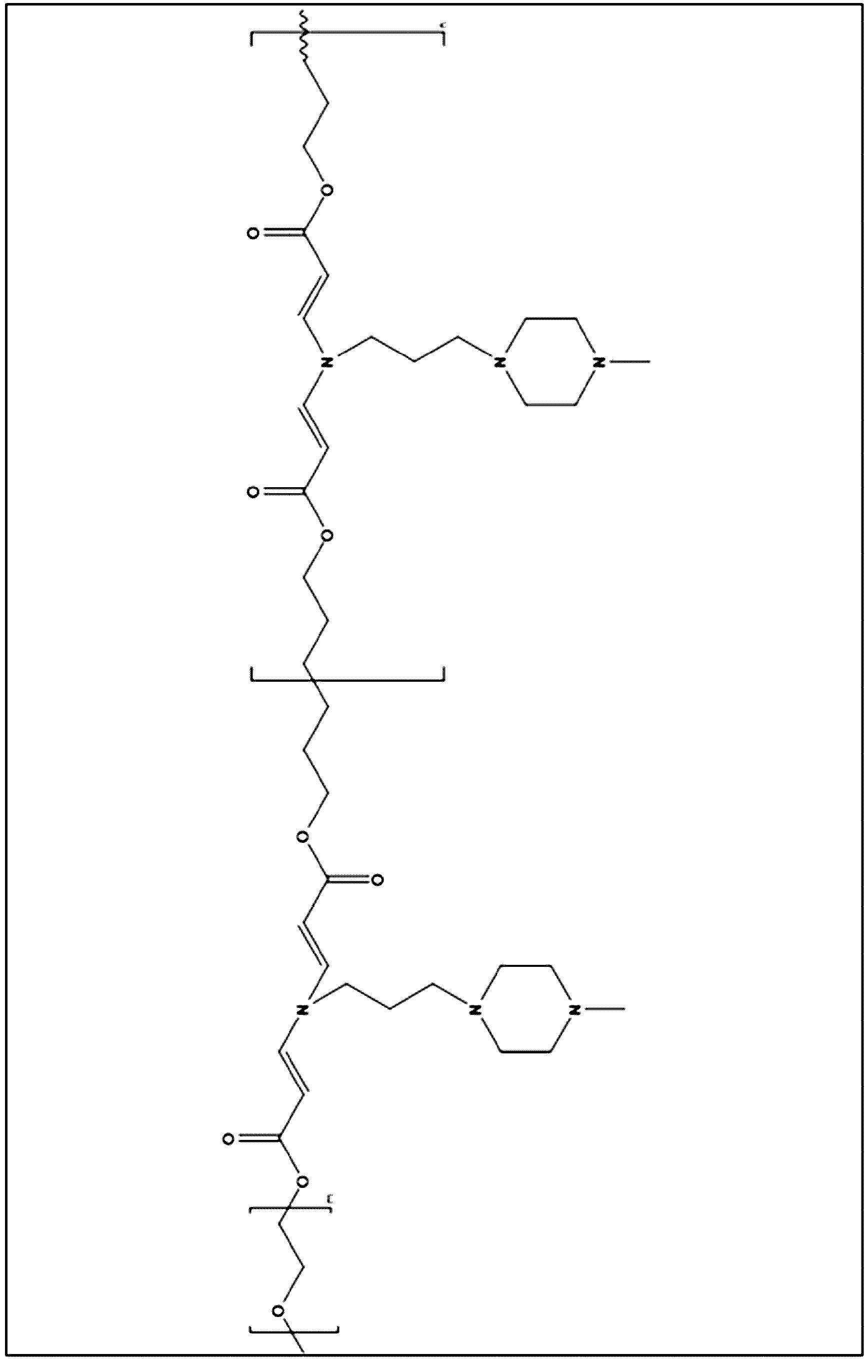

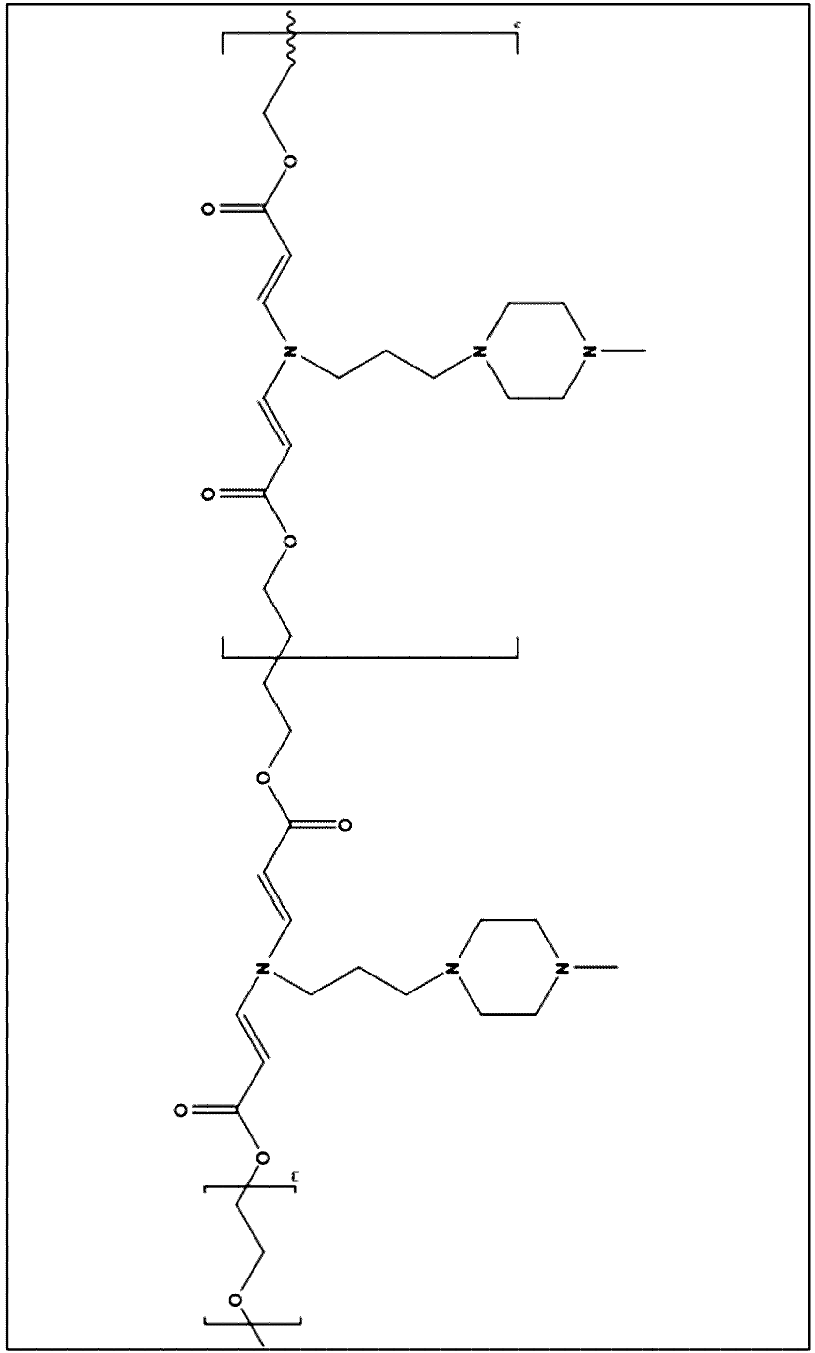

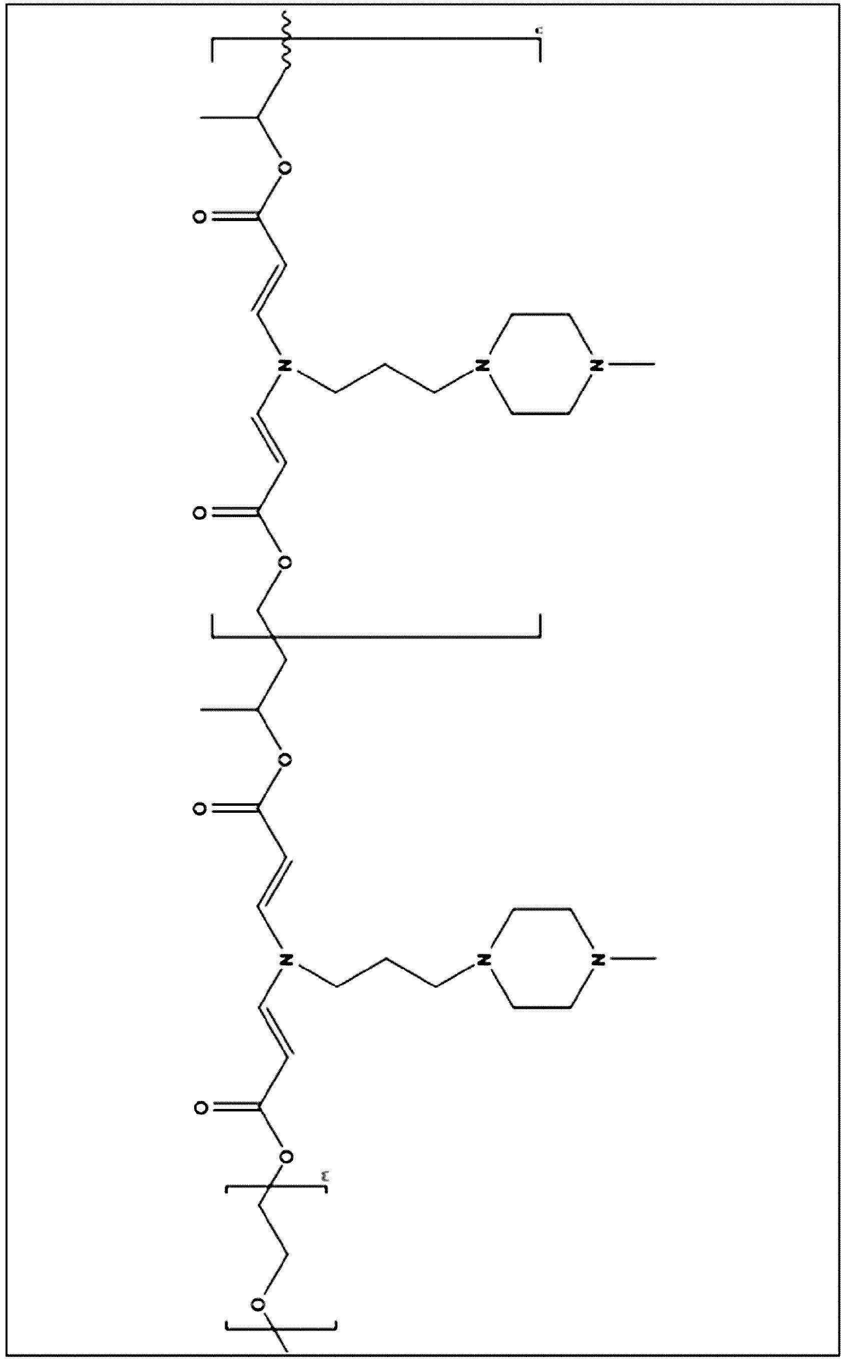

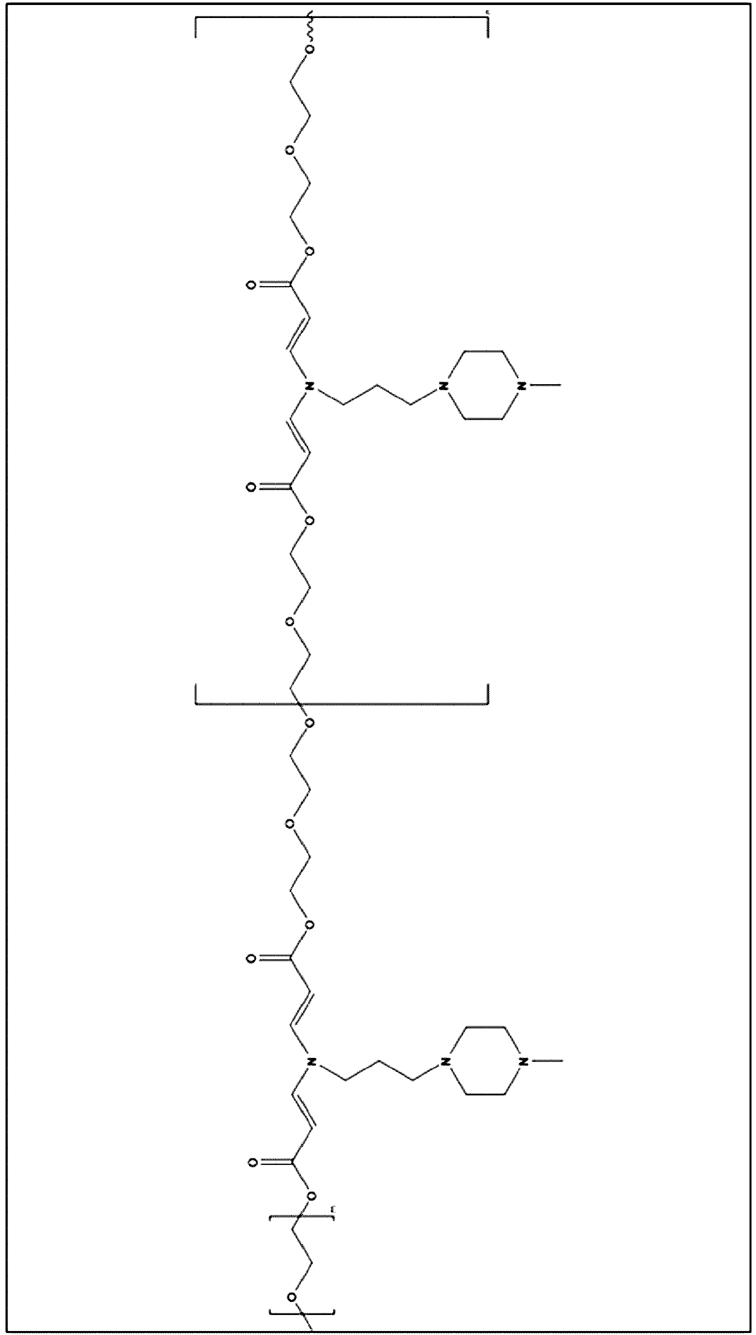

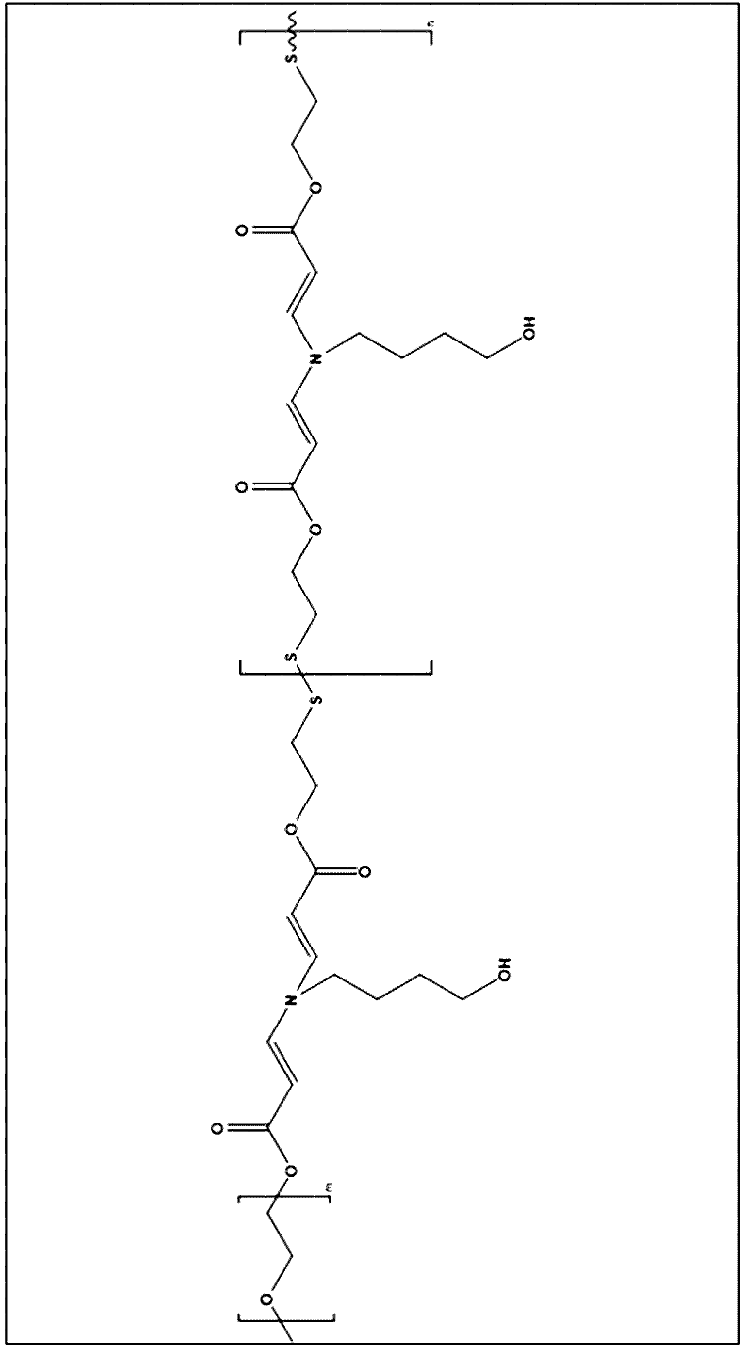

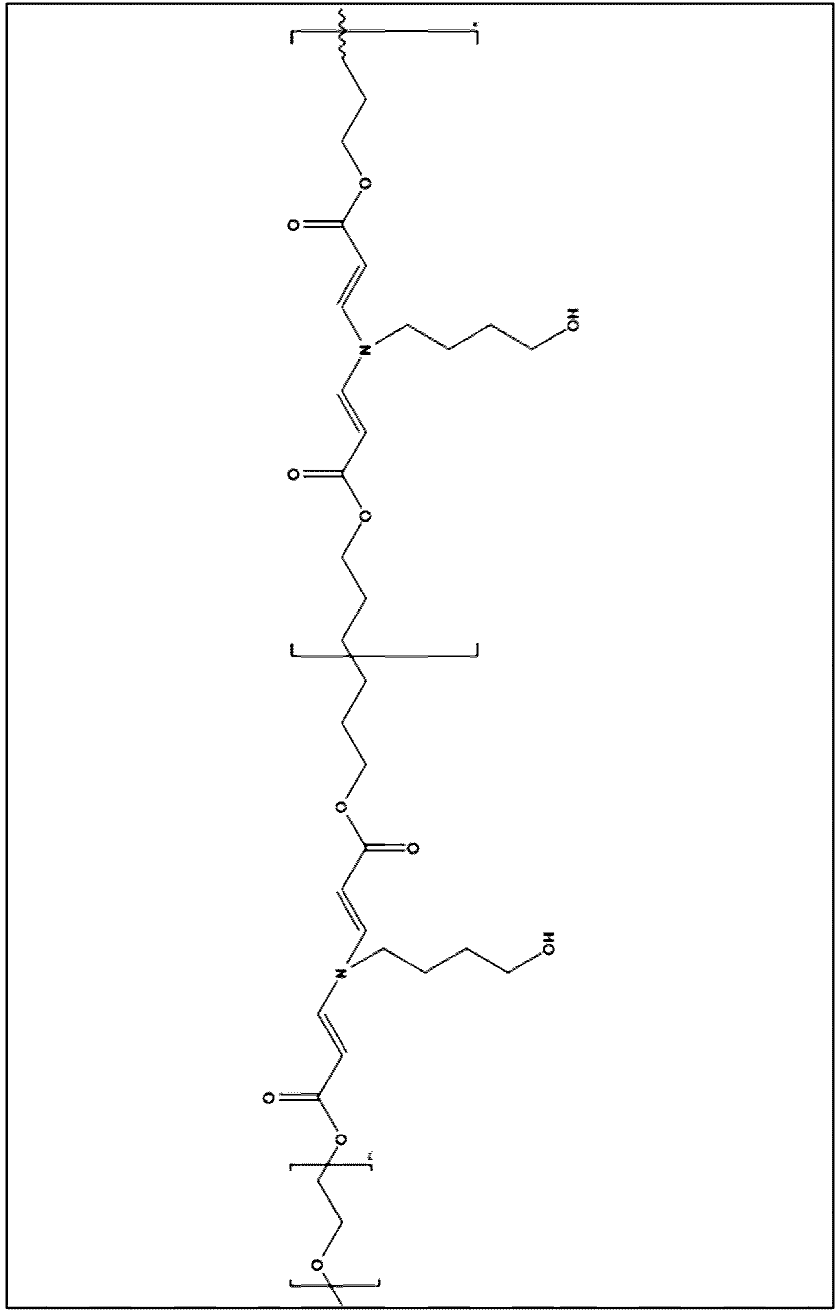

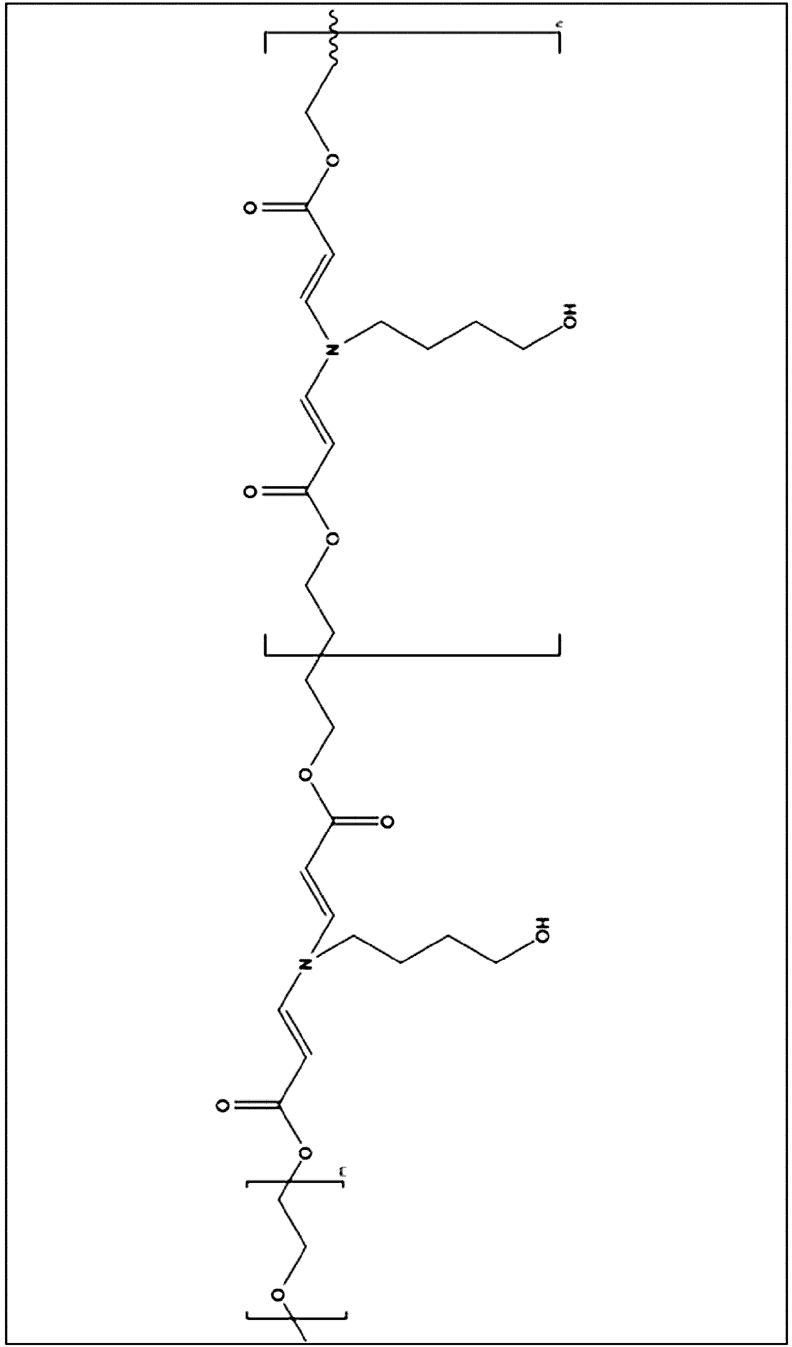

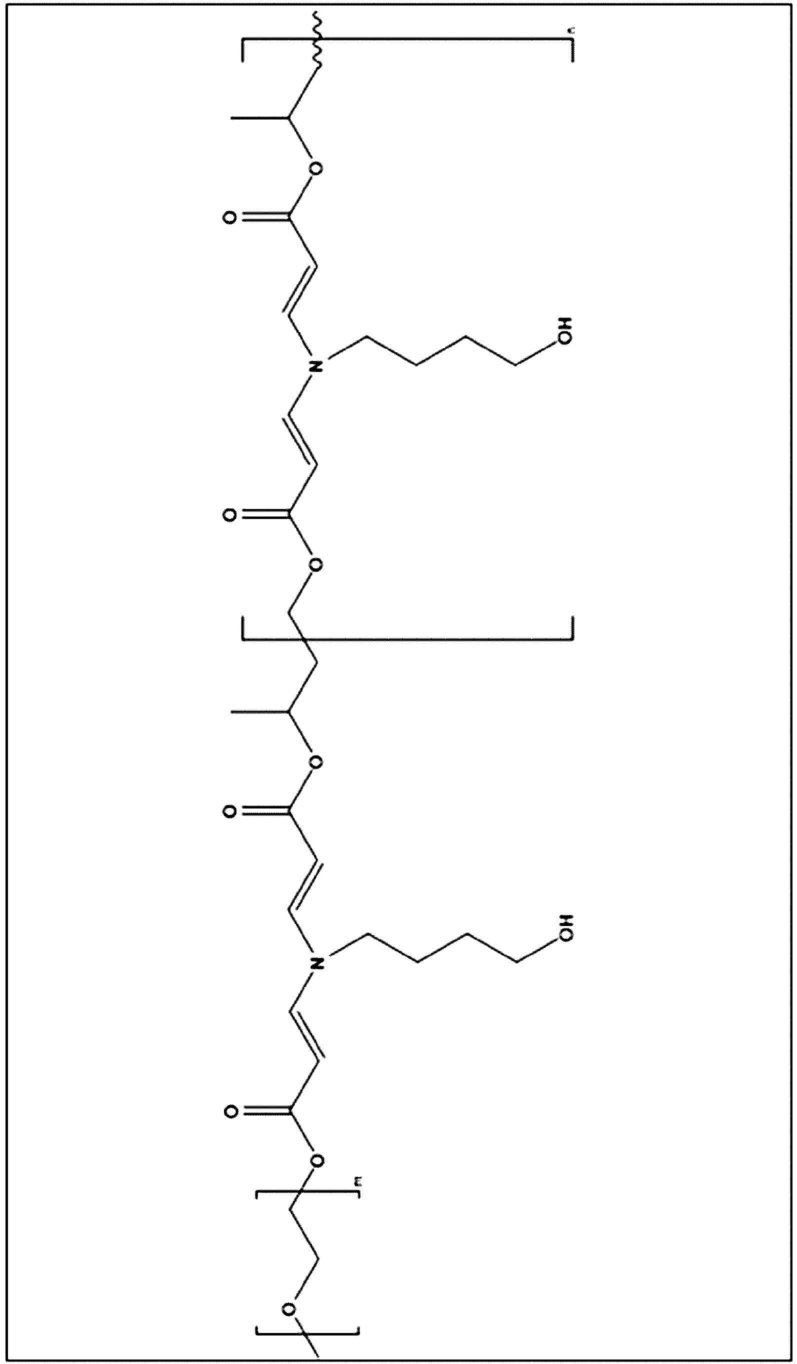

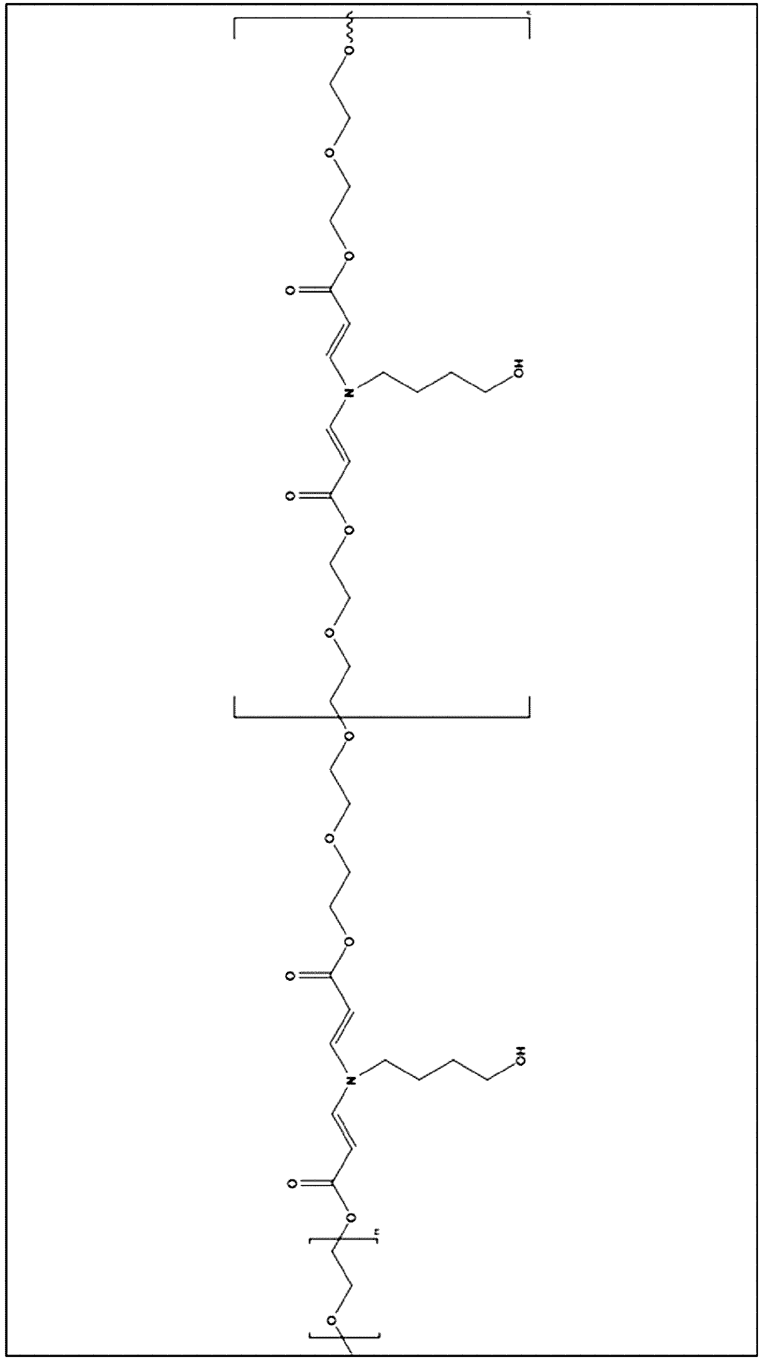

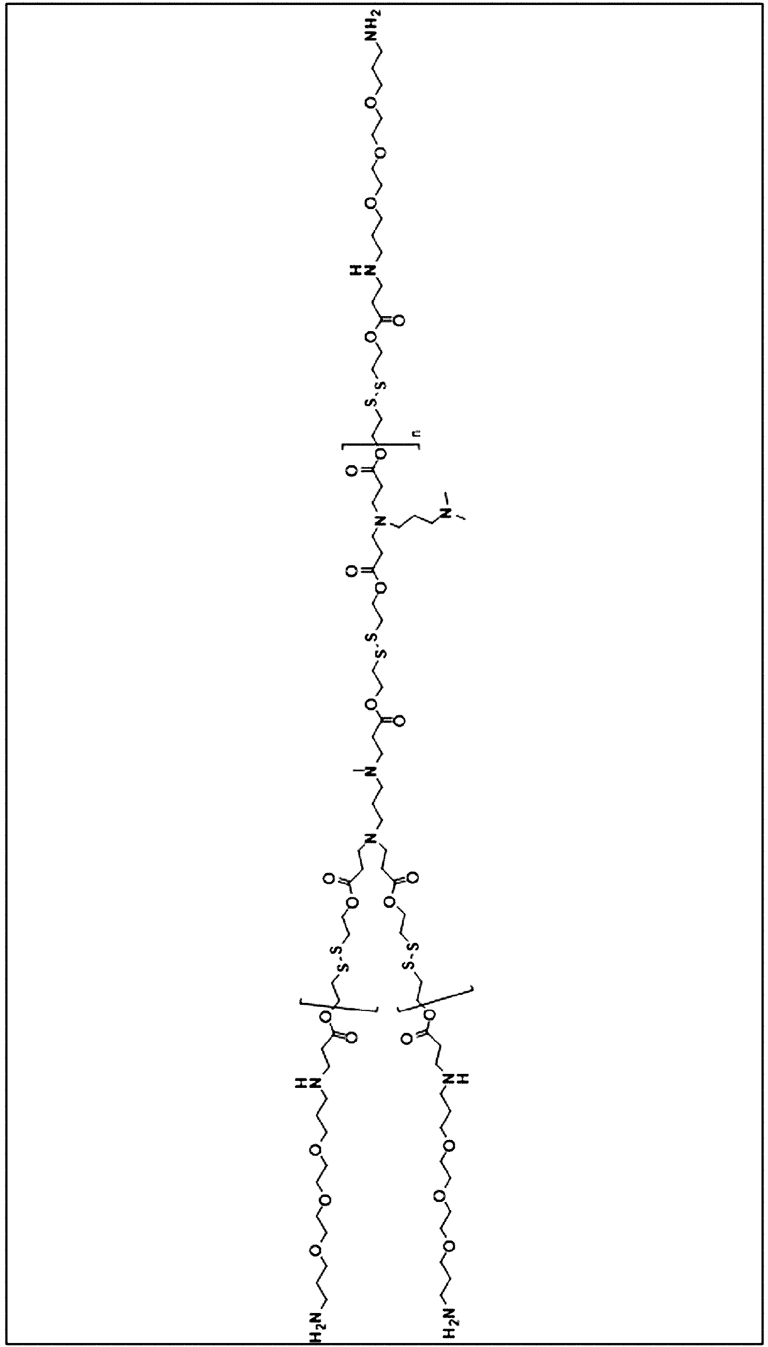

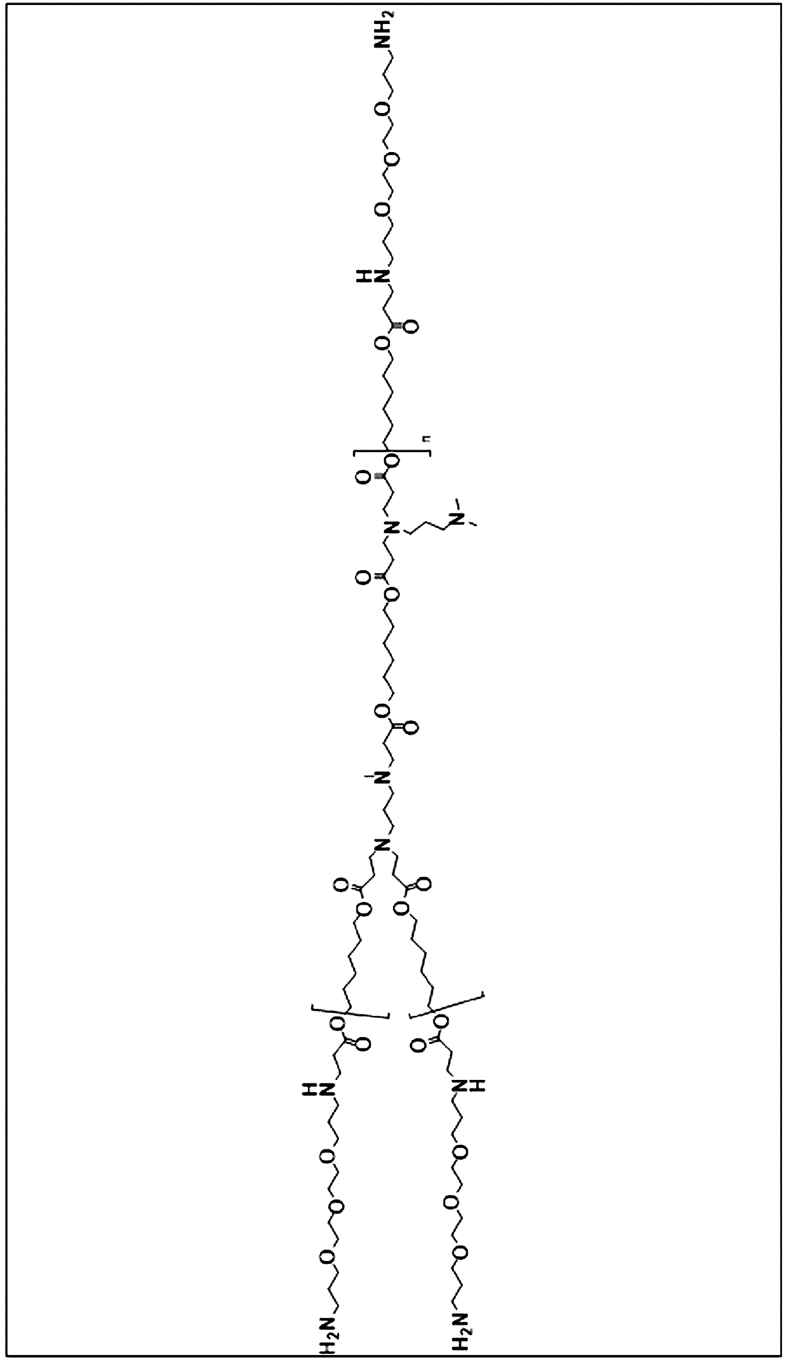

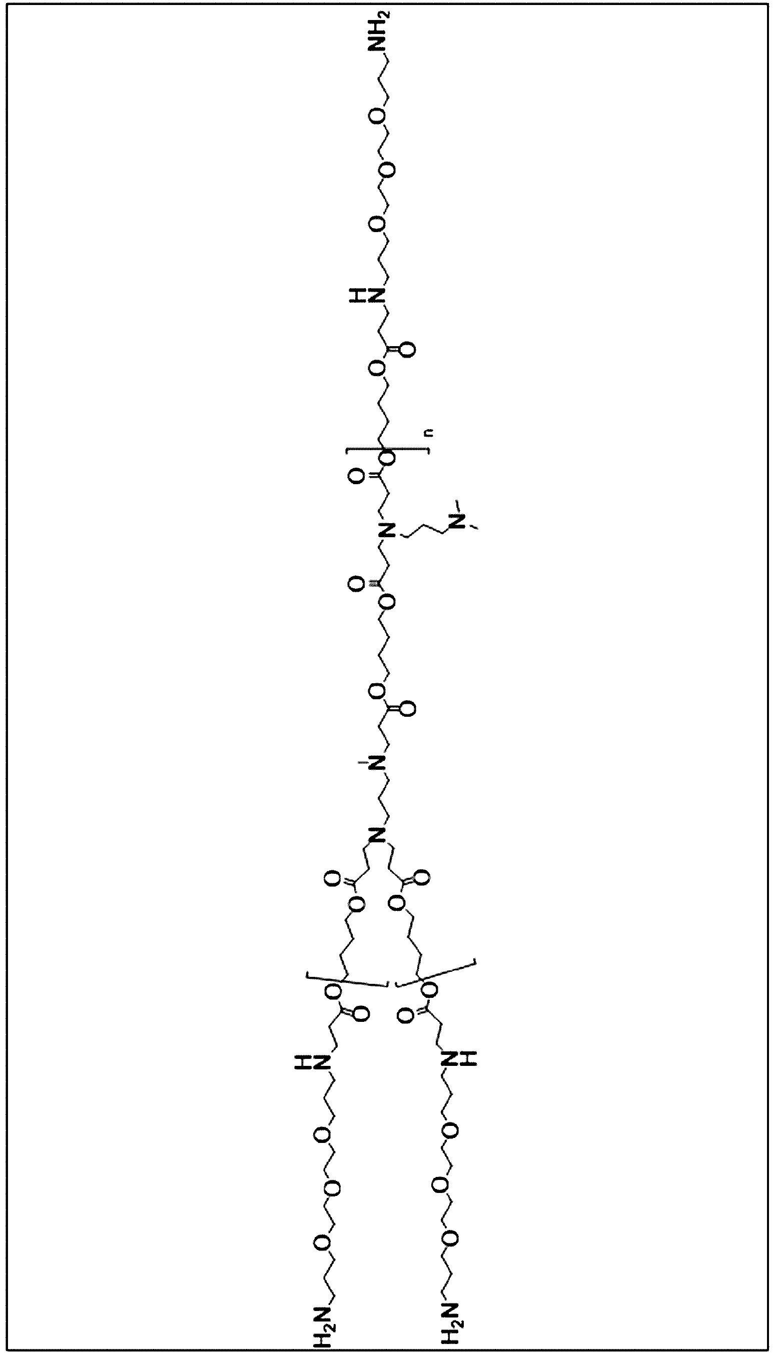

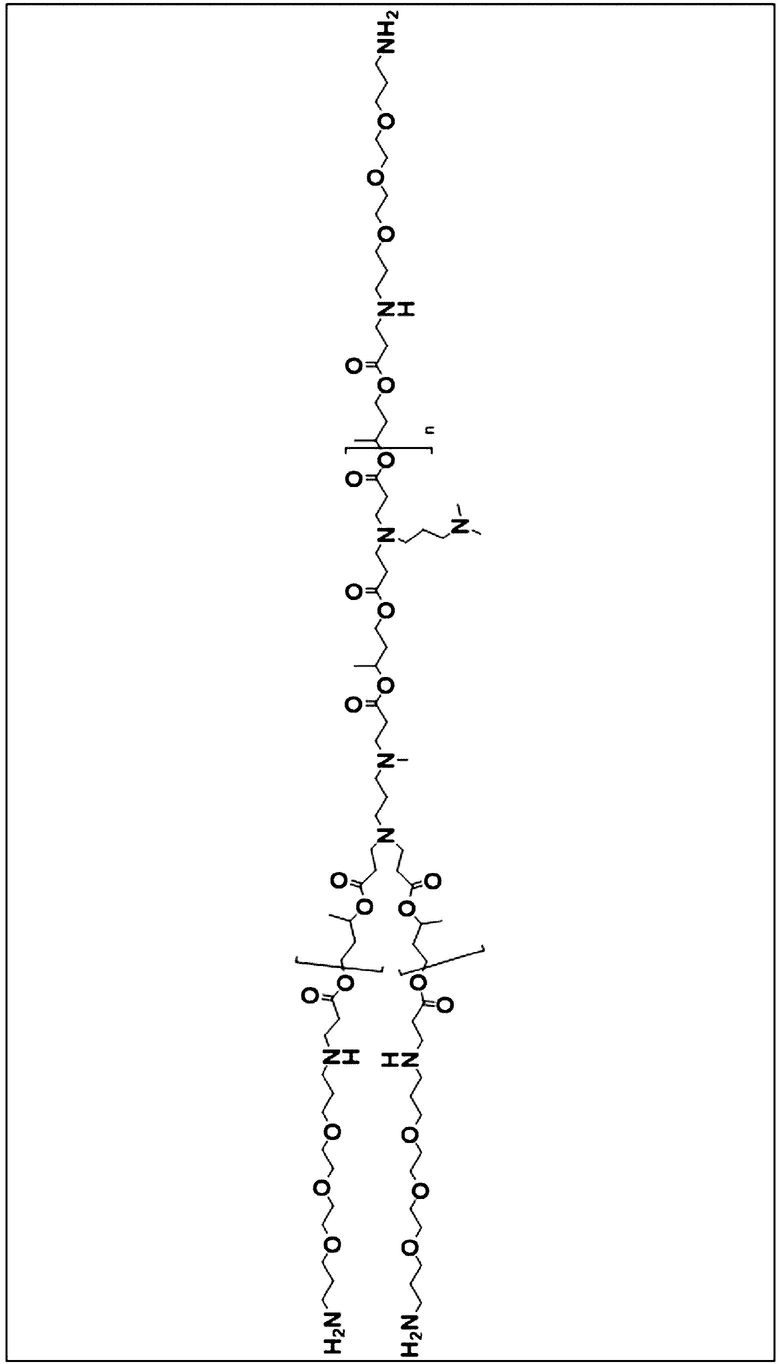

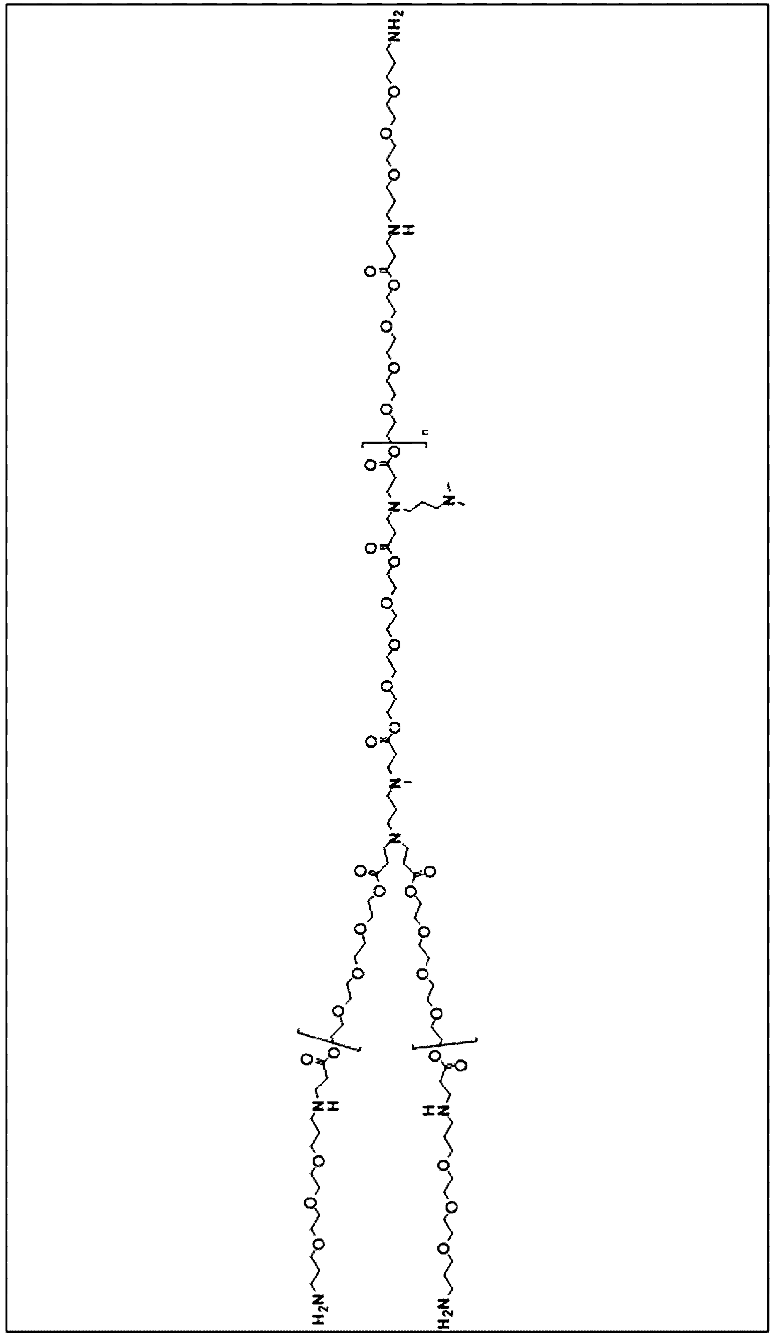

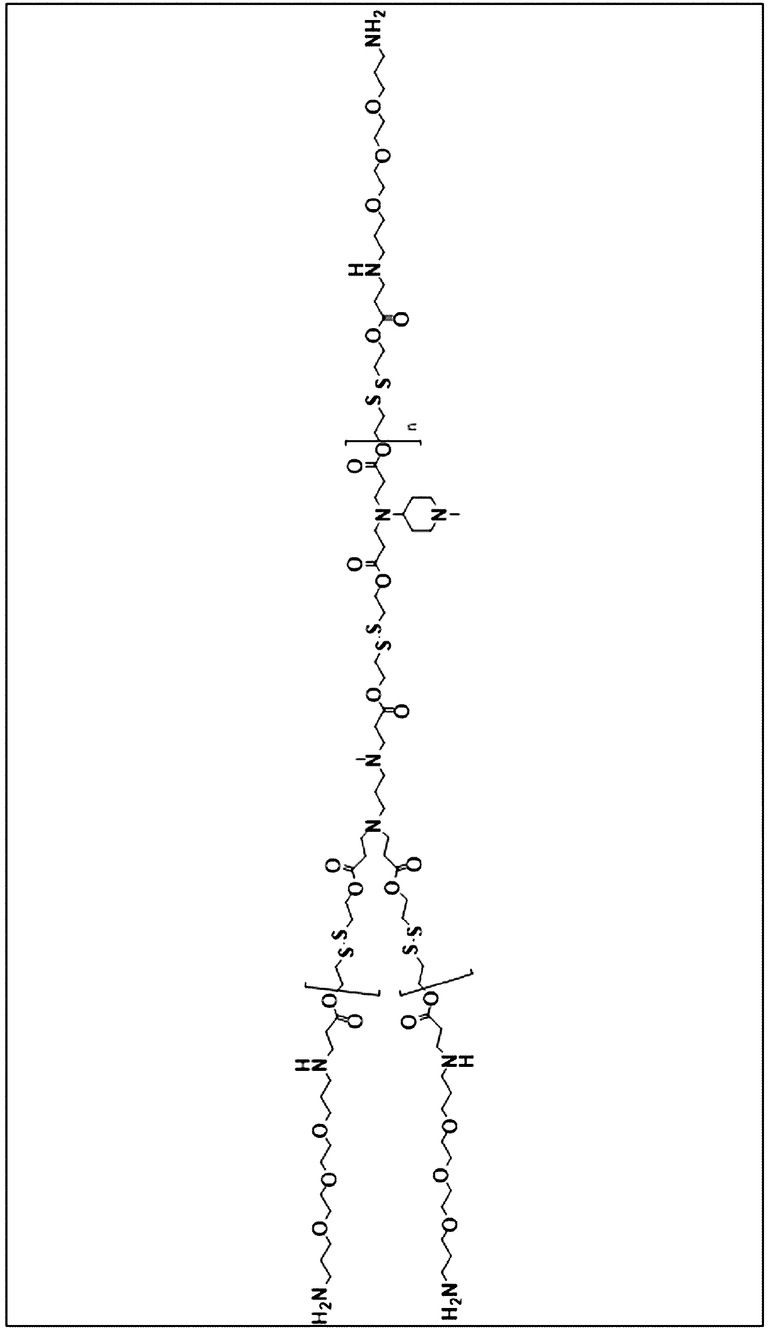

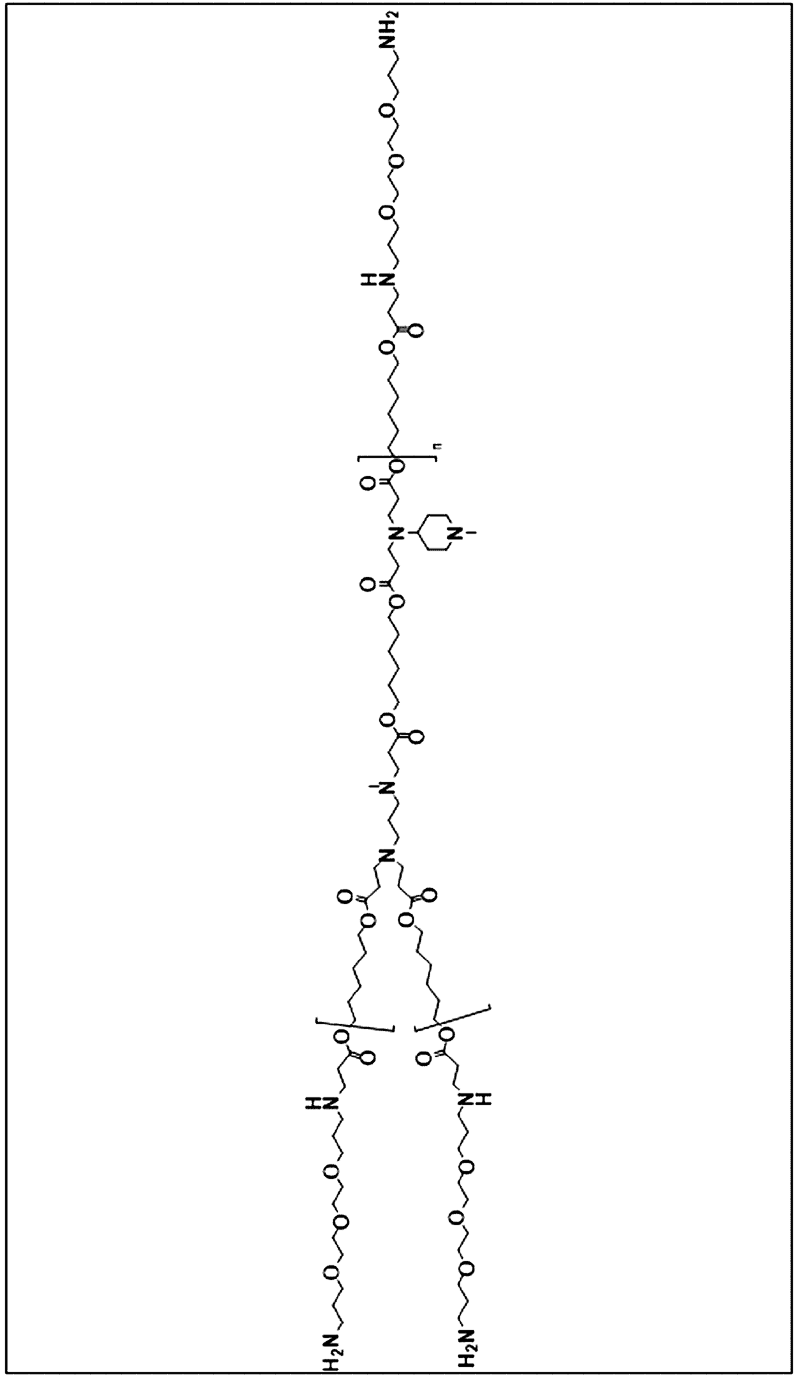

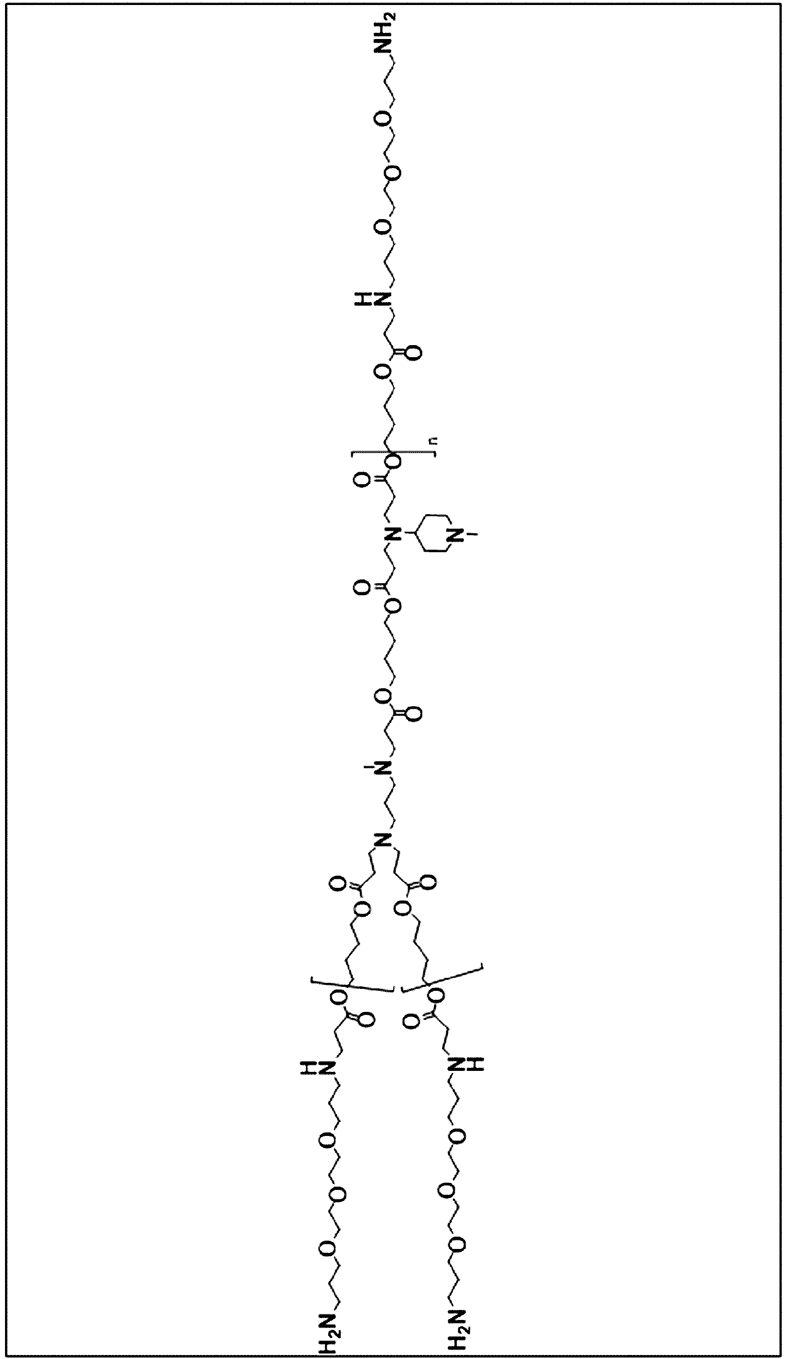

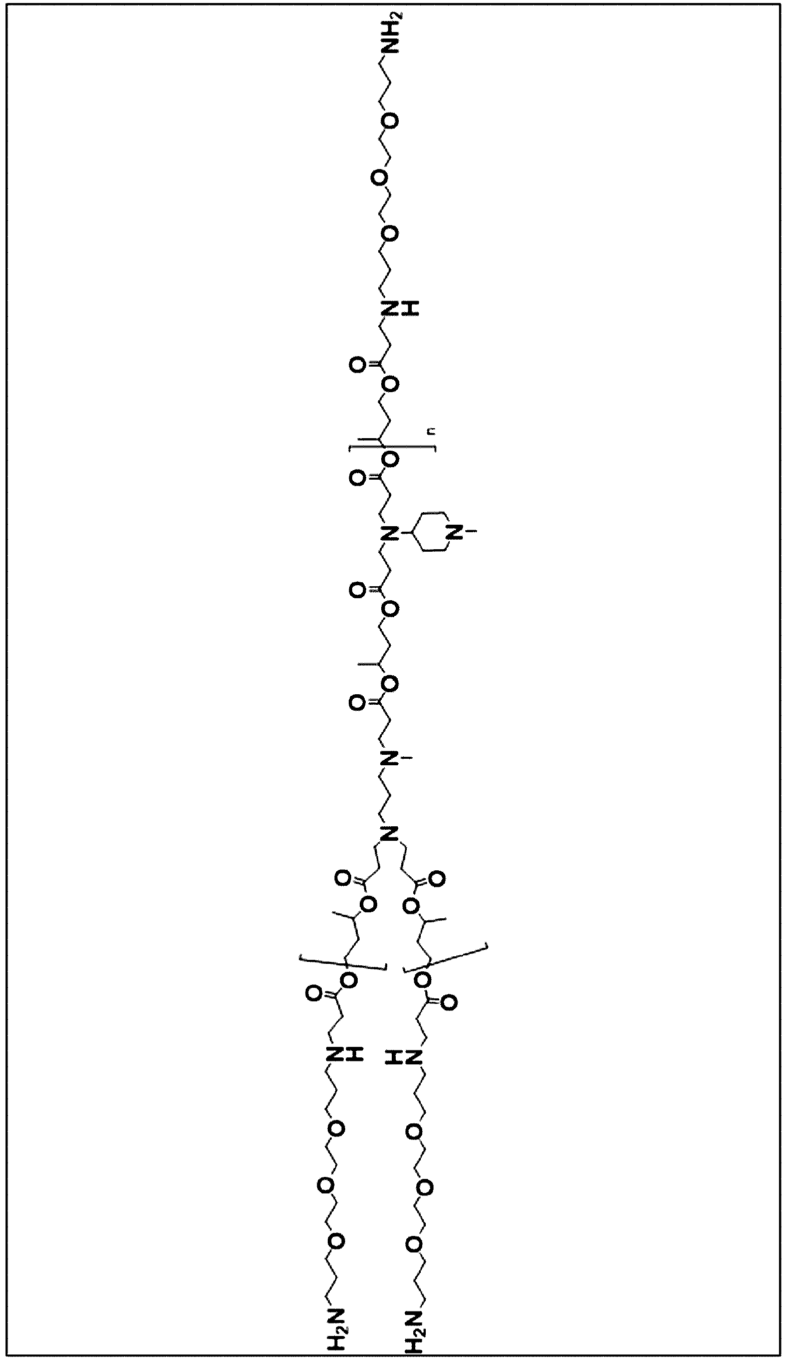

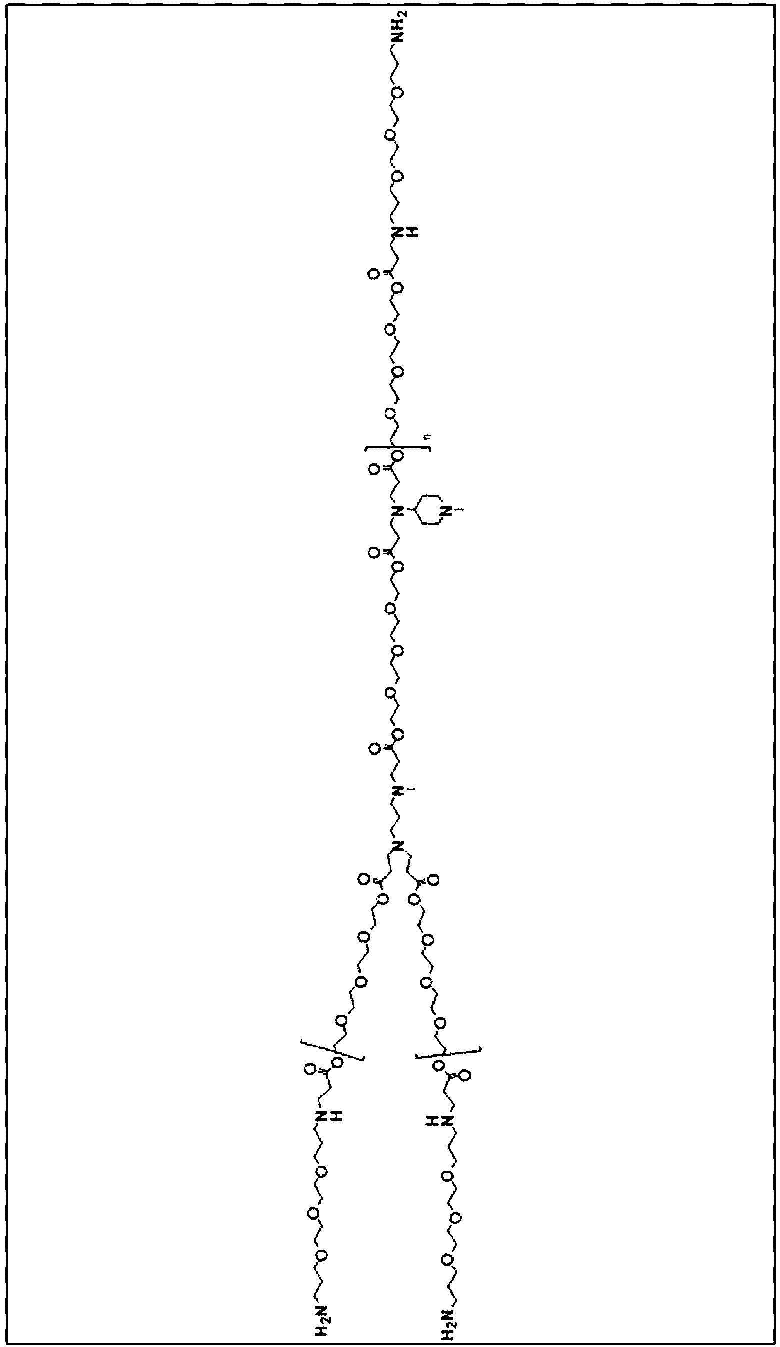

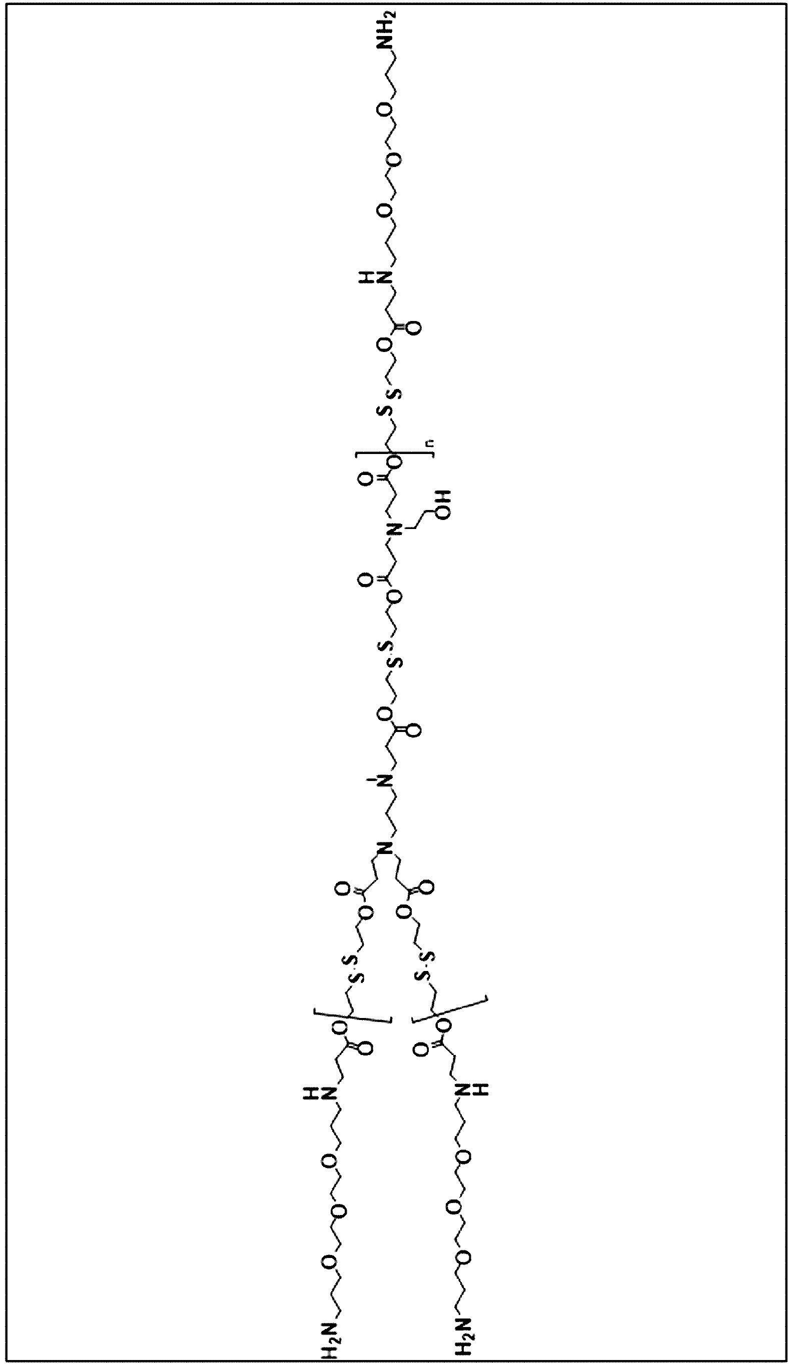

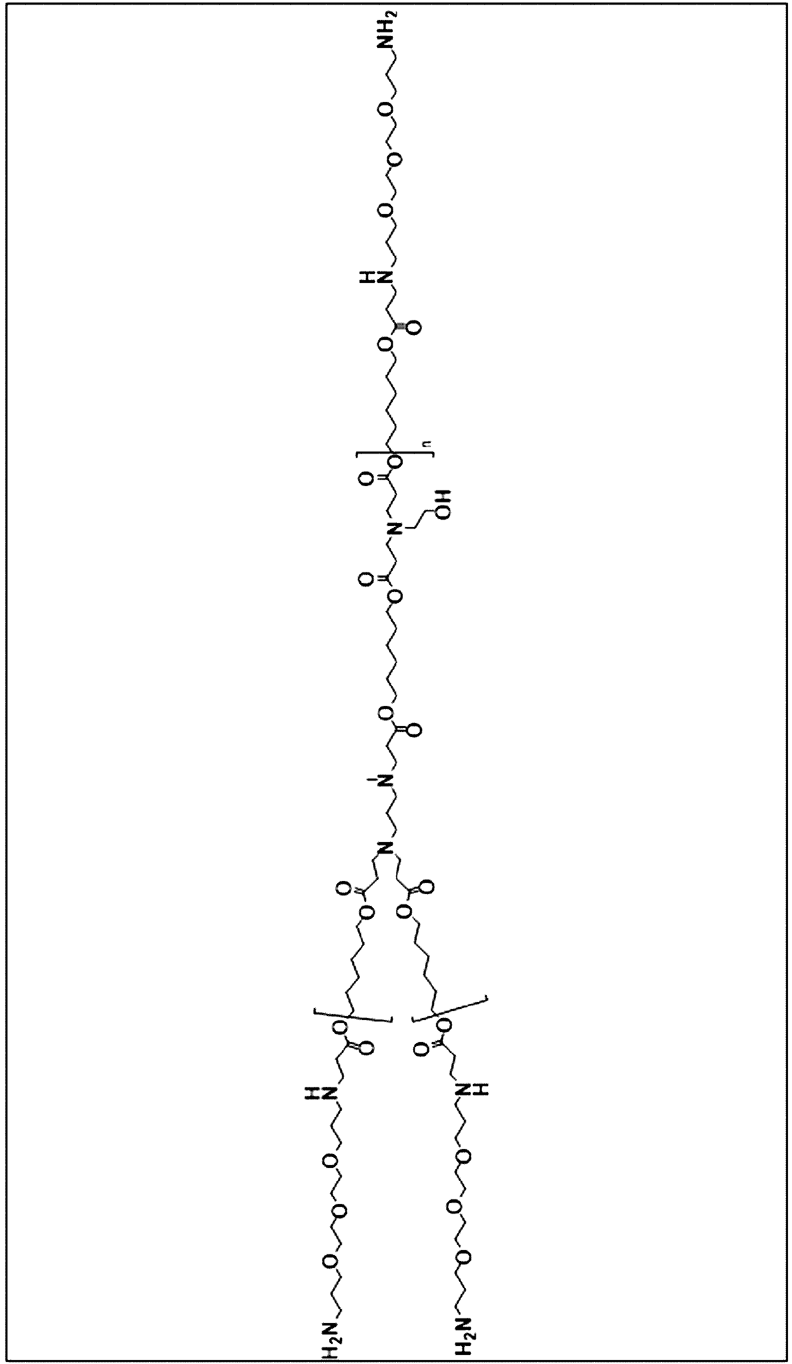

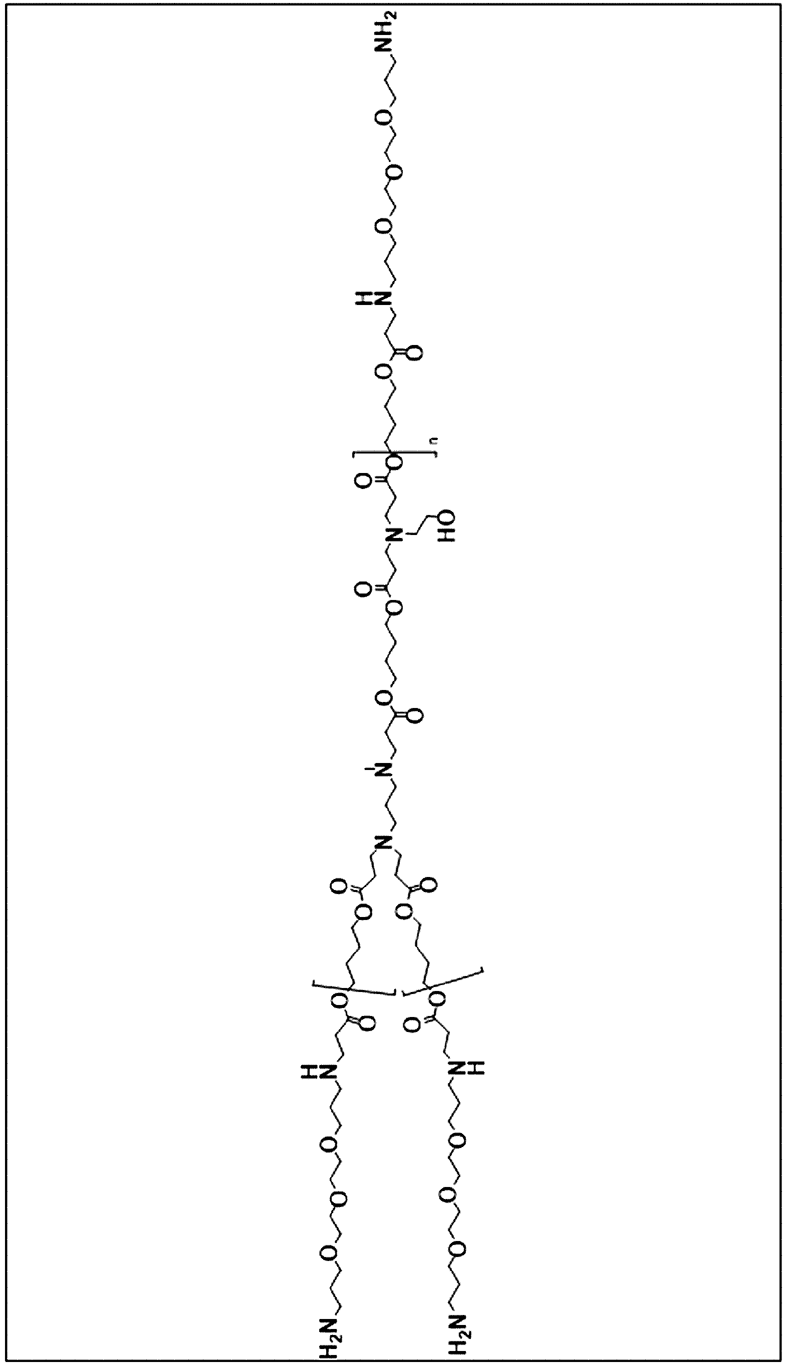

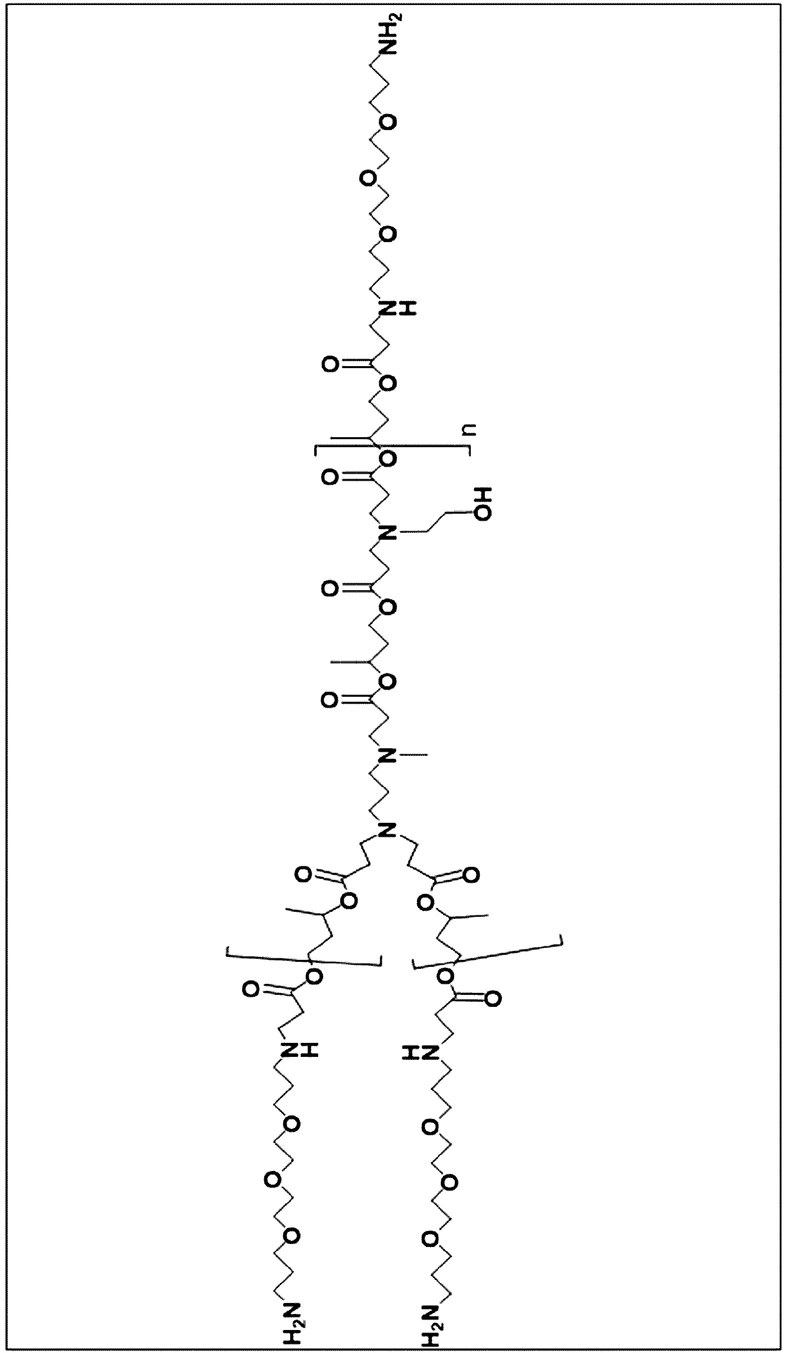

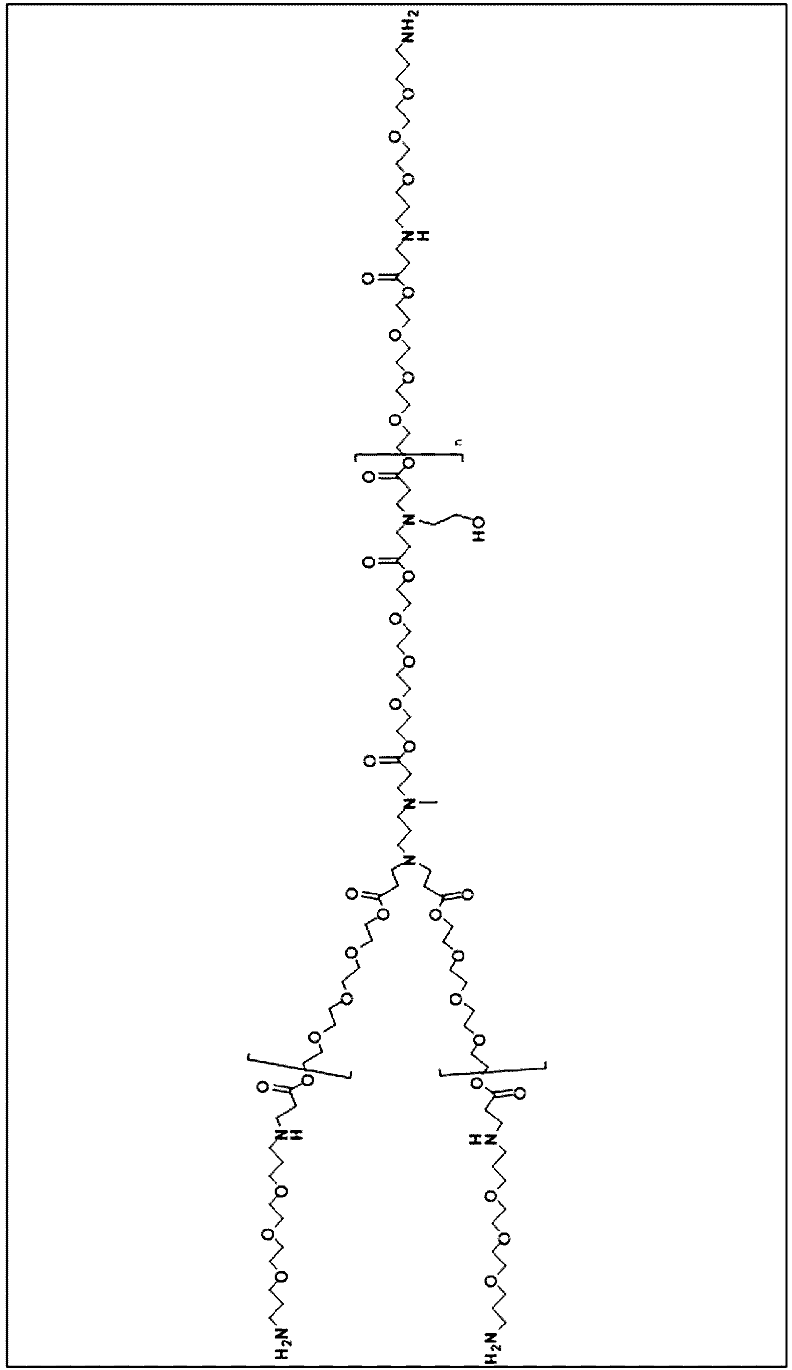

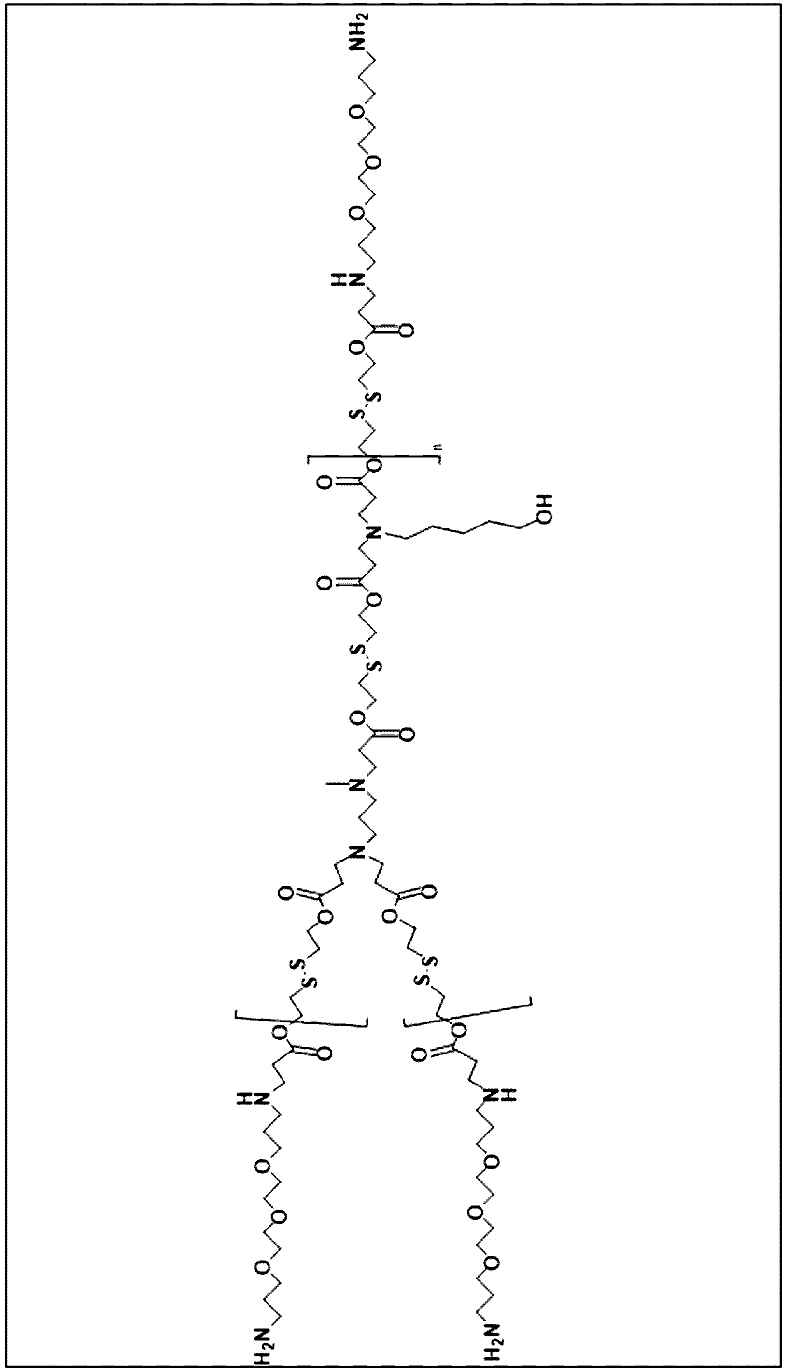

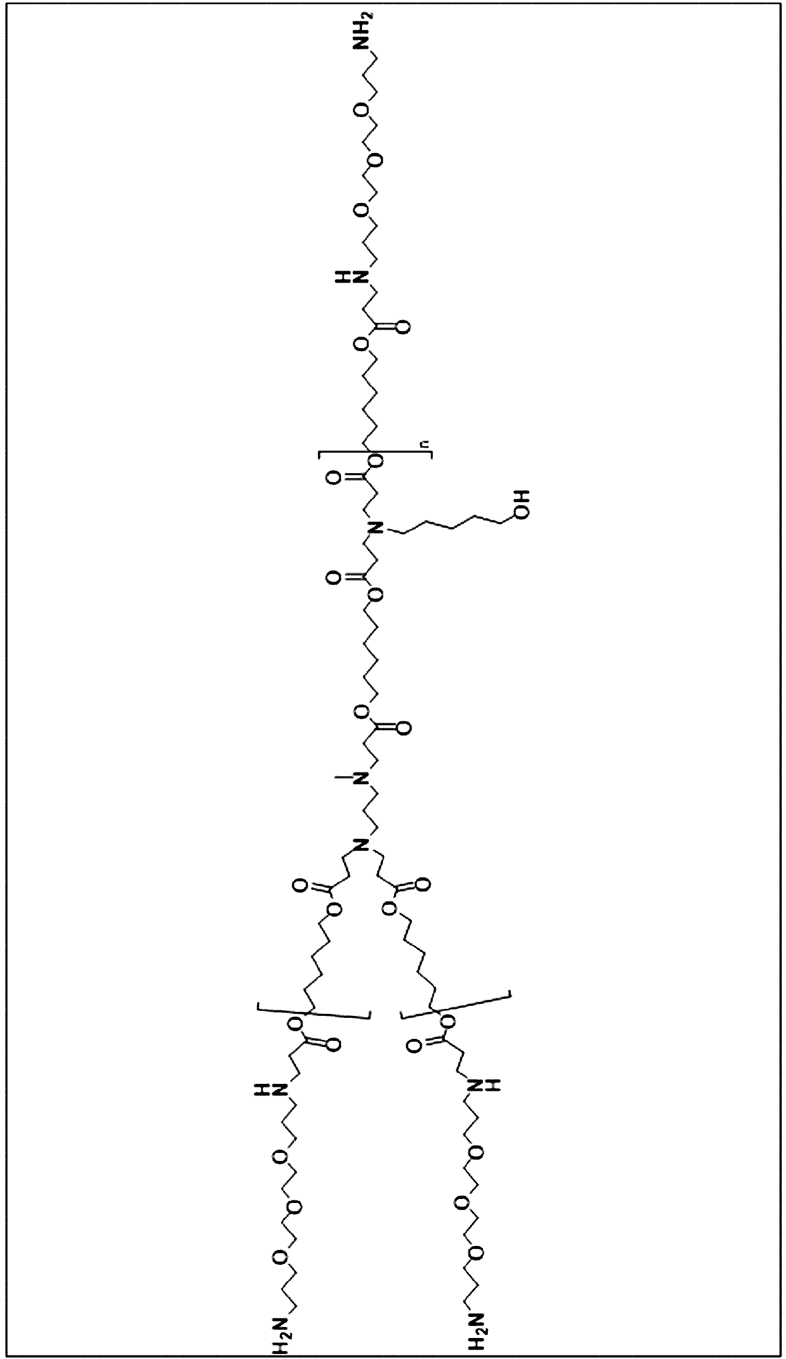

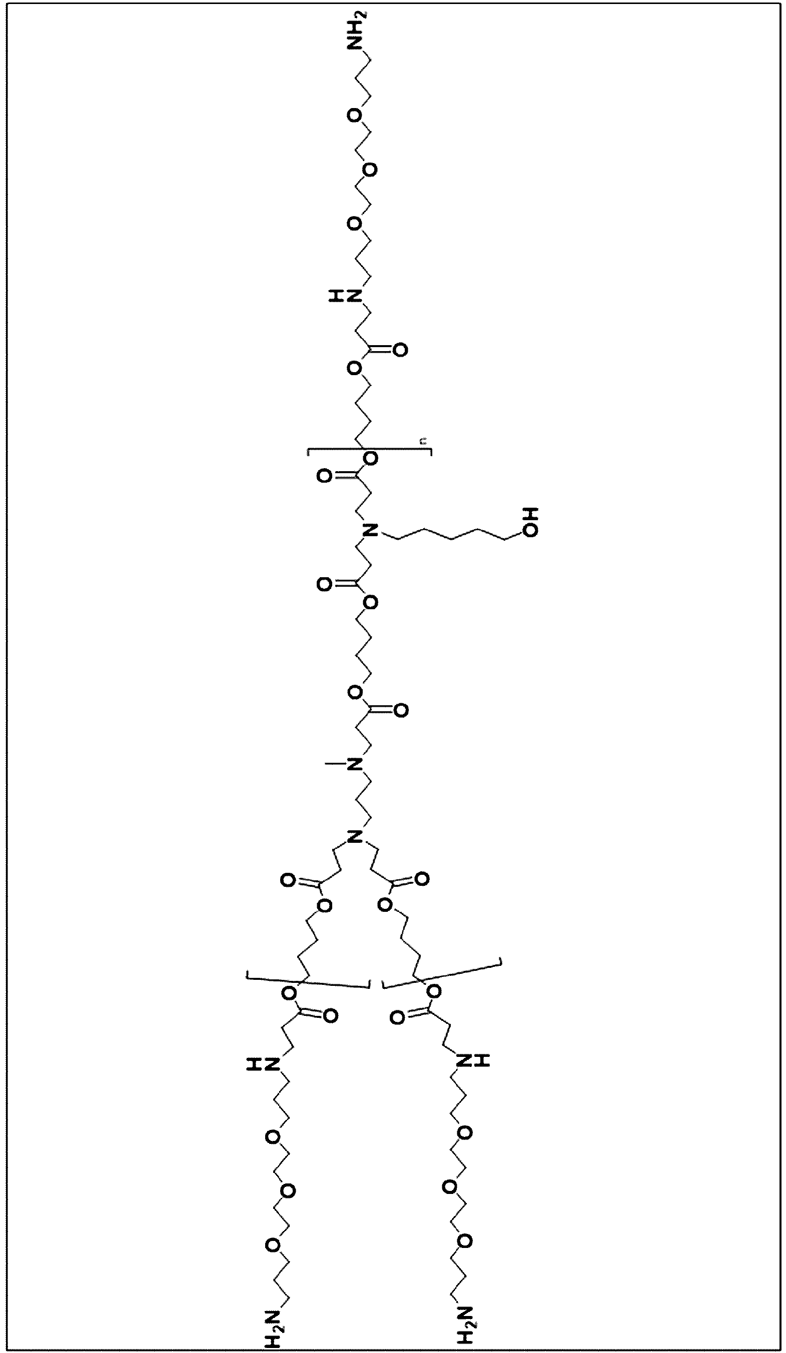

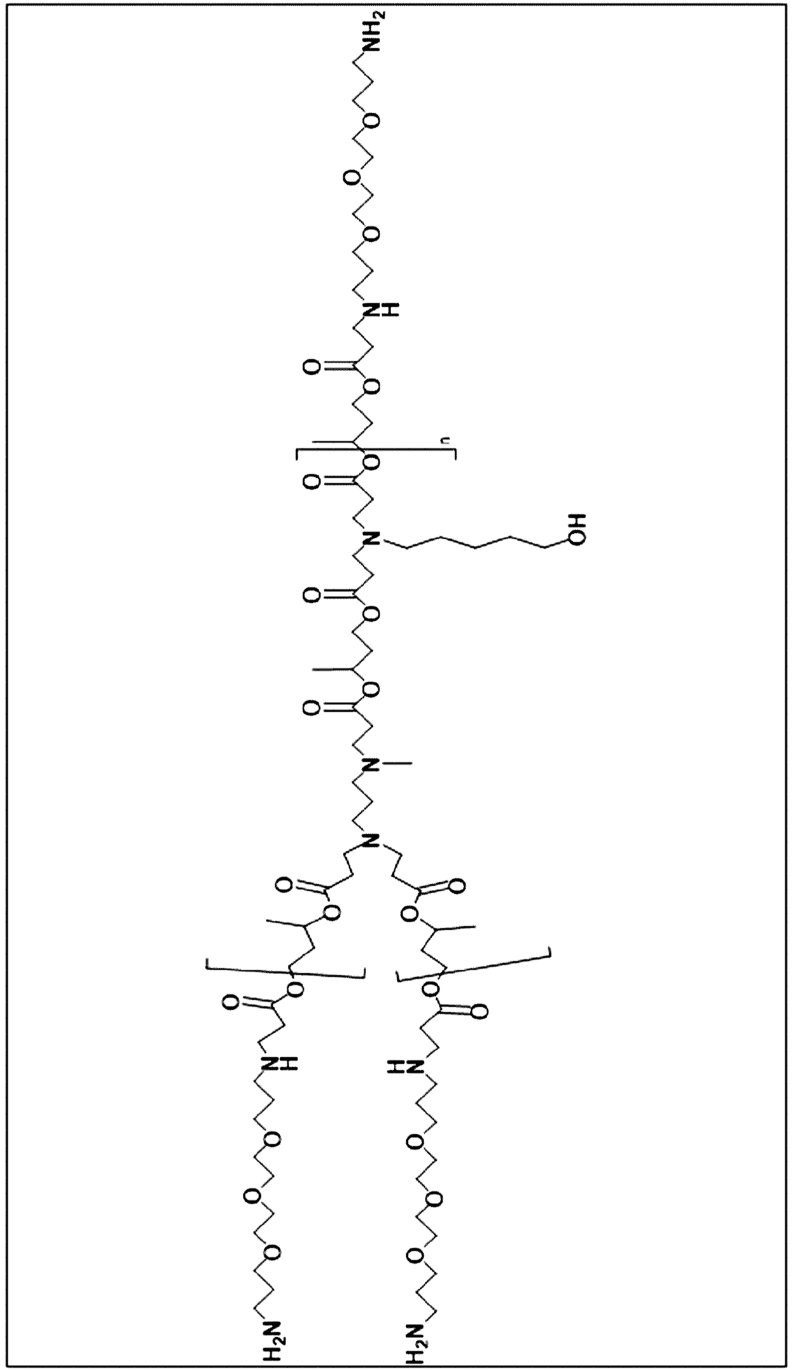

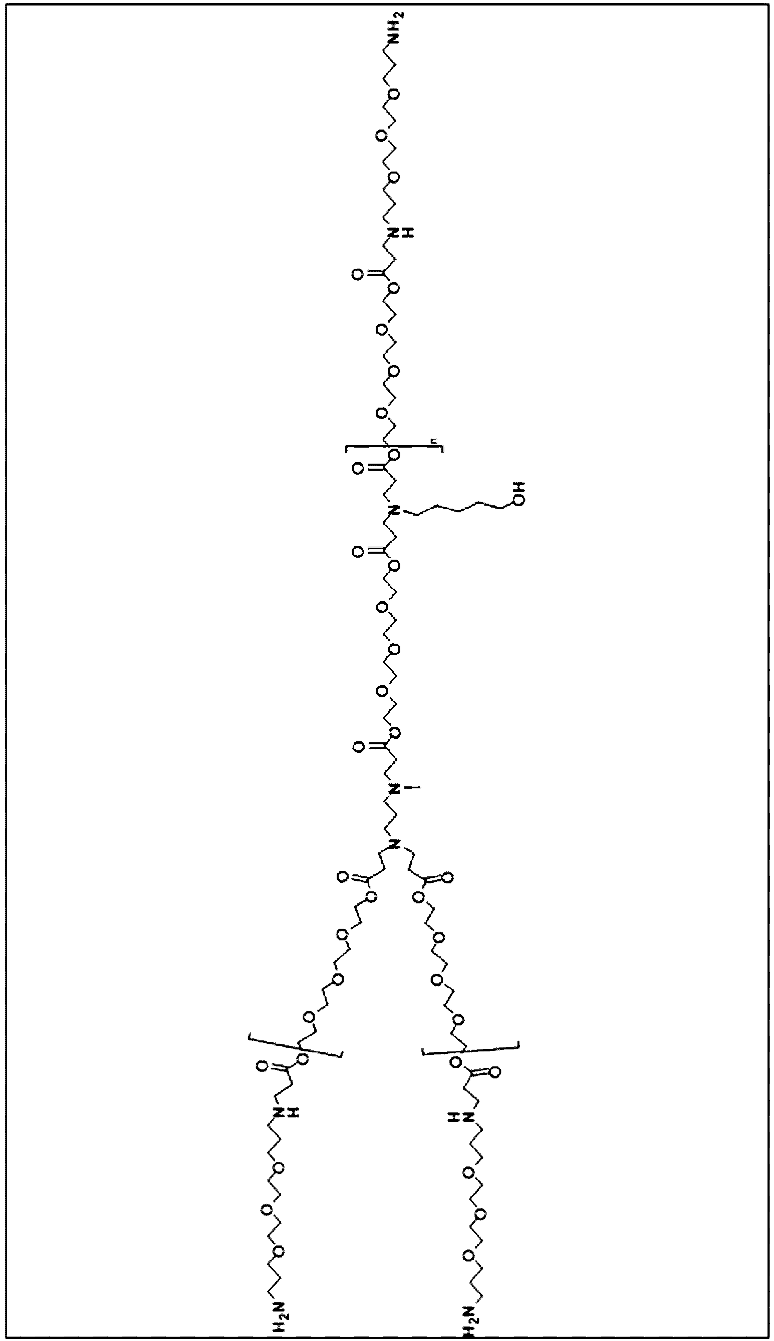

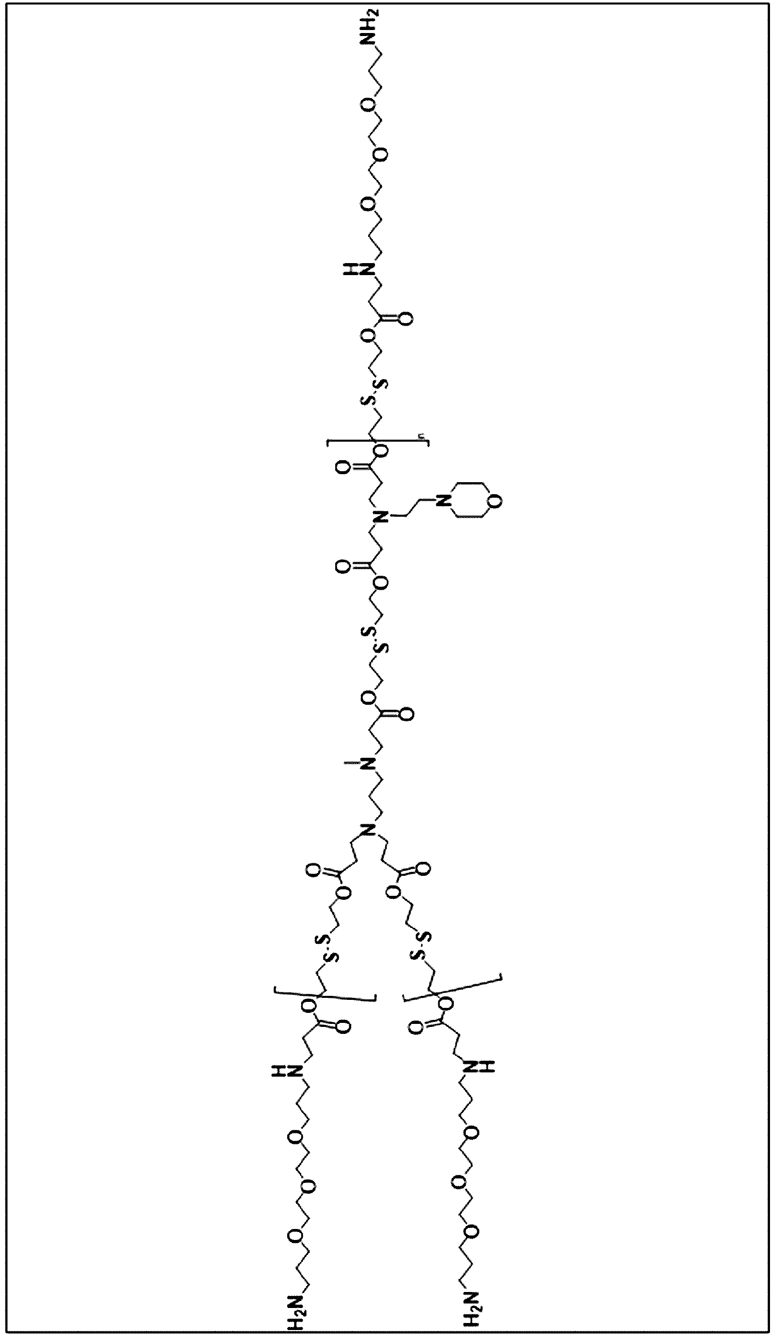

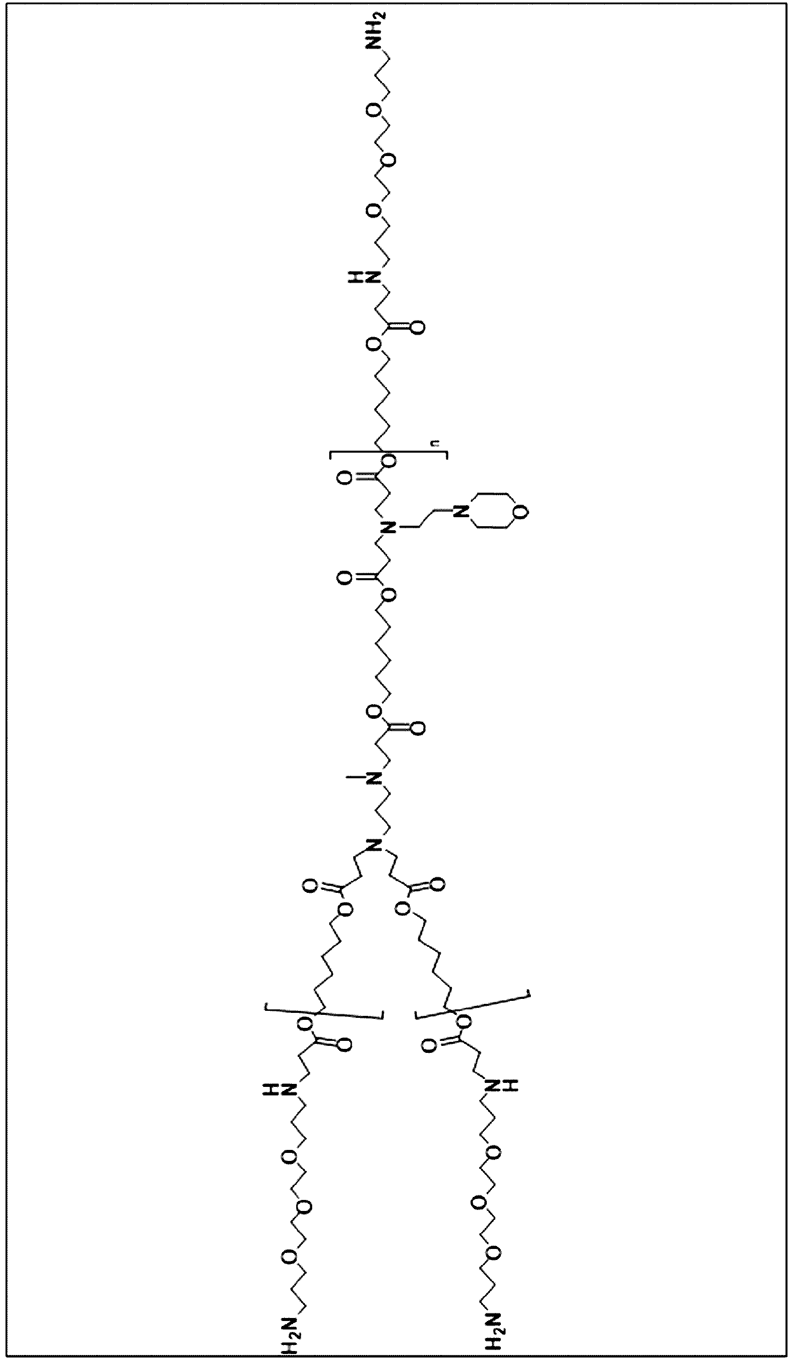

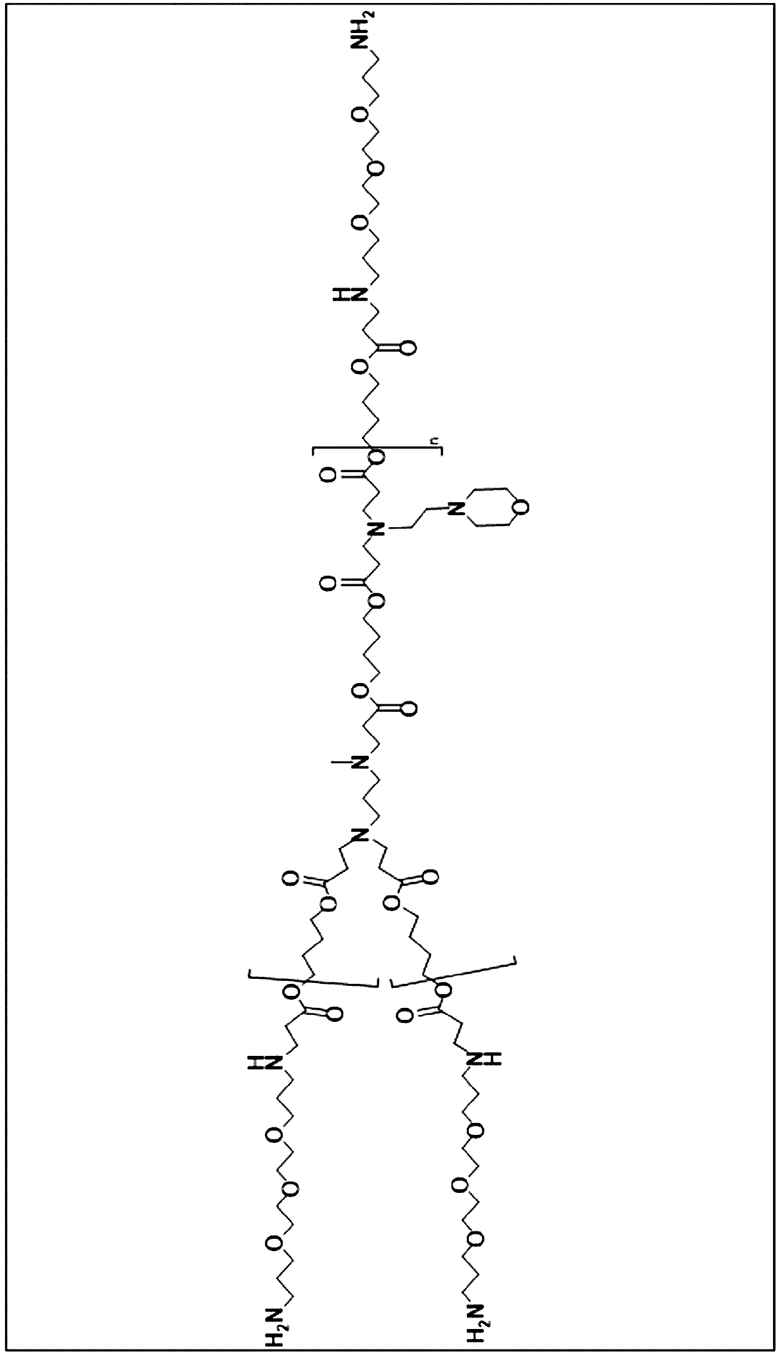

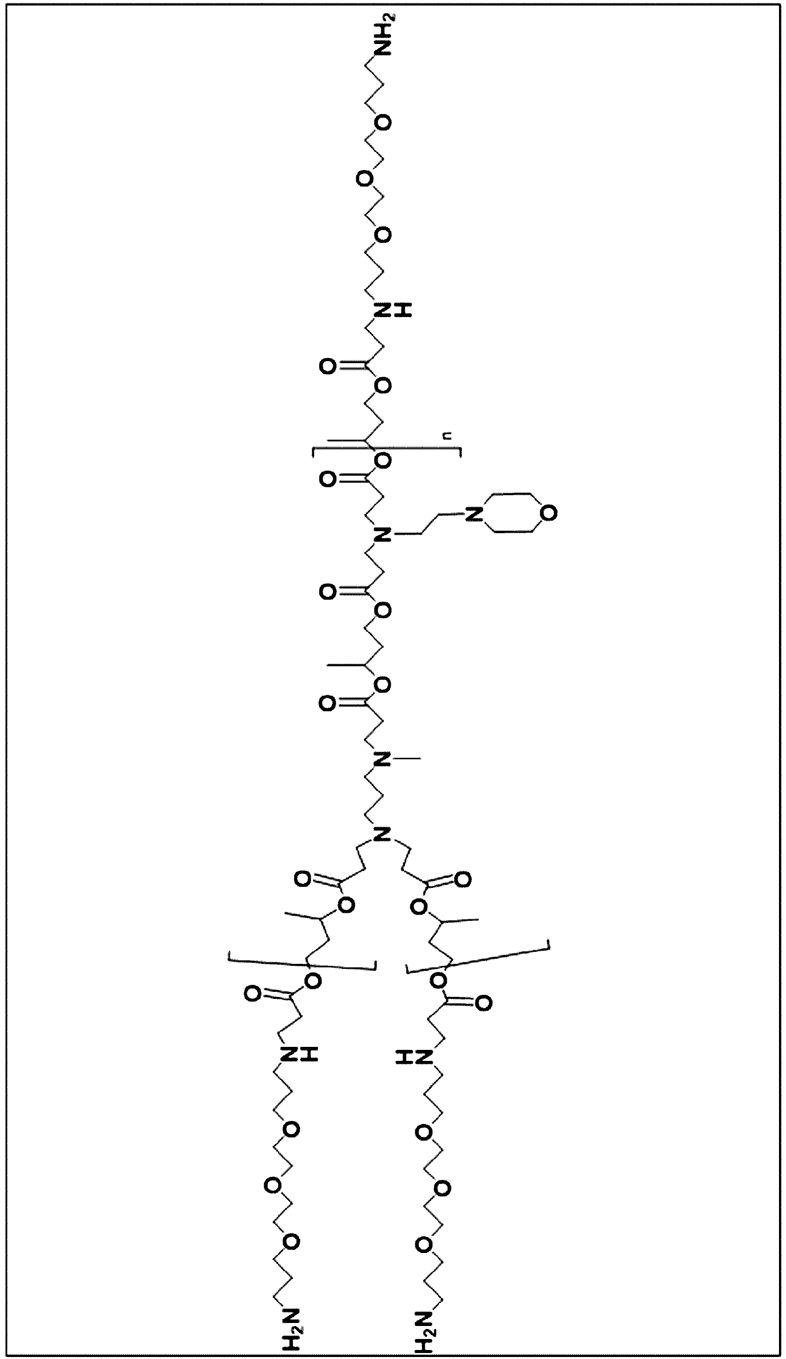

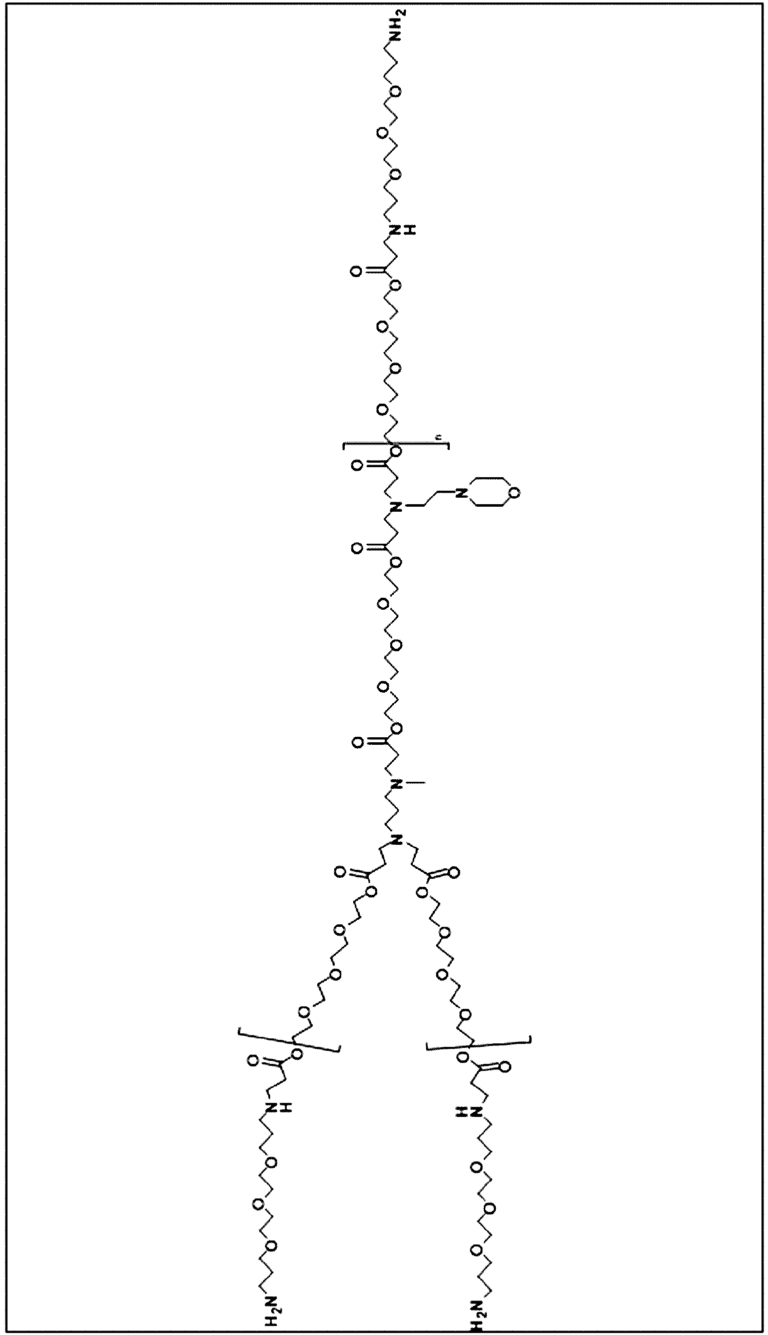

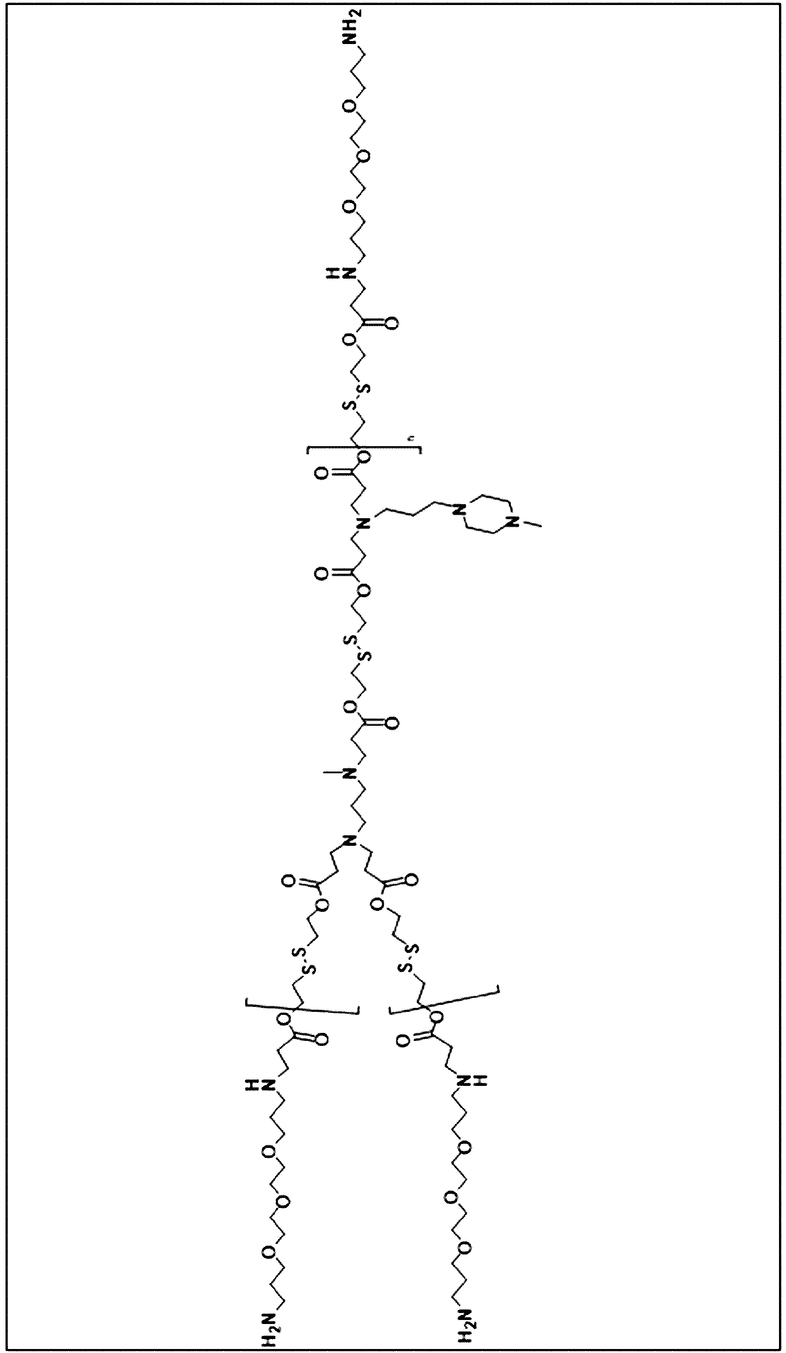

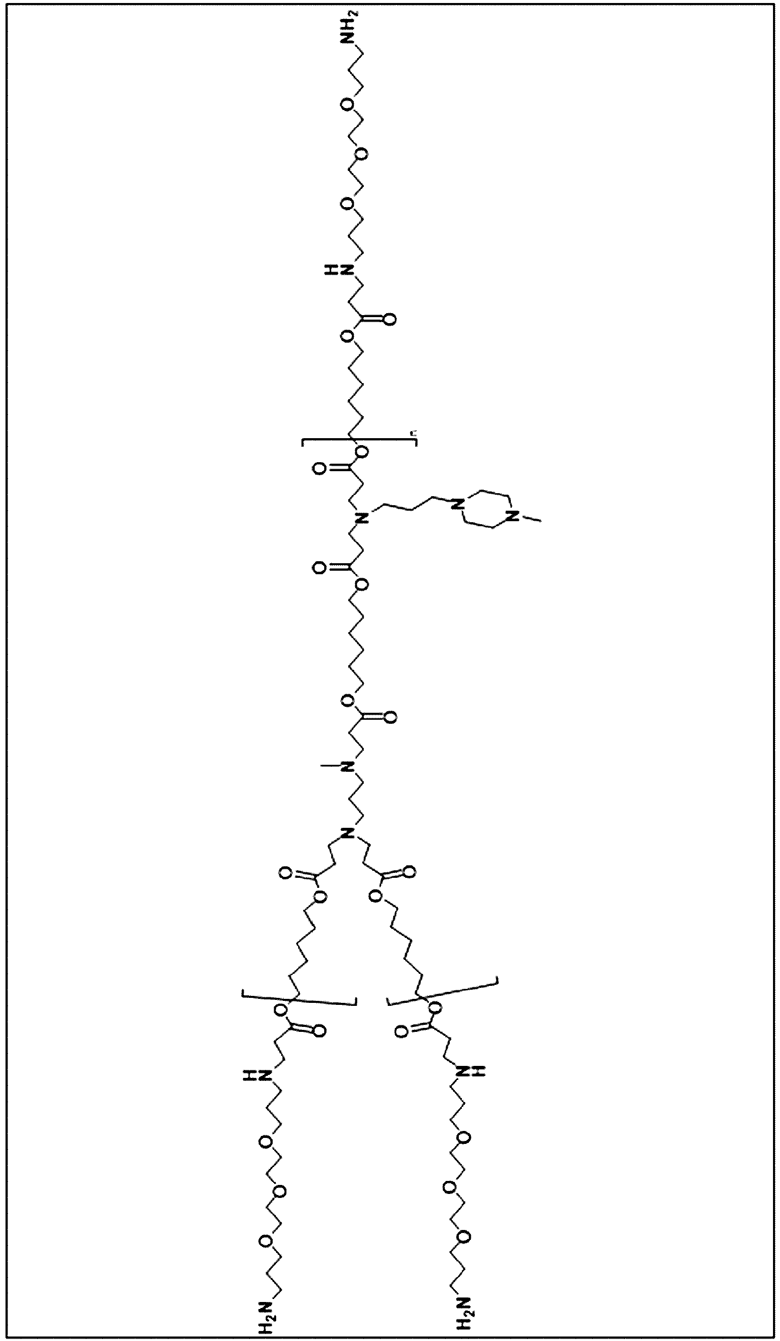

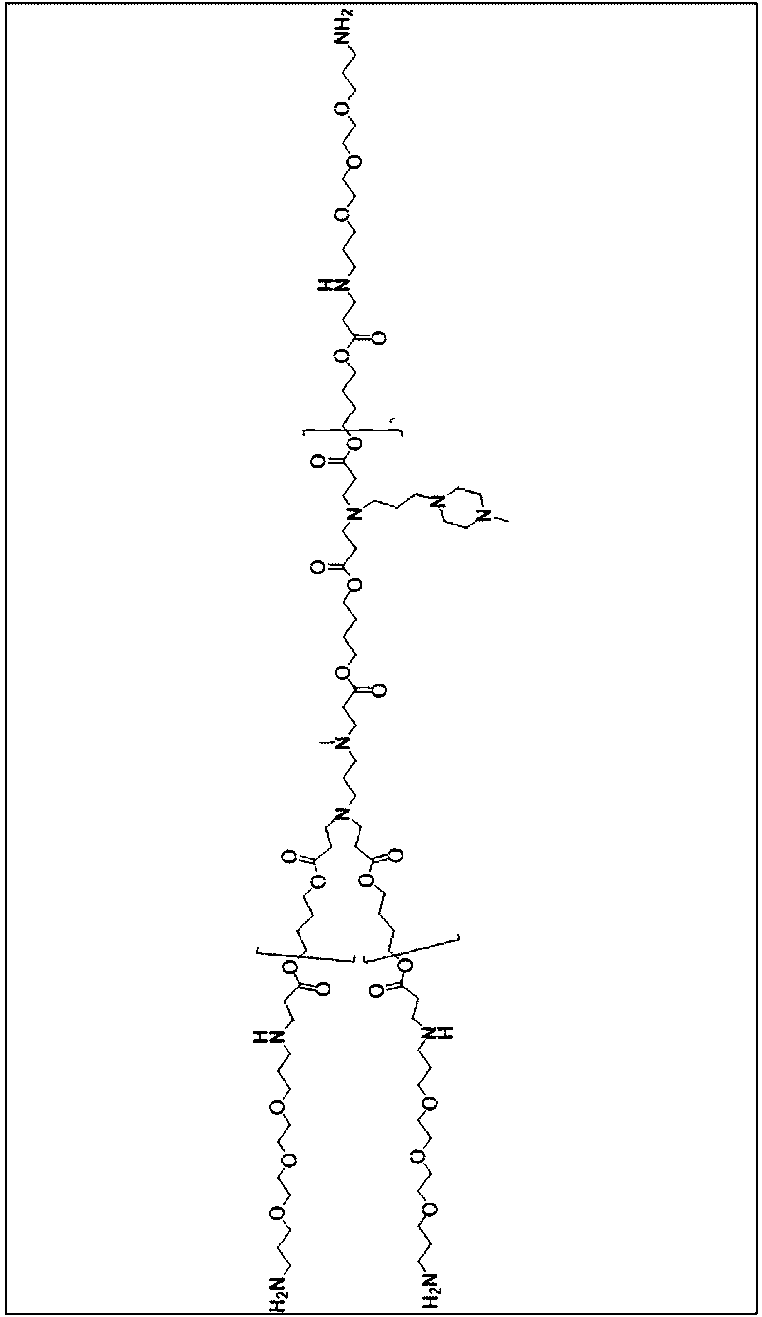

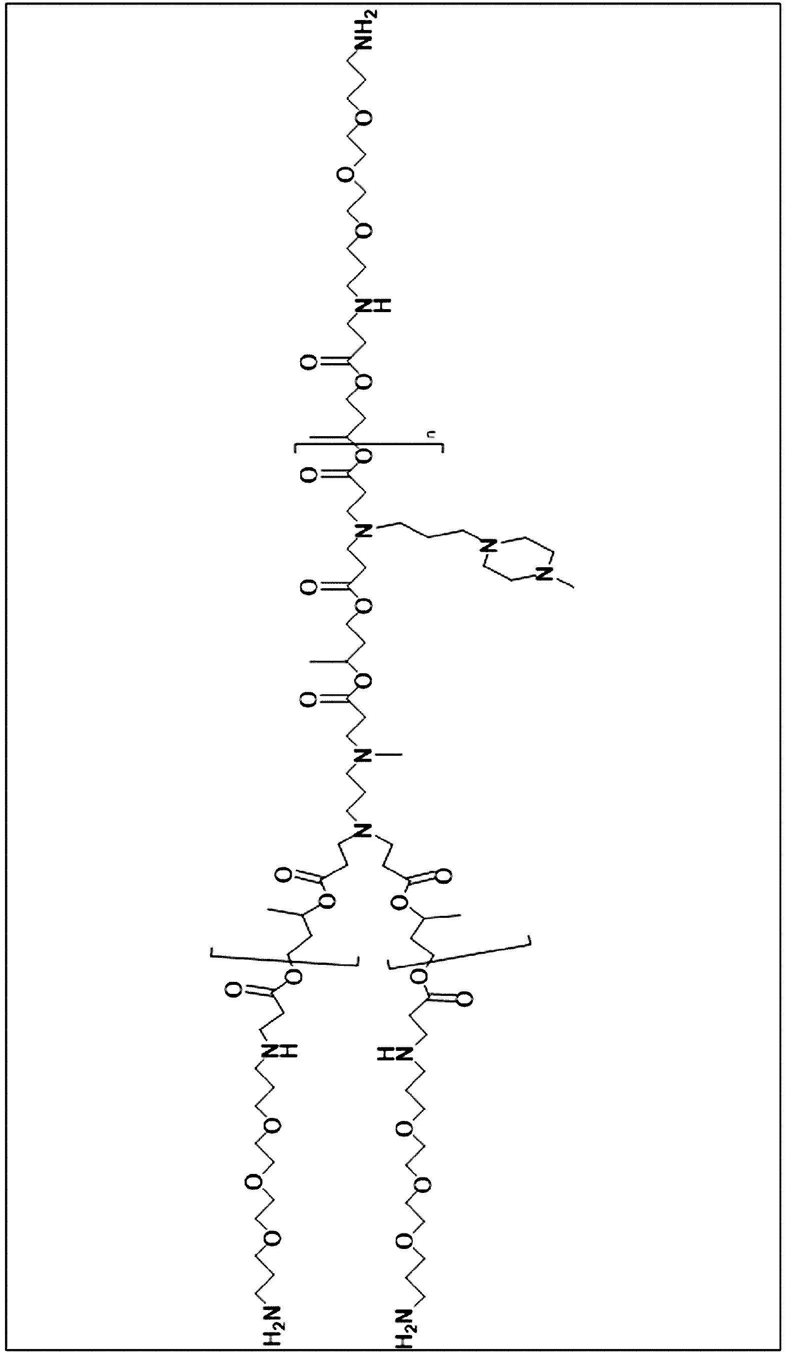

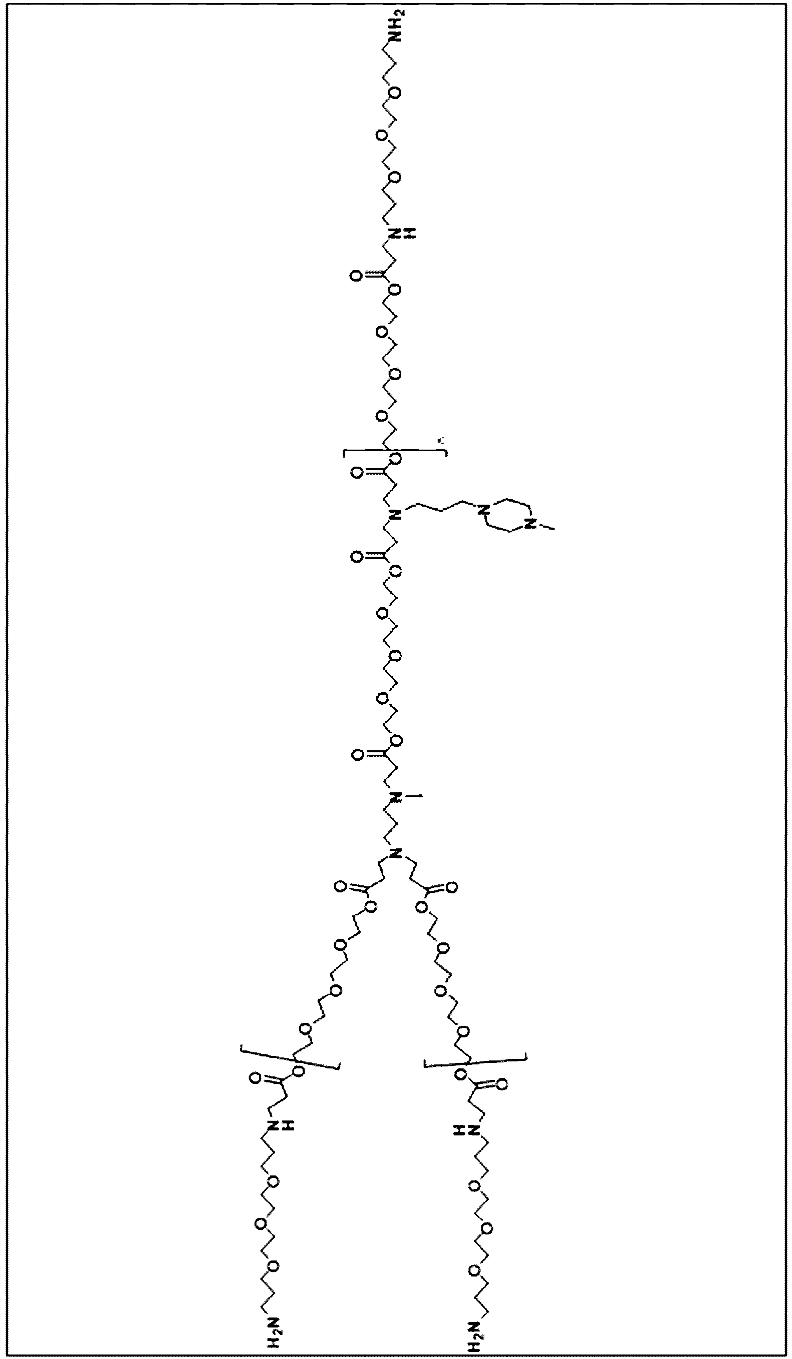

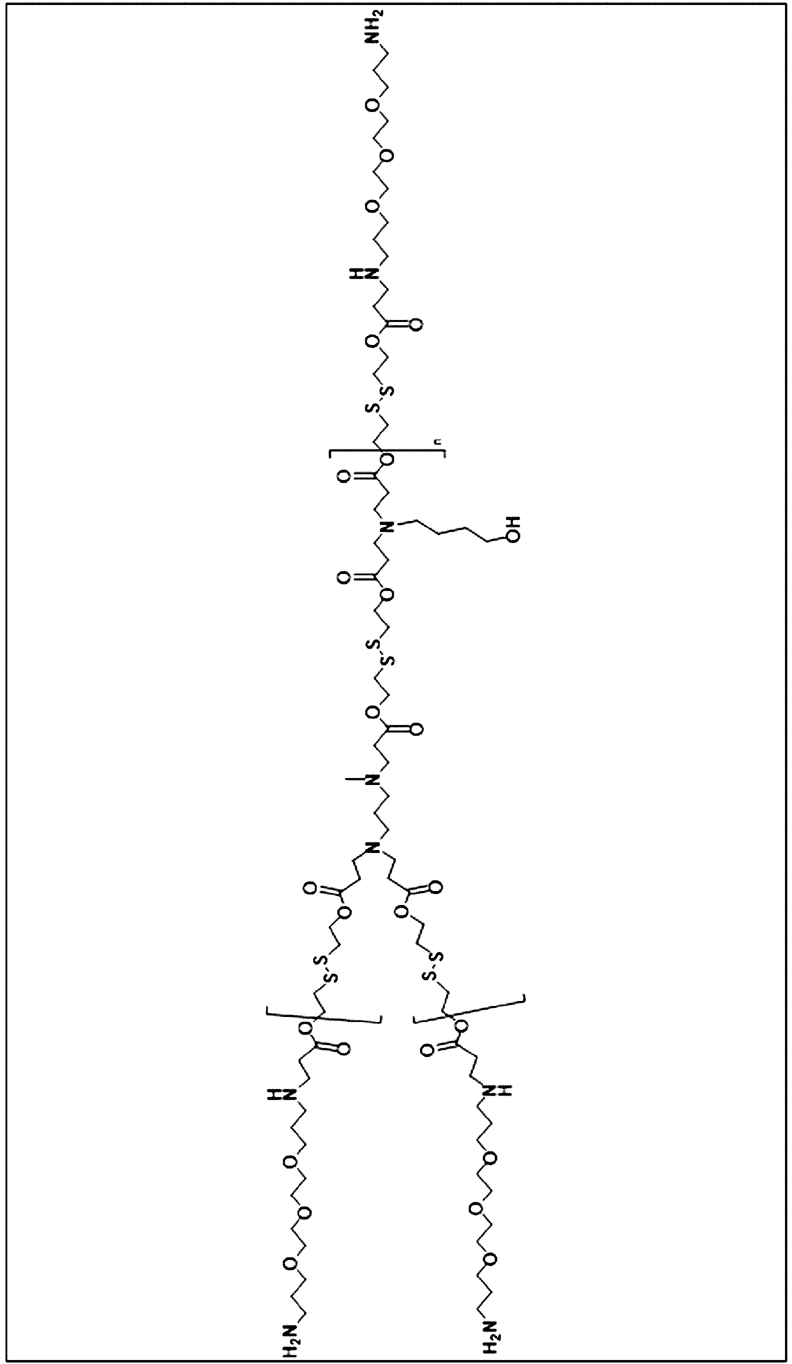

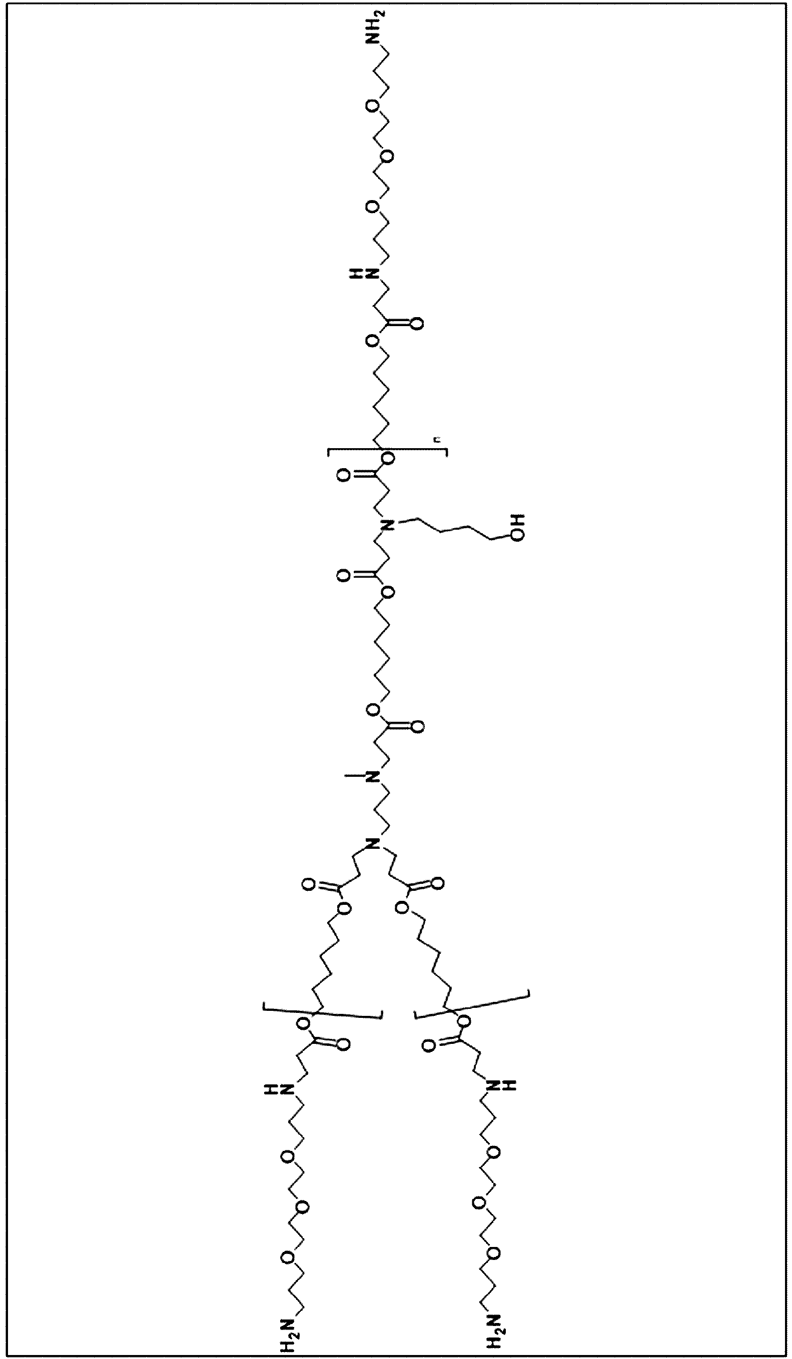

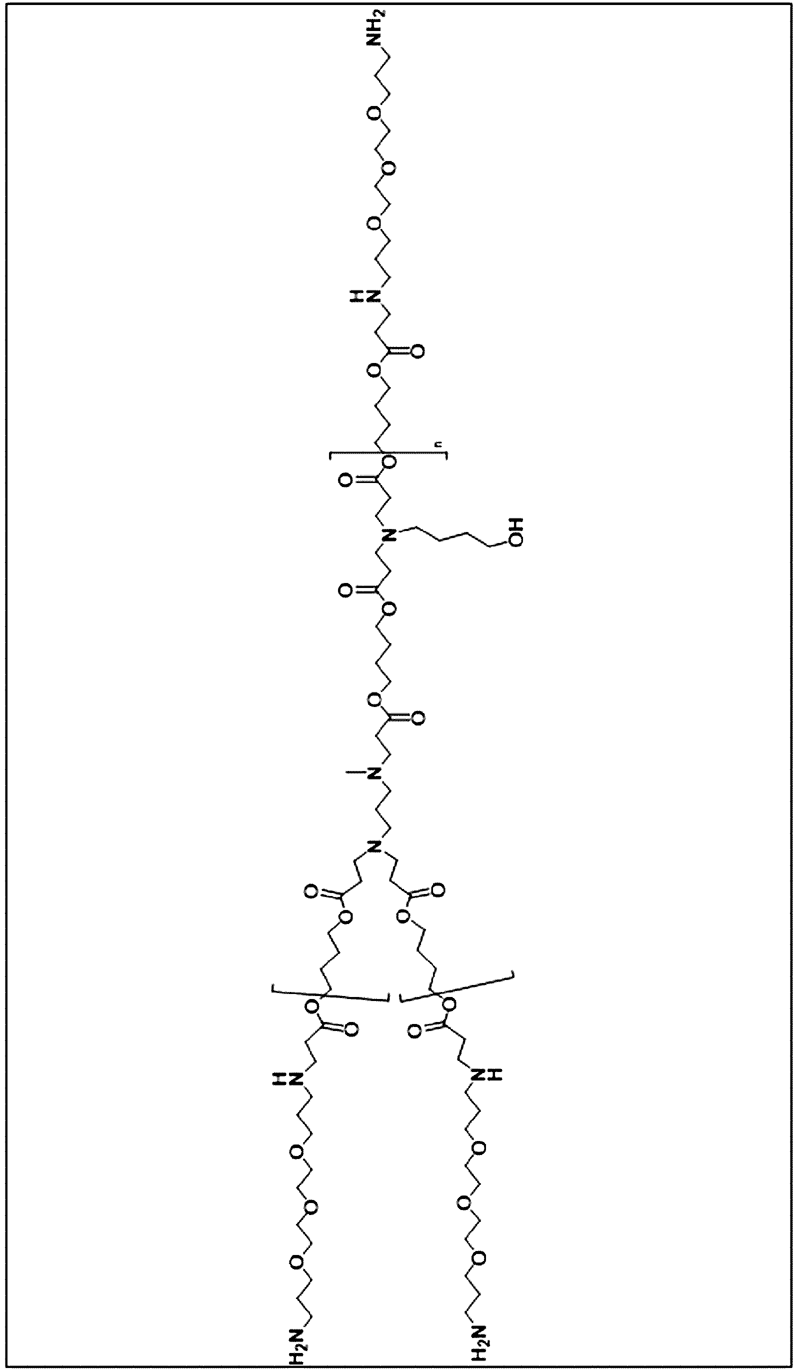

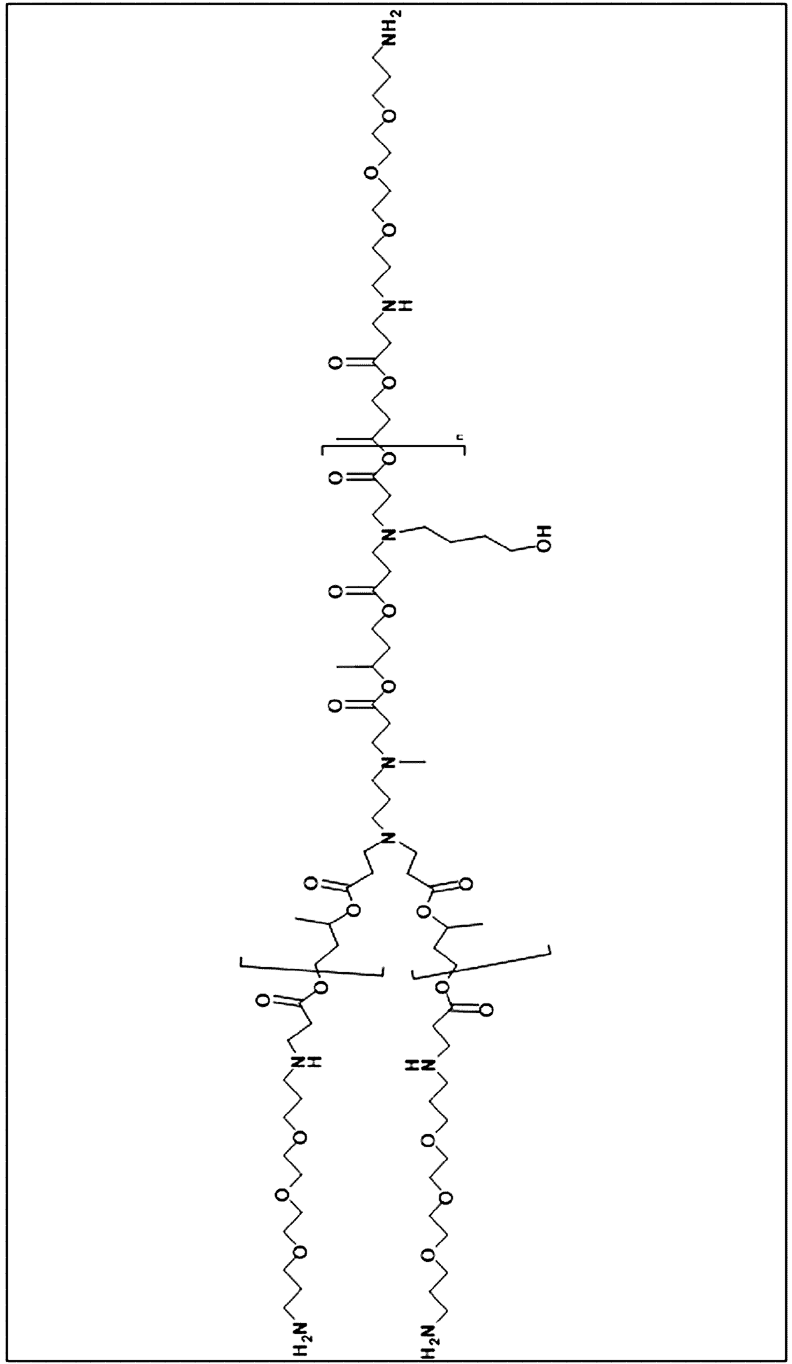

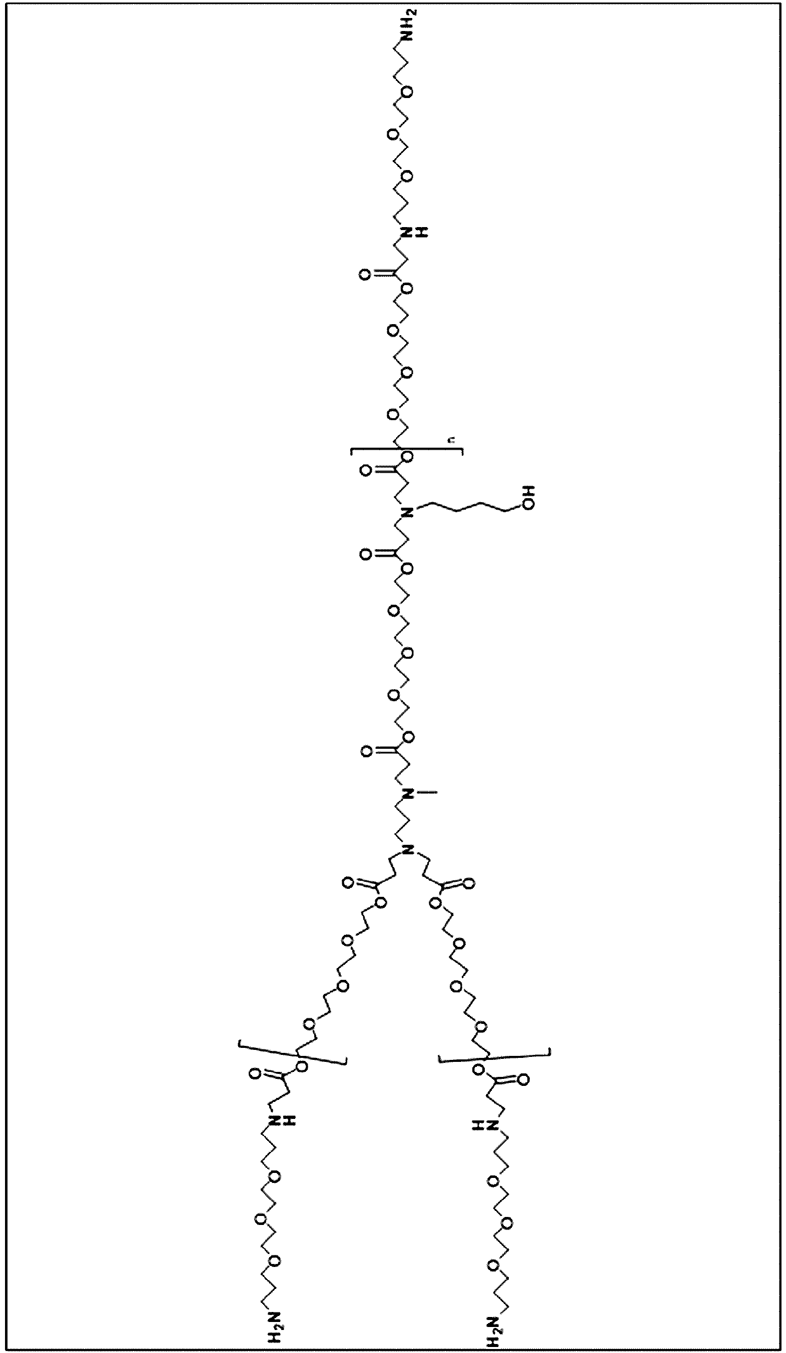

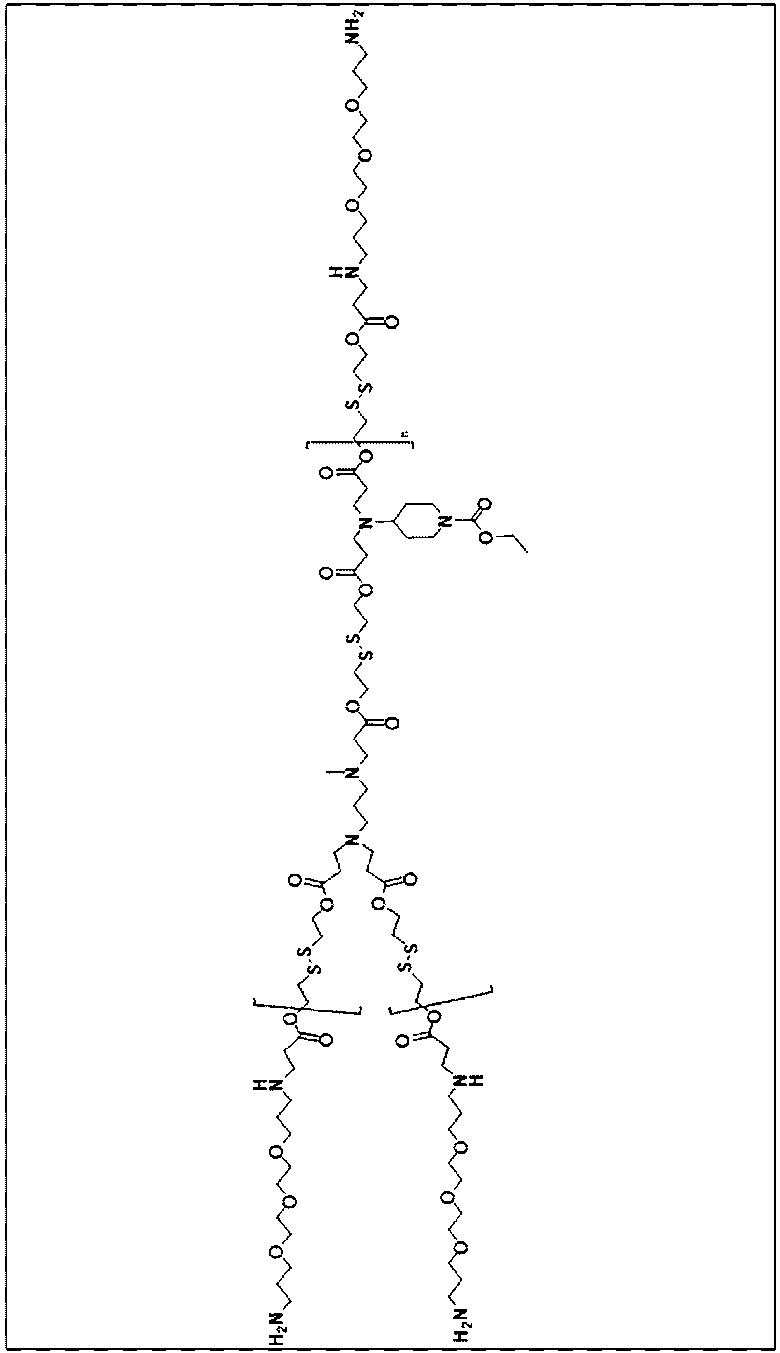

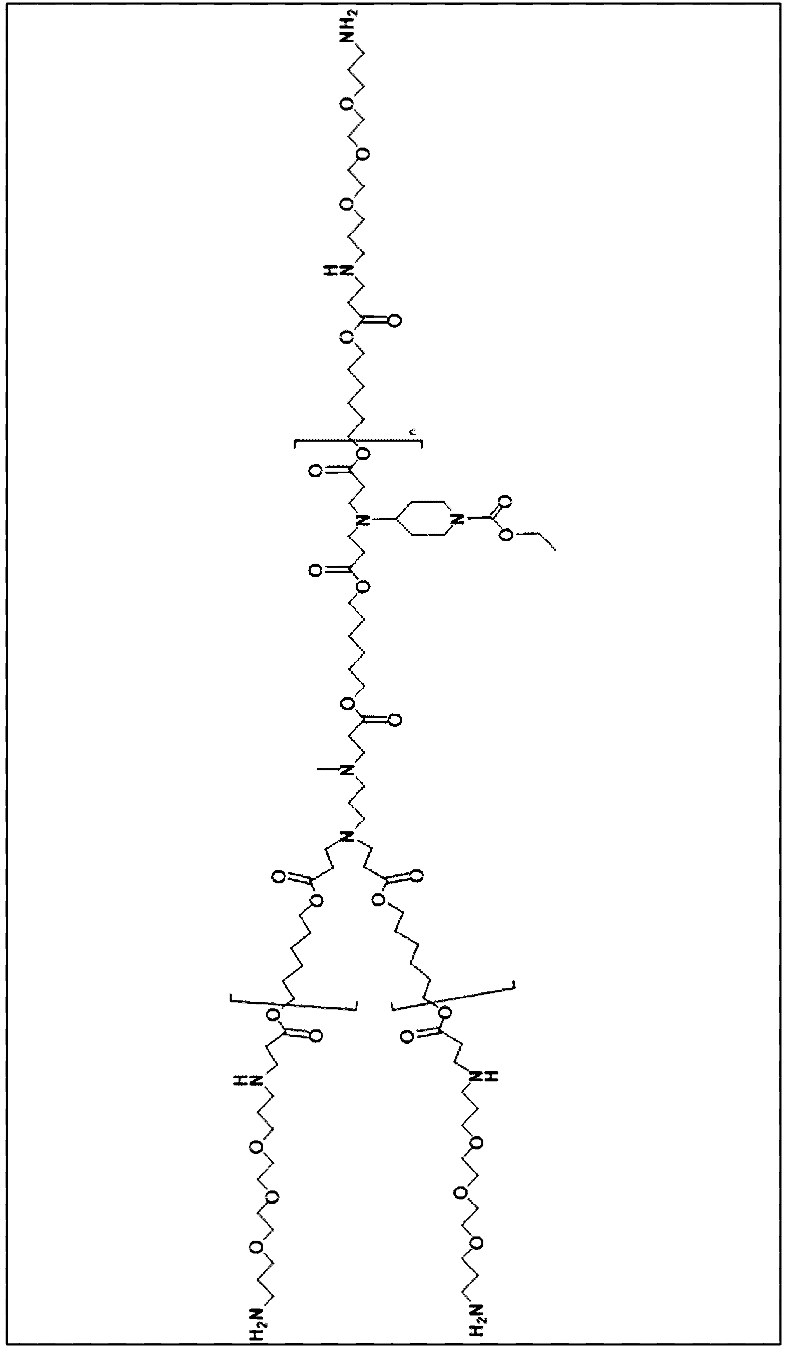

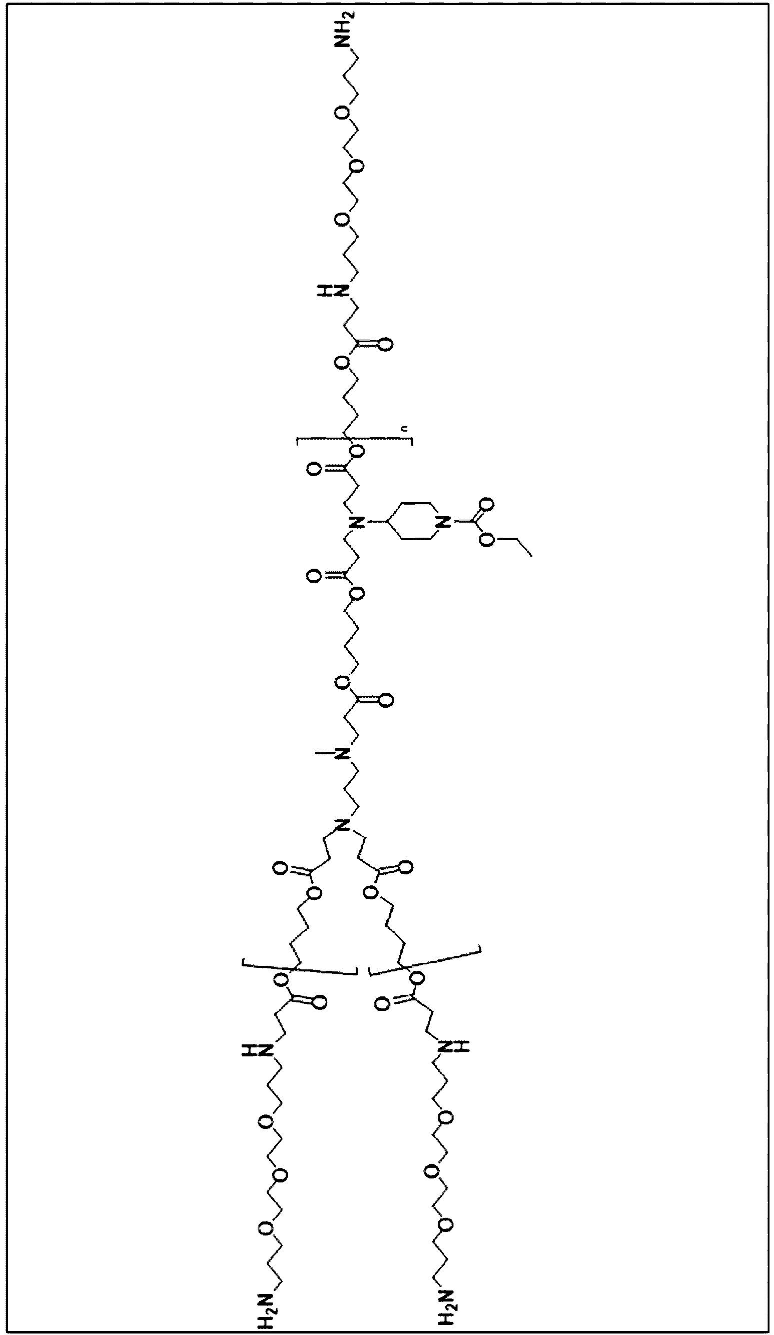

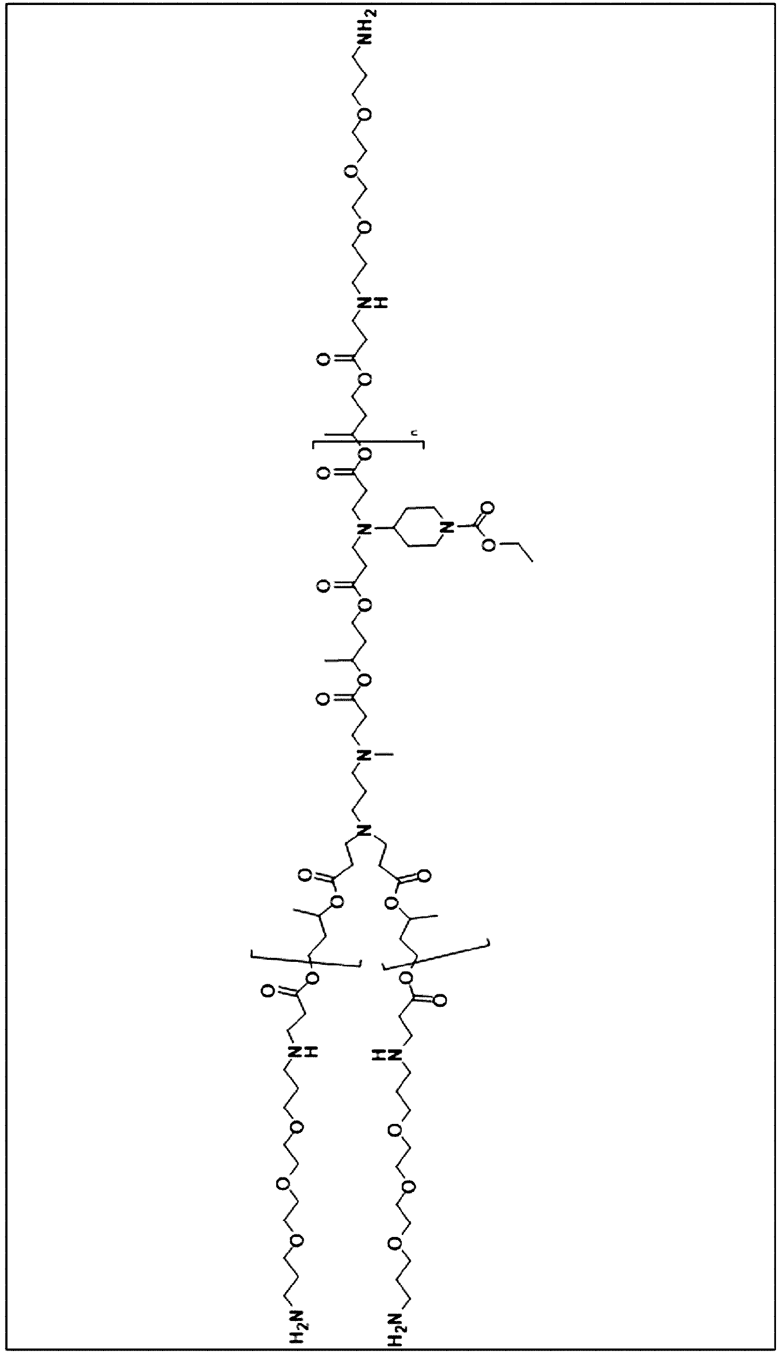

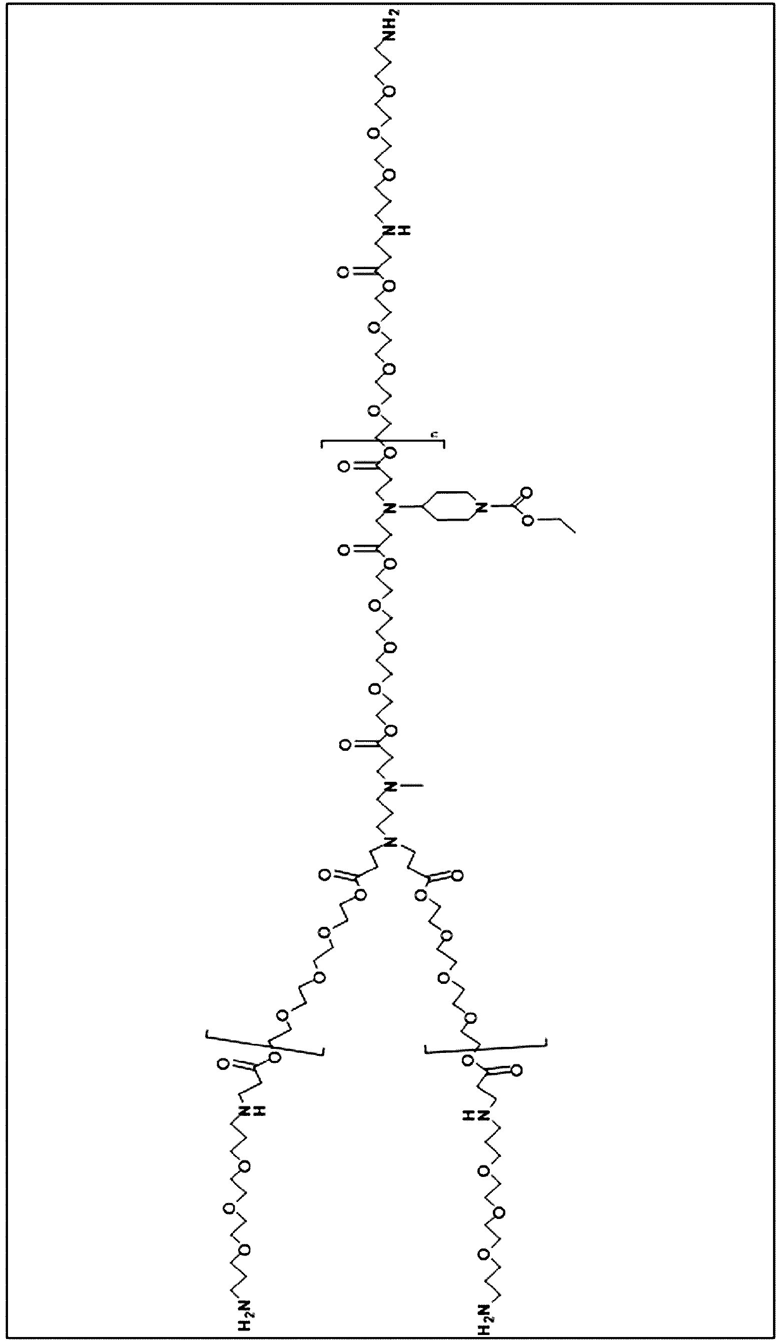

6. The polymer according to any one of claims 1 to 5, wherein the polymer comprises a branched monomer of Formula 25 below:

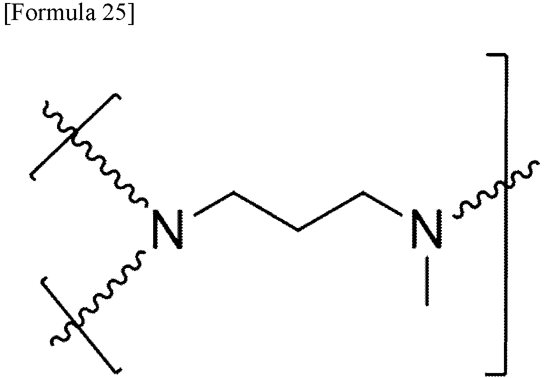

7. The polymer according to any one of claims 1, 3, and 6, wherein the polymer comprises a structure of Formula 26 below: 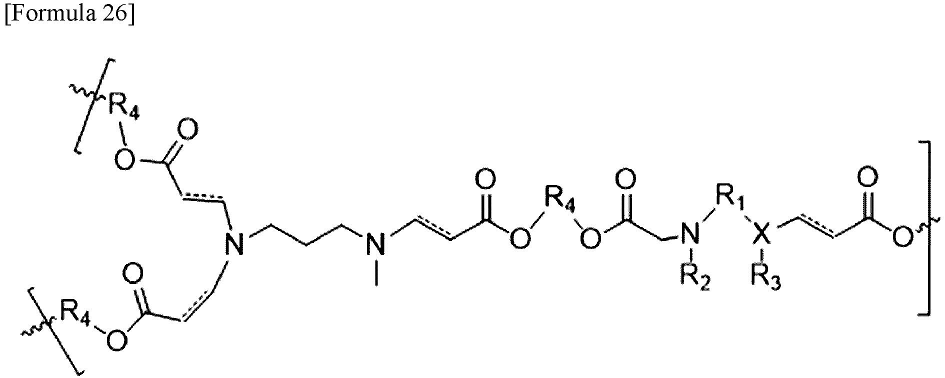 in the formula,
R1 is a bond or a C1-6 alkylene,
R2 and R3 are each independently (i) hydrogen, (ii) C1-6 alkyl which may be substituted with alkylamine or hydroxy, (iii) a 5- or 6-membered carbocycle or heterocycle, or (iv) C1-6 alkyl substituted with a 5- or 6-membered carbocycle or heterocycle,
said R2 and R3 may be linked together to form a C1-6 alkylene,
said 5- or 6-membered carbocycle or heterocycle may be substituted with C1-3 alkyl or C1-3 alkoxyacyl,
X is a nitrogen atom or a bond, and when X is a bond, said R3 is absent,
R4 is a linear or branched C1-15 alkylene, 1 to 5 carbon atoms of said alkylene may be replaced by oxygen or sulfur, and
- - - - - represents a single bond or a double bond.

8. The polymer according to any one of claims 2, 4, and 6, wherein the polymer comprises a structure of Formula 27 below: 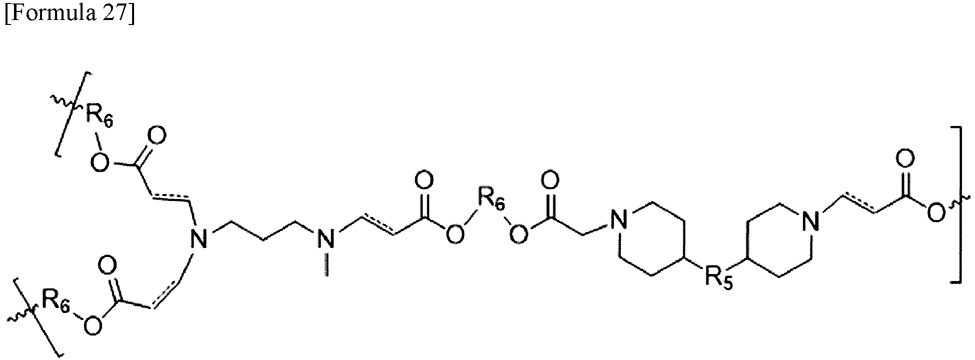 in the formula,
R5 is a C1-6 alkylene,
R6 is a linear or branched C1-15 alkylene, 1 to 5 carbon atoms of said alkylene may be replaced by oxygen or sulfur, and
- - - - - represents a single bond or a double bond.

9. The polymer according to any one of claims 1 to 8, wherein one or both ends of the polymer are linked to a PEG-containing moiety comprising a structure of Formula 28 below: 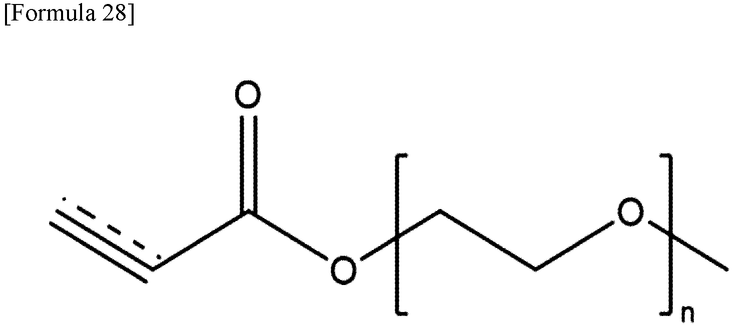 in the formula,
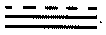 represents a double bond or a triple bond, and
n is an integer in the range of 1 to 500.

10. A polymeric nanoparticle in which the polymer according to any one of claims 1 to 9 and a nucleic acid molecule form a complex.

11. The polymeric nanoparticle according to claim 10, wherein the nucleic acid molecule is RNA.

12. The polymeric nanoparticle according to claim 10, wherein the nucleic acid molecule is DNA.

13. The polymeric nanoparticle according to any one of claims 10 to 12, wherein the nucleic acid molecule is isolated in a pH-dependent manner and delivered into cells in the body.

14. The polymeric nanoparticle according to claim 11, wherein when delivered into the body, the expression of the nucleic acid molecule is maintained for a longer period of time compared to when the same nucleic acid molecule is delivered into the body in the form of a non-polymeric lipid nanoparticle.

15. A method for delivering a nucleic acid molecule into a cell using the polymeric nanoparticle according to any one of claims 10 to 14.

16. The method according to claim 15, wherein the polymeric nanoparticle is delivered by parenteral, intramuscular, subcutaneous, or intravenous administration.

17. A pharmaceutical composition comprising the polymeric nanoparticle according to any one of claims 10 to 14.

18. The pharmaceutical composition according to claim 17, wherein the pharmaceutical composition is a gene therapeutic agent.

19. The pharmaceutical composition according to claim 17, wherein the pharmaceutical composition is a vaccine.
